# EUROPEAN PATENT SPECIFICATION

(11) **EP 3 274 454 B1**
(45) Date of publication and mention of the grant of the patent: **25.08.2021**
(21) Application number: 16717736.9
(22) Date of filing: 25.03.2016
(51) Int. Cl.: C12N 15/11, C12N 15/113

(54) **CRISPR/CAS-RELATED METHODS, COMPOSITIONS AND COMPONENTS**
CRISPR-/CAS-VERWANDTE VERFAHREN, ZUSAMMENSETZUNGEN UND KOMPONENTEN
PROCÉDÉS, COMPOSITIONS ET CONSTITUANTS LIÉS À CRISPR/CAS

(30) Priority: 25.03.2015 US 201562138273 P; 26.03.2015 US 201562138939 P; 01.04.2015 US 201562141810 P; 11.05.2015 US 201562159932 P; 24.06.2015 US 201562184103 P; 26.08.2015 US 201562210392 P; 18.09.2015 US 201562220815 P; 21.10.2015 US 201562244572 P
(43) Date of publication of application: 31.01.2018
(60) Divisional application: 21186459.0
(73) Proprietor: Editas Medicine, Inc., Cambridge, MA 02142 (US)
(72) Inventor: WELSTEAD, Gordon Grant, Cambridge, Massachusetts 02139 (US); GORI, Jennifer Leah, Jamaica Plain, Massachusetts 02130 (US); HEATH, Jack Michael, Boston, Massachusetts 02118 (US); BUMCROT, David A., Belmont, Massachusetts 02478 (US)
(74) Representative: Uexküll & Stolberg
(86) International application number: PCT/US2016/024353
(87) International publication number: WO 2016/154596

(56) References cited:
- WO-A2-2015/006747
- WO-A2-2015/161276
- US-A1- 2015 031 132
- B. FARBOUD ET AL: "Dramatic Enhancement of Genome Editing by CRISPR/Cas9 Through Improved Guide RNA Design", GENETICS, vol. 199, no. 4, 18 February 2015 (2015-02-18), pages 959-971, XP055284263, US ISSN: 0016-6731, DOI: 10.1534/genetics.115.175166

## Description

### FIELD OF THE INVENTION

The invention relates to CRISPR/Cas-related methods, compositions and components for editing a target nucleic acid sequence, or modulating expression of a target nucleic acid sequence, and applications thereof in connection with certain diseases, cell types and genes.

### BACKGROUND

The CRISPR (Clustered Regularly Interspaced Short Palindromic Repeats)/Cas (CRISPR-associated) system evolved in bacteria and archaea as an adaptive immune system to defend against viral attack. Upon exposure to a virus, short segments of viral DNA are integrated into the CRISPR locus. RNA is transcribed from a portion of the CRISPR locus that includes the viral sequence. That RNA, which contains sequence complementary to the viral genome, mediates targeting of a Cas protein (e.g., Cas9 protein) to the sequence in the viral genome. The Cas protein cleaves and thereby silences the viral target.

Recently, the CRISPR/Cas system has been adapted for genome editing in eukaryotic cells. The introduction of site-specific double strand breaks (DSBs) enables target sequence alteration through one of two endogenous DNA repair mechanisms-either non-homologous end-joining (NHEJ) or homology-directed repair (HDR). The CRISPR/Cas system has also been used for gene regulation including transcription repression and activation without altering the target sequence. Targeted gene regulation based on the CRISPR/Cas system can, for example, use an enzymatically inactive Cas9 (also known as a catalytically dead Cas9).

B. Farboud ET AL, 2015 discloses a GG motif at 3' end of guide RNA.

WO 2015006747 teaches capping of gRNA by 5' ARCA cap but does not disclose such modified sgRNAs explicitly complexed with Cas9

US 2015/031132 also teaches either phosphatase treated or 5' capped modified RNA encoding gRNA and introduction of the RNA into Cas9-iPSCs in complex with liposome.

### SUMMARY

In one aspect, methods and compositions discussed herein provide for efficient *in vitro* or *ex vivo* gene editing of eukaryotic cells. In particular, we have found that the guide RNA (gRNA) component of the CRISPR/Cas system is more efficient at editing genes in certain cell types *ex vivo* when it has been modified at or near its 5' end (e.g., when the 5' end of a gRNA is modified by the inclusion of a eukaryotic mRNA cap structure or cap analog) and/or when it has been modified to include a 3' polyA tail. gRNA is not an mRNA and it was therefore unexpected that these mRNA structures should lead to such an improvement. We have also demonstrated that the presence of a 3' polyA tails with defined lengths can also lead to improved gene editing and that the combination of a 5' end modification and a 3' polyA tail can lead to even further improvements. While not wishing to be bound by theory it is believed that these and other modified gRNAs described herein exhibit enhanced stability with certain cell types (e.g., circulating cells such as T cells) and that this might be responsible for the observed improvements.

The present invention also encompasses the realization that the improvements observed with a gRNA with 3' polyA tails and/or 5' caps can be extended to gRNAs that have been modified in other ways to achieve the same type of structural or functional result (e.g., by the inclusion of modified nucleosides or nucleotides, or when an *in vitro* transcribed gRNA is modified by treatment with a phosphatase to remove the 5' triphosphate group).

Thus, in some embodiments, the present invention provides a composition comprising a gRNA molecule comprising a targeting domain which is complementary with a target domain from a gene expressed in a eukaryotic cell, wherein the gRNA molecule comprises a 3' polyA tail and is complexed with a Cas9 protein.

Preferably, the gRNA molecule is modified at its 5' end such that it lacks a 5' triphosphate group. The gRNA molecule may be complexed with the Cas9 protein in a ribonucleoprotein (RNP) complex. In some embodiments the 3' polyA tail comprises between 15 and 25 adenine nucleotides.

The present invention also provides *in vitro* or *ex vivo* methods for editing or modulating expression of a gene in a eukaryotic cell from a subject, the method comprising contacting the cell with a composition comprising a gRNA molecule as described above.

### BRIEF DESCRIPTION OF THE DRAWINGS

**Figs. 1A-1G** are representations of several exemplary gRNAs.
**Fig. 1A** depicts a modular gRNA molecule derived in part (or modeled on a sequence in part) from *Streptococcus pyogenes (S. pyogenes)* as a duplexed structure (SEQ ID NO:42 and 43, respectively, in order of appearance);
**Fig. 1B** depicts a unimolecular (or chimeric) gRNA molecule derived in part from *S*. *pyogenes* as a duplexed structure (SEQ ID NO:44);
**Fig. 1C** depicts a unimolecular gRNA molecule derived in part from *S. pyogenes* as a duplexed structure (SEQ ID NO:45);
**Fig. 1D** depicts a unimolecular gRNA molecule derived in part from *S. pyogenes* as a duplexed structure (SEQ ID NO:46);
**Fig. 1E** depicts a unimolecular gRNA molecule derived in part from *S. pyogenes* as a duplexed structure (SEQ ID NO:47);
**Fig. 1F** depicts a modular gRNA molecule derived in part from *Streptococcus thermophilus (S. thermophilus)* as a duplexed structure (SEQ ID NO:48 and 49, respectively, in order of appearance);
**Fig. 1G** depicts an alignment of modular gRNA molecules of *S. pyogenes* and *S*. *thermophilus* (SEQ ID NO:50-53, respectively, in order of appearance).

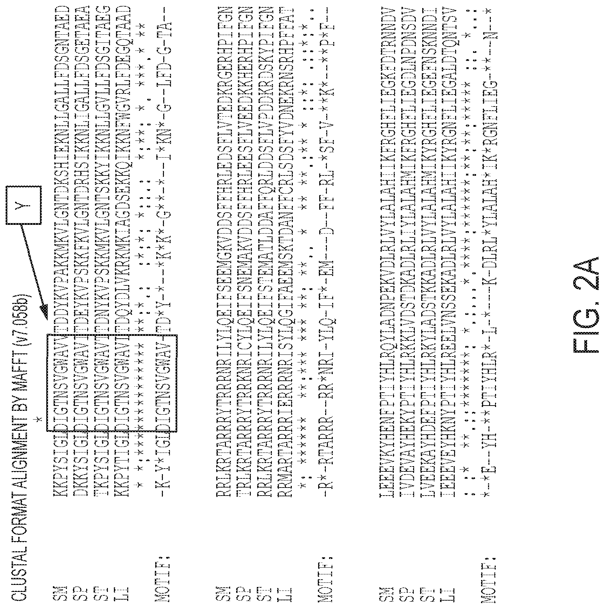

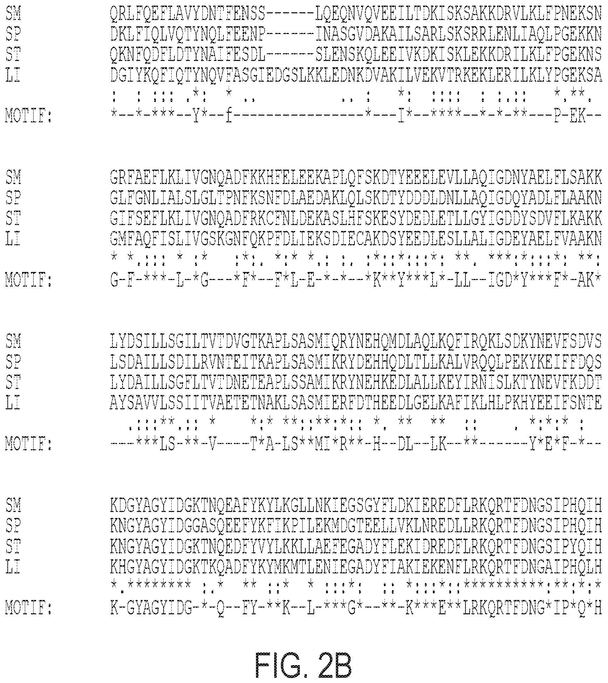

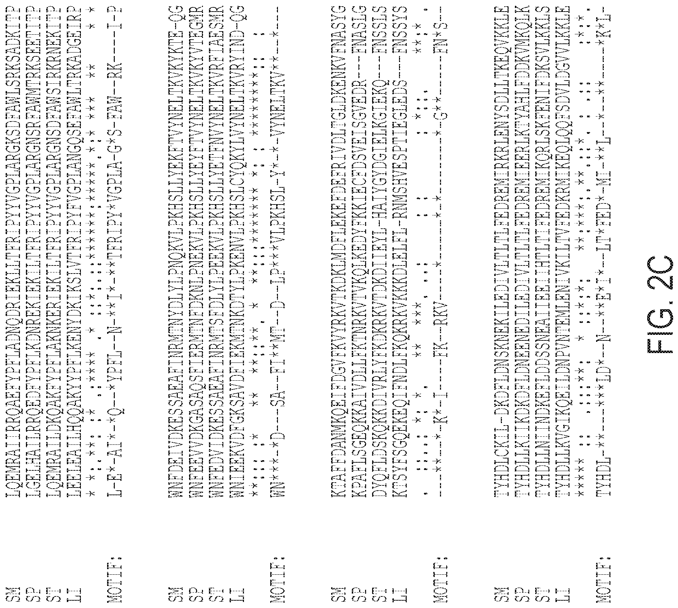

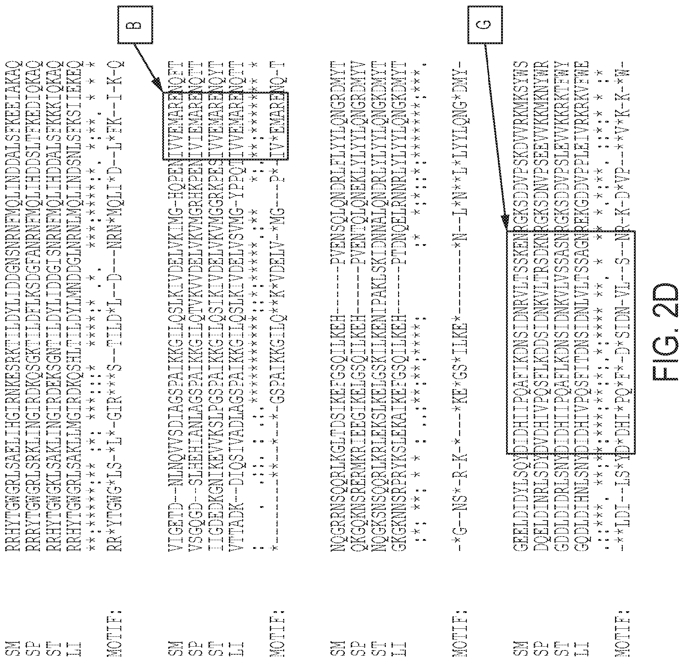

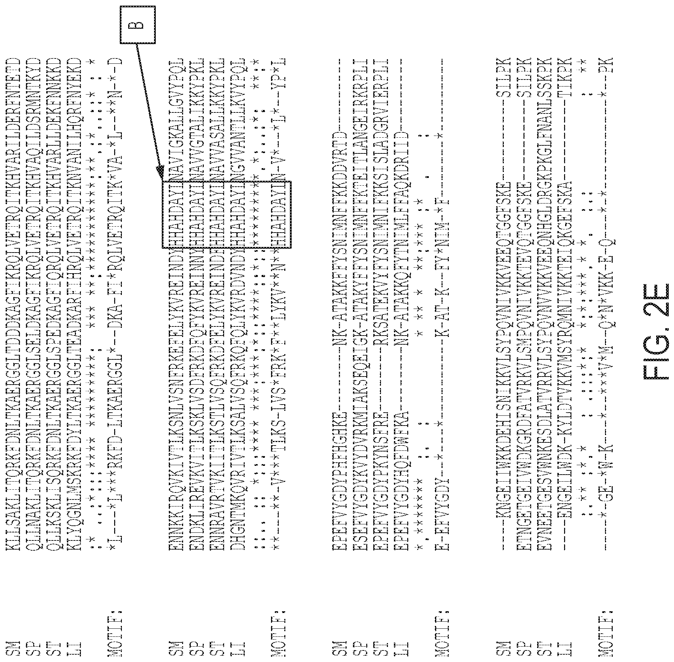

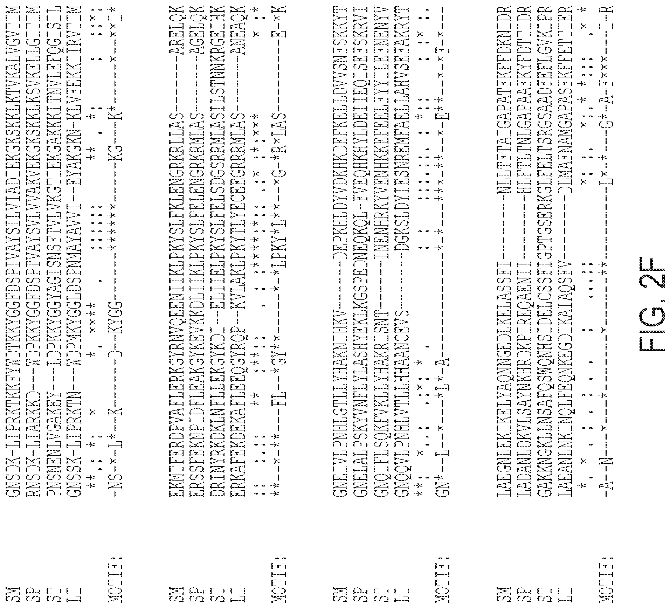

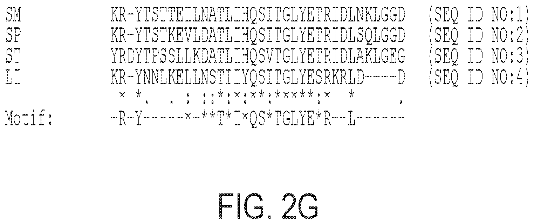

**Figs. 2A-2G** depict an alignment of Cas9 sequences from Chylinski et al. (RNA Biol. 2013; 10(5): 726-737). The N-terminal RuvC-like domain is boxed and indicated with a "Y". The other two RuvC-like domains are boxed and indicated with a "B". The HNH-like domain is boxed and indicated by a "G". Sm: *S. mutans* (SEQ ID NO:1); Sp: *S. pyogenes* (SEQ ID NO:2); St: *S. thermophilus* (SEQ ID NO:3); Li: *L. innocua* (SEQ ID NO:4). Motif: this is a motif based on the four sequences: residues conserved in all four sequences are indicated by single letter amino acid abbreviation; "*" indicates any amino acid found in the corresponding position of any of the four sequences; and "-" indicates any amino acid, e.g., any of the 20 naturally occurring amino acids.
**Figs. 3A-3B** show an alignment of the N-terminal RuvC-like domain from the Cas9 molecules disclosed in Chylinski *et al* (SEQ ID NO:54-103, respectively, in order of appearance). The last line of **Fig. 3B** identifies 4 highly conserved residues.
**Figs. 4A-4B** show an alignment of the N-terminal RuvC-like domain from the Cas9 molecules disclosed in Chylinski *et al.* with sequence outliers removed (SEQ ID NO: 104-177, respectively, in order of appearance). The last line of **Fig. 4B** identifies 3 highly conserved residues.
**Figs. 5A-5C** show an alignment of the HNH-like domain from the Cas9 molecules disclosed in Chylinski *et al* (SEQ ID NO: 178-252, respectively, in order of appearance). The last line of **Fig. 5C** identifies conserved residues.
**Figs. 6A-6B** show an alignment of the HNH-like domain from the Cas9 molecules disclosed in Chylinski *et al.* with sequence outliers removed (SEQ ID NO:253-302, respectively, in order of appearance). The last line of **Fig. 6B** identifies 3 highly conserved residues.

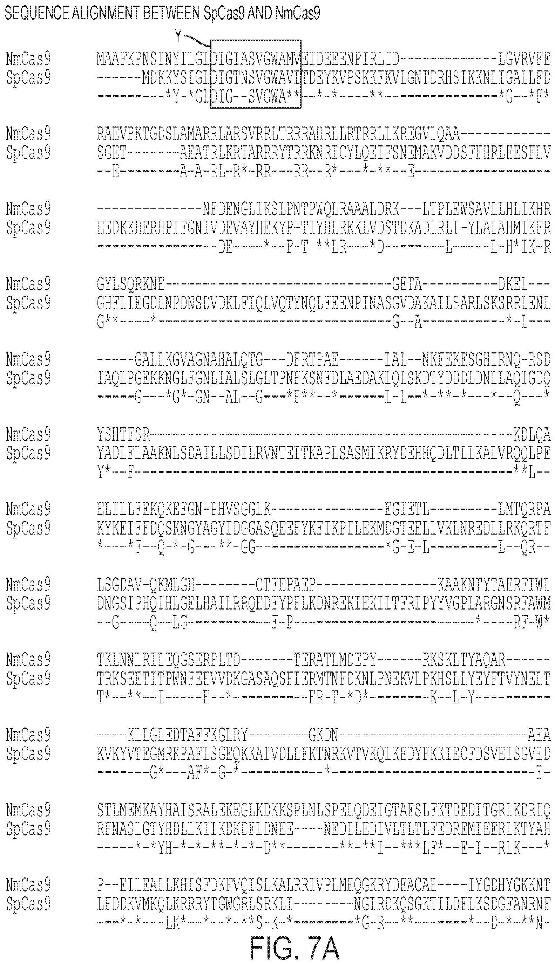

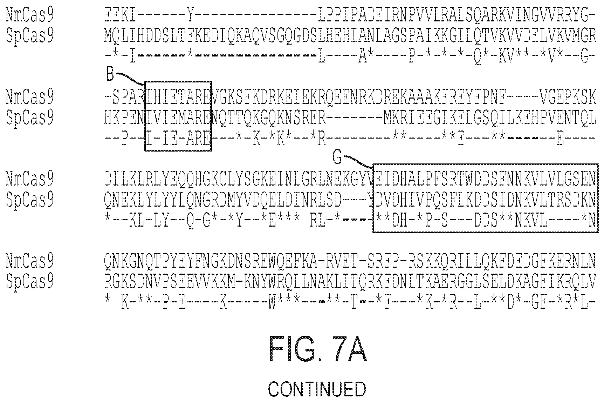

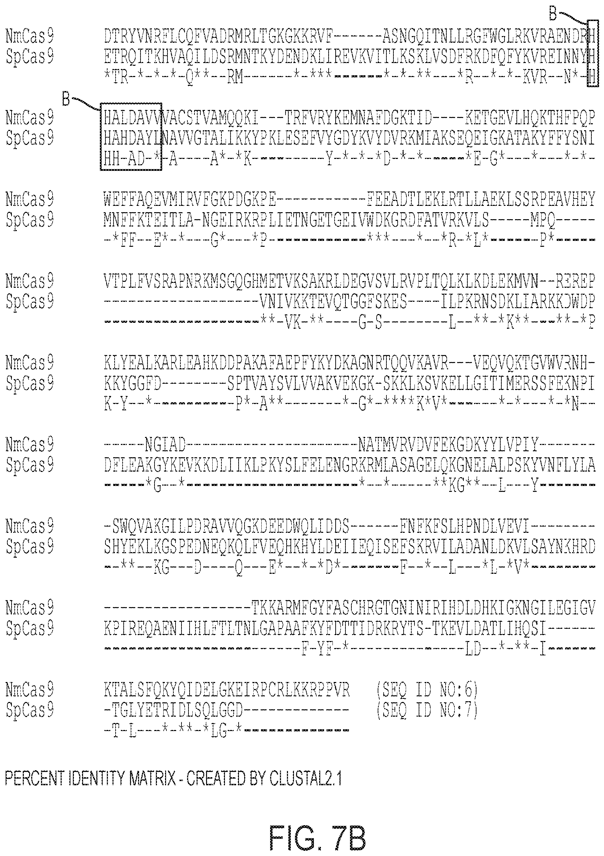

**Figs. 7A-7B** depict an alignment of Cas9 sequences from *S. pyogenes* and *Neisseria meningitidis (N. meningitidis).* The N-terminal RuvC-like domain is boxed and indicated with a "Y". The other two RuvC-like domains are boxed and indicated with a "B". The HNH-like domain is boxed and indicated with a "G". Sp: *S. pyogenes;* Nm: *N. meningitidis.* Motif: this is a motif based on the two sequences: residues conserved in both sequences are indicated by a single amino acid designation; "*" indicates any amino acid found in the corresponding position of any of the two sequences; "-" indicates any amino acid, e.g., any of the 20 naturally occurring amino acids, and "-" indicates any amino acid, e.g., any of the 20 naturally occurring amino acids, or absent.
**Fig. 8** shows a nucleic acid sequence encoding Cas9 of *N. meningitidis* (SEQ ID NO:303). Sequence indicated by an "R" is an SV40 NLS; sequence indicated as "G" is an HA tag; and sequence indicated by an "O" is a synthetic NLS sequence; the remaining (unmarked) sequence is the open reading frame (ORF).
**Fig. 9A** shows the organization of the Cas9 domains, including amino acid positions, in reference to the two lobes of Cas9 (recognition (REC) and nuclease (NUC) lobes).
**Fig. 9B** shows the percent homology of each domain across 83 Cas9 orthologs.
**Fig. 10A** shows an exemplary structure of a unimolecular gRNA molecule derived in part from *S. pyogenes* as a duplexed structure (SEQ ID NO:40).
**Fig. 10B** shows an exemplary structure of a unimolecular gRNA molecule derived in part from *S. aureus* as a duplexed structure (SEQ ID NO:41).
**Fig. 11** shows the structure of the 5' ARCA cap.
**Fig. 12** depicts results from the quantification of live Jurkat T cells post electroporation with Cas9 mRNA and *AAVS1* gRNAs. Jurkat T cells were electroporated with *S. pyogenes* Cas9 mRNA and the respective modified gRNA. 24 hours after electroporation, 1 x 10⁵ cells were stained with FITC-conjugated Annexin-V specific antibody for 15 minutes at room temperature followed by staining with propidium iodide immediately before analysis by flow cytometry. The percentage of cells that did not stain for either Annexin-V or PI is presented in the bar graph.

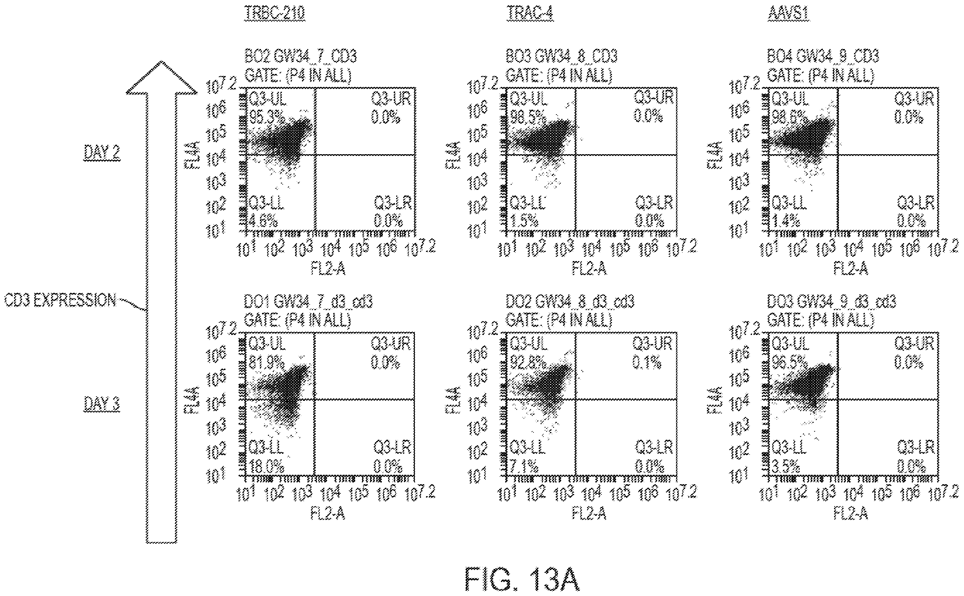

**Fig. 13A** shows CD4+ T cells electroporated with *S. pyogenes* Cas9 mRNA and the gRNA indicated (TRBC-210 (SEQ ID NO:388), TRAC-4 (SEQ ID NO:389) or AAVS1_1 (SEQ ID NO:387)) and stained with an APC-CD3 antibody and analyzed by FACS. The cells are stained with a CD3 antibody since CD3 expression on the surface of cells requires the TCR, which comprises 2 subunits: TRAC and TRBC. Staining for CD3, therefore, serves as a proxy for assessing the expression of TRAC and/or TRBC subunits and is used to determine whether gene editing results in the loss of TCR surface expression in targeted T cells. The cells were analyzed on day 2 and day 3 after the electroporation.
**Fig. 13B** shows quantification of the CD3 negative population from the plots in **Fig. 13A****.**
**Fig. 13C** shows %NHEJ results from the T7E1 assay performed on *TRBC2* and *TRAC* loci.
**Fig. 14A** shows Jurkat T cells electroporated with *S. aureus* Cas9/gRNA (TRAC-233 (SEQ ID NO:390)) RNPs targeting *TRAC* gene and stained with an APC-CD3 antibody and analyzed by FACS. The cells were analyzed on day 1, day 2 and day 3 after the electroporation.
**Fig. 14B** shows quantification of the CD3 negative population from the plots in **Fig. 14A****.**
**Fig. 14C** shows % NHEJ results from the T7E1 assay performed on the *TRAC* locus.
**Fig. 15** shows chromosome 2 locations (according to UCSC Genome Browser hg 19 human genome assembly) that corresponds to *BCL11A* intron 2. Three erythroid DNase 1-hypersensitivie sites (DHSs) are labeled as distance in kilobases from *BCL11A* TSS (+62, +58 and +55). *BCL11A* transcription is from right to left.
**Fig. 16** depicts a scheme of the pair of *HBB-8* (SEQ ID NO:398) and *HBB-15* (SEQ ID NO:397) surrounding the sickle mutation in combination with a Cas9 nickase (D10A or N863A). The nickases are shown as the grey ovals.
**Fig. 17** depicts the percentages of total editing event after a wildtype Cas9 was combined with *HBB-*8 (SEQ ID NO:398) or *HBB*-15 (SEQ ID NO:397) or a Cas9 nickase (D10A orN863A) was combined with the pair of *HBB-8* (SEQ ID NO:398) and *HBB*-15 (SEQ ID NO:397). At least three independent experiments for each condition were used to generate the percentages.
**Fig. 18A** depicts the frequency of deletions after a wildtype Cas9 was combined with *HBB-8* (SEQ ID NO:398) or *HBB-15* (SEQ ID NO:397) or a Cas9 nickase (D10A or N863A) was combined with the pair of *HBB-8* (SEQ ID NO:398) and *HBB-15* (SEQ ID NO:397). At least 3 independent experiments for each condition were used to generate the percentages.
**Fig. 18B** depicts the frequency distribution of the length of deletions using a wildtype Cas9 and *HBB-8* (SEQ ID NO:398) (similar results were obtained with *HBB-15* (SEQ ID NO:397)).
**Fig. 18C** depicts the frequency distribution of the length of deletions using a Cas9 nickase (D10A) with the pair of *HBB-8* (SEQ ID NO:398) and *HBB-15* (SEQ ID NO:397) (similar results have been obtained using Cas9 N863A).
**Fig. 19A** depicts the frequency of gene conversion after a wildtype Cas9 was combined with *HBB-*8 (SEQ ID NO:398) or *HBB*-15 (SEQ ID NO:397) or a Cas9 nickase (D10A orN863A) was combined with the pair of *HBB-8* (SEQ ID NO:398) and *HBB-15* (SEQ ID NO:397).
**Fig. 19B** shows a scheme representing the region of similarity between the *HBB* and *HBD* loci.
**Fig. 20** depicts the frequency of different lengths of *HBD* sequences that were incorporated into the *HBB* locus.

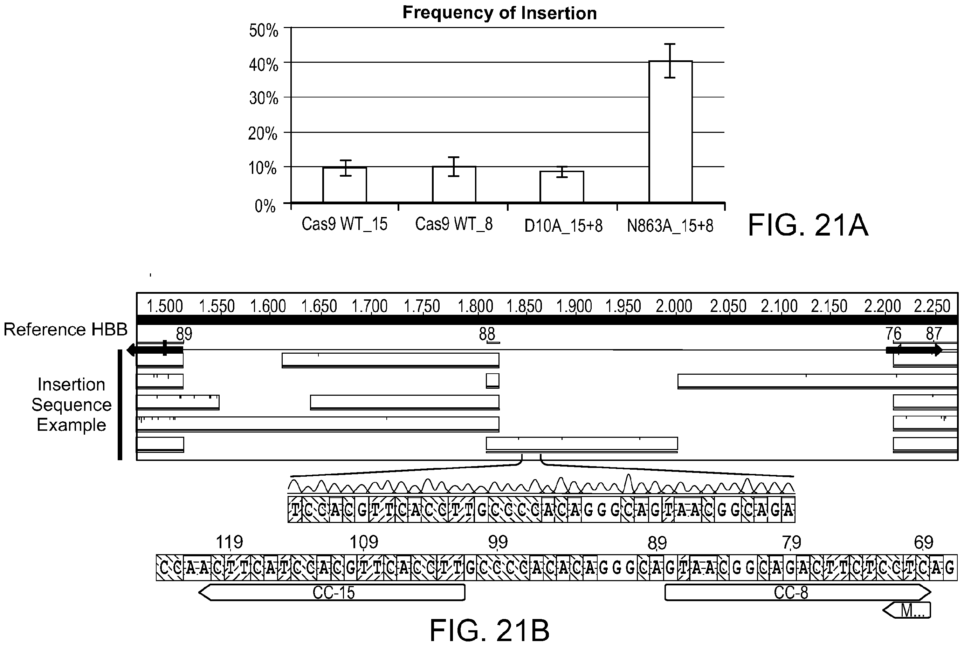

**Fig. 21A** depicts the frequency of insertions after a wildtype Cas9 was combined with *HBB-8* (SEQ ID NO:398) or *HBB-15* (SEQ ID NO:397) or a Cas9 nickase (D10A or N863A) was combined with the pair of *HBB-8* (SEQ ID NO:398) and *HBB-15* (SEQ ID NO:397). At least three independent experiments for each condition were used to generate the frequencies.
**Fig. 21B** depicts examples of common reads observed in U2OS cells electroporated with plasmid encoding a Cas9 nickase (N863A) and the *HBB-8* (SEQ ID NO:398) and *HBB-15* (SEQ ID NO:397) pair. The *HBB* reference is shown on the top.
**Fig. 22A** is a schematic representation of a donor template and an *HBB* reference sequence.
**Fig. 22B** depicts the frequency of HDR using a wildtype Cas9 with *HBB-8* (SEQ ID NO:398) or *HBB-15* (SEQ ID NO:397) or a Cas9 nickase (D10A or N863A) with the pair of *HBB-8* (SEQ ID NO:398) or *HBB-15* (SEQ ID NO:397).
**Fig. 22C** depicts the frequency of HDR using a wildtype Cas9 with *HBB-8* (SEQ ID NO:398) or *HBB-15* (SEQ ID NO:397) or a Cas9 nickase (N863A) with the pair of *HBB-8* (SEQ ID NO:398) or *HBB-15* (SEQ ID NO:397), each with a double stranded DNA donor or a Cas9 nickase (D10A) with the pair of *HBB-8* (SEQ ID NO:398) or *HBB-15* (SEQ ID NO:397) and different forms of donors .
**Fig. 23A** depicts genome editing of the *HBB* locus in human K562 erythroleukemia cells after electroporation of Cas9 protein complexed to *HBB*-8 (SEQ ID NO:398), or *HBB*-15 (SEQ ID NO:397) (RNP) or Cas9 mRNA co-delivered with *HBB*-8 (SEQ ID NO:398) or *HBB-15* (SEQ ID NO:397) (RNA).
**Fig. 23B** depicts the distribution of the editing events in the presence of a wild type Cas9 with *HBB*-8 (SEQ ID NO:398), *HBB-15* (SEQ ID NO:397) and with a Cas9 nickase (D10A) with the *HBB-8* (SEQ ID NO:398) and *HBB-15* (SEQ ID NO:397) pair.
**Fig. 24A** depicts kinetics of human adult CD34⁺ cell expansion after electroporation with uncapped/untailed gRNAs or capped/tailed gRNAs with paired Cas9 mRNA (either *S. pyogenes* (Sp) or *S. aureus* (Sa) Cas9). Control samples include: cells that were electroporated with GFP mRNA alone or were not electroporated but were cultured for the indicated time frame.
**Fig. 24B** depicts fold change in total live human CD34⁺ cells 72 hours after electroporation.
**Fig. 24C** depicts representative flow cytometry data showing maintenance of viable (propidium iodide negative) human adult mobilized peripheral blood CD34⁺ cells after electroporation with capped and tailed *AAVS1* gRNA and Cas9 mRNA.
**Figs. 25A-25G** depict electroporation of Cas9 mRNA and capped and tailed gRNA supports efficient editing in human adult mobilized peripheral blood CD34⁺ cells and their progeny.

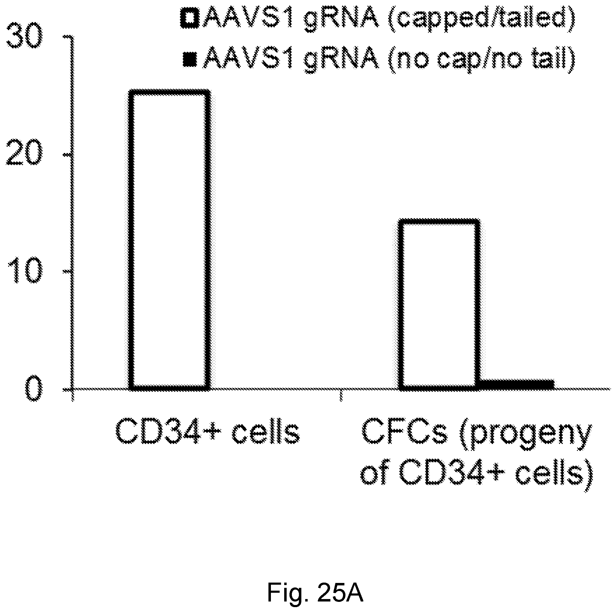

**Fig. 25A** depicts the percentage of insertions/deletions (indels) detected in human adult mobilized peripheral blood CD34⁺ cells and their hematopoietic colony forming cell (CFC) progeny at the targeted *AAVS1* locus after delivery of Cas9 mRNA with capped and tailed *AAVS1* gRNA compared to uncapped and untailed *AAVS1* gRNA.

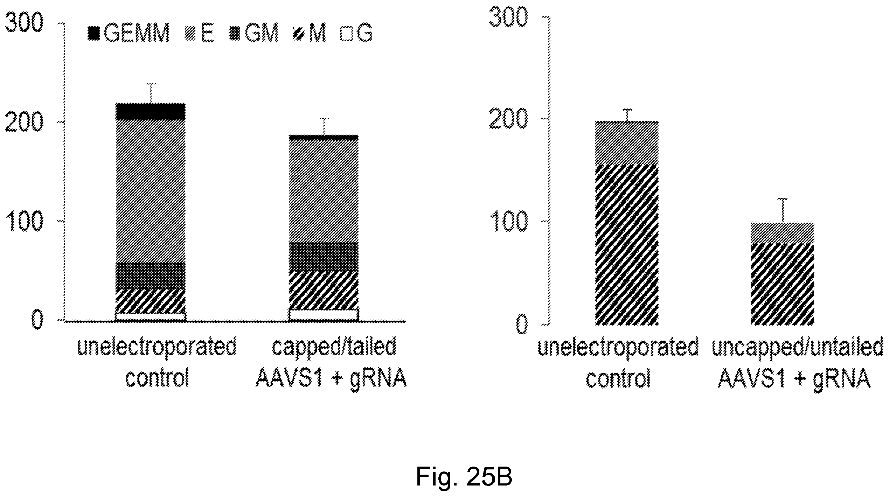

**Fig. 25B** depicts the hematopoietic colony forming potential (CFCs) maintained in human adult mobilized peripheral blood CD34⁺ cells after editing with capped/tailed *AAVS1* gRNA. Note loss of CFC potential for cells electroporated with uncapped/untailed *AAVS1* gRNA. E: erythroid, G: granulocyte, M: macrophage, GM: granulocyte-macrophage, GEMM granulocyte-erythrocyte-macrophage-monocyte CFCs.

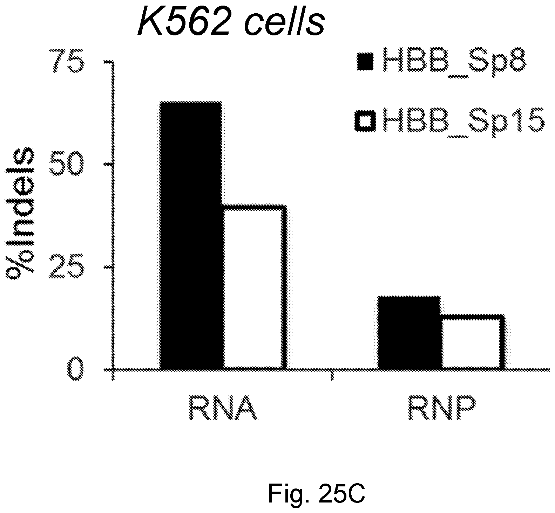

**Fig. 25C** depicts delivery of capped and tailed *HBB* gRNA with S. pyogenes Cas9 mRNA (RNA) or ribonucleoprotein (RNP) supports efficient targeted locus editing (% indels) in the K562 erythroleukemia cell line, a human erythroleukemia cell line has similar properties to HSCs.

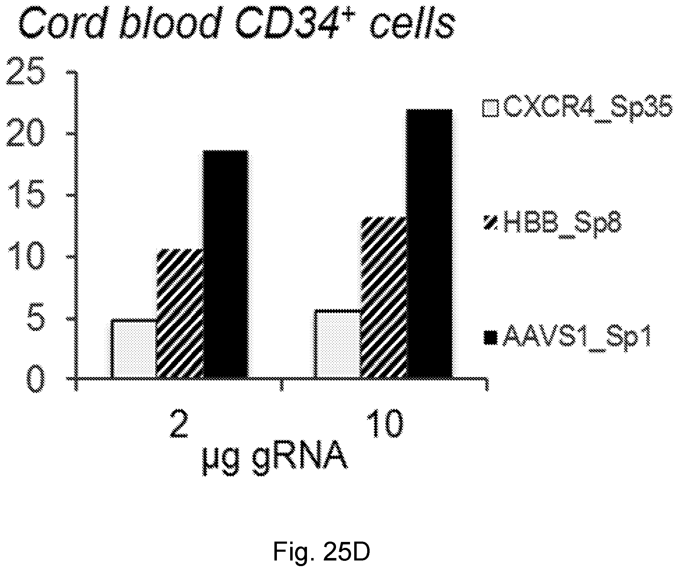

**Fig. 25D** depicts Cas9-mediated / capped and tailed gRNA mediated editing (% indels) at the indicated target genetic loci (*AAVS1, HBB, CXCR4)* in human cord blood CD34⁺ cells. Cells were electroporated with Cas9 mRNA and 2 or 10 µg of gRNA.

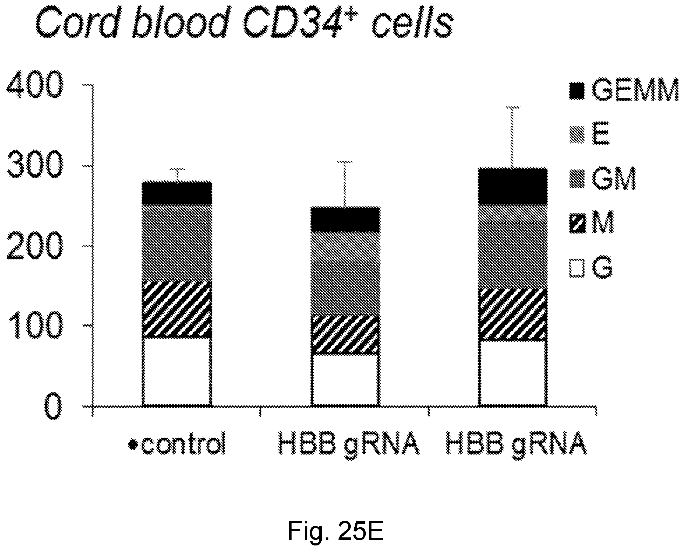

**Fig. 25E** depicts CFC assays for human umbilical cord blood CD34⁺ cells electroporated with 2 µg or 10 µg of capped/tailed *HBB* gRNA. CFCs: colony forming cells, GEMM: mixed hematopoietic colony granulocyte-erythrocyte-macrophage-monocyte, E: erythrocyte colony, GM: granulocyte-macrophage colong, G: granulocyte colony.

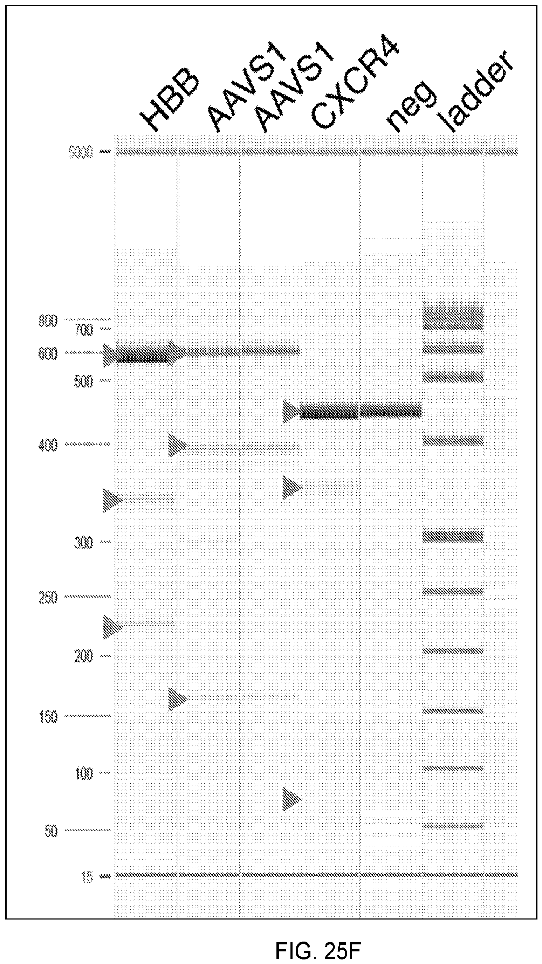

**Fig. 25F** depicts a representative gel image showing cleavage at the indicated loci (T7E1 analysis) in human cord blood CD34⁺ cells at 72 hours after delivery of capped and tailed *AAVS1, HBB,* or *CXCR4* gRNA and *S. pyogenes* Cas9 mRNA. The example gel corresponds to the summary data shown in **Fig. 25D****.**

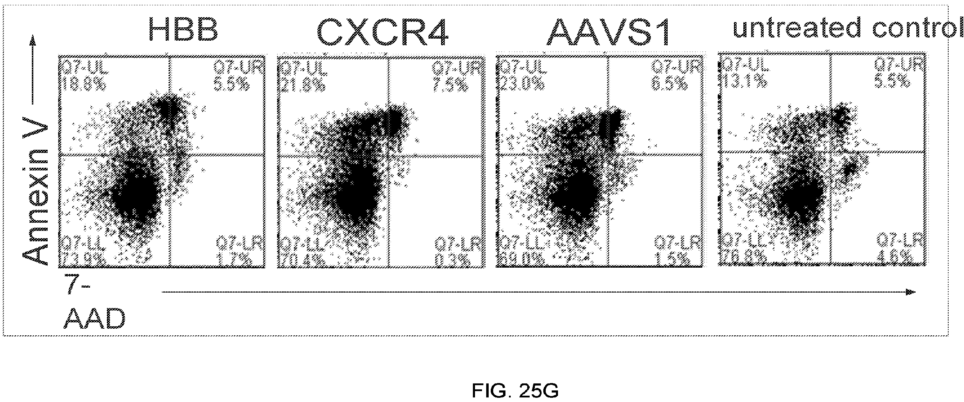

**Fig. 25G** depicts cell viability in human cord blood (CB) CD34⁺ cells 48 hours after delivery of Cas9 mRNA and indicated gRNAs as determined by co-staining with 7-AAD and Annexin V and flow cytometry analysis.

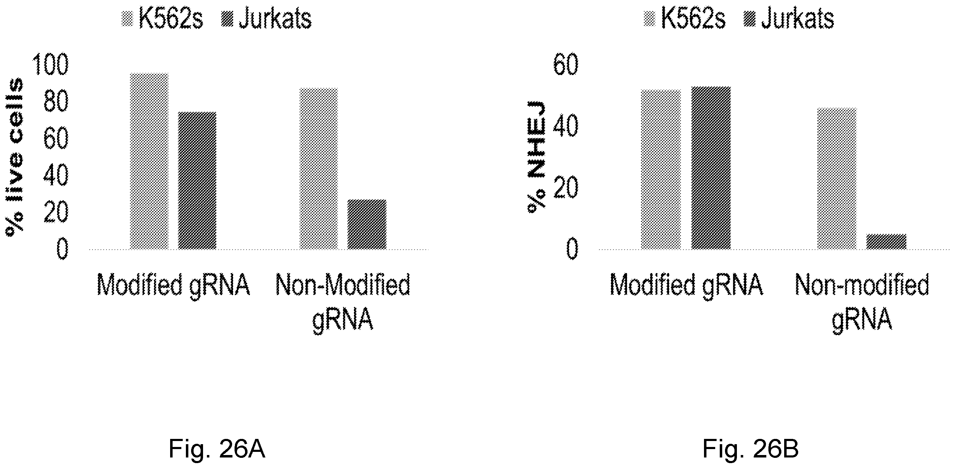

**Fig. 26A** depicts cell viability at 72 hours post-electroporation as determined by co-staining with Annexin V and propidium iodide. The fraction of cells that were negative for Annexin V and PI were categorized as viable and that percentage is represented in the graph.
**Fig. 26B** depicts quantification of %NHEJ results from the T7E1 assay performed on the *AAVS1* integration site locus at 72 hours. The % NHEJ rates correlated with the viability of the cells. Modified gRNAs outperformed non-modified gRNAs in Jurkat T cells.

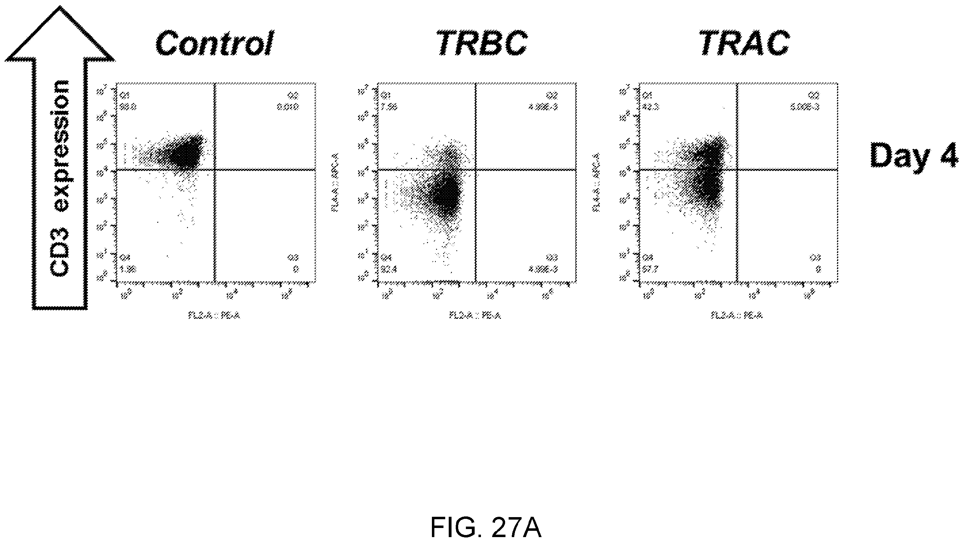

**Figs. 27A-27C** depict loss of CD3 expression in CD4⁺ T cells due to delivery of Cas9/gRNA RNP targeting *TRBC* or *TRAC.*
**Fig. 27A** depicts CD4⁺ T cells electroporated with *S. pyogenes* Cas9/gRNA (TRBC-210 (SEQ ID NO:388)) RNPs targeting TRBC or *S. aureus* Cas9/gRNA (TRAC-233 (SEQ ID NO:390)) RNPs targeting TRAC were stained with an APC-CD3 antibody and analyzed by FACS. The cells were analyzed on day 1, 2, 3 and 4 after the electroporation. Representative data from day 4 is shown.

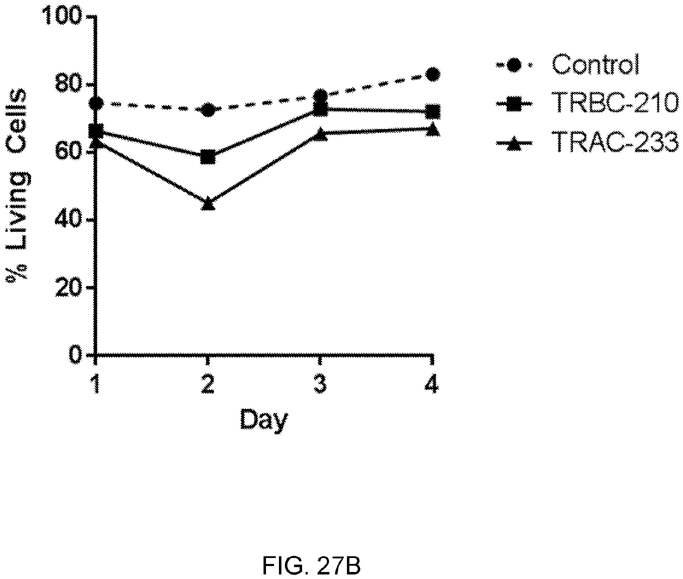

**Fig. 27B** depicts cell viability over a 4 day time course.

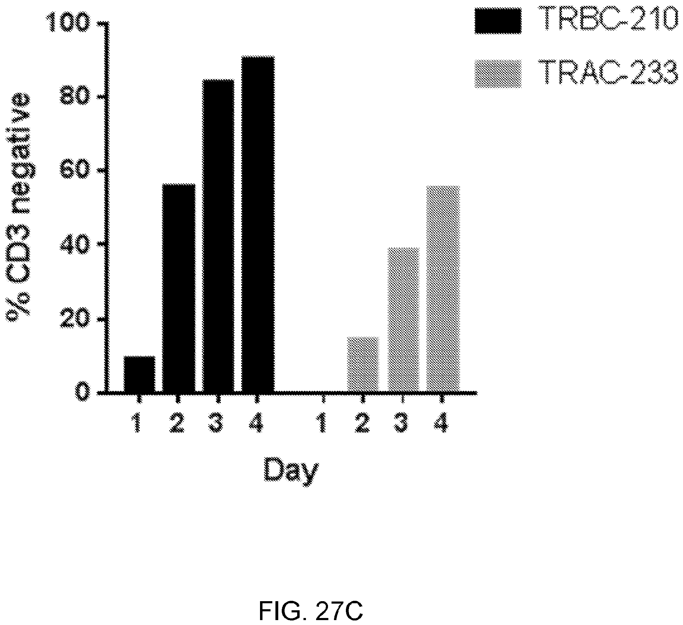

**Fig. 27C** depicts quantification of the CD3 negative population over a 4 day time course as determined by FACS.

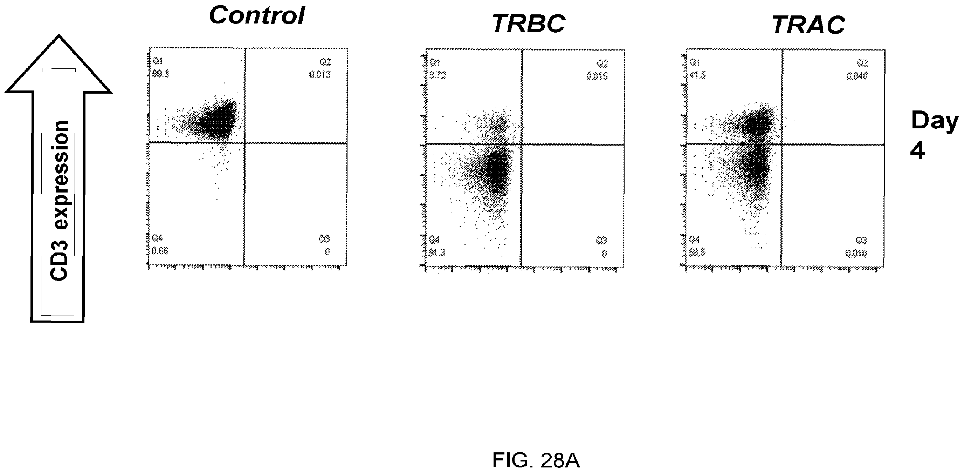

**Figs. 28A-28C** depict loss of CD3 expression in CD8+ T cells due to delivery of Cas9/gRNA RNP targeting *TRBC* or *TRAC.*
**Fig. 28A** depicts CD8⁺ T cells electroporated with *S. pyogenes* Cas9/gRNA (TRBC-210 (SEQ ID NO:388)) RNPs targeting TRBC or *S. aureus* Cas9/gRNA (TRAC-233 (SEQ ID NO:390)) RNPs targeting TRAC that were stained with an APC-CD3 antibody and analyzed by FACS. The cells were analyzed on day 2, 3, and 4 after the electroporation. Representative data from day 4 is shown.

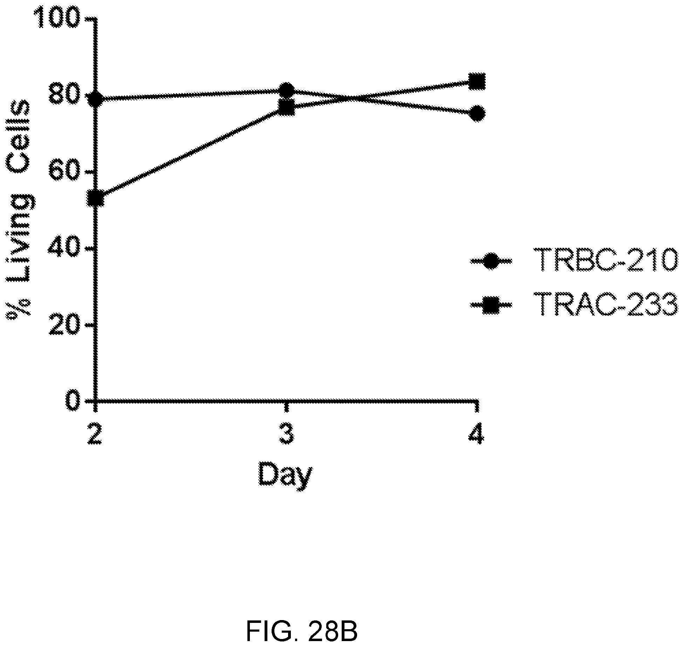

**Fig. 28B** depicts cell viability over a 4 day time course.

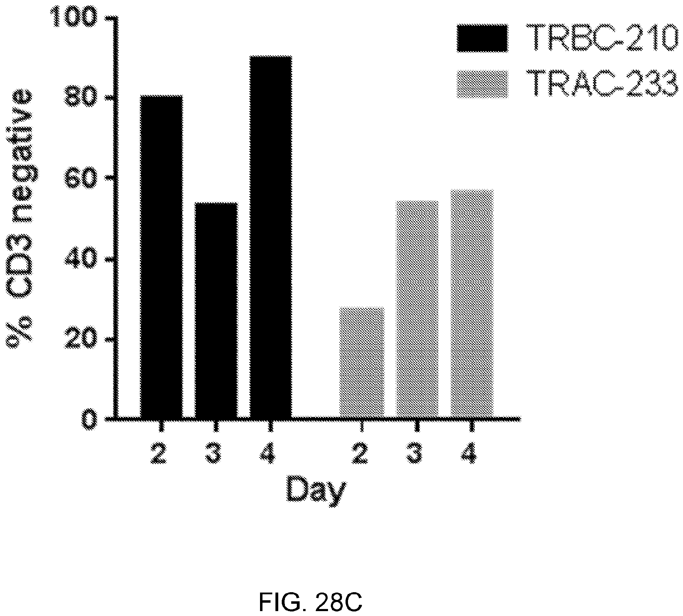

**Fig. 28C** depicts quantification of the CD3 negative population over a 4 day time course as determined by FACS.

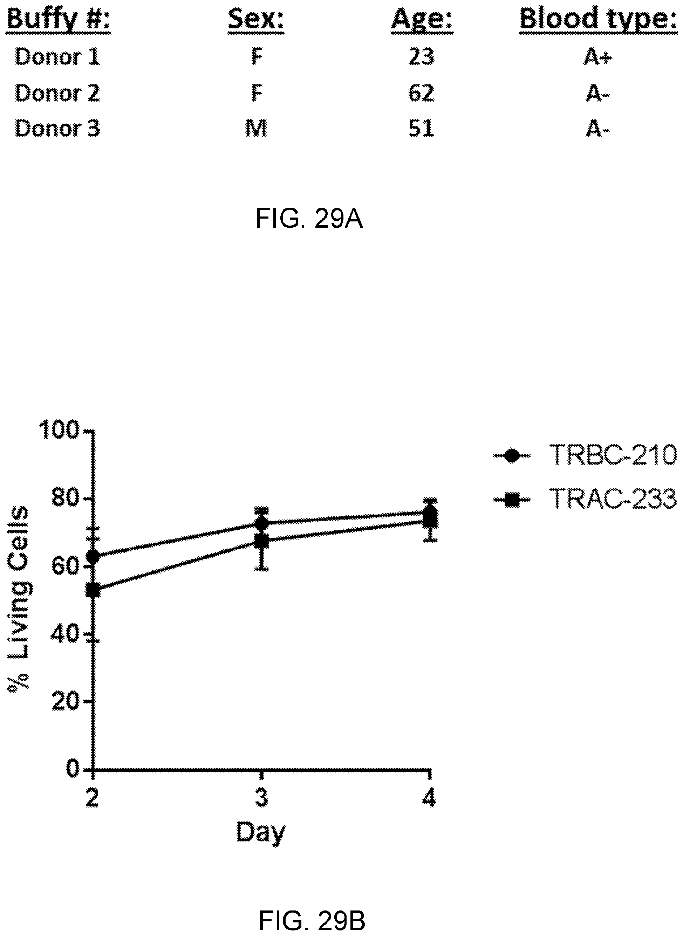

**Figs. 29A-29C** depict loss of CD3 expression in CD4+ T cells from multiple donors due to delivery of Cas9/gRNA RNP targeting *TRBC* and *TRAC.*
**Fig. 29A** depicts that CD4⁺ T cells from 3 different donors that were electroporated with *S*. *pyogenes* Cas9/gRNA (TRBC-210 (SEQ ID NO:388)) RNPs targeting *TRBC* or *S. aureus* Cas9/gRNA (TRAC-233 (SEQ ID NO:390)) RNPs targeting *TRAC.* The sex, age and blood type is shown.
**Fig. 29B** depicts that the cell viability was monitored by trypan blue over a 4 day time course post electroporation. The averages from the 3 separate donors for days 2, 3, and 4 are shown with the error bars indicating the standard deviation.

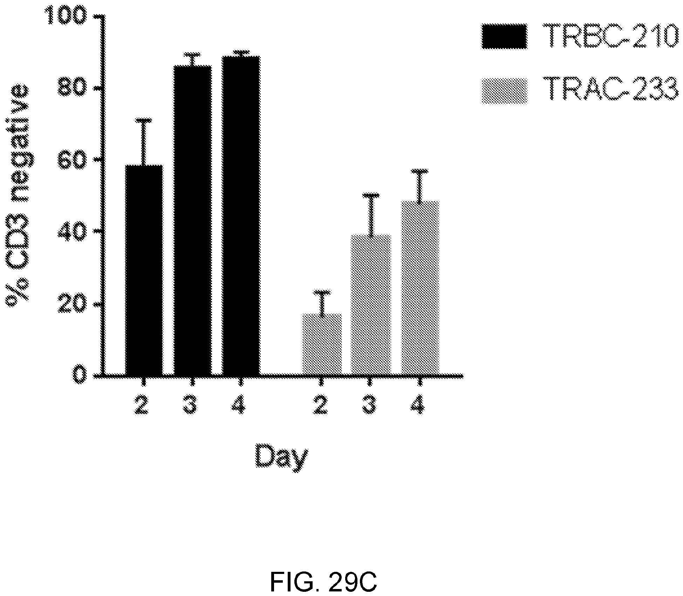

**Fig. 29C** depicts that transfected cells were stained with an APC-CD3 antibody and analyzed by FACS and analyzed on day 2, 3 and 4 after the electroporation. The average CD3 negative population for editing in the 3 separate donors is quantified for days 2, 3, and 4 post electroporation.

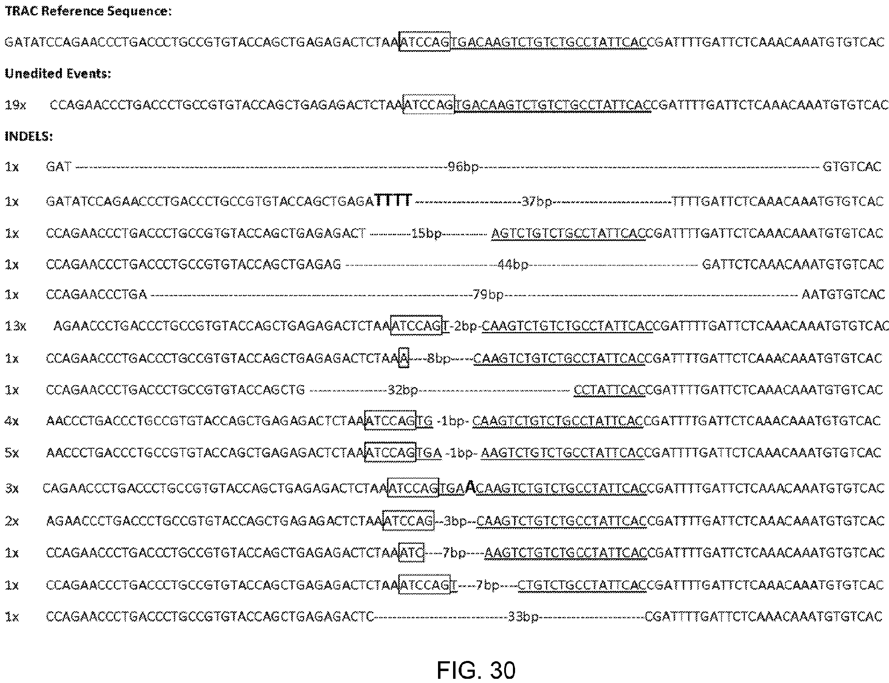

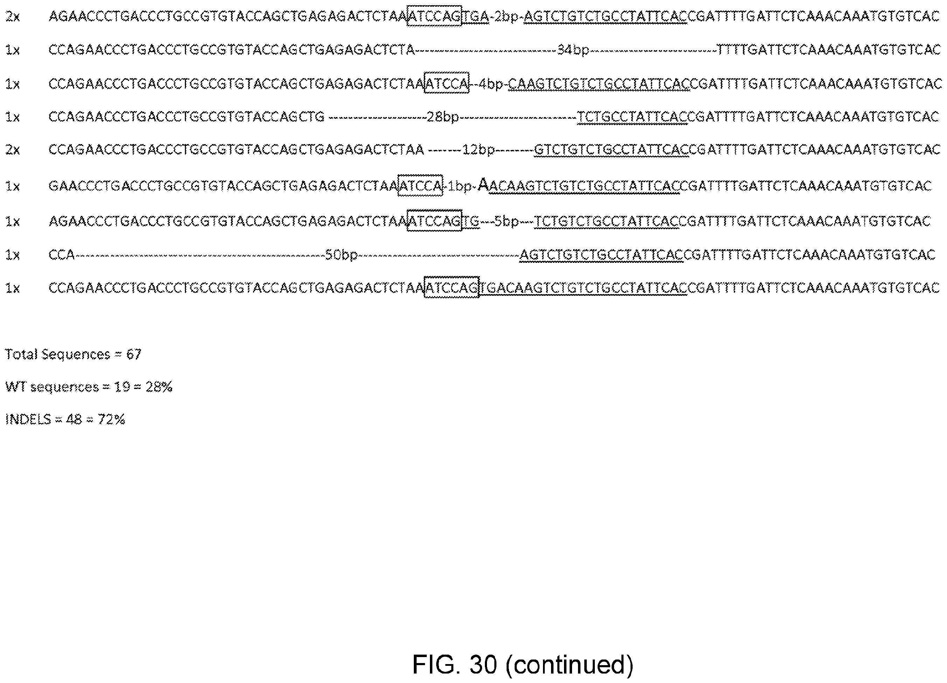

**Fig. 30** depicts characterization of genome editing events at the *TRAC* locus. The *TRAC* locus around the targeting site of the *TRAC* specific RNP was amplified from genomic DNA isolated from cells treated with the RNP. The PCR products were cloned into a sequencing vector and the cloned DNA was sequenced by Sanger sequencing using a vector specific sequencing primer. The resulting sequences are shown with the frequency of each sequence tabulated on the left hand side. At the top, the reference sequence is shown. The sequence that is boxed is the PAM site of the gRNA sequence which is underlined. Deletion events are annotated with hyphens and the length of deletion is noted. Insertions are denoted as larger letters in bold text (SEQ ID NOS:419-444).

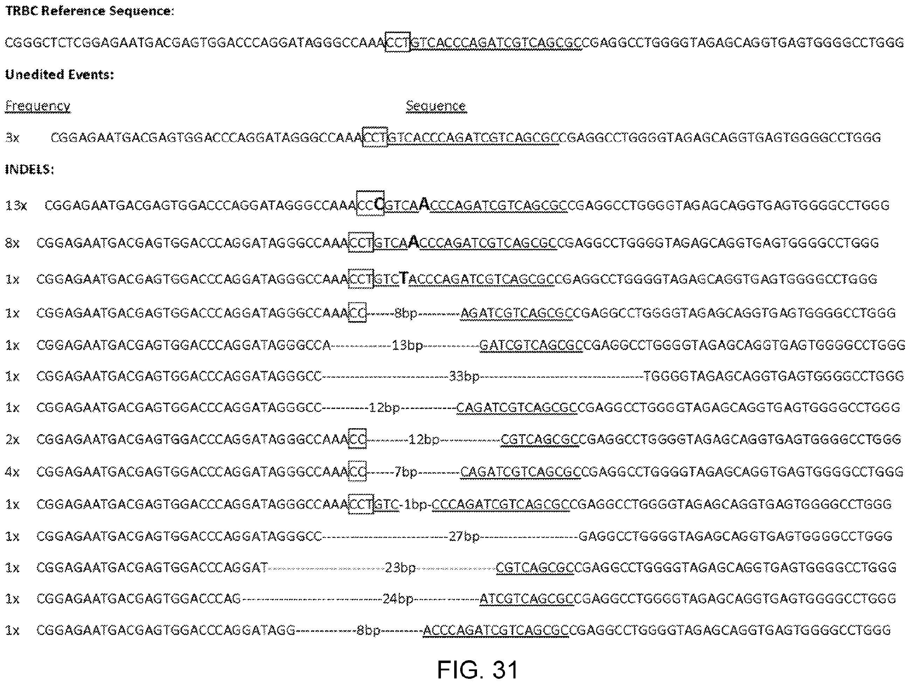

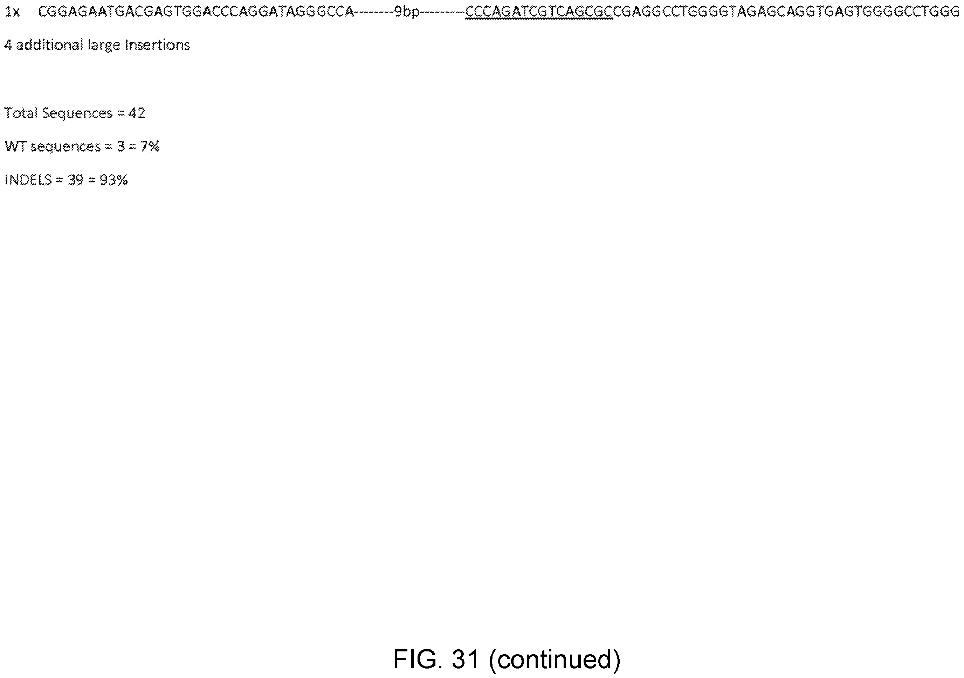

**Fig. 31** depicts characterization of genome editing events at the *TRBC* locus. The *TRBC* locus around the targeting site of the *TRBC* specific RNP was amplified from genomic DNA isolated from cells treated with the RNP. The PCR products were cloned into a sequencing vector and the cloned DNA was sequenced by Sanger sequencing using a vector specific sequencing primer. The resulting sequences are shown with the frequency of each sequence tabulated on the left hand side. At the top, the reference sequence is shown. The sequence that is boxed is the PAM site of the gRNA sequence which is underlined. Deletion events are annotated with hyphens and the length of deletion is noted. Insertions are denoted as larger letters in bold text (SEQ ID NOS:445-461).

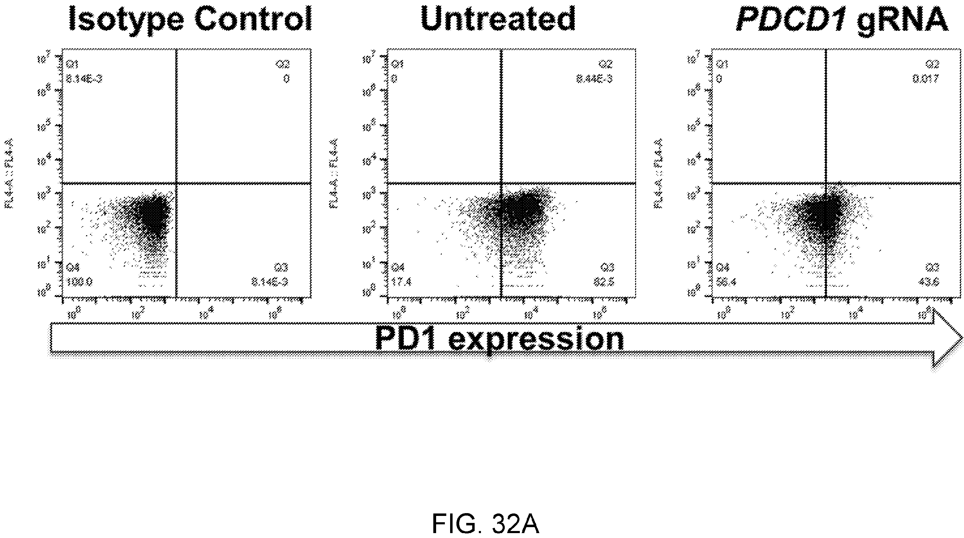

**Figs. 32A-32C** depict loss of PD-1 expression in CD4+ T cells due to the delivery of Cas9/gRNA RNP targeting *PDCD1.*
**Fig. 32A** depicts CD4⁺ T cells that were electroporated with *S. pyogenes* Cas9/gRNA (PDCD1-108 (SEQ ID NO:399)) RNPs targeting *PDCD1* and reactivated using CD3/CD28-conjugated beads and stained with a PE-PD1 antibody 7 days post electroporation by FACS. Representative data from day 7 is shown.

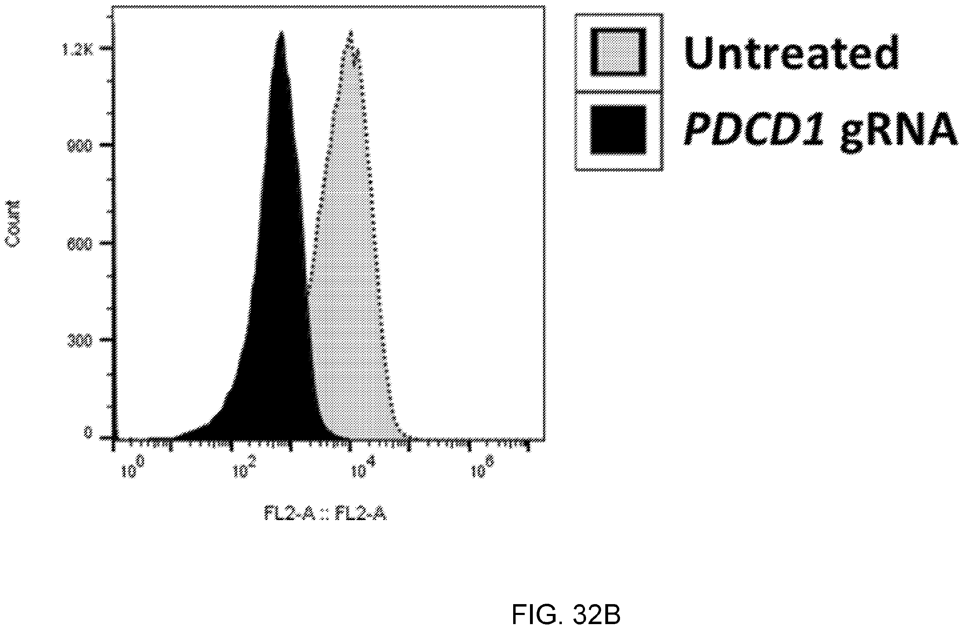

**Fig. 32B** depicts PD1 fluorescence of the *PDCD1* RNP treated cells was directly compared with the untreated control cells on an overlay histogram plot.

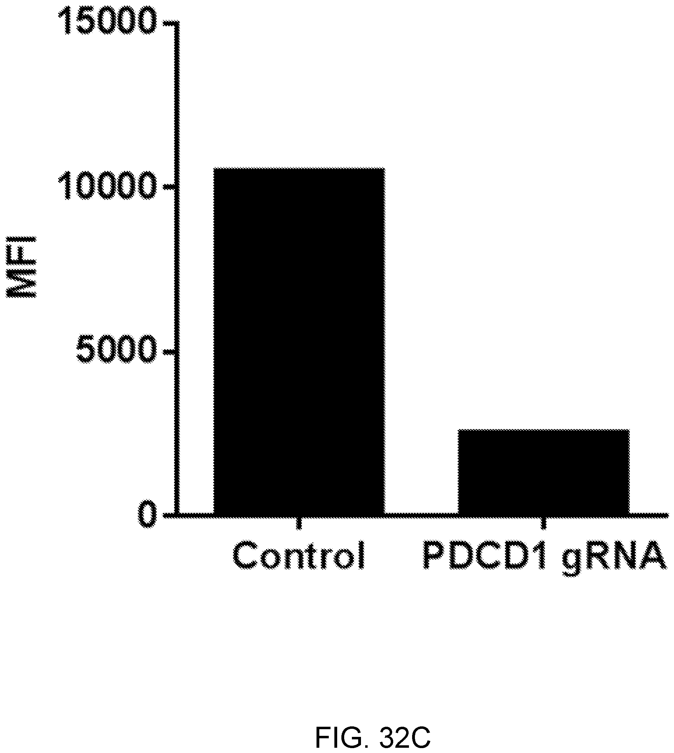

**Fig. 32C** depicts quantification of the mean fluorescence intensity (MFI) for the control cells and PDCD1 RNP treated cells.

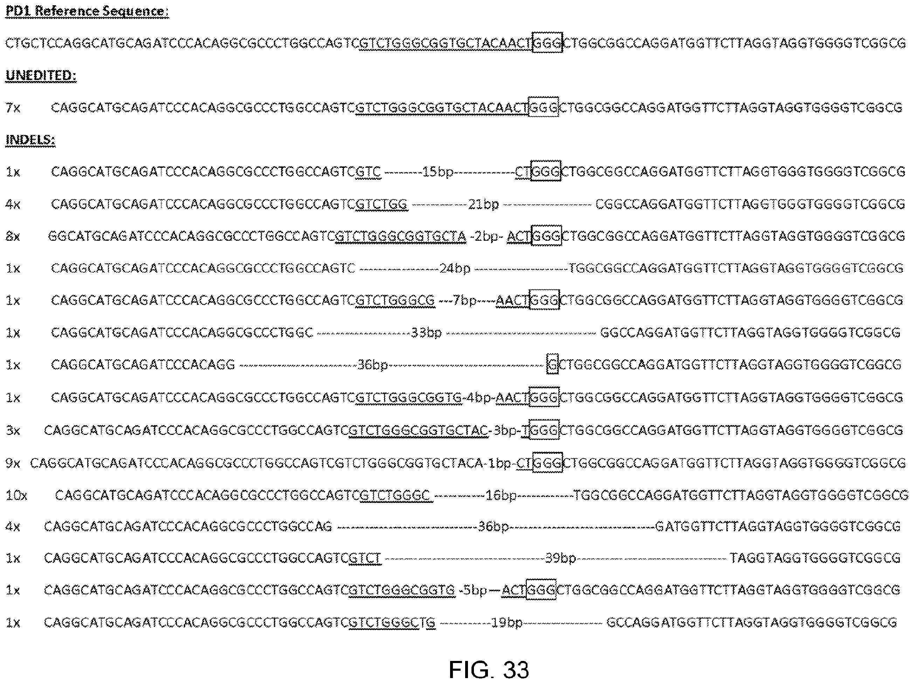

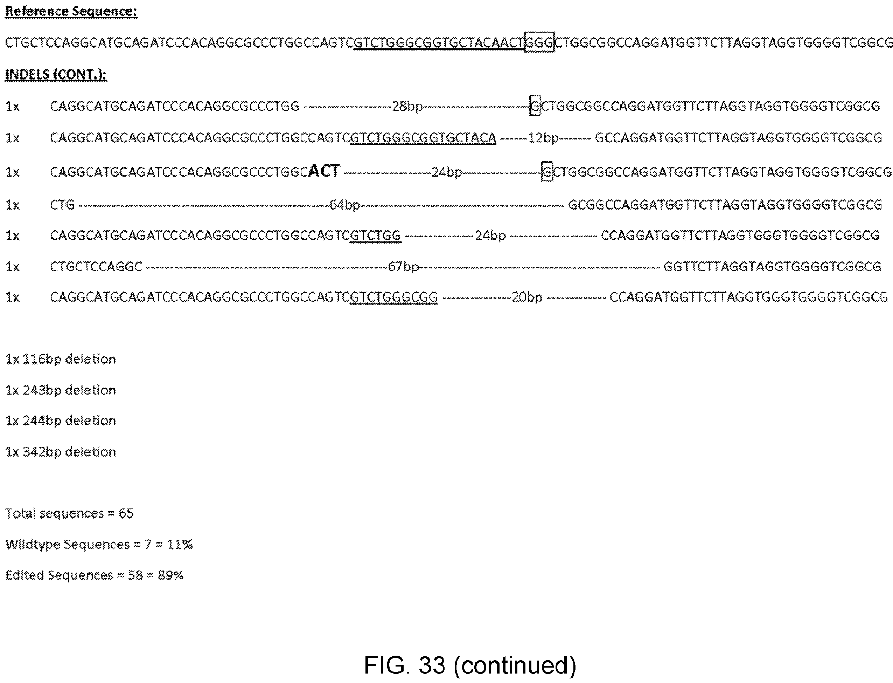

**Fig. 33** depicts characterization of genome editing events at the *PDCD1* locus. The *PDCD1* locus around the targeting site of the *PDCD1* specific RNP was amplified from genomic DNA isolated from cells treated with the RNP. The PCR products were cloned into a sequencing vector and the cloned DNA was sequenced by Sanger sequencing using a vector specific sequencing primer. The resulting sequences are shown with the frequency of each sequence tabulated on the left hand side. At the top, the reference sequence is shown. The sequence that is boxed is the PAM site of the gRNA sequence which is underlined. Deletion events are annotated with hyphens and the length of deletion is noted. Insertions are denoted as larger letters in bold text (SEQ ID NOS:462-486).

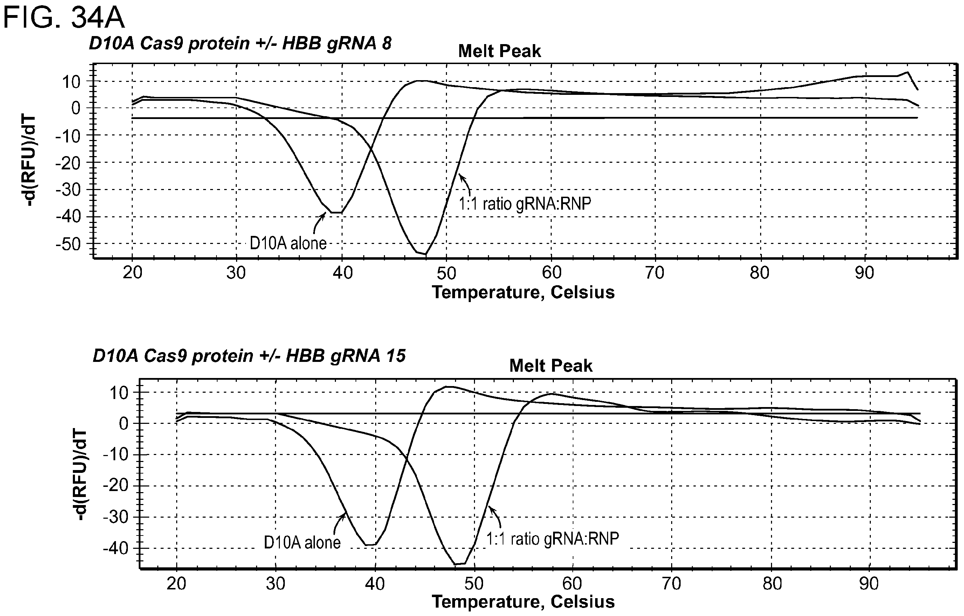

**Figs. 34A-34B** depict an analysis of stability of D10A nickase RNP *in vitro* and *ex vivo* in human adult CD34⁺HSCs.
**Fig. 34A** depicts differential Scanning Fluorimetry Shift Assay after complexing D10A protein with the indicated *HBB* gRNAs added at 1:1 molar ratio gRNA:RNP.

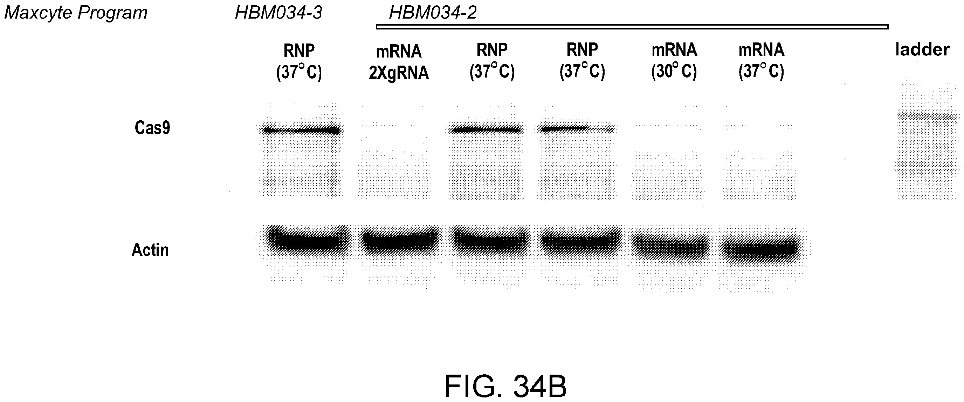

**Fig. 34B** depicts detection of Cas9 protein in cell lysates 72 hours after human adult CD34⁺ HSCs were electroporated with D10A nickase RNP or D10A mRNA with gRNAs *HBB*-8 (SEQ ID NO:398) and *HBB-15* (SEQ ID NO:397). The electroporation program (P2 or P3) used is indicated at the top of the image. 2X gRNA: 10µg of each gRNA was co-delivered with D10A mRNA (vs. 5µg of each gRNA in the other experiments).

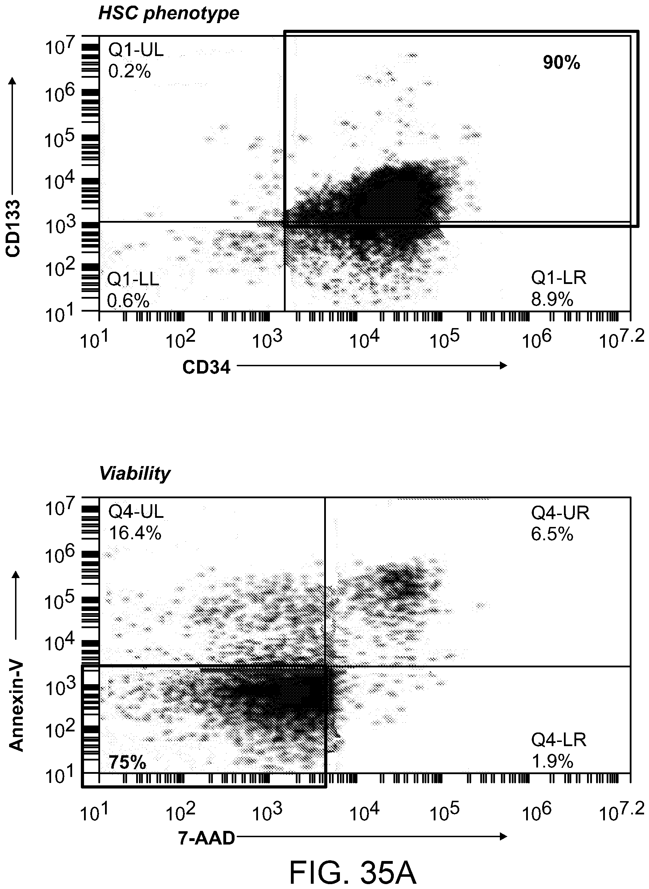

**Figs. 35A-35C** show that human adult CD34⁺ HSCs maintain stem cell phenotype after electroporation with D10A nickase RNP and *HBB* gRNA pair.
**Fig. 35A** shows that gene edited adult CD34⁺ cells maintain expression of stem cell markers CD34 and CD133 at 72 hours after electroporation.

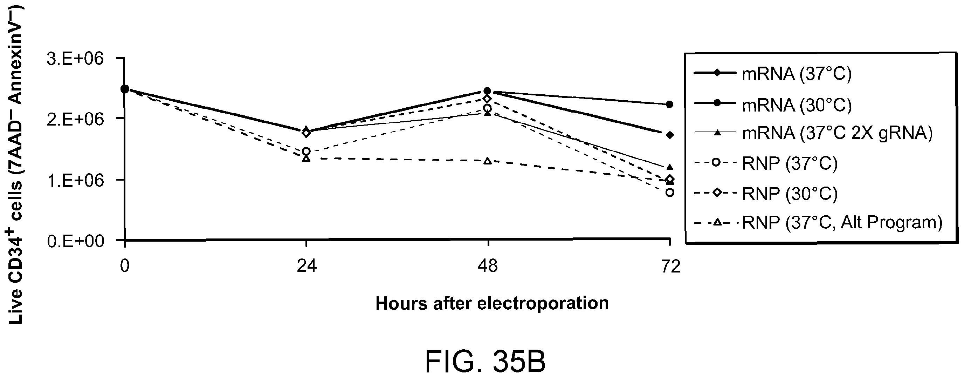

**Fig. 35B** depicts absolute live (7-AAD⁻AnnexinV⁻) CD34⁺ cell number at indicated time points relative to electroporation of D10A nickase RNP and *HBB* gRNA pair.

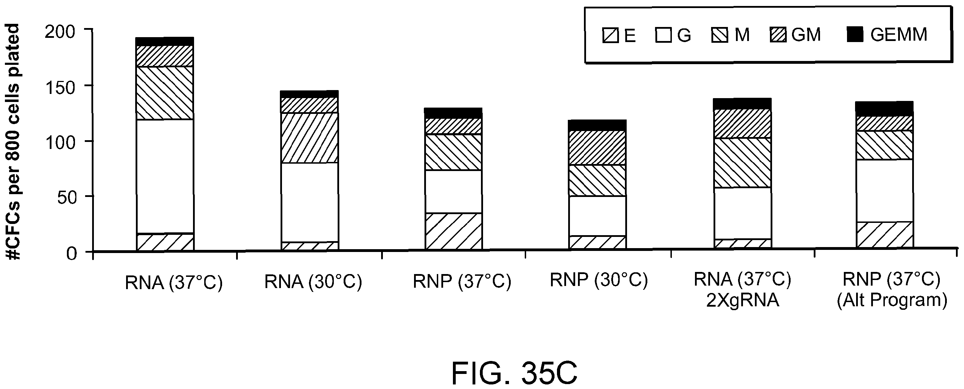

**Fig. 35C** shows that gene edited adult CD34⁺ cells maintain hematopoietic colony forming cell (CFC) activity and multipotency. E: erythroid, G: granulocyte, M: macrophage, GM: granulocyte-macrophage, GEMM: granulocyte-erythrocyte-macrophage-monocyte CFCs.

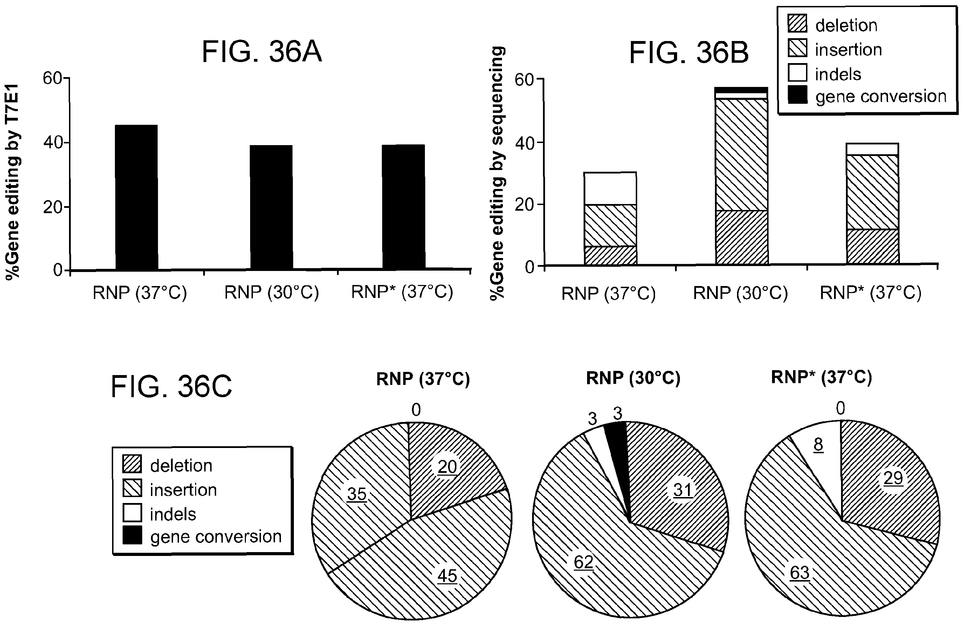

**Figs. 36A-36C** show that D10A nickase RNP co-delivered with *HBB* gRNA pair supports gene editing and HDR in human adult CD34⁺HSCs.
**Fig. 36A** depicts percentage of gene editing events as detected by T7E1 endonuclease assay analysis of the *HBB* locus in human adult CD34⁺HSCs. RNP* refers to use of alternate electroporation program (P3).
**Fig. 36B** depicts DNA sequence analysis of the *HBB* locus from human adult CD34⁺ HSCs. The subtypes of gene editing events (insertions, deletions, indels, and gene conversion events) are indicated. RNP* refers to use of alternate electroporation program (P3).
**Fig. 36C** depicts percentages of types of editing events detected in the gDNA from the human adult CD34⁺HSCs electroporated with the conditions shown in **Fig. 36B****.** Data are shown as a percentage of all gene editing events.

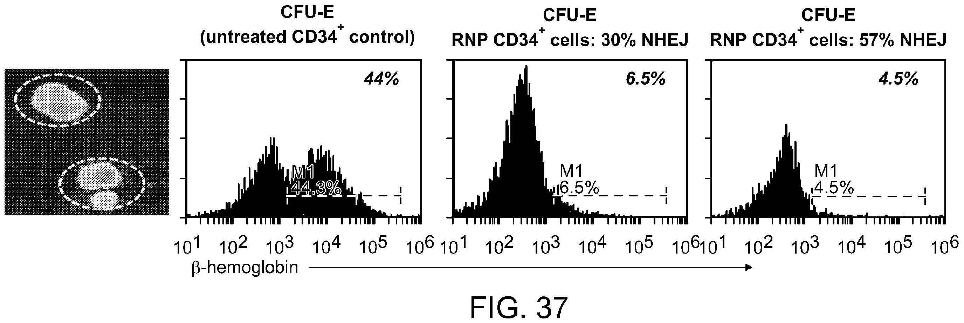

**Fig. 37** shows flow cytometry analysis of beta-hemoglobin expression in the erythroid progeny differentiated from erythroid progeny of D10A nickase RNP gene-edited adult CD34⁺ HSCs. CFU-E colonies (far left) differentiated from D10A nickase RNP *HBB* gRNA electroporated CD34⁺ cells were dissociated, fixed, permeabilized, and stained for beta-hemoglobin expression. The gene editing frequencies in the parental CD34⁺ cell population are indicated above the histograms for the indicated samples. The percentage of beta-hemoglobin expression in each colony was determined by flow cytometry and is indicated at the top right of each histogram.

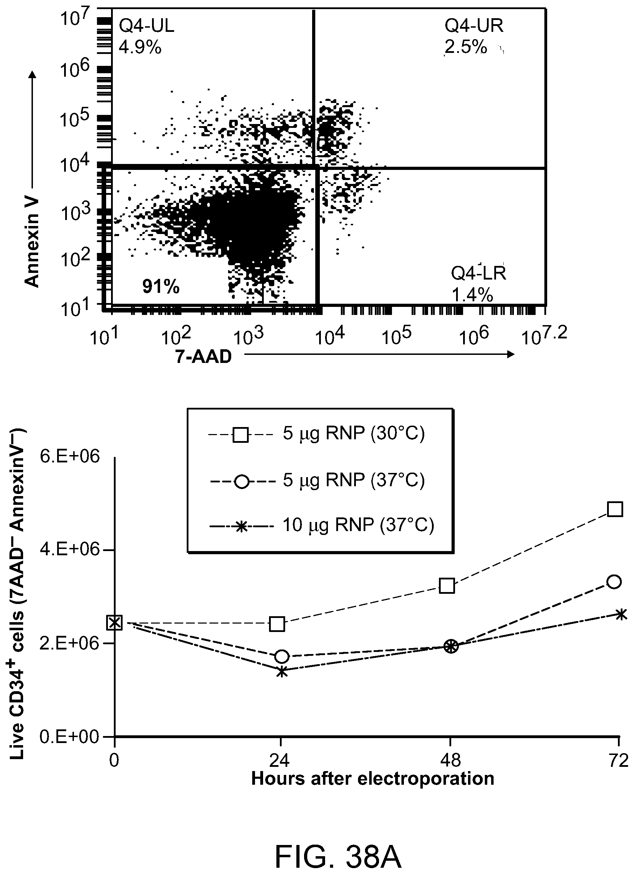

**Figs. 38A-38B** show that human cord blood (CB) CD34⁺HSCs maintained stem cell phenotype after electroporation with D10A nickase RNP and *HBB* gRNA pair.
**Fig. 38A** left panel depicts a representative flow cytometry analysis plot showing viability (AnnexinV⁻7AAD⁻) human CB CD34⁺ HSCs at 72 hours after electroporation with D10A Cas9 RNP with *HBB-8* (SEQ ID NO:398) and *HBB-15* (SEQ ID NO:397) gRNAs. Right panel: Absolute live (7-AAD⁻AnnexinV-) human CB CD34⁺ HSC cell number at indicated time points relative to electroporation of D10A nickase RNP *HBB* gRNA pair.

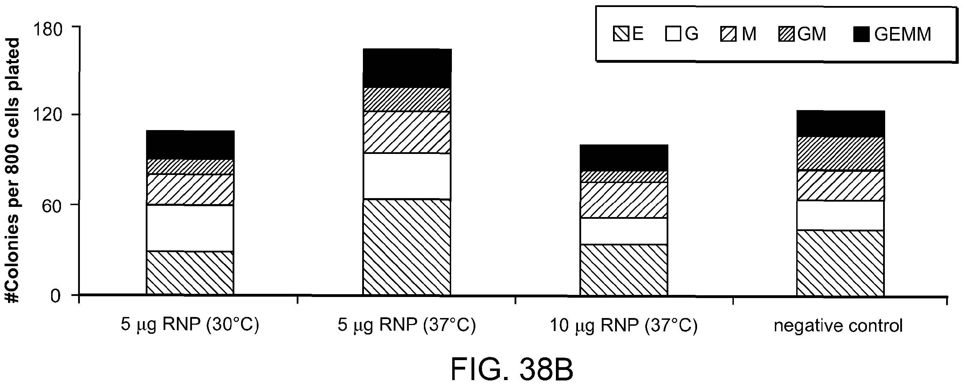

**Fig. 38B** shows that gene edited CB CD34⁺ cells maintained hematopoietic colony forming cell (CFC) activity and multipotency. E: erythroid, G: granulocyte, M: macrophage, GM: granulocyte-macrophage, GEMM: granulocyte-erythrocyte-macrophage-monocyte CFCs. The amounts of D10A nickase RNP delivered per million cells (5 or 10µg) and the 2-hour recovery temperature (parentheses) after electroporation of the parental CB CD34⁺ cells are indicated.

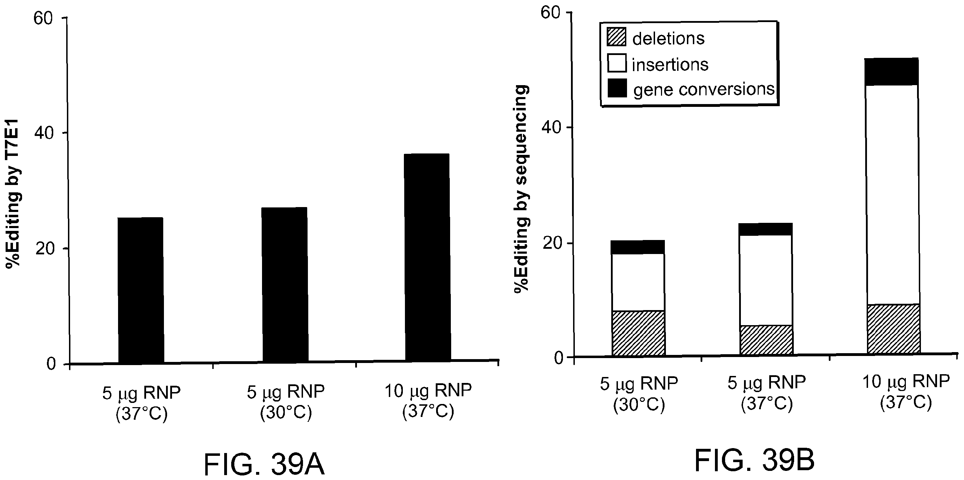

**Figs. 39A-39C** show that D10A nickase RNP co-delivered with *HBB* gRNA pair supported gene editing and HDR in human CB CD34⁺HSCs.
**Fig. 39A** depicts percentage of gene editing events as detected by T7E1 endonuclease assay analysis of the *HBB* locus in gene-edited human CB CD34⁺ HSCs.
**Fig. 39B** depicts DNA sequence analysis of the *HBB* locus in gene-edited human CB CD34⁺ HSCs. The subtypes of gene editing events (insertions, deletions, indels, and gene conversion events) are indicated as a fraction of the total sequencing reads.

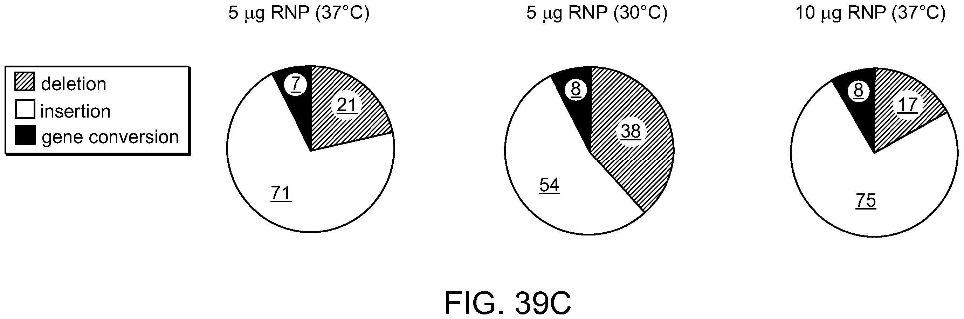

**Fig. 39C** depicts subtypes of gene editing events expressed as relative percentage to the total number gene editing events detected. The amounts of D10A nickase RNP delivered per million cells (5 or 10µg) and the 2-hour recovery temperature (parentheses) after electroporation of the parental CB CD34⁺ HSCs are indicated.

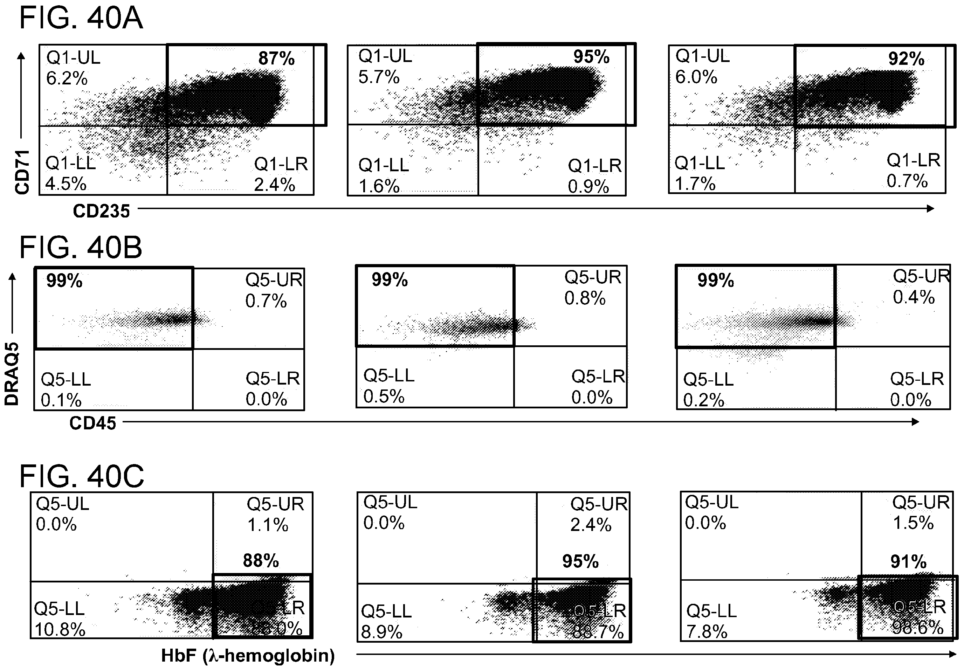

**Figs 40A-40C** show directed differentiation of gene-edited human CB CD34⁺HSCs into erythroblasts. Flow cytometry analysis of day 18 erythroblasts differentiated from gene edited human CB CD34⁺ HSCs.
**Fig. 40A** depicts CD71 (transferrin receptor) and CD235 (Glycophorin A) expression.
**Fig. 40B** depicts loss of CD45 and dsDNA through enucleation as indicated by the absence of dsDNA (negative for dsDNA binding dye DRAQ5). Note that unlike adult CD34⁺ cells, CB CD34⁺ cells differentiated into fetal-like erythroblasts that express fetal g-hemoglobin (not adult b-hemoglobin).
**Fig. 40C** depicts fetal hemoglobin (g-hemoglobin) expression.

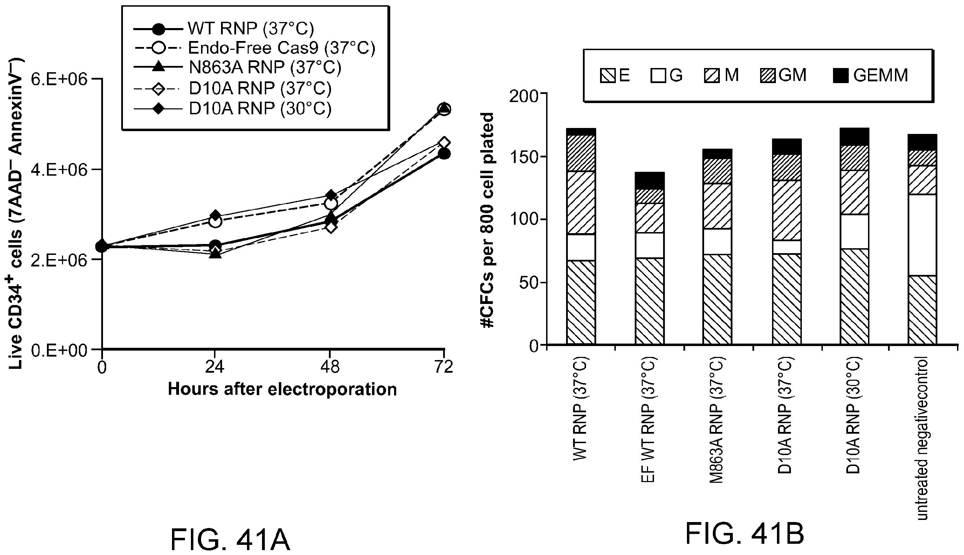

**Figs. 41A-41B** show that human CB CD34⁺HSCs maintained stem cell phenotype after electroporation with Cas9 variant RNPs and *HBB* gRNA pair.
**Fig. 41A** shows that gene edited human CB CD34⁺ cells maintained viability after electroporation with WT Cas9 endotoxin-free (EF WT) Cas9, N863A nickase, or D10A nickase co-delivered with *HBB* gRNA pair. Absolute live (7-AAD⁻AnnexinV⁻) CD34⁺ cell number at indicated time points relative to electroporation.
**Fig. 41B** shows that gene edited CB CD34⁺ cells maintained hematopoietic colony forming cell (CFC) activity and multipotency. E: erythroid, G: granulocyte, M: macrophage, GM: granulocyte-macrophage, GEMM: granulocyte-erythrocyte-macrophage-monocyte CFCs. The amounts of RNP delivered per million cells (10µg) and the 2-hour recovery temperature (parentheses) after electroporation of the parental human CB CD34⁺ cells are indicated.

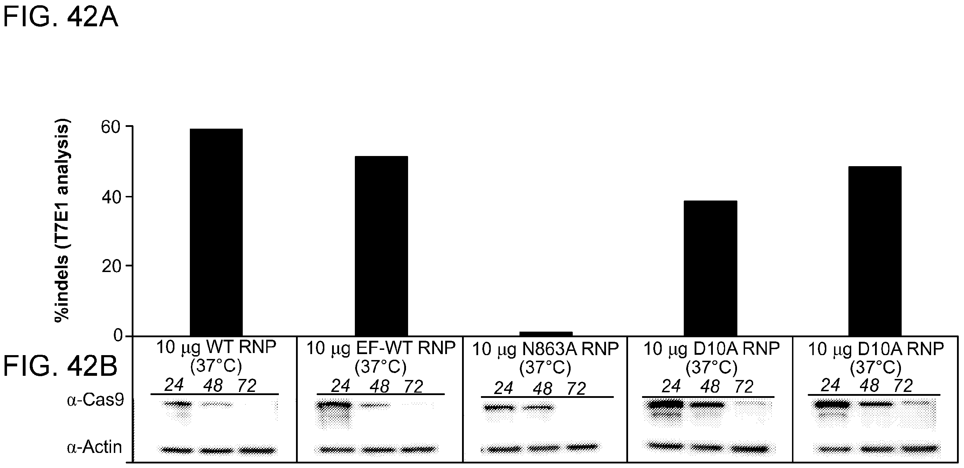

**Figs 42A-42B** compare gene editing at the *HBB* locus in human CB CD34⁺ cells mediated by WT and nickase Cas9 variant RNPs.
**Fig. 42A** depicts T7E1 analysis of the percentage of indels detected 72 hours after electroporation at the targeted site in the *HBB* locus after electroporation of WT Cas9, Endotoxin-free WT Cas9 (EF-WT), N863A nickase, and D10A nickase RNPs, each co-delivered with *HBB-8* (SEQ ID NO:398) and *HBB-15* (SEQ ID NO:397) gRNA pair.
**Fig. 42B** depicts Western blot analysis showing detection of Cas9 variants in cell lysates of CB CD34⁺ cells at the indicated time points after electroporation. The amounts of RNP delivered per million cells (10µg) and the 2-hour recovery temperature (parentheses) after electroporation of the parental human CB CD34⁺ cells are indicated.

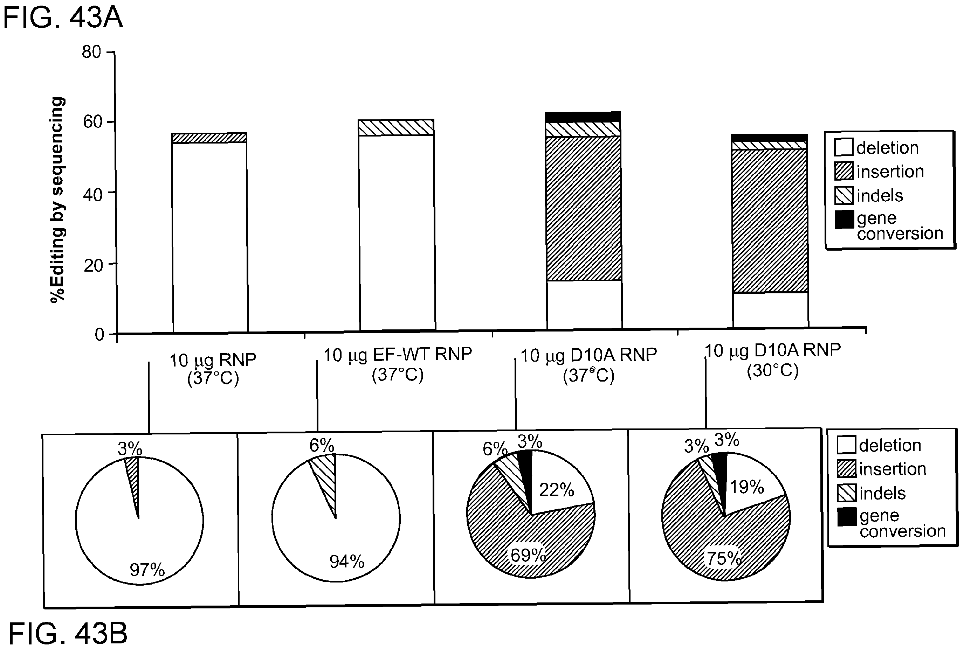

**Figs. 43A-43B** depict comparison of HDR and NHEJ events detected at the *HBB* locus after gene editing with WT Cas9 and D10A nickase in human CB CD34⁺ HSCs.
**Fig. 43A** depicts percentage of gene editing events (72 hours after electroporation) detected by DNA sequencing analysis and shown as a percentage of the total sequence reads. CB CD34⁺ HSCs received RNP (WT Cas9, Endotoxin-free WT Cas9 [EF-WT], N863A and D10A nickases) with *HBB*-8 (SEQ ID NO:398) and *HBB*-15 (SEQ ID NO:397) gRNA pair.
**Fig. 43B** depicts percentages of types of editing events detected in the gDNA from the cells electroporated with the conditions shown in **Fig. 43A****.** Data are shown as a percentage of all gene editing events. *Left to Right:* WT Cas9 RNP (37°C), endotoxin-free (EF) WT Cas9 RNP (37°C), D10A Cas9 RNP (37°C), D10A Cas9 RNP (30°C).

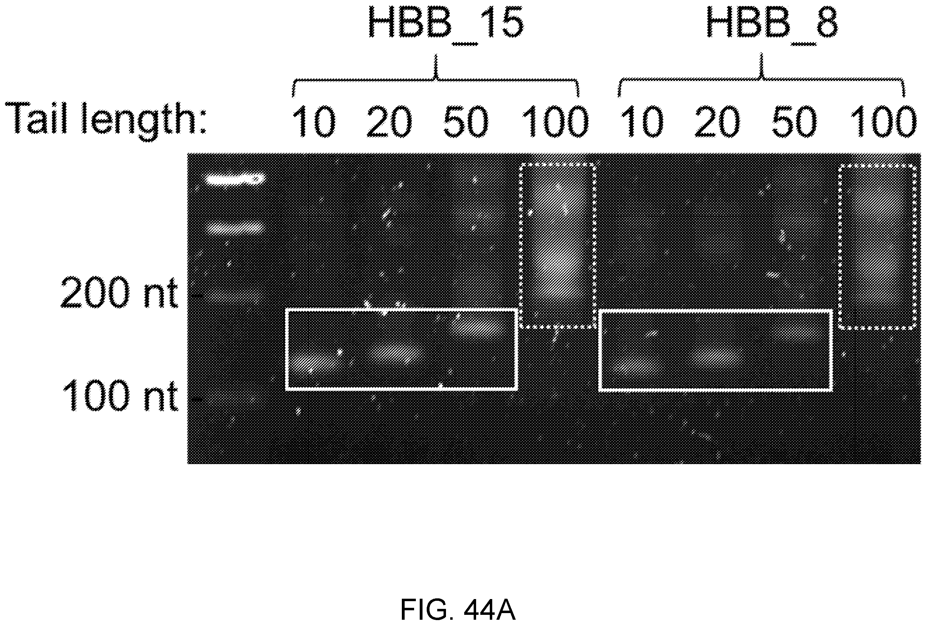

**Figs. 44A-44B** depict *in vitro* transcribed *HBB*-specific gRNAs generated with polyA tail encoded in DNA template.
**Fig. 44A** depicts PCR products of DNA templates for the *HBB* gRNAs with encoded polyA tails of the indicated lengths. The dominant size-correct PCR products for gRNAs with 10, 20 and 50 length polyA tails are indicated in the solid boxed area. A distribution of PCR products is shown by dashed boxes.

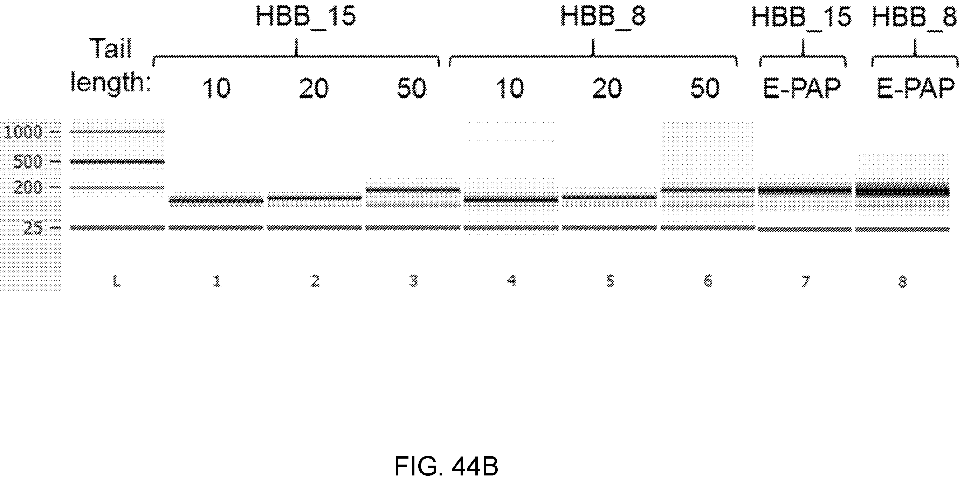

**Fig. 44B** depicts a bioanalyzer analysis of *in vitro* transcribed gRNAs with indicated tail lengths engineered in the DNA template or added enzymatically (E-PAP).

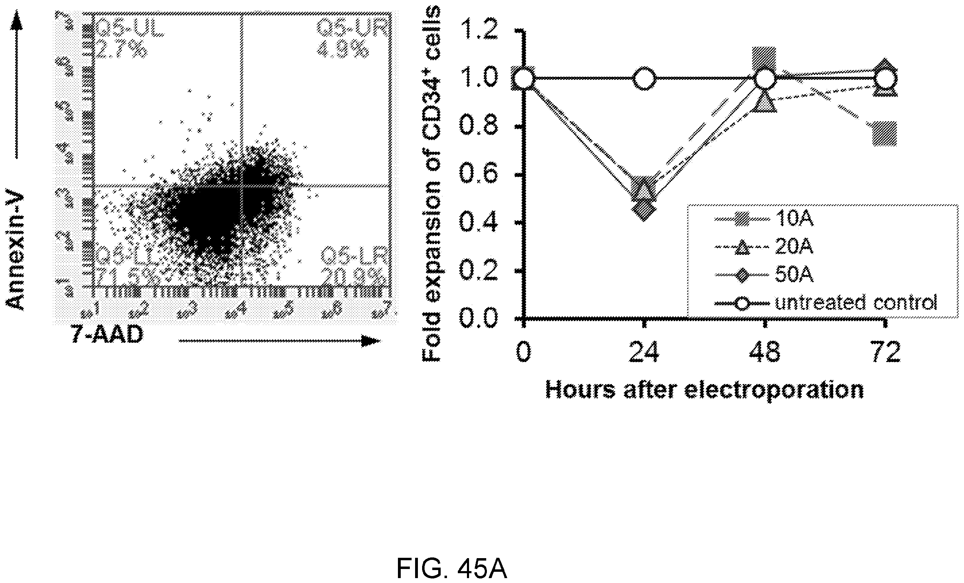

**Figs. 45A-45C** depict gRNAs engineered with 10 and 20 length polyA tails supported gene editing in human CB CD34⁺HSCs.
**Fig. 45A** left panel depicts a representative flow cytometry analysis plot showing viability (AnnexinV⁻7AAD⁻) human CB CD34⁺ HSCs at 72 hours after electroporation with D10A Cas9 RNP with *HBB-8* (SEQ ID NO:398) and *HBB-15* (SEQ ID NO:397) gRNAs. Right panel: Kinetics of CD34+ cell expansion after electroporation with D10A RNP and *HBB-8* (SEQ ID NO:398) and *HBB-15* (SEQ ID NO:397) gRNAs with indicated polyA tails.

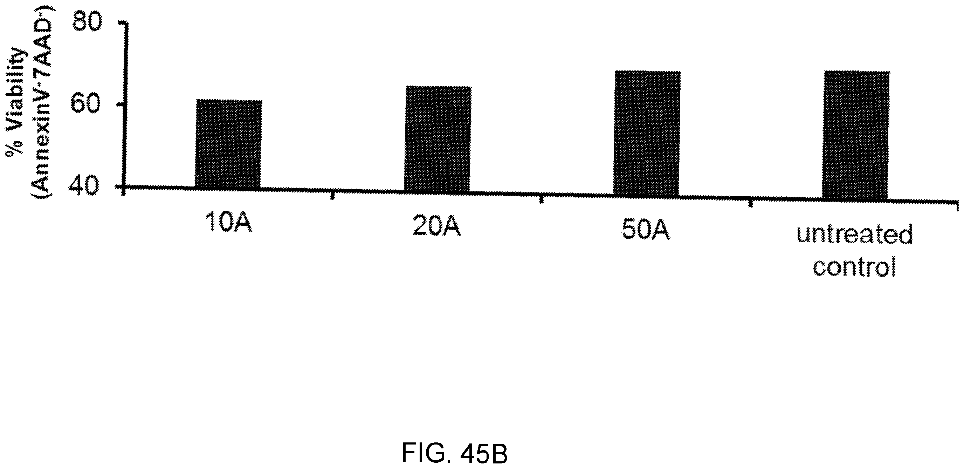

**Fig. 45B** depicts percent viability (AnnexinV⁻7AAD⁻) of cord blood CD34⁺ HSPCs at 72 hours after electroporation with D10A RNP and *HBB-8* (SEQ ID NO:398) and *HBB-15* (SEQ ID NO:397) gRNAs engineered with the indicated tail lengths.

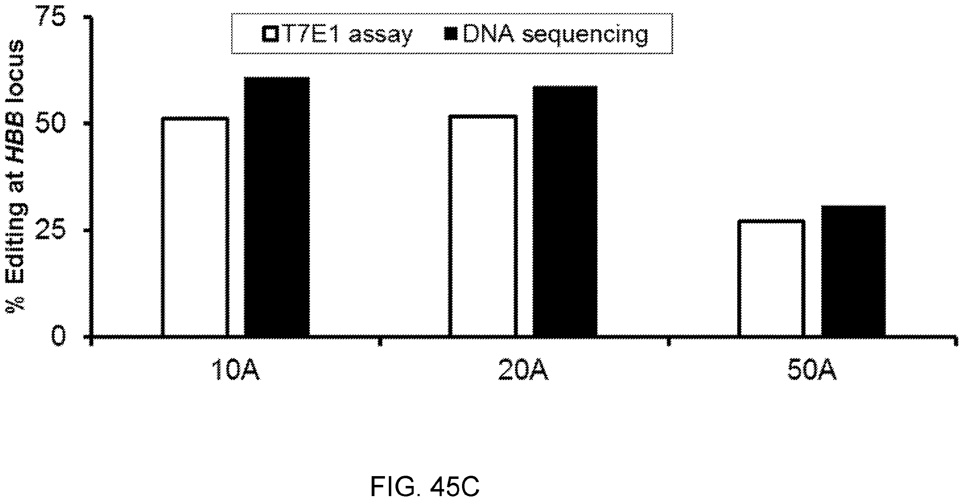

**Fig. 45C** depicts gene editing as detected by T7E1 endonuclease assay and Sanger DNA sequencing of the PCR product of the *HBB* genomic locus in human CB CD34⁺ HSCs after electroporation with D10A RNP and *HBB-8* (SEQ ID NO:398) and *HBB-15* (SEQ ID NO:397) gRNAs with the indicated polyA tails.

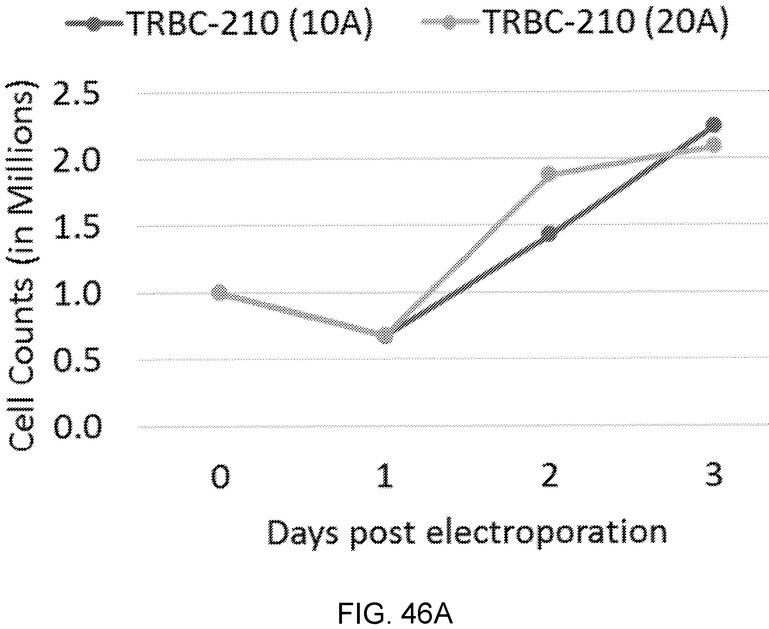

**Figs. 46A-46B** depict delivery of RNP targeting *TRBC* using gRNAs that have a polyA tail with a length of 10 or 20 results in efficient knockout as monitored by CD3 expression.
**Fig. 46A** depicts cells counts using trypan blue as a Live/Dead cell measure for 72 hours post electroporation.

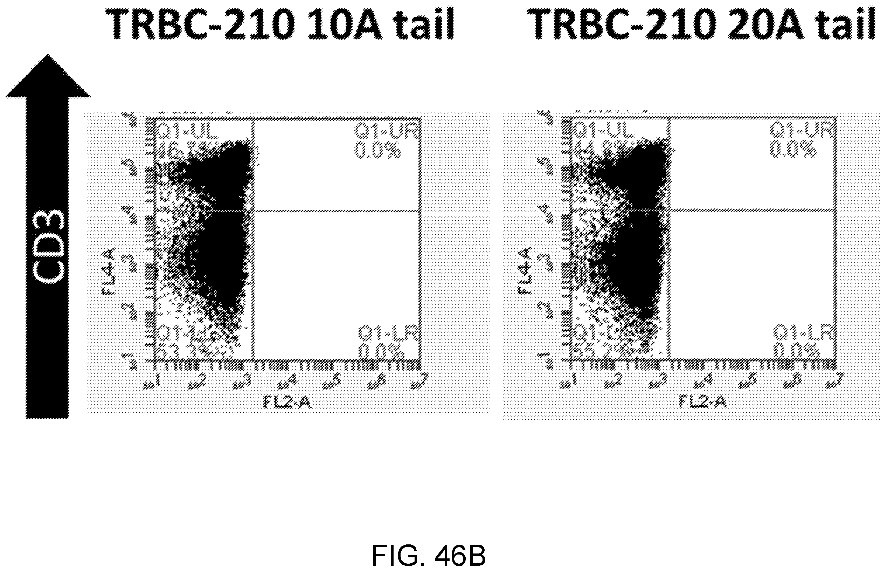

**Fig. 46B** depicts analysis of CD3 expression at day 6 post electroporation by FACS analysis.

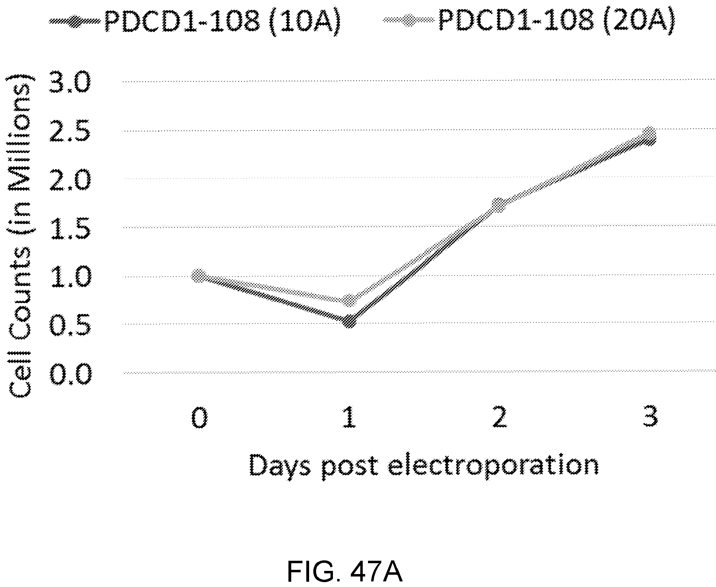

**Figs. 47A-47B** depict delivery of RNP targeting *PDCD1* using gRNAs that have a polyA tail with a length of 10 or 20 results in efficient knockout as monitored by PD1 expression.
**Fig. 47A** depicts cell counts using trypan blue as a Live/Dead cell measure for 72 hours post electroporation.

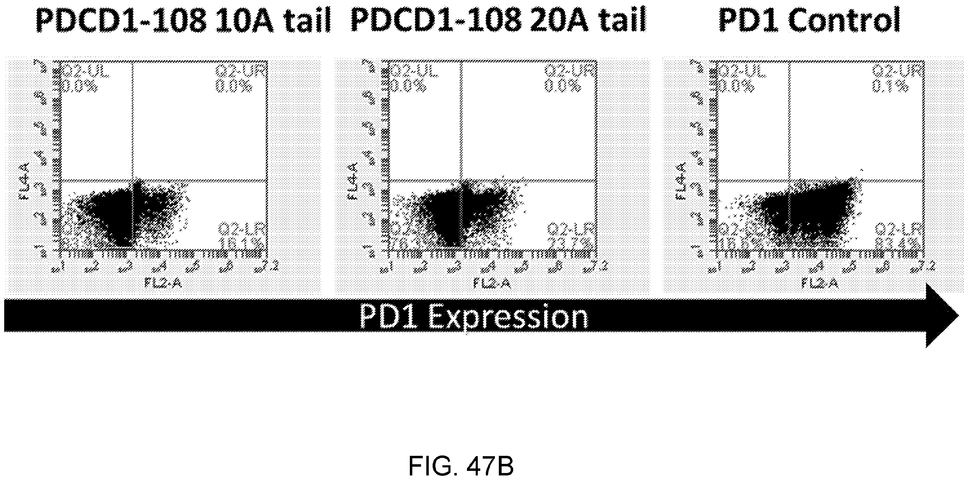

**Fig. 47B** depicts re-stimulated cells at 72 hours post electroporation and analysis of PD-1 expression at day 6 post electroporation by FACS analysis.

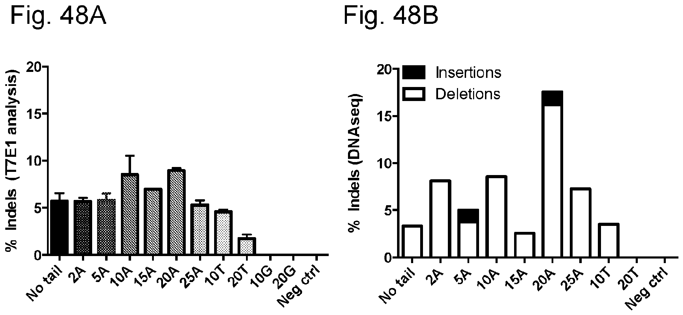

**Figs. 48A** and **48B** depict the effect of different gRNA modifications at the 3' end on gene editing percentages in adult mPB CD34⁺ cells.
**Fig. 48A** depicts gene editing percentages that were determined by T7E1 assay analysis.
**Fig. 48B** depicts DNA sequencing analysis at the human *HBB* locus from *HBB* PCR products generated from the genomic DNA isolated from adult mPB CD34⁺ cells after electroporation (Amaxa Nucleofector) with Cas9 RNP (wild-type Cas9 protein complexed to *HBB-8* gRNA (SEQ ID NO:398)).

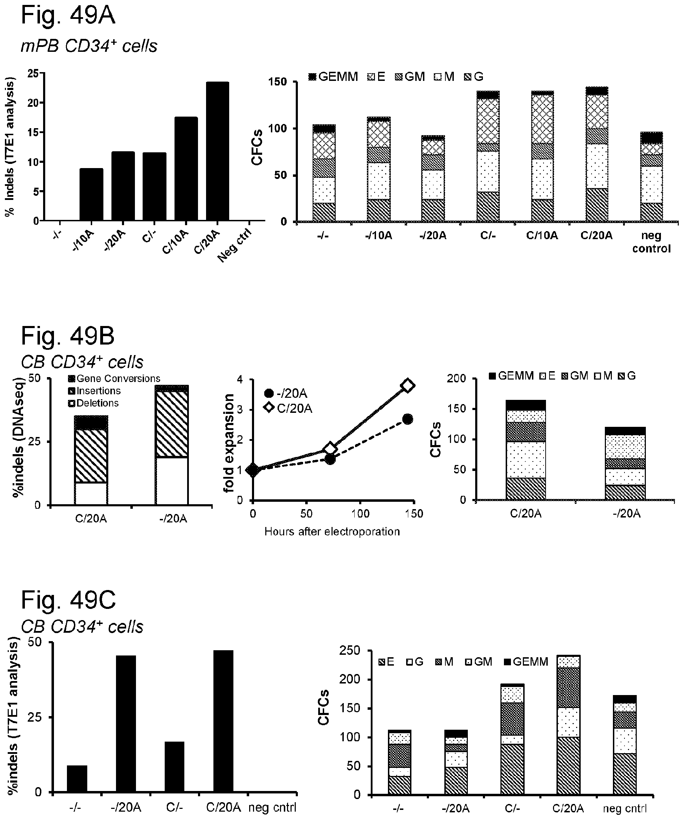

**Figs. 49A, 49B,** and **49C** depict the effect of including a 5' end modification (ARCA cap) and/or a 3' end modification (polyA tail of a specific length) in gRNAs on the gene editing and hematopoietic potential (e.g., CFC) of adult mPB CD34⁺ cells and CB CD34⁺ cells.
**Fig. 49A** depicts the results of an experiment where gRNAs *HBB-8* (SEQ ID NO:398) and *HBB*-15 (SEQ ID NO:397) were in vitro transcribed with the indicated 5' and 3' end modifications, complexed to D10A Cas9 protein, and electroporated into adult mPB CD34⁺ cells (Amaxa Nucleofector). Gene editing results are shown in the left panel while CFC potential results are showin the right panel.
**Fig. 49B** depicts comparative gene editing (left panel), fold expansion (middle panel) and CFC potential (right panel) of CB CD34⁺ cells after electroporation with D10A Cas9 RNP targeting *HBB* locus (dual nickase strategy, in which *HBB-8* (SEQ ID NO:398) and *HBB-15* (SEQ ID NO:397) gRNAs are both modified at 5' and/or 3' end).
**Fig. 49C** depicts the effect of electroporation of CB CD34⁺ cells with unmodified IVT gRNAs, single (5' or 3') or double (both 5' and 3') end modified IVT gRNAs, on gene editing (left panel) and CFC potential (right panel) in CB CD34⁺ cells.

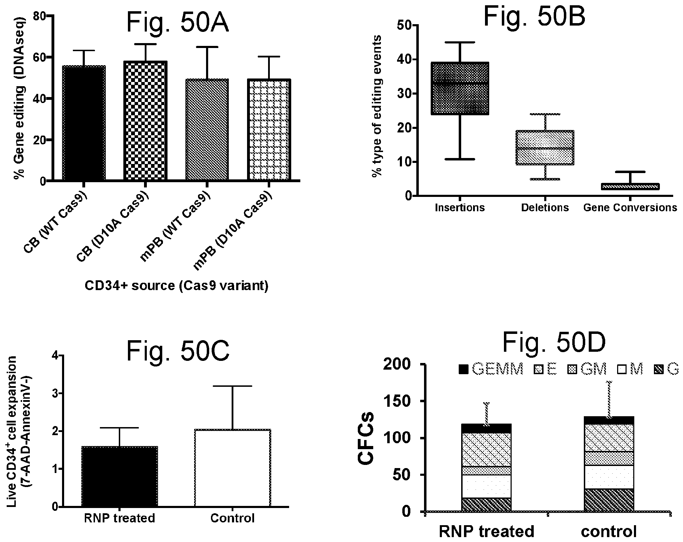

**Figs. 50A, 50B, 50C,** and **50D** demonstrate that Cas9 RNP supports highly efficient gene editing at the *HBB* locus in human adult and cord blood CD34⁺ hematopoietic stem/progenitor cells from 15 different stem cell donors after electroporation of RNP. Paired t-tests were performed for each donor pair represented by the data in **Fig. 50A****-****Fig. 50D****.**
**Fig. 50A** depicts a summary of gene editing results as determined by DNA sequencing of composite data from n=15 CD34⁺ cell donors and 15 experiments. Gene editing is shown for cord blood (CB) and adult mobilized peripheral blood (mPB) CD34⁺ cell donors. The Cas9 variant (D10A nickase or wild type, WT) are indicated.
**Fig. 50B** depicts a summary of types of editing events detected in experiments in which CD34⁺ cells (n=10 donors) were contacted with D10A nickase RNP and gRNA pair (*HBB*-8 (SEQ ID NO:398) and *HBB*-15 (SEQ ID NO:397)).
**Fig. 50C** shows fold expansion of RNP treated and paired untreated control CD34⁺ cells 2-3 days after electroporation.
**Fig. 50D** depicts a composite summary of CFC data indicating the total colonies differentiated from human CD34⁺ cells, erythroid and myeloid subtypes (n=10 donors). Mean and standard deviation are shown for all plots.

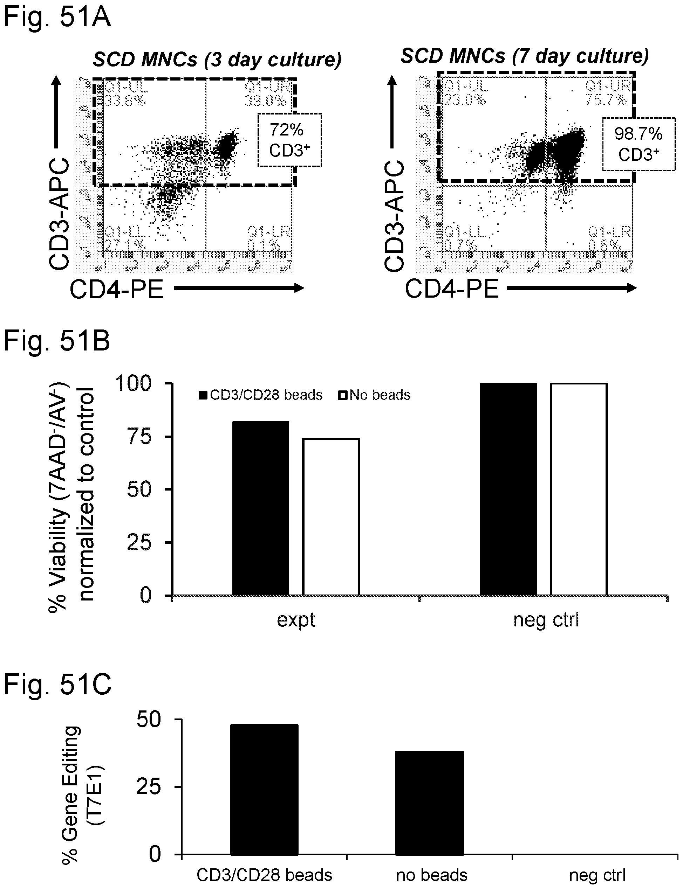

**Fig. 51A** shows that the cell population of MNC culture in T cell media was 72% CD3⁺ by day 3 (left panel) and greater than 98% by day 7 (right panel).
**Fig. 51B** shows that re-plating the edited cells into culture with CD3/CD28 beads for cell reactivation improved the total viability of the electroporated cells in comparison to cells plated in T cell media without CD3/CD28 beads (% viability determined by flow cytometry analysis after staining with apoptosis stains 7-AAD and Annexin V).
**Fig. 51C** shows that the gRNA combination *HBB-*8-sickle (SEQ ID NO:414) and *HBB-15* (SEQ ID NO:397) (each complexed to D10A Cas9 protein) supported 48% total editing, as detected by T7E1 endonuclease assay analysis of the *HBB* PCR product.

### DETAILED DESCRIPTION

### Definitions

"ALT-HDR" or "alternative HDR", or alternative homology-directed repair, as used herein, refers to the process of repairing DNA damage using a homologous nucleic acid (e.g., an endogenous homologous sequence, e.g., a sister chromatid, or an exogenous nucleic acid, e.g., a template nucleic acid). ALT-HDR is distinct from canonical HDR in that the process utilizes different pathways from canonical HDR, and can be inhibited by the canonical HDR mediators, RAD51 and BRCA2. Also, ALT-HDR uses a single-stranded or nicked homologous nucleic acid for repair of the break.

"Canonical HDR", or canonical homology-directed repair, as used herein, refers to the process of repairing DNA damage using a homologous nucleic acid (e.g., an endogenous homologous sequence, e.g., a sister chromatid, or an exogenous nucleic acid, e.g., a template nucleic acid). Canonical HDR typically acts when there has been significant resection at the double strand break, forming at least one single stranded portion of DNA. In a normal cell, HDR typically involves a series of steps such as recognition of the break, stabilization of the break, resection, stabilization of single stranded DNA, formation of a DNA crossover intermediate, resolution of the crossover intermediate, and ligation. The process requires RAD51 and BRCA2, and the homologous nucleic acid is typically double-stranded.

Unless indicated otherwise, the term "HDR" as used herein encompasses canonical HDR and alt-HDR.

"Domain", as used herein, is used to describe segments of a protein or nucleic acid. Unless otherwise indicated, a domain is not required to have any specific functional property.

Calculations of homology or sequence identity between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frame shift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences.

"Governing gRNA molecule", as used herein, refers to a gRNA molecule that comprises a targeting domain that is complementary to a target domain on a nucleic acid that comprises a sequence that encodes a component of the CRISPR/Cas system that is introduced into a cell. A governing gRNA does not target an endogenous cell. In an embodiment, a governing gRNA molecule comprises a targeting domain that is complementary with a target sequence on:
(a) a nucleic acid that encodes a Cas (e.g., Cas9) protein,
(b) a nucleic acid that encodes a gRNA which comprises a targeting domain for a gene (a target gene gRNA); or on more than one nucleic acid that encodes a CRISPR/Cas component, e.g., both (a) and (b). In an embodiment, a nucleic acid molecule that encodes a CRISPR/Cas component, e.g., that encodes a Cas9 protein or a target gene gRNA, comprises more than one target domain that is complementary with a governing gRNA targeting domain. While not wishing to be bound by theory, it is believed that in such embodiments a governing gRNA molecule complexes with a Cas9 protein and results in Cas9 mediated inactivation of the targeted nucleic acid, e.g., by cleavage or by binding to the nucleic acid, and results in cessation or reduction of the production of a CRISPR/Cas system component. In an embodiment, the Cas9 protein forms two complexes: a complex comprising a Cas9 protein with a target gene gRNA, which complex will alter the gene; and a complex comprising a Cas9 protein with a governing gRNA molecule, which complex will act to prevent further production of a CRISPR/Cas system component, e.g., a Cas9 protein or a target gene gRNA molecule. In an embodiment, a governing gRNA molecule/Cas protein (e.g., gRNA molecule/Cas9 protein) complex binds to or promotes cleavage of a control region sequence, e.g., a promoter, operably linked to a sequence that encodes a Cas9 protein, a sequence that encodes a transcribed region, an exon, or an intron, for the Cas9 protein. In an embodiment, a governing gRNA molecule/Cas protein (e.g., gRNA molecule/Cas9 protein complex binds to or promotes cleavage of a control region sequence, e.g., a promoter, operably linked to a gRNA molecule, or a sequence that encodes the gRNA molecule. In an embodiment, the governing gRNA, e.g., a Cas9-targeting governing gRNA molecule, or a target gene gRNA-targeting governing gRNA molecule, limits the effect of the Cas9 protein/target gene gRNA molecule complex-mediated gene targeting. In an embodiment, a governing gRNA places temporal, level of expression, or other limits, on activity of the Cas9 protein/target gene gRNA molecule complex.
In an embodiment, a governing gRNA reduces off-target or other unwanted activity. In an embodiment, a governing gRNA molecule inhibits, e.g., entirely or substantially entirely inhibits, the production of a component of the Cas9 system and thereby limits, or governs, its activity.

"Modulator", as used herein, refers to an entity, e.g., a drug, which can alter the activity (e.g., enzymatic activity, transcriptional activity, or translational activity), amount, distribution, or structure of a subject molecule or genetic sequence. In an embodiment, modulation comprises cleavage, e.g., breaking of a covalent or non-covalent bond, or the forming of a covalent or non-covalent bond, e.g., the attachment of a moiety, to the subject molecule. In an embodiment, a modulator alters the, three dimensional, secondary, tertiary, or quaternary structure, of a subject molecule. A modulator can increase, decrease, initiate, or eliminate a subject activity.

"Large molecule", as used herein, refers to a molecule having a molecular weight of at least 2, 3, 5, 10, 20, 30, 40, 50, 60, 70, 80, 90, or 100 kD. Large molecules include proteins, polypeptides, nucleic acids, biologics, and carbohydrates.

"Polypeptide", as used herein, refers to a polymer of amino acids having less than 100 amino acid residues. In an embodiment, it has less than 50, 20, or 10 amino acid residues.

"Non-homologous end joining" or "NHEJ", as used herein, refers to ligation mediated repair and/or non-template mediated repair including, e.g., canonical NHEJ (cNHEJ), alternative NHEJ (altNHEJ), microhomology-mediated end joining (MMEJ), single-strand annealing (SSA), and synthesis-dependent microhomology-mediated end joining (SD-MMEJ).

"Reference molecule", e.g., a reference Cas (e.g., Cas9) molecule or reference gRNA, as used herein, refers to a molecule to which a subject molecule, e.g., a subject Cas9 molecule of subject gRNA molecule, e.g., a modified or candidate Cas9 molecule is compared. For example, a Cas9 molecule can be characterized as having no more than 10% of the nuclease activity of a reference Cas9 molecule. Examples of reference Cas9 molecules include naturally occurring unmodified Cas9 molecules, e.g., a naturally occurring Cas9 molecule such as a Cas9 molecule of *S. pyogenes, S. aureus,* or *S. thermophilus.* In an embodiment, the reference Cas9 molecule is the naturally occurring Cas9 molecule having the closest sequence identity or homology with the Cas9 molecule to which it is being compared. In an embodiment, the reference Cas9 molecule is a sequence, e.g., a naturally occurring or known sequence, which is the parental form on which a change, e.g., a mutation has been made.

"Replacement", or "replaced", as used herein with reference to a modification of a molecule does not require a process limitation but merely indicates that the replacement entity is present.

"Small molecule", as used herein, refers to a compound having a molecular weight less than about 2 kD, e.g., less than about 2 kD, less than about 1.5 kD, less than about 1 kD, or less than about 0.75 kD.

"Subject", as used herein, may mean either a human or non-human animal. The term includes, but is not limited to, mammals (e.g., humans, other primates, pigs, rodents (e.g., mice and rats or hamsters), rabbits, guinea pigs, cows, horses, cats, dogs, sheep, and goats). In an embodiment, the subject is a human. In other embodiments, the subject is poultry.

"Treat", "treating" and "treatment", as used herein, mean the treatment of a disease in a mammal, e.g., in a human, including (a) inhibiting the disease, i.e., arresting or preventing its development; (b) relieving the disease, i.e., causing regression of the disease state; and (c) curing the disease.

"X" as used herein in the context of an amino acid sequence, refers to any amino acid (e.g., any of the twenty natural amino acids) unless otherwise specified.

### I. gRNA Molecules

A gRNA molecule, as that term is used herein, refers to a nucleic acid that promotes the specific targeting or homing of a gRNA molecule/Cas molecule complex (e.g., a gRNA/Cas9 complex) to a target nucleic acid. gRNA molecules can be unimolecular (having a single RNA molecule), sometimes referred to herein as "chimeric" gRNAs, or modular (comprising more than one, and typically two, separate RNA molecules). A gRNA molecule comprises a number of domains. The gRNA molecule domains are described in more detail below.

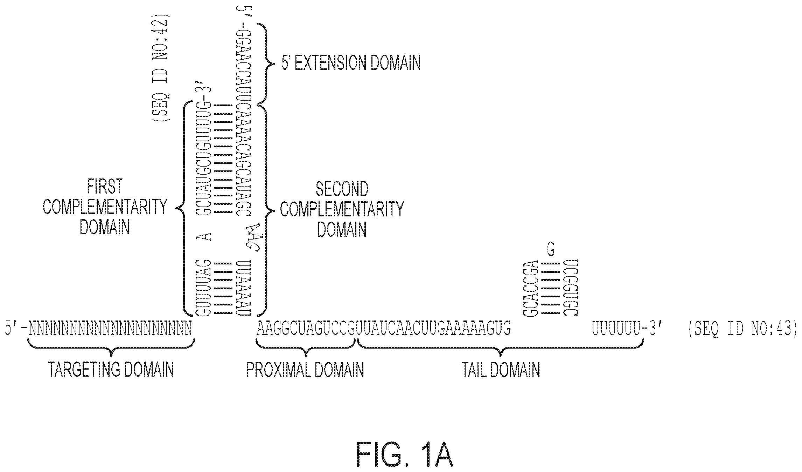

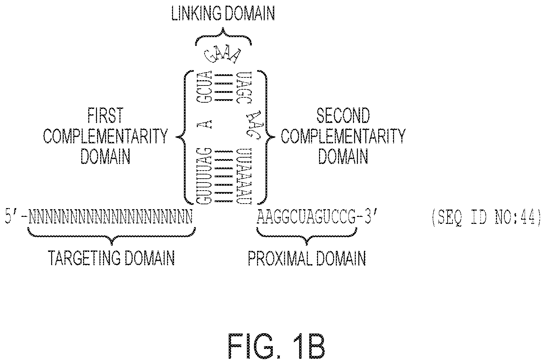

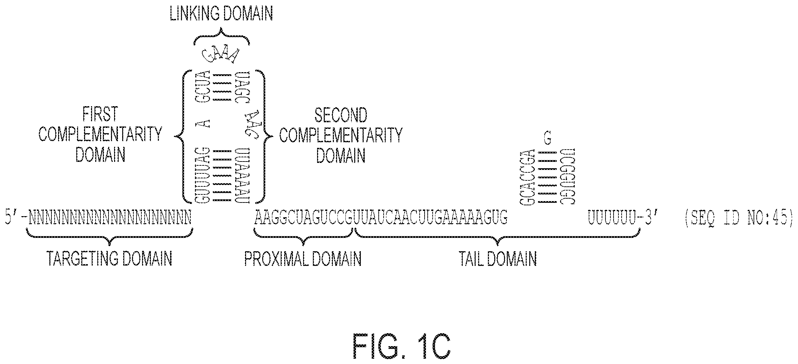

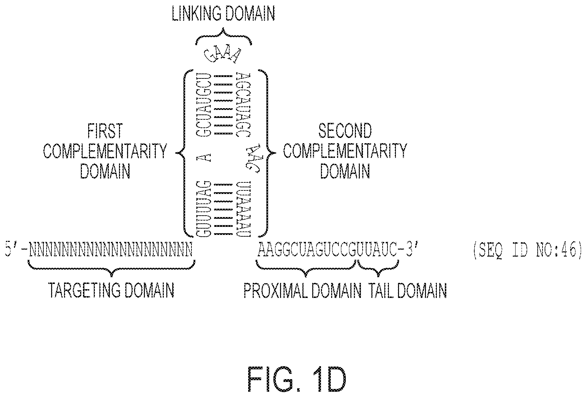

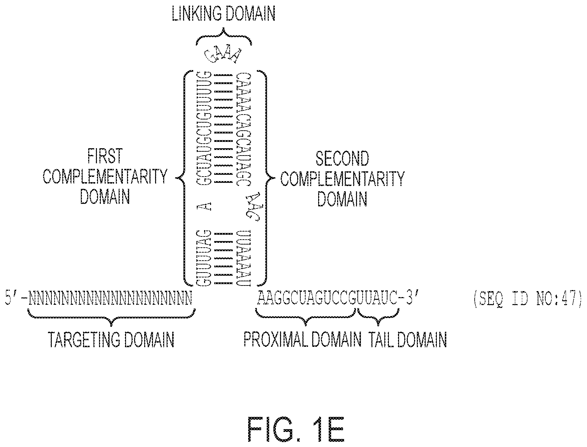

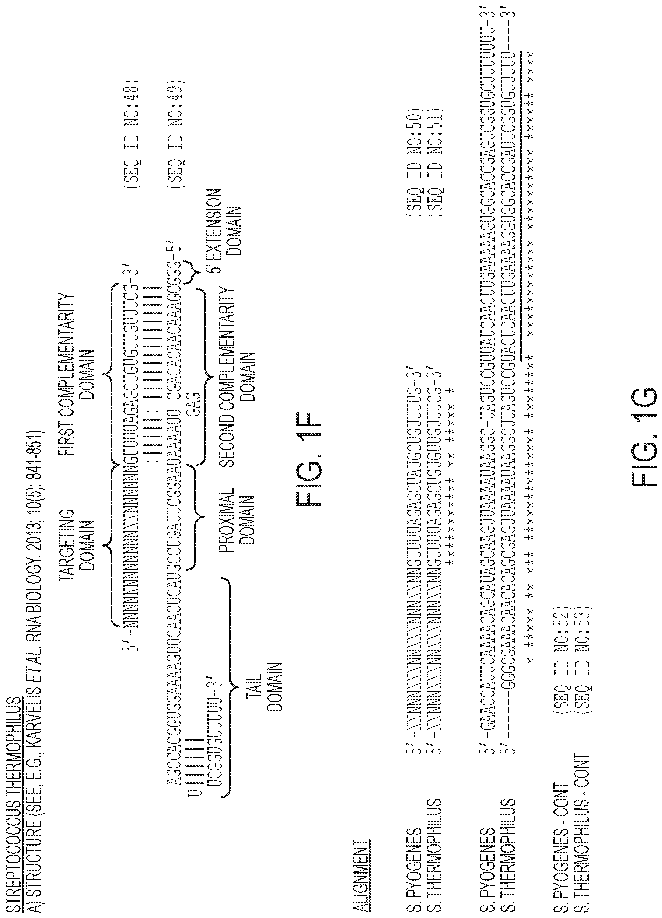

Several exemplary gRNA structures, with domains indicated thereon, are provided in **Fig. 1****.** While not wishing to be bound by theory, with regard to the three dimensional form, or intra- or inter-strand interactions of an active form of a gRNA, regions of high complementarity are sometimes shown as duplexes in **Fig. 1** and other depictions provided herein.

In an embodiment, a unimolecular, or chimeric, gRNA comprises, preferably from 5' to 3':
a targeting domain (which is complementary to a target nucleic acid in a gene);
a first complementarity domain;
a linking domain;
a second complementarity domain (which is complementary to the first complementarity domain);
a proximal domain; and
optionally, a tail domain.

In an embodiment, a modular gRNA comprises:
a first strand comprising, preferably from 5' to 3';
   a targeting domain (which is complementary to a target nucleic acid in a gene); and
   a first complementarity domain; and
a second strand, comprising, preferably from 5' to 3':
   optionally, a 5' extension domain;
   a second complementarity domain;
   a proximal domain; and
   optionally, a tail domain.

The domains are discussed briefly below.

### The Targeting Domain

**Figs. 1A-1G** provide examples of the placement of targeting domains.

The targeting domain comprises a nucleotide sequence that is complementary, e.g., at least 80, 85, 90, 95, 98 or 99% complementary, e.g., fully complementary, to the target sequence on the target nucleic acid. The targeting domain is part of an RNA molecule and will therefore comprise the base uracil (U), while any DNA encoding the gRNA molecule will comprise the base thymine (T). While not wishing to be bound by theory, in an embodiment, it is believed that the complementarity of the targeting domain with the target sequence contributes to specificity of the interaction of the gRNA molecule/Cas molecule (e.g., gRNA molecule/Cas9 molecule) complex with a target nucleic acid. It is understood that in a targeting domain and target sequence pair, the uracil bases in the targeting domain will pair with the adenine bases in the target sequence. In an embodiment, the target domain itself comprises in the 5' to 3' direction, an optional secondary domain, and a core domain. In an embodiment, the core domain is fully complementary with the target sequence. In an embodiment, the targeting domain is 5 to 50 nucleotides in length. The strand of the target nucleic acid with which the targeting domain is complementary is referred to herein as the complementary strand. Some or all of the nucleotides of the domain can have a modification, e.g., modification found in Section VIII herein.

In an embodiment, the targeting domain is 16 nucleotides in length.

In an embodiment, the targeting domain is 17 nucleotides in length.

In an embodiment, the targeting domain is 18 nucleotides in length.

In an embodiment, the targeting domain is 19 nucleotides in length.

In an embodiment, the targeting domain is 20 nucleotides in length.

In an embodiment, the targeting domain is 21 nucleotides in length.

In an embodiment, the targeting domain is 22 nucleotides in length.

In an embodiment, the targeting domain is 23 nucleotides in length.

In an embodiment, the targeting domain is 24 nucleotides in length.

In an embodiment, the targeting domain is 25 nucleotides in length.

In an embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

Targeting domains are discussed in more detail below.

### The First Complementarity Domain

**Figs. 1A-1G** provide examples of first complementarity domains.

The first complementarity domain is complementary with the second complementarity domain, and in an embodiment, has sufficient complementarity to the second complementarity domain to form a duplexed region under at least some physiological conditions. In an embodiment, the first complementarity domain is 5 to 30 nucleotides in length. In an embodiment, the first complementarity domain is 5 to 25 nucleotides in length. In an embodiment, the first complementary domain is 7 to 25 nucleotides in length. In an embodiment, the first complementary domain is 7 to 22 nucleotides in length. In an embodiment, the first complementary domain is 7 to 18 nucleotides in length. In an embodiment, the first complementary domain is 7 to 15 nucleotides in length. In an embodiment, the first complementary domain is 5, 6, 7, 8, 9, 10, 11, 12, 13, 14, 15, 16, 17, 18, 19, 20, 21, 22, 23, 24, or 25 nucleotides in length.

In an embodiment, the first complementarity domain comprises 3 subdomains, which, in the 5' to 3' direction are: a 5' subdomain, a central subdomain, and a 3' subdomain. In an embodiment, the 5' subdomain is 4-9, e.g., 4, 5, 6, 7, 8 or 9 nucleotides in length. In an embodiment, the central subdomain is 1, 2, or 3, e.g., 1, nucleotide in length. In an embodiment, the 3' subdomain is 3 to 25, e.g., 4-22, 4-18, or 4 to 10, or 3, 4, 5, 6, 7, 8, 9, 10, 11, 12, 13, 14, 15, 16, 17, 18, 19, 20, 21, 22, 23, 24, or 25, nucleotides in length.

The first complementarity domain can share homology with, or be derived from, a naturally occurring first complementarity domain. In an embodiment, it has at least 50% homology with a first complementarity domain disclosed herein, e.g., an *S. pyogenes, S. aureus, N. meningtidis,* or *S. thermophilus,* first complementarity domain.

Some or all of the nucleotides of the first complementarity domain can have a modification, e.g., a modification found in Section VIII herein.

First complementarity domains are discussed in more detail below.

### The Linking Domain

**Figs. 1A-1G** provide examples of linking domains.

A linking domain serves to link the first complementarity domain with the second complementarity domain of a unimolecular gRNA. The linking domain can link the first and second complementarity domains covalently or non-covalently. In an embodiment, the linkage is covalent. In an embodiment, the linking domain covalently couples the first and second complementarity domains, see, e.g., **Figs. 1B-1E****.** In an embodiment, the linking domain is, or comprises, a covalent bond interposed between the first complementarity domain and the second complementarity domain. Typically the linking domain comprises one or more, e.g., 2, 3, 4, 5, 6, 7, 8, 9, or 10 nucleotides.

In modular gRNA molecules the two molecules are associated by virtue of the hybridization of the complementarity domains see e.g., **Fig. 1A****.**

A wide variety of linking domains are suitable for use in unimolecular gRNA molecules. Linking domains can consist of a covalent bond, or be as short as one or a few nucleotides, e.g., 1, 2, 3, 4, or 5 nucleotides in length. In an embodiment, a linking domain is 2, 3, 4, 5, 6, 7, 8, 9, 10, 15, 20, or 25 or more nucleotides in length. In an embodiment, a linking domain is 2 to 50, 2 to 40, 2 to 30, 2 to 20, 2 to 10, or 2 to 5 nucleotides in length. In an embodiment, a linking domain shares homology with, or is derived from, a naturally occurring sequence, e.g., the sequence of a tracrRNA that is 5' to the second complementarity domain. In an embodiment, the linking domain has at least 50% homology with a linking domain disclosed herein.

Some or all of the nucleotides of the linking domain can have a modification, e.g., a modification found in Section VIII herein.

Linking domains are discussed in more detail below.

### The 5' Extension Domain

In an embodiment, a modular gRNA can comprise additional sequence, 5' to the second complementarity domain, referred to herein as the 5' extension domain, see, e.g., **Fig. 1A****.** In an embodiment, the 5' extension domain is, 2-10, 2-9, 2-8, 2-7, 2-6, 2-5, or 2-4 nucleotides in length. In an embodiment, the 5' extension domain is 2, 3, 4, 5, 6, 7, 8, 9, or 10 or more nucleotides in length.

### The Second Complementarity Domain

**Figs. 1A-1G** provide examples of second complementarity domains.

The second complementarity domain is complementary with the first complementarity domain, and in an embodiment, has sufficient complementarity to the second complementarity domain to form a duplexed region under at least some physiological conditions. In an embodiment, e.g., as shown in **Figs. 1A-1B****,** the second complementarity domain can include sequence that lacks complementarity with the first complementarity domain, e.g., sequence that loops out from the duplexed region.

In an embodiment, the second complementarity domain is 5 to 27 nucleotides in length. In an embodiment, it is longer than the first complementarity region. In an embodiment the second complementary domain is 7 to 27 nucleotides in length. In an embodiment, the second complementary domain is 7 to 25 nucleotides in length. In an embodiment, the second complementary domain is 7 to 20 nucleotides in length. In an embodiment, the second complementary domain is 7 to 17 nucleotides in length. In an embodiment, the complementary domain is 5, 6, 7, 8, 9, 10, 11, 12, 13, 14, 15, 16, 17, 18, 19, 20, 21, 22, 23, 24, 25, or 26 nucleotides in length.

In an embodiment, the second complementarity domain comprises 3 subdomains, which, in the 5' to 3' direction are: a 5' subdomain, a central subdomain, and a 3' subdomain. In an embodiment, the 5' subdomain is 3 to 25, e.g., 4 to 22, 4 tol8, or 4 to 10, or 3, 4, 5, 6, 7, 8, 9, 10, 11, 12, 13, 14, 15, 16, 17, 18, 19, 20, 21, 22, 23, 24, or 25 nucleotides in length. In an embodiment, the central subdomain is 1, 2, 3, 4 or 5, e.g., 3, nucleotides in length. In an embodiment, the 3' subdomain is 4 to 9, e.g., 4, 5, 6, 7, 8 or 9 nucleotides in length.

In an embodiment, the 5' subdomain and the 3' subdomain of the first complementarity domain, are respectively, complementary, e.g., fully complementary, with the 3' subdomain and the 5' subdomain of the second complementarity domain.

The second complementarity domain can share homology with or be derived from a naturally occurring second complementarity domain. In an embodiment, it has at least 50% homology with a second complementarity domain disclosed herein, e.g., an *S. pyogenes, S. aureus, N. meningtidis,* or *S. thermophilus,* first complementarity domain.

Some or all of the nucleotides of the second complementarity domain can have a modification, e.g., a modification found in Section VIII herein.

### A Proximal domain

**Figs. 1A-1G** provide examples of proximal domains.

In an embodiment, the proximal domain is 5 to 20 nucleotides in length. In an embodiment, the proximal domain can share homology with or be derived from a naturally occurring proximal domain. In an embodiment, it has at least 50% homology with a proximal domain disclosed herein, e.g., an *S. pyogenes, S. aureus, N. meningtidis,* or *S. thermophilus,* proximal domain.

Some or all of the nucleotides of the proximal domain can have a modification, e.g., a modification found in Section VIII herein.

### A Tail Domain

**Figs. 1A-1G** provide examples of tail domains.

As can be seen by inspection of the tail domains in **Fig. 1A** and **Figs. 1B****-1F,** a broad spectrum of tail domains are suitable for use in gRNA molecules. In an embodiment, the tail domain is 0 (absent), 1, 2, 3, 4, 5, 6, 7, 8, 9, or 10 nucleotides in length. In embodiment, the tail domain nucleotides are from or share homology with sequence from the 5' end of a naturally occurring tail domain, see e.g., **Fig. 1D** **or** **1E****.** In an embodiment, the tail domain includes sequences that are complementary to each other and which, under at least some physiological conditions, form a duplexed region.

In an embodiment, the tail domain is absent or is 1 to 50 nucleotides in length. In an embodiment, the tail domain can share homology with or be derived from a naturally occurring proximal tail domain. In an embodiment, it has at least 50% homology with a tail domain disclosed herein, e.g., an *S. pyogenes, S. aureus, N. meningtidis,* or *S. thermophilus,* tail domain.

In an embodiment, the tail domain includes nucleotides at the 3' end that are related to the method of *in vitro* transcription. When a T7 promoter is used for *in vitro* transcription of the gRNA, these nucleotides may be any nucleotides present before the 3' end of the DNA template. When alternate pol-III promoters are used, these nucleotides may be various numbers or uracil bases or may include alternate bases.

The domains of gRNA molecules are described in more detail below.

### The Targeting Domain

The "targeting domain" of the gRNA is complementary to the "target domain" on the target nucleic acid. The strand of the target nucleic acid comprising the core domain target is referred to herein as the "complementary strand" of the target nucleic acid. Guidance on the selection of targeting domains can be found, e.g., in Fu Y et al., Nat Biotechnol 2014 (doi: 10.1038/nbt.2808) and Sternberg SH et al., Nature 2014 (doi: 10.1038/nature13011).

In an embodiment, the targeting domain is 16, 17, 18, 19, 20, 21, 22, 23, 24, 25 or 26 nucleotides in length.

In an embodiment, the targeting domain is 16 nucleotides in length.

In an embodiment, the targeting domain is 17 nucleotides in length.

In an embodiment, the targeting domain is 18 nucleotides in length.

In an embodiment, the targeting domain is 19 nucleotides in length.

In an embodiment, the targeting domain is 20 nucleotides in length.

In an embodiment, the targeting domain is 21 nucleotides in length.

In an embodiment, the targeting domain is 22 nucleotides in length.

In an embodiment, the targeting domain is 23 nucleotides in length.

In an embodiment, the targeting domain is 24 nucleotides in length.

In an embodiment, the targeting domain is 25 nucleotides in length.

In an embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides. In an embodiment, the targeting domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the targeting domain is 20+/-5 nucleotides in length.

In an embodiment, the targeting domain is 20+/-10, 30+/-10, 40+/-10, 50+/-10, 60+/-10, 70+/-10, 80+/-10, 90+/-10, or 100+/-10 nucleotides, in length.

In an embodiment, the targeting domain is 30+/-10 nucleotides in length.

In an embodiment, the targeting domain is 10 to 100, 10 to 90, 10 to 80, 10 to 70, 10 to 60, 10 to 50, 10 to 40, 10 to 30, 10 to 20 or 10 to 15 nucleotides in length.

In other embodiments, the targeting domain is 20 to 100, 20 to 90, 20 to 80, 20 to 70, 20 to 60, 20 to 50, 20 to 40, 20 to 30, or 20 to 25 nucleotides in length.

Typically the targeting domain has full complementarity with the target sequence. In some embodiments the targeting domain has or includes 1, 2, 3, 4, 5, 6, 7 or 8 nucleotides that are not complementary with the corresponding nucleotide of the targeting domain.

In an embodiment, the target domain includes 1, 2, 3, 4 or 5 nucleotides that are complementary with the corresponding nucleotide of the targeting domain within 5 nucleotides of its 5' end. In an embodiment, the target domain includes 1, 2, 3, 4 or 5 nucleotides that are complementary with the corresponding nucleotide of the targeting domain within 5 nucleotides of its 3' end.

In an embodiment, the target domain includes 1, 2, 3, or 4 nucleotides that are not complementary with the corresponding nucleotide of the targeting domain within 5 nucleotides of its 5' end. In an embodiment, the target domain includes 1, 2, 3, or 4 nucleotides that are not complementary with the corresponding nucleotide of the targeting domain within 5 nucleotides of its 3' end.

In an embodiment, the degree of complementarity, together with other properties of the gRNA, is sufficient to allow targeting of a Cas9 molecule to the target nucleic acid.

In some embodiments, the targeting domain comprises two consecutive nucleotides that are not complementary to the target domain ("non-complementary nucleotides"), e.g., two consecutive noncomplementary nucleotides that are within 5 nucleotides of the 5' end of the targeting domain, within 5 nucleotides of the 3' end of the targeting domain, or more than 5 nucleotides away from one or both ends of the targeting domain.

In an embodiment, no two consecutive nucleotides within 5 nucleotides of the 5' end of the targeting domain, within 5 nucleotides of the 3' end of the targeting domain, or within a region that is more than 5 nucleotides away from one or both ends of the targeting domain, are not complementary to the targeting domain.

In an embodiment, there are no noncomplementary nucleotides within 5 nucleotides of the 5' end of the targeting domain, within 5 nucleotides of the 3' end of the targeting domain, or within a region that is more than 5 nucleotides away from one or both ends of the targeting domain.

In an embodiment, the targeting domain nucleotides do not comprise modifications, e.g., modifications of the type provided in Section VIII. However, in an embodiment, the targeting domain comprises one or more modifications, e.g., modifications that render it less susceptible to degradation or more bio-compatible, e.g., less immunogenic. By way of example, the backbone of the targeting domain can be modified with a phosphorothioate, or other modification(s) from Section VIII. In an embodiment, a nucleotide of the targeting domain can comprise a 2' modification, e.g., a 2-acetylation, e.g., a 2' methylation, or other modification(s) from Section VIII.

In some embodiments, the targeting domain includes 1, 2, 3, 4, 5, 6, 7 or 8 or more modifications. In an embodiment, the targeting domain includes 1, 2, 3, or 4 modifications within 5 nucleotides of its 5' end. In an embodiment, the targeting domain comprises as many as 1, 2, 3, or 4 modifications within 5 nucleotides of its 3' end.

In some embodiments, the targeting domain comprises modifications at two consecutive nucleotides, e.g., two consecutive nucleotides that are within 5 nucleotides of the 5' end of the targeting domain, within 5 nucleotides of the 3' end of the targeting domain, or more than 5 nucleotides away from one or both ends of the targeting domain.

In an embodiment, no two consecutive nucleotides are modified within 5 nucleotides of the 5' end of the targeting domain, within 5 nucleotides of the 3' end of the targeting domain, or within a region that is more than 5 nucleotides away from one or both ends of the targeting domain. In an embodiment, no nucleotide is modified within 5 nucleotides of the 5' end of the targeting domain, within 5 nucleotides of the 3' end of the targeting domain, or within a region that is more than 5 nucleotides away from one or both ends of the targeting domain.

Modifications in the targeting domain can be selected to not interfere with targeting efficacy, which can be evaluated by testing a candidate modification in the system described in Section IV. gRNAs having a candidate targeting domain having a selected length, sequence, degree of complementarity, or degree of modification, can be evaluated in a system in Section IV. The candidate targeting domain can be placed, either alone, or with one or more other candidate changes in a gRNA molecule/Cas molecule (e.g., gRNA molecule/Cas9 molecule) system known to be functional with a selected target and evaluated.

In some embodiments, all of the modified nucleotides are complementary to and capable of hybridizing to corresponding nucleotides present in the target domain. In other embodiments, 1, 2, 3, 4, 5, 6, 7 or 8 or more modified nucleotides are not complementary to or capable of hybridizing to corresponding nucleotides present in the target domain.

In an embodiment, the targeting domain comprises, preferably in the 5'→3' direction: a secondary domain and a core domain. These domains are discussed in more detail below.

### The Core Domain and Secondary Domain of the Targeting Domain

The "core domain" of the targeting domain is complementary to the "core domain target" on the target nucleic acid. In an embodiment, the core domain comprises about 8 to about 13 nucleotides from the 3' end of the targeting domain (e.g., the most 3' 8 to 13 nucleotides of the targeting domain).

In an embodiment, the secondary domain is absent or optional.

In an embodiment, the core domain and targeting domain are independently 6 +/-2, 7+/-2, 8+/-2, 9+/-2, 10+/-2, 11+/-2, 12+/-2, 13+/-2, 14+/-2, 15+/-2, or 16+-2, nucleotides in length.

In an embodiment, the core domain and targeting domain are independently 10+/-2 nucleotides in length.

In an embodiment, the core domain and targeting domain are independently 10+/-4 nucleotides in length.

In an embodiment, the core domain and targeting domain are independently 6, 7, 8, 9, 10, 11, 12, 13, 14, 15 or 16 nucleotides in length.

In an embodiment, the core and targeting domain are independently 3 to 20, 4 to 20, 5 to 20, 6 to 20, 7 to 20, 8 to 20, 9 to 20 10 to 20 or 15 to 20 nucleotides in length.

In an embodiment, the core and targeting domain, are independently 3 to 15, e.g., 6 to 15, 7 to 14, 7 to 13, 6 to 12, 7 to 12, 7 to 11, 7 to 10, 8 to 14, 8 to 13, 8 to 12, 8 to 11, 8 to 10 or 8 to 9 nucleotides in length.

The core domain is complementary with the core domain target. Typically the core domain has exact complementarity with the core domain target. In some embodiments, the core domain can have 1, 2, 3, 4 or 5 nucleotides that are not complementary with the corresponding nucleotide of the core domain. In an embodiment, the degree of complementarity, together with other properties of the gRNA, is sufficient to allow targeting of a Cas molecule (e.g., Cas9 molecule) to the target nucleic acid.

The "secondary domain" of the targeting domain of the gRNA is complementary to the "secondary domain target" of the target nucleic acid.

In an embodiment, the secondary domain is positioned 5' to the core domain.

In an embodiment, the secondary domain is absent or optional.

In an embodiment, if the targeting domain is 26 nucleotides in length and the core domain (counted from the 3' end of the targeting domain) is 8 to 13 nucleotides in length, the secondary domain is 12 to 17 nucleotides in length.

In an embodiment, if the targeting domain is 25 nucleotides in length and the core domain (counted from the 3' end of the targeting domain) is 8 to 13 nucleotides in length, the secondary domain is 12 to 17 nucleotides in length.

In an embodiment, if the targeting domain is 24 nucleotides in length and the core domain (counted from the 3' end of the targeting domain) is 8 to 13 nucleotides in length, the secondary domain is 11 to 16 nucleotides in length.

In an embodiment, if the targeting domain is 23 nucleotides in length and the core domain (counted from the 3' end of the targeting domain) is 8 to 13 nucleotides in length, the secondary domain is 10 to 15 nucleotides in length.

In an embodiment, if the targeting domain is 22 nucleotides in length and the core domain (counted from the 3' end of the targeting domain) is 8 to 13 nucleotides in length, the secondary domain is 9 to 14 nucleotides in length.

In an embodiment, if the targeting domain is 21 nucleotides in length and the core domain (counted from the 3' end of the targeting domain) is 8 to 13 nucleotides in length, the secondary domain is 8 to 13 nucleotides in length.

In an embodiment, if the targeting domain is 20 nucleotides in length and the core domain (counted from the 3' end of the targeting domain) is 8 to 13 nucleotides in length, the secondary domain is 7 to 12 nucleotides in length.

In an embodiment, if the targeting domain is 19 nucleotides in length and the core domain (counted from the 3' end of the targeting domain) is 8 to 13 nucleotides in length, the secondary domain is 6 to 11 nucleotides in length.

In an embodiment, if the targeting domain is 18 nucleotides in length and the core domain (counted from the 3' end of the targeting domain) is 8 to 13 nucleotides in length, the secondary domain is 5 to 10 nucleotides in length.

In an embodiment, if the targeting domain is 17 nucleotides in length and the core domain (counted from the 3' end of the targeting domain) is 8 to 13 nucleotides in length, the secondary domain is 4 to 9 nucleotides in length.

In an embodiment, if the targeting domain is 16 nucleotides in length and the core domain (counted from the 3' end of the targeting domain) is 8 to 13 nucleotides in length, the secondary domain is 3 to 8 nucleotides in length.

In an embodiment, the secondary domain is 0, 1, 2, 3, 4, 5, 6, 7, 8, 9, 10, 11, 12, 13, 14 or 15 nucleotides in length.

The secondary domain is complementary with the secondary domain target. Typically the secondary domain has exact complementarity with the secondary domain target. In some embodiments the secondary domain can have 1, 2, 3, 4 or 5 nucleotides that are not complementary with the corresponding nucleotide of the secondary domain. In an embodiment, the degree of complementarity, together with other properties of the gRNA, is sufficient to allow targeting of a Cas9 molecule to the target nucleic acid.

In an embodiment, the core domain nucleotides do not comprise modifications, e.g., modifications of the type provided in Section VIII. However, in an embodiment, the core domain comprises one or more modifications, e.g., modifications that render it less susceptible to degradation or more bio-compatible, e.g., less immunogenic. By way of example, the backbone of the core domain can be modified with a phosphorothioate, or other modification(s) from Section VIII. In an embodiment a nucleotide of the core domain can comprise a 2' modification (e.g., a modification at the 2' position on ribose), e.g., a 2-acetylation, e.g., a 2' methylation, or other modification(s) from Section VIII. Typically, a core domain will contain no more than 1, 2, or 3 modifications.

Modifications in the core domain can be selected to not interfere with targeting efficacy, which can be evaluated by testing a candidate modification in the system described in Section IV. gRNAs having a candidate core domain having a selected length, sequence, degree of complementarity, or degree of modification, can be evaluated in the system described at Section IV. The candidate core domain can be placed, either alone, or with one or more other candidate changes in a gRNA molecule/Cas molecule (e.g., gRNA molecule/Cas9 molecule) system known to be functional with a selected target and evaluated.

In an embodiment, the secondary domain nucleotides do not comprise modifications, e.g., modifications of the type provided in Section VIII. However, in an embodiment, the secondary domain comprises one or more modifications, e.g., modifications that render it less susceptible to degradation or more bio-compatible, e.g., less immunogenic. By way of example, the backbone of the secondary domain can be modified with a phosphorothioate, or other modification(s) from Section VIII. In an embodiment a nucleotide of the secondary domain can comprise a 2' modification, e.g., a 2-acetylation, e.g., a 2' methylation, or other modification(s) from Section VIII. Typically, a secondary domain will contain no more than 1, 2, or 3 modifications.

Modifications in the secondary domain can be selected to not interfere with targeting efficacy, which can be evaluated by testing a candidate modification in the system described in Section IV. gRNAs having a candidate secondary domain having a selected length, sequence, degree of complementarity, or degree of modification, can be evaluated in the system described at Section IV. The candidate secondary domain can be placed, either alone, or with one or more other candidate changes in a gRNA molecule/Cas molecule (e.g., gRNA molecule/Cas9 molecule) system known to be functional with a selected target and evaluated.

In an embodiment, (1) the degree of complementarity between the core domain and its target, and (2) the degree of complementarity between the secondary domain and its target, may differ. In an embodiment, (1) may be greater than (2). In an embodiment, (1) may be less than (2). In an embodiment, (1) and (2) are the same, e.g., each may be completely complementary with its target.

In an embodiment, (1) the number of modifications (e.g., modifications from Section VIII) of the nucleotides of the core domain and (2) the number of modification (e.g., modifications from Section VIII) of the nucleotides of the secondary domain, may differ. In an embodiment, (1) may be less than (2). In an embodiment, (1) may be greater than (2). In an embodiment, (1) and (2) may be the same, e.g., each may be free of modifications.

### The First and Second Complementarity Domains

The first complementarity domain is complementary with the second complementarity domain.

Typically the first complementarity domain does not have exact complementarity with the second complementarity domain target. In some embodiments, the first complementarity domain can have 1, 2, 3, 4 or 5 nucleotides that are not complementary with the corresponding nucleotide of the second complementarity domain. In an embodiment, 1, 2, 3, 4, 5 or 6, e.g., 3 nucleotides, will not pair in the duplex, and, e.g., form a non-duplexed or looped-out region. In an embodiment, an unpaired, or loop-out, region, e.g., a loop-out of 3 nucleotides, is present on the second complementarity domain. In an embodiment, the unpaired region begins 1, 2, 3, 4, 5, or 6, e.g., 4, nucleotides from the 5' end of the second complementarity domain.

In an embodiment, the degree of complementarity, together with other properties of the gRNA, is sufficient to allow targeting of a Cas9 molecule to the target nucleic acid.

In an embodiment, the first and second complementarity domains are:
independently, 6 +/-2, 7+/-2, 8+/-2, 9+/-2, 10+/-2, 11+/-2, 12+/-2, 13+/-2, 14+/-2, 15+/-2, 16+/-2, 17+/-2, 18+/-2, 19+/-2, or 20+/-2, 21+/-2, 22+/-2, 23+/-2, or 24+/-2 nucleotides in length;
independently, 6, 7, 8, 9, 10, 11, 12, 13, 14, 14, 16, 17, 18, 19, 20, 21, 22, 23, 24, 25, or 26, nucleotides in length; or
independently, 5 to 24, 5 to 23, 5 to 22, 5 to 21, 5 to 20, 7 to 18, 9 to 16, or 10 to 14 nucleotides in length.

In an embodiment, the second complementarity domain is longer than the first complementarity domain, e.g., 2, 3, 4, 5, or 6, e.g., 6, nucleotides longer.

In an embodiment, the first and second complementary domains, independently, do not comprise modifications, e.g., modifications of the type provided in Section VIII.

In an embodiment, the first and second complementary domains, independently, comprise one or more modifications, e.g., modifications that the render the domain less susceptible to degradation or more bio-compatible, e.g., less immunogenic. By way of example, the backbone of the domain can be modified with a phosphorothioate, or other modification(s) from Section VIII. In an embodiment a nucleotide of the domain can comprise a 2' modification, e.g., a 2-acetylation, e.g., a 2' methylation, or other modification(s) from Section VIII.

In an embodiment, the first and second complementary domains, independently, include 1, 2, 3, 4, 5, 6, 7 or 8 or more modifications. In an embodiment, the first and second complementary domains, independently, include 1, 2, 3, or 4 modifications within 5 nucleotides of its 5' end. In an embodiment, the first and second complementary domains, independently, include as many as 1, 2, 3, or 4 modifications within 5 nucleotides of its 3' end.

In an embodiment, the first and second complementary domains, independently, include modifications at two consecutive nucleotides, e.g., two consecutive nucleotides that are within 5 nucleotides of the 5' end of the domain, within 5 nucleotides of the 3' end of the domain, or more than 5 nucleotides away from one or both ends of the domain. In an embodiment, the first and second complementary domains, independently, include no two consecutive nucleotides that are modified, within 5 nucleotides of the 5' end of the domain, within 5 nucleotides of the 3' end of the domain, or within a region that is more than 5 nucleotides away from one or both ends of the domain. In an embodiment, the first and second complementary domains, independently, include no nucleotide that is modified within 5 nucleotides of the 5' end of the domain, within 5 nucleotides of the 3' end of the domain, or within a region that is more than 5 nucleotides away from one or both ends of the domain.

Modifications in a complementarity domain can be selected to not interfere with targeting efficacy, which can be evaluated by testing a candidate modification in the system described in Section IV. gRNAs having a candidate complementarity domain having a selected length, sequence, degree of complementarity, or degree of modification, can be evaluated in the system described in Section IV. The candidate complementarity domain can be placed, either alone, or with one or more other candidate changes in a gRNA molecule/Cas molecule (e.g., gRNA molecule/Cas9 molecule) system known to be functional with a selected target and evaluated.

In an embodiment, the first complementarity domain has at least 60, 70, 80, 85%, 90% or 95% homology with, or differs by no more than 1, 2, 3, 4, 5, or 6 nucleotides from, a reference first complementarity domain, e.g., a naturally occurring, e.g., an *S. pyogenes, S. aureus, N. meningtidis,* or *S. thermophilus,* first complementarity domain, or a first complementarity domain described herein, e.g., from **Figs. 1A-1G****.**

In an embodiment, the second complementarity domain has at least 60, 70, 80, 85%, 90%, or 95% homology with, or differs by no more than 1, 2, 3, 4, 5, or 6 nucleotides from, a reference second complementarity domain, e.g., a naturally occurring, e.g., an *S. pyogenes, S. aureus, N. meningtidis,* or *S. thermophilus,* second complementarity domain, or a second complementarity domain described herein, e.g., from **Figs. 1A-1G****.**

The duplexed region formed by first and second complementarity domains is typically 6, 7, 8, 9, 10, 11, 12, 13, 14, 15, 16, 17, 18, 19, 20, 21 or 22 base pairs in length (excluding any looped out or unpaired nucleotides).

In some embodiments, the first and second complementarity domains, when duplexed, comprise 11 paired nucleotides, for example, in the gRNA sequence (one paired strand underlined, one bolded):

NNNNNNNNNNNNNNNNNNNNGUUUUAGAGCUAGAAAUAGCAAGUUAAAAUAAGG
CUAGUCCGUUAUCAACUUGAAAAAGUGGCACCGAGUCGGUGC (SEQ ID NO:5).

In some embodiments, the first and second complementarity domains, when duplexed, comprise 15 paired nucleotides, for example in the gRNA sequence (one paired strand underlined, one bolded):

NNNNNNNNNNNNNNNNNNNNGUUUUAGAGCUAUGCUGAAAAGCAUAGCAAGUUA
AAAUAAGGCUAGUCCGUUAUCAACUUGAAAAAGUGGCACCGAGUCGGUGC (SEQ
ID NO:27).

In some embodiments the first and second complementarity domains, when duplexed, comprise 16 paired nucleotides, for example in the gRNA sequence (one paired strand underlined, one bolded):

NNNNNNNNNNNNNNNNNNNNGUUUUAGAGCUAUGCUGGAAACAGCAUAGCAAGU
UAAAAUAAGGCUAGUCCGUUAUCAACUUGAAAAAGUGGCACCGAGUCGGUGC
(SEQ ID NO:28).

In some embodiments the first and second complementarity domains, when duplexed, comprise 21 paired nucleotides, for example in the gRNA sequence (one paired strand underlined, one bolded):

NNNNNNNNNNNNNNNNNNNNGUUUUAGAGCUAUGCUGUUUUGGAAACAAAACAG
CAUAGCAAGUUAAAAUAAGGCUAGUCCGUUAUCAACUUGAAAAAGUGGCACCGA
GUCGGUGC (SEQ ID NO:29).

In some embodiments, nucleotides are exchanged to remove poly-U tracts, for example in the gRNA sequences (exchanged nucleotides underlined):

NNNNNNNNNNNNNNNNNNNNGUAUUAGAGCUAGAAAUAGCAAGUUAAUAUAAGG
CUAGUCCGUUAUCAACUUGAAAAAGUGGCACCGAGUCGGUGC (SEQ ID NO:30);
NNNNNNNNNNNNNNNNNNNNGUUUAAGAGCUAGAAAUAGCAAGUUUAAAUAAGG
CUAGUCCGUUAUCAACUUGAAAAAGUGGCACCGAGUCGGUGC (SEQ ID NO:31); and
NNNNNNNNNNNNNNNNNNNNGUAUUAGAGCUAUGCUGUAUUGGAAACAAUACAG
CAUAGCAAGUUAAUAUAAGGCUAGUCCGUUAUCAACUUGAAAAAGUGGCACCGA
GUCGGUGC (SEQ ID NO:32).

### The 5' Extension Domain

In an embodiment, a modular gRNA can comprise additional sequence, 5' to the second complementarity domain. In an embodiment, the 5' extension domain is 2 to 10, 2 to 9, 2 to 8, 2 to 7, 2 to 6, 2 to 5, or 2 to 4 nucleotides in length. In an embodiment, the 5' extension domain is 2, 3, 4, 5, 6, 7, 8, 9, or 10 or more nucleotides in length.

In an embodiment, the 5' extension domain nucleotides do not comprise modifications, e.g., modifications of the type provided in Section VIII. However, in an embodiment, the 5' extension domain comprises one or more modifications, e.g., modifications that render it less susceptible to degradation or more bio-compatible, e.g., less immunogenic. By way of example, the backbone of the 5' extension domain can be modified with a phosphorothioate, or other modification(s) from Section VIII. In an embodiment, a nucleotide of the 5' extension domain can comprise a 2' modification, e.g., a 2-acetylation, e.g., a 2' methylation, or other modification(s) from Section VIII.

In some embodiments, the 5' extension domain can comprise as many as 1, 2, 3, 4, 5, 6, 7 or 8 modifications. In an embodiment, the 5' extension domain comprises as many as 1, 2, 3, or 4 modifications within 5 nucleotides of its 5' end, e.g., in a modular gRNA molecule. In an embodiment, the 5' extension domain comprises as many as 1, 2, 3, or 4 modifications within 5 nucleotides of its 3' end, e.g., in a modular gRNA molecule.

In some embodiments, the 5' extension domain comprises modifications at two consecutive nucleotides, e.g., two consecutive nucleotides that are within 5 nucleotides of the 5' end of the 5' extension domain, within 5 nucleotides of the 3' end of the 5' extension domain, or more than 5 nucleotides away from one or both ends of the 5' extension domain. In an embodiment, no two consecutive nucleotides are modified within 5 nucleotides of the 5' end of the 5' extension domain, within 5 nucleotides of the 3' end of the 5' extension domain, or within a region that is more than 5 nucleotides away from one or both ends of the 5' extension domain. In an embodiment, no nucleotide is modified within 5 nucleotides of the 5' end of the 5' extension domain, within 5 nucleotides of the 3' end of the 5' extension domain, or within a region that is more than 5 nucleotides away from one or both ends of the 5' extension domain.

Modifications in the 5' extension domain can be selected to not interfere with gRNA molecule efficacy, which can be evaluated by testing a candidate modification in the system described in Section IV. gRNAs having a candidate 5' extension domain having a selected length, sequence, degree of complementarity, or degree of modification, can be evaluated in the system described at Section IV. The candidate 5' extension domain can be placed, either alone, or with one or more other candidate changes in a gRNA molecule/Cas molecule (e.g., gRNA molecule/Cas9 molecule) system known to be functional with a selected target and evaluated.

In an embodiment, the 5' extension domain has at least 60, 70, 80, 85, 90, 95, 98 or 99% homology with, or differs by no more than 1, 2, 3, 4, 5, or 6 nucleotides from, a reference 5' extension domain, e.g., a naturally occurring, e.g., an *S. pyogenes, S. aureus, N. meningtidis,* or *S. thermophilus,* 5' extension domain, or a 5' extension domain described herein, e.g., from **Figs. 1A-1G****.**

### The Linking Domain

In a unimolecular gRNA molecule the linking domain is disposed between the first and second complementarity domains. In a modular gRNA molecule, the two molecules are associated with one another by the complementarity domains.

In an embodiment, the linking domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the linking domain is 20+/-10, 30+/-10, 40+/-10, 50+/-10, 60+/-10, 70+/-10, 80+/-10, 90+/-10, or 100+/-10 nucleotides, in length.

In an embodiment, the linking domain is 10 to 100, 10 to 90, 10 to 80, 10 to 70, 10 to 60, 10 to 50, 10 to 40, 10 to 30, 10 to 20 or 10 to 15 nucleotides in length. In other embodiments, the linking domain is 20 to 100, 20 to 90, 20 to 80, 20 to 70, 20 to 60, 20 to 50, 20 to 40, 20 to 30, or 20 to 25 nucleotides in length.

In an embodiment, the linking domain is 1, 2, 3, 4, 5, 6, 7, 8, 9, 10, 11, 12, 13, 14, 15, 16 17, 18, 19, or 20 nucleotides in length.

In an embodiment, the linking domain is a covalent bond.

In an embodiment, the linking domain comprises a duplexed region, typically adjacent to or within 1, 2, or 3 nucleotides of the 3' end of the first complementarity domain and/or the 5- end of the second complementarity domain. In an embodiment, the duplexed region can be 20+/-10 base pairs in length. In an embodiment, the duplexed region can be 10+/-5, 15+/-5, 20+/-5, or 30+/-5 base pairs in length. In an embodiment, the duplexed region can be 1, 2, 3, 4, 5, 6, 7, 8, 9, 10, 11, 12, 13, 14, or 15 base pairs in length.

Typically the sequences forming the duplexed region have exact complementarity with one another, though in some embodiments as many as 1, 2, 3, 4, 5, 6, 7 or 8 nucleotides are not complementary with the corresponding nucleotides.

In an embodiment, the linking domain nucleotides do not comprise modifications, e.g., modifications of the type provided in Section VIII. However, in an embodiment, the linking domain comprises one or more modifications, e.g., modifications that render it less susceptible to degradation or more bio-compatible, e.g., less immunogenic. By way of example, the backbone of the linking domain can be modified with a phosphorothioate, or other modification(s) from Section VIII. In an embodiment a nucleotide of the linking domain can comprise a 2' modification, e.g., a 2-acetylation, e.g., a 2' methylation, or other modification(s) from Section VIII. In some embodiments, the linking domain can comprise as many as 1, 2, 3, 4, 5, 6, 7 or 8 modifications.

Modifications in a linking domain can be selected to not interfere with targeting efficacy, which can be evaluated by testing a candidate modification in the system described in Section IV. gRNAs having a candidate linking domain having a selected length, sequence, degree of complementarity, or degree of modification, can be evaluated a system described in Section IV. A candidate linking domain can be placed, either alone, or with one or more other candidate changes in a gRNA molecule/Cas molecule (e.g., gRNA molecule/Cas9 molecule) system known to be functional with a selected target and evaluated.

In an embodiment, the linking domain has at least 60, 70, 80, 85, 90, 95, 98 or 99% homology with, or differs by no more than 1, 2, 3, 4, 5 ,or 6 nucleotides from, a reference linking domain, e.g., a linking domain described herein, e.g., from **Figs. 1A-1G****.**

### The Proximal Domain

In an embodiment, the proximal domain is 6 +/-2, 7+/-2, 8+/-2, 9+/-2, 10+/-2, 11+/-2, 12+/-2, 13+/-2, 14+/-2, 14+/-2, 16+/-2, 17+/-2, 18+/-2, 19+/-2, or 20+/-2 nucleotides in length.

In an embodiment, the proximal domain is 6, 7, 8, 9, 10, 11, 12, 13, 14, 14, 16, 17, 18, 19, or 20 nucleotides in length.

In an embodiment, the proximal domain is 5 to 20, 7, to 18, 9 to 16, or 10 to 14 nucleotides in length.

In an embodiment, the proximal domain nucleotides do not comprise modifications, e.g., modifications of the type provided in Section VIII. However, in an embodiment, the proximal domain comprises one or more modifications, e.g., modifications that render it less susceptible to degradation or more bio-compatible, e.g., less immunogenic. By way of example, the backbone of the proximal domain can be modified with a phosphorothioate, or other modification(s) from Section VIII. In an embodiment a nucleotide of the proximal domain can comprise a 2' modification, e.g., a 2-acetylation, e.g., a 2' methylation, or other modification(s) from Section VIII.

In some embodiments, the proximal domain can comprise as many as 1, 2, 3, 4, 5, 6, 7 or 8 modifications. In an embodiment, the proximal domain comprises as many as 1, 2, 3, or 4 modifications within 5 nucleotides of its 5' end, e.g., in a modular gRNA molecule. In an embodiment, the target domain comprises as many as 1, 2, 3, or 4 modifications within 5 nucleotides of its 3' end, e.g., in a modular gRNA molecule.

In some embodiments, the proximal domain comprises modifications at two consecutive nucleotides, e.g., two consecutive nucleotides that are within 5 nucleotides of the 5' end of the proximal domain, within 5 nucleotides of the 3' end of the proximal domain, or more than 5 nucleotides away from one or both ends of the proximal domain. In an embodiment, no two consecutive nucleotides are modified within 5 nucleotides of the 5' end of the proximal domain, within 5 nucleotides of the 3' end of the proximal domain, or within a region that is more than 5 nucleotides away from one or both ends of the proximal domain. In an embodiment, no nucleotide is modified within 5 nucleotides of the 5' end of the proximal domain, within 5 nucleotides of the 3' end of the proximal domain, or within a region that is more than 5 nucleotides away from one or both ends of the proximal domain.

Modifications in the proximal domain can be selected to not interfere with gRNA molecule efficacy, which can be evaluated by testing a candidate modification in the system described in Section IV. gRNAs having a candidate proximal domain having a selected length, sequence, degree of complementarity, or degree of modification, can be evaluated in the system described at Section IV. The candidate proximal domain can be placed, either alone, or with one or more other candidate changes in a gRNA molecule/Cas molecule (e.g., gRNA molecule/Cas9 molecule) system known to be functional with a selected target and evaluated.

In an embodiment, the proximal domain has at least 60, 70, 80, 85 90, 95, 98 or 99% homology with, or differs by no more than 1, 2, 3, 4, 5 ,or 6 nucleotides from, a reference proximal domain, e.g., a naturally occurring, e.g., an *S. pyogenes, S. aureus, N. meningtidis,* or *S*. *thermophilus,* proximal domain, or a proximal domain described herein, e.g., from **Figs. 1A-1G****.**

### The Tail Domain

In an embodiment, the tail domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the tail domain is 20+/-5 nucleotides in length.

In an embodiment, the tail domain is 20+/-10, 30+/-10, 40+/-10, 50+/-10, 60+/-10, 70+/-10, 80+/-10, 90+/-10, or 100+/-10 nucleotides, in length.

In an embodiment, the tail domain is 25+/-10 nucleotides in length.

In an embodiment, the tail domain is 10 to 100, 10 to 90, 10 to 80, 10 to 70, 10 to 60, 10 to 50, 10 to 40, 10 to 30, 10 to 20 or 10 to 15 nucleotides in length.

In other embodiments, the tail domain is 20 to 100, 20 to 90, 20 to 80, 20 to 70, 20 to 60, 20 to 50, 20 to 40, 20 to 30, or 20 to 25 nucleotides in length.

In an embodiment, the tail domain is 1 to 20, 1 to 1, 1 to 10, or 1 to 5 nucleotides in length.

In an embodiment, the tail domain nucleotides do not comprise modifications, e.g., modifications of the type provided in Section VIII. However, in an embodiment, the tail domain comprises one or more modifications, e.g., modifications that render it less susceptible to degradation or more bio-compatible, e.g., less immunogenic. By way of example, the backbone of the tail domain can be modified with a phosphorothioate, or other modification(s) from Section VIII. In an embodiment a nucleotide of the tail domain can comprise a 2' modification, e.g., a 2-acetylation, e.g., a 2' methylation, or other modification(s) from Section VIII.

In some embodiments, the tail domain can have as many as 1, 2, 3, 4, 5, 6, 7 or 8 modifications. In an embodiment, the target domain comprises as many as 1, 2, 3, or 4 modifications within 5 nucleotides of its 5' end. In an embodiment, the target domain comprises as many as 1, 2, 3, or 4 modifications within 5 nucleotides of its 3' end.

In an embodiment, the tail domain comprises a tail duplex domain, which can form a tail duplexed region. In an embodiment, the tail duplexed region can be 3, 4, 5, 6, 7, 8, 9, 10, 11, or 12 base pairs in length. In an embodiment, a further single stranded domain, exists 3' to the tail duplexed domain. In an embodiment, this domain is 3, 4, 5, 6, 7, 8, 9, or 10 nucleotides in length. In an embodiment it is 4 to 6 nucleotides in length.

In an embodiment, the tail domain has at least 60, 70, 80, 90, 95, 98 or 99% homology with, or differs by no more than 1, 2, 3, 4, 5 ,or 6 nucleotides from, a reference tail domain, e.g., a naturally occurring, e.g., an *S. pyogenes, S. aureus, N. meningtidis, or S. thermophilus,* tail domain, or a tail domain described herein, e.g., from **Figs. 1A-1G****.**

In an embodiment, the proximal and tail domain, taken together comprise the following sequences:
AAGGCUAGUCCGUUAUCAACUUGAAAAAGUGGCACCGAGUCGGUGCU (SEQ ID NO:33),
AAGGCUAGUCCGUUAUCAACUUGAAAAAGUGGCACCGAGUCGGUGGUGC (SEQ ID NO:34),
AAGGCUAGUCCGUUAUCAACUUGAAAAAGUGGCACCGAGUCGGUGCGGAUC (SEQ ID NO:35),
AAGGCUAGUCCGUUAUCAACUUGAAAAAGUG (SEQ ID NO:36),
AAGGCUAGUCCGUUAUCA (SEQ ID NO:37), or
AAGGCUAGUCCG (SEQ ID NO:38).

In an embodiment, the tail domain comprises the 3' sequence UUUUUU, e.g., if a U6 promoter is used for transcription.

In an embodiment, the tail domain comprises the 3' sequence UUUU, e.g., if an HI promoter is used for transcription.

In an embodiment, tail domain comprises variable numbers of 3' Us depending, e.g., on the termination signal of the pol-III promoter used.

In an embodiment, the tail domain comprises variable 3' sequence derived from the DNA template if a T7 promoter is used.

In an embodiment, the tail domain comprises variable 3' sequence derived from the DNA template, e.g., if *in vitro* transcription is used to generate the RNA molecule.

In an embodiment, the tail domain comprises variable 3' sequence derived from the DNA template, e.g., if a pol-II promoter is used to drive transcription.

Modifications in the tail domain can be selected to not interfere with targeting efficacy, which can be evaluated by testing a candidate modification in the system described in Section IV. gRNAs having a candidate tail domain having a selected length, sequence, degree of complementarity, or degree of modification, can be evaluated in the system described in Section IV. The candidate tail domain can be placed, either alone, or with one or more other candidate changes in a gRNA molecule/Cas molecule (e.g., gRNA molecule/Cas9 molecule) system known to be functional with a selected target and evaluated.

In some embodiments, the tail domain comprises modifications at two consecutive nucleotides, e.g., two consecutive nucleotides that are within 5 nucleotides of the 5' end of the tail domain, within 5 nucleotides of the 3' end of the tail domain, or more than 5 nucleotides away from one or both ends of the tail domain. In an embodiment, no two consecutive nucleotides are modified within 5 nucleotides of the 5' end of the tail domain, within 5 nucleotides of the 3' end of the tail domain, or within a region that is more than 5 nucleotides away from one or both ends of the tail domain. In an embodiment, no nucleotide is modified within 5 nucleotides of the 5' end of the tail domain, within 5 nucleotides of the 3' end of the tail domain, or within a region that is more than 5 nucleotides away from one or both ends of the tail domain.

In an embodiment a gRNA has the following structure:
5' [targeting domain]-[first complementarity domain]-[linking domain]-[second complementarity domain]-[proximal domain]-[tail domain]-3'
wherein, the targeting domain comprises a core domain and optionally a secondary domain, and is 10 to 50 nucleotides in length;
the first complementarity domain is 5 to 25 nucleotides in length and, In an embodiment has at least 50, 60, 70, 80, 85, 90, 95, 98 or 99% homology with a reference first complementarity domain disclosed herein;
the linking domain is 1 to 5 nucleotides in length;
the proximal domain is 5 to 20 nucleotides in length and, in an embodiment has at least 50, 60, 70, 80, 85, 90, 95, 98 or 99% homology with a reference proximal domain disclosed herein; and
the tail domain is absent or a nucleotide sequence is 1 to 50 nucleotides in length and, in an embodiment has at least 50, 60, 70, 80, 85, 90, 95, 98 or 99% homology with a reference tail domain disclosed herein.

### Exemplary Chimeric gRNAs

In an embodiment, a unimolecular, or chimeric, gRNA comprises, preferably from 5' to 3':
a targeting domain, e.g., comprising 15, 16, 17, 18, 19, 20, 21, 22, 23, 24, 25, or 26 nucleotides (which is complementary to a target nucleic acid);
a first complementarity domain;
a linking domain;
a second complementarity domain (which is complementary to the first complementarity domain);
a proximal domain; and
a tail domain,
wherein,
   (a) the proximal and tail domain, when taken together, comprise at least 15, 18, 20, 25, 30, 31, 35, 40, 45, 49, 50, or 53 nucleotides;
   (b) there are at least 15, 18, 20, 25, 30, 31, 35, 40, 45, 49, 50, or 53 nucleotides 3' to the last nucleotide of the second complementarity domain; or
   (c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the sequence from (a), (b), or (c), has at least 60, 75, 80, 85, 90, 95, or 99% homology with the corresponding sequence of a naturally occurring gRNA, or with a gRNA described herein.

In an embodiment, the proximal and tail domain, when taken together, comprise at least 15, 18, 20, 25, 30, 31, 35, 40, 45, 49, 50, or 53 nucleotides.

In an embodiment, there are at least 15, 18, 20, 25, 30, 31, 35, 40, 45, 49, 50, or 53 nucleotides 3' to the last nucleotide of the second complementarity domain.

In an embodiment, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 16, 17, 18, 19, 20, 21, 22, 23, 24, 25 or 26 nucleotides (e.g., 16, 17, 18, 19, 20, 21, 22, 23, 24, 25 or 26 consecutive nucleotides) having complementarity with the target domain, e.g., the targeting domain is 16, 17, 18, 19, 20, 21, 22, 23, 24, 25 or 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length.

In an embodiment, the targeting domain comprises, has, or consists of, 17 nucleotides (e.g., 17 consecutive nucleotides) having complementarity with the target domain, e.g., the targeting domain is 17 nucleotides in length.

In an embodiment, the targeting domain comprises, has, or consists of, 18 nucleotides (e.g., 18 consecutive nucleotides) having complementarity with the target domain, e.g., the targeting domain is 18 nucleotides in length.

In an embodiment, the targeting domain comprises, has, or consists of, 19 nucleotides (e.g., 19 consecutive nucleotides) having complementarity with the target domain, e.g., the targeting domain is 19 nucleotides in length.

In an embodiment, the targeting domain comprises, has, or consists of, 20 nucleotides (e.g., 20 consecutive nucleotides) having complementarity with the target domain, e.g., the targeting domain is 20 nucleotides in length.

In an embodiment, the targeting domain comprises, has, or consists of, 21 nucleotides (e.g., 21 consecutive nucleotides) having complementarity with the target domain, e.g., the targeting domain is 21 nucleotides in length.

In an embodiment, the targeting domain comprises, has, or consists of, 22 nucleotides (e.g., 22 consecutive nucleotides) having complementarity with the target domain, e.g., the targeting domain is 22 nucleotides in length.

In an embodiment, the targeting domain comprises, has, or consists of, 23 nucleotides (e.g., 23 consecutive nucleotides) having complementarity with the target domain, e.g., the targeting domain is 23 nucleotides in length.

In an embodiment, the targeting domain comprises, has, or consists of, 24 nucleotides (e.g., 24 consecutive nucleotides) having complementarity with the target domain, e.g., the targeting domain is 24 nucleotides in length.

In an embodiment, the targeting domain comprises, has, or consists of, 25 nucleotides (e.g., 25 consecutive nucleotides) having complementarity with the target domain, e.g., the targeting domain is 25 nucleotides in length.

In an embodiment, the targeting domain comprises, has, or consists of, 26 nucleotides (e.g., 26 consecutive nucleotides) having complementarity with the target domain, e.g., the targeting domain is 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the unimolecular, or chimeric, gRNA molecule (comprising a targeting domain, a first complementary domain, a linking domain, a second complementary domain, a proximal domain and, optionally, a tail domain) comprises the following sequence in which the targeting domain is depicted as 20 Ns but could be any sequence and range in length from 16 to 26 nucleotides and in which the gRNA sequence is followed by 6 Us, which serve as a termination signal for the U6 promoter, but which could be either absent or fewer in number:
NNNNNNNNNNNNNNNNNNNNGUUUUAGAGCUAGAAAUAGCAAGUUAAAAU AAGGCUAGUCCGUUAUCAACUUGAAAAAGUGGCACCGAGUCGGUGCUUUUUU (SEQ ID NO:40). In an embodiment, the unimolecular, or chimeric, gRNA molecule is a *S*. *pyogenes* gRNA molecule.

In some embodiments, the unimolecular, or chimeric, gRNA molecule (comprising a targeting domain, a first complementary domain, a linking domain, a second complementary domain, a proximal domain and, optionally, a tail domain) comprises the following sequence in which the targeting domain is depicted as 20 Ns but could be any sequence and range in length from 16 to 26 nucleotides and in which the gRNA sequence is followed by 6 Us, which serve as a termination signal for the U6 promoter, but which could be either absent or fewer in number:
NNNNNNNNNNNNNNNNNNNNGUUUUAGUACUCUGGAAACAGAAUCUACUA AAACAAGGCAAAAUGCCGUGUUUAUCUCGUCAACUUGUUGGCGAGAUUUUUU (SEQ ID NO:41). In an embodiment, the unimolecular, or chimeric, gRNA molecule is a *S*. *aureus* gRNA molecule.

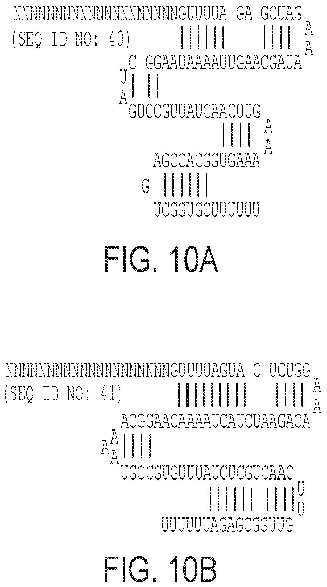

The sequences and structures of exemplary chimeric gRNAs are also shown in **Figs. 10A-10B**

### Exemplary Modular gRNAs

In an embodiment, a modular gRNA comprises:
a first strand comprising, preferably from 5' to 3';
   a targeting domain, e.g., comprising 16, 17, 18, 19, 20, 21, 22, 23, 24, 25, or 26 nucleotides;
   a first complementarity domain; and
   a second strand, comprising, preferably from 5' to 3':
      optionally a 5' extension domain;
      a second complementarity domain;
      a proximal domain; and
      a tail domain,
wherein:
   (a) the proximal and tail domain, when taken together, comprise at least 15, 18, 20, 25, 30, 31, 35, 40, 45, 49, 50, or 53 nucleotides;
   (b) there are at least 15, 18, 20, 25, 30, 31, 35, 40, 45, 49, 50, or 53 nucleotides 3' to the last nucleotide of the second complementarity domain; or
   (c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the sequence from (a), (b), or (c), has at least 60, 75, 80, 85, 90, 95, or 99% homology with the corresponding sequence of a naturally occurring gRNA, or with a gRNA described herein.

In an embodiment, the proximal and tail domain, when taken together, comprise at least 15, 18, 20, 25, 30, 31, 35, 40, 45, 49, 50, or 53 nucleotides.

In an embodiment there are at least 15, 18, 20, 25, 30, 31, 35, 40, 45, 49, 50, or 53 nucleotides 3' to the last nucleotide of the second complementarity domain.

In an embodiment, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain has, or consists of, 16, 17, 18, 19, 20, 21, 22, 23, 24, 25 or 26 nucleotides (e.g., 16, 17, 18, 19, 20, 21, 22, 23, 24, 25 or 26 consecutive nucleotides) having complementarity with the target domain, e.g., the targeting domain is 16, 17, 18, 19, 20, 21, 22, 23, 24, 25 or 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length.

In an embodiment, the targeting domain comprises, has, or consists of, 17 nucleotides (e.g., 17 consecutive nucleotides) having complementarity with the target domain, e.g., the targeting domain is 17 nucleotides in length.

In an embodiment, the targeting domain comprises, has, or consists of, 18 nucleotides (e.g., 18 consecutive nucleotides) having complementarity with the target domain, e.g., the targeting domain is 18 nucleotides in length.

In an embodiment, the targeting domain comprises, has, or consists of, 19 nucleotides (e.g., 19 consecutive nucleotides) having complementarity with the target domain, e.g., the targeting domain is 19 nucleotides in length.

In an embodiment, the targeting domain comprises, has, or consists of, 20 nucleotides (e.g., 20 consecutive nucleotides) having complementarity with the target domain, e.g., the targeting domain is 20 nucleotides in length.

In an embodiment, the targeting domain comprises, has, or consists of, 21 nucleotides (e.g., 21 consecutive nucleotides) having complementarity with the target domain, e.g., the targeting domain is 21 nucleotides in length.

In an embodiment, the targeting domain comprises, has, or consists of, 22 nucleotides (e.g., 22 consecutive nucleotides) having complementarity with the target domain, e.g., the targeting domain is 22 nucleotides in length.

In an embodiment, the targeting domain comprises, has, or consists of, 23 nucleotides (e.g., 23 consecutive nucleotides) having complementarity with the target domain, e.g., the targeting domain is 23 nucleotides in length.

In an embodiment, the targeting domain comprises, has, or consists of, 24 nucleotides (e.g., 24 consecutive nucleotides) having complementarity with the target domain, e.g., the targeting domain is 24 nucleotides in length.

In an embodiment, the targeting domain comprises, has, or consists of, 25 nucleotides (e.g., 25 consecutive nucleotides) having complementarity with the target domain, e.g., the targeting domain is 25 nucleotides in length.

In an embodiment, the targeting domain comprises, has, or consists of, 26 nucleotides (e.g., 26 consecutive nucleotides) having complementarity with the target domain, e.g., the targeting domain is 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

### Exemplary Modified gRNAs

As discussed above and in the Examples, we have found that the guide RNA (gRNA) component of the CRISPR/Cas system (e.g., a CRISPR/Cas9 system) is more efficient at editing genes in certain circulatory cell types (e.g., T cells) *ex vivo* when it has been modified at or near its 5' end (e.g., when the 5' end of a gRNA is modified by the inclusion of a eukaryotic mRNA cap structure or cap analog). While not wishing to be bound by theory it is believed that these and other modified gRNAs described herein exhibit enhanced stability with certain cell types (e.g., circulating cells such as T cells) and that this might be responsible for the observed improvements.

The present invention encompasses the realization that the improvements observed with a gRNA with 3' polyA tails and/or 5' caps can be extended to gRNAs that have been modified in other ways to achieve the same type of structural or functional result (e.g., by the inclusion of modified nucleosides or nucleotides, or when an *in vitro* transcribed gRNA is modified by treatment with a phosphatase such as calf intestinal alkaline phosphatase to remove the 5' triphosphate group). While not wishing to be bound by theory, in some embodiments, the modified gRNAs described herein may contain one or more modifications (e.g., modified nucleosides or nucleotides) which introduce stability toward nucleases (e.g., by the inclusion of modified nucleosides or nucleotides).

Thus, in one aspect, methods and compositions discussed herein provide methods and ) compositions for gene editing of certain cells (e.g., *ex vivo* gene editing) by using gRNAs which have been modified at or near their 5' end (e.g., within 1-10, 1-5, or 1-2 nucleotides of their 5' end).

In some embodiments, the 5' end of the gRNA molecule lacks a 5' triphosphate group. In some embodiments, the 5' end of the targeting domain lacks a 5' triphosphate group. In some embodiments, the 5' end of the gRNA molecule includes a 5' cap. In some embodiments, the 5' end of the targeting domain includes a 5' cap. In some embodiments, the gRNA molecule lacks a 5' triphosphate group. In some embodiments, the gRNA molecule comprises a targeting domain and the 5' end of the targeting domain lacks a 5' triphosphate group. In some embodiments, gRNA molecule includes a 5' cap. In some embodiments, the gRNA molecule comprises a targeting ) domain and the 5' end of the targeting domain includes a 5' cap.

In an embodiment, the 5' end of a gRNA is modified by the inclusion of a eukaryotic mRNA cap structure or cap analog (e.g., without limitation a *G(5')ppp(5')G* cap analog, a *m7G(5')ppp(5')G* cap analog, or a 3*'-O-Me-m7G(5')ppp(5')G* anti reverse cap analog (ARCA)). In certain embodiments the 5' cap comprises a modified guanine nucleotide that is linked to the remainder of the gRNA molecule via a 5'-5' triphosphate linkage. In some embodiments, the 5' cap comprises two optionally modified guanine nucleotides that are linked via a 5'-5' triphosphate linkage. In some embodiments, the 5' end of the gRNA molecule has the chemical formula: 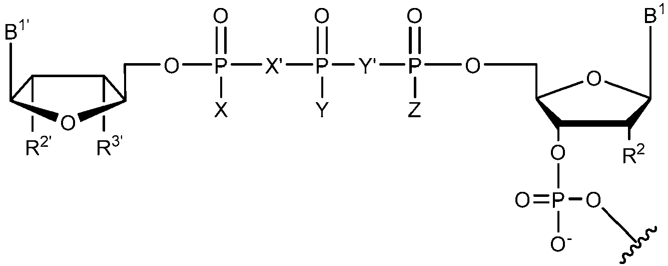 wherein:
each of B¹ and B^{1'} is independently 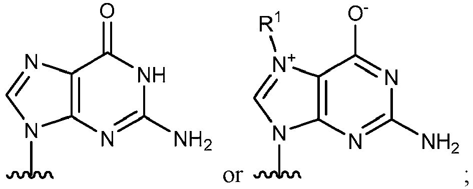
each R¹ is independently C₁₋₄ alkyl, optionally substituted by a phenyl or a 6-membered heteroaryl;
each of R², R^{2'}, and R^{3'} is independently H, F, OH, or O-C₁₋₄ alkyl;
each of X, Y, and Z is independently O or S; and
each of X' and Y' is independently O or CH₂.

In an embodiment, each R¹ is independently -CH₃, -CH₂CH₃, or -CH₂C₆H₅. In an embodiment, R¹ is -CH₃.

In an embodiment, B^{1'} is

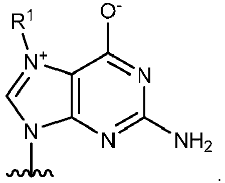

In an embodiment, each of R², R^{2'}, and R^{3'} is independently H, OH, or O-CH₃.

In an embodiment, each of X, Y, and Z is O.

In an embodiment, X' and Y' are O.

In an embodiment, the 5' end of the gRNA molecule has the chemical formula:

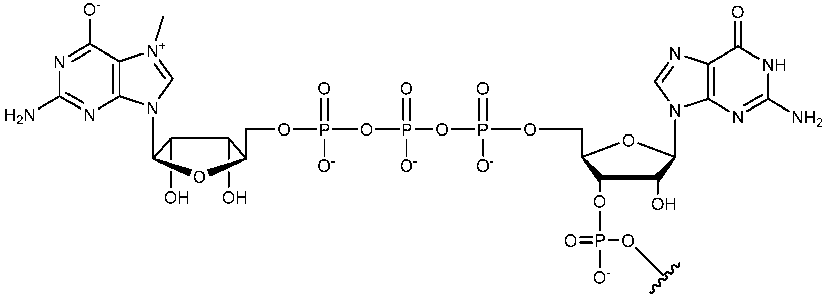

In an embodiment, the 5' end of the gRNA molecule has the chemical formula:

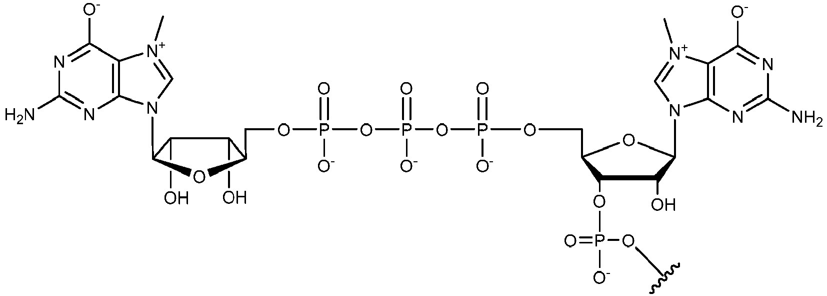

In an embodiment, the 5' end of the gRNA molecule has the chemical formula:

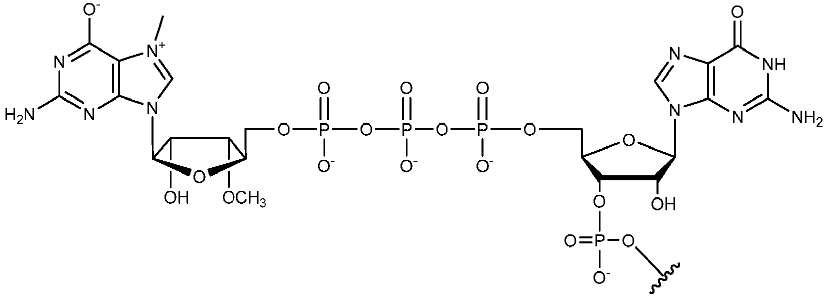

In an embodiment, the 5' end of the gRNA molecule has the chemical formula:

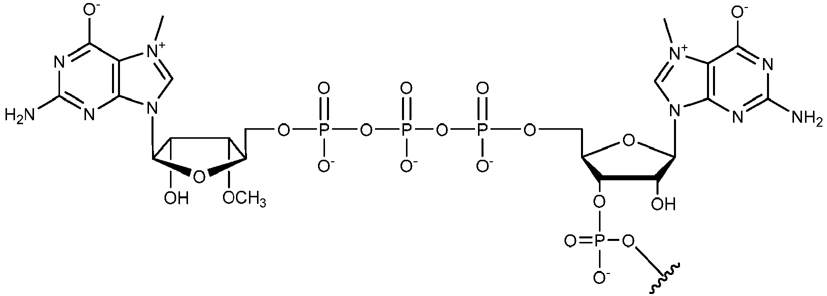

In an embodiment, X is S, and Y and Z are O.

In an embodiment, Y is S, and X and Z are O.

In an embodiment, Z is S, and X and Y are O.

In an embodiment, the phosphorothioate is the Sp diastereomer.

In an embodiment, X' is CH₂, and Y' is O.

In an embodiment, X' is O, and Y' is CH₂.

In an embodiment, the 5' cap comprises two optionally modified guanine nucleotides that are linked via an optionally modified 5'-5' tetraphosphate linkage.

In an embodiment, the 5' end of the gRNA molecule has the chemical formula: 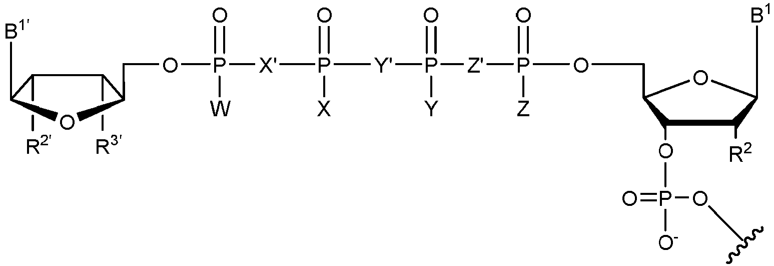 wherein:
each of B¹ and B^{1'} is independently 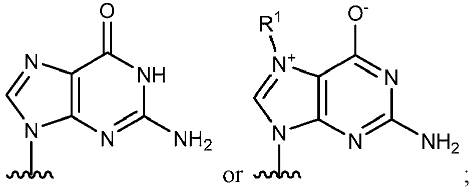
each R¹ is independently C₁₋₄ alkyl, optionally substituted by a phenyl or a 6-membered heteroaryl;
each of R², R^{2'}, and R^{3'} is independently H, F, OH, or O-C₁₋₄ alkyl;
each of W, X, Y, and Z is independently O or S; and
each of X', Y', and Z' is independently O or CH₂.

In an embodiment, each R¹ is independently -CH₃, -CH₂CH₃, or -CH₂C₆H₅.

In an embodiment, R¹ is -CH₃.

In an embodiment, B^{1'} is

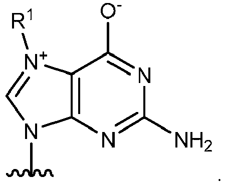

In an embodiment, each of R², R^{2'}, and R^{3'} is independently H, OH, or O-CH₃.

In an embodiment, each of W, X, Y, and Z is O.

In an embodiment, each of X', Y', and Z' are O.

In an embodiment, X' is CH₂, and Y' and Z' are O.

In an embodiment, Y' is CH₂, and X' and Z' are O.

In an embodiment, Z' is CH₂, and X' and Y' are O.

In an embodiment, the 5' cap comprises two optionally modified guanine nucleotides that are linked via an optionally modified 5'-5' pentaphosphate linkage.

In an embodiment, the 5' end of the gRNA molecule has the chemical formula: 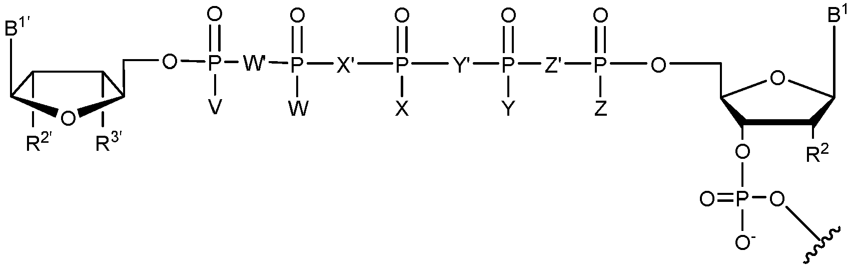 wherein:
each of B¹ and B^{1'} is independently 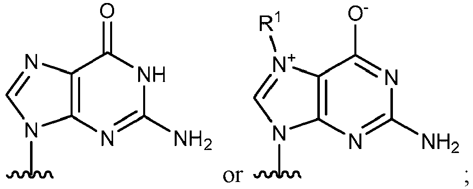
each R¹ is independently C₁₋₄ alkyl, optionally substituted by a phenyl or a 6-membered heteroaryl;
each of R², R^{2'}, and R^{3'} is independently H, F, OH, or O-C₁₋₄ alkyl;
each of V, W, X, Y, and Z is independently O or S; and
each of W', X', Y', and Z' is independently O or CH₂.

In an embodiment, each R¹ is independently -CH₃, -CH₂CH₃, or -CH₂C₆H₅.

In an embodiment, R¹ is -CH₃.

In an embodiment, B^{1'} is

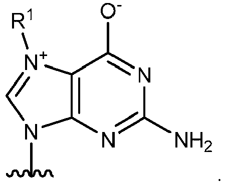

In an embodiment, each of R², R^{2'}, and R^{3'} is independently H, OH, or O-CH₃.

In an embodiment, each of V, W, X, Y, and Z is O.

In an embodiment, each of W', X', Y', and Z' is O.

It is to be understood that as used herein, the term "5' cap" encompasses traditional mRNA 5' cap structures but also analogs of these. For example, in addition to the 5' cap structures that are encompassed by the chemical structures shown above, one may use, e.g., tetraphosphate analogs having a methylene-bis(phosphonate) moiety (e.g., see Rydzik, A M et al., (2009) Org Biomol Chem 7(22):4763-76), analogs having a sulfur substitution for a non-bridging oxygen (e.g., see Grudzien-Nogalska, E. et al, (2007) RNA 13(10): 1745-1755), N7-benzylated dinucleoside tetraphosphate analogs (e.g., see Grudzien, E. et al., (2004) RNA 10(9): 1479-1487), or anti-reverse cap analogs (e.g., see US Patent No. 7,074,596 and Jemielity, J. et al., (2003) RNA 9(9): 1 108-1 122 and Stepinski, J. et al., (2001) RNA 7(10):1486-1495). The present application also encompasses the use of cap analogs with halogen groups instead of OH or OMe (e.g., see US Patent No. 8,304,529); cap analogs with at least one phosphorothioate (PS) linkage (e.g., see US Patent No. 8,153,773 and Kowalska, J. et al., (2008) RNA 14(6): 1 1 19-1131); and cap analogs with at least one boranophosphate or phosphoroselenoate linkage (e.g., see US Patent No. 8,519,110); and alkynyl-derivatized 5' cap analogs (e.g., see US Patent No. 8,969,545).

In general, the 5' cap can be included during either chemical synthesis or *in vitro* transcription of the gRNA. In an embodiment, a 5' cap is not used and the gRNA (e.g., an *in vitro* transcribed gRNA) is instead modified by treatment with a phosphatase (e.g., calf intestinal alkaline phosphatase) to remove the 5' triphosphate group.

Methods and compositions discussed herein also provide methods and compositions for gene editing by using gRNAs which comprise a 3' polyA tail (also called a polyA tract herein). Such gRNAs may, for example, be prepared by adding a polyA tail to a gRNA molecule precursor using a polyadenosine polymerase following *in vitro* transcription of the gRNA molecule precursor. For example, in one embodiment, a polyA tail may be added enzymatically using a polymerase such as *E. coli* polyA polymerase (E-PAP). gRNAs including a polyA tail may also be prepared by *in vitro* transcription from a DNA template. In one embodiment, a polyA tail of defined length is encoded on a DNA template and transcribed with the gRNA via an RNA polymerase (such as T7 RNA polymerase). gRNAs with a polyA tail may also be prepared by ligating a polyA oligonucleotide to a gRNA molecule precursor following *in vitro* transcription using an RNA ligase or a DNA ligase with or without a splinted DNA oligonucleotide complementary to the gRNA molecule precursor and the polyA oligonucleotide. For example, in one embodiment, a polyA tail of defined length is synthesized as a synthetic oligonucleotide and ligated on the 3' end of the gRNA with either an RNA ligase or a DNA ligase with or without a splinted DNA oligonucleotide complementary to the guide RNA and the polyA oligonucleotide. gRNAs including the polyA tail may also be prepared synthetically, in one or several pieces that are ligated together by either an RNA ligase or a DNA ligase with or without one or more splinted DNA oligonucleotides.

In some embodiments, the polyA tail is comprised of fewer than 50 adenine nucleotides, for example, fewer than 45 adenine nucleotides, fewer than 40 adenine nucleotides, fewer than 35 adenine nucleotides, fewer than 30 adenine nucleotides, fewer than 25 adenine nucleotides or fewer than 20 adenine nucleotides. In some embodiments the polyA tail is comprised of between 5 and 50 adenine nucleotides, for example between 5 and 40 adenine nucleotides, between 5 and 30 adenine nucleotides, between 10 and 50 adenine nucleotides, or between 15 and 25 adenine nucleotides. In some embodiments, the polyA tail is comprised of about 20 adenine nucleotides.

Methods and compositions discussed herein also provide methods and compositions for gene editing (e.g., *ex vivo* gene editing) by using gRNAs which include one or more modified nucleosides or nucleotides that are described herein.

While some of the exemplary modifications discussed in this section may be included at any position within the gRNA sequence, in some embodiments, a gRNA comprises a modification at or near its 5' end (e.g., within 1-10, 1-5, or 1-2 nucleotides of its 5' end). In some embodiments, a gRNA comprises a modification at or near its 3' end (e.g., within 1-10, 1-5, or 1-2 nucleotides of its 3' end). In some embodiments, a gRNA comprises both a modification at or near its 5' end and a modification at or near its 3' end. For example, in some embodiments, a gRNA molecule (e.g., an *in vitro* transcribed gRNA) comprises a targeting domain which is complementary with a target domain from a gene expressed in a eukaryotic cell, wherein the gRNA molecule is modified at its 5' end and comprises a 3' polyA tail. The gRNA molecule may, for example, lack a 5' triphosphate group (e.g., the 5' end of the targeting domain lacks a 5' triphosphate group). In an embodiment, a gRNA (e.g., an *in vitro* transcribed gRNA) is modified by treatment with a phosphatase (e.g., calf intestinal alkaline phosphatase) to remove the 5' triphosphate group and comprises a 3' polyA tail as described herein. The gRNA molecule may alternatively include a 5' cap (e.g., the 5' end of the targeting domain includes a 5' cap). In an embodiment, a gRNA (e.g., an *in vitro* transcribed gRNA) contains both a 5' cap structure or cap analog and a 3' polyA tail as described herein. In some embodiments, the 5' cap comprises a modified guanine nucleotide that is linked to the remainder of the gRNA molecule via a 5'-5' triphosphate linkage. In some embodiments, the 5' cap comprises two optionally modified guanine nucleotides that are linked via an optionally modified 5'-5' triphosphate linkage (e.g., as described above). In some embodiments the polyA tail is comprised of between 5 and 50 adenine nucleotides, for example between 5 and 40 adenine nucleotides, between 5 and 30 adenine nucleotides, between 10 and 50 adenine nucleotides, between 15 and 25 adenine nucleotides, fewer than 30 adenine nucleotides, fewer than 25 adenine nucleotides or about 20 adenine nucleotides.

In yet other embodiments, the present invention provides a gRNA molecule comprising a targeting domain which is complementary with a target domain from a gene expressed in a eukaryotic cell, wherein the gRNA molecule comprises a 3' polyA tail which is comprised of fewer than 30 adenine nucleotides (e.g., fewer than 25 adenine nucleotides, between 15 and 25 adenine nucleotides, or about 20 adenine nucleotides), and is complexed with a Cas9 molecule. In some embodiments, these gRNA molecules are further modified at their 5' end (e.g., the gRNA molecule is modified by treatment with a phosphatase to remove the 5' triphosphate group or modified to include a 5' cap as described herein).

In some embodiments, gRNAs can be modified at a 3' terminal U ribose. In some embodiments, the 5' end and a 3' terminal U ribose of the gRNA are modified (e.g., the gRNA is modified by treatment with a phosphatase to remove the 5' triphosphate group or modified to include a 5' cap as described herein). For example, the two terminal hydroxyl groups of the U ribose can be oxidized to aldehyde groups and a concomitant opening of the ribose ring to afford a modified nucleoside as shown below: 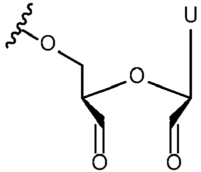 wherein "U" can be an unmodified or modified uridine.

In another embodiment, the 3' terminal U can be modified with a 2'3' cyclic phosphate as shown below: 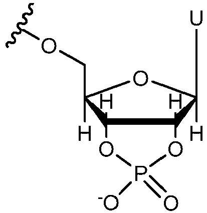 wherein "U" can be an unmodified or modified uridine.

In some embodiments, the gRNA molecules may contain 3' nucleotides which can be stabilized against degradation, e.g., by incorporating one or more of the modified nucleotides described herein. In this embodiment, e.g., uridines can be replaced with modified uridines, e.g., 5-(2-amino)propyl uridine, and 5-bromo uridine, or with any of the modified uridines described herein; adenosines, cytidines and guanosines can be replaced with modified adenosines, cytidines and guanosines, e.g., with modifications at the 8-position, e.g., 8-bromo guanosine, or with any of the modified adenosines, cytidines or guanosines described herein.

In some embodiments, sugar-modified ribonucleotides can be incorporated into the gRNA, e.g., wherein the 2' OH-group is replaced by a group selected from H, -OR, -R (wherein R can be, e.g., alkyl, cycloalkyl, aryl, aralkyl, heteroaryl or sugar), halo, -SH, -SR (wherein R can be, e.g., alkyl, cycloalkyl, aryl, aralkyl, heteroaryl or sugar), amino (wherein amino can be, e.g., NH₂; alkylamino, dialkylamino, heterocyclylamino, arylamino, diarylamino, heteroarylamino, diheteroarylamino, or amino acid); or cyano (-CN). In some embodiments, the phosphate backbone can be modified as described herein, e.g., with a phosphothioate group. In some embodiments, one or more of the nucleotides of the gRNA can each independently be a modified or unmodified nucleotide including, but not limited to 2'-sugar modified, such as, 2'-O-methyl, 2'-O-methoxyethyl, or 2'-Fluoro modified including, e.g., 2'-F or 2'-O-methyl, adenosine (A), 2'-F or 2'-O-methyl, cytidine (C), 2'-F or 2'-O-methyl, uridine (U), 2'-F or 2'-O-methyl, thymidine (T), 2'-F or 2'-O-methyl, guanosine (G), 2'-O-methoxyethyl-5-methyluridine (Teo), 2'-O-methoxyethyladenosine (Aeo), 2'-O-methoxyethyl-5-methylcytidine (m5Ceo), and any combinations thereof.

In some embodiments, a gRNA can include "locked" nucleic acids (LNA) in which the 2' OH-group can be connected, e.g., by a C1-6 alkylene or C1-6 heteroalkylene bridge, to the 4' carbon of the same ribose sugar, where exemplary bridges can include methylene, propylene, ether, or amino bridges; O-amino (wherein amino can be, e.g., NH₂; alkylamino, dialkylamino, heterocyclylamino, arylamino, diarylamino, heteroarylamino, or diheteroarylamino, ethylenediamine, or polyamino) and aminoalkoxy or O(CH₂)ₙ-amino (wherein amino can be, e.g., NH₂; alkylamino, dialkylamino, heterocyclylamino, arylamino, diarylamino, heteroarylamino, or diheteroarylamino, ethylenediamine, or polyamino).

In some embodiments, a gRNA can include a modified nucleotide which is multicyclic (e.g., tricyclo; and "unlocked" forms, such as glycol nucleic acid (GNA) (e.g., R-GNA or S-GNA, where ribose is replaced by glycol units attached to phosphodiester bonds), or threose nucleic acid (TNA, where ribose is replaced with α-L-threofuranosyl-(3'→2')).

Generally, gRNA molecules include the sugar group ribose, which is a 5-membered ring having an oxygen. Exemplary modified gRNAs can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). Although the majority of sugar analog alterations are localized to the 2' position, other sites are amenable to modification, including the 4' position. In an embodiment, a gRNA comprises a 4'-S, 4'-Se or a 4'-C-aminomethyl-2'-O-Me modification.

In some embodiments, deaza nucleotides, e.g., 7-deaza-adenosine, can be incorporated into the gRNA. In some embodiments, O- and N-alkylated nucleotides, e.g., N6-methyl adenosine, can be incorporated into the gRNA. In some embodiments, one or more or all of the nucleotides in a gRNA molecule are deoxynucleotides.

### II. Methods for Designing gRNAs

Methods for designing gRNAs are described herein, including methods for selecting, designing and validating targeting domains. Methods for selection and validation of target sequences as well as off-target analyses are described, e.g., in Mali et al., 2013 SCIENCE 339(6121): 823-826; Hsu et al. NAT BIOTECHNOL, 31(9): 827-32; Fu et al., 2014 NAT BIOTECHNOL, doi: 10.1038/nbt.2808. PubMed PMID: 24463574; Heigwer et al., 2014 NAT METHODS 11(2):122-3. doi: 10.1038/nmeth.2812. PubMed PMID: 24481216; Bae et al., 2014 BIOINFORMATICS PubMed PMID: 24463181; Xiao A et al., 2014 BIOINFORMATICS PubMed PMID: 24389662.

In some embodiments, a software tool can be used to optimize the choice of gRNA within a target sequence, e.g., to minimize total off-target activity across the genome. Off target activity may be other than cleavage. For example, for each possible gRNA choice using *S. pyogenes* Cas9, software tools can identify all potential off-target sequences (preceding either NAG or NGG PAMs) across the genome that contain up to a certain number (e.g., 1, 2, 3, 4, 5, 6, 7, 8, 9, or 10) of mismatched base-pairs. The cleavage efficiency at each off-target sequence can be predicted, e.g., using an experimentally-derived weighting scheme. Each possible gRNA can then be ranked according to its total predicted off-target cleavage; the top-ranked gRNAs represent those that are likely to have the greatest on-target and the least off-target cleavage. Other functions, e.g., automated reagent design for gRNA vector construction, primer design for the on-target Surveyor assay, and primer design for high-throughput detection and quantification of off-target cleavage via next-generation sequencing, can also be included in the tool. Candidate gRNA molecules can be evaluated by art-known methods or as described in Section IV herein.

In some embodiments, gRNAs for use with *S. pyogenes, S. aureus,* and *N. meningitidis* Cas9s are identified using a DNA sequence searching algorithm, e.g., using a custom gRNA design software based on the public tool cas-offinder (Bae et al. Bioinformatics. 2014; 30(10): 1473-1475). Said custom gRNA design software scores guides after calculating their genomewide off-target propensity. Typically matches ranging from perfect matches to 7 mismatches are considered for guides ranging in length from 17 to 24. Once the off-target sites are computationally determined, an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene are obtained from the UCSC Genome browser and sequences are screened for repeat elements using the publicly available RepeatMasker program. RepeatMasker searches input DNA sequences for repeated elements and regions of low complexity. The output is a detailed annotation of the repeats present in a given query sequence.

Following identification, gRNAs are ranked into tiers based on on one or more of their distance to the target site, their orthogonality and presence of a 5' G (based on identification of close matches in the human genome containing a relavant PAM, e.g., in the case *of S. pyogenes,* a NGG PAM, in the case of *S*. *aureus,* NNGRR (e.g, a NNGRRT (R = A or G) or NNGRRV) PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer targeting domains that have no identical sequences in the human genome besides the intended target, nor any sequences that contain one or two mismatches in the target sequence. Targeting domains with good orthogonality are selected to minimize off-target DNA cleavage. It is to be understood that this is a non-limiting example and that a variety of strategies could be utilized to identify gRNAs for use with *S. pyogenes, S. aureus* and *N. meningitidis* or other Cas9 enzymes.

### First Strategy for Designing and Tiering gRNAs

In a first strategy, gRNAs for use with the *S. pyogenes* Cas9 are identified using the publicly available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequences for each gene are obtained from the UCSC Genome browser and sequences are screened for repeat elements using the publicly available RepeatMasker program. RepeatMasker searches input DNA sequences for repeated elements and regions of low complexity. The output is a detailed annotation of the repeats present in a given query sequence. Following identification, gRNAs for use with a *S*. *pyogenes* Cas9 are ranked into 5 tiers.

The targeting domains for gRNA molecules are selected based on one or more of their distance to the target site, their orthogonality and presence of a 5' G (based on the ZiFiT identification of close matches in the human genome containing an NGG PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome nor any sequences that contain one or two mismatches in the target sequence. Targeting domains with good orthogonality are selected to miminize off-target DNA cleavage. As an example, for all targets, gRNAs with 16, 17, 18, 19, 20, 21, 22, 23, 24, 25, and/or 26 mer target domains are designed. gRNAs are also selected both for single-gRNA nuclease cutting and for the dual gRNA nickase strategy. Criteria for selecting gRNAs and the determination for which gRNAs can be used for which strategy is based on several considerations:
gRNAs are identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs canalso often result in indel mutations at the site of only one of the gRNAs. Candidate pair members can be tested for how efficiently they remove the entire sequence versus just causing indel mutations at the site of one gRNA.

The targeting domains for first tier gRNA molecules are selected based on (1) a reasonable distance to the target position, e.g., within the first 500bp of coding sequence downstream of start codon, (2) a high level of orthogonality, and (3) the presence of a 5' G. For selection of second tier gRNAs, the requirement for a 5'G is removed, but the distance restriction is required and a high level of orthogonality is required. Third tier selection uses the same distance restriction and the requirement for a 5'G, but removes the requirement of good orthogonality. Fourth tier selection uses the same distance restriction but removes the requirement of good orthogonality and start with a 5'G. Fifth tier selection removes the requirement of good orthogonality and a 5'G, and a longer sequence (e.g., the rest of the coding sequence, e.g., additional 500 bp upstream or downstream to the transcription target site) is scanned. Note that tiers are non-inclusive (each gRNA is listed only once).

### Second Strategy for Designing and Tiering gRNAs

In a second strategy, gRNAs for use with the *S. pyogenes, S. aureus* and *N. meningitidis* Cas9 are identified. The second strategy differs from the first strategy as follows.

Guide RNAs (gRNAs) for use with *S. pyogenes, S. aureus and N meningitidis* Cas9s are identified using a DNA sequence searching algorithm. Guide RNA design is carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID:24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined, an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene is obtained from a database (e.g., the UCSC Genome browser) and sequences are screened for repeat elements using the publically available RepeatMasker program. RepeatMasker searches input DNA sequences for repeated elements and regions of low complexity. The output is a detailed annotation of the repeats present in a given query sequence.

Following identification, gRNAs are ranked into tiers based on their distance to the target site, their orthogonality or presence of a 5' G (based on identification of close matches in the human genome containing a relavant PAM, e.g., in the case of *S. pyogenes,* a NGG PAM, in the case of *S. aureus,* NNGRR (e.g, a NNGRRT (R = A or G) or NNGRRV) PAM, and in the case of *N. meningitides,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

gRNAs may be identified for both single-gRNA nuclease cleavage and for a dual-gRNA paired "nickase" strategy. gRNAs may have any of the lengths and properties described herein. Criteria for selecting gRNAs and the determination for which gRNAs can be used for which strategy is based on several considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

For designing knockout strategies, in some embodiments, the targeting domains for tier 1 gRNA molecules for *S. pyogenes* are selected based on their distance to the target site and their orthogonality (PAM is NGG). The targeting domains for tier 1 gRNA molecules are selected based on (1) a reasonable distance to the target position, e.g., within the first 500bp of coding sequence downstream of start codon and (2) a high level of orthogonality. For selection of tier 2 gRNAs, a high level of orthogonality is not required. Tier 3 gRNAs remove the requirement of good orthogonality and a longer sequence (e.g., the rest of the coding sequence) is scanned. Note that tiers are non-inclusive (each gRNA is listed only once). In certain instances, no gRNAs are identified based on the criteria of the particular tier.

For designing knockout strategies, in some embodiments, the targeting domain for tier 1 gRNA molecules for *N. meningtidis* are selected within the first 500bp of the coding sequence and have a high level of orthogonality. The targeting domain for tier 2 gRNA molecules for N. *meningtidis* are selected within the first 500bp of the coding sequence and do not require high orthogonality. The targeting domain for tier 3 gRNA molecules for *N. meningtidis* are selected within a remainder of coding sequence downstream of the 500bp. Note that tiers are non-inclusive (each gRNA is listed only once). In certain instances, no gRNAs are identified based on the criteria of the particular tier.

For designing knockout strategies, in some embodiments, the targeting domain for tier 1 grNA molecules for *S. aureus* are selected within the first 500bp of the coding sequence, have a high level of orthogonality, and contain a NNGRRT (R = A or G) PAM. The targeting domain for tier 2 grNA molecules for *S. aureus* are selected within the first 500bp of the coding sequence, no level of orthogonality is required, and contain a NNGRRT (R = A or G) PAM. The targeting domain for tier 3 grNA molecules for *S. aureus* are selected within the remainder of the coding sequence downstream and contain a NNGRRT (R = A or G) PAM. The targeting domain for tier 4 grNA molecules for *S. aureus* are selected within the first 500bp of the coding sequence and contain a NNGRRV PAM. The targeting domain for tier 5 grNA molecules for *S. aureus* are selected within the remainder of the coding sequence downstream and contain a NNGRRV PAM. Note that tiers are non-inclusive (each gRNA is listed only once). In certain instances, no gRNAs are identified based on the criteria of the particular tier.

For designing of gRNA molecules for knockdown strategies, in some embodiments, the targeting domain for tier 1 gRNA molecules for *S. pyogenes* are selected within the first 500bp upstream and downstream of the transcription start site and have a high level of orthogonality. The targeting domain for tier 2 gRNA molecules for *S. pyogenes* are selected within the first 500bp upstream and downstream of the transcription start site and do not require high orthogonality. The targeting domain for tier 3 gRNA molecules for *S. pyogenes* are selected within the additional 500bp upstream and downstream of transcription start site (e.g., extending to 1kb up and downstream of the transcription start site). Note that tiers are non-inclusive (each gRNA is listed only once). In certain instances, no gRNAs are identified based on the criteria of the particular tier.

For designing of gRNA molecules for knockdown strategies, in some embodiments, the targeting domain for tier 1 gRNA molecules for *N. meningtidis* are selected within the first 500bp upstream and downstream of the transcription start site and have a high level of orthogonality. The targeting domain for tier 2 gRNA molecules for *N. meningtidis* are selected within the first 500bp upstream and downstream of the transcription start site and do not require high orthogonality. The targeting domain for tier 3 gRNA molecules for *N. meningtidis* are selected within the additional 500bp upstream and downstream of transcription start site (e.g., extending to 1kb up and downstream of the transcription start site). Note that tiers are non-inclusive (each gRNA is listed only once). In certain instances, no gRNAs are identified based on the criteria of the particular tier.

For designing of gRNA molecules for knockdown strategies, in some embodiments, the targeting domain for tier 1 gRNA molecules for *S. aureus* are selected within 500bp upstream and downstream of transcription start site, a high level of orthogonality and PAM is NNGRRT (R = A or G). The targeting domain for tier 2 gRNA molecules for *S. aureus* are selected within 500bp upstream and downstream of transcription start site, no orthogonality requirement and PAM is NNGRRT (R = A or G). The targeting domain for tier 3 gRNA molecules for *S. aureus* are selected within the additional 500bp upstream and downstream of transcription start site (e.g., extending to 1kb up and downstream of the transcription start site) and PAM is NNGRRT (R = A or G). The targeting domain for tier 4 gRNA molecules for *S. aureus* are selected within 500bp upstream and downstream of transcription start site and PAM is NNGRRV. The targeting domain for tier 5 gRNA molecules for *S. aureus* are selected within the additional 500bp upstream and downstream of transcription start site (extending to 1kb up and downstream of the transcription start site) and PAM is NNGRRV. Note that tiers are non-inclusive (each gRNA is listed only once). In certain instances, no gRNAs are identified based on the criteria of the particular tier.

### III. Cas Molecules

Cas molecules(e.g., Cas9 molecules) of a variety of species can be used in the methods and compositions described herein. While the *S. pyogenes, S. aureus, N. meningitidis,* and *S*. *thermophilus* Cas9 molecules are the subject of much of the disclosure herein, Cas molecules (e.g., Cas9 molecules) of, derived from, or based on the Cas proteins of other species listed herein can be used as well. In other words, while the much of the description herein uses *S. pyogenes, S. aureus, N. meningitidis,* and *S. thermophilus* Cas9 molecules, Cas molecules from the other species can replace them. Such species include but are not limited to: *Acidovorax avenae, Actinobacillus pleuropneumoniae, Actinobacillus succinogenes, Actinobacillus suis, Actinomyces sp., Cycliphilusdenitrificans, Aminomonas paucivorans, Bacillus cereus, Bacillus smithii, Bacillus thuringiensis, Bacteroides sp., Blastopirellula marina, Bradyrhizobium sp., Brevibacillus laterosporus, Campylobacter coli, Campylobacter jejuni, Campylobacter lari, Candidatus puniceispirillum, Clostridium cellulolyticum, Clostridium perfringens, Corynebacterium accolens, Corynebacterium diphtheria, Corynebacterium matruchotii, Dinoroseobacter shibae, Eubacterium dolichum, Gammaproteobacterium, Gluconacetobacter diazotrophicus, Haemophilus parainfluenzae, Haemophilus sputorum, Helicobacter canadensis, Helicobacter cinaedi, Helicobacter mustelae, Ilyobacter polytropus, Kingella kingae, Lactobacillus crispatus, Listeria ivanovii, Listeria monocytogenes, Listeriaceae bacterium, Methylocystis sp., Methylosinus trichosporium, Mobiluncus mulieris, Neisseria bacilliformis, Neisseria cinerea, Neisseria flavescens, Neisseria lactamica, Neisseria meningitidis, Neisseria sp., Neisseria wadsworthii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus aureus, Staphylococcus lugdunensis, Streptococcus sp., Subdoligranulum sp., Tistrella mobilis, Treponema sp.,* or *Verminephrobacter eiseniae.*

A Cas-like molecule (e.g., Cas9-like molecule), or Cas-like polypeptide (e.g., Cas9-like polypeptide), as those terms are used herein, refer to a polypeptide that can interact with a gRNA molecule and, in concert with the gRNA molecule, home or localize to a site which comprises a target domain and PAM sequence. For example, Cas9 molecule and Cas9 polypeptide, as those terms are used herein, refer to naturally occurring Cas9 and to engineered, altered, or modified Cas9 polypeptides that differ, e.g., by at least one amino acid residue, from a reference sequence, e.g., the most similar naturally occurring Cas9 molecule or a sequence of **Table 100.**

### Cas9 Domains

Crystal structures have been determined for two different naturally occurring bacterial Cas9 molecules (Jinek et al., Science, 343(6176): 1247997, 2014) and for *S. pyogenes* Cas9 with a guide RNA (e.g., a synthetic fusion of crRNA and tracrRNA) (Nishimasu et al., Cell, 156:935-949, 2014; and Anders et al., Nature, 2014, doi: 10.1038/nature13579).

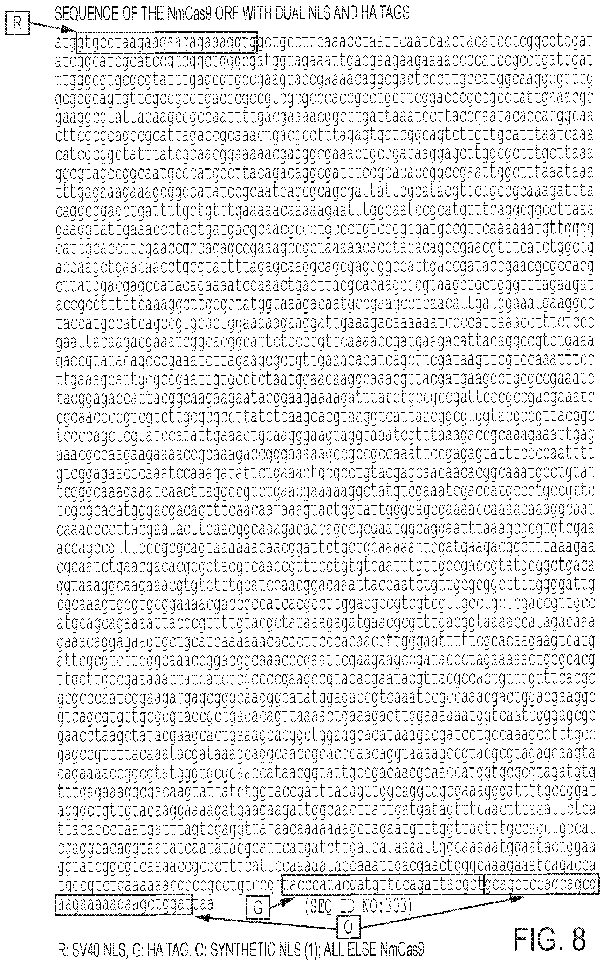

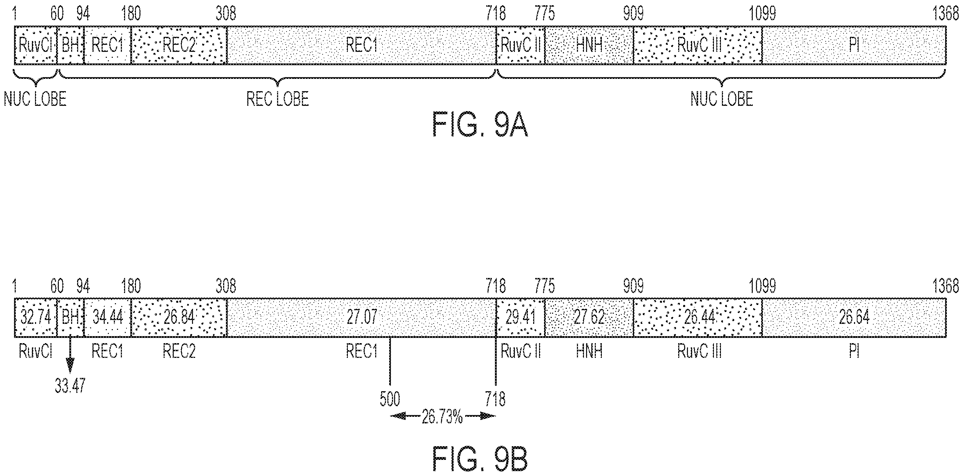

A naturally occurring Cas9 molecule comprises two lobes: a recognition (REC) lobe and a nuclease (NUC) lobe; each of which further comprises domains described herein. **Figs. 8A-8B** provide a schematic of the organization of important Cas9 domains in the primary structure. The domain nomenclature and the numbering of the amino acid residues encompassed by each domain used throughout this disclosure is as described in Nishimasu et al. The numbering of the amino acid residues is with reference to Cas9 from *S. pyogenes.* **Figs. 9A-9B** show schematic representations of the domain organization of *S. pyogenes* Cas9.

The REC lobe comprises the arginine-rich bridge helix (BH), the REC1 domain, and the REC2 domain. The REC lobe does not share structural similarity with other known proteins, indicating that it is a Cas9-specific functional domain. The BH domain is a long a-helix and arginine rich region and comprises amino acids 60-93 of the sequence of *S. pyogenes* Cas9. The REC1 domain is important for recognition of the repeat:anti-repeat duplex, e.g., of a gRNA or a tracrRNA, and is therefore critical for Cas9 activity by recognizing the target sequence. The REC1 domain comprises two REC1 motifs at amino acids 94 to 179 and 308 to 717 of the sequence of *S*. *pyogenes* Cas9. These two REC1 domains, though separated by the REC2 domain in the linear primary structure, assemble in the tertiary structure to form the REC1 domain. The REC2 domain, or parts thereof, may also play a role in the recognition of the repeat:anti-repeat duplex. The REC2 domain comprises amino acids 180-307 of the sequence of *S. pyogenes* Cas9.

The NUC lobe comprises the RuvC domain (also referred to herein as RuvC-like domain), the HNH domain (also referred to herein as HNH-like domain), and the PAM-interacting (PI) domain. The RuvC domain shares structural similarity to retroviral integrase superfamily members and cleaves a single strand, e.g., the non-complementary strand of the target nucleic acid molecule. The RuvC domain is assembled from the three split RuvC motifs (RuvC I, RuvCII, and RuvCIII, which are often commonly referred to in the art as RuvCI domain, or N-terminal RuvC domain, RuvCII domain, and RuvCIII domain) at amino acids 1-59, 718-769, and 909-1098, respectively, of the sequence of *S. pyogenes* Cas9. Similar to the REC1 domain, the three RuvC motifs are linearly separated by other domains in the primary structure, however in the tertiary structure, the three RuvC motifs assemble and form the RuvC domain. The HNH domain shares structural similarity with HNH endonucleases, and cleaves a single strand, e.g., the complementary strand of the target nucleic acid molecule. The HNH domain lies between the RuvC II-III motifs and comprises amino acids 775-908 of the sequence of *S. pyogenes* Cas9. The PI domain interacts with the PAM of the target nucleic acid molecule, and comprises amino acids 1099-1368 of the sequence *of S. pyogenes* Cas9.

### A RuvC-like domain and an HNH-like domain

In an embodiment, a Cas9 molecule or Cas9 polypeptide comprises an HNH-like domain and a RuvC-like domain. In an embodiment, cleavage activity is dependent on a RuvC-like domain and an HNH-like domain. A Cas9 molecule or Cas9 polypeptide, e.g., an eaCas9 molecule or eaCas9 polypeptide, can comprise one or more of the following domains: a RuvC-like domain and an HNH-like domain. In an embodiment, a Cas9 molecule or Cas9 polypeptide is an eaCas9 molecule or eaCas9 polypeptide and the eaCas9 molecule or eaCas9 polypeptide comprises a RuvC-like domain, e.g., a RuvC-like domain described below, and/or an HNH-like domain, e.g., an HNH-like domain described below.

### RuvC-like domains

In an embodiment, a RuvC-like domain cleaves, a single strand, e.g., the non-complementary strand of the target nucleic acid molecule. The Cas9 molecule or Cas9 polypeptide can include more than one RuvC-like domain (e.g., one, two, three or more RuvC-like domains). In an embodiment, a RuvC-like domain is at least 5, 6, 7, 8 amino acids in length but not more than 20, 19, 18, 17, 16 or 15 amino acids in length. In an embodiment, the Cas9 molecule or Cas9 polypeptide comprises an N-terminal RuvC-like domain of about 10 to 20 amino acids, e.g., about 15 amino acids in length.

### N-terminal RuvC-like domains

Some naturally occurring Cas9 molecules comprise more than one RuvC-like domain with cleavage being dependent on the N-terminal RuvC-like domain. Accordingly, Cas9 molecules or Cas9 polypeptide can comprise an N-terminal RuvC-like domain. Exemplary N-terminal RuvC-like domains are described below.

In an embodiment, an eaCas9 molecule or eaCas9 polypeptide comprises an N-terminal RuvC-like domain comprising an amino acid sequence of formula I:
D-X1-G-X2-X3-X4-X5-G-X6-X7-X8-X9 (SEQ ID NO:8),
wherein,
X1 is selected from I, V, M, L and T (e.g., selected from I, V, and L);
X2 is selected from T, I, V, S, N, Y, E and L (e.g., selected from T, V, and I);
X3 is selected from N, S, G, A, D, T, R, M and F (e.g., A or N);
X4 is selected from S, Y, N and F (e.g., S);
X5 is selected from V, I, L, C, T and F (e.g., selected from V, I and L);
X6 is selected from W, F, V, Y, S and L (e.g., W);
X7 is selected from A, S, C, V and G (e.g., selected from A and S);
X8 is selected from V, I, L, A, M and H (e.g., selected from V, I, M and L); and
X9 is selected from any amino acid or is absent, designated by Δ (e.g., selected from T, V, I, L, Δ, F, S, A, Y, M and R, or, e.g., selected from T, V, I, L and Δ).

In an embodiment, the N-terminal RuvC-like domain differs from a sequence of SEQ ID NO:8, by as many as 1 but no more than 2, 3, 4, or 5 residues.

In embodiment, the N-terminal RuvC-like domain is cleavage competent.

In embodiment, the N-terminal RuvC-like domain is cleavage incompetent.

In an embodiment, a eaCas9 molecule or eaCas9 polypeptide comprises an N-terminal RuvC-like domain comprising an amino acid sequence of formula II:
D-X1-G-X2-X3-S-X5-G-X6-X7-X8-X9 (SEQ ID NO:9),
wherein
X1 is selected from I, V, M, L and T (e.g., selected from I, V, and L);
X2 is selected from T, I, V, S, N, Y, E and L (e.g., selected from T, V, and I);
X3 is selected from N, S, G, A, D, T, R, M and F (e.g., A or N);
X5 is selected from V, I, L, C, T and F (e.g., selected from V, I and L);
X6 is selected from W, F, V, Y, S and L (e.g., W);
X7 is selected from A, S, C, V and G (e.g., selected from A and S);
X8 is selected from V, I, L, A, M and H (e.g., selected from V, I, M and L); and
X9 is selected from any amino acid or is absent (e.g., selected from T, V, I, L, Δ, F, S, A, Y, M and R or selected from e.g., T, V, I, L and Δ).

In an embodiment, the N-terminal RuvC-like domain differs from a sequence of SEQ ID NO:9 by as many as 1 but no more than 2, 3, 4, or 5 residues.

In an embodiment, the N-terminal RuvC-like domain comprises an amino acid sequence of formula III:
D-I-G-X2-X3-S-V-G-W-A-X8-X9 (SEQ ID NO:10),
wherein
X2 is selected from T, I, V, S, N, Y, E and L (e.g., selected from T, V, and I);
X3 is selected from N, S, G, A, D, T, R, M and F (e.g., A or N);
X8 is selected from V, I, L, A, M and H (e.g., selected from V, I, M and L); and
X9 is selected from any amino acid or is absent (e.g., selected from T, V, I, L, Δ, F, S, A, Y, M and R or selected from e.g., T, V, I, L and Δ).

In an embodiment, the N-terminal RuvC-like domain differs from a sequence of SEQ ID NO:10 by as many as 1 but no more than, 2, 3, 4, or 5 residues.

In an embodiment, the N-terminal RuvC-like domain comprises an amino acid sequence of formula III:
D-I-G-T-N-S-V-G-W-A-V-X (SEQ ID NO:11),
wherein
X is a non-polar alkyl amino acid or a hydroxyl amino acid, e.g., X is selected from V, I, L and T (e.g., the eaCas9 molecule can comprise an N-terminal RuvC-like domain shown in **Figs. 2A-2G** (is depicted as Y)).

In an embodiment, the N-terminal RuvC-like domain differs from a sequence of SEQ ID NO:11 by as many as 1 but no more than, 2, 3, 4, or 5 residues.

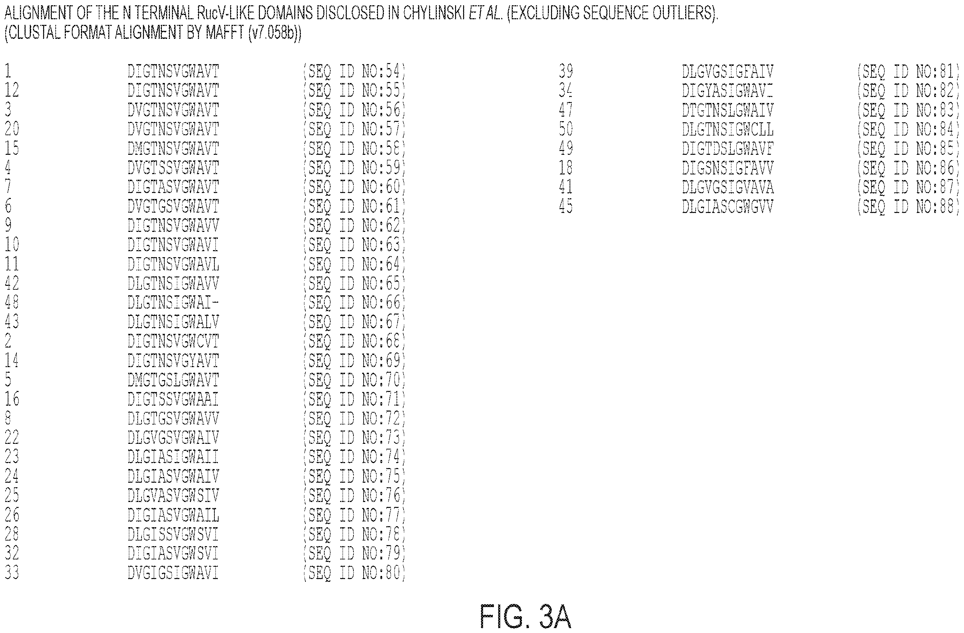

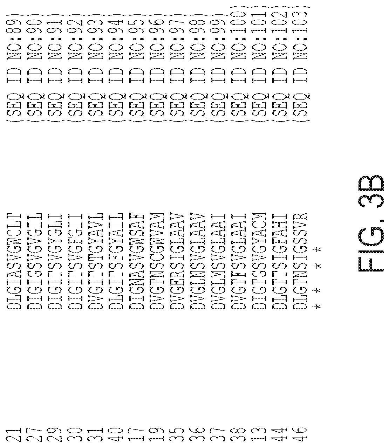

In an embodiment, the N-terminal RuvC-like domain differs from a sequence of an N-terminal RuvC like domain disclosed herein, e.g., in **Figs. 3A-3B** or **Figs. 7A-7B****,** as many as 1 but no more than 2, 3, 4, or 5 residues. In an embodiment, 1, 2, or all 3 of the highly conserved residues identified **in** **Figs. 3A-3B** **or** **Figs. 7A-7B** are present.

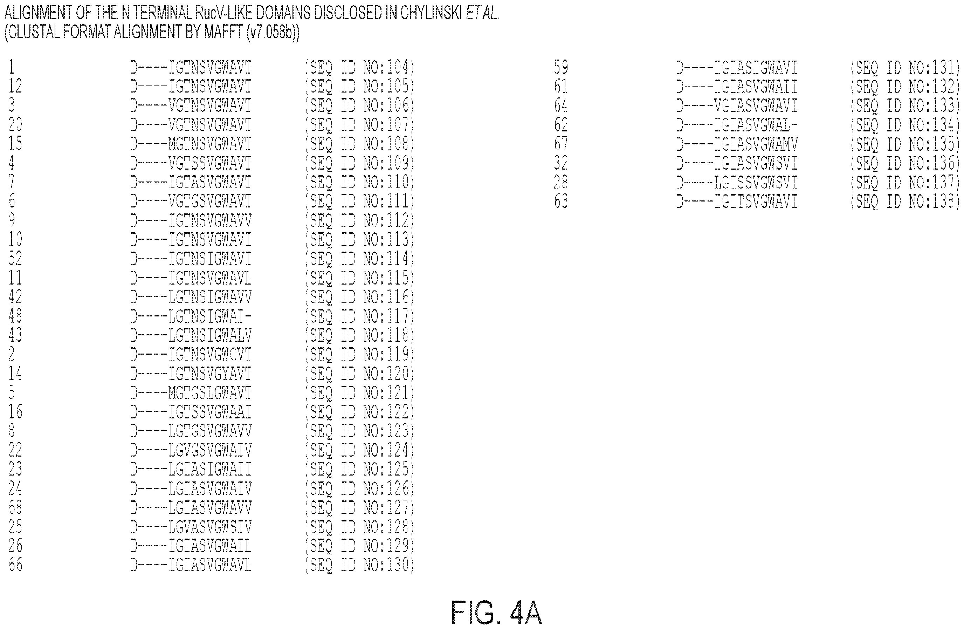

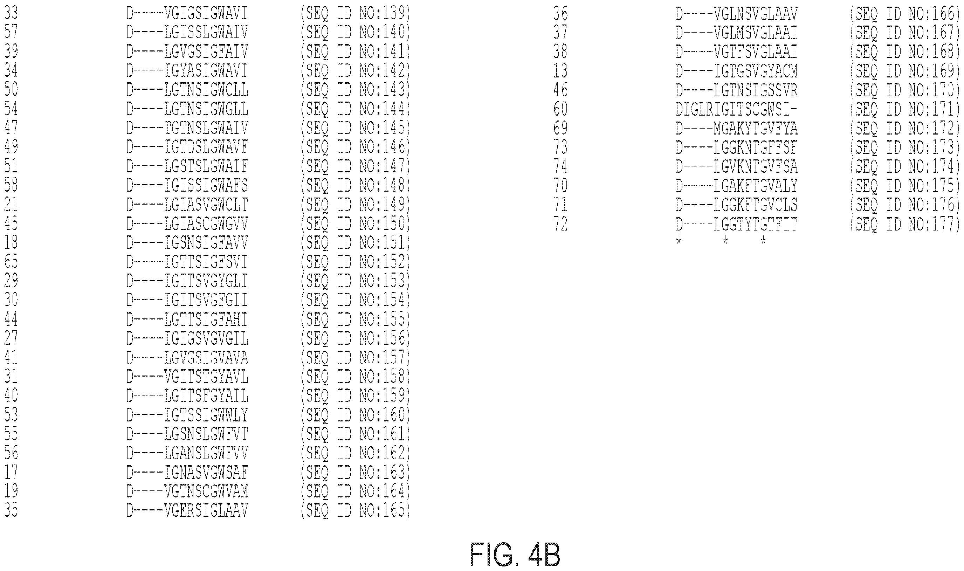

In an embodiment, the N-terminal RuvC-like domain differs from a sequence of an N-terminal RuvC-like domain disclosed herein, e.g., in **Figs. 4A-4B** or **Figs. 7A-7B****,** as many as 1 but no more than 2, 3, 4, or 5 residues. In an embodiment, 1, 2, 3 or all 4 of the highly conserved residues identified in **Figs. 4A-4B** or **Figs. 7A-7B** are present.

### Additional RuvC-like domains

In addition to the N-terminal RuvC-like domain, the Cas9 molecule or Cas9 polypeptide, e.g., an eaCas9 molecule or eaCas9 polypeptide, can comprise one or more additional RuvC-like domains. In an embodiment, the Cas9 molecule or Cas9 polypeptide can comprise two additional RuvC-like domains. Preferably, the additional RuvC-like domain is at least 5 amino acids in length and, e.g., less than 15 amino acids in length, e.g., 5 to 10 amino acids in length, e.g., 8 amino acids in length.

An additional RuvC-like domain can comprise an amino acid sequence:
I-X1-X2-E-X3-A-R-E (SEQ ID NO: 12),
wherein
X1 is V or H,
X2 is I, L or V (e.g., I or V); and
X3 is M or T.

In an embodiment, the additional RuvC-like domain comprises the amino acid sequence:
I-V-X2-E-M-A-R-E (SEQ ID NO: 13),
wherein
X2 is I, L or V (e.g., I or V) (e.g., the eaCas9 molecule or eaCas9 polypeptide can comprise an additional RuvC-like domain shown in **Figs. 2A-2G** or **Figs. 7A-7B** (depicted as B)).

An additional RuvC-like domain can comprise an amino acid sequence:
H-H-A-X1-D-A-X2-X3 (SEQ ID NO:14),
wherein
X1 is H or L;
X2 is R or V; and
X3 is E or V.

In an embodiment, the additional RuvC-like domain comprises the amino acid sequence:
H-H-A-H-D-A-Y-L (SEQ ID NO:15).

In an embodiment, the additional RuvC-like domain differs from a sequence of SEQ ID NO: 12, 13, 14 or 15 by as many as 1 but no more than 2, 3, 4, or 5 residues.

In some embodiments, the sequence flanking the N-terminal RuvC-like domain is a sequence of formula V:
K-X1'-Y-X2'-X3'-X4'-Z-T-D-X9'-Y (SEQ ID NO:16),
wherein
X1' is selected from K and P,
X2' is selected from V, L, I, and F (e.g., V, I and L);
X3' is selected from G, A and S (e.g., G),
X4' is selected from L, I, V and F (e.g., L);
X9' is selected from D, E, N and Q; and
Z is an N-terminal RuvC-like domain, e.g., as described above.

### HNH-like domains

In an embodiment, an HNH-like domain cleaves a single stranded complementary domain, e.g., a complementary strand of a double stranded nucleic acid molecule. In an embodiment, an HNH-like domain is at least 15, 20, 25 amino acids in length but not more than 40, 35 or 30 amino acids in length, e.g., 20 to 35 amino acids in length, e.g., 25 to 30 amino acids in length. Exemplary HNH-like domains are described below.

In an embodiment, an eaCas9 molecule or eaCas9 polypeptide comprises an HNH-like domain having an amino acid sequence of formula VI: 
X1-X2-X3-H-X4-X5-P-X6-X7-X8-X9-X10-X11-X12-X13-X14-X15-N-X16-X17-X18-
X19-X20-X21-X22-X23-N (SEQ ID NO:17),
 wherein
X1 is selected from D, E, Q and N (e.g., D and E);
X2 is selected from L, I, R, Q, V, M and K;
X3 is selected from D and E;
X4 is selected from I, V, T, A and L (e.g., A, I and V);
X5 is selected from V, Y, I, L, F and W (e.g., V, I and L);
X6 is selected from Q, H, R, K, Y, I, L, F and W;
X7 is selected from S, A, D, T and K (e.g., S and A);
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X11 is selected from D, S, N, R, L and T (e.g., D);
X12 is selected from D, N and S;
X13 is selected from S, A, T, G and R (e.g., S);
X14 is selected from I, L, F, S, R, Y, Q, W, D, K and H (e.g., I, L and F);
X15 is selected from D, S, I, N, E, A, H, F, L, Q, M, G, Y and V;
X16 is selected from K, L, R, M, T and F (e.g., L, R and K);
X17 is selected from V, L, I, A and T;
X18 is selected from L, I, V and A (e.g., L and I);
X19 is selected from T, V, C, E, S and A (e.g., T and V);
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, a HNH-like domain differs from a sequence of SEQ ID NO:17 by at least one but no more than, 2, 3, 4, or 5 residues.

In an embodiment, the HNH-like domain is cleavage competent.

In an embodiment, the HNH-like domain is cleavage incompetent.

In an embodiment, an eaCas9 molecule or eaCas9 polypeptide comprises an HNH-like domain comprising an amino acid sequence of formula VII: 
X1-X2-X3-H-X4-X5-P-X6-S-X8-X9-X10-D-D-S-X14-X15-N-K-V-L-X19-X20-X21-X2
2-X23-N (SEQ ID NO:18),
 wherein
X1 is selected from D and E;
X2 is selected from L, I, R, Q, V, M and K;
X3 is selected from D and E;
X4 is selected from I, V, T, A and L (e.g., A, I and V);
X5 is selected from V, Y, I, L, F and W (e.g., V, I and L);
X6 is selected from Q, H, R, K, Y, I, L, F and W;
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X14 is selected from I, L, F, S, R, Y, Q, W, D, K and H (e.g., I, L and F);
X15 is selected from D, S, I, N, E, A, H, F, L, Q, M, G, Y and V;
X19 is selected from T, V, C, E, S and A (e.g., T and V);
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, the HNH-like domain differs from a sequence of SEQ ID NO:18 by 1, 2, 3, 4, or 5 residues.

In an embodiment, an eaCas9 molecule or eaCas9 polypeptide comprises an HNH-like domain comprising an amino acid sequence of formula VII: 
X1-V-X3-H-I-V-P-X6-S-X8-X9-X10-D-D-S-X14-X15-N-K-V-L-T-X20-X21-X22-X23-
N (SEQ ID NO:19),
 wherein
X1 is selected from D and E;
X3 is selected from D and E;
X6 is selected from Q, H, R, K, Y, I, L and W;
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X14 is selected from I, L, F, S, R, Y, Q, W, D, K and H (e.g., I, L and F);
X15 is selected from D, S, I, N, E, A, H, F, L, Q, M, G, Y and V;
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, the HNH-like domain differs from a sequence of SEQ ID NO:19 by 1, 2, 3, 4, or 5 residues.

In an embodiment, an eaCas9 molecule or eaCas9 polypeptide comprises an HNH-like domain having an amino acid sequence of formula VIII:
D-X2-D-H-I-X5-P-Q-X7-F-X9-X10-D-X12-S-I-D-N-X16-V-L-X19-X20-S-X22-X23-N (SEQ ID NO:20),
wherein
X2 is selected from I and V;
X5 is selected from I and V;
X7 is selected from A and S;
X9 is selected from I and L;
X10 is selected from K and T;
X12 is selected from D and N;
X16 is selected from R, K and L; X19 is selected from T and V;
X20 is selected from S and R;
X22 is selected from K, D and A; and
X23 is selected from E, K, G and N (e.g., the eaCas9 molecule or eaCas9 polypeptide can comprise an HNH-like domain as described herein).

In an embodiment, the HNH-like domain differs from a sequence of SEQ ID NO:20 by as many as 1 but no more than 2, 3, 4, or 5 residues.

In an embodiment, an eaCas9 molecule or eaCas9 polypeptide comprises the amino acid sequence of formula IX: 
L-Y-Y-L-Q-N-G-X1'-D-M-Y-X2'-X3'-X4'-X5'-L-D-I—X6'-X7'-L-S-X8'-Y-Z-N-R-X
9'-K-X10'-D-X11'-V-P (SEQ ID NO:21),
 wherein
X1' is selected from K and R;
X2' is selected from V and T;
X3' is selected from G and D;
X4' is selected from E, Q and D;
X5' is selected from E and D;
X6' is selected from D, N and H;
X7' is selected from Y, R and N;
X8' is selected from Q, D and N; X9' is selected from G and E;
X10' is selected from S and G;
X11' is selected from D and N; and
Z is an HNH-like domain, e.g., as described above.

In an embodiment, the eaCas9 molecule or eaCas9 polypeptide comprises an amino acid sequence that differs from a sequence of SEQ ID NO:21 by as many as 1 but no more than 2, 3, 4, or 5 residues.

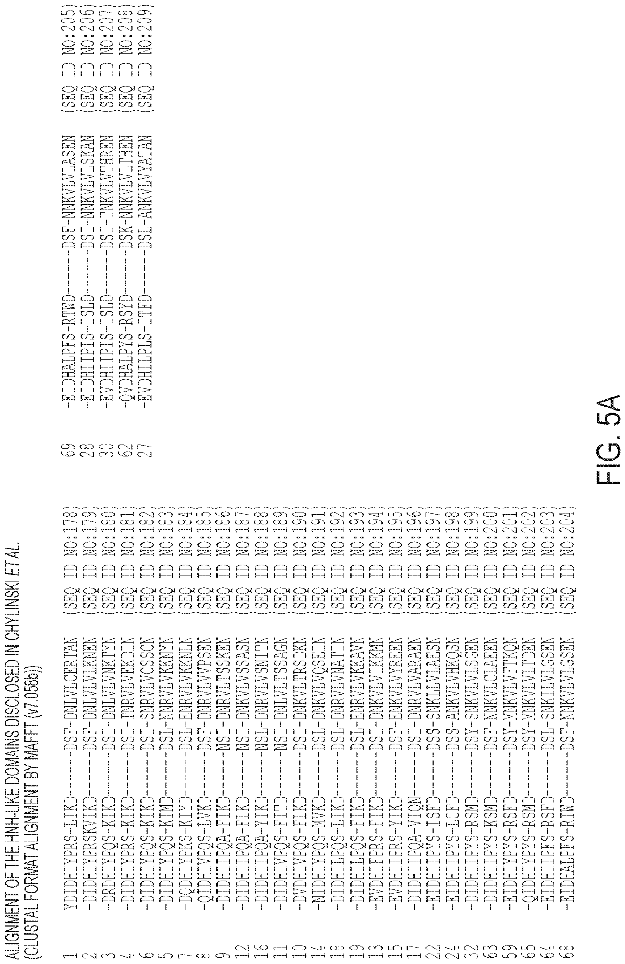

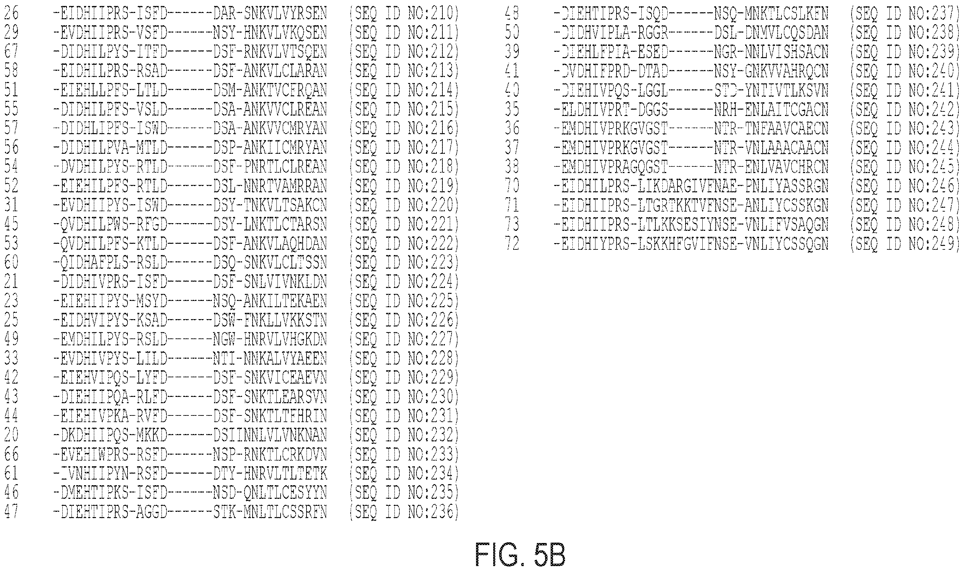

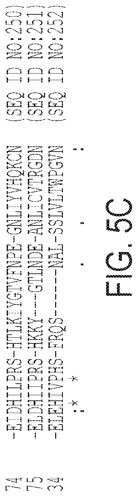

In an embodiment, the HNH-like domain differs from a sequence of an HNH-like domain disclosed herein, e.g., in **Figs. 5A-5C** or **Figs. 7A-7B****,** as many as 1 but no more than 2, 3, 4, or 5 residues. In an embodiment, 1 or both of the highly conserved residues identified in **Figs. 5A-5C** or **Figs. 7A-7B** are present.

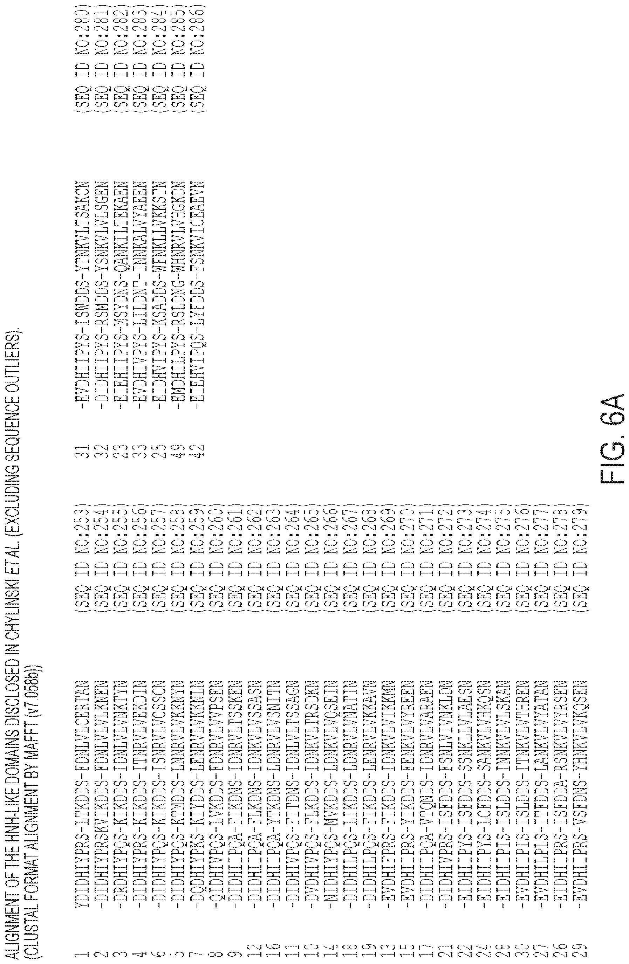

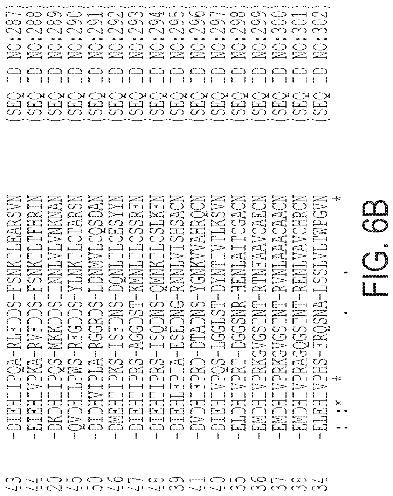

In an embodiment, the HNH -like domain differs from a sequence of an HNH-like domain disclosed herein, e.g., in **Figs. 6A-6B** or **Figs. 7A-7B****,** as many as 1 but no more than 2, 3, 4, or 5 residues. In an embodiment, 1, 2, all 3 of the highly conserved residues identified in **Figs. 6A-6B** or **Figs. 7A-7B** are present.

### Cas9 Activities

### Nuclease and Helicase Activities

In an embodiment, the Cas9 molecule or Cas9 polypeptide is capable of cleaving a target nucleic acid molecule. Typically wild type Cas9 molecules cleave both strands of a target nucleic acid molecule. Cas9 molecules and Cas9 polypeptides can be engineered to alter nuclease cleavage (or other properties), e.g., to provide a Cas9 molecule or Cas9 peolypeptide which is a nickase, or which lacks the ability to cleave target nucleic acid. A Cas9 molecule or Cas9 polypeptide that is capable of cleaving a target nucleic acid molecule is referred to herein as an eaCas9 molecule or eaCas9 polypeptide

In an embodiment, an eaCas9 molecule or eaCas9 polypeptide comprises one or more of the following activities:
a nickase activity, i.e., the ability to cleave a single strand, e.g., the non-complementary strand or the complementary strand, of a nucleic acid molecule;
a double stranded nuclease activity, i.e., the ability to cleave both strands of a double stranded nucleic acid and create a double stranded break, which in an embodiment is the presence of two nickase activities;
an endonuclease activity;
an exonuclease activity; and
a helicase activity, i.e., the ability to unwind the helical structure of a double stranded nucleic acid.

In an embodiment, an enzymatically active or eaCas9 molecule or eaCas9 polypeptide cleaves both strands and results in a double stranded break. In an embodiment, an eaCas9 molecule cleaves only one strand, e.g., the strand to which the gRNA hybridizes to, or the strand complementary to the strand the gRNA hybridizes with. In an embodiment, an eaCas9 molecule or eaCas9 polypeptide comprises cleavage activity associated with an HNH-like domain. In an embodiment, an eaCas9 molecule or eaCas9 polypeptide comprises cleavage activity associated with an N-terminal RuvC-like domain. In an embodiment, an eaCas9 molecule or eaCas9 polypeptide comprises cleavage activity associated with an HNH-like domain and cleavage activity associated with an N-terminal RuvC-like domain. In an embodiment, an eaCas9 molecule or eaCas9 polypeptide comprises an active, or cleavage competent, HNH-like domain and an inactive, or cleavage incompetent, N-terminal RuvC-like domain. In an embodiment, an eaCas9 molecule or eaCas9 polypeptide comprises an inactive, or cleavage incompetent, HNH-like domain and an active, or cleavage competent, N-terminal RuvC-like domain.

Some Cas9 molecules or Cas9 polypeptides have the ability to interact with a gRNA molecule, and in conjunction with the gRNA molecule localize to a core target domain, but are incapable of cleaving the target nucleic acid, or incapable of cleaving at efficient rates. Cas9 molecules having no, or no substantial, cleavage activity are referred to herein as an eiCas9 molecule or eiCas9 polypeptide. For example, an eiCas9 molecule or eiCas9 polypeptide can lack cleavage activity or have substantially less, e.g., less than 20, 10, 5, 1 or 0.1 % of the cleavage activity of a reference Cas9 molecule or eiCas9 polypeptide, as measured by an assay described herein.

### Targeting and PAMs

A Cas9 molecule or Cas9 polypeptide, is a polypeptide that can interact with a guide RNA (gRNA) molecule and, in concert with the gRNA molecule, localizes to a site which comprises a target domain and a PAM sequence.

In an embodiment, the ability of an eaCas9 molecule or eaCas9 polypeptide to interact with and cleave a target nucleic acid is PAM sequence dependent. A PAM sequence is a sequence in the target nucleic acid. In an embodiment, cleavage of the target nucleic acid occurs upstream from the PAM sequence. EaCas9 molecules from different bacterial species can recognize different sequence motifs (e.g., PAM sequences). In an embodiment, an eaCas9 molecule of *S*. *pyogenes* recognizes the sequence motif NGG, NAG, NGA and directs cleavage of a target nucleic acid sequence 1 to 10, e.g., 3 to 5, base pairs upstream from that sequence. See, e.g., Mali et al., SCIENCE 2013; 339(6121): 823-826. In an embodiment, an eaCas9 molecule of *S. thermophilus* recognizes the sequence motif NGGNG and/or NNAGAAW (W = A or T) and directs cleavage of a target nucleic acid sequence 1 to 10, e.g., 3 to 5, base pairs upstream from these sequences. See, e.g., Horvath et al., SCIENCE 2010; 327(5962): 167-170, and Deveau et al., JBACTERIOL 2008; 190(4): 1390-1400. In an embodiment, an eaCas9 molecule of *S. mutans* recognizes the sequence motif NGG and/or NAAR (R = A or G)) and directs cleavage of a core target nucleic acid sequence 1 to 10, e.g., 3 to 5 base pairs, upstream from this sequence. See, e.g., Deveau et al., JBACTERIOL 2008; 190(4): 1390-1400. In an embodiment, an eaCas9 molecule of *S. aureus* recognizes the sequence motif NNGRR (R = A or G) and directs cleavage of a target nucleic acid sequence 1 to 10, e.g., 3 to 5, base pairs upstream from that sequence. In an embodiment, an eaCas9 molecule of *S. aureus* recognizes the sequence motif NNGRRT (R = A or G) and directs cleavage of a target nucleic acid sequence 1 to 10, e.g., 3 to 5, base pairs upstream from that sequence. In an embodiment, an eaCas9 molecule of *S. aureus* recognizes the sequence motif NNGRRV (R = A or G) and directs cleavage of a target nucleic acid sequence 1 to 10, e.g., 3 to 5, base pairs upstream from that sequence. In an embodiment, an eaCas9 molecule of *N. meningitidis* recognizes the sequence motif NNNNGATT or NNNGCTT (R = A or G, V = A, G or C and directs cleavage of a target nucleic acid sequence 1 to 10, e.g., 3 to 5, base pairs upstream from that sequence. See, e.g., Hou et al., PNAS EARLY EDITION 2013, 1-6. The ability of a Cas9 molecule to recognize a PAM sequence can be determined, e.g., using a transformation assay described in Jinek et al., SCIENCE 2012 337:816. In the aforementioned embodiments, N can be any nucleotide residue, e.g., any of A, G, C or T.

As is discussed herein, Cas9 molecules can be engineered to alter the PAM specificity of the Cas9 molecule.

Exemplary naturally occurring Cas9 molecules are described in Chylinski et al., RNA Biology 2013 10:5, 727-737. Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 11 bacterial family, a cluster 12 bacterial family, a cluster 13 bacterial family, a cluster 14 bacterial family, a cluster 15 bacterial family, a cluster 16 bacterial family, a cluster 17 bacterial family, a cluster 18 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family, a cluster 49 bacterial family, a cluster 50 bacterial family, a cluster 51 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

Exemplary naturally occurring Cas9 molecules include a Cas9 molecule of a cluster 1 bacterial family. Examples include a Cas9 molecule of: *S. pyogenes* (e.g., strain SF370, MGAS10270, MGAS10750, MGAS2096, MGAS315, MGAS5005, MGAS6180, MGAS9429, NZ131 and SSI-1), *S. thermophilus* (e.g., strain LMD-9), *S. pseudoporcinus* (e.g., strain SPIN 20026), *S. mutans* (e.g., strain UA159, NN2025), *S. macacae* (e.g., strain NCTC11558), *S. gallolyticus* (e.g., strain UCN34, ATCC BAA-2069), *S. equines* (e.g., strain ATCC 9812, MGCS 124), *S. dysdalactiae* (e.g., strain GGS 124), *S. bovis* (e.g., strain ATCC 700338), *S. anginosus* (e.g., strain F0211), *S. agalactiae* (e.g., strain NEM316, A909), *Listeria monocytogenes* (e.g., strain F6854), *Listeria innocua* (*L. innocua,* e.g., strain Clip1 1262), *Enterococcus italicus* (e.g., strain DSM 15952), or *Enterococcus faecium* (e.g., strain 1,231,408). Another exemplary Cas9 molecule is a Cas9 molecule of *Neisseria meningitidis* (Hou et al., PNAS Early Edition 2013, 1-6).

In an embodiment, a Cas9 molecule or Cas9 polypeptide, e.g., an eaCas9 molecule or eaCas9 polypeptide, comprises an amino acid sequence:
having 60%, 65%, 70%, 75%, 80%, 85%, 90%, 95%, 96%, 97%, 98% or 99% homology with;
differs at no more than, 2, 5, 10, 15, 20, 30, or 40% of the amino acid residues when compared with;
differs by at least 1, 2, 5, 10 or 20 amino acids but by no more than 100, 80, 70, 60, 50, 40 or 30 amino acids from; or
is identical to any Cas9 molecule sequence described herein, or a naturally occurring Cas9 molecule sequence, e.g., a Cas9 molecule from a species listed herein or described in Chylinski et al., RNA Biology 2013 10:5, 727-737; Hou et al., PNAS Early Edition 2013, 1-6; SEQ ID NO:1-4. In an embodiment, the Cas9 molecule or Cas9 polypeptide comprises one or more of the following activities: a nickase activity; a double stranded cleavage activity (e.g., an endonuclease and/or exonuclease activity); a helicase activity; or the ability, together with a gRNA molecule, to home to a target nucleic acid.

In an embodiment, a Cas9 molecule or Cas9 polypeptide comprises the amino acid sequence of the consensus sequence of **Figs. 2A-2G****,** wherein "*" indicates any amino acid found in the corresponding position in the amino acid sequence of a Cas9 molecule *of S. pyogenes, S. thermophilus, S. mutans and L. innocua,* and "-" indicates any amino acid. In an embodiment, a Cas9 molecule or Cas9 polypeptide differs from the sequence of the consensus sequence disclosed in **Figs. 2A-2G** by at least 1, but no more than 2, 3, 4, 5, 6, 7, 8, 9, or 10 amino acid residues. In an embodiment, a Cas9 molecule or Cas9 polypeptide comprises the amino acid sequence of SEQ ID NO:7 of **Figs. 7A-7B****,** wherein "*" indicates any amino acid found in the corresponding position in the amino acid sequence of a Cas9 molecule *of S. pyogenes,* or *N. meningitidis,* "-" indicates any amino acid, and "-" indicates any amino acid or absent. In an embodiment, a Cas9 molecule or Cas9 polypeptide differs from the sequence of SEQ ID NO:6 or 7 disclosed in **Figs. 7A-7B** by at least 1, but no more than 2, 3, 4, 5, 6, 7, 8, 9, or 10 amino acid residues.

A comparison of the sequence of a number of Cas9 molecules indicate that certain regions are conserved. These are identified below as:
region 1 (residues 1 to 180, or in the case of region 1'residues 120 to 180)
region 2 (residues360 to 480);
region 3 (residues 660 to 720);
region 4 (residues 817 to 900); and
region 5 (residues 900 to 960);

In an embodiment, a Cas9 molecule or Cas9 polypeptide comprises regions 1-5, together with sufficient additional Cas9 molecule sequence to provide a biologically active molecule, e.g., a Cas9 molecule having at least one activity described herein. In an embodiment, each of regions 1-6, independently, have, 50%, 60%, 70%, or 80% homology with the corresponding residues of a Cas9 molecule or Cas9 polypeptide described herein, e.g., a sequence from **Figs. 2A-2G** or from **Figs. 7A-7B****.**

In an embodiment, a Cas9 molecule or Cas9 polypeptide, e.g., an eaCas9 molecule or eaCas9 polypeptide, comprises an amino acid sequence referred to as region 1:
having 50%, 60%, 70%, 80%, 85%, 90%, 95%, 96%, 97%, 98% or 99% homology with amino acids 1-180 (the numbering is according to the motif sequence in **Figs. 2A-2G****;** 52% of residues in the four Cas9 sequences in **Figs. 2A-2G** are conserved) of the amino acid sequence of Cas9 of *S. pyogenes;*
differs by at least 1, 2, 5, 10 or 20 amino acids but by no more than 90, 80, 70, 60, 50, 40 or 30 amino acids from amino acids 1-180 of the amino acid sequence of Cas9 *of S. pyogenes, S. thermophilus, S. mutans or L. innocua;* or
is identical to 1-180 of the amino acid sequence of Cas9 of *S. pyogenes, S. thermophilus, S. mutans or L. innocua.*
In an embodiment, a Cas9 molecule or Cas9 polypeptide, e.g., an eaCas9 molecule or eaCas9 polypeptide, comprises an amino acid sequence referred to as region 1':
having 55%, 60%, 65%, 70%, 75%, 80%, 85%, 90%, 95%, 96%, 97%, 98% or 99% homology with amino acids 120-180 (55% of residues in the four Cas9 sequences in **Figs. 2A-2G** are conserved) of the amino acid sequence of Cas9 of *S. pyogenes, S. thermophilus, S. mutans* or *L. innocua;*
differs by at least 1, 2, or 5 amino acids but by no more than 35, 30, 25, 20 or 10 amino acids from amino acids 120-180 of the amino acid sequence of Cas9 *of S. pyogenes, S. thermophilus, S. mutans* or *L. innocua;* or
is identical to 120-180 of the amino acid sequence of Cas9 of *S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 2:
having 50%, 55%, 60%, 65%, 70%, 75%, 80%, 85%, 90%, 95%, 96%, 97%, 98% or 99% homology with amino acids 360-480 (52% of residues in the four Cas9 sequences in **Figs. 2A-2G** are conserved) of the amino acid sequence of Cas9 of *S. pyogenes, S. thermophilus, S. mutans or L. innocua;*
differs by at least 1, 2, or 5 amino acids but by no more than 35, 30, 25, 20 or 10 amino acids from amino acids 360-480 of the amino acid sequence of Cas9 *of S. pyogenes, S. thermophilus, S. mutans or L. innocua;* or
is identical to 360-480 of the amino acid sequence of Cas9 of *S. pyogenes, S. thermophilus, S. mutans or L. innocua.*

In an embodiment, a Cas9 molecule or Cas9 polypeptide, e.g., an eaCas9 molecule or eaCas9 polypeptide, comprises an amino acid sequence referred to as region 3:
having 55%, 60%, 65%, 70%, 75%, 80%, 85%, 90%, 95%, 96%, 97%, 98%, or 99% homology with amino acids 660-720 (56% of residues in the four Cas9 sequences in **Figs. 2A-2G** are conserved) of the amino acid sequence of Cas9 of *S. pyogenes, S.* thermophilus, S. mutans or L. innocua;
differs by at least 1, 2, or 5 amino acids but by no more than 35, 30, 25, 20 or 10 amino acids from amino acids 660-720 of the amino acid sequence of Cas9 *of S. pyogenes, S. thermophilus, S. mutans or L. innocua;* or
is identical to 660-720 of the amino acid sequence of Cas9 *of S. pyogenes, S. thermophilus, S. mutans or L. innocua.*

In an embodiment, a Cas9 molecule or Cas9 polypeptide, e.g., an eaCas9 molecule or eaCas9 polypeptide, comprises an amino acid sequence referred to as region 4:
having 50%, 55%, 60%, 65%, 70%, 75%, 80%, 85%, 90%, 95%, 96%, 97%, 98%, or 99% homology with amino acids 817-900 (55% of residues in the four Cas9 sequences in **Figs. 2A-2G** are conserved) of the amino acid sequence of Cas9 of *S. pyogenes, S. thermophilus, S. mutans or L. innocua;*
differs by at least 1, 2, or 5 amino acids but by no more than 35, 30, 25, 20 or 10 amino acids from amino acids 817-900 of the amino acid sequence of Cas9 *of S. pyogenes, S. thermophilus, S. mutans or L. innocua;* or
is identical to 817-900 of the amino acid sequence of Cas9 *of S. pyogenes, S. thermophilus, S. mutans or L. innocua.*

In an embodiment, a Cas9 molecule or Cas9 polypeptide, e.g., an eaCas9 molecule or eaCas9 polypeptide, comprises an amino acid sequence referred to as region 5:
having 50%, 55%, 60%, 65%, 70%, 75%, 80%, 85%, 90%, 95%, 96%, 97%, 98%, or 99% homology with amino acids 900-960 (60% of residues in the four Cas9 sequences in **Figs. 2A-2G** are conserved) of the amino acid sequence of Cas9 of *S. pyogenes, S. thermophilus, S. mutans or L. innocua;*
differs by at least 1, 2, or 5 amino acids but by no more than 35, 30, 25, 20 or 10 amino acids from amino acids 900-960 of the amino acid sequence of Cas9 *of S. pyogenes, S. thermophilus, S. mutans or L. innocua;* or
is identical to 900-960 of the amino acid sequence of Cas9 of *S. pyogenes, S. thermophilus, S. mutans or L. innocua.*

### Engineered or Altered Cas9 Molecules and Cas9 Polypeptides

Cas9 molecules and Cas9 polypeptides described herein, e.g., naturally occurring Cas9 molecules, can possess any of a number of properties, including: nickase activity, nuclease activity (e.g., endonuclease and/or exonuclease activity); helicase activity; the ability to associate functionally with a gRNA molecule; and the ability to target (or localize to) a site on a nucleic acid (e.g., PAM recognition and specificity). In an embodiment, a Cas9 molecule or Cas9 polypeptide can include all or a subset of these properties. In typical embodiments, a Cas9 molecule or Cas9 polypeptide has the ability to interact with a gRNA molecule and, in concert with the gRNA molecule, localize to a site in a nucleic acid. Other activities, e.g., PAM specificity, cleavage activity, or helicase activity can vary more widely in Cas9 molecules and Cas9 polypeptides.

Cas9 molecules include engineered Cas9 molecules and engineered Cas9 polypeptides (engineered, as used in this context, means merely that the Cas9 molecule or Cas9 polypeptide differs from a reference sequences, and implies no process or origin limitation). An engineered Cas9 molecule or Cas9 polypeptide can comprise altered enzymatic properties, e.g., altered nuclease activity, (as compared with a naturally occurring or other reference Cas9 molecule) or altered helicase activity. As discussed herein, an engineered Cas9 molecule or Cas9 polypeptide can have nickase activity (as opposed to double strand nuclease activity). In an embodiment an engineered Cas9 molecule or Cas9 polypeptide can have an alteration that alters its size, e.g., a deletion of amino acid sequence that reduces its size, e.g., without significant effect on one or more, or any Cas9 activity. In an embodiment, an engineered Cas9 molecule or Cas9 polypeptide can comprise an alteration that affects PAM recognition. E.g., an engineered Cas9 molecule can be altered to recognize a PAM sequence other than that recognized by the endogenous wild-type PI domain. In an embodiment a Cas9 molecule or Cas9 polypeptide can differ in sequence from a naturally occurring Cas9 molecule but not have significant alteration in one or more Cas9 activities.

Cas9 molecules or Cas9 polypeptides with desired properties can be made in a number of ways, e.g., by alteration of a parental, e.g., naturally occurring, Cas9 molecules or Cas9 polypeptides, to provide an altered Cas9 molecule or Cas9 polypeptide having a desired property. For example, one or more mutations or differences relative to a parental Cas9 molecule, e.g., a naturally occurring or engineered Cas9 molecule, can be introduced. Such mutations and differences comprise: substitutions (e.g., conservative substitutions or substitutions of non-essential amino acids); insertions; or deletions. In an embodiment, a Cas9 molecule or Cas9 polypeptide can comprises one or more mutations or differences, e.g., at least 1, 2, 3, 4, 5, 10, 15, 20, 30, 40 or 50 mutations but less than 200, 100, or 80 mutations relative to a reference, e.g., a parental, Cas9 molecule.

In an embodiment, a mutation or mutations do not have a substantial effect on a Cas9 activity, e.g. a Cas9 activity described herein. In an embodiment, a mutation or mutations have a substantial effect on a Cas9 activity, e.g. a Cas9 activity described herein.

### Non-Cleaving and Modified Cleavage Cas9 Molecules and Cas9 Polypeptides

In an embodiment, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule or Cas9 polypeptide can differ from naturally occurring Cas9 molecules, e.g., a Cas9 molecule of *S. pyogenes,* as follows: its ability to modulate, e.g., decreased or increased, cleavage of a double stranded nucleic acid (endonuclease and/or exonuclease activity), e.g., as compared to a naturally occurring Cas9 molecule (e.g., a Cas9 molecule of *S. pyogenes*); its ability to modulate, e.g., decreased or increased, cleavage of a single strand of a nucleic acid, e.g., a non-complementary strand of a nucleic acid molecule or a complementary strand of a nucleic acid molecule (nickase activity), e.g., as compared to a naturally occurring Cas9 molecule (e.g., a Cas9 molecule of *S. pyogenes*); or the ability to cleave a nucleic acid molecule, e.g., a double stranded or single stranded nucleic acid molecule, can be eliminated.

### Modified Cleavage eaCas9 Molecules and eaCas9 Polypeptides

In an embodiment, an eaCas9 molecule or eaCas9 polypeptide comprises one or more of the following activities: cleavage activity associated with an N-terminal RuvC-like domain; cleavage activity associated with an HNH-like domain; cleavage activity associated with an HNH-like domain and cleavage activity associated with an N-terminal RuvC-like domain.

In an embodiment, an eaCas9 molecule or eaCas9 polypeptide comprises an active, or cleavage competent, HNH-like domain (e.g., an HNH-like domain described herein, e.g., SEQ ID NO:17, SEQ ID NO:18, SEQ ID NO:19, SEQ ID NO:20, or SEQ ID NO:21) and an inactive, or cleavage incompetent, N-terminal RuvC-like domain. An exemplary inactive, or cleavage incompetent N-terminal RuvC-like domain can have a mutation of an aspartic acid in an N-terminal RuvC-like domain, e.g., an aspartic acid at position 9 of the consensus sequence disclosed in **Figs. 2A-2G** or an aspartic acid at position 10 of SEQ ID NO:7, e.g., can be substituted with an alanine. In an embodiment, the eaCas9 molecule or eaCas9 polypeptide differs from wild type in the N-terminal RuvC-like domain and does not cleave the target nucleic acid, or cleaves with significantly less efficiency, e.g., less than 20, 10, 5, 1 or. 1 % of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S. pyogenes,* or S. thermophilus. In an embodiment, the reference Cas9 molecule is the naturally occurring Cas9 molecule having the closest sequence identity or homology.

In an embodiment, an eaCas9 molecule or eaCas9 polypeptide comprises an inactive, or cleavage incompetent, HNH domain and an active, or cleavage competent, N-terminal RuvC-like domain (e.g., an N-terminal RuvC-like domain described herein, e.g., SEQ ID NO:8, SEQ ID NO:9, SEQ ID NO:10, SEQ ID NO:11, SEQ ID NO:12, SEQ ID NO:13, SEQ ID NO:14, SEQ ID NO:15, or SEQ ID NO:16). Exemplary inactive, or cleavage incompetent HNH-like domains can have a mutation at one or more of: a histidine in an HNH-like domain, e.g., a histidine shown at position 856 of **Figs. 2A-2G****,** e.g., can be substituted with an alanine; and one or more asparagines in an HNH-like domain, e.g., an asparagine shown at position 870 of **Figs. 2A-2G** and/or at position 879 of **Figs. 2A-2G****,** e.g., can be substituted with an alanine. In an embodiment, the eaCas9 differs from wild type in the HNH-like domain and does not cleave the target nucleic acid, or cleaves with significantly less efficiency, e.g., less than 20, 10, 5, 1 or 0.1% of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S. pyogenes, or S. thermophilus.* In an embodiment, the reference Cas9 molecule is the naturally occurring Cas9 molecule having the closest sequence identity or homology.

In an embodiment, an eaCas9 molecule or eaCas9 polypeptide comprises an inactive, or cleavage incompetent, HNH domain and an active, or cleavage competent, N-terminal RuvC-like domain (e.g., an N-terminal RuvC-like domain described herein, e.g., SEQ ID NO:8, SEQ ID NO:9, SEQ ID NO:10, SEQ ID NO:11, SEQ ID NO:12, SEQ ID NO:13, SEQ ID NO:14, SEQ ID NO:15, or SEQ ID NO:16). Exemplary inactive, or cleavage incompetent HNH-like domains can have a mutation at one or more of: a histidine in an HNH-like domain, e.g., a histidine shown at position 856 of **Figs. 2A-2G****,** e.g., can be substituted with an alanine; and one or more asparagines in an HNH-like domain, e.g., an asparagine shown at position 870 of **Figs. 2A-2G** and/or at position 879 of **Figs. 2A-2G****,** e.g., can be substituted with an alanine. In an embodiment, the eaCas9 differs from wild type in the HNH-like domain and does not cleave the target nucleic acid, or cleaves with significantly less efficiency, e.g., less than 20, 10, 5, 1 or 0.1% of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S. pyogenes, or S. thermophilus.* In an embodiment, the reference Cas9 molecule is the naturally occurring Cas9 molecule having the closest sequence identity or homology.

### Alterations in the Ability to Cleave One or Both Strands of a Target Nucleic Acid

In an embodiment, exemplary Cas9 activities comprise one or more of PAM specificity, cleavage activity, and helicase activity. A mutation(s) can be present, e.g., in: one or more RuvC-like domain, e.g., an N-terminal RuvC-like domain; an HNH-like domain; a region outside the RuvC-like domains and the HNH-like domain. In some embodiments, a mutation(s) is present in a RuvC-like domain, e.g., an N-terminal RuvC-like. In some embodiments, a mutation(s) is present in an HNH-like domain. In some embodiments, mutations are present in both a RuvC-like domain, e.g., an N-terminal RuvC-like domain, and an HNH-like domain.

Exemplary mutations that may be made in the RuvC domain or HNH domain with reference to the *S. pyogenes* sequence include: D10A, E762A, H840A, N854A, N863A and/or D986A.

In an embodiment, a Cas9 molecule or Cas9 polypeptide is an eiCas9 molecule or eiCas9 polypeptide comprising one or more differences in a RuvC domain and/or in an HNH domain as compared to a reference Cas9 molecule, and the eiCas9 molecule or eiCas9 polypeptide does not cleave a nucleic acid, or cleaves with significantly less efficiency than does wildype, e.g., when compared with wild type in a cleavage assay, e.g., as described herein, cuts with less than 50, 25, 10, or 1% of a reference Cas9 molecule, as measured by an assay described herein.

Whether or not a particular sequence, e.g., a substitution, may affect one or more activity, such as targeting activity, cleavage activity, etc, can be evaluated or predicted, e.g., by evaluating whether the mutation is conservative or by the method described in Section IV. In an embodiment, a "non-essential" amino acid residue, as used in the context of a Cas9 molecule, is a residue that can be altered from the wild-type sequence of a Cas9 molecule, e.g., a naturally occurring Cas9 molecule, e.g., an eaCas9 molecule, without abolishing or more preferably, without substantially altering a Cas9 activity (e.g., cleavage activity), whereas changing an "essential" amino acid residue results in a substantial loss of activity (e.g., cleavage activity).

In an embodiment, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule or Cas9 polypeptide can differ from naturally occurring Cas9 molecules, e.g., a Cas9 molecule of *S aureus, S. pyogenes,* or *C. jejuni* as follows: its ability to modulate, e.g., decreased or increased, cleavage of a double stranded break (endonuclease and/or exonuclease activity), e.g., as compared to a naturally occurring Cas9 molecule (e.g., a Cas9 molecule of *S aureus, S. pyogenes,* or *C. jejuni);* its ability to modulate, e.g., decreased or increased, cleavage of a single strand of a nucleic acid, e.g., a non-complementary strand of a nucleic acid molecule or a complementary strand of a nucleic acid molecule (nickase activity), e.g., as compared to a naturally occurring Cas9 molecule (e.g., a Cas9 molecule of *S aureus, S. pyogenes,* or *C. jejuni*); or the ability to cleave a nucleic acid molecule, e.g., a double stranded or single stranded nucleic acid molecule, can be eliminated.

In an embodiment, the altered Cas9 molecule or Cas9 polypeptide is an eaCas9 molecule or eaCas9 polypeptide comprising one or more of the following activities: cleavage activity associated with a RuvC domain; cleavage activity associated with an HNH domain; cleavage activity associated with an HNH domain and cleavage activity associated with a RuvC domain.

In an embodiment, the altered Cas9 molecule or Cas9 polypeptide is an eiCas9 molecule or eaCas9 polypeptide which does not cleave a nucleic acid molecule (either double stranded or single stranded nucleic acid molecules) or cleaves a nucleic acid molecule with significantly less efficiency, e.g., less than 20, 10, 5, 1 or 0.1% of the cleavage activity of a reference Cas9 molecule, e.g., as measured by an assay described herein. The reference Cas9 molecule can be a naturally occurring unmodified Cas9 molecule, e.g., a naturally occurring Cas9 molecule such as a Cas9 molecule of *S. pyogenes, S. thermophilus, S. aureus, C. jejuni* or *N. meningitidis.* In an embodiment, the reference Cas9 molecule is the naturally occurring Cas9 molecule having the closest sequence identity or homology. In an embodiment, the eiCas9 molecule or eiCas9 polypeptide lacks substantial cleavage activity associated with a RuvC domain and cleavage activity associated with an HNH domain.

In an embodiment, the altered Cas9 molecule or Cas9 polypeptide is an eaCas9 molecule or eaCas9 polypeptide comprising the fixed amino acid residues of *S. pyogenes* shown in the consensus sequence disclosed in **Figs. 2A-2G****,** and has one or more amino acids that differ from the amino acid sequence of *S. pyogenes* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G** or SEQ ID NO:7.

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *S*. *pyogenes* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S*. *pyogenes* Cas9 molecule.

In an embodiment, the altered Cas9 molecule or Cas9 polypeptide is an eaCas9 molecule or eaCas9 polypeptide comprising the fixed amino acid residues of *S. thermophilus* shown in the consensus sequence disclosed in **Figs. 2A-2G****,** and has one or more amino acids that differ from the amino acid sequence of *S. thermophilus* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G****.**

In an embodiment the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*"in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *S. thermophilus* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S. thermophilus* Cas9 molecule.

In an embodiment, the altered Cas9 molecule or Cas9 polypeptide is an eaCas9 molecule or eaCas9 polypeptide comprising the fixed amino acid residues of *S. mutans* shown in the consensus sequence disclosed in **Figs. 2A-2G****,** and has one or more amino acids that differ from the amino acid sequence of *S.* mutans (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G****.**

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *S. mutans* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S. mutans* Cas9 molecule.

In an embodiment, the altered Cas9 molecule or Cas9 polypeptide is an eaCas9 molecule or eaCas9 polypeptide comprising the fixed amino acid residues of *L. innocula* shown in the consensus sequence disclosed in **Figs. 2A-2G****,** and has one or more amino acids that differ from the amino acid sequence of *L. innocula* (e.g., has a substitution) at one or more residue (e.g., 2, 3, 5, 10, 15, 20, 30, 50, 70, 80, 90, 100, 200 amino acid residues) represented by an "-"in the consensus sequence disclosed in **Figs. 2A-2G****.**

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *L*. *innocula* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *L*. *innocula* Cas9 molecule.

In an embodiment, the altered Cas9 molecule or Cas9 polypeptide, e.g., an eaCas9 molecule, can be a fusion, e.g., of two of more different Cas9 molecules or Cas9 polypeptides, e.g., of two or more naturally occurring Cas9 molecules of different species. For example, a fragment of a naturally occurring Cas9 molecule of one species can be fused to a fragment of a Cas9 molecule of a second species. As an example, a fragment of Cas9 molecule *of S. pyogenes* comprising an N-terminal RuvC-like domain can be fused to a fragment of Cas9 molecule of a species other than *S. pyogenes* (e.g., *S. thermophilus*) comprising an HNH-like domain.

### Cas9 Molecules With Altered PAM Recognition Or No PAM Recognition

Naturally occurring Cas9 molecules can recognize specific PAM sequences, for example the PAM recognition sequences described above for, e.g., *S. pyogenes, S. thermophilus, S. mutans, S. aureus* and *N. meningitidis.*

In an embodiment, a Cas9 molecule or Cas9 polypeptide has the same PAM specificities as a naturally occurring Cas9 molecule. In other embodiments, a Cas9 molecule or Cas9 polypeptide has a PAM specificity not associated with a naturally occurring Cas9 molecule, or a PAM specificity not associated with the naturally occurring Cas9 molecule to which it has the closest sequence homology. For example, a naturally occurring Cas9 molecule can be altered, e.g., to alter PAM recognition, e.g., to alter the PAM sequence that the Cas9 molecule or Cas9 polypeptide recognizes to decrease off target sites and/or improve specificity; or eliminate a PAM recognition requirement. In an embodiment, a Cas9 molecule can be altered, e.g., to increase length of PAM recognition sequence and/or improve Cas9 specificity to high level of identity, e.g., to decrease off target sites and increase specificity. In an embodiment, the length of the PAM recognition sequence is at least 4, 5, 6, 7, 8, 9, 10 or 15 amino acids in length.

Cas9 molecules or Cas9 polypeptides that recognize different PAM sequences and/or have reduced off-target activity can be generated using directed evolution. Exemplary methods and systems that can be used for directed evolution of Cas9 molecules are described, e.g., in Esvelt et al. Nature 2011, 472(7344): 499-503. Candidate Cas9 molecules can be evaluated, e.g., by methods described in Section IV.

Alterations of the PI domain, which mediates PAM recognition, are discussed below.

### Synthetic Cas9 Molecules and Cas9 Polypeptides with Altered PI Domains

Current genome-editing methods are limited in the diversity of target sequences that can be targeted by the PAM sequence that is recognized by the Cas9 molecule utilized. A synthetic Cas9 molecule (or Syn-Cas9 molecule), or synthetic Cas9 polypeptide (or Syn-Cas9 polypeptide), as that term is used herein, refers to a Cas9 molecule or Cas9 polypeptide that comprises a Cas9 core domain from one bacterial species and a functional altered PI domain, i.e., a PI domain other than that naturally associated with the Cas9 core domain, e.g., from a different bacterial species.

In an embodiment, the altered PI domain recognizes a PAM sequence that is different from the PAM sequence recognized by the naturally-occurring Cas9 from which the Cas9 core domain is derived. In an embodiment, the altered PI domain recognizes the same PAM sequence recognized by the naturally-occurring Cas9 from which the Cas9 core domain is derived, but with different affinity or specificity. A Syn-Cas9 molecule or Syn-Cas9 polypetide can be, respectively, a Syn-eaCas9 molecule or Syn-eaCas9 polypeptide or a Syn-eiCas9 molecule Syn-eiCas9 polypeptide.

An exemplary Syn-Cas9 molecule or Syn-Cas9 polypetide comprises:
a) a Cas9 core domain, e.g., a Cas9 core domain from **Table 100** or **200,** e.g., a *S*. *aureus, S. pyogenes,* or *C. jejuni* Cas9 core domain; and
b) an altered PI domain from a species X Cas9 sequence selected from **Tables 400** and **500.**

In an embodiment, the RKR motif (the PAM binding motif) of said altered PI domain comprises: differences at 1, 2, or 3 amino acid residues; a difference in amino acid sequence at the first, second, or third position; differences in amino acid sequence at the first and second positions, the first and third positions, or the second and third positions; as compared with the sequence of the RKR motif of the native or endogenous PI domain associated with the Cas9 core domain.

In an embodiment, the Cas9 core domain comprises the Cas9 core domain from a species X Cas9 from **Table 100** and said altered PI domain comprises a PI domain from a species Y Cas9 from **Table 100**.

In an embodiment, the RKR motif of the species X Cas9 is other than the RKR motif of the species Y Cas9.

In an embodiment, the RKR motif of the altered PI domain is selected from XXY, XNG, and XNQ.

In an embodiment, the altered PI domain has at least 60, 70, 80, 90, 95, or 100% homology with the amino acid sequence of a naturally occurring PI domain of said species Y from **Table 100.**

In an embodiment, the altered PI domain differs by no more than 50, 40, 30, 25, 20, 15, 10, 5, 4, 3, 2, or 1 amino acid residue from the amino acid sequence of a naturally occurring PI domain of said second species from **Table 100.**

In an embodiment, the Cas9 core domain comprises a *S*. *aureus* core domain and altered PI domain comprises: an *A. denitrificans* PI domain; a *C. jejuni* PI domain; a *H. mustelae* PI domain; or an altered PI domain of species X PI domain, wherein species X is selected from **Table 500.**

In an embodiment, the Cas9 core domain comprises a *S*. *pyogenes* core domain and the altered PI domain comprises: an *A. denitrificans* PI domain; a *C. jejuni* PI domain; a *H. mustelae* PI domain; or an altered PI domain of species X PI domain, wherein species X is selected from **Table 500**.

In an embodiment, the Cas9 core domain comprises a *C. jejuni* core domain and the altered PI domain comprises: an *A. denitrificans* PI domain; a *H. mustelae* PI domain; or an altered PI domain of species X PI domain, wherein species X is selected from **Table 500.**

In an embodiment, the Cas9 molecule or Cas9 polypeptide further comprises a linker disposed between said Cas9 core domain and said altered PI domain.

In an embodiment, the linker comprises: a linker described elsewhere herein disposed between the Cas9 core domain and the heterologous PI domain. Suitable linkers are further described in Section V.

Exemplary altered PI domains for use in Syn-Cas9 molecules are described in **Tables 400** and **500.** The sequences for the 83 Cas9 orthologs referenced in **Tables 400** and **500** are provided in **Table 100. Table 300** provides the Cas9 orthologs with known PAM sequences and the corresponding RKR motif.

In an embodiment, a Syn-Cas9 molecule or Syn-Cas9 polypeptide may also be size-optimized, e.g., the Syn-Cas9 molecule or Syn-Cas9 polypeptide comprises one or more deletions, and optionally one or more linkers disposed between the amino acid residues flanking the deletions. In an embodiment, a Syn-Cas9 molecule or Syn-Cas9 polypeptide comprises a REC deletion.

### Size-Optimized Cas9 Molecules and Cas9 Polypeptides

Engineered Cas9 molecules and engineered Cas9 polypeptides described herein include a Cas9 molecule or Cas9 polypeptide comprising a deletion that reduces the size of the molecule while still retaining desired Cas9 properties, e.g., essentially native conformation, Cas9 nuclease activity, and/or target nucleic acid molecule recognition. Provided herein are Cas9 molecules or Cas9 polypeptides comprising one or more deletions and optionally one or more linkers, wherein a linker is disposed between the amino acid residues that flank the deletion. Methods for identifying suitable deletions in a reference Cas9 molecule, methods for generating Cas9 molecules with a deletion and a linker, and methods for using such Cas9 molecules will be apparent to one of ordinary skill in the art upon review of this document.

A Cas9 molecule, e.g., a *S*. *aureus, S. pyogenes,* or *C. jejuni,* Cas9 molecule, having a deletion is smaller, e.g., has reduced number of amino acids, than the corresponding naturally-occurring Cas9 molecule. The smaller size of the Cas9 molecules allows increased flexibility for delivery methods, and thereby increases utility for genome-editing. A Cas9 molecule or Cas9 polypeptide can comprise one or more deletions that do not substantially affect or decrease the activity of the resultant Cas9 molecules or Cas9 polypeptides described herein. Activities that are retained in the Cas9 molecules or Cas9 polypeptides comprising a deletion as described herein include one or more of the following:
a nickase activity, i.e., the ability to cleave a single strand, e.g., the non-complementary strand or the complementary strand, of a nucleic acid molecule; a double stranded nuclease activity, i.e., the ability to cleave both strands of a double stranded nucleic acid and create a double stranded break, which in an embodiment is the presence of two nickase activities;
an endonuclease activity;
an exonuclease activity;
a helicase activity, i.e., the ability to unwind the helical structure of a double stranded nucleic acid;
and recognition activity of a nucleic acid molecule, e.g., a target nucleic acid or a gRNA.

Activity of the Cas9 molecules or Cas9 polypeptides described herein can be assessed using the activity assays described herein or in the art.

### Identifying regions suitable for deletion

Suitable regions of Cas9 molecules for deletion can be identified by a variety of methods. Naturally-occurring orthologous Cas9 molecules from various bacterial species, e.g., any one of those listed in **Table 100,** can be modeled onto the crystal structure *of S. pyogenes* Cas9 (Nishimasu et al., Cell, 156:935-949, 2014) to examine the level of conservation across the selected Cas9 orthologs with respect to the three-dimensional conformation of the protein. Less conserved or unconserved regions that are spatially located distant from regions involved in Cas9 activity, e.g., interface with the target nucleic acid molecule and/or gRNA, represent regions or domains are candidates for deletion without substantially affecting or decreasing Cas9 activity.

### REC-Optimized Cas9 Molecules and Cas9 Polypeptides

A REC-optimized Cas9 molecule, or a REC-optimized Cas9 polypeptide, as that term is used herein, refers to a Cas9 molecule or Cas9 polypeptide that comprises a deletion in one or both of the REC2 domain and the RE1_{CT} domain (collectively a REC deletion), wherein the deletion comprises at least 10% of the amino acid residues in the cognate domain. A REC-optimized Cas9 molecule or Cas9 polypeptide can be an eaCas9 molecule or eaCas9 polypetide, or an eiCas9 molecule or eiCas9 polypeptide. An exemplary REC-optimized Cas9 molecule or REC-optimized Cas9 polypeptide comprises:
a) a deletion selected from:
   i) a REC2 deletion;
   ii) a REC1_{CT} deletion; or
   iii) a REC1_{SUB} deletion.

Optionally, a linker is disposed between the amino acid residues that flank the deletion. In an embodiment a Cas9 molecule or Cas9 polypeptide includes only one deletion, or only two deletions. A Cas9 molecule or Cas9 polypeptide can comprise a REC2 deletion and a REC1_{CT} deletion. A Cas9 molecule or Cas9 polypeptide can comprise a REC2 deletion and a REC1_{SUB} deletion.

Generally, the deletion will contain at least 10% of the amino acids in the cognate domain, e.g., a REC2 deletion will include at least 10% of the amino acids in the REC2 domain. A deletion can comprise: at least 10, 20, 30, 40, 50, 60, 70, 80, or 90% of the amino acid residues of its cognate domain; all of the amino acid residues of its cognate domain; an amino acid residue outside its cognate domain; a plurality of amino acid residues outside its cognate domain; the amino acid residue immediately N terminal to its cognate domain; the amino acid residue immediately C terminal to its cognate domain; the amino acid residue immediately N terminal to its cognate and the amino acid residue immediately C terminal to its cognate domain; a plurality of, e.g., up to 5, 10, 15, or 20, amino acid residues N terminal to its cognate domain; a plurality of, e.g., up to 5, 10, 15, or 20, amino acid residues C terminal to its cognate domain; a plurality of, e.g., up to 5, 10, 15, or 20, amino acid residues N terminal to to its cognate domain and a plurality of e.g., up to 5, 10, 15, or 20, amino acid residues C terminal to its cognate domain.

In an embodiment, a deletion does not extend beyond: its cognate domain; the N terminal amino acid residue of its cognate domain; the C terminal amino acid residue of its cognate domain.

A REC-optimized Cas9 molecule or REC-optimized Cas9 polypeptide can include a linker disposed between the amino acid residues that flank the deletion. Suitable linkers for use between the amino acid resides that flank a REC deletion in a REC-optimized Cas9 molecule is disclosed in Section V.

In an embodiment, a REC-optimized Cas9 molecule or REC-optimized Cas9 polypeptide comprises an amino acid sequence that, other than any REC deletion and associated linker, has at least 50, 55, 60, 65, 70, 75, 80, 85, 90, 95, 99, or 100% homology with the amino acid sequence of a naturally occurring Cas 9, e.g., a Cas9 molecule described in **Table 100,** e.g., a *S*. *aureus* Cas9 molecule, a *S*. *pyogenes* Cas9 molecule, or a *C. jejuni* Cas9 molecule.

In an embodiment, a a REC-optimized Cas9 molecule or REC-optimized Cas9 polypeptide comprises an amino acid sequence that, other than any REC deletion and associated linker, differs by no more than 1, 2, 3, 4, 5, 6, 7, 8, 9, 10, 15, 20, or 25, amino acid residues from the amino acid sequence of a naturally occurring Cas 9, e.g., a Cas9 molecule described in **Table 100,** e.g., a *S*. *aureus* Cas9 molecule, a *S*. *pyogenes* Cas9 molecule, or a *C. jejuni* Cas9 molecule.

In an embodiment, a REC-optimized Cas9 molecule or REC-optimized Cas9 polypeptide comprises an amino acid sequence that, other than any REC deletion and associate linker, differs by no more than 1, 2, 3, 4, 5, 6, 7, 8, 9, 10, 15, 20, or 25% of the, amino acid residues from the amino acid sequence of a naturally occurring Cas 9, e.g., a Cas9 molecule described in **Table 100,** e.g., a *S*. *aureus* Cas9 molecule, a *S*. *pyogenes* Cas9 molecule, or a *C. jejuni* Cas9 molecule.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

Sequence information for exemplary REC deletions are provided for 83 naturally-occurring Cas9 orthologs in **Table 100.** The amino acid sequences of exemplary Cas9 molecules from different bacterial species are shown below.

**Table 100. Amino Acid Sequence of Cas9 Orthologs**

| | | REC2 | | | REC1_{CT} | | | REC1_{SUB} | | |
|---|---|---|---|---|---|---|---|---|---|---|
| **Species / Composite ID** | **Amino acid sequence** | start (AA pos) | stop (AA pos) | # AA delet ed (n) | start (AA pos) | stop (AA pos) | # AA delet ed (n) | start (AA pos) | stop (AA pos) | #AA delet ed (n) |
| Staphylococcus Aureus tr\|J7RUA5\|J7RU A5_STAAU | SEQ ID NO:304 | 126 | 166 | 41 | 296 | 352 | 57 | 296 | 352 | 57 |
| Streptococcus Pyogenes sp\|Q99ZW21CAS 9_STRP1 | SEQ ID NO:305 | 176 | 314 | 139 | 511 | 592 | 82 | 511 | 592 | 82 |
| Campylobacter jejuni NCTC 11168 gi\|218563121\|ref\| YP_002344900.1 | SEQ ID NO:306 | 137 | 181 | 45 | 316 | 360 | 45 | 316 | 360 | 45 |
| Bacteroides fragilis NCTC 9343 gi\|60683389\|ref\| YP_213533.1\| | SEQ ID NO:307 | 148 | 339 | 192 | 524 | 617 | 84 | 524 | 617 | 84 |
| Bifidobacterium bifidum S17 gi\|310286728\|ref\| YP_003937986. | SEQ ID NO:308 | 173 | 335 | 163 | 516 | 607 | 87 | 516 | 607 | 87 |
| Veillonella atypica ACS-134-V-Col 7a gi\|303229466\|ref\| ZP_07316256.1 | SEQ ID NO:309 | 185 | 339 | 155 | 574 | 663 | 79 | 574 | 663 | 79 |
| Lactobacillus rhamnosus GG gi\|258509199\|ref\| YP_003171950.1 | SEQ ID NO:310 | 169 | 320 | 152 | 559 | 645 | 78 | 559 | 645 | 78 |
| Filifactor alocis ATCC 35896 gi\|374307738\|ref\| YP_005054169.1 | SEQ ID NO:311 | 166 | 314 | 149 | 508 | 592 | 76 | 508 | 592 | 76 |
| Oenococcus kitaharae DSM 17330 gi\|366983953gb\| EHN59352.1\| | SEQ ID NO:312 | 169 | 317 | 149 | 555 | 639 | 80 | 555 | 639 | 80 |
| Fructobacillus fructosus KCTC 3544 gi\|339625081\|ref\| ZP_08660870.1 | SEQ ID NO:313 | 168 | 314 | 147 | 488 | 571 | 76 | 488 | 571 | 76 |
| Catenibacterium mitsuokai DSM 15897 gi\|224543312\|ref\| ZP_03683851.1 | SEQ ID NO:314 | 173 | 318 | 146 | 511 | 594 | 78 | 511 | 594 | 78 |
| Finegoldia magna ATCC 29328 gi\|169823755\|ref\| YP_001691366.1 | SEQ ID NO:315 | 168 | 313 | 146 | 452 | 534 | 77 | 452 | 534 | 77 |
| Coriobacteriumg lomeransPW2 gi\|328956315\|ref\| YP_004373648.1 | SEQ ID NO:316 | 175 | 318 | 144 | 511 | 592 | 82 | 511 | 592 | 82 |
| Eubacterium yurii ATCC 43715 gi\|306821691\|ref\| ZP_07455288.1 | SEQ ID NO:317 | 169 | 310 | 142 | 552 | 633 | 76 | 552 | 633 | 76 |
| Peptoniphilus duerdenii ATCC BAA-1640 gi\|304438954\|ref\| ZP_07398877.1 | SEQ ID NO:318 | 171 | 311 | 141 | 535 | 615 | 76 | 535 | 615 | 76 |
| Acidaminococcu s sp. D21 gi\|227824983\|ref\| ZP_03989815.1 | SEQ ID NO:319 | 167 | 306 | 140 | 511 | 591 | 75 | 511 | 591 | 75 |
| Lactobacillus farciminis KCTC 3681 gi\|336394882\|ref\| ZP_08576281.1 | SEQ ID NO:320 | 171 | 310 | 140 | 542 | 621 | 85 | 542 | 621 | 85 |
| Streptococcus sanguinis SK49 gi\|422884106\|ref\| ZP_16930555.1 | SEQ ID NO:321 | 185 | 324 | 140 | 411 | 490 | 85 | 411 | 490 | 85 |
| Coprococcus catus GD-7 gi\|291520705\|em b\|CBK78998.1\| | SEQ ID NO:322 | 172 | 310 | 139 | 556 | 634 | 76 | 556 | 634 | 76 |
| Streptococcus mutans UA159 gi\|24379809\|ref\| NP_721764.1\| | SEQ ID NO:323 | 176 | 314 | 139 | 392 | 470 | 84 | 392 | 470 | 84 |
| Streptococcus pyogenes M1 GAS gi\|13622193\|gb\| AAK33936.1\| | SEQ ID NO:324 | 176 | 314 | 139 | 523 | 600 | 82 | 523 | 600 | 82 |
| Streptococcus thermophilus LMD-9 gi\|116628213\|ref] YP_820832.1\| | SEQ ID NO:325 | 176 | 314 | 139 | 481 | 558 | 81 | 481 | 558 | 81 |
| Fusobacteriumnu cleatum ATCC49256 gi\|34762592\|ref\| ZP_00143587.1\| | SEQ ID NO:326 | 171 | 308 | 138 | 537 | 614 | 76 | 537 | 614 | 76 |
| Planococcus antarcticus DSM 14505 gi\|389815359\|ref\| ZP_10206685.1 | SEQ ID NO:327 | 162 | 299 | 138 | 538 | 614 | 94 | 538 | 614 | 94 |
| Treponema denticola ATCC 35405 gi\|42525843\|ref\| NP_970941.1\| | SEQ ID NO:328 | 169 | 305 | 137 | 524 | 600 | 81 | 524 | 600 | 81 |
| Solobacterium moorei F0204 gi\|320528778\|ref\| ZP_08029929.1 | SEQ ID NO:329 | 179 | 314 | 136 | 544 | 619 | 77 | 544 | 619 | 77 |
| Staphylococcus pseudintermediu s ED99 gi\|323463801\|gb\| ADX75954.1\| | SEQ ID NO:330 | 164 | 299 | 136 | 531 | 606 | 92 | 531 | 606 | 92 |
| Flavobacterium branchiophilum FL-15 gi\|347536497\|ref\| YP_004843922.1 | SEQ ID NO:331 | 162 | 286 | 125 | 538 | 613 | 63 | 538 | 613 | 63 |
| Ignavibacterium album JCM 16511 gi\|385811609\|ref\| YP_005848005.1 | SEQ ID NO:332 | 223 | 329 | 107 | 357 | 432 | 90 | 357 | 432 | 90 |
| Bergeyella zoohelcum ATCC 43767 gi\|423317190\|ref\| ZP_17295095.1 | SEQ ID NO:333 | 165 | 261 | 97 | 529 | 604 | 56 | 529 | 604 | 56 |
| Nitrobacter hamburgensis X14 gi\|92109262\|ref\| YP_571550.1\| | SEQ ID NO:334 | 169 | 253 | 85 | 536 | 611 | 48 | 536 | 611 | 48 |
| Odoribacter laneus YIT 12061 gi\|374384763\|ref\| ZP_09642280.1 | SEQ ID NO:335 | 164 | 242 | 79 | 535 | 610 | 63 | 535 | 610 | 63 |
| Legionella pneumophila str. Paris gi\|54296138\|ref\| YP_122507.1\| | SEQ ID NO:336 | 164 | 239 | 76 | 402 | 476 | 67 | 402 | 476 | 67 |
| Bacteroides sp. 20 3 gi\|301311869\|ref\| ZP_07217791.1 | SEQ ID NO:337 | 198 | 269 | 72 | 530 | 604 | 83 | 530 | 604 | 83 |
| Akkermansia muciniphila ATCC BAA-835 gi\|187736489\|ref\| YP_001878601. | SEQ ID NO:338 | 136 | 202 | 67 | 348 | 418 | 62 | 348 | 418 | 62 |
| Prevotella sp. C561 gi\|345885718\|ref\| ZP_08837074.1 | SEQ ID NO:339 | 184 | 250 | 67 | 357 | 425 | 78 | 357 | 425 | 78 |
| Wolinella succinogenes DSM 1740 gi\|34557932\|ref\| NP_907747.1\| | SEQ ID NO:340 | 157 | 218 | 36 | 401 | 468 | 60 | 401 | 468 | 60 |
| Alicyclobacillus hesperidum URH17-3-68 gi\|403744858\|ref\| ZP_10953934.1 | SEQ ID NO:341 | 142 | 196 | 55 | 416 | 482 | 61 | 416 | 482 | 61 |
| Caenispirillum salinarum AK4 gi\|427429481\|ref\| ZP_18919511.1 | SEQ ID NO:342 | 161 | 214 | 54 | 330 | 393 | 68 | 330 | 393 | 68 |
| Eubacterium rectale ATCC 33656 gi\|238924075\|ref\| YP_002937591.1 | SEQ ID NO:343 | 133 | 185 | 53 | 322 | 384 | 60 | 322 | 384 | 60 |
| Mycoplasma synoviae 53 gi\|71894592\|ref\| YP_278700.1\| | SEQ ID NO:344 | 187 | 239 | 53 | 319 | 381 | 80 | 319 | 381 | 80 |
| Porphyromonas sp. oral taxon 279 str. F0450 gi\|402847315\|ref\| ZP_10895610.1 | SEQ ID NO:345 | 150 | 202 | 53 | 309 | 371 | 60 | 309 | 371 | 60 |
| Streptococcus thermophilus LMD-9 gi\|116627542\|ref\| YP_820161.1\| | SEQ ID NO:346 | 127 | 178 | 139 | 424 | 486 | 81 | 424 | 486 | 81 |
| Roseburia inulinivorans DSM 16841 gi\|225377804\|ref\| ZP_03755025.1 | SEQ ID NO:347 | 154 | 204 | 51 | 318 | 380 | 69 | 318 | 380 | 69 |
| Methylosinus trichosporium OB3b gi\|296446027\|ref\| ZP_06887976.1 | SEQ ID NO:348 | 144 | 193 | 50 | 426 | 488 | 64 | 426 | 488 | 64 |
| Ruminococcus albus 8 gi\|325677756\|ref\| ZP_08157403.1 | SEQ ID NO:349 | 139 | 187 | 49 | 351 | 412 | 55 | 351 | 412 | 55 |
| Bifidobacterium longum DJO10A gi\|189440764\|ref\| YP_001955845. | SEQ ID NO:350 | 183 | 230 | 48 | 370 | 431 | 44 | 370 | 431 | 44 |
| Enterococcus faecalis TX0012 gi\|315149830\|gb\| EFT93846.1\| | SEQ ID NO:351 | 123 | 170 | 48 | 327 | 387 | 60 | 327 | 387 | 60 |
| Mycoplasma mobile 163K gi\|47458868\|ref\| YP_015730.1\| | SEQ ID NO:352 | 179 | 226 | 48 | 314 | 374 | 79 | 314 | 374 | 79 |
| Actinomyces coleocanis DSM 15436 gi\|227494853\|ref\| ZP_03925169.1 | SEQ ID NO:353 | 147 | 193 | 47 | 358 | 418 | 40 | 358 | 418 | 40 |
| Dinoroseobacter shibae DFL 12 gi\|159042956\|ref\| YP_001531750.1 | SEQ ID NO:354 | 138 | 184 | 47 | 338 | 398 | 48 | 338 | 398 | 48 |
| Actinomyces sp. oral taxon 180 str. F0310 gi\|315605738\|ref\| ZP_07880770.1 | SEQ ID NO:355 | 183 | 228 | 46 | 349 | 409 | 40 | 349 | 409 | 40 |
| Alcanivorax sp. W11-5 gi\|407803669\|ref\| ZP_11150502.1 | SEQ ID NO:356 | 139 | 183 | 45 | 344 | 404 | 61 | 344 | 404 | 61 |
| Aminomonas paucivorans DSM 12260 gi\|312879015\|ref\| ZP_07738815.1 | SEQ ID NO:357 | 134 | 178 | 45 | 341 | 401 | 63 | 341 | 401 | 63 |
| Mycoplasma canis PG 14 gi\|384393286\|gb\| EIE39736.1\| | SEQ ID NO:358 | 139 | 183 | 45 | 319 | 379 | 76 | 319 | 379 | 76 |
| Lactobacillus coryniformis KCTC 3535 gi\|336393381\|ref\| ZP_08574780.1 | SEQ ID NO:359 | 141 | 184 | 44 | 328 | 387 | 61 | 328 | 387 | 61 |
| Elusimicrobium minutum Pei191 gi\|187250660\|ref\| YP_001875142.1 | SEQ ID NO:360 | 177 | 219 | 43 | 322 | 381 | 47 | 322 | 381 | 47 |
| Neisseria meningitidis Z2491 gi\|218767588\|ref\| YP_002342100.1 | SEQ ID NO:361 | 147 | 189 | 43 | 360 | 419 | 61 | 360 | 419 | 61 |
| Pasteurella multocida str. Pm70 gi\|15602992\|ref\| NP_246064.1\| | SEQ ID NO:362 | 139 | 181 | 43 | 319 | 378 | 61 | 319 | 378 | 61 |
| Rhodovulum sp. PH10 gi\|402849997\|ref\| ZP_10898214.1 | SEQ ID NO:363 | 141 | 183 | 43 | 319 | 378 | 48 | 319 | 378 | 48 |
| Eubacterium dolichum DSM 3991 gi\|160915782\|ref\| ZP_02077990.1 | SEQ ID NO:364 | 131 | 172 | 42 | 303 | 361 | 59 | 303 | 361 | 59 |
| Nitratifractor salsuginis DSM 16511 gi\|319957206\|ref\| YP_004168469.1 | SEQ ID NO:365 | 143 | 184 | 42 | 347 | 404 | 61 | 347 | 404 | 61 |
| Rhodospirillum rubrum ATCC 11170 gi\|83591793\|ref\| YP_425545.1\| | SEQ ID NO:366 | 139 | 180 | 42 | 314 | 371 | 55 | 314 | 371 | 55 |
| Clostridium cellulolyticum H10 gi\|220930482\|ref\| YP_002507391.1 | SEQ ID NO:367 | 137 | 176 | 40 | 320 | 376 | 61 | 320 | 376 | 61 |
| Helicobacter mustelae 12198 gi\|291276265\|ref\| YP_003516037.1 | SEQ ID NO:368 | 148 | 187 | 40 | 298 | 354 | 48 | 298 | 354 | 48 |
| Ilyobacter polytropus DSM 2926 gi\|310780384\|ref\| YP_003968716.1 | SEQ ID NO:369 | 134 | 173 | 40 | 462 | 517 | 63 | 462 | 517 | 63 |
| Sphaerochaeta globus str. Buddy gi\|325972003\|ref\| YP_004248194.1 | SEQ ID NO:370 | 163 | 202 | 40 | 335 | 389 | 45 | 335 | 389 | 45 |
| Staphylococcus lugdunensis M23590 gi\|315659848\|ref\| ZP_07912707.1 | SEQ ID NO:371 | 128 | 167 | 40 | 337 | 391 | 57 | 337 | 391 | 57 |
| Treponema sp. JC4 gi\|384109266\|ref\| ZP_10010146.1 | SEQ ID NO:372 | 144 | 183 | 40 | 328 | 382 | 63 | 328 | 382 | 63 |
| uncultured delta proteobacterium HF0070 07E19 gi\|297182908\|gb\| ADI19058.1\| | SEQ ID NO:373 | 154 | 193 | 40 | 313 | 365 | 55 | 313 | 365 | 55 |
| Alicycliphilus denitrificans K601 gi\|330822845\|ref\| YP_004386148.1 | SEQ ID NO:374 | 140 | 178 | 39 | 317 | 366 | 48 | 317 | 366 | 48 |
| Azospirillum sp. B510 gi\|288957741\|ref\| YP_003448082.1 | SEQ ID NO:375 | 205 | 243 | 39 | 342 | 389 | 46 | 342 | 389 | 46 |
| Bradyrhizobium sp. BTAi1 gi\|148255343ref\| YP_001239928.1 | SEQ ID NO:376 | 143 | 181 | 39 | 323 | 370 | 48 | 323 | 370 | 48 |
| Parvibaculum lavamentivorans DS-1 gi\|154250555\|ref\| YP_001411379.1 | SEQ ID NO:377 | 138 | 176 | 39 | 327 | 374 | 58 | 327 | 374 | 58 |
| Prevotella timonensis CRIS 5C-B1 gi\|282880052\|ref\| ZP_06288774.1 | SEQ ID NO:378 | 170 | 208 | 39 | 328 | 375 | 61 | 328 | 375 | 61 |
| Bacillus smithii 7 3 47FAA gi\|365156657\|ref\| ZP_09352959.1 | SEQ ID NO:379 | 134 | 171 | 38 | 401 | 448 | 63 | 401 | 448 | 63 |
| Cand. Puniceispirillum marinum IMCC1322 gi\|294086111\|ref\| YP_003552871.1 | SEQ ID NO:380 | 135 | 172 | 38 | 344 | 391 | 53 | 344 | 391 | 53 |
| Barnesiella intestinihominis YIT 11860 gi\|404487228\|ref\| ZP_11022414.1 | SEQ ID NO:381 | 140 | 176 | 37 | 371 | 417 | 60 | 371 | 417 | 60 |
| Ralstonia syzygii R24 gi\|344171927\|em b\|CCA84553.1\| | SEQ ID NO:382 | 140 | 176 | 37 | 395 | 440 | 50 | 395 | 440 | 50 |
| Wolinella succinogenes DSM 1740 gi\|34557790\|ref\| NP_907605.1\| | SEQ ID NO:383 | 145 | 180 | 36 | 348 | 392 | 60 | 348 | 392 | 60 |
| Mycoplasma gallisepticum str. F gi\|284931710\|gb\| ADC31648.1\| | SEQ ID NO:384 | 144 | 177 | 34 | 373 | 416 | 71 | 373 | 416 | 71 |
| Acidothermus cellulolyticus 11B gi\|117929158\|ref\| YP_873709.1\| | SEQ ID NO:385 | 150 | 182 | 33 | 341 | 380 | 58 | 341 | 380 | 58 |
| Mycoplasma ovipneumoniae SC01 gi\|363542550\|ref\| ZP_09312133.1 | SEQ ID NO:386 | 156 | 184 | 29 | 381 | 420 | 62 | 381 | 420 | 62 |

**Table 200. Amino Acid Sequence of Cas9 Core Domains**

| **Strain Name** | **Cas9 Start (AA pos)** | **Cas9 Stop (AA pos)** |
|---|---|---|
| | **Start and Stop numbers refer to the sequence in Table 100** | |
| Staphylococcus Aureus | 1 | 772 |
| Streptococcus Pyogenes | 1 | 1099 |
| Campulobacter Jejuni | 1 | 741 |

**Table 300. Identified PAM sequences and corresponding RKR motifs.**

| **Strain Name** | **PAM sequence (NA)** | **RKR motif (AA)** |
|---|---|---|
| Streptococcus pyogenes | NGG | RKR |
| Streptococcus mutans | NGG | RKR |
| Streptococcus thermophilus A | NGGNG | RYR |
| Treponema denticola | NAAAAN | VAK |
| Streptococcus thermophilus B | NNAAAAW | IYK |
| Campylobacter jejuni | NNNNACA | NLK |
| Pasteurella multocida | GNNNCNNA | KDG |
| Neisseria meningitidis | NNNNGATT or | IGK |
| | NNNNGCTT | |
| Staphylococcus aureus | NNGRRV (R = A or G; V = A, G or C) or NNGRRT (R = A or G) | NDK |

PI domains are provided in **Tables 400** and **500.**

**Table 400. Altered PI Domains**

| **Strain Name** | **PI Start (AA pos)** | **PI Stop (AA pos)** | **Length of PI (AA)** | **RKR motif (AA)** |
|---|---|---|---|---|
| | **Start and Stop numbers refer to the sequences in Table 100** | | | |
| Alicycliphilus denitrificans K601 | 837 | 1029 | 193 | --Y |
| Campylobacter jejuni NCTC 11168 | 741 | 984 | 244 | -NG |
| Helicobacter mustelae 12198 | 771 | 1024 | 254 | -NQ |

**Table 500. Other Altered PI Domains**

| **Strain Name** | **PI Start (AA pos)** | **PI Stop (AA pos)** | **Length of PI (AA)** | **RKR motif (AA)** |
|---|---|---|---|---|
| | **Start and Stop numbers refer to the sequences in Table 100** | | | |
| Akkermansia muciniphila ATCC BAA-835 | 871 | 1101 | 231 | ALK |
| Ralstonia syzygii R24 | 821 | 1062 | 242 | APY |
| Cand. Puniceispirillum marinum IMCC1322 | 815 | 1035 | 221 | AYK |
| Fructobacillus fructosus KCTC 3544 | 1074 | 1323 | 250 | DGN |
| Eubacterium yurii ATCC 43715 | 1107 | 1391 | 285 | DGY |
| Eubacterium dolichum DSM 3991 | 779 | 1096 | 318 | DKK |
| Dinoroseobacter shibae DFL 12 | 851 | 1079 | 229 | DPI |
| Clostridium cellulolyticum H10 | 767 | 1021 | 255 | EGK |
| Pasteurella multocida str. Pm70 | 815 | 1056 | 242 | ENN |
| Mycoplasma canis PG 14 | 907 | 1233 | 327 | EPK |
| Porphyromonas sp. oral taxon 279 str. F0450 | 935 | 1197 | 263 | EPT |
| Filifactor alocis ATCC 35896 | 1094 | 1365 | 272 | EVD |
| Aminomonas paucivorans DSM 12260 | 801 | 1052 | 252 | EVY |
| Wolinella succinogenes DSM 1740 | 1034 | 1409 | 376 | EYK |
| Oenococcus kitaharae DSM 17330 | 1119 | 1389 | 271 | GAL |
| CoriobacteriumglomeransPW2 | 1126 | 1384 | 259 | GDR |
| Peptoniphilus duerdenii ATCC BAA-1640 | 1091 | 1364 | 274 | GDS |
| Bifidobacterium bifidum S17 | 1138 | 1420 | 283 | GGL |
| Alicyclobacillus hesperidum URH17-3-68 | 876 | 1146 | 271 | GGR |
| Roseburia inulinivorans DSM 16841 | 895 | 1152 | 258 | GGT |
| Actinomyces coleocanis DSM 15436 | 843 | 1105 | 263 | GKK |
| Odoribacter laneus YIT 12061 | 1103 | 1498 | 396 | GKV |
| Coprococcus catus GD-7 | 1063 | 1338 | 276 | GNQ |
| Enterococcus faecalis TX0012 | 829 | 1150 | 322 | GRK |
| Bacillus smithii 7 3 47FAA | 809 | 1088 | 280 | GSK |
| Legionella pneumophila str. Paris | 1021 | 1372 | 352 | GTM |
| Bacteroides fragilis NCTC 9343 | 1140 | 1436 | 297 | IPV |
| Mycoplasma ovipneumoniae SC01 | 923 | 1265 | 343 | IRI |
| Actinomyces sp. oral taxon 180 str. F0310 | 895 | 1181 | 287 | KEK |
| Treponema sp. JC4 | 832 | 1062 | 231 | KIS |
| Fusobacteriumnucleatum ATCC49256 | 1073 | 1374 | 302 | KKV |
| Lactobacillus farciminis KCTC 3681 | 1101 | 1356 | 256 | KKV |
| Nitratifractor salsuginis DSM 16511 | 840 | 1132 | 293 | KMR |
| Lactobacillus coryniformis KCTC 3535 | 850 | 1119 | 270 | KNK |
| Mycoplasma mobile 163K | 916 | 1236 | 321 | KNY |
| Flavobacterium branchiophilum FL-15 | 1182 | 1473 | 292 | KQK |
| Prevotella timonensis CRIS 5C-B1 | 957 | 1218 | 262 | KQQ |
| Methylosinus trichosporium OB3b | 830 | 1082 | 253 | KRP |
| Prevotella sp. C561 | 1099 | 1424 | 326 | KRY |
| Mycoplasma gallisepticum str. F | 911 | 1269 | 359 | KTA |
| Lactobacillus rhamnosus GG | 1077 | 1363 | 287 | KYG |
| Wolinella succinogenes DSM 1740 | 811 | 1059 | 249 | LPN |
| Streptococcus thermophilus LMD-9 | 1099 | 1388 | 290 | MLA |
| Treponema denticola ATCC 35405 | 1092 | 1395 | 304 | NDS |
| Bergeyella zoohelcum ATCC 43767 | 1098 | 1415 | 318 | NEK |
| Veillonella atypica ACS-134-V-Col7a | 1107 | 1398 | 292 | NGF |
| Neisseria meningitidis Z2491 | 835 | 1082 | 248 | NHN |
| Ignavibacterium album JCM 16511 | 1296 | 1688 | 393 | NKK |
| Ruminococcus albus 8 | 853 | 1156 | 304 | NNF |
| Streptococcus thermophilus LMD-9 | 811 | 1121 | 311 | NNK |
| Barnesiella intestinihominis YIT 11860 | 871 | 1153 | 283 | NPV |
| Azospirillum sp. B510 | 911 | 1168 | 258 | PFH |
| Rhodospirillum rubrum ATCC 11170 | 863 | 1173 | 311 | PRG |
| Planococcus antarcticus DSM 14505 | 1087 | 1333 | 247 | PYY |
| Staphylococcus pseudintermedius ED99 | 1073 | 1334 | 262 | QIV |
| Alcanivorax sp. W11-5 | 843 | 1113 | 271 | RIE |
| Bradyrhizobium sp. BTAi1 | 811 | 1064 | 254 | RIY |
| Streptococcus pyogenes M1 GAS | 1099 | 1368 | 270 | RKR |
| Streptococcus mutans UA159 | 1078 | 1345 | 268 | RKR |
| Streptococcus Pyogenes | 1099 | 1368 | 270 | RKR |
| Bacteroides sp. 20 3 | 1147 | 1517 | 371 | RNI |
| *S. aureus* | 772 | 1053 | 282 | RNK |
| Solobacterium moorei F0204 | 1062 | 1327 | 266 | RSG |
| Finegoldia magna ATCC 29328 | 1081 | 1348 | 268 | RTE |
| uncultured delta proteobacterium HF0070 07E19 | 770 | 1011 | 242 | SGG |
| Acidaminococcus sp. D21 | 1064 | 1358 | 295 | SIG |
| Eubacterium rectale ATCC 33656 | 824 | 1114 | 291 | SKK |
| Caenispirillum salinarum AK4 | 1048 | 1442 | 395 | SLV |
| Acidothermus cellulolyticus 11B | 830 | 1138 | 309 | SPS |
| Catenibacterium mitsuokai DSM 15897 | 1068 | 1329 | 262 | SPT |
| Parvibaculum lavamentivorans DS-1 | 827 | 1037 | 211 | TGN |
| Staphylococcus lugdunensis M23590 | 772 | 1054 | 283 | TKK |
| Streptococcus sanguinis SK49 | 1123 | 1421 | 299 | TRM |
| Elusimicrobium minutum Pei191 | 910 | 1195 | 286 | TTG |
| Nitrobacter hamburgensis X14 | 914 | 1166 | 253 | VAY |
| Mycoplasma synoviae 53 | 991 | 1314 | 324 | VGF |
| Sphaerochaeta globus str. Buddy | 877 | 1179 | 303 | VKG |
| Ilyobacter polytropus DSM 2926 | 837 | 1092 | 256 | VNG |
| Rhodovulum sp. PH10 | 821 | 1059 | 239 | VPY |
| Bifidobacterium longum DJO10A | 904 | 1187 | 284 | VRK |

### Nucleic Acids Encoding Cas9 Molecules

Nucleic acids encoding the Cas9 molecules or Cas9 polypeptides, e.g., an eaCas9 molecule or eaCas9 polypeptide, are provided herein.

Exemplary nucleic acids encoding Cas9 molecules or Cas9 polypeptides are described in Cong et al., Science 2013, 399(6121):819-823; Wang et al., Cell 2013, 153(4):910-918; Mali et al., Science 2013, 399(6121):823-826; Jinek et al., Science 2012, 337(6096):816-821. Another exemplary nucleic acid encoding a Cas9 molecule or Cas9 polypeptide is shown in black in **Fig. 8****.**

In an embodiment, a nucleic acid encoding a Cas9 molecule or Cas9 polypeptide can be a synthetic nucleic acid sequence. For example, the synthetic nucleic acid molecule can be chemically modified, e.g., as described in Section VIII. In an embodiment, the Cas9 mRNA has one or more (e.g., all of the following properties: it is capped, polyadenylated, substituted with 5-methylcytidine and/or pseudouridine.

In addition, or alternatively, the synthetic nucleic acid sequence can be codon optimized, e.g., at least one non-common codon or less-common codon has been replaced by a common codon. For example, the synthetic nucleic acid can direct the synthesis of an optimized messenger mRNA, e.g., optimized for expression in a mammalian expression system, e.g., described herein. In addition, or alternatively, a nucleic acid encoding a Cas9 molecule or Cas9 polypeptide may comprise a nuclear localization sequence (NLS). Nuclear localization sequences are known in the art. An exemplary codon optimized nucleic acid sequence encoding a Cas9 molecule of *S*. *pyogenes* is provided as SEQ ID NO:22 with the corresponding amino acid sequence as SEQ ID NO:23. An exemplary codon optimized nucleic acid sequence encoding a Cas9 molecule of *N. meningitidis* is provided as SEQ ID NO:24 with the corresponding amino acid sequence as SEQ ID NO:25. Exemplary codon optimized nucleic acid sequences encoding a Cas9 molecule of *S*. *aureus* are provided as SEQ ID NO:39, SEQ ID NO:415, and SEQ ID NO:416 with the corresponding amino acid sequence as SEQ ID NO:26. An exemplary codon optimized nucleic acid sequence encoding a Cas9 molecule of *S. aureus* Cas9 nickase D10A is provided as SEQ ID NO:417. An exemplary codon optimized nucleic acid sequence encoding a Cas9 molecule of *S*. *aureus* Cas9 nickase N580A is provided as SEQ ID NO:418. If any of the above Cas9 sequences are fused with a peptide or polypeptide at the C-terminus, it is understood that the stop codon will be removed.

### Other Cas Molecules and Cas Polypeptides

Various types of Cas molecules or Cas polypeptides can be used to practice the inventions disclosed herein. In some embodiments, Cas molecules of Type II Cas systems are used. In other embodiments, Cas molecules of other Cas systems are used. For example, Type I or Type III Cas molecules may be used. Exemplary Cas molecules (and Cas systems) are described, e.g., in Haft et al., PLoS Computational Biology 2005, 1(6): e60 and Makarova et al., Nature Review Microbiology 2011, 9:467-477 Exemplary Cas molecules (and Cas systems) are also shown in **Table 600.**

**Table 600. Cas Systems**

| **Gene name^{‡}** | **System type or subtype** | **Name from Haft *et al*.^{§}** | **Structure of encoded protein (PDB accessions)** | **Families (and superfamily) of encoded protein^{#**}** | **Representatives** |
|---|---|---|---|---|---|
| *cas1* | • Type I | *cas1* | 3GOD, 3LFX and 2YZS | COG1518 | SERP2463, SPy1047 and *ygbT* |
| | • Type II | | | | |
| | • Type III | | | | |
| *cas2* | • Type I | *cas2* | 2IVY, 2I8E and 3EXC | COG1343 and COG3512 | SERP2462, |
| | • Type II | | | | SPy1048, |
| | • Type III | | | | SPy 1723 (N-terminal domain) and *ygbF* |
| *cas3'* | • Type I^{‡‡} | *cas3* | NA | COG1203 | APE1232 and *ygcB* |
| *cas3"* | • Subtype I-A | NA | NA | COG2254 | APE1231 and BH0336 |
| | • Subtype I-B | | | | |
| *cas4* | • Subtype I-A | *cas4* and *csa1* | NA | COG1468 | APE1239 and BH0340 |
| | • Subtype I-B | | | | |
| | • Subtype I-C | | | | |
| | • Subtype I-D | | | | |
| | • Subtype II-B | | | | |
| *cas5* | • Subtype I-A | *cas5a*, *cas5d*, *cas5e, cas5h, cas5p, cas5t* and *cmx5* | 3KG4 | COG1688 (RAMP) | APE1234, |
| | • Subtype I-B | | | | BH0337, *devS* and *ygcI* |
| | • Subtype I-C | | | | |
| | • Subtype I-E | | | | |
| *cas6* | • Subtype I-A | *cas6* and *cmx6* | 3I4H | COG1583 and COG5551 (RAMP) | PF1131 and slr7014 |
| | • Subtype I-B | | | | |
| | • Subtype I-D | | | | |
| | • Subtype III-A | | | | |
| | • Subtype III-B | | | | |
| *cas6e* | • Subtype I-E | *cse3* | 1WJ9 | (RAMP) | *ygcH* |
| *cas6f* | • Subtype I-F | *csy4* | 2XLJ | (RAMP) | y1727 |
| *cas7* | • Subtype I-A | *csa2*, *csd2, cse4, csh2, csp1* and *cst2* | NA | COG1857 and COG3649 (RAMP) | *devR* and *ygcJ* |
| | • Subtype I-B | | | | |
| | • Subtype I-C | | | | |
| | • Subtype I-E | | | | |
| *cas8a 1* | • Subtype I-A^{‡‡} | *cmx1, cst1, csx8, csx13* and CXXC-CX XC | NA | BH0338-like | LA3191^{§§} and PG2018^{§§} |
| *cas8a* 2 | • Subtype I-A^{‡‡} | *csa4* and *csx9* | NA | PH0918 | AF0070, AF1873, MJ0385, PF0637, PH0918 and SSO1401 |
| *cas8b* | • Subtype I-B^{‡‡} | *csh1* and TM1802 | NA | BH0338-like | MTH1090 and TM1802 |
| *cas8c* | • Subtype I-C^{‡‡} | *csd1* and *csp2* | NA | BH0338-like | BH0338 |
| *cas9* | • Type II^{‡‡} | *csn1* and *csx12* | NA | COG3513 | FTN_0757 and SPy1046 |
| *cas10* | • Type III^{‡‡} | *cmr2, csm1* and *csx11* | NA | COG1353 | MTH326, Rv2823c^{§§} and TM1794^{§§} |
| *cas10 d* | • Subtype I-D^{‡‡} | *csc3* | NA | COG1353 | slr7011 |
| *csy1* | • Subtype I-F^{‡‡} | *csy1* | NA | y1724-like | y1724 |
| *csy2* | • Subtype I-F | *csy2* | NA | (RAMP) | y1725 |
| *csy3* | • Subtype I-F | *csy3* | NA | (RAMP) | y1726 |
| *cse1* | • Subtype I-E^{‡‡} | *cse1* | NA | YgcL-like | *ygcL* |
| *cse2* | • Subtype I-E | *cse2* | 2ZCA | YgcK-like | *ygcK* |
| *csc1* | • Subtype I-D | *csc1* | NA | alr1563-like (RAMP) | alr1563 |
| *csc2* | • Subtype I-D | *csc1* and *csc2* | NA | COG1337 (RAMP) | slr7012 |
| *csa5* | • Subtype I-A | *csa5* | NA | AF1870 | AF1870, MJ0380, PF0643 and SSO1398 |
| *csn2* | • Subtype II-A | *csn2* | NA | SPy 1049-like | SPy1049 |
| *csm2* | • Subtype III-^{‡‡} | *csm2* | NA | COG1421 | MTH1081 and SERP2460 |
| *csm3* | • Subtype III-A | *csc2* and *csm3* | NA | COG1337 (RAMP) | MTH1080 and SERP2459 |
| *csm4* | • Subtype III-A | *csm4* | NA | COG1567 (RAMP) | MTH1079 and SERP2458 |
| *csm5* | • Subtype III-A | *csm5* | NA | COG1332 (RAMP) | MTH1078 and SERP2457 |
| *csm6* | • Subtype III-A | APE2256 and *csm6* | 2WTE | COG1517 | APE2256 and SSO1445 |
| *cmr1* | • Subtype III-B | *cmr1* | NA | COG1367 (RAMP) | PF1130 |
| *cmr3* | • Subtype III-B | *cmr3* | NA | COG1769 (RAMP) | PF1128 |
| *cmr4* | • Subtype III-B | *cmr4* | NA | COG1336 (RAMP) | PF1126 |
| *cmr5* | • Subtype III-B^{‡‡} | *cmr5* | 2ZOP and 20EB | COG3337 | MTH324 and PF1125 |
| *cmr6* | • Subtype III-B | *cmr6* | NA | COG1604 (RAMP) | PF1124 |
| *csb1* | • Subtype I-U | GSU0053 | NA | (RAMP) | Balac_1306 and GSU0053 |
| *csb2* | • Subtype I-U^{§§} | NA | NA | (RAMP) | Balac_1305 and GSU0054 |
| *csb3* | • Subtype I-U | NA | NA | (RAMP) | Balac_1303^{§§} |
| *csx17* | • Subtype I-U | NA | NA | NA | Btus_2683 |
| *csx14* | • Subtype I-U | NA | NA | NA | GSU0052 |
| *csx10* | • Subtype I-U | *csx10* | NA | (RAMP) | Caur_2274 |
| *csx16* | • Subtype III-U | VVA1548 | NA | NA | VVA1548 |
| *csaX* | • Subtype III-U | *csaX* | NA | NA | SSO1438 |
| *csx3* | • Subtype III-U | *csx3* | NA | NA | AF1864 |
| *csx1* | • Subtype III-U | *csa3, csx1, csx2,* DXTHG, NE0113 and TIGR0271 0 | 1XMX and 2171 | COG1517 and COG4006 | MJ1666, NE0113, PF1127 and TM1812 |
| *csx15* | • Unknown | NA | NA | TTE2665 | TTE2665 |
| *csf1* | • Type U | *csf1* | NA | NA | AFE_1038 |
| *csf2* | • Type U | *csf2* | NA | (RAMP) | AFE_1039 |
| *csf3* | • Type U | *csf3* | NA | (RAMP) | AFE_1040 |
| *csf4* | • Type U | *csf4* | NA | NA | AFE_1037 |

### IV. Functional Analysis of Candidate Molecules

Candidate Cas9 molecules, candidate gRNA molecules, candidate Cas9 molecule/gRNA molecule complexes, can be evaluated by art-known methods or as described herein. For example, exemplary methods for evaluating the endonuclease activity of Cas9 molecule are described, e.g., in Jinek et al., SCIENCE 2012, 337(6096):816-821.

### Binding and Cleavage Assay: Testing the endonuclease activity of Cas9 molecule

The ability of a Cas9 molecule/gRNA molecule complex to bind to and cleave a target nucleic acid can be evaluated in a plasmid cleavage assay. In this assay, synthetic or *in vitro*-transcribed gRNA molecule is pre-annealed prior to the reaction by heating to 95°C and slowly cooling down to room temperature. Native or restriction digest-linearized plasmid DNA (300 ng (∼8 nM)) is incubated for 60 min at 37°C with purified Cas9 protein molecule (50-500 nM) and gRNA (50-500 nM, 1:1) in a Cas9 plasmid cleavage buffer (20 mM HEPES pH 7.5, 150 mMKC1, 0.5 mMDTT, 0.1 mMEDTA) with or without 10 mM MgCl₂. The reactions are stopped with 5X DNA loading buffer (30% glycerol, 1.2% SDS, 250 mM EDTA), resolved by a 0.8 or 1% agarose gel electrophoresis and visualized by ethidium bromide staining. The resulting cleavage products indicate whether the Cas9 molecule cleaves both DNA strands, or only one of the two strands. For example, linear DNA products indicate the cleavage of both DNA strands. Nicked open circular products indicate that only one of the two strands is cleaved.

Alternatively, the ability of a Cas9 molecule/gRNA molecule complex to bind to and cleave a target nucleic acid can be evaluated in an oligonucleotide DNA cleavage assay. In this assay, DNA oligonucleotides (10 pmol) are radiolabeled by incubating with 5 units T4 polynucleotide kinase and ∼3-6 pmol (∼20-40 mCi) [γ-32P]-ATP in 1X T4 polynucleotide kinase reaction buffer at 37°C for 30 min, in a 50 µL reaction. After heat inactivation (65°C for 20 min), reactions are purified through a column to remove unincorporated label. Duplex substrates (100 nM) are generated by annealing labeled oligonucleotides with equimolar amounts of unlabeled complementary oligonucleotide at 95°C for 3 min, followed by slow cooling to room temperature. For cleavage assays, gRNA molecules are annealed by heating to 95°C for 30 s, followed by slow cooling to room temperature. Cas9 (500 nM final concentration) is pre-incubated with the annealed gRNA molecules (500 nM) in cleavage assay buffer (20 mM HEPES pH 7.5, 100 mM KCl, 5 mM MgC12, 1 mM DTT, 5% glycerol) in a total volume of 9 µl. Reactions are initiated by the addition of 1 µl target DNA (10 nM) and incubated for 1 h at 37°C. Reactions are quenched by the addition of 20 µl of loading dye (5 mM EDTA, 0.025% SDS, 5% glycerol in formamide) and heated to 95°C for 5 min. Cleavage products are resolved on 12% denaturing polyacrylamide gels containing 7 M urea and visualized by phosphorimaging. The resulting cleavage products indicate that whether the complementary strand, the non-complementary strand, or both, are cleaved.

One or both of these assays can be used to evaluate the suitability of a candidate gRNA molecule or candidate Cas9 molecule.

### Binding Assay: Testing the binding of Cas9 molecule to target DNA

Exemplary methods for evaluating the binding of Cas9 molecule to target DNA are described, e.g., in Jinek et al., SCIENCE 2012; 337(6096):816-821.

For example, in an electrophoretic mobility shift assay, target DNA duplexes are formed by mixing of each strand (10 nmol) in deionized water, heating to 95°C for 3 min and slow cooling to room temperature. All DNAs are purified on 8% native gels containing 1X TBE. DNA bands are visualized by UV shadowing, excised, and eluted by soaking gel pieces in DEPC-treated H₂O. Eluted DNA is ethanol precipitated and dissolved in DEPC-treated H₂O. DNA samples are 5' end labeled with [y-32P]-ATP using T4 polynucleotide kinase for 30 min at 37°C. Polynucleotide kinase is heat denatured at 65°C for 20 min, and unincorporated radiolabel is removed using a column. Binding assays are performed in buffer containing 20 mM HEPES pH 7.5, 100 mM KCl, 5 mM MgCl₂, 1 mM DTT and 10% glycerol in a total volume of 10 µl. Cas9 protein molecule is programmed with equimolar amounts of pre-annealed gRNA molecule and titrated from 100 pM to 1 µM. Radiolabeled DNA is added to a final concentration of 20 pM. Samples are incubated for 1 h at 37°C and resolved at 4°C on an 8% native polyacrylamide gel containing 1X TBE and 5 mM MgCl₂. Gels are dried and DNA visualized by phosphorimaging.

### Differential Scanning Flourimetry (DSF)

The thermostability of Cas9-gRNA ribonucleoprotein (RNP) complexes can be measured via DSF. This technique measures the thermostability of a protein, which can increase under favorable conditions such as the addition of a binding RNA molecule, e.g., a gRNA.

The assay is performed using two different protocols, one to test the best stoichiometric ratio of gRNA:Cas9 protein and another to determine the best solution conditions for RNP formation.

To determine the best solution to form RNP complexes, a 2uM solution of Cas9 in water+10x SYPRO Orange® (Life Techonologies cat#S-6650) and dispensed into a 384 well plate. An equimolar amount of gRNA diluted in solutions with varied pH and salt is then added. After incubating at room temperature for 10'and brief centrifugation to remove any bubbles,a Bio-Rad CFX384™ Real-Time System C1000 Touch™ Thermal Cycler with the Bio-Rad CFX Manager software is used to run a gradient from 20°C to 90°C with a 1° increase in temperature every 10seconds.

The second assay consists of mixing various concentrations of gRNA with 2uM Cas9 in optimal buffer from assay 1 above and incubating at RT for 10' in a 384 well plate. An equal volume of optimal buffer + 10x SYPRO Orange® (Life Techonologies cat#S-6650) is added and the plate sealed with Microseal® B adhesive (MSB-1001). Following brief centrifugation to remove any bubbles, a Bio-Rad CFX384™ Real-Time System C1000 Touch™ Thermal Cycler with the Bio-Rad CFX Manager software is used to run a gradient from 20°C to 90°C with a 1° increase in temperature every 10seconds.

### V. Genome Editing Approaches

Described herein are methods for targeted knockout of one or both alleles of a gene using NHEJ (see Section V.1). In another embodiment, methods are provided for targeted knockdown of a gene (see Section V.2). It is further contemplated that the disclosed methods may target two or more genes for knockdown or knockout or a combination thereof.

Mutations in a target gene (e.g., the *HBB* gene) may be corrected using one of the approaches discussed herein. In an embodiment, a mutation is corrected by homology directed repair (HDR) using an exogenously provided template nucleic acid (see Section V.3). In another embodiment, a mutation is corrected by homology directed repair without using an exogenously provided template nucleic acid (see Section V.4).

In general, it is to be understood that the alteration of any gene according to the methods described herein can be mediated by any mechanism and that any methods are not limited to a particular mechanism. Exemplary mechanisms that can be associated with the alteration of a gene include, but are not limited to, non-homologous end joining (e.g., classical or alternative), microhomology-mediated end joining (MMEJ), homology-directed repair (e.g., endogenous donor template mediated), SDSA (synthesis dependent strand annealing), single strand annealing or single strand invasion (e.g., see exemplary mechanisms in Section V.5 and Section V.6).

### V.1 NHEJ Approaches for Gene Targeting

As described herein, nuclease-induced non-homologous end-joining (NHEJ) can be used to target gene-specific knockouts. Nuclease-induced NHEJ can also be used to remove (e.g., delete) sequence insertions in a genge.

While not wishing to be bound by theory, it is believed that, in an embodiment, the genomic alterations associated with the methods described herein rely on nuclease-induced NHEJ and the error-prone nature of the NHEJ repair pathway. NHEJ repairs a double-strand break in the DNA by joining together the two ends; however, generally, the original sequence is restored only if two compatible ends, exactly as they were formed by the double-strand break, are perfectly ligated. The DNA ends of the double-strand break are frequently the subject of enzymatic processing, resulting in the addition or removal of nucleotides, at one or both strands, prior to rejoining of the ends. This results in the presence of insertion and/or deletion (indel) mutations in the DNA sequence at the site of the NHEJ repair. Two-thirds of these mutations typically alter the reading frame and, therefore, produce a non-functional protein. Additionally, mutations that maintain the reading frame, but which insert or delete a significant amount of sequence, can destroy functionality of the protein. This is locus dependent as mutations in critical functional domains are likely less tolerable than mutations in non-critical regions of the protein.

The indel mutations generated by NHEJ are unpredictable in nature; however, at a given break site certain indel sequences are favored and are over represented in the population, likely due to small regions of microhomology. The lengths of deletions can vary widely; most commonly in the 1-50 bp range, but they can easily reach greater than 100-200 bp. Insertions tend to be shorter and often include short duplications of the sequence immediately surrounding the break site. However, it is possible to obtain large insertions, and in these cases, the inserted sequence has often been traced to other regions of the genome or to plasmid DNA present in the cells.

Because NHEJ is a mutagenic process, it can also be used to delete small sequence motifs as long as the generation of a specific final sequence is not required. If a double-strand break is targeted near to a short target sequence, the deletion mutations caused by the NHEJ repair often span, and therefore remove, the unwanted nucleotides. For the deletion of larger DNA segments, introducing two double-strand breaks, one on each side of the sequence, can result in NHEJ between the ends with removal of the entire intervening sequence. Both of these approaches can be used to delete specific DNA sequences; however, the error-prone nature of NHEJ may still produce indel mutations at the site of repair.

Both double strand cleaving eaCas9 molecules and single strand, or nickase, eaCas9 molecules can be used in the methods and compositions described herein to generate NHEJ-mediated indels. NHEJ-mediated indels targeted to the gene, e.g., a coding region, e.g., an early coding region of a gene can be used to knockout (i.e., eliminate expression of) a gene. For example, early coding region of a gene includes sequence immediately following a transcription start site, within a first exon of the coding sequence, or within 500 bp of the transcription start site (e.g., less than 500, 450, 400, 350, 300, 250, 200, 150, 100 or 50 bp).

### Placement of double strand or single strand breaks relative to the target position

In an embodiment, in which a gRNA and Cas9 nuclease generate a double strand break for the purpose of inducing NHEJ-mediated indels, a gRNA, e.g., a unimolecular (or chimeric) or modular gRNA molecule, is configured to position one double-strand break in close proximity to a nucleotide of the target position. In an embodiment, the cleavage site is between 0-30 bp away from the target position (e.g., less than 30, 25, 20, 15, 10, 9, 8, 7, 6, 5, 4, 3, 2 or 1 bp from the target position).

In an embodiment, in which two gRNAs complexing with Cas9 nickases induce two single strand breaks for the purpose of inducing NHEJ-mediated indels, two gRNAs, e.g., independently, unimolecular (or chimeric) or modular gRNA, are configured to position two single-strand breaks to provide for NHEJ repair a nucleotide of the target position. In an embodiment, the gRNAs are configured to position cuts at the same position, or within a few nucleotides of one another, on different strands, essentially mimicking a double strand break. In an embodiment, the closer nick is between 0-30 bp away from the target position (e.g., less than 30, 25, 20, 15, 10, 9, 8, 7, 6, 5, 4, 3, 2 or 1 bp from the target position), and the two nicks are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp). In an embodiment, the gRNAs are configured to place a single strand break on either side of a nucleotide of the target position.

Both double strand cleaving eaCas9 molecules and single strand, or nickase, eaCas9 molecules can be used in the methods and compositions described herein to generate breaks both sides of a target position. Double strand or paired single strand breaks may be generated on both sides of a target position to remove the nucleic acid sequence between the two cuts (e.g., the region between the two breaks in deleted). In an embodiment, two gRNAs, e.g., independently, unimolecular (or chimeric) or modular gRNA, are configured to position a double-strand break on both sides of a target position. In an alternate embodiment, three gRNAs, e.g., independently, unimolecular (or chimeric) or modular gRNA, are configured to position a double strand break (i.e., one gRNA complexes with a cas9 nuclease) and two single strand breaks or paired single stranded breaks (i.e., two gRNAs complex with Cas9 nickases) on either side of the target position. In another embodiment, four gRNAs, e.g., independently, unimolecular (or chimeric) or modular gRNA, are configured to generate two pairs of single stranded breaks (i.e., two pairs of two gRNAs complex with Cas9 nickases) on either side of the target position. The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp).

### V.2 Targeted Knockdown

Unlike CRISPR/Cas-mediated gene knockout, which permanently eliminates or reduces expression by mutating the gene at the DNA level, CRISPR/Cas knockdown allows for temporary reduction of gene expression through the use of artificial transcription factors. Mutating key residues in both DNA cleavage domains of the Cas9 protein (e.g., the D10A and H840A or N863A mutations) results in the generation of a catalytically inactive Cas9 (eiCas9 which is also known as dead Cas9 or dCas9). A catalytically inactive Cas9 complexes with a gRNA and localizes to the DNA sequence specified by that gRNA's targeting domain, however, it does not cleave the target DNA. Fusion of the dCas9 to an effector domain, e.g., a transcription repression domain, enables recruitment of the effector to any DNA site specified by the gRNA. While it has been shown that the eiCas9 itself can block transcription when recruited to early regions in the coding sequence, more robust repression can be achieved by fusing a transcriptional repression domain (for example KRAB, SID or ERD) to the Cas9 and recruiting it to the promoter region of a gene. It is likely that targeting DNAseI hypersensitive regions of the promoter may yield more efficient gene repression or activation because these regions are more likely to be accessible to the Cas9 protein and are also more likely to harbor sites for endogenous transcription factors. Especially for gene repression, it is contemplated herein that blocking the binding site of an endogenous transcription factor would aid in downregulating gene expression. In an embodiment, one or more eiCas9s may be used to block binding of one or more endogenous transcription factors. In another embodiment, an eiCas9 can be fused to a chromatin modifying protein. Altering chromatin status can result in decreased expression of the target gene. One or more eiCas9s fused to one or more chromatin modifying proteins may be used to alter chromatin status.

In an embodiment, a gRNA molecule can be targeted to a known transcription response elements (e.g., promoters, enhancers, etc.), a known upstream activating sequences (UAS), and/or sequences of unknown or known function that are suspected of being able to control expression of the target DNA.

CRISPR/Cas-mediated gene knockdown can be used to reduce expression of an unwanted allele or transcript. Contemplated herein are scenarios wherein permanent destruction of the gene is not ideal. In these scenarios, site-specific repression may be used to temporarily reduce or eliminate expression. It is also contemplated herein that the off-target effects of a Cas-repressor may be less severe than those of a Cas-nuclease as a nuclease can cleave any DNA sequence and cause mutations whereas a Cas-repressor may only have an effect if it targets the promoter region of an actively transcribed gene. However, while nuclease-mediated knockout is permanent, repression may only persist as long as the Cas-repressor is present in the cells. Once the repressor is no longer present, it is likely that endogenous transcription factors and gene regulatory elements would restore expression to its natural state.

### V.3 HDR Repair, HDR mediated Knockin, and Template Nucleic Acids

As described herein, nuclease-induced homology directed repair (HDR) can be used to alter a target sequence and correct (e.g., repair or edit) a mutation in the genome. While not wishing to be bound by theory, it is believed that alteration of the target sequence occurs by homology-directed repair (HDR) with an exogenously provided donor template or template nucleic acid. For example, the donor template or the template nucleic acid provides for alteration of the target sequence. It is contemplated that a plasmid donor can be used as a template for homologous recombination. It is further contemplated that a single stranded donor template can be used as a template for alteration of the target sequence by alternate methods of homology directed repair (e.g., single strand annealing) between the target sequence and the donor template. Donor template-effected alteration of a target sequence depends on cleavage by a Cas9 molecule. Cleavage by Cas9 can comprise a double strand break or two single strand breaks. As described herein, nuclease-induced homology directed repair (HDR) can be used to alter a target sequence and correct (e.g., repair or edit) a mutation in the genome without the use of an exogenously provided donor template or template nucleic acid. While not wishing to be bound by theory, it is believed that alteration of the target sequence occurs by homology-directed repair (HDR) with endogenous genomic donor sequence. For example, the endogenous genomic donor sequence provides for alteration of the target sequence. It is contemplated that in an embodiment the endogenous genomic donor sequence is located on the same chromosome as the target sequence. It is further contemplated that in another embodiment the endogenous genomic donor sequence is located on a different chromosome from the target sequence. In an embodiment, the endogenous genomic donor sequence comprises one or more nucleotides derived from the *HBB* gene. Alteration of a target sequence by endogenous genomic donor sequence depends on cleavage by a Cas9 molecule. Cleavage by Cas9 can comprise a double strand break or two single strand breaks.

Mutations that can be corrected by HDR using a template nucleic acid, or using endogenous genomic donor sequence, include point mutations. In an embodiment, a point mutation can be corrected by either a single double-strand break or two single strand breaks. In an embodiment, a point mutation can be corrected by (1) a single double-strand break, (2) two single strand breaks, (3) two double stranded breaks with a break occurring on each side of the target position, (4) one double stranded break and two single strand breaks with the double strand break and two single strand breaks occurring on each side of the target position (5) four single stranded breaks with a pair of single stranded breaks occurring on each side of the target position, or (6) one single stranded break.

In an embodiment where a single-stranded template nucleic acid is used, the target position can be altered by alternative HDR.

Donor template-effected alteration of a target position depends on cleavage by a Cas9 molecule. Cleavage by Cas9 can comprise a nick, a double strand break, or two single strand breaks, e.g., one on each strand of the target nucleic acid. After introduction of the breaks on the target nucleic acid, resection occurs at the break ends resulting in single stranded overhanging DNA regions.

In canonical HDR, a double-stranded donor template is introduced, comprising homologous sequence to the target nucleic acid that will either be directly incorporated into the target nucleic acid or used as a template to correct the sequence of the target nucleic acid. After resection at the break, repair can progress by different pathways, e.g., by the double Holliday junction model (or double strand break repair, DSBR, pathway) or the synthesis-dependent strand annealing (SDSA) pathway. In the double Holliday junction model, strand invasion by the two single stranded overhangs of the target nucleic acid to the homologous sequences in the donor template occurs, resulting in the formation of an intermediate with two Holliday junctions. The junctions migrate as new DNA is synthesized from the ends of the invading strand to fill the gap resulting from the resection. The end of the newly synthesized DNA is ligated to the resected end, and the junctions are resolved, resulting in the correction of the target nucleic acid, e.g., incorporation of the correct sequence of the donor template at the corresponding target position. Crossover with the donor template may occur upon resolution of the junctions. In the SDSA pathway, only one single stranded overhang invades the donor template and new DNA is synthesized from the end of the invading strand to fill the gap resulting from resection. The newly synthesized DNA then anneals to the remaining single stranded overhang, new DNA is synthesized to fill in the gap, and the strands are ligated to produce the corrected DNA duplex.

In alternative HDR, a single strand donor template, e.g., template nucleic acid, is introduced. A nick, single strand break, or double strand break at the target nucleic acid, for altering a desired target position, is mediated by a Cas9 molecule, e.g., described herein, and resection at the break occurs to reveal single stranded overhangs. Incorporation of the sequence of the template nucleic acid to correct or alter the target position of the target nucleic acid typically occurs by the SDSA pathway, as described above.

Methods of promoting HDR pathways, e.g., canonical HDR or alt-HDR, are described herein in Section VI.

Additional details on template nucleic acids are provided in Section IV entitled "Template nucleic acids" in International Application PCT/US2014/057905.

Mutations in the *HBB* gene that can be corrected (e.g., altered) by HDR with a template nucleic acid or with endogenous genomic donor sequence include, e.g., point mutation at E6, e.g., E6V.

### Double strand break mediated correction or knockin

In an embodiment, double strand cleavage is effected by a Cas9 molecule having cleavage activity associated with an HNH-like domain and cleavage activity associated with a RuvC-like domain, e.g., an N-terminal RuvC-like domain, e.g., a wild type Cas9. Such embodiments require only a single gRNA.

### Single strand break mediated correction or knockin

In some embodiments, one single strand break, or nick, is effected by a Cas9 molecule having nickase activity, e.g., a Cas9 nickase as described herein. A nicked target nucleic acid can be a substrate for alt-HDR.

In other embodiments, two single strand breaks, or nicks, are effected by a Cas9 molecule having nickase activity, e.g., cleavage activity associated with an HNH-like domain or cleavage activity associated with an N-terminal RuvC-like domain. Such embodiments usually require two gRNAs, one for placement of each single strand break. In an embodiment, the Cas9 molecule having nickase activity cleaves the strand to which the gRNA hybridizes, but not the strand that is complementary to the strand to which the gRNA hybridizes. In an embodiment, the Cas9 molecule having nickase activity does not cleave the strand to which the gRNA hybridizes, but rather cleaves the strand that is complementary to the strand to which the gRNA hybridizes.

In an embodiment, the nickase has HNH activity, e.g., a Cas9 molecule having the RuvC activity inactivated, e.g., a Cas9 molecule having a mutation at D10, e.g., the D10A mutation. D10A inactivates RuvC; therefore, the Cas9 nickase has (only) HNH activity and will cut on the strand to which the gRNA hybridizes (e.g., the complementary strand, which does not have the NGG PAM on it). In other embodiments, a Cas9 molecule having an H840, e.g., an H840A, mutation can be used as a nickase. H840A inactivates HNH; therefore, the Cas9 nickase has (only) RuvC activity and cuts on the non-complementary strand (e.g., the strand that has the NGG PAM and whose sequence is identical to the gRNA). In an embodiment, the Cas9 molecule is an N-terminal RuvC-like domain nickase, e.g., the Cas9 molecule comprises a mutation at N863, e.g., N863A.

In an embodiment, in which a nickase and two gRNAs are used to position two single strand nicks, one nick is on the + strand and one nick is on the - strand of the target nucleic acid. The PAMs are outwardly facing. The gRNAs can be selected such that the gRNAs are separated by, from about 0-50, 0-100, or 0-200 nucleotides. In an embodiment, there is no overlap between the target sequences that are complementary to the targeting domains of the two gRNAs. In an embodiment, the gRNAs do not overlap and are separated by as much as 50, 100, or 200 nucleotides. In an embodiment, the use of two gRNAs can increase specificity, e.g., by decreasing off-target binding (Ran et al., Cell 2013).

In an embodiment, a single nick can be used to induce HDR, e.g., alt-HDR. It is contemplated herein that a single nick can be used to increase the ratio of HR to NHEJ at a given cleavage site. In an embodiment, a single strand break is formed in the strand of the target nucleic acid to which the targeting domain of said gRNA is complementary. In another embodiment, a single strand break is formed in the strand of the target nucleic acid other than the strand to which the targeting domain of said gRNA is complementary.

### Placement of the double strand break or a single strand break relative to the target position

The double strand break or single strand break in one of the strands should be sufficiently close to target position such that an alteration is produced in the desired region, e.g., correction of a mutation occurs. In an embodiment, the distance is not more than 10, 25, 50, 100, 200, 300, 350, 400 or 500 nucleotides. While not wishing to be bound by theory, in some embodiments, it is believed that the break should be sufficiently close to target position such that the target position is within the region that is subject to exonuclease-mediated removal during end resection. If the distance between the target position and a break is too great, the mutation or other sequence desired to be altered may not be included in the end resection and, therefore, may not be corrected, as donor sequence, either exogenously provided donor sequence or endogenous genomic donor sequence, in some embodiments is only used to correct sequence within the end resection region.

In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 300, 350, 400 or 500 nucleotides of the region desired to be altered, e.g., a mutation. The break, e.g., a double strand or single strand break, can be positioned upstream or downstream of the region desired to be altered, e.g., a mutation. In some embodiments, a break is positioned within the region desired to be altered, e.g., within a region defined by at least two mutant nucleotides. In some embodiments, a break is positioned immediately adjacent to the region desired to be altered, e.g., immediately upstream or downstream of a mutation.
In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 300, 350, 400 or 500 nucleotides of a target position. In an embodiment, the first and second gRNA molecules are configured such, that when guiding a Cas9 nickase, a single strand break will be accompanied by an additional single strand break, positioned by a second gRNA, sufficiently close to one another to result in alteration of the desired region. In an embodiment, the first and second gRNA molecules are configured such that a single strand break positioned by said second gRNA is within 10, 20, 30, 40, or 50 nucleotides of the break positioned by said first gRNA molecule, e.g., when the Cas9 is a nickase. In an embodiment, the two gRNA molecules are configured to position cuts at the same position, or within a few nucleotides of one another, on different strands, e.g., essentially mimicking a double strand break.

In an embodiment, in which a gRNA (unimolecular (or chimeric) or modular gRNA) and Cas9 nuclease induce a double strand break for the purpose of inducing HDR-mediated correction, the cleavage site is between 0-200 bp (e.g., 0-175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, 75 to 100 bp) away from the target position. In an embodiment, the cleavage site is between 0-100 bp (e.g., 0 to 75, 0 to 50, 0 to 25, 25 to 100, 25 to 75, 25 to 50, 50 to 100, 50 to 75 or 75 to 100 bp) away from the target position.

In embodiments, one can promote HDR by using nickases to generate a break with overhangs. While not wishing to be bound by theory, the single stranded nature of the overhangs can enhance the cell's likelihood of repairing the break by HDR as opposed to, e.g., NHEJ.

Specifically, in some embodiments, HDR is promoted by selecting a first gRNA that targets a first nickase to a first target sequence, and a second gRNA that targets a second nickase to a second target sequence which is on the opposite DNA strand from the first target sequence and offset from the first nick.

In an embodiment, the targeting domain of a gRNA molecule is configured to position a cleavage event sufficiently far from a preselected nucleotide, e.g., the nucleotide of a coding region, such that the nucleotide is not altered. In an embodiment, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

### Placement of a first break and a second break relative to each other

In an embodiment, a double strand break can be accompanied by an additional double strand break, positioned by a second gRNA molecule, as is discussed below.

In an embodiment, a double strand break can be accompanied by two additional single strand breaks, positioned by a second gRNA molecule and a third gRNA molecule.

In an embodiment, a first and second single strand breaks can be accompanied by two additional single strand breaks positioned by a third gRNA molecule and a fourth gRNA molecule.

When two or more gRNAs are used to position two or more cleavage events, e.g., double strand or single strand breaks, in a target nucleic acid, it is contemplated that the two or more cleavage events may be made by the same or different Cas9 proteins. For example, when two gRNAs are used to position two double stranded breaks, a single Cas9 nuclease may be used to create both double stranded breaks. When two or more gRNAs are used to position two or more single stranded breaks (nicks), a single Cas9 nickase may be used to create the two or more nicks. When two or more gRNAs are used to position at least one double stranded break and at least one single stranded break, two Cas9 proteins may be used, e.g., one Cas9 nuclease and one Cas9 nickase. It is contemplated that when two or more Cas9 proteins are used that the two or more Cas9 proteins may be delivered sequentially to control specificity of a double stranded versus a single stranded break at the desired position in the target nucleic acid.

In some embodiments, the targeting domain of the first gRNA molecule and the targeting domain of the second gRNA molecules are complementary to opposite strands of the target nucleic acid molecule. In some embodiments, the gRNA molecule and the second gRNA molecule are configured such that the PAMs are oriented outward.

In certain embodiments, two gRNA are selected to direct Cas9-mediated cleavage at two positions that are a preselected distance from each other. In embodiments, the two points of cleavage are on opposite strands of the target nucleic acid. In some embodiments, the two cleavage points form a blunt ended break, and in other embodiments, they are offset so that the DNA ends comprise one or two overhangs (e.g., one or more 5' overhangs and/or one or more 3' overhangs). In some embodiments, each cleavage event is a nick. In embodiments, the nicks are close enough together that they form a break that is recognized by the double stranded break machinery (as opposed to being recognized by, e.g., the SSBr machinery). In embodiments, the nicks are far enough apart that they create an overhang that is a substrate for HDR, i.e., the placement of the breaks mimics a DNA substrate that has experienced some resection. For instance, in some embodiments the nicks are spaced to create an overhang that is a substrate for processive resection. In some embodiments, the two breaks are spaced within 25-65 nucleotides of each other. The two breaks may be, e.g., about 25, 30, 35, 40, 45, 50, 55, 60 or 65 nucleotides of each other. The two breaks may be, e.g., at least about 25, 30, 35, 40, 45, 50, 55, 60 or 65 nucleotides of each other. The two breaks may be, e.g., at most about 30, 35, 40, 45, 50, 55, 60 or 65 nucleotides of each other. In embodiments, the two breaks are about 25-30, 30-35, 35-40, 40-45, 45-50, 50-55, 55-60, or 60-65 nucleotides of each other.

In some embodiments, the break that mimics a resected break comprises a 3' overhang (e.g., generated by a DSB and a nick, where the nick leaves a 3' overhang), a 5' overhang (e.g., generated by a DSB and a nick, where the nick leaves a 5' overhang), a 3' and a 5' overhang (e.g., generated by three cuts), two 3' overhangs (e.g., generated by two nicks that are offset from each other), or two 5' overhangs (e.g., generated by two nicks that are offset from each other).

In an embodiment, in which two gRNAs (independently, unimolecular (or chimeric) or modular gRNA) complexing with Cas9 nickases induce two single strand breaks for the purpose of inducing HDR-mediated correction, the closer nick is between 0-200 bp (e.g., 0-175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, 75 to 100 bp) away from the target position and the two nicks will ideally be within 25-65 bp of each other (e.g., 25 to 50, 25 to 45, 25 to 40, 25 to 35, 25 to 30, 30 to 55, 30 to 50, 30 to 45, 30 to 40, 30 to 35, 35 to 55, 35 to 50, 35 to 45, 35 to 40, 40 to 55, 40 to 50, 40 to 45 bp, 45 to 50 bp, 50 to 55 bp, 55 to 60 bp, 60 to 65 bp) and no more than 100 bp away from each other (e.g., no more than 90, 80, 70, 60, 50, 40, 30, 20, 10 or 5 bp away from each other). In an embodiment, the cleavage site is between 0-100 bp (e.g., 0 to 75, 0 to 50, 0 to 25, 25 to 100, 25 to 75, 25 to 50, 50 to 100, 50 to 75 or 75 to 100 bp) away from the target position.

In one embodiment, two gRNAs, e.g., independently, unimolecular (or chimeric) or modular gRNA, are configured to position a double-strand break on both sides of a target position. In an alternate embodiment, three gRNAs, e.g., independently, unimolecular (or chimeric) or modular gRNA, are configured to position a double strand break (i.e., one gRNA complexes with a cas9 nuclease) and two single strand breaks or paired single stranded breaks (i.e., two gRNAs complex with Cas9 nickases) on either side of the target position. In another embodiment, four gRNAs, e.g., independently, unimolecular (or chimeric) or modular gRNA, are configured to generate two pairs of single stranded breaks (i.e., two pairs of two gRNAs complex with Cas9 nickases) on either side of the target position. The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are, in embodiments, within 25-65 bp of each other (e.g., between 25 to 55, 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, 40 to 45 bp, 45 to 50 bp, 50 to 55 bp, 55 to 60 bp, or 60 to 65 bp) and no more than 100 bp away from each other (e.g., no more than 90, 80, 70, 60, 50, 40, 30, 20 or 10 bp).

When two gRNAs are used to target Cas9 molecules to breaks, different combinations of Cas9 molecules are envisioned. In some embodiments, a first gRNA is used to target a first Cas9 molecule to a first target position, and a second gRNA is used to target a second Cas9 molecule to a second target position. In some embodiments, the first Cas9 molecule creates a nick on the first strand of the target nucleic acid, and the second Cas9 molecule creates a nick on the opposite strand, resulting in a double stranded break (e.g., a blunt ended cut or a cut with overhangs).

Different combinations of nickases can be chosen to target one single stranded break to one strand and a second single stranded break to the opposite strand. When choosing a combination, one can take into account that there are nickases having one active RuvC-like domain, and nickases having one active HNH domain. In an embodiment, a RuvC-like domain cleaves the non-complementary strand of the target nucleic acid molecule. In an embodiment, an HNH-like domain cleaves a single stranded complementary domain, e.g., a complementary strand of a double stranded nucleic acid molecule. Generally, if both Cas9 molecules have the same active domain (e.g., both have an active RuvC domain or both have an active HNH domain), one will choose two gRNAs that bind to opposite strands of the target. In more detail, in some embodiments, a first gRNA is complementary with a first strand of the target nucleic acid and binds a nickase having an active RuvC-like domain and causes that nickase to cleave the strand that is non-complementary to that first gRNA, i.e., a second strand of the target nucleic acid; and a second gRNA is complementary with a second strand of the target nucleic acid and binds a nickase having an active RuvC-like domain and causes that nickase to cleave the strand that is non-complementary to that second gRNA, i.e., the first strand of the target nucleic acid. Conversely, in some embodiments, a first gRNA is complementary with a first strand of the target nucleic acid and binds a nickase having an active HNH domain and causes that nickase to cleave the strand that is complementary to that first gRNA, i.e., a first strand of the target nucleic acid; and a second gRNA is complementary with a second strand of the target nucleic acid and binds a nickase having an active HNH domain and causes that nickase to cleave the strand that is complementary to that second gRNA, i.e., the second strand of the target nucleic acid. In another arrangement, if one Cas9 molecule has an active RuvC-like domain and the other Cas9 molecule has an active HNH domain, the gRNAs for both Cas9 molecules can be complementary to the same strand of the target nucleic acid, so that the Cas9 molecule with the active RuvC-like domain will cleave the non-complementary strand and the Cas9 molecule with the HNH domain will cleave the complementary strand, resulting in a double stranded break.

### Length of the homology arms of the donor template

The homology arm should extend at least as far as the region in which end resection may occur, e.g., in order to allow the resected single stranded overhang to find a complementary region within the donor template. The overall length could be limited by parameters such as plasmid size or viral packaging limits. In an embodiment, a homology arm does not extend into repeated elements, e.g., ALU repeats or LINE repeats.

Exemplary homology arm lengths include at least 50, 100, 250, 500, 750, 1000, 2000, 3000, 4000, or 5000 nucleotides. In some embodiments, the homology arm length is 50-100, 100-250, 250-500, 500-750, 750-1000, 1000-2000, 2000-3000, 3000-4000, or 4000-5000 nucleotides.

Target position, as used herein, refers to a site on a target nucleic acid (e.g., the chromosome) that is modified by a Cas9 molecule-dependent process. For example, the target position can be a modified Cas9 molecule cleavage of the target nucleic acid and template nucleic acid directed modification, e.g., correction, of the target position. In an embodiment, a target position can be a site between two nucleotides, e.g., adjacent nucleotides, on the target nucleic acid into which one or more nucleotides is added. The target position may comprise one or more nucleotides that are altered, e.g., corrected, by a template nucleic acid. In an embodiment, the target position is within a target sequence (e.g., the sequence to which the gRNA binds). In an embodiment, a target position is upstream or downstream of a target sequence (e.g., the sequence to which the gRNA binds).

A template nucleic acid, as that term is used herein, refers to a nucleic acid sequence which can be used in conjunction with a Cas9 molecule and a gRNA molecule to alter the structure of a target position. In an embodiment, the target nucleic acid is modified to have the some or all of the sequence of the template nucleic acid, typically at or near cleavage site(s). In an embodiment, the template nucleic acid is single stranded. In an alternate embodiment, the template nucleic acid is double stranded. In an embodiment, the template nucleic acid is DNA, e.g., double stranded DNA. In an alternate embodiment, the template nucleic acid is single stranded DNA. In an embodiment, the template nucleic acid is encoded on the same vector backbone, e.g. AAV genome, plasmid DNA, as the Cas9 and gRNA. In an embodiment, the template nucleic acid is excised from a vector backbone *in vivo,* e.g., it is flanked by gRNA recognition sequences. In an embodiment, the template nucleic acid comprises endogenous genomic sequence

In an embodiment, the template nucleic acid alters the structure of the target position by participating in a homology directed repair event. In an embodiment, the template nucleic acid alters the sequence of the target position. In an embodiment, the template nucleic acid results in the incorporation of a modified, or non-naturally occurring base into the target nucleic acid.

Typically, the template sequence undergoes a breakage mediated or catalyzed recombination with the target sequence. In an embodiment, the template nucleic acid includes sequence that corresponds to a site on the target sequence that is cleaved by an eaCas9 mediated cleavage event. In an embodiment, the template nucleic acid includes sequence that corresponds to both, a first site on the target sequence that is cleaved in a first Cas9 mediated event, and a second site on the target sequence that is cleaved in a second Cas9 mediated event.

In an embodiment, the template nucleic acid can include sequence which results in an alteration in the coding sequence of a translated sequence, e.g., one which results in the substitution of one amino acid for another in a protein product, e.g., transforming a mutant allele into a wild type allele, transforming a wild type allele into a mutant allele, and/or introducing a stop codon, insertion of an amino acid residue, deletion of an amino acid residue, or a nonsense mutation.

In other embodiments, the template nucleic acid can include sequence which results in an alteration in a non-coding sequence, e.g., an alteration in an exon or in a 5' or 3' non-translated or non-transcribed region. Such alterations include an alteration in a control element, e.g., a promoter, enhancer, and an alteration in a cis-acting or trans-acting control element.

A template nucleic acid having homology with a target position in the *HBB* gene can be used to alter the structure of a target sequence. The template sequence can be used to alter an unwanted structure, e.g., an unwanted or mutant nucleotide.

A template nucleic acid typically comprises the following components:
[5' homology arm]-[replacement sequence]-[3' homology arm].

The homology arms provide for recombination into the chromosome, thus replacing the undesired element, e.g., a mutation or signature, with the replacement sequence. In an embodiment, the homology arms flank the most distal cleavage sites.

In an embodiment, the 3' end of the 5' homology arm is the position next to the 5' end of the replacement sequence. In an embodiment, the 5' homology arm can extend at least 10, 20, 30, 40, 50, 100, 200, 300, 400, 500, 600, 700, 800, 900, 1000, 1500, 2000, 3000, 4000, or 5000 nucleotides 5' from the 5' end of the replacement sequence.

In an embodiment, the 5' end of the 3' homology arm is the position next to the 3' end of the replacement sequence. In an embodiment, the 3' homology arm can extend at least 10, 20, 30, 40, 50, 100, 200, 300, 400, 500, 600, 700, 800, 900, 1000, 1500, 2000, 3000, 4000, or 5000 nucleotides 3' from the 3' end of the replacement sequence.

In an embodiment, to correct a mutation, the homology arms, e.g., the 5' and 3' homology arms, may each comprise about 1000 base pairs (bp) of sequence flanking the most distal gRNAs (e.g., 1000bp of sequence on either side of the mutation).

It is contemplated herein that one or both homology arms may be shortened to avoid including certain sequence repeat elements, e.g., Alu repeats or LINE elements. For example, a 5' homology arm may be shortened to avoid a sequence repeat element. In other embodiments, a 3' homology arm may be shortened to avoid a sequence repeat element. In some embodiments, both the 5' and the 3' homology arms may be shortened to avoid including certain sequence repeat elements.

It is contemplated herein that template nucleic acids for correcting a mutation may be designed for use as a single-stranded oligonucleotide, e.g., a single-stranded oligodeoxynucleotide (ssODN). When using a ssODN, 5' and 3' homology arms may range up to about 200 base pairs (bp) in length, e.g., at least 25, 50, 75, 100, 125, 150, 175, or 200 bp in length. Longer homology arms are also contemplated for ssODNs as improvements in oligonucleotide synthesis continue to be made. In some embodiments, a longer homology arm is made by a method other than chemical synthesis, e.g., by denaturing a long double stranded nucleic acid and purifying one of the strands, e.g., by affinity for a strand-specific sequence anchored to a solid substrate.

While not wishing to be bound by theory, in some embodiments alt-HDR proceeds more efficiently when the template nucleic acid has extended homology 5' to the nick (i.e., in the 5' direction of the nicked strand). Accordingly, in some embodiments, the template nucleic acid has a longer homology arm and a shorter homology arm, wherein the longer homology arm can anneal 5' of the nick. In some embodiments, the arm that can anneal 5' to the nick is at least 25, 50, 75, 100, 125, 150, 175, or 200, 300, 400, 500, 600, 700, 800, 900, 1000, 1500, 2000, 3000, 4000, or 5000 nucleotides from the nick or the 5' or 3' end of the replacement sequence. In some embodiemtns, the arm that can anneal 5' to the nick is at least 10%, 20%, 30%, 40%, or 50% longer than the arm that can anneal 3' to the nick. In some embodiments, the arm that can anneal 5' to the nick is at least 2x, 3x, 4x, or 5x longer than the arm that can anneal 3' to the nick. Depending on whether a ssDNA template can anneal to the intact strand or the nicked strand, the homology arm that anneals 5' to the nick may be at the 5' end of the ssDNA template or the 3' end of the ssDNA template, respectively.

Similarly, in some embodiments, the template nucleic acid has a 5' homology arm, a replacement sequence, and a 3' homology arm, such that the template nucleic acid has extended homology to the 5' of the nick. For example, the 5' homology arm and 3' homology arm may be substantially the same length, but the replacement sequence may extend farther 5' of the nick than 3' of the nick. In some embodiments, the replacement sequence extends at least 10%, 20%, 30%, 40%, 50%, 2x, 3x, 4x, or 5x further to the 5' end of the nick than the 3' end of the nick.
While not wishing to be bound by theory, in some embodiments alt-HDR proceeds more efficiently when the template nucleic acid is centered on the nick. Accordingly, in some embodiments, the template nucleic acid has two homology arms that are essentially the same size. For instance, the first homology arm of a template nucleic acid may have a length that is within 10%, 9%, 8%, 7%, 6%, 5%, 4%, 3%, 2%, or 1% of the second homology arm of the template nucleic acid.

Similarly, in some embodiments, the template nucleic acid has a 5' homology arm, a replacement sequence, and a 3' homology arm, such that the template nucleic acid extends substantially the same distance on either side of the nick. For example, the homology arms may have different lengths, but the replacement sequence may be selected to compensate for this. For example, the replacement sequence may extend further 5' from the nick than it does 3' of the nick, but the homology arm 5' of the nick is shorter than the homology arm 3' of the nick, to compensate. The converse is also possible, e.g., that the replacement sequence may extend further 3' from the nick than it does 5' of the nick, but the homology arm 3' of the nick is shorter than the homology arm 5' of the nick, to compensate.

### Exemplary arrangements of linear nucleic acid template systems

In an embodiment, the nucleic acid template system is double stranded. In an embodiment, the nucleic acid template system is single stranded. In an embodiment, the nucleic acid template system comprises a single stranded portion and a double stranded portion. In an embodiment, the template nucleic acid comprises about 50 to 100, e.g., 55 to 95, 60 to 90, 65 to 85, or 70 to 80, base pairs, homology on either side of the nick and/or replacement sequence. In an embodiment, the template nucleic acid comprises about 50, 55, 60, 65, 70, 75, 80, 85, 90, 95, or 100 base pairs homology 5' of the nick or replacement sequence, 3' of the nick or replacement sequence, or both 5' and 3' of the nick or replacement sequences.

In an embodiment, the template nucleic acid comprises about 150 to 200, e.g., 155 to 195, 160 to 190, 165 to 185, or 170 to 180, base pairs homology 3' of the nick and/or replacement sequence. In an embodiment, the template nucleic acid comprises about 150, 155, 160, 165, 170, 175, 180, 185, 190, 195, or 200 base pairs homology 3' of the nick or replacement sequence. In an embodiment, the template nucleic acid comprises less than about 100, 90, 80, 70, 60, 50, 40, 30, 20, 15, or 10 base pairs homology 5' of the nick or replacement sequence.

In an embodiment, the template nucleic acid comprises about 150 to 200, e.g., 155 to 195, 160 to 190, 165 to 185, or 170 to 180, base pairs homology 5' of the nick and/or replacement sequence. In an embodiment, the template nucleic acid comprises about 150, 155, 160, 165, 170, 175, 180, 185, 190, 195, or 200 base pairs homology 5' of the nick or replacement sequence. In an embodiment, the template nucleic acid comprises less than about 100, 90, 80, 70, 60, 50, 40, 30, 20, 15, or 10 base pairs homology 3' of the nick or replacement sequence.

### Exemplary Template Nucleic Acids

In an embodiment, the template nucleic acid is a single stranded nucleic acid. In another embodiment, the template nucleic acid is a double stranded nucleic acid. In some embodiments, the template nucleic acid comprises a nucleotide sequence, e.g., of one or more nucleotides, that will be added to or will template a change in the target nucleic acid. In other embodiments, the template nucleic acid comprises a nucleotide sequence that may be used to modify the target position. In other embodiments, the template nucleic acid comprises a nucleotide sequence, e.g., of one or more nucleotides, that corresponds to wild type sequence of the target nucleic acid, e.g., of the target position.

The template nucleic acid may comprise a replacement sequence. In some embodiments, the template nucleic acid comprises a 5' homology arm. In other embodiments, the template nucleic acid comprises a 3' homology arm.

In embodiments, the template nucleic acid is linear double stranded DNA. The length may be, e.g., about 150-200 base pairs, e.g., about 150, 160, 170, 180, 190, or 200 base pairs. The length may be, e.g., at least 150, 160, 170, 180, 190, or 200 base pairs. In some embodiments, the length is no greater than 150, 160, 170, 180, 190, or 200 base pairs. In some embodiments, a double stranded template nucleic acid has a length of about 160 base pairs, e.g., about 155-165, 150-170, 140-180, 130-190, 120-200, 110-210, 100-220, 90-230, or 80-240 base pairs.

The template nucleic acid can be linear single stranded DNA. In embodiments, the template nucleic acid is (i) linear single stranded DNA that can anneal to the nicked strand of the target nucleic acid, (ii) linear single stranded DNA that can anneal to the intact strand of the target nucleic acid, (iii) linear single stranded DNA that can anneal to the transcribed strand of the target nucleic acid, (iv) linear single stranded DNA that can anneal to the non-transcribed strand of the target nucleic acid, or more than one of the preceding. The length may be, e.g., about 150-200 nucleotides, e.g., about 150, 160, 170, 180, 190, or 200 nucleotides. The length may be, e.g., at least 150, 160, 170, 180, 190, or 200 nucleotides. In some embodiments, the length is no greater than 150, 160, 170, 180, 190, or 200 nucleotides. In some embodiments, a single stranded template nucleic acid has a length of about 160 nucleotides, e.g., about 155-165, 150-170, 140-180, 130-190, 120-200, 110-210, 100-220, 90-230, or 80-240 nucleotides.

In some embodiments, the template nucleic acid is circular double stranded DNA, e.g., a plasmid. In some embodiments, the template nucleic acid comprises about 500 to 1000 base pairs of homology on either side of the replacement sequence and/or the nick. In some embodiments, the template nucleic acid comprises about 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence. In some embodiments, the template nucleic acid comprises at least 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence. In some embodiments, the template nucleic acid comprises no more than 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence.

In some embodiments, the template nucleic acid is an adenovirus vector, e.g., an AAV vector, e.g., a ssDNA molecule of a length and sequence that allows it to be packaged in an AAV capsid. The vector may be, e.g., less than 5 kb and may contain an ITR sequence that promotes packaging into the capsid. The vector may be integration-deficient. In some embodiments, the template nucleic acid comprises about 150 to 1000 nucleotides of homology on either side of the replacement sequence and/or the nick. In some embodiments, the template nucleic acid comprises about 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. In some embodiments, the template nucleic acid comprises at least 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. In some embodiments, the template nucleic acid comprises at most 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence.

In some embodiments, the template nucleic acid is a lentiviral vector, e.g., an IDLV (integration deficiency lentivirus). In some embodiments, the template nucleic acid comprises about 500 to 1000 base pairs of homology on either side of the replacement sequence and/or the nick. In some embodiments, the template nucleic acid comprises about 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence. In some embodiments, the template nucleic acid comprises at least 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence. In some embodiments, the template nucleic acid comprises no more than 300, 400, 500, 600, 700, 800, 900, 1000, 1500, or 2000 base pairs of homology 5' of the nick or replacement sequence, 3' of the nick or replacement sequence, or both 5' and 3' of the nick or replacement sequence.

In many embodiments, the template nucleic acid comprises one or more mutations, e.g., silent mutations, that prevent Cas9 from recognizing and cleaving the template nucleic acid. The template nucleic acid may comprise, e.g., at least 1, 2, 3, 4, 5, 10, 20, or 30 silent mutations relative to the corresponding sequence in the genome of the cell to be altered. In embodiments, the template nucleic acid comprises at most 2, 3, 4, 5, 10, 20, 30, or 50 silent mutations relative to the corresponding sequence in the genome of the cell to be altered. In an embodiment, the cDNA comprises one or more mutations, e.g., silent mutations that prevent Cas9 from recognizing and cleaving the template nucleic acid. The template nucleic acid may comprise, e.g., at least 1, 2, 3, 4, 5, 10, 20, or 30 silent mutations relative to the corresponding sequence in the genome of the cell to be altered. In embodiments, the template nucleic acid comprises at most 2, 3, 4, 5, 10, 20, 30, or 50 silent mutations relative to the corresponding sequence in the genome of the cell to be altered.

In an embodiment, the template nucleic acid alters the structure of the target position by participating in a homology directed repair event. In an embodiment, the template nucleic acid alters the sequence of the target position. In an embodiment, the template nucleic acid results in the incorporation of a modified, or non-naturally occurring base into the target nucleic acid.

Typically, the template sequence undergoes a breakage mediated or catalyzed recombination with the target sequence. In an embodiment, the template nucleic acid includes sequence that corresponds to a site on the target sequence that is cleaved by an eaCas9 mediated cleavage event. In an embodiment, the template nucleic acid includes sequence that corresponds to both, a first site on the target sequence that is cleaved in a first Cas9 mediated event, and a second site on the target sequence that is cleaved in a second Cas9 mediated event.

In an embodiment, the template nucleic acid can include sequence which results in an alteration in the coding sequence of a translated sequence, e.g., one which results in the substitution of one amino acid for another in a protein product, e.g., transforming a mutant allele into a wild type allele, transforming a wild type allele into a mutant allele, and/or introducing a stop codon, insertion of an amino acid residue, deletion of an amino acid residue, or a nonsense mutation.

In other embodiments, the template nucleic acid can include sequence which results in an alteration in a non-coding sequence, e.g., an alteration in an exon or in a 5' or 3' non-translated or non-transcribed region. Such alterations include an alteration in a control element, e.g., a promoter, enhancer, and an alteration in a cis-acting or trans-acting control element.

A template nucleic acid having homology with a target position can be used to alter the structure of a target sequence. The template sequence can be used to alter an unwanted structure, e.g., an unwanted or mutant nucleotide.

Exemplary template nucleic acids (also referred to herein as donor constructs) to correction a mutation, e.g., at E6, e.g., E6V, in the *HBB* gene, are provided.

Suitable sequence for the 5' homology arm can be selected from (e.g., includes a portion of) or include the following sequence:

SEQ ID NO:391 (5'H arm)
ATAGGAACTTGAATCAAGGAAATGATTTTAAAACGCAGTATTCTTAGTGGACTAGA
GGAAAAAAATAATCTGAGCCAAGTAGAAGACCTTTTCCCCTCCTACCCCTACTTTCT

AAGTCACAGAGGCTTTTTGTTCCCCCAGACACTCTTGCAGATTAGTCCAGGCAGAAA
CAGTTAGATGTCCCCAGTTAACCTCCTATTTGACACCACTGATTACCCCATTGATAGT
CACACTTTGGGTTGTAAGTGACTTTTTATTTATTTGTATTTTTGACTGCATTAAGAGG
TCTCTAGTTTTTTATCTCTTGTTTCCCAAAACCTAATAAGTAACTAATGCACAGAGCA
CATTGATTTGTATTTATTCTATTTTTAGACATAATTTATTAGCATGCATGAGCAAATT
AAGAAAAACAACAACAAATGAATGCATATATATGTATATGTATGTGTGTATATATAC
ACACATATATATATATATTTTTTCTTTTCTTACCAGAAGGTTTTAATCCAAATAAGGA
GAAGATATGCTTAGAACCGAGGTAGAGTTTTCATCCATTCTGTCCTGTAAGTATTTT
GCATATTCTGGAGACGCAGGAAGAGATCCATCTACATATCCCAAAGCTGAATTATG
GTAGACAAAACTCTTCCACTTTTAGTGCATCAACTTCTTATTTGTGTAATAAGAAAAT
TGGGAAAACGATCTTCAATATGCTTACCAAGCTGTGATTCCAAATATTACGTAAATA
CACTTGCAAAGGAGGATGTTTTTAGTAGCAATTTGTACTGATGGTATGGGGCCAAGA
GATATATCTTAGAGGGAGGGCTGAGGGTTTGAAGTCCAACTCCTAAGCCAGTGCCA
GAAGAGCCAAGGACAGGTACGGCTGTCATCACTTAGACCTCACCCTGTGGAGCCAC
ACCCTAGGGTTGGCCAATCTACTCCCAGGAGCAGGGAGGGCAGGAGCCAGGGCTGG
GCATAAAAGTCAGGGCAGAGCCATCTATTGCTTACATTTGCTTCTGACACAACTGTG
TTCACTAGCAACCTCAAACAGACACCATGGTGCATCTGACTCCTG

Suitable sequence for the 3' homology arm can be selected from (e.g., includes a portion of) or include the following sequence:

SEQ ID NO:392 (3'H arm)
GGAGAAGTCTGCCGTTACTGCCCTGTGGGGCAAGGTGAACGTGGATGAAGTTGGTG
GTGAGGCCCTGGGCAGGTTGGTATCAAGGTTACAAGACAGGTTTAAGGAGACCAAT
AGAAACTGGGCATGTGGAGACAGAGAAGACTCTTGGGTTTCTGATAGGCACTGACT
CTCTCTGCCTATTGGTCTATTTTCCCACCCTTAGGCTGCTGGTGGTCTACCCTTGGAC
CCAGAGGTTCTTTGAGTCCTTTGGGGATCTGTCCACTCCTGATGCTGTTATGGGCAAC
CCTAAGGTGAAGGCTCATGGCAAGAAAGTGCTCGGTGCCTTTAGTGATGGCCTGGCT
CACCTGGACAACCTCAAGGGCACCTTTGCCACACTGAGTGAGCTGCACTGTGACAA
GCTGCACGTGGATCCTGAGAACTTCAGGGTGAGTCTATGGGACGCTTGATGTTTTCT
TTCCCCTTCTTTTCTATGGTTAAGTTCATGTCATAGGAAGGGGATAAGTAACAGGGT

ACAGTTTAGAATGGGAAACAGACGAATGATTGCATCAGTGTGGAAGTCTCAGGATC
GTTTTAGTTTCTTTTATTTGCTGTTCATAACAATTGTTTTCTTTTGTTTAATTCTTGCTT
TCTTTTTTTTCTTCTCCGCAATTTTTACTATTATACTTAATGCCTTAACATTGTGTAT
AACAAAAGGAAATATCTCTGAGATACATTAAGTAACTTAAAAAAAAACTTTACACA
GTCTGCCTAGTACATTACTATTTGGAATATATGTGTGCTTATTTGCATATTCATAATC
TCCCTACTTTATTTTCTTTTATTTTTAATTGATACATAATCATTATACATATTTATGGG
TTAAAGTGTAATGTTTTAATATGTGTACACATATTGACCAAATCAGGGTAATTTTGC
ATTTGTAATTTTAAAAAATGCTTTCTTCTTTTAATATACTTTTTTGTTTATCTTATTTCT
AATACTTTCCCTAATCTCTTTCTTTCAGGGCAATAATGATACAATGTATCATGCCTCT
TTGCACCATTCTAAAGAATAACAGTGATAATTTCTGGGTTAAGGCAATAGCAATATC
TCTGCATATAAATATTTCTGCATATAAATTGTAACTG

In an embodiment, the replacement sequence comprises or consists of an adenine (A) residue to correct the amino acid sequence to a glutamic acid (E) residue.

In an embodiment, to correct a mutation, e.g., at E6, e.g., E6V, in the *HBB* gene, the homology arms, e.g., the 5' and 3' homology arms, may each comprise about 1000 base pairs (bp) of sequence flanking the most distal gRNAs (e.g., 1100 bp of sequence on either side of the mutation). The 5' homology arm is shown as bold sequence, codon 6 is shown as underlined sequence, the inserted base to correct the mutation at E6, e.g., E6V, is shown as boxed sequence, and the 3' homology arm is shown as no emphasis sequence.

SEQ ID NO:393 (Template Construct 1)
ATAGGAACTTGAATCAAGGAAATGATTTTAAAACGCAGTATTCTTAGTGGACTA
GAGGAAAAAAATAATCTGAGCCAAGTAGAAGACCTTTTCCCCTCCTACCCCTAC
TTTCTAAGTCACAGAGGCTTTTTGTTCCCCCAGACACTCTTGCAGATTAGTCCA
GGCAGAAACAGTTAGATGTCCCCAGTTAACCTCCTATTTGACACCACTGATTAC
CCCATTGATAGTCACACTTTGGGTTGTAAGTGACTTTTTATTTATTTGTATTTTT
GACTGCATTAAGAGGTCTCTAGTTTTTTATCTCTTGTTTCCCAAAACCTAATAA
GTAACTAATGCACAGAGCACATTGATTTGTATTTATTCTATTTTTAGACATAATT
TATTAGCATGCATGAGCAAATTAAGAAAAACAACAACAAATGAATGCATATATA
TGTATATGTATGTGTGTATATATACACACATATATATATATATATTTTTTCTTTTCT

TACCAGAAGGTTTTAATCCAAATAAGGAGAAGATATGCTTAGAACCGAGGTAG
AGTTTTCATCCATTCTGTCCTGTAAGTATTTTGCATATTCTGGAGACGCAGGAA
GAGATCCATCTACATATCCCAAAGCTGAATTATGGTAGACAAAACTCTTCCACT
TTTAGTGCATCAACTTCTTATTTGTGTAATAAGAAAATTGGGAAAACGATCTTC
AATATGCTTACCAAGCTGTGATTCCAAATATTACGTAAATACACTTGCAAAGGA
GGATGTTTTTAGTAGCAATTTGTACTGATGGTATGGGGCCAAGAGATATATCTT
AGAGGGAGGGCTGAGGGTTTGAAGTCCAACTCCTAAGCCAGTGCCAGAAGAGC
CAAGGACAGGTACGGCTGTCATCACTTAGACCTCACCCTGTGGAGCCACACCC
TAGGGTTGGCCAATCTACTCCCAGGAGCAGGGAGGGCAGGAGCCAGGGCTGG
GCATAAAAGTCAGGGCAGAGCCATCTATTGCTTACATTTGCTTCTGACACAACT
GTGTTCACTAGCAACCTCAAACAGACACCATGGTGCATCTGACTCCTGAGGAG
AAGTCTGCCGTTACTGCCCTGTGGGGCAAGGTGAACGTGGATGAAGTTGGTGG
TGAGGCCCTGGGCAGGTTGGTATCAAGGTTACAAGACAGGTTTAAGGAGACCA
ATAGAAACTGGGCATGTGGAGACAGAGAAGACTCTTGGGTTTCTGATAGGCAC
TGACTCTCTCTGCCTATTGGTCTATTTTCCCACCCTTAGGCTGCTGGTGGTCTA
CCCTTGGACCCAGAGGTTCTTTGAGTCCTTTGGGGATCTGTCCACTCCTGATGC
TGTTATGGGCAACCCTAAGGTGAAGGCTCATGGCAAGAAAGTGCTCGGTGCCT
TTAGTGATGGCCTGGCTCACCTGGACAACCTCAAGGGCACCTTTGCCACACTG
AGTGAGCTGCACTGTGACAAGCTGCACGTGGATCCTGAGAACTTCAGGGTGAG
TCTATGGGACGCTTGATGTTTTCTTTCCCCTTCTTTTCTATGGTTAAGTTCATGT
CATAGGAAGGGGATAAGTAACAGGGTACAGTTTAGAATGGGAAACAGACGAAT
GATTGCATCAGTGTGGAAGTCTCAGGATCGTTTTAGTTTCTTTTATTTGCTGTT
CATAACAATTGTTTTCTTTTGTTTAATTCTTGCTTTCTTTTTTTTTCTTCTCCGCA
ATTTTTACTATTATACTTAATGCCTTAACATTGTGTATAACAAAAGGAAATATCT
CTGAGATACATTAAGTAACTTAAAAAAAAACTTTACACAGTCTGCCTAGTACAT
TACTATTTGGAATATATGTGTGCTTATTTGCATATTCATAATCTCCCTACTTTAT
TTTCTTTTATTTTTAATTGATACATAATCATTATACATATTTATGGGTTAAAGTG
TAATGTTTTAATATGTGTACACATATTGACCAAATCAGGGTAATTTTGCATTTGT
AATTTTAAAAAATGCTTTCTTCTTTTAATATACTTTTTTGTTTATCTTATTTCTAA
TACTTTCCCTAATCTCTTTCTTTCAGGGCAATAATGATACAATGTATCATGCCTC

TTTGCACCATTCTAAAGAATAACAGTGATAATTTCTGGGTTAAGGCAATAGCAA
TATCTCTGCATATAAATATTTCTGCATATAAATTGTAACTG

As described below in **Table 650,** shorter homology arms, e.g., 5' and/or 3' homology arms may be used.

It is contemplated herein that one or both homology arms may be shortened to avoid including certain sequence repeat elements, e.g., Alu repeats, LINE elements. For example, a 5' homology arm may be shortened to avoid a sequence repeat element. In another embodiment, a 3' homology arm may be shortened to avoid a sequence repeat element. In an embodiment, both the 5' and the 3' homology arms may be shortened to avoid including certain sequence repeat elements.

It is contemplated herein that template nucleic acids for correcting a mutation may designed for use as a single-stranded oligonucleotide (ssODN). When using a ssODN, 5' and 3' homology arms may range up to about 200 base pairs (bp) in length, e.g., at least 25, 50, 75, 100, 125, 150, 175, or 200 bp in length. Longer homology arms are also contemplated for ssODNs as improvements in oligonucleotide synthesis continue to be made.

In an embodiment, an ssODN may be used to correct a mutation, e.g., E6V in the *HBB* gene. For example, the ssODN may include 50 bp 5' and 3' homology arms as shown below. The 5' homology arm is shown as bold sequence, codon 6 is shown as underlined sequence, the inserted base to correct the E6V mutation is shown as boxed sequence, and the 3' homology arm is shown as no emphasis sequence.

SEQ ID NO:394 (Template Construct 2)
ACTGTGTTCACTAGCAACCTCAAACAGACACCATGGTGCATCTGACTCCTGGGG
AGAAGTCTGCCGTTACTGCCCTGTGGGGCAAGGTGAACGTGGATGAAGT

### Silent mutations in donor construct

It is contemplated herein that Cas9 could potentially cleave donor constructs either prior to or following homology directed repair (e.g., homologous recombination), resulting in a possible non-homologous-end-joining event and further DNA sequence mutation at the chromosomal locus of interest. Therefore, to avoid cleavage of the donor sequence before and/or after Cas9-mediated homology directed repair, alternate versions of the donor sequence may be used where silent mutations are introduced. These silent mutations may disrupt Cas9 binding and cleavage, but not disrupt the amino acid sequence of the repaired gene. For example, mutations may include those made to a donor sequence to repair the *HBB* gene, the mutant form of which can cause Sickle Cell Disease. If gRNA *HBB*-6 with the 20-base target sequence CGUUACUGCCCUGUGGGGCA (SEQ ID NO:400) is used to insert a donor sequence including **CTCCTGAGGAGAAGTCTGCCGTTACTGCCCTGTGGGGCAAGgGTGAACGTGGA TGAAGT (SEQ ID NO:395),** where the *italic* A is the base being corrected and the bracketed bases are those that match the guide RNA, the donor sequence may be changed to **CTCCTGAGGAGAAGTCTGCCGTTACTGCCCTGTGGGGCAAGaTGAACGTGGAT GAAGT (SEQ ID NO:396),** where the lowercase a has been changed from a G (lower case g in SEQ ID NO:395) at that position so that codon 15 still codes for the amino acid Arginine but the PAM sequence AGG has been modified to AGA to reduce or eliminate Cas9 cleavage at that locus.

**Table 650** below provides exemplary template nucleic acids. In an embodiment, the template nucleic acid includes the 5' homology arm and the 3' homology arm of a row from **Table 650.** In another embodiment, a 5' homology arm from the first column can be combined with a 3' homology arm from **Table 650.** In each embodiment, a combination of the 5' and 3' homology arms include a replacement sequence, e.g., an adenine (A) residue.

**Table 650**

| 5' homology arm (the number of nucleotides from SEQ ID NO:391 5'H, beginning at the 3' end of SEQ ID NO:391 5'H) | Replacement Sequence: G, A, C or T, or a cDNA sequence described herein, optionally a promoter, further optionally a poly A signal, as described herein. | 3' homology arm (the number of nucleotides from SEQ ID NO:392 3'H, beginning at the 5' end of SEQ ID NO:392 3'H) |
|---|---|---|
| 10 or more | | 10 or more |
| 20 or more | | 20 or more |
| 50 or more | | 50 or more |
| 100 or more | | 100 or more |
| 150 or more | | 150 or more |
| 200 or more | | 200 or more |
| 250 or more | | 250 or more |
| 300 or more | | 300 or more |
| 350 or more | | 350 or more |
| 400 or more | | 400 or more |
| 450 or more | | 450 or more |
| 500 or more | | 500 or more |
| 550 or more | | 550 or more |
| 600 or more | | 600 or more |
| 650 or more | | 650 or more |
| 700 or more | | 700 or more |
| 750 or more | | 750 or more |
| 800 or more | | 800 or more |
| 850 or more | | 850 or more |
| 900 or more | | 900 or more |
| 1000 or more | | 1000 or more |
| 1100 or more | | 1100 or more |
| 1200 or more | | 1200 or more |
| 1300 or more | | 1300 or more |
| 1400 or more | | 1400 or more |
| 1500 or more | | 1500 or more |
| 1600 or more | | 1600 or more |
| 1700 or more | | 1700 or more |
| 1800 or more | | 1800 or more |
| 1900 or more | | 1900 or more |
| 1200 or more | | 1200 or more |
| At least 50 but not long enough to include a repeated element. | | At least 50 but not long enough to include a repeated element. |
| At least 100 but not long enough to include a repeated element. | | At least 100 but not long enough to include a repeated element. |
| At least 150 but not long enough to include a repeated element. | | At least 150 but not long enough to include a repeated element. |
| 5 to 100 nucleotides | | 5 to 100 nucleotides |
| 10 to 150 nucleotides | | 10 to 150 nucleotides |
| 20 to 150 nucleotides | | 20 to 150 nucleotides |
| Template Construct No. 1 | | |
| Template Construct No. 2 | | |

### V.4 Single-Strand Annealing

Single strand annealing (SSA) is another DNA repair process that repairs a double-strand break between two repeat sequences present in a target nucleic acid. Repeat sequences utilized by the SSA pathway are generally greater than 30 nucleotides in length. Resection at the break ends occurs to reveal repeat sequences on both strands of the target nucleic acid. After resection, single strand overhangs containing the repeat sequences are coated with RPA protein to prevent the repeats sequences from inappropriate annealing, e.g., to themselves. RAD52 binds to and each of the repeat sequences on the overhangs and aligns the sequences to enable the annealing of the complementary repeat sequences. After annealing, the single-strand flaps of the overhangs are cleaved. New DNA synthesis fills in any gaps, and ligation restores the DNA duplex. As a result of the processing, the DNA sequence between the two repeats is deleted. The length of the deletion can depend on many factors including the location of the two repeats utilized, and the pathway or processivity of the resection.

In contrast to HDR pathways, SSA does not require a template nucleic acid to alter or correct a target nucleic acid sequence. Instead, the complementary repeat sequence is utilized.

### V.5 Other DNA Repair Pathways

### SSBR (single strand break repair)

Single-stranded breaks (SSB) in the genome are repaired by the SSBR pathway, which is a distinct mechanism from the DSB repair mechanisms discussed above. The SSBR pathway has four major stages: SSB detection, DNA end processing, DNA gap filling, and DNA ligation. A more detailed explanation is given in Caldecott, Nature Reviews Genetics 9, 619-631 (August 2008), and a summary is given here.

In the first stage, when a SSB forms, PARP1 and/or PARP2 recognize the break and recruit repair machinery. The binding and activity of PARP1 at DNA breaks is transient and it seems to accelerate SSBr by promoting the focal accumulation or stability of SSBr protein complexes at the lesion. Arguably the most important of these SSBr proteins is XRCC1, which functions as a molecular scaffold that interacts with, stabilizes, and stimulates multiple enzymatic components of the SSBr process including the protein responsible for cleaning the DNA 3' and 5' ends. For instance, XRCC1 interacts with several proteins (DNA polymerase beta, PNK, and three nucleases, APE1, APTX, and APLF) that promote end processing. APE1 has endonuclease activity. APLF exhibits endonuclease and 3' to 5' exonuclease activities. APTX has endonuclease and 3' to 5' exonuclease activity.

This end processing is an important stage of SSBR since the 3'- and/or 5'-termini of most, if not all, SSBs are 'damaged'. End processing generally involves restoring a damaged 3'-end to a hydroxylated state and and/or a damaged 5' end to a phosphate moiety, so that the ends become ligation-competent. Enzymes that can process damaged 3' termini include PNKP, APE1, and TDP1. Enzymes that can process damaged 5' termini include PNKP, DNA polymerase beta, and APTX. LIG3 (DNA ligase III) can also participate in end processing. Once the ends are cleaned, gap filling can occur.

At the DNA gap filling stage, the proteins typically present are PARP1, DNA polymerase beta, XRCC1, FEN1 (flap endonculease 1), DNA polymerase delta/epsilon, PCNA, and LIG1. There are two ways of gap filling, the short patch repair and the long patch repair. Short patch repair involves the insertion of a single nucleotide that is missing. At some SSBs, "gap filling" might continue displacing two or more nucleotides (displacement of up to 12 bases have been reported). FEN1 is an endonuclease that removes the displaced 5"-residues. Multiple DNA polymerases, including Pol β, are involved in the repair of SSBs, with the choice of DNA polymerase influenced by the source and type of SSB.

In the fourth stage, a DNA ligase such as LIG1 (Ligase I) or LIG3 (Ligase III) catalyzes joining of the ends. Short patch repair uses Ligase III and long patch repair uses Ligase I.

Sometimes, SSBR is replication-coupled. This pathway can involve one or more of CtIP, MRN, ERCC1, and FEN1. Additional factors that may promote SSBR include: aPARP, PARP1, PARP2, PARG, XRCC1, DNA polymerase b, DNA polymerase d, DNA polymerase e, PCNA, LIG1, PNK, PNKP, APE1, APTX, APLF, TDP1, LIG3, FEN1, CtIP, MRN, and ERCC1.

### MMR (mismatch repair)

Cells contain three excision repair pathways: MMR, BER, and NER. The excision repair pathways hace a common feature in that they typically recognize a lesion on one strand of the DNA, then exo/endonucleaseases remove the lesion and leave a 1-30 nucleotide gap that is sub-sequentially filled in by DNA polymerase and finally sealed with ligase. A more complete picture is given in Li, Cell Research (2008) 18:85-98, and a summary is provided here.

Mismatch repair (MMR) operates on mispaired DNA bases.

The MSH2/6 or MSH2/3 complexes both have ATPases activity that plays an important role in mismatch recognition and the initiation of repair. MSH2/6 preferentially recognizes base-base mismatches and identifies mispairs of 1 or 2 nucleotides, while MSH2/3 preferentially recognizes larger ID mispairs.

hMLH1 heterodimerizes with hPMS2 to form hMutLα which possesses an ATPase activity and is important for multiple steps of MMR. It possesses a PCNA/replication factor C (RFC)-dependent endonuclease activity which plays an important role in 3' nick-directed MMR involving EXO1. (EXO1 is a participant in both HR and MMR.) It regulates termination of mismatch-provoked excision. Ligase I is the relevant ligase for this pathway. Additional factors that may promote MMR include: EXO1, MSH2, MSH3, MSH6, MLH1, PMS2, MLH3, DNA Pol d, RPA, HMGB1, RFC, and DNA ligase I.

### Base excision repair (BER)

The base excision repair (BER) pathway is active throughout the cell cycle; it is responsible primarily for removing small, non-helix-distorting base lesions from the genome. In contrast, the related Nucleotide Excision Repair pathway (discussed in the next section) repairs bulky helix-distorting lesions. A more detailed explanation is given in Caldecott, Nature Reviews Genetics 9, 619-631 (August 2008), and a summary is given here.

Upon DNA base damage, base excision repair (BER) is initiated and the process can be simplified into five major steps: (a) removal of the damaged DNA base; (b) incision of the subsequent a basic site; (c) clean-up of the DNA ends; (d) insertion of the correct nucleotide into the repair gap; and (e) ligation of the remaining nick in the DNA backbone. These last steps are similar to the SSBR.

In the first step, a damage-specific DNA glycosylase excises the damaged base through cleavage of the N-glycosidic bond linking the base to the sugar phosphate backbone. Then AP endonuclease-1 (APE1) or bifunctional DNA glycosylases with an associated lyase activity incised the phosphodiester backbone to create a DNA single strand break (SSB). The third step of BER involves cleaning-up of the DNA ends. The fourth step in BER is conducted by Pol β that adds a new complementary nucleotide into the repair gap and in the final step XRCC1/Ligase III seals the remaining nick in the DNA backbone. This completes the short-patch BER pathway in which the majority (∼80%) of damaged DNA bases are repaired. However, if the 5'-ends in step 3 are resistant to end processing activity, following one nucleotide insertion by Pol β there is then a polymerase switch to the replicative DNA polymerases, Pol δ/ε, which then add ∼2-8 more nucleotides into the DNA repair gap. This creates a 5'-flap structure, which is recognized and excised by flap endonuclease-1 (FEN-1) in association with the processivity factor proliferating cell nuclear antigen (PCNA). DNA ligase I then seals the remaining nick in the DNA backbone and completes long-patch BER. Additional factors that may promote the BER pathway include: DNA glycosylase, APE1, Polb, Pold, Pole, XRCC1, Ligase III, FEN-1, PCNA, RECQL4, WRN, MYH, PNKP, and APTX.

### Nucleotide excision repair (NER)

Nucleotide excision repair (NER) is an important excision mechanism that removes bulky helix-distorting lesions from DNA. Additional details about NER are given in Marteijn et al., Nature Reviews Molecular Cell Biology 15, 465-481 (2014), and a summary is given here. NER a broad pathway encompassing two smaller pathways: global genomic NER (GG-NER) and transcription coupled repair NER (TC-NER). GG-NER and TC-NER use different factors for recognizing DNA damage. However, they utilize the same machinery for lesion incision, repair, and ligation.

Once damage is recognized, the cell removes a short single-stranded DNA segment that contains the lesion. Endonucleases XPF/ERCC1 and XPG (encoded by ERCC5) remove the lesion by cutting the damaged strand on either side of the lesion, resulting in a single-strand gap of 22-30 nucleotides. Next, the cell performs DNA gap filling synthesis and ligation. Involved in this process are: PCNA, RFC, DNA Pol δ, DNA Pol ε or DNA Pol κ, and DNA ligase I or XRCC1/Ligase III. Replicating cells tend to use DNA pol ε and DNA ligase I, while non-replicating cells tend to use DNA Pol δ, DNA Pol κ, and the XRCC1/ Ligase III complex to perform the ligation step.

NER can involve the following factors: XPA-G, POLH, XPF, ERCC1, XPA-G, and LIG1. Transcription-coupled NER (TC-NER) can involve the following factors: CSA, CSB, XPB, XPD, XPG, ERCC1, and TTDA. Additional factors that may promote the NER repair pathway include XPA-G, POLH, XPF, ERCC1, XPA-G, LIG1, CSA, CSB, XPA, XPB, XPC, XPD, XPF, XPG, TTDA, UVSSA, USP7, CETN2, RAD23B, UV-DDB, CAK subcomplex, RPA, and PCNA.

### Interstrand Crosslink (ICL)

A dedicated pathway called the ICL repair pathway repairs interstrand crosslinks. Interstrand crosslinks, or covalent crosslinks between bases in different DNA strand, can occur during replication or transcription. ICL repair involves the coordination of multiple repair processes, in particular, nucleolytic activity, translesion synthesis (TLS), and HDR. Nucleases are recruited to excise the ICL on either side of the crosslinked bases, while TLS and HDR are coordinated to repair the cut strands. ICL repair can involve the following factors: endonucleases, e.g., XPF and RAD51C, endonucleases such as RAD51, translesion polymerases, e.g., DNA polymerase zeta and Rev1), and the Fanconi anemia (FA) proteins, e.g., FancJ.

### Other pathways

Several other DNA repair pathways exist in mammals.

Translesion synthesis (TLS) is a pathway for repairing a single stranded break left after a defective replication event and involves translesion polymerases, e.g., DNA pol□ and Rev1.

Error-free postreplication repair (PRR) is another pathway for repairing a single stranded break left after a defective replication event.

### V.6 Examples of gRNAs in Genome Editing Methods

gRNA molecules as described herein can be used with Cas9 molecules that generate a double strand break or a single strand break to alter the sequence of a target nucleic acid, e.g., a target position or target genetic signature. gRNA molecules useful in these methods are described below.

In an embodiment, the gRNA, e.g., a chimeric gRNA, is configured such that it comprises one or more of the following properties;
a) it can position, e.g., when targeting a Cas9 molecule that makes double strand breaks, a double strand break (i) within 50, 100, 150, 200, 250, 300, 350, 400, 450, or 500 nucleotides of a target position, or (ii) sufficiently close that the target position is within the region of end resection;
b) it has a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
c)
   (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes, S. thermophilus, S. aureus,* or *N. meningitidis* tail and proximal domain, or a sequence that differs by no more than 1, 2, 3, 4, 5; 6, 7, 8, 9 or 10 nucleotides therefrom;
   (ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes, S. thermophilus, S. aureus,* or *N. meningitidis* tail domain, or a sequence that differs by no more than 1, 2, 3, 4, 5; 6, 7, 8, 9 or 10 nucleotides therefrom; or
   (v) the tail domain comprises 15, 20, 25, 30, 35, 40 nucleotides or all of the corresponding portions of a naturally occurring tail domain, e.g., a naturally occurring *S. pyogenes, S. thermophilus, S. aureus,* or *N. meningitidis* tail domain.

In an embodiment, the gRNA is configured such that it comprises properties: a and b(i).

In an embodiment, the gRNA is configured such that it comprises properties: a and b(ii).

In an embodiment, the gRNA is configured such that it comprises properties: a and b(iii).

In an embodiment, the gRNA is configured such that it comprises properties: a and b(iv).

In an embodiment, the gRNA is configured such that it comprises properties: a and b(v).

In an embodiment, the gRNA is configured such that it comprises properties: a and b(vi).

In an embodiment, the gRNA is configured such that it comprises properties: a and b(vii).

In an embodiment, the gRNA is configured such that it comprises properties: a and b(viii).

In an embodiment, the gRNA is configured such that it comprises properties: a and b(ix).

In an embodiment, the gRNA is configured such that it comprises properties: a and b(x).

In an embodiment, the gRNA is configured such that it comprises properties: a and b(xi).

In an embodiment, the gRNA is configured such that it comprises properties: a and c.

In an embodiment, the gRNA is configured such that in comprises properties: a, b, and c.

In an embodiment, the gRNA is configured such that in comprises properties: a(i), b(i), and c(i).

In an embodiment, the gRNA is configured such that in comprises properties: a(i), b(i), and c(ii).

In an embodiment, the gRNA is configured such that in comprises properties: a(i), b(ii), and c(i).

In an embodiment, the gRNA is configured such that in comprises properties: a(i), b(ii), and c(ii).

In an embodiment, the gRNA is configured such that in comprises properties: a(i), b(iii), and c(i).

In an embodiment, the gRNA is configured such that in comprises properties: a(i), b(iii), and c(ii).

In an embodiment, the gRNA is configured such that in comprises properties: a(i), b(iv), and c(i).

In an embodiment, the gRNA is configured such that in comprises properties: a(i), b(iv), and c(ii).

In an embodiment, the gRNA is configured such that in comprises properties: a(i), b(v), and c(i).

In an embodiment, the gRNA is configured such that in comprises properties: a(i), b(v), and c(ii).

In an embodiment, the gRNA is configured such that in comprises properties: a(i), b(vi), and c(i).

In an embodiment, the gRNA is configured such that in comprises properties: a(i), b(vi), and c(ii).

In an embodiment, the gRNA is configured such that in comprises properties: a(i), b(vii), and c(i).

In an embodiment, the gRNA is configured such that in comprises properties: a(i), b(vii), and c(ii).

In an embodiment, the gRNA is configured such that in comprises properties: a(i), b(viii), and c(i).

In an embodiment, the gRNA is configured such that in comprises properties: a(i), b(viii), and c(ii).

In an embodiment, the gRNA is configured such that in comprises properties: a(i), b(ix), and c(i).

In an embodiment, the gRNA is configured such that in comprises properties: a(i), b(ix), and c(ii).

In an embodiment, the gRNA is configured such that in comprises properties: a(i), b(x), and c(i).

In an embodiment, the gRNA is configured such that in comprises properties: a(i), b(x), and c(ii).

In an embodiment, the gRNA is configured such that in comprises properties: a(i), b(xi), and c(i).

In an embodiment, the gRNA is configured such that in comprises properties: a(i), b(xi), and c(ii).

In an embodiment, the gRNA, e.g., a chimeric gRNA, is configured such that it comprises one or more of the following properties;
a) one or both of the gRNAs can position, e.g., when targeting a Cas9 molecule that makes single strand breaks, a single strand break within (i) 50, 100, 150, 200, 250, 300, 350, 400, 450, or 500 nucleotides of a target position, or (ii) sufficiently close that the target position is within the region of end resection;
b) one or both have a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
c)
   (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes, S. thermophilus, S. aureus,* or *N. meningitidis* tail and proximal domain, or a sequence that differs by no more than 1, 2, 3, 4, 5; 6, 7, 8, 9 or 10 nucleotides therefrom;
   (ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes, S. thermophilus, S. aureus,* or *N. meningitidis* tail domain, or a sequence that differs by no more than 1, 2, 3, 4, 5; 6, 7, 8, 9 or 10 nucleotides therefrom; or
   (v) the tail domain comprises 15, 20, 25, 30, 35, 40 nucleotides or all of the corresponding portions of a naturally occurring tail domain, e.g., a naturally occurring *S. pyogenes, S. thermophilus, S. aureus,* or *N. meningitidis* tail domain.

In an embodiment, the gRNA is configured such that it comprises properties: a and b(i).

In an embodiment, the gRNA is configured such that it comprises properties: a and b(ii).

In an embodiment, the gRNA is configured such that it comprises properties: a and b(iii).

In an embodiment, the gRNA is configured such that it comprises properties: a and b(iv).

In an embodiment, the gRNA is configured such that it comprises properties: a and b(v).

In an embodiment, the gRNA is configured such that it comprises properties: a and b(vi).

In an embodiment, the gRNA is configured such that it comprises properties: a and b(vii).

In an embodiment, the gRNA is configured such that it comprises properties: a and b(viii).

In an embodiment, the gRNA is configured such that it comprises properties: a and b(ix).

In an embodiment, the gRNA is configured such that it comprises properties: a and b(x).

In an embodiment, the gRNA is configured such that it comprises properties: a and b(xi).

In an embodiment, the gRNA is configured such that it comprises properties: a and c.

In an embodiment, the gRNA is configured such that in comprises properties: a, b, and c.

In an embodiment, the gRNA is configured such that in comprises properties: a(i), b(i), and c(i).

In an embodiment, the gRNA is configured such that in comprises properties: a(i), b(i), and c(ii).

In an embodiment, the gRNA is configured such that in comprises properties: a(i), b(ii), and c(i).

In an embodiment, the gRNA is configured such that in comprises properties: a(i), b(ii), and c(ii).

In an embodiment, the gRNA is configured such that in comprises properties: a(i), b(iii), and c(i).

In an embodiment, the gRNA is configured such that in comprises properties: a(i), b(iii), and c(ii).

In an embodiment, the gRNA is configured such that in comprises properties: a(i), b(iv), and c(i).

In an embodiment, the gRNA is configured such that in comprises properties: a(i), b(iv), and c(ii).

In an embodiment, the gRNA is configured such that in comprises properties: a(i), b(v), and c(i).

In an embodiment, the gRNA is configured such that in comprises properties: a(i), b(v), and c(ii).

In an embodiment, the gRNA is configured such that in comprises properties: a(i), b(vi), and c(i).

In an embodiment, the gRNA is configured such that in comprises properties: a(i), b(vi), and c(ii).

In an embodiment, the gRNA is configured such that in comprises properties: a(i), b(vii), and c(i).

In an embodiment, the gRNA is configured such that in comprises properties: a(i), b(vii), and c(ii).

In an embodiment, the gRNA is configured such that in comprises properties: a(i), b(viii), and c(i).

In an embodiment, the gRNA is configured such that in comprises properties: a(i), b(viii), and c(ii).

In an embodiment, the gRNA is configured such that in comprises properties: a(i), b(ix), and c(i).

In an embodiment, the gRNA is configured such that in comprises properties: a(i), b(ix), and c(ii).

In an embodiment, the gRNA is configured such that in comprises properties: a(i), b(x), and c(i).

In an embodiment, the gRNA is configured such that in comprises properties: a(i), b(x), and c(ii).

In an embodiment, the gRNA is configured such that in comprises properties: a(i), b(xi), and c(i).

In an embodiment, the gRNA is configured such that in comprises properties: a(i), b(xi), and c(ii).

In an embodiment, the gRNA is used with a Cas9 nickase molecule having HNH activity, e.g., a Cas9 molecule having the RuvC activity inactivated, e.g., a Cas9 molecule having a mutation at D10, e.g., the D10A mutation.

In an embodiment, the gRNA is used with a Cas9 nickase molecule having RuvC activity, e.g., a Cas9 molecule having the HNH activity inactivated, e.g., a Cas9 molecule having a mutation at H840, e.g., a H840A or a mutation at N863, e.g., N863A.

In an embodiment, a pair of gRNAs, e.g., a pair of chimeric gRNAs, comprising a first and a second gRNA, is configured such that they comprises one or more of the following properties;
a) one or both of the gRNAs can position, e.g., when targeting a Cas9 molecule that makes single strand breaks, a single strand break within (i) 50, 100, 150, 200, 250, 300, 350, 400, 450, or 500 nucleotides of a target position, or (ii) sufficiently close that the target position is within the region of end resection;
b) one or both have a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides;
c) for one or both:
   (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes, S. thermophilus, S. aureus,* or *N. meningitidis* tail and proximal domain, or a sequence that differs by no more than 1, 2, 3, 4, 5; 6, 7, 8, 9 or 10 nucleotides therefrom;
   (ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes, S. thermophilus, S. aureus,* or *N. meningitidis* tail domain; or, or a sequence that differs by no more than 1, 2, 3, 4, 5; 6, 7, 8, 9 or 10 nucleotides therefrom; or
   (v) the tail domain comprises 15, 20, 25, 30, 35, 40 nucleotides or all of the corresponding portions of a naturally occurring tail domain, e.g., a naturally occurring *S. pyogenes, S. thermophilus, S. aureus,* or *N. meningitidis* tail domain;
d) the gRNAs are configured such that, when hybridized to target nucleic acid, they are separated by 0-50, 0-100, 0-200, at least 10, at least 20, at least 30 or at least 50 nucleotides;
e) the breaks made by the first gRNA and second gRNA are on different strands; and
f) the PAMs are facing outwards.

In an embodiment, one or both of the gRNAs is configured such that it comprises properties: a and b(i).

In an embodiment, one or both of the gRNAs is configured such that it comprises properties: a and b(ii).

In an embodiment, one or both of the gRNAs is configured such that it comprises properties: a and b(iii).

In an embodiment, one or both of the gRNAs is configured such that it comprises properties: a and b(iv).

In an embodiment, one or both of the gRNAs is configured such that it comprises properties: a and b(v).

In an embodiment, one or both of the gRNAs is configured such that it comprises properties: a and b(vi).

In an embodiment, one or both of the gRNAs is configured such that it comprises properties: a and b(vii).

In an embodiment, one or both of the gRNAs is configured such that it comprises properties: a and b(viii).

In an embodiment, one or both of the gRNAs is configured such that it comprises properties: a and b(ix).

In an embodiment, one or both of the gRNAs is configured such that it comprises properties: a and b(x).

In an embodiment, one or both of the gRNAs is configured such that it comprises properties: a and b(xi).

In an embodiment, one or both of the gRNAs configured such that it comprises properties: a and c.

In an embodiment, one or both of the gRNAs is configured such that it comprises properties: a, b, and c.

In an embodiment, one or both of the gRNAs is configured such that it comprises properties: a(i), b(i), and c(i).

In an embodiment, one or both of the gRNAs is configured such that it comprises properties: a(i), b(i), and c(ii).

In an embodiment, one or both of the gRNAs is configured such that it comprises properties: a(i), b(i), c, and d.

In an embodiment, one or both of the gRNAs is configured such that it comprises properties: a(i), b(i), c, and e.

In an embodiment, one or both of the gRNAs is configured such that it comprises properties: a(i), b(i), c, d, and e.

In an embodiment, one or both of the gRNAs is configured such that it comprises properties: a(i), b(ii), and c(i).

In an embodiment, one or both of the gRNAs is configured such that it comprises properties: a(i), b(ii), and c(ii).

In an embodiment, one or both of the gRNAs is configured such that it comprises properties: a(i), b(ii), c, and d.

In an embodiment, one or both of the gRNAs is configured such that it comprises properties: a(i), b(ii), c, and e.

In an embodiment, one or both of the gRNAs is configured such that it comprises properties: a(i), b(ii), c, d, and e.

In an embodiment, one or both of the gRNAs is configured such that it comprises properties: a(i), b(iii), and c(i).

In an embodiment, one or both of the gRNAs is configured such that it comprises properties: a(i), b(iii), and c(ii).

In an embodiment, one or both of the gRNAs is configured such that it comprises properties: a(i), b(iii), c, and d.

In an embodiment, one or both of the gRNAs is configured such that it comprises properties: a(i), b(iii), c, and e.

In an embodiment, one or both of the gRNAs is configured such that it comprises properties: a(i), b(iii), c, d, and e.

In an embodiment, one or both of the gRNAs is configured such that it comprises properties: a(i), b(iv), and c(i).

In an embodiment, one or both of the gRNAs is configured such that it comprises properties: a(i), b(iv), and c(ii).

In an embodiment, one or both of the gRNAs is configured such that it comprises properties: a(i), b(iv), c, and d.

In an embodiment, one or both of the gRNAs is configured such that it comprises properties: a(i), b(iv), c, and e.

In an embodiment, one or both of the gRNAs is configured such that it comprises properties: a(i), b(iv), c, d, and e.

In an embodiment, one or both of the gRNAs is configured such that it comprises properties: a(i), b(v), and c(i).

In an embodiment, one or both of the gRNAs is configured such that it comprises properties: a(i), b(v), and c(ii).

In an embodiment, one or both of the gRNAs is configured such that it comprises properties: a(i), b(v), c, and d.

In an embodiment, one or both of the gRNAs is configured such that it comprises properties: a(i), b(v), c, and e.

In an embodiment, one or both of the gRNAs is configured such that it comprises properties: a(i), b(v), c, d, and e.

In an embodiment, one or both of the gRNAs is configured such that it comprises properties: a(i), b(vi), and c(i).

In an embodiment, one or both of the gRNAs is configured such that it comprises properties: a(i), b(vi), and c(ii).

In an embodiment, one or both of the gRNAs is configured such that it comprises properties: a(i), b(vi), c, and d.

In an embodiment, one or both of the gRNAs is configured such that it comprises properties: a(i), b(vi), c, and e.

In an embodiment, one or both of the gRNAs is configured such that it comprises properties: a(i), b(vi), c, d, and e.

In an embodiment, one or both of the gRNAs is configured such that it comprises properties: a(i), b(vii), and c(i).

In an embodiment, one or both of the gRNAs is configured such that it comprises properties: a(i), b(vii), and c(ii).

In an embodiment, one or both of the gRNAs is configured such that it comprises properties: a(i), b(vii), c, and d.

In an embodiment, one or both of the gRNAs is configured such that it comprises properties: a(i), b(vii), c, and e.

In an embodiment, one or both of the gRNAs is configured such that it comprises properties: a(i), b(vii), c, d, and e.

In an embodiment, one or both of the gRNAs is configured such that it comprises properties: a(i), b(viii), and c(i).

In an embodiment, one or both of the gRNAs is configured such that it comprises properties: a(i), b(viii), and c(ii).

In an embodiment, one or both of the gRNAs is configured such that it comprises properties: a(i), b(viii), c, and d.

In an embodiment, one or both of the gRNAs is configured such that it comprises properties: a(i), b(viii), c, and e.

In an embodiment, one or both of the gRNAs is configured such that it comprises properties: a(i), b(viii), c, d, and e.

In an embodiment, one or both of the gRNAs is configured such that it comprises properties: a(i), b(ix), and c(i).

In an embodiment, one or both of the gRNAs is configured such that it comprises properties: a(i), b(ix), and c(ii).

In an embodiment, one or both of the gRNAs is configured such that it comprises properties: a(i), b(ix), c, and d.

In an embodiment, one or both of the gRNAs is configured such that it comprises properties: a(i), b(ix), c, and e.

In an embodiment, one or both of the gRNAs is configured such that it comprises properties: a(i), b(ix), c, d, and e.

In an embodiment, one or both of the gRNAs is configured such that it comprises properties: a(i), b(x), and c(i).

In an embodiment, one or both of the gRNAs is configured such that it comprises properties: a(i), b(x), and c(ii).

In an embodiment, one or both of the gRNAs is configured such that it comprises properties: a(i), b(x), c, and d.

In an embodiment, one or both of the gRNAs is configured such that it comprises properties: a(i), b(x), c, and e.

In an embodiment, one or both of the gRNAs is configured such that it comprises properties: a(i), b(x), c, d, and e.

In an embodiment, one or both of the gRNAs is configured such that it comprises properties: a(i), b(xi), and c(i).

In an embodiment, one or both of the gRNAs is configured such that it comprises properties: a(i), b(xi), and c(ii).

In an embodiment, one or both of the gRNAs is configured such that it comprises properties: a(i), b(xi), c, and d.

In an embodiment, one or both of the gRNAs is configured such that it comprises properties: a(i), b(xi), c, and e.

In an embodiment, one or both of the gRNAs is configured such that it comprises properties: a(i), b(xi), c, d, and e.

In an embodiment, the gRNAs are used with a Cas9 nickase molecule having HNH activity, e.g., a Cas9 molecule having the RuvC activity inactivated, e.g., a Cas9 molecule having a mutation at D10, e.g., the D10A mutation.

In an embodiment, the gRNAs are used with a Cas9 nickase molecule having RuvC activity, e.g., a Cas9 molecule having the HNH activity inactivated, e.g., a Cas9 molecule having a mutation at H840, e.g., a H840A or a mutation at N863, e.g., N863A.

### VI. Target Cells and Genes

In some embodiments, Cas9 molecules, gRNA molecules (e.g., a Cas9 molecule/gRNA molecule RNP complex), and optionally donor template nucleic acids, of the present disclosure can be used to manipulate a cell *ex vivo,* e.g., to edit a target nucleic acid.

In an embodiment, a cell is manipulated *ex vivo* by editing (e.g., inducing a mutation in) one or more target genes, e.g., as described herein. In some embodiments, the expression of one or more target genes is modulated. In another embodiment, a cell is manipulated *ex vivo* by editing (e.g., inducing a mutation in) one or more target genes and/or modulating the expression of one or more target genes, and administered to a subject. Sources of target cells for *ex vivo* manipulation may include, e.g., the subject's blood, the subject's cord blood, or the subject's bone marrow. Sources of target cells for *ex vivo* manipulation may also include, e.g., heterologous donor blood, cord blood, or bone marrow.

### VI.1 T cells (e.g., targeting FAS, BID, CTLA4, PDCD1, CBLB, PTPN6, TRAC or TRBC genes)

In one aspect, the target cell is a T cell, e.g., a CD8+ T cell (e.g., a CD8+ naive T cell, central memory T cell, or effector memory T cell), a CD4+ T cell, a natural killer T cell (NKT cells), a regulatory T cell (Treg), a stem cell memory T cell, a lymphoid progenitor cell a hematopoietic stem cell, a natural killer cell (NK cell) or a dendritic cell. In an embodiment, the target cell is an induced pluripotent stem cells (iPS) cell or a cell derived from an iPS cell, e.g., an iPS cell generated from a subject, manipulated to alter (e.g., induce a mutation in) or manipulate the expression of one or more target genes, e.g., *FAS, BID, CTLA4, PDCD1, CBLB, PTPN6, TRAC* or *TRBC* gene, and differentiated into, e.g., a T cell, e.g., a CD8+ T cell (e.g., a CD8+ naive T cell, central memory T cell, or effector memory T cell), a CD4+ T cell, a stem cell memory T cell, a lymphoid progenitor cell or a hematopoietic stem cell.

In an embodiment, the target cell is manipulated *ex vivo* by editing (e.g., introducing a mutation in) the *FAS, BID, CTLA4, PDCD1, CBLB, PTPN6, TRAC* or *TRBC* target gene and/or modulating the expression of the *FAS, BID, CTLA4, PDCD1, CBLB, PTPN6, TRAC* or *TRBC* target gene, and administered to the subject. Sources of target cells for *ex vivo* manipulation may include, by way of example, the subject's blood, the subject's cord blood, or the subject's bone marrow. Sources of target cells for *ex vivo* manipulation may also include, by way of example, heterologous donor blood, cord blood, or bone marrow.

In an embodiment, the target cell has been altered to contain specific T cell receptor (TCR) genes (e.g., a *TRAC* and *TRBC* gene). In another embodiment, the TCR has binding specificity for a tumor associated antigen, e.g., carcinoembryonic antigen (CEA), GP100, melanoma antigen recognized by T cells 1 (MART1), melanoma antigen A3 (MAGEA3), NYESO1 or p53.

In an embodiment, the target cell has been altered to contain a specific chimeric antigen receptor (CAR). In an embodiment, the CAR has binding specificity for a tumor associated antigen, e.g., CD19, CD20, carbonic anhydrase IX (CAIX), CD171, CEA, ERBB2, GD2, alpha-folate receptor, Lewis Y antigen, prostate specific membrane antigen (PSMA) or tumor associated glycoprotein 72 (TAG72).

In another embodiment, the target cell has been altered to bind one or more of the following tumor antigens, e.g., by a TCR or a CAR. Tumor antigens may include, but are not limited to, AD034, AKT1, BRAP, CAGE, CDX2, CLP, CT-7, CT8/HOM-TES-85, cTAGE-1, Fibulin-1, HAGE, HCA587/MAGE-C2, hCAP-G, HCE661, HER2/neu, HLA-Cw, HOM-HD-21/Galectin9, HOM-MEEL-40/SSX2, HOM-RCC-3.1.3/CAXII, HOXA7, HOXB6, Hu, HUB1, KM-HN-3, KM-KN-1, KOC1, KOC2, KOC3, KOC3, LAGE-1, MAGE-1, MAGE-4a, MPP11, MSLN, NNP-1, NY-BR-1, NY-BR-62, NY-BR-85, NY-CO-37, NY-CO-38, NY-ESO-1, NY-ESO-5, NY-LU-12, NY-REN-10, NY-REN-19/LKB/STK11, NY-REN-21, NY-REN-26/BCR, NY-REN-3/NY-CO-38, NY-REN-33/SNC6, NY-REN-43, NY-REN-65, NY-REN-9, NY-SAR-35, OGFr, PLU-1, Rab38, RBPJkappa, RHAMM, SCP1, SCP-1, SSX3, SSX4, SSX5, TOP2A, TOP2B, or Tyrosinase.

### Improving cancer immunotherapy

Adoptive transfer of genetically engineered T cells has entered clinical testing as a cancer therapeutic modality. Typically, the approach consists of the following steps: 1) obtaining leukocytes from the subject by apheresis; 2) selecting/enriching for T cells; 3) activating the T cells by cytokine treatment; 4) introducing cloned T cell receptor (TCR) genes or a chimeric antigen receptor (CAR) gene by retroviral transduction, lentiviral transduction or electroporation; 5) expanding the T cells by cytokine treatment; 6) conditioning the subject, usually by lymphodepletion; and 7) infusion of the engineered T cells into the subject.

Sources of cloned TCR genes (*TRAC* and *TRBC*) include rare T cell populations isolated from individuals with particular malignancies and T cell clones isolated from T cell receptor-humanized mice immunized with specific tumor antigens or tumor cells. Following adoptive transfer, TCR-engineered T cells recognize their cognate antigen peptides presented by major histocompatibility complex (MHC) proteins on the tumor cell surface. Antigen engagement stimulates signal transduction pathways leading to T cell activation and proliferation. Stimulated T cells then mount a cytotoxic anti-tumor cell response, typically involving a secreted complex comprising Granzyme B, perforin and granulysin, inducing tumor cell apoptosis.

Chimeric antigen receptor (CAR) genes encode artificial T cell receptors comprising an extra-cellular tumor antigen binding domain, typically derived from the single-chain antibody variable fragment (scFv) domain of a monoclonal antibody, fused via hinge and transmembrane domains to a cytoplasmic effector domain. The effector domain is typically derived from the CD3-zeta chain of the T cell co-receptor complex, and can also include domains derived from CD28 and/or CD137 receptor proteins. The CAR extra-cellular domain binds the tumor antigen in an MHC-independent manner leading to T cell activation and proliferation, culminating in cytotoxic anti-tumor activity as described for TCR engineered T cells.

To date, at least 15 different tumor antigens have been targeted in clinical trials of engineered T cells. In several trials, anti-tumor activity has been reported. The greatest success has been achieved in hematologic malignancies. For example, adoptive transfer of CAR-T cells engineered to target the B cell antigen, CD19, led to multiple partial and complete responses in subjects with lymphoma, acute lymphoblastic leukemia, acute lymphocytic leukemia and B-cell acute lymphocytic leukemia. In contrast, trials targeting other tumor types, especially solid tumors, including renal cell carcinoma, neuroblastoma, colorectal cancer, breast cancer, ovarian cancer, melanoma, sarcoma and prostate cancer, have been less successful. In many of these trials, very few patients experienced objective responses. Thus, there is a need to improve the anti-tumor efficacy of adoptively transferred engineered T cells.

In order for engineered T cells to mount an effective anti-tumor response they need to: 1) proliferate adequately following transfer to the subject to provide a sufficient number of specific tumor-targeting T cells; 2) survive in the subject for a length of time sufficient to maintain the required anti-tumor activity; and 3) evade the influence of suppressive factors produced by immune cells, tumor cells and other cells in the tumor environment so that the engineered T cells maintain a functional anti-tumor phenotype. Insufficient proliferation and/or survival, as well as susceptibility to inhibitory factors, can contribute to lack of efficacy of engineered T cells in subjects suffering from cancer. The methods and compositions disclosed herein address these issues in order to improve efficacy of engineered T cells as a cancer therapeutic modality.

In an embodiment, compositions and methods described herein can be used to affect proliferation of engineered T cells by altering the *CBLB* gene. While not wishing to be bound by theory, it is considered that reduced or absent expression of Casitas B-lineage lymphoma b protein (encoded by *CBLB*) reduces the requirement for exogenous interleukin signaling to promote proliferation of engineered T cells following transfer to the subject (Stromnes, I. M. et al., 2010 J. Clin. Invest. 120, 3722-3734).

In an embodiment, compositions and methods described herein can be used to affect proliferation of engineered T cells by altering the *PTPN6* gene. While not wishing to be bound by theory, it is considered that reduced or absent expression of Src homology region 2 domain-containing phosphatase-1 protein (encoded by *PTPN6*) leads to increased short-term accumulation of transferred T cells with subsequently improved anti-tumor activity (Stromnes, I. M. et al., 2012 J. Immunol. 189, 1812-1825).

In an embodiment, compositions and methods described herein can be used to affect proliferation of engineered T cells by altering the *FAS* gene. While not wishing to be bound by theory, it is considered that reduced or absent expression of the Fas protein will inhibit induction of T cell apoptosis by Fas-ligand; a factor expressed by many cancer types (Dotti, G. et al., 2005 Blood 105, 4677-4684).

In an embodiment, compositions and methods described herein can be used to affect proliferation of engineered T cells by altering the *BID* gene. While not wishing to be bound by theory, it is considered that reduced or absent expression of the Bid protein prevents the induction of T cell apoptosis following activation of the Fas pathway (Lei, X. Y. et al., 2009 Immunol. Lett. 122, 30-36).

In an embodiment, compositions and methods described herein can be used to decrease the effect of immune suppressive factors on engineered T cells by altering the *CTLA4* gene. While not wishing to be bound by theory, it is considered that reduced or absent expression of cytotoxic T-lymphocyte-associated antigen 4 (encoded by *CTLA4*) abrogates the induction of a non-responsive state ("anergy") following binding of CD80 or CD86 expressed by antigen presenting cells in the tumor environment (Shrikant, P. et al, 1999 Immunity 11, 483-493).

In an embodiment, compositions and methods described herein can be used to decrease the effect of immune suppressive factors on engineered T cells by altering the *PDCD1* gene. While not wishing to be bound by theory, it is considered that reduced or absent expression of the Programmed Cell Death Protein 1 (encoded by *PDCD1*) prevents induction of T cell apoptosis by engagement of PD1 Ligand expressed by tumor cells or cells in the tumor environment (Topalian, S. L. et al., 2012 N. Engl. J. Med. 366, 2443-2454).

In an embodiment, compositions and methods described herein can be used to improve T cell specificity and safety by altering the *TRAC* and/or *TRBC* gene. While not wishing to be bound by theory, it is considered that reduced or absent expression of T-cell receptors (encoded by *TRAC* and *TRBC*) prevents graft vs. host disease by eliminating T cell receptor recognition of and response to host tissues. This approach, therefore, could be used to generate "off the shelf' T cells (Torikai et al., 2012 Blood 119, 5697-5705). Also, while not wishing to be bound by theory, it is considered that reduced or absent expression of the *TRAC* and/or *TRBC* gene will reduce or eliminate mis-pairing of endogenous T cell receptors with exogenously introduced engineered T cell receptors, thus improving therapeutic efficacy (Provasi et al., 2012, Nature Medicine 18, 807-815).

In an embodiment, compositions and methods described herein can be used to decrease one or more of *FAS, BID, CTLA4, PDCD1, CBLB, PTPN6, TRAC* and *TRBC* genes to improve treatment of cancer immunotherapy using engineered T cells.

Disclosed herein are approaches to treat cancer via immunotherapy, using the compositions and methods described herein.

In one approach, one or more of the *FAS, BID, CTLA4, PDCD1, CBLB, PTPN6, TRAC* and *TRBC* genes are targeted as a targeted knockout or knockdown, e.g., to affect T cell proliferation, survival and/or function. In an embodiment, said approach comprises knocking out or knocking down one T-cell expressed gene (e.g., *FAS, BID, CTLA4, PDCD1, CBLB, PTPN6, TRAC* or *TRBC* gene). In another embodiment, the approach comprises knocking out or knocking down two T-cell expressed genes, e.g., two of *FAS, BID, CTLA4, PDCD1, CBLB, PTPN6, TRAC* or *TRBC* genes. In another embodiment, the approach comprises knocking out or knocking down three T-cell expressed genes, e.g., three of *FAS, BID, CTLA4, PDCD1, CBLB, PTPN6, TRAC* or *TRBC* genes. In another embodiment, the approach comprises knocking out or knocking down four T-cell expressed genes, e.g., four of *FAS, BID, CTLA4, PDCD1, CBLB, PTPN6, TRAC* or *TRBC* genes. In another embodiment, the approach comprises knocking out or knocking down five T-cell expressed genes, e.g., five of *FAS, BID, CTLA4, PDCD1, CBLB, PTPN6, TRAC* or *TRBC* genes. In another embodiment, the approach comprises knocking out or knocking down six T-cell expressed genes, e.g., six of *FAS, BID, CTLA4, PDCD1, CBLB, PTPN6, TRAC* or *TRBC* genes. In another embodiment, the approach comprises knocking out or knocking down seven T-cell expressed genes, e.g., seven of *FAS, BID, CTLA4, PDCD1, CBLB, PTPN6, TRAC* or *TRBC* genes. In another embodiment, the approach comprises knocking out or knocking down eight T-cell expressed genes, e.g., each of *FAS, BID, CTLA4, PDCD1, CBLB, PTPN6, TRAC* and *TRBC* genes.

While not wishing to be bound by theory, it is considered that adoptive transfer of genetically engineered T cells may provide a potential treatment for cancer. Genes encoding cell surface receptors are inserted into the T cells. The genetically engineered T cells are able to detect tumor associated antigens, which can be used to discriminate tumor cells from most normal tissues.

Knockout or knockdown of one or two alleles of the target gene (e.g., *FAS, BID, CTLA4, PDCD1, CBLB, PTPN6, TRAC* or *TRBC* gene) may be performed after disease onset, but preferably early in the disease course.

In an embodiment, the methods comprise initiating treatment of a subject after disease onset. In an embodiment, the method comprises initiating treatment of a subject well after disease onset, e.g., 1, 2, 3, 4, 5, 6, 7, 8, 9, 10, 12, 24, or 36 months after onset of cancer.

In an embodiment, the method comprises initiating treatment of a subject in an advanced stage of disease.

Overall, initiation of treatment for subjects at all stages of disease is expected to be of benefit to subjects.

Cancers that may be treated using the compositions and methods disclosed herein include cancers of the blood and solid tumors. For example, cancers that may be treated using the compositions and methods disclosed herein include, but are not limited to, lymphoma, chronic lymphocytic leukemia (CLL), B cell acute lymphocytic leukemia (B-ALL), acute lymphoblastic leukemia, acute myeloid leukemia, non-Hodgkin's lymphoma (NHL), diffuse large cell lymphoma (DELL), multiple myeloma, renal cell carcinoma (RCC), neuroblastoma, colorectal cancer, breast cancer, ovarian cancer, melanoma, sarcoma, prostate cancer, lung cancer, esophageal cancer, hepatocellular carcinoma, pancreatic cancer, astrocytoma, mesothelioma, head and neck cancer, and medulloblastoma.

### Other embodiments involving FAS, BID, CTLA4, PDCD1, CBLB, PTPN6, TRAC or TRBC genes

In an embodiment, methods and compositions discussed herein can be used to affect T cell proliferation (e.g., by inactivating genes that inhibit T cell proliferation). In an embodiment, methods and compositions discussed herein can be used to affect T cell survival (e.g., by inactivating genes mediating T cell apoptosis). In an embodiment, methods and composition discussed herein can be used to affect T cell function (e.g., by inactivating genes encoding immunosuppressive and inhibitory (e.g., anergy-inducing) signaling factors). It is contemplated herein that the methods and compositions described above can be utilized individually or in combination to affect one or more of the factors limiting the efficacy of genetically modified T cells as cancer therapeutics, e.g., T cell proliferation, T cell survival, T cell function, or any combination thereof.

Methods and compositions discussed herein can be used to affect T cell proliferation, survival and/or function by altering one or more T-cell expressed genes, e.g., one or more of *FAS, BID, CTLA4, PDCD1, CBLB, PTPN6, TRAC* and/or *TRBC* genes. In an embodiment, methods and compositions described herein can be used to affect T cell proliferation by altering one or more T-cell expressed genes, e.g., the *CBLB* and/or *PTPN6* gene. In an embodiment, methods and compositions described herein can be used to affect T cell survival by altering one or more T-cell expressed genes, e.g., *FAS* and/or *BID* gene. In an embodiment, methods and compositions described herein can be used to affect T cell function by altering one or more T-cell expressed gene or genes, e.g., *CTLA4* and/or *PDCD1* and/or *TRAC* and/or *TRBC* gene.

In one approach, one or more T-cell expressed genes, e.g., *FAS, BID, CTLA4, PDCD1, CBLB, PTPN6, TRAC* and/or *TRBC* genes, are independently targeted as a targeted knockout or knockdown, e.g., to influence T cell proliferation, survival and/or function. In an embodiment, the approach comprises knocking out or knocking down one T-cell expressed gene (e.g., *FAS, BID, CTLA4, PDCD1, CBLB, PTPN6, TRAC* or *TRBC* gene). In another embodiment, the approach comprises independently knocking out or knocking down two T-cell expressed genes, e.g., two of *FAS, BID, CTLA4, PDCD1, CBLB, PTPN6, TRAC* or *TRBC* genes. In another embodiment, the approach comprises independently knocking out or knocking down three T-cell expressed genes, e.g., three of *FAS, BID, CTLA4, PDCD1, CBLB, PTPN6, TRAC* or *TRBC* genes. In another embodiment, the approach comprises independently knocking out or knocking down four T-cell expressed genes, e.g., four of *FAS, BID, CTLA4, PDCD1, CBLB, PTPN6, TRAC* or *TRBC* genes. In another embodiment, the approach comprises independently knocking out or knocking down five T-cell expressed genes, e.g., five of *FAS, BID, CTLA4, PDCD1, CBLB, PTPN6, TRAC* or *TRBC* genes. In another embodiment, the approach comprises independently knocking out or knocking down six T-cell expressed genes, e.g., six of *FAS, BID, CTLA4, PDCD1, CBLB, PTPN6, TRAC* or *TRBC* genes. In another embodiment, the approach comprises independently knocking out or knocking down seven T-cell expressed genes, e.g., seven of *FAS, BID, CTLA4, PDCD1, CBLB, PTPN6, TRAC* or *TRBC* genes. In another embodiment, the approach comprises independently knocking out or knocking down eight T-cell expressed genes, e.g., each of *FAS, BID, CTLA4, PDCD1, CBLB, PTPN6, TRAC* and *TRBC* genes.

In addition to the genes described above, a number of other T-cell expressed genes may be targeted to affect the efficacy of engineered T cells. These genes include, but are not limited to *TGFBRI, TGFBRII* and *TGFBRIII* (Kershaw et al. 2013 NatRevCancer 13, 525-541). It is contemplated herein that one or more of *TGFBRI, TGFBRII* or *TGFBRIII* gene can be altered either individually or in combination using the methods disclosed herein. It is further contemplated that one or more of *TGFBRI, TGFBRII* or *TGFBRIII* gene can be altered either individually or in combination with any one or more of the eight genes described above (i.e., *FAS, BID, CTLA4, PDCD1, CBLB, PTPN6, TRAC* or *TRBC* gene) using the methods disclosed herein.

In one aspect, methods and compositions discussed herein may be used to alter one or more of the *FAS, BID, CTLA4, PDCD1, CBLB, PTPN6, TRAC* and/or *TRBC* genes to affect T cell proliferation, survival and/or function by targeting the gene, e.g., the non-coding or coding regions, e.g., the promoter region, or a transcribed sequence, e.g., intronic or exonic sequence. In an embodiment, coding sequence, e.g., a coding region, e.g., an early coding region, of the *FAS, BID, CTLA4, PDCD1, CBLB, PTPN6, TRAC* and/or *TRBC* gene, is targeted for alteration and knockout of expression.

In another aspect, the methods and compositions discussed herein may be used to alter the *FAS, BID, CTLA4, PDCD1, CBLB, PTPN6, TRAC* and/or *TRBC* gene to affect T cell proliferation, survival and/or function by targeting the coding sequence of the *FAS, BID, CTLA4, PDCD1, CBLB, PTPN6, TRAC* and/or *TRBC* gene. In an embodiment, the gene, e.g., the coding sequence of the *FAS, BID, CTLA4, PDCD1, CBLB, PTPN6, TRAC* and/or *TRBC* gene, is targeted to knockout one or more of *FAS, BID, CTLA4, PDCD1, CBLB, PTPN6, TRAC* and/or *TRBC* gene, respectively, e.g., to eliminate expression of one or more of *FAS, BID, CTLA4, PDCD1, CBLB, PTPN6, TRAC* and/or *TRBC* gene, e.g., to knockout one or two alleles of one or more of *FAS, BID, CTLA4, PDCD1, CBLB, PTPN6, TRAC* and/or *TRBC* gene, e.g., by induction of an alteration comprising a deletion or mutation in one or more of *FAS, BID, CTLA4, PDCD1, CBLB, PTPN6, TRAC* and/or *TRBC* gene. In an embodiment, the method provides an alteration that comprises an insertion or deletion. As described herein, a targeted knockout approach is mediated by non-homologous end joining (NHEJ) using a CRISPR/Cas system comprising an enzymatically active Cas9 (eaCas9).

In an embodiment, an early coding sequence of the *FAS, BID, CTLA4, PDCD1, CBLB, PTPN6, TRAC* and/or *TRBC* gene is targeted to knockout one or more of *FAS, BID, CTLA4, PDCD1, CBLB, PTPN6, TRAC* and/or *TRBC* gene, respectively. In an embodiment, targeting affects one or two alleles of the *FAS, BID, CTLA4, PDCD1, CBLB, PTPN6, TRAC* and/or *TRBC* gene. In an embodiment, a targeted knockout approach reduces or eliminates expression of functional *FAS, BID, CTLA4, PDCD1, CBLB, PTPN6, TRAC* and/or *TRBC* gene product. In an embodiment, the method provides an alteration that comprises an insertion or deletion.

In another aspect, the methods and compositions discussed herein may be used to alter the *FAS, BID, CTLA4, PDCD1, CBLB, PTPN6, TRAC* and/or *TRBC* gene to affect T cell function by targeting non-coding sequence of the *FAS, BID, CTLA4, PDCD1, CBLB, PTPN6, TRAC* and/or *TRBC* gene, e.g., promoter, an enhancer, an intron, 3'UTR, and/or polyadenylation signal. In an embodiment, the gene, e.g., the non-coding sequence of the *FAS, BID, CTLA4, PDCD1, CBLB, PTPN6, TRAC* and/or *TRBC* gene, is targeted to knockout the gene, e.g., to eliminate expression of the gene, e.g., to knockout one or two alleles of the *FAS, BID, CTLA4, PDCD1, CBLB, PTPN6, TRAC* and/or *TRBC* gene, e.g., by induction of an alteration comprising a deletion or mutation in the *FAS, BID, CTLA4, PDCD1, CBLB, PTPN6, TRAC* and/or *TRBC* gene. In an embodiment, the method provides an alteration that comprises an insertion or deletion.

"T cell target FAS knockout position", as used herein, refers to a position in the *FAS* gene, which if altered by NHEJ-mediated alteration, results in a reduction or elimination of expression of functional *FAS* gene product (e.g., knockout of expression of functional *FAS* gene product). In an embodiment, the position is in the *FAS* gene coding region, e.g., an early coding region.

"T cell target BID knockout position", as used herein, refers to a position in the *BID* gene, which if altered by NHEJ-mediated alteration, results in a reduction or elimination of expression of functional *BID* gene product (e.g., knockout of expression of functional *BID* gene product). In an embodiment, the position is in the *BID* gene coding region, e.g., an early coding region.

"T cell target CTLA4 knockout position", as used herein, refers to a position in the *CTLA4* gene, which if altered by NHEJ-mediated alteration, results in a reduction or elimination of expression of functional *CTLA4* gene product (e.g., knockout of expression of functional *CTLA4* gene product). In an embodiment, the position is in the *CTLA4* gene coding region, e.g., an early coding region.

"T cell target PDCD1 knockout position", as used herein, refers to a position in the *PDCD1* gene, which if altered by NHEJ-mediated alteration, results in a reduction or elimination of expression of functional *PDCD1* gene product (e.g., knockout of expression of functional *PDCD1* gene product). In an embodiment, the position is in the *PDCD1* gene coding region, e.g., an early coding region.

"T cell target CBLB knockout position", as used herein, refers to a position in the *CBLB* gene, which if altered by NHEJ-mediated alteration, results in a reduction or elimination of expression of functional *CBLB* gene product (e.g., knockout of expression of functional *CBLB* gene product). In an embodiment, the position is in the *CBLB* gene coding region, e.g., an early coding region.

"T cell target PTPN6 knockout position", as used herein, refers to a position in the *PTPN6* gene, which if altered by NHEJ-mediated alteration, results in a reduction or elimination of expression of functional *PTPN6* gene product (e.g., knockout of expression of functional *PTPN6* gene product). In an embodiment, the position is in the *PTPN6* gene coding region, e.g., an early coding region.

"T cell target TRAC knockout position", as used herein, refers to a position in the *TRAC* gene, which if altered by NHEJ-mediated alteration, results in a reduction or elimination of expression of functional *TRAC* gene product (e.g., knockout of expression of functional *TRAC* gene product). In an embodiment, the position is in the *TRAC* gene coding region, e.g., an early coding region.

"T cell target TRBC knockout position", as used herein, refers to a position in the *TRBC* gene, which if altered by NHEJ-mediated alteration, results in a reduction or elimination of expression of functional *TRBC* gene product (e.g., knockout of expression of functional *TRBC* gene product). In an embodiment, the position is in the *TRBC* gene coding region, e.g., an early coding region.

In another aspect, methods and compositions discussed herein may be used to alter the expression of one or more T cell-expressed genes, e.g., the *FAS, BID, CTLA4, PDCD1, CBLB,* or *PTPN6* genes, to affect T cell function by targeting a promoter region of the *FAS, BID, CTLA4, PDCD1, CBLB,* or *PTPN6* gene. In an embodiment, the promoter region of the *FAS, BID, CTLA4, PDCD1, CBLB,* or *PTPN6* gene is targeted to knockdown expression of the *FAS, BID, CTLA4, PDCD1, CBLB,* or *PTPN6* gene. A targeted knockdown approach reduces or eliminates expression of functional the *FAS, BID, CTLA4, PDCD1, CBLB,* and/or *PTPN6* gene product. As described herein, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *FAS, BID, CTLA4, PDCD1, CBLB* and/or *PTPN6* genes.

"T cell target FAS knockdown position", as used herein, refers to a position, e.g., in the *FAS* gene, which if targeted by an eiCas9 or an eiCas9 fusion protein described herein, results in reduction or elimination of expression of functional *FAS* gene product. In an embodiment, transcription is reduced or eliminated. In an embodiment, the position is in the *FAS* promoter sequence. In an embodiment, a position in the promoter sequence of the *FAS* gene is targeted by an enzymatically inactive Cas9 (eiCas9) or an eiCas9-fusion protein, as described herein.

"T cell target BID knockdown position", as used herein, refers to a position, e.g., in the *BID* gene, which if targeted by an eiCas9 or an eiCas9 fusion protein described herein, results in reduction or elimination of expression of functional *BID* gene product. In an embodiment, transcription is reduced or eliminated. In an embodiment, the position is in the *BID* promoter sequence. In an embodiment, a position in the promoter sequence of the *BID* gene is targeted by an enzymatically inactive Cas9 (eiCas9) or an eiCas9-fusion protein, as described herein.

"T cell target CTLA4 knockdown position", as used herein, refers to a position, e.g., in the *CTLA4* gene, which if targeted by an eiCas9 or an eiCas9 fusion protein described herein, results in reduction or elimination of expression of functional *CTLA4* gene product. In an embodiment, transcription is reduced or eliminated. In an embodiment, the position is in the *CTLA4* promoter sequence. In an embodiment, a position in the promoter sequence of the *CTLA4* gene is targeted by an enzymatically inactive Cas9 (eiCas9) or an eiCas9-fusion protein, as described herein.

"T cell target PDCD1 knockdown position", as used herein, refers to a position, e.g., in the *PDCD1* gene, which if targeted by an eiCas9 or an eiCas9 fusion protein described herein, results in reduction or elimination of expression of functional *PDCD1* gene product. In an embodiment, transcription is reduced or eliminated. In an embodiment, the position is in the *PDCD1* promoter sequence. In an embodiment, a position in the promoter sequence of the *PDCD1* gene is targeted by an enzymatically inactive Cas9 (eiCas9) or an eiCas9-fusion protein, as described herein.

"T cell target CBLB knockdown position", as used herein, refers to a position, e.g., in the *CBLB* gene, which if targeted by an eiCas9 or an eiCas9 fusion protein described herein, results in reduction or elimination of expression of functional *CBLB* gene product. In an embodiment, transcription is reduced or eliminated. In an embodiment, the position is in the *CBLB* promoter sequence. In an embodiment, a position in the promoter sequence of the *CBLB* gene is targeted by an enzymatically inactive Cas9 (eiCas9) or an eiCas9-fusion protein, as described herein.

"T cell target PTPN6 knockdown position", as used herein, refers to a position, e.g., in the *PTPN6* gene, which if targeted by an eiCas9 or an eiCas9 fusion protein described herein, results in reduction or elimination of expression of functional *PTPN6* gene product. In an embodiment, transcription is reduced or eliminated. In an embodiment, the position is in the *PTPN6* promoter sequence. In an embodiment, a position in the promoter sequence of the *PTPN6* gene is targeted by an enzymatically inactive Cas9 (eiCas9) or an eiCas9-fusion protein, as described herein.

"T cell target FAS position", as used herein, refers to any of the T cell target FAS knockout position and/or T cell target FAS knockdown position, as described herein.

"T cell target BID position", as used herein, refers to any of the T cell target BID knockout position and/or T cell target BID knockdown position, as described herein.

"T cell target CTLA4 position", as used herein, refers to any of the T cell target CTLA4 knockout position and/or T cell target CTLA4 knockdown position, as described herein.

"T cell target PDCD1 position", as used herein, refers to any of the T cell target PDCD1 knockout position and/or T cell target PDCD1 knockdown position, as described herein.

"T cell target CBLB position", as used herein, refers to any of the T cell target CBLB knockout position and/or T cell target CBLB knockdown position, as described herein.

"T cell target PTPN6 position", as used herein, refers to any of the T cell target PTPN6 knockout position and/or T cell target PTPN6 knockdown position, as described herein.

"T cell target TRAC position", as used herein, refers to any of the T cell target TRAC knockout position, as described herein.

"T cell target TRBC position", as used herein, refers to any of the T cell target TRBC knockout position, as described herein.

"T cell target knockout position", as used herein, refers to any of the T cell target FAS knockout position, T cell target BID knockout position, T cell target CTLA4 knockout position, T cell target PDCD1 knockout position, T cell target CBLB knockout position, T cell target PTPN6 knockout position, T cell target TRAC knockout position, or T cell target TRBC knockout position, as described herein.

"T cell target knockdown position", as used herein, refers to any of the T cell target FAS knockdown position, T cell target BID knockdown position, T cell target CTLA4 knockdown position, T cell target PDCD1 knockdown position, T cell target CBLB knockdown position, or T cell target PTPN6 knockdown position, as described herein.

"T cell target position", as used herein, refers to any of a T cell target knockout position or T cell target knockdown position, as described herein.

In one aspect, disclosed herein is a gRNA molecule, e.g., an isolated or non-naturally occurring gRNA molecule, comprising a targeting domain which is complementary with a target domain from the *FAS, BID, CTLA4, PDCD1, CBLB, PTPN6, TRAC* or *TRBC* gene.

In an embodiment, the targeting domain of the gRNA molecule is configured to provide a cleavage event, e.g., a double strand break or a single strand break, sufficiently close to a T cell target position in the *FAS, BID, CTLA4, PDCD1, CBLB, PTPN6, TRAC* or *TRBC* gene to allow alteration, e.g., alteration associated with NHEJ, of a T cell target position in the *FAS, BID, CTLA4, PDCD1, CBLB, PTPN6, TRAC* or *TRBC* gene. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of a T cell target position. The break, e.g., a double strand or single strand break, can be positioned upstream or downstream of a T cell target position in the *FAS, BID, CTLA4, PDCD1, CBLB, PTPN6, TRAC* or *TRBC* gene.

In an embodiment, a second gRNA molecule comprising a second targeting domain is configured to provide a cleavage event, e.g., a double strand break or a single strand break, sufficiently close to the T cell target position in the *FAS, BID, CTLA4, PDCD1, CBLB, PTPN6, TRAC* or *TRBC* gene, to allow alteration, e.g., alteration associated with NHEJ, of the T cell target position in the *FAS, BID, CTLA4, PDCD1, CBLB, PTPN6, TRAC* or *TRBC* gene, either alone or in combination with the break positioned by the first gRNA molecule. In an embodiment, the targeting domains of the first and second gRNA molecules are configured such that a cleavage event, e.g., a double strand or single strand break, is positioned, independently for each of the gRNA molecules, within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the breaks, e.g., double strand or single strand breaks, are positioned on both sides of a nucleotide of a T cell target position in the *FAS, BID, CTLA4, PDCD1, CBLB, PTPN6, TRAC* or *TRBC* gene. In an embodiment, the breaks, e.g., double strand or single strand breaks, are positioned on one side, e.g., upstream or downstream, of a nucleotide of a T cell target position in the *FAS, BID, CTLA4, PDCD1, CBLB, PTPN6, TRAC* or *TRBC* gene.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of a T cell target position. In an embodiment, the first and second gRNA molecules are configured such that, when guiding a Cas9 nickase, a single strand break will be accompanied by an additional single strand break, positioned by a second gRNA, sufficiently close to one another to result in alteration of a T cell target position in the *FAS, BID, CTLA4, PDCD1, CBLB, PTPN6, TRAC* or *TRBC* gene. In an embodiment, the first and second gRNA molecules are configured such that a single strand break positioned by the second gRNA is within 10, 20, 30, 40, or 50 nucleotides of the break positioned by the first gRNA molecule, e.g., when the Cas9 is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domain of a second gRNA molecule is configured such that a double strand break is positioned downstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domains of a second and third gRNA molecule are configured such that two single strand breaks are positioned downstream of a T cell target position in the *FAS, BID, CTLA4, PDCD1, CBLB, PTPN6, TRAC or TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the targeting domain of the first, second and third gRNA molecules are configured such that a cleavage event, e.g., a double strand or single strand break, is positioned, independently for each of the gRNA molecules.

In an embodiment, a first and second single strand break can be accompanied by two additional single strand breaks positioned by a third gRNA molecule and a fourth gRNA molecule. For example, the targeting domain of a first and second gRNA molecule are configured such that two single strand breaks are positioned upstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domains of a third and fourth gRNA molecule are configured such that two single strand breaks are positioned downstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position.

It is contemplated herein that when multiple gRNAs are used to generate (1) two single stranded breaks in close proximity, (2) two double stranded breaks, e.g., flanking a position (e.g., to remove a piece of DNA, e.g., to create a deletion mutation) or to create more than one indel in the gene, e.g., in a coding region, e.g., an early coding region, (3) one double stranded break and two paired nicks flanking a position (e.g., to remove a piece of DNA, e.g., to insert a deletion) or (4) four single stranded breaks, two on each side of a position, that they are targeting the same T cell target position. It is further contemplated herein that multiple gRNAs may be used to target more than one position in the same gene.

When two or more gRNAs are used to position two or more cleavage events, e.g., double strand or single strand breaks, in a target nucleic acid, it is contemplated that the two or more cleavage events may be made by the same or different Cas9 proteins. For example, when two gRNAs are used to position two double stranded breaks in a target nucleic acid, a single Cas9 nuclease may be used to create both double stranded breaks. When two or more gRNAs are used to position two or more single stranded breaks (also referred to as nicks) in a target nucleic acid, a single Cas9 nickase may be used to create the two or more nicks. When two or more gRNAs are used to position at least one double stranded break and at least one single stranded break in a target nucleic acid, two Cas9 proteins may be used, e.g., one Cas9 nuclease and one Cas9 nickase. It is contemplated that when two or more Cas9 proteins are used that the two or more Cas9 proteins may be delivered sequentially to control specificity of a double stranded versus a single stranded break at the desired position in the target nucleic acid. In another embodiment, when two or more Cas9 proteins are used, the Cas9 proteins may be from different species. For example, when two or more gRNAs are used to position at least one double stranded break and at least one single stranded break in a target nucleic acid, the Cas9 nuclease generating the double stranded break may be from one bacterial species and the Cas9 nickase generating the single stranded break may be from a different bacterial species.

When more than one gene is targeted for alteration in a cell, the targeted nucleic acids may be altered, e.g., cleaved, by one or more Cas9 proteins. For example, if two genes are targeted for alteration, e.g., both genes are targeted for knockdown, the same or a different Cas9 protein may be used to target each gene. In one embodiment, both genes (or each gene targeted in a cell), are cleaved by a Cas9 nuclease to generate a double stranded break. In another embodiment, both genes (or each gene targeted in a cell), are cleaved by a Cas9 nuclease to generate a double stranded break. In another embodiment, one or more genes in a cell may be altered by cleavage with a Cas9 nuclease and one or more genes in the same cell may be altered by cleavage with a Cas9 nickase. When two or more Cas9 proteins are used to cut a target nucleic acid, e.g., different genes in a cell, the Cas9 proteins may be from different bacterial species. For example, one or more genes in a cell may be altered by cleavage with a Cas9 protein from one bacterial species, and one or more genes in the same cell may be altered by cleavage with a Cas9 protein from a different bacterial species. It is contemplated that when two or more Cas9 proteins from different species are used that they may be delivered at the same time or delivered sequentially to control specificity of cleavage in the desired gene at the desired position in the target nucleic acid.

In some embodiments, the targeting domain of the first gRNA molecule and the targeting domain of the second gRNA molecules are complementary to opposite strands of the target nucleic acid molecule. In some embodiments, the gRNA molecule and the second gRNA molecule are configured such that the PAMs are oriented outward.

In an embodiment, the targeting domain of a gRNA molecule is configured to avoid unwanted target chromosome elements, such as repeat elements, e.g., Alu repeats, in the target domain. The gRNA molecule may be a first, second, third and/or fourth gRNA molecule.

In an embodiment, the targeting domain of a gRNA molecule is configured to position a cleavage event sufficiently far from a preselected nucleotide, e.g., the nucleotide of a coding region, such that the nucleotide is not be altered. In an embodiment, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

In other embodiments, a position in the coding region, e.g., the early coding region, of the *FAS, BID, CTLA4, PDCD1, CBLB, PTPN6, TRAC* or *TRBC* gene is targeted, e.g., for knockout.

In an embodiment, the targeting domain of the gRNA molecule is configured to target an enzymatically inactive Cas9 (eiCas9) or an eiCas9 fusion protein (e.g., an eiCas9 fused to a transcription repressor domain), sufficiently close to a T cell knockdown target position to reduce, decrease or repress expression of the *FAS, BID, CTLA4, PDCD1, CBLB,* or *PTPN6* gene. In an embodiment, the targeting domain is configured to target the promoter region of the *FAS, BID, CTLA4, PDCD1, CBLB,* or *PTPN6* gene to block transcription initation, binding of one or more transcription enhancers or activators, and/or RNA polymerase. One or more gRNA may be used to target an eiCas9 to the promoter region of the *FAS, BID, CTLA4, PDCD1, CBLB,* or *PTPN6* gene.

In an embodiment, the gRNA, e.g., a gRNA comprising a targeting domain, which is complementary with the *FAS, BID, CTLA4, PDCD1, CBLB, PTPN6, TRAC* or *TRBC* gene, is a modular gRNA. In other embodiments, the gRNA is a unimolecular or chimeric gRNA.

In an embodiment, the targeting domain which is complementary with the *FAS, BID, CTLA4, PDCD1, CBLB, PTPN6, TRAC* or *TRBC* gene comprises 16 or more nucleotides in length. In an embodiment, the targeting domain which is complementary with a target domain from the *FAS, BID, CTLA4, PDCD1, CBLB, PTPN6, TRAC* or *TRBC* gene is 16 nucleotides or more in length. In an embodiment, the targeting domain is 16 nucleotides in length. In an embodiment, the targeting domain is 17 nucleotides in length. In an embodiment, the targeting domain is 18 nucleotides in length. In an embodiment, the targeting domain is 19 nucleotides in length. In an embodiment, the targeting domain is 20 nucleotides in length. In an embodiment, the targeting domain is 21 nucleotides in length. In an embodiment, the targeting domain is 22 nucleotides in length. In an embodiment, the targeting domain is 23 nucleotides in length. In an embodiment, the targeting domain is 24 nucleotides in length. In an embodiment, the targeting domain is 25 nucleotides in length. In an embodiment, the targeting domain is 26 nucleotides in length.

In an embodiment, the targeting domain comprises 16 nucleotides. In an embodiment, the targeting domain comprises 17 nucleotides. In an embodiment, the targeting domain comprises 18 nucleotides. In an embodiment, the targeting domain comprises 19 nucleotides. In an embodiment, the targeting domain comprises 20 nucleotides. In an embodiment, the targeting domain comprises 21 nucleotides. In an embodiment, the targeting domain comprises 22 nucleotides. In an embodiment, the targeting domain comprises 23 nucleotides. In an embodiment, the targeting domain comprises 24 nucleotides. In an embodiment, the targeting domain comprises 25 nucleotides. In an embodiment, the targeting domain comprises 26 nucleotides.

A gRNA as described herein may comprise from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a gRNA comprises a linking domain of no more than 25 nucleotides in length; a proximal and tail domain, that taken together, are at least 20 nucleotides in length; and a targeting domain of equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain of equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain of equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 40 nucleotides in length; and a targeting domain of equal to or greater than 16, 17, 18, 19, 20, 21, 22, 23, 24, 25 or 26 nucleotides in length.

A cleavage event, e.g., a double strand or single strand break, is generated by a Cas9 molecule. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule). Alternatively, in some embodiments, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

In an embodiment, the eaCas9 molecule catalyzes a double strand break.

In some embodiments, the eaCas9 molecule comprises HNH-like domain cleavage activity but has no, or no significant, N-terminal RuvC-like domain cleavage activity. In this case, the eaCas9 molecule is an HNH-like domain nickase, e.g., the eaCas9 molecule comprises a mutation at D10, e.g., D10A. In other embodiments, the eaCas9 molecule comprises N-terminal RuvC-like domain cleavage activity but has no, or no significant, HNH-like domain cleavage activity. In an embodiment, the eaCas9 molecule is an N-terminal RuvC-like domain nickase, e.g., the eaCas9 molecule comprises a mutation at H840, e.g., H840A. In an embodiment, the eaCas9 molecule is an N-terminal RuvC-like domain nickase, e.g., the eaCas9 molecule comprises a mutation at N863, e.g., N863A.

In an embodiment, a single strand break is formed in the strand of the target nucleic acid to which the targeting domain of the gRNA is complementary. In another embodiment, a single strand break is formed in the strand of the target nucleic acid other than the strand to which the targeting domain of the gRNA is complementary.

In another aspect, disclosed herein is a composition comprising (a) a gRNA molecule comprising a targeting domain that is complementary with a target domain in the *FAS, BID, CTLA4, PDCD1, CBLB, PTPN6, TRAC* or *TRBC* gene, as described herein. The composition of (a) may further comprise (b) a Cas9 molecule, e.g., a Cas9 molecule as described herein. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule). Alternatively, in some embodiments, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

A composition of (a) and (b) may further comprise (c) a second, third and/or fourth gRNA molecule, e.g., a second, third and/or fourth gRNA molecule described herein. In an embodiment, a composition may comprise at least two gRNA molecules to target two or more of *FAS, BID, CTLA4, PDCD1, CBLB, PTPN6, TRAC* and *TRBC* genes. In an embodiment, the composition further comprises a governing gRNA molecule, or a nucleic acid that encodes a governing gRNA molecule.

In another aspect, disclosed herein is a method of altering a cell, e.g., altering the structure, e.g., altering the sequence, of a target nucleic acid of a cell, comprising contacting the cell with: (a) a gRNA that targets the *FAS, BID, CTLA4, PDCD1, CBLB, PTPN6, TRAC* or *TRBC* gene, e.g., a gRNA as described herein; (b) a Cas9 molecule, e.g., a Cas9 molecule as described herein; and optionally, (c) a second, third and/or fourth gRNA that targets *FAS, BID, CTLA4, PDCD1, CBLB, PTPN6, TRAC* or *TRBC* gene, e.g., a gRNA, as described herein. In an embodiment, the method further comprises, introducing into the cell, a governing gRNA molecule, or a nucleic acid that encodes a governing gRNA molecule, into the cell.

In some embodiments, the method comprises contacting the cell with (a) and (b).

In some embodiments, the method comprises contacting the cell with (a), (b), and (c).

In an embodiment, the method of altering a cell, e.g., altering the structure, e.g., altering the sequence, of a target nucleic acid of a cell, comprising altering two or more of *FAS, BID, CTLA4, PDCD1, CBLB, PTPN6, TRAC* or *TRBC* genes. When two or more genes are altered in a cell, the cell is contacted with: (a) gRNAs that target two or more of the *FAS, BID, CTLA4, PDCD1, CBLB, PTPN6, TRAC* or *TRBC* genes; (b) a Cas9 molecule, e.g., a Cas9 molecule as described herein; and optionally, (c) second, third and/or fourth gRNAs that respectively targets the two or more genes selected in (a). In an embodiment, the method further comprises, introducing into the cell, a governing gRNA molecule, or a nucleic acid that encodes a governing gRNA molecule, into the cell.

In an embodiment, the method of altering a cell comprises altering two or more of *FAS, BID, CTLA4, PDCD1, CBLB, PTPN6, TRAC* or *TRBC* genes.

In an embodiment, the method of altering a cell comprises altering three or more of *FAS, BID, CTLA4, PDCD1, CBLB, PTPN6, TRAC* or *TRBC* genes.

In an embodiment, the method of altering a cell comprises altering four or more of *FAS, BID, CTLA4, PDCD1, CBLB, PTPN6, TRAC* or *TRBC* genes.

In an embodiment, the method of altering a cell comprises altering five or more of *FAS, BID, CTLA4, PDCD1, CBLB, PTPN6, TRAC* or *TRBC* genes.

In an embodiment, the method of altering a cell comprises altering six or more of *FAS, BID, CTLA4, PDCD1, CBLB, PTPN6, TRAC* or *TRBC* genes.

In an embodiment, the method of altering a cell comprises altering seven or more of *FAS, BID, CTLA4, PDCD1, CBLB, PTPN6, TRAC* or *TRBC* genes.

In an embodiment, the method of altering a cell comprises altering each of *FAS, BID, CTLA4, PDCD1, CBLB, PTPN6, TRAC* or *TRBC* genes.

In some embodiments, the method comprises contacting a cell from a subject suffering from cancer. The cell may be from a subject that would benefit from having a mutation at a T cell target position.

In some embodiments, the cell being contacted in the disclosed method is a T cell. The contacting may be performed *ex vivo* and the contacted cell may be returned to the subject's body after the contacting step. The T cell may be an engineered T cell, e.g., an engineered CAR (chimeric antigen receptor) T cell or an engineered TCR (T-cell receptor) T cell. A T cell may engineered to express a TCR or a CAR prior to, after, or at the same time as introducing a T cell target position mutation in one or more of the *FAS, BID, CTLA4, PDCD1, CBLB, PTPN6, TRAC* or *TRBC* gene.

In some embodiments, the method of altering a cell as described herein comprises acquiring knowledge of the sequence of a T cell target position in the cell, prior to the contacting step. Acquiring knowledge of the sequence of a T cell target position in the cell may be by sequencing the *FAS, BID, CTLA4, PDCD1, CBLB, PTPN6, TRAC* or *TRBC* gene, or a portion of the *FAS, BID, CTLA4, PDCD1, CBLB, PTPN6, TRAC* or *TRBC* gene.

In an embodiment, contacting comprises delivering to the cell a Cas9 molecule of (b), as a protein or an mRNA, the gRNA of (a), as an RNA, and optionally the second gRNA of (c), as an RNA.

In an embodiment, contacting comprises delivering to the cell a gRNA of (a) as an RNA, optionally the second gRNA of (c) as an RNA, and a nucleic acid that encodes the Cas9 molecule of (b).

In another aspect, disclosed herein is a method of treating a subject suffering from cancer, e.g., altering the structure, e.g., sequence, of a target nucleic acid of the subject, comprising contacting a cell from the subject with:
(a) a gRNA that targets the *FAS, BID, CTLA4, PDCD1, CBLB, PTPN6, TRAC* or *TRBC* gene, e.g., a gRNA disclosed herein;
(b) a Cas9 molecule, e.g., a Cas9 molecule disclosed herein; and
optionally, (c)(i) a second gRNA that targets the *FAS, BID, CTLA4, PDCD1, CBLB, PTPN6, TRAC* or *TRBC* gene, e.g., a second gRNA disclosed herein, and
further optionally, (c)(ii) a third gRNA, and still further optionally, (c)(iii) a fourth gRNA that target the *FAS, BID, CTLA4, PDCD1, CBLB, PTPN6, TRAC* or *TRBC* gene, e.g., a third and fourth gRNA disclosed herein. In an embodiment, the method further comprises, introducing into a cell of the subject, a governing gRNA molecule, or a nucleic acid that encodes a governing gRNA molecule.

In some embodiments, contacting comprises contacting with (a) and (b).

In some embodiments, contacting comprises contacting with (a), (b), and (c)(i).

In some embodiments, contacting comprises contacting with (a), (b), (c)(i) and (c)(ii).

In some embodiments, contacting comprises contacting with (a), (b), (c)(i), (c)(ii) and (c)(iii).

In an embodiment, the method of treating a subject suffering from cancer, comprises altering two or more of *FAS, BID, CTLA4, PDCD1, CBLB, PTPN6, TRAC* or *TRBC* genes, e.g., altering the structure, e.g., sequence, of two or more target nucleic acids of the subject (e.g., two or more of *FAS, BID, CTLA4, PDCD1, CBLB, PTPN6, TRAC* or *TRBC* genes). When two or more genes are altered in a cell from the subject, a cell from the subject is contacted with: (a) gRNAs that target two or more of the *FAS, BID, CTLA4, PDCD1, CBLB, PTPN6, TRAC* or *TRBC* genes, e.g., two or more of the gRNA as described herein; (b) a Cas9 molecule, e.g., a Cas9 molecule as described herein; and optionally, (c) second, third and/or fourth gRNAs that respectively targets the two or more genes selected in (a), e.g., a gRNA, as described herein. In an embodiment, the method further comprises, introducing into a cell of the subject, a governing gRNA molecule, or a nucleic acid that encodes a governing gRNA molecule.

In an embodiment, the method of treating a subject suffering from cancer comprises altering two or more of *FAS, BID, CTLA4, PDCD1, CBLB, PTPN6, TRAC* or *TRBC* genes.

In an embodiment, the method of treating a subject suffering from cancer comprises altering three or more of *FAS, BID, CTLA4, PDCD1, CBLB, PTPN6, TRAC* or *TRBC* genes.

In an embodiment, the method of treating a subject suffering from cancer comprises altering four or more of *FAS, BID, CTLA4, PDCD1, CBLB, PTPN6, TRAC* or *TRBC* genes.

In an embodiment, the method of treating a subject suffering from cancer comprises altering five or more of *FAS, BID, CTLA4, PDCD1, CBLB, PTPN6, TRAC* or *TRBC* genes.

In an embodiment, the method of treating a subject suffering from cancer comprises altering six or more of *FAS, BID, CTLA4, PDCD1, CBLB, PTPN6, TRAC* or *TRBC* genes.

In an embodiment, the method of treating a subject suffering from cancer comprises altering seven or more of *FAS, BID, CTLA4, PDCD1, CBLB, PTPN6, TRAC* or *TRBC* genes.

In an embodiment, the method of treating a subject suffering from cancer comprises altering each of *FAS, BID, CTLA4, PDCD1, CBLB, PTPN6, TRAC* or *TRBC* genes.

In an embodiment, the method comprises acquiring knowledge of the sequence at a T cell target position in the subject.

In an embodiment, the method comprises acquiring knowledge of the sequence at a T cell target position in the subject by sequencing one or more of the *FAS, BID, CTLA4, PDCD1, CBLB, PTPN6, TRAC* or *TRBC* gene or a portion of one or more of the *FAS, BID, CTLA4, PDCD1, CBLB, PTPN6, TRAC* or *TRBC* gene.

In an embodiment, the method comprises inducing a mutation at a T cell target position in the *FAS, BID, CTLA4, PDCD1, CBLB, PTPN6, TRAC* or *TRBC* gene.

In an embodiment, the method comprises inducing a mutation at a T cell target position in one or more of the *FAS, BID, CTLA4, PDCD1, CBLB, PTPN6, TRAC* and *TRBC* genes.

In an embodiment, the method comprises inducing a mutation at a T cell target position in two or more of the *FAS, BID, CTLA4, PDCD1, CBLB, PTPN6, TRAC* and *TRBC* genes.

In an embodiment, the method comprises inducing a mutation at a T cell target position in three or more of the *FAS, BID, CTLA4, PDCD1, CBLB, PTPN6, TRAC* and *TRBC* genes.

In an embodiment, the method comprises inducing a mutation at a T cell target position in four or more of the *FAS, BID, CTLA4, PDCD1, CBLB, PTPN6, TRAC* and *TRBC* genes.

In an embodiment, the method comprises inducing a mutation at a T cell target position in five or more of the *FAS, BID, CTLA4, PDCD1, CBLB, PTPN6, TRAC* and *TRBC* genes.

In an embodiment, the method comprises inducing a mutation at a T cell target position in six or more of the *FAS, BID, CTLA4, PDCD1, CBLB, PTPN6, TRAC* and *TRBC* genes.

In an embodiment, the method comprises inducing a mutation at a T cell target position in seven or more of the *FAS, BID, CTLA4, PDCD1, CBLB, PTPN6, TRAC* and *TRBC* genes.

In an embodiment, the method comprises inducing a mutation at a T cell target position in each of the *FAS, BID, CTLA4, PDCD1, CBLB, PTPN6, TRAC* and *TRBC* genes.

In an embodiment, the method comprises inducing a mutation at a T cell target position in one or more of the *FAS, BID, CTLA4, PDCD1, CBLB, PTPN6, TRAC* or *TRBC* gene by NHEJ.

In an embodiment, a cell of the subject is contacted *ex vivo* with (a), (b), and optionally (c). In an embodiment, the cell is returned to the subject's body. In an embodiment, the cell of the subject being contacted *ex vivo* is a T cell. The T cell may be an engineered T cell, e.g., an engineered CAR (chimeric antigen receptor) T cell or an engineered TCR (T-cell receptor) T cell. A T cell may engineered to express a TCR or a CAR prior to, after, or at the same time as introducing a T cell target position mutation in one or more of the *FAS, BID, CTLA4, PDCD1, CBLB, PTPN6, TRAC* or *TRBC* gene.

When the method comprises (1) inducing a mutation at a T cell target position by NHEJ or (2) knocking down expression of the *FAS, BID, CTLA4, PDCD1, CBLB,* or *PTPN6* gene, e.g., by targeting the promoter region, a Cas9 of (b) and at least one guide RNA, e.g., a guide RNA of (a) are included in the contacting step.

In another aspect, disclosed herein is a reaction mixture comprising a, gRNA, a nucleic acid, or a composition described herein, and a cell, e.g., a cell from a subject having cancer, or a subject which would benefit from a mutation at a T cell target position.

In another aspect, disclosed herein is a kit comprising, (a) gRNA molecule described herein, and one or more of the following:
(b) a Cas9 molecule, e.g., a Cas9 molecule described herein, or a nucleic acid or mRNA that encodes the Cas9;
(c)(i) a second gRNA molecule, e.g., a second gRNA molecule described herein;
(c)(ii) a third gRNA molecule, e.g., a second gRNA molecule described herein;
(c)(iii) a fourth gRNA molecule, e.g., a second gRNA molecule described herein.

The compositions, reaction mixtures and kits, as disclosed herein, can also include a governing gRNA molecule, e.g., a governing gRNA molecule disclosed herein,

### VI.2 Circulating blood cells (e.g., targeting CCR5 gene)

In another aspect, the target cell is a circulating blood cell, e.g., a T cell (e.g., a CD4+ T cell, a CD8+ T cell, a helper T cell, a regulatory T cell, a cytotoxic T cell, a memory T cell, a T cell precursor or a natural killer T cell), a B cell (e.g., a progenitor B cell, a Pre B cell, a Pro B cell, a memory B cell, a plasma B cell), a monocyte, a megakaryocyte, a neutrophil, an eosinophil, a basophil, a mast cell, a reticulocyte, a lymphoid progenitor cell, a myeloid progenitor cell, a gut-associated lymphoid tissue (GALT) cell, a dendritic cell, a macrophage, a microglial cell, or a hematopoietic stem cell. In an embodiment, the target cell is a bone marrow cell, (e.g., a lymphoid progenitor cell, a myeloid progenitor cell, an erythroid progenitor cell, a hematopoietic stem cell, or a mesenchymal stem cell). In an embodiment, the target cell is a CD4+ T cell. In an embodiment, the target cell is a lymphoid progenitor cell (e.g., a common lymphoid progenitor (CLP) cell). In an embodiment, the target cell is a myeloid progenitor cell (e.g. a common myeloid progenitor (CMP) cell). In an embodiment, the target cell is a hematopoietic stem cell (e.g. a long term hematopoietic stem cell (LT-HSC), a short term hematopoietic stem cell (ST-HSC), a multipotent progenitor (MPP) cell, a lineage restricted progenitor (LRP) cell).

In an embodiment, the target cell is manipulated *ex vivo* by editing (e.g., introducing a mutation in) the *CCR5* target gene and/or modulating the expression of the *CCR5* target gene, and administered to the subject. Sources of target cells for *ex vivo* manipulation may include, by way of example, the subject's blood, the subject's cord blood, or the subject's bone marrow. Sources of target cells for *ex vivo* manipulation may also include, by way of example, heterologous donor blood, cord blood, or bone marrow.

In an embodiment, a CD4+T cell is removed from the subject, manipulated *ex vivo* as described above, and the CD4+T cell is returned to the subject. In an embodiment, a lymphoid progenitor cell is removed from the subject, manipulated *ex vivo* as described herein, and the lymphoid progenitor cell is returned to the subject. In an embodiment, a myeloid progenitor cell is removed from the subject, manipulated *ex vivo* as described herein, and the myeloid progenitor cell is returned to the subject. In an embodiment, a hematopoietic stem cell is removed from the subject, manipulated *ex vivo* as described herein, and the hematopoietic stem cell is returned to the subj ect.

A suitable cell can also include a stem cell such as, by way of example, an embryonic stem cell, an induced pluripotent stem cell, a hematopoietic stem cell, a neuronal stem cell and a mesenchymal stem cell. In an embodiment, the cell is an induced pluripotent stem cells (iPS) cell or a cell derived from an iPS cell, e.g., an iPS cell generated from the subject, modified to correct the mutation and differentiated into a clinically relevant cell such as e.g, a CD4+ T cell, a lymphoid progenitor cell, myeloid progenitor cell, a macrophage, dendritic cell, gut associated lymphoid tissue or a hematopoietic stem cell. In an embodiment, AAV is used to transduce the target cells, e.g., the target cells described herein.

### Human Immunodeficiency Virus

Human Immunodeficiency Virus (HIV) is a virus that causes severe immunodeficiency. In the United States, more than 1 million people are infected with the virus. Worldwide, approximately 30-40 million people are infected.

HIV is a single-stranded RNA virus that preferentially infects CD4 cells. The virus binds to receptors on the surface of CD4+ cells to enter and infect these cells. This binding and infection step is vital to the pathogenesis of HIV. The virus attaches to the CD4 receptor on the cell surface via its own surface glycoproteins, gp120 and gp41. These proteins are made from the cleavage product of gp160. Gp120 binds to a CD4 receptor and must also bind to another coreceptor in order for the virus to enter the host cell. In macrophage-(M-tropic) viruses, the coreceptor is *CCR5* occassionaly referred to as the CCR5 receptor. In thymic-(T-tropic) viruses, the coreceptor is *CXCR4.* M-tropic virus is found most commonly in the early stages of HIV infection.

There are two types of HIV-HIV-1 and HIV-2. HIV-1 is the predominant global form and is a more virulent strain of the virus. HIV-2 has lower rates of infection and, at present, predominantly affects populations in West Africa. HIV is transmitted primarily through sexual exposure, although the sharing of needles in intravenous drug use is another mode of transmission.

As HIV progresses, the virus infects CD4 cells and a subject's CD4 counts fall. With declining CD4 counts, a subject is subject to increasing risk of opportunistic infections (OI). Severely declining CD4 counts are associated with a very high likelihood of OIs, specific cancers (such as Kaposi's sarcoma, Burkitt's lymphoma) and wasting syndrome. Normal CD4 counts are between 600-1200 cells/microliter.

Untreated HIV is a chronic, progressive disease that leads to acquired immunodeficiency syndrome (AIDS) and death in the vast majority of subjects. Diagnosis of AIDS is made based on infection with a variety of opportunistic pathogens, presence of certain cancers and/or CD4 counts below 200 cells/µL.

HIV was untreatable and invariably led to death until the late 1980's. Since then, antiretroviral therapy (ART) has dramatically slowed the course of HIV infection. Highly active antiretroviral therapy (HAART) is the use of three or more agents in combination to slow HIV. Antiretroviral therapy (ART) is indicated in a subject whose CD4 counts has dropped below 500 cells/µL. Viral load is the most common measurement of the efficacy of HIV treatment and disease progression. Viral load measures the amount of HIV RNA present in the blood.

Treatment with HAART has significantly altered the life expectancy of those infected with HIV. A subject in the developed world who maintains their HAART regimen can expect to live into their 60's and possibly 70's. However, HAART regimens are associated with significant, long term side effects. First, the dosing regimens are complex and associated with strict food requirements. Compliance rates with dosing can be lower than 50% in some populations in the United States. In addition, there are significant toxicities associated with HAART treatment, including diabetes, nausea, malaise, sleep disturbances. A subject who does not adhere to dosing requirements of HAART therapy may have return of viral load in their blood and are at risk for progression to disease and its associated complications.

Methods and compositions described herein provide for a therapy, e.g., a one-time therapy, or a multi-dose therapy, that prevents or treats HIV infection and/or AIDS. In an embodiment, a disclosed therapy prevents the entry of HIV into CD4 cells of a subject who is already infected. While not wishing to be bound by theory, it is believed that knocking out *CCR5* on CD4 cells, renders the HIV virus unable to enter CD4 cells. Viral entry into CD4 cells requires interaction of the viral glycoproteins gp41 and gp120 with both the CD4 receptor and the CCR5 coreceptor. Once a functional *CCR5* viral receptor has been eliminated from the surface of the CD4 cells, the virus is prevented from binding and entering the host CD4 cells. In an embodiment, the disease does not progress.

While not wishing to be bound by theory, subjects with naturally occurring CCR5 receptor mutations who have delayed HIV progression may confer protection by the mechanism of action described herein. Subjects with a specific deletion in the *CCR5* gene (e.g., the delta 32 deletion) have been shown to have much higher likelihood of being long-term non-progressors (meaning they did not require HAART and their HIV infection did not progress). (See, e.g., Stewart GJ et al., 1997 The Australian Long-Term Non-Progressor Study Group. Aids.11:1833-1838.) In addition, a subject who was CCR5+ (had a wild type CCR5 receptor) and infected with HIV underwent a bone marrow transplant for acute myeloid lymphoma. (See,, e.g., Hutter G et al., 2009N ENGL J MED.360:692-698.) The bone marrow transplant (BMT) was from a subject homozygous for a *CCR5* delta 32 deletion. Following BMT, the subject did not have progression of HIV and did not require treatment with ART. These subjects offer evidence for the fact that induction of a protective mutation of the *CCR5* gene or a knockout of the *CCR5* gene prevents, delays or diminishes the ability of HIV to infect the subject. Mutation or deletion of the *CCR5* gene should therefore reduce the progression, virulence and pathology of HIV. In an embodiment, a method described herein is used to treat a subject having HIV.

In an embodiment, a method described herein is used to treat a subject having AIDS.

In an embodiment, a method described herein is used to prevent HIV infection and AIDS in a subject at high risk for HIV infection.

In an embodiment, a method described herein is results in a selective advantage to survival of treated CD4 cells. Some proportion of CD4 cells will be modified and have a *CCR5* protective mutation. These cells are not be subject to infection with HIV. Cells that were not modified may be infected with HIV and may be expected to undergo cell death. In an embodiment, after the treatment described herein, treated cells survive, while untreated cells should die. This selective advantage should drive eventual colonization in all body compartments with 100% CCR5-negative CD4 cells derived from treated cells, conferring complete protection in treated subjects against infection with M tropic HIV.

In an embodiment, the method comprises initiating treatment of a subject prior to disease onset.

In an embodiment, the method comprises initiating treatment of a subject after disease onset.

In an embodiment, the method comprises initiating treatment of a subject after disease onset, e.g., 1, 2, 3, 4, 5, 6, 7, 8, 9, 10, 12, 24, or 36 months after onset of HIV infection or AIDs. While not wishing to be bound by theory, it is believed that this may be effective as disease progression is slow in some cases and a subject may present well into the course of illness.

In an embodiment, the method comprises initiating treatment of a suject in an advanced stage of disease, e.g., to slow viral replication and viral load.

Overall, initiation of treatment for a subject at all stages of disease is expected to prevent or reduce disease progression and benefit a subject.

In an embodiment, the method comprises initiating treatment of a subject prior to disease onset and prior to infection with HIV.

In an embodiment, the method comprises initiating treatment of a subject in an early stage of disease, e.g., when when a subject has tested positive for HIV infection but has no signs or symptoms associated with HIV.

In an embodiment, the method comprises initiating treatment of a patient at the appearance of a reduced CD4 count or a positive HIV test.

In an embodiment, the method comprises treating a subject considered at risk for developing HIV infection.

In an embodiment, the method comprises treating a subject who is the spouse, partner, sexual partner, newborn, infant, or child of subject with HIV.

In an embodiment, the method comprises treating a subject for the prevention of HIV infection.

In an embodiment, the method comprises treating a subject at the appearance of any of the following findings consistent with HIV: low CD4 count; opportunistic infections associated with HIV, including but not limited to: candidiasis, mycobacterium tuberculosis, cryptococcosis, cryptosporidiosis, cytomegalovirus; and/or malignancy associated with HIV, including but not limited to: lymphoma, Burkitt's lymphoma, Kaposi's sarcoma.

In an embodiment, a target cell is treated *ex vivo* and returned to a patient.

In an embodiment, an autologous CD4 cell can be treated *ex vivo* and returned to the subj ect.

In an embodiment, a heterologous CD4 cell can be treated *ex vivo* and transplanted into the subj ect.

In an embodiment, an autologous stem cell can be treated *ex vivo* and returned to the subj ect.

In an embodiment, a heterologous stem cell can be treated *ex vivo* and transplanted into the subj ect.

### Other embodiments involving circulating blood cells and CCR5 gene

In an embodiment, methods and compositions discussed herein, allow for the prevention and treatment of HIV infection and AIDS, by inducing mutations in the gene for *CCR5.* In an embodiment, methods and compositions discussed can prevent HIV infection and/or prevent the ability for HIV to enter CD4 cells of subjects who are already infected. While not wishing to be bound by theory, by knocking out *CCR5* in CD4 cells or inducing a protective mutation (such as a *CCR5* delta 32 mutation), entry of the HIV virus into CD4 cells is prevented. Viral entry into CD4 cells requires interaction of the viral glycoproteins gp41 and gp120 with both the CD4 receptor and*CCR5,* which acts as a co-receptor. If *CCR5* is not present on the surface of the CD4 cells, the virus cannot bind and enter the host CD4 cells. The progress of the disease is thus impeded.

In one aspect, the methods and compositions discussed herein, block a critical aspect of the HIV life cycle, i.e., *CCR5*-mediated entry into T cells, by NHEJ-mediated inactivation of the *CCR5* gene. The mutations, which typically comprise a deletion or insertion (indel) mediated by NHEJ, can take place in any region of the gene, e.g., a promoter region or other non-coding region, or a coding region, so long as the mutation results in loss of the ability to mediate HIV entry into the cell.

In another aspect, the methods and compositions discussed herein may be used to alter the *CCR5* gene to treat or prevent HIV infection or AIDS by targeting the coding sequence of the *CCR5* gene. In one embodiment, the gene, e.g., the coding sequence of the *CCR5* gene, is targeted to knockout the gene, e.g., to eliminate expression of the gene, e.g., to knockout both alleles of the CCR5 gene, e.g., by induction of an alteration comprising a deletion or mutation in the *CCR5* gene. As described herein, a targeted knockout approach is mediated by non-homologous end joining (NHEJ) using a CRISPR/Cas system comprising an enzymatically active Cas9 (eaCas9).

In another aspect, the methods and compositions discussed herein may be used to alter the *CCR5* gene to treat or prevent HIV infection or AIDS by targeting non-coding sequence of the *CCR5* gene, e.g., promoter, an enhancer, an intron, 3'UTR, and/or polyadenylation signal. In one embodiment, the gene, e.g., the non-coding sequence of the *CCR5* gene, is targeted to knockout the gene, e.g., to eliminate expression of the gene, e.g., to knockout both alleles of the CCR5 gene, e.g., by induction of an alteration comprising a deletion or mutation in the *CCR5* gene. In an embodiment, the method provides an alteration that comprises an insertion or deletion.

In one aspect, disclosed herein is a gRNA molecule, e.g., an isolated or non-naturally occurring gRNA molecule, comprising a targeting domain which is complementary with a target domain from the *CCR5* gene.

In an embodiment, the targeting domain of the gRNA molecule is configured to provide a cleavage event, e.g., a double strand break or a single strand break, sufficiently close to a *CCR5* target position in the *CCR5* gene to allow alteration, e.g., alteration associated with NHEJ, of a *CCR5* target position in the *CCR5* gene. In an embodiment the alteration comprises an insertion or deletion. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides *of a CCR5* target position. The break, e.g., a double strand or single strand break, can be positioned upstream or downstream of a *CCR5* target position in the *CCR5* gene.

In an embodiment, a second gRNA molecule comprising a second targeting domain is configured to provide a cleavage event, e.g., a double strand break or a single strand break, sufficiently close to the *CCR5* target position in the *CCR5* gene, to allow alteration, e.g., alteration associated with NHEJ, of the *CCR5* target position in the *CCR5* gene, either alone or in combination with the break positioned by said first gRNA molecule. In an embodiment, the targeting domains of the first and second gRNA molecules are configured such that a cleavage event, e.g., a double strand or single strand break, is positioned, independently for each of the gRNA molecules, within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the breaks, e.g., double strand or single strand breaks, are positioned on both sides of a nucleotide of a *CCR5* target position in the *CCR5* gene. In an embodiment, the breaks, e.g., double strand or single strand breaks, are positioned on one side, e.g., upstream or downstream, of a nucleotide of a *CCR5* target position in the *CCR5* gene.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides *of a CCR5* target position. In an embodiment, the first and second gRNA molecules are configured such, that when guiding a Cas9 nickase, a single strand break will be accompanied by an additional single strand break, positioned by a second gRNA, sufficiently close to one another to result in alteration of a *CCR5* target position in the *CCR5*gene. In an embodiment, the first and second gRNA molecules are configured such that a single strand break positioned by said second gRNA is within 10, 20, 30, 40, or 50 nucleotides of the break positioned by said first gRNA molecule, e.g., when the Cas9 is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream *of a CCR5* target position in the *CCR5* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domain of a second gRNA molecule is configured such that a double strand break is positioned downstream of a *CCR5* target position in the *CCR5* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream *of a CCR5* target position in the *CCR5* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domains of a second and third gRNA molecule are configured such that two single strand breaks are positioned downstream of a *CCR5* target position in the *CCR5* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the targeting domain of the first, second and third gRNA molecules are configured such that a cleavage event, e.g., a double strand or single strand break, is positioned, independently for each of the gRNA molecules

In an embodiment, a first and second single strand breaks can be accompanied by two additional single strand breaks positioned by a third gRNA molecule and a fourth gRNA molecule. For example, the targeting domain of a first and second gRNA molecule are configured such that two single strand breaks are positioned upstream of a *CCR5* target position in the *CCR5* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domains of a third and fourth gRNA molecule are configured such that two single strand breaks are positioned downstream of a *CCR5* target position in the *CCR5* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position.

It is contemplated herein that when multiple gRNAs are used to generate (1) two single stranded breaks in close proximity, (2) two double stranded breaks, e.g., flanking a mutation (e.g., to remove a piece of DNA, e.g., a insertion mutation) or to create more than one indel in an early coding region, (3) one double stranded break and two paired nicks flanking a mutation (e.g., to remove a piece of DNA, e.g., a insertion mutation) or (4) four single stranded breaks, two on each side of a mutation, that they are targeting the same *CCR5* target position. It is further contemplated herein that multiple gRNAs may be used to target more than one mutation in the same gene.

In some embodiments, the targeting domain of the first gRNA molecule and the targeting domain of the second gRNA molecules are complementary to opposite strands of the target nucleic acid molecule. In some embodiments, the gRNA molecule and the second gRNA molecule are configured such that the PAMs are oriented outward.

In an embodiment, the targeting domain of a gRNA molecule is configured to avoid unwanted target chromosome elements, such as repeat elements, e.g., Alu repeats, in the target domain. The gRNA molecule may be a first, second, third and/or fourth gRNA molecule.

In an embodiment, the targeting domain of a gRNA molecule is configured to position a cleavage event sufficiently far from a preselected nucleotide, e.g., the nucleotide of a coding region, such that the nucleotide is not be altered. In an embodiment, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

In an embodiment, more than one gRNA is used to position breaks, e.g., two single stranded breaks or two double stranded breaks, or a combination of single strand and double strand breaks, e.g., to create one or more indels, in the target nucleic acid sequence.

In an embodiment, the targeting domain which is complementary with the *CCR5* gene is 16 nucleotides or more in length. In an embodiment, the targeting domain is 16 nucleotides in length. In an embodiment, the targeting domain is 17 nucleotides in length. In an embodiment, the targeting domain is 18 nucleotides in length. In an embodiment, the targeting domain is 19 nucleotides in length. In an embodiment, the targeting domain is 20 nucleotides in length. In an embodiment, the targeting domain is 21 nucleotides in length. In an embodiment, the targeting domain is 22 nucleotides in length. In an embodiment, the targeting domain is 23 nucleotides in length. In an embodiment, the targeting domain is 24 nucleotides in length. In an embodiment, the targeting domain is 25 nucleotides in length. In an embodiment, the targeting domain is 26 nucleotides in length.

In an embodiment, the targeting domain comprises 16 nucleotides. In an embodiment, the targeting domain comprises 17 nucleotides. In an embodiment, the targeting domain comprises 18 nucleotides. In an embodiment, the targeting domain comprises 19 nucleotides. In an embodiment, the targeting domain comprises 20 nucleotides. In an embodiment, the targeting domain comprises 21 nucleotides. In an embodiment, the targeting domain comprises 22 nucleotides. In an embodiment, the targeting domain comprises 23 nucleotides. In an embodiment, the targeting domain comprises 24 nucleotides. In an embodiment, the targeting domain comprises 25 nucleotides. In an embodiment, the targeting domain comprises 26 nucleotides.

A gRNA as described herein may comprise from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a gRNA comprises a linking domain of no more than 25 nucleotides in length; a proximal and tail domain, that taken together, are at least 20 nucleotides in length; and a targeting domain of equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain of equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain of equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 40 nucleotides in length; and a targeting domain of equal to or greater than 16, 17, 18, 19, 20, 21, 22, 23, 24, 25 or 26 nucleotides in length.

A cleavage event, e.g., a double strand or single strand break, is generated by a Cas9 molecule. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule).

In an embodiment, the eaCas9 molecule catalyzes a double strand break.

In some embodiments, the eaCas9 molecule comprises HNH-like domain cleavage activity but has no, or no significant, N-terminal RuvC-like domain cleavage activity. In this case, the eaCas9 molecule is an HNH-like domain nickase, e.g., the eaCas9 molecule comprises a mutation at D10, e.g., D10A. In other embodiments, the eaCas9 molecule comprises N-terminal RuvC-like domain cleavage activity but has no, or no significant, HNH-like domain cleavage activity. In this instance, the eaCas9 molecule is an N-terminal RuvC-like domain nickase, e.g., the eaCas9 molecule comprises a mutation at H840, e.g., H840A. In an embodiment, the eaCas9 molecule is an N-terminal RuvC-like domain nickase, e.g., the eaCas9 molecule comprises a mutation at N863, e.g., N863A.

In an embodiment, a single strand break is formed in the strand of the target nucleic acid to which the targeting domain of said gRNA is complementary. In another embodiment, a single strand break is formed in the strand of the target nucleic acid other than the strand to which the targeting domain of said gRNA is complementary.

In another aspect, disclosed herein is a composition comprising (a) a gRNA molecule comprising a targeting domain that is complementary with a target domain in the *CCR5* gene, as described herein. The composition of (a) may further comprise (b) a Cas9 molecule, e.g., a Cas9 molecule as described herein. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule). Alternatively, in some embodiments, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

A composition of (a) and (b) may further comprise (c) a second, third and/or fourth gRNA molecule, e.g., a second, third and/or fourth gRNA molecule described herein. In an embodiment, a composition may comprise at least two gRNA molecules to target two or more of target position on the *CCR5* gene. In an embodiment, the composition further comprises a governing gRNA molecule, or a nucleic acid that encodes a governing gRNA molecule.

In another aspect, disclosed herein is a method of altering a cell, e.g., altering the structure, e.g., altering the sequence, of a target nucleic acid of a cell, comprising contacting said cell with: (a) a gRNA that targets the *CCR5* gene, e.g., a gRNA as described herein; (b) a Cas9 molecule, e.g., a Cas9 molecule as described herein; and optionally, (c) a second, third and/or fourth gRNA that targets *CCR5* gene, e.g., a gRNA.

In some embodiments, the method comprises contacting said cell with (a) and (b).

In some embodiments, the method comprises contacting said cell with (a), (b), and (c).

In some embodiments, the method comprises contacting a cell from a subject suffering from or likely to develop an HIV infection or AIDS. The cell may be from a subject having a mutation at a *CCR5* target position.

In some embodiments, the method of altering a cell as described herein comprises acquiring knowledge of the presence of a *CCR5* target position in said cell, prior to the contacting step. Acquiring knowledge of the presence of a *CCR5* target position in the cell may be by sequencing the *CCR5* gene, or a portion of the *CCR5* gene.

In an embodiment, contacting comprises delivering to the cell a Cas9 molecule of (b), as a protein or an mRNA, said gRNA of (a), as an RNA, and optionally said second gRNA of (c), as an RNA.

In an embodiment, contacting comprises delivering to the cell a gRNA of (a) as an RNA, optionally said second gRNA of (c) as an RNA, and a nucleic acid that encodes the Cas9 molecule of (b).

In another aspect, disclosed herein is a method of treating a subject suffering from or likely to develop an HIV infection or AIDS, e.g., altering the structure, e.g., sequence, of a target nucleic acid of the subject, comprising contacting a cell from the subject with:
(a) a gRNA that targets the *CCR5* gene;
(b) a Cas9 molecule, e.g., a Cas9 molecule disclosed herein; and
optionally, (c)(i) a second gRNA that targets the *CCR5* gene, and
further optionally, (c)(ii) a third gRNA, and still further optionally, (c)(iii) a fourth gRNA that target the *CCR5* gene.

In some embodiments, contacting comprises contacting with (a) and (b).

In some embodiments, contacting comprises contacting with (a), (b), and (c)(i).

In some embodiments, contacting comprises contacting with (a), (b), (c)(i) and (c)(ii).

In some embodiments, contacting comprises contacting with (a), (b), (c)(i), (c)(ii) and (c)(iii).

In an embodiment, the method comprises acquiring knowledge of the presence of a mutation at a *CCR5* target position in said subject.

In an embodiment, the method comprises acquiring knowledge of the presence of a mutation at a *CCR5* target position in said subject by sequencing the *CCR5* gene or a portion of the *CCR5* gene.

In an embodiment, the method comprises correcting a mutation at a *CCR5* target position.

In an embodiment, the method comprises correcting a mutation at a *CCR5* target position by NHEJ.

When the method comprises inducing a mutation at a *CCR5* target position by NHEJ in the coding region or a non-coding region, a Cas9 of (b) and at least one guide RNA (e.g., a guide RNA of (a) are included in the contacting step.

In an embodiment, a cell of the subject is contacted *ex vivo* with (a), (b) and optionally (c). In an embodiment, said cell is returned to the subject's body.

In an embodiment, contacting comprises delivering to said subject said Cas9 molecule of (b), as a protein or mRNA, and a nucleic acid which encodes (a) and optionally (c).

In an embodiment, contacting comprises delivering to the subject the Cas9 molecule of (b), as a protein or mRNA, the gRNA of (a), as an RNA, and optionally the second gRNA of (c), as an RNA.

In an embodiment, contacting comprises delivering to the subject the gRNA of (a), as an RNA, optionally said second gRNA of (c), as an RNA, and a nucleic acid that encodes the Cas9 molecule of (b).

In another aspect, disclosed herein is a reaction mixture comprising a gRNA, a nucleic acid, or a composition described herein, and a cell, e.g., a cell from a subject having, or likely to develop *CCR5,* or a subject having a mutation at a *CCR5* target position

In another aspect, disclosed herein is a kit comprising, (a) gRNA molecule described herein, and one or more of the following:
(b) a Cas9 molecule, e.g., a Cas9 molecule described herein, or a nucleic acid or mRNA that encodes the Cas9;
(c)(i) a second gRNA molecule, e.g., a second gRNA molecule described herein;
(c)(ii) a third gRNA molecule, e.g., a third gRNA molecule described herein;
(c)(iii) a fourth gRNA molecule, e.g., a fourth gRNA molecule described herein.

The compositions, reaction mixtures and kits, as disclosed herein, can also include a governing gRNA molecule, e.g., a governing gRNA molecule disclosed herein.

### VI.3 Circulating blood cells (e.g., targeting HBB or BCL11A genes for treating SCD)

In another aspect, the target cell is a circulating blood cell, e.g., a reticulocyte, a myeloid progenitor cell, or a hematopoietic stem cell. In an embodiment, the target cell is a bone marrow cell (e.g., a myeloid progenitor cell, an erythroid progenitor cell, a hematopoietic stem cell, or a mesenchymal stem cell). In an embodiment, the target cell is a myeloid progenitor cell (e.g. a common myeloid progenitor (CMP) cell). In an embodiment, the target cell is an erythroid progenitor cell (e.g. a megakaryocyte erythroid progenitor (MEP) cell). In an embodiment, the target cell is a hematopoietic stem cell (e.g. a long term hematopoietic stem cell (LT-HSC), a short term hematopoietic stem cell (ST-HSC), a multipotent progenitor (MPP) cell, a lineage restricted progenitor (LRP) cell).

In an embodiment, the target cell is manipulated *ex vivo* by editing (e.g., repairing a mutation in) the *HBB* target gene and/or modulating the expression of the *BCL11A* target gene, and administered to the subject. Sources of target cells for *ex vivo* manipulation may include, by way of example, the subject's blood, the subject's cord blood, or the subject's bone marrow. Sources of target cells for *ex vivo* manipulation may also include, by way of example, heterologous donor blood, cord blood, or bone marrow.

In an embodiment, a myeloid progenitor cell is removed from the subject, manipulated *ex vivo* as described above, and the myeloid progenitor cell is returned to the subject. In an embodiment, an erythroid progenitor cell is removed from the subject, manipulated *ex vivo* as described above, and the erythroid progenitor cell is returned to the subject. In an embodiment, a hematopoietic stem cell is removed from the subject, manipulated *ex vivo* as described above, and the hematopoietic stem cell is returned to the subject. In an embodiment, a CD34+ hematopoietic stem cell is removed from the subject, manipulated *ex vivo* as described above, and the CD34+ hematopoietic stem cell is returned to the subject.

A suitable cell can also include a stem cell such as, by way of example, an embryonic stem cell, an induced pluripotent stem cell, a hematopoietic stem cell, a neuronal stem cell and a mesenchymal stem cell. In an embodiment, the cell is an induced pluripotent stem (iPS) cell or a cell derived from an iPS cell, e.g., an iPS cell generated from the subject, modified to induce a mutation and differentiated into a clinically relevant cell such as a myeloid progenitor cell, an erythroid progenitor cell or a hematopoietic stem cell.

Cells produced by the methods described herein may be used immediately. Alternatively, the cells may be frozen (e.g., in liquid nitrogen) and stored for later use. The cells will usually be frozen in 10% dimehtylsulfoxide (DMSO), 50% serum, 40% buffered medium, or some other such solution as is commonly used in the art to preserve cells at such freezing temperature and thawed in such a manner as commonly known in the art for thawing frozen cultured cells.

### Methods to Treat or Prevent Sickle Cell Disease (SCD)

Disclosed herein are approaches to treat or prevent SCD, using the compositions and methods described herein. One approach to treat or prevent SCD is to repair (i.e., correct) one or more mutations in the *HBB* gene by HDR. In this approach, mutant *HBB* allele(s) are corrected and restored to wild type state. While not wishing to be bound by theory, it is believed that correction of the glutamic acid to valine substitution at amino acid 6 in the beta-globin gene restores wild type beta-globin production within erythroid cells. The methods described herein can be performed in all cell types. Beta-globin is expressed in cells of erythroid cell lineage. In an embodiment, an erythroid cell is targeted.

In an embodiment, one *HBB* allele is repaired in the subject. In another embodiment, both *HBB* alleles are repaired in the subject. In either situation, the subjects can be cured of disease. As the disease only displays a phenotype when both alleles are mutated, repair of a single allele is adequate for a cure.

In one approach, the *BCL11A* gene is targeted as a targeted knockout or knockdown, e.g., to increase expression of fetal hemoglobin.

While not wishing to be bound by theory, it is considered that increasing levels of fetal hemoglobin (HbF) in subjects with SCD may ameliorate disease. Fetal hemoglobin can replace beta hemoglobin in the hemoglobin complex, form adequate tetramers with alpha hemoglobin, and effectively carry oxygen to tissues. Subjects with SCD who express higher levels of fetal hemoglobin have been found to have a less severe phenotype. Hydroxyurea, often used in the treatment of SCD, may exert its mechanism of action via increasing levels of HbF production.

In an embodiment, knockout or knockdown of the *BCL11A* gene increases fetal hemoglobin levels in SCD subjects and improves phenotype and/or reduces or prevents disease progression. *BCL11A* is a zinc-finger repressor that is involved in the regulation of fetal hemoglobin and acts to repress the synthesis of fetal hemoglobin. Knockout of the *BCL11A* gene in erythroid cells induces increased fetal hemoglobin (HbF) synthesis and increased HbF can result in more effective oxygen carrying capacity in subjects with SCD (HbF will form tetramers with hemoglobin alpha).

In an embodiment, the *BCL11A* knockout or knockdown is targeted specifically to cells of the erythroid lineage. *BCL11A* knockout in erythroid cells has been found in *in vitro* studies to have no effect on erythroid growth, maturation and function. In an embodiment, erythroid cells are preferentially targeted, e.g., at least 90%, 95%, 96%, 97%, 98%, 99%, or 100% of the targeted cells are erythroid cells. For example, if cells are treated *ex vivo* and returned to the subject, erythroid cells are preferentially modified.

In an embodiment, the methods described herein result in increased fetal hemoglobin synthesis in SCD subjects, thereby improving disease phenotype in subjects with SCD. For example, subjects with SCD will suffer from less severe anemia and will need fewer blood transfusions. They will therefore have fewer complications arising from transfusions and chelation therapy. In an embodiment, the method described herein increases fetal hemoglobin synthesis and improves the oxygen carrying capacity of erythroid cells. For example, subjects are expected to demonstrate decreased rates of extramedullary erythropoiesis and decreased erythroid hypertrophy within the bone marrow. In an embodiment, the method described herein results in reduction of bone fractures, bone abnormalities, splenomegaly, and thrombosis.

Knockdown or knockout of one or both *BCL11A* alleles may be performed prior to disease onset or after disease onset, but preferably early in the disease course.

In an embodiment, the method comprises initiating treatment of a subject prior to disease onset.

In an embodiment, the method comprises initiating treatment of a subject after disease onset.

In an embodiment, the method comprises initiating treatment of a subject well after disease onset, e.g., 1, 2, 3, 4, 5, 6, 7, 8, 9, 10, 12, 24, or 36 months after onset of SCD. While not wishing to be bound by theory it is believed that this treatment may be effective if subjects present well into the course of illness.

In an embodiment, the method comprises initiating treatment of a subject in an advanced stage of disease.

Overall, initiation of treatment for subjects at all stages of disease is expected to prevent negative consequences of disease and be of benefit to subjects.

In an embodiment, the method comprises initiating treatment of a subject prior to disease expression. In an embodiment, the method comprises initiating treatment of a subject in an early stage of disease, e.g., when a subject has tested positive for SCD mutations but has no signs or symptoms associated with SCD.

In an embodiment, the method comprises initiating treatment of a subject who is transfusion-dependent.

In an embodiment, a cell is treated, e.g., *ex vivo.* In an embodiment, an *ex vivo* treated cell is returned to a subject.

In an embodiment, allogenic or autologous bone marrow or erythroid cells are treated *ex vivo.* In an embodiment, an *ex vivo* treated allogenic or autologous bone marrow or erythroid cells are administered to the subject. In an embodiment, an erythroid cell, e.g., an autologous erythroid cell, is treated *ex vivo* and returned to the subject. In an embodiment, an autologous stem cell, is treated *ex vivo* and returned to the subject. In an embodiment, the modified HSCs are administered to the patient following no myeloablative pre-conditioning. In an embodiment, the modified HSCs are administered to the patient following mild myeloablative pre-conditioning such that following engraftment, some of the hematopoietic cells are devied from the modified HSCs. In other aspects, the HSCs are administered after full myeloablation such that following engraftment, 100% of the hematopoietic cells are derived from the modified HSCs.

### Methods of repairing mutation(s) in the HBB gene

One approach to treat or prevent SCD is to repair (i.e., correct) one or more mutations in the *HBB* gene, e.g., by HDR. In this approach, mutant *HBB* allele(s) are corrected and restored to wild type state. While not wishing to be bound by theory, it is believed that correction of the glutamic acid to valine substitution at amino acid 6 in the beta-globin gene restores wild type beta-globin production within erythroid cells. The method described herein can be performed in all cell types. Beta-globin is expressed in cells of erythroid cell lineage. In an embodiment, an erythroid cell is targeted.

In an embodiment, one *HBB* allele is repaired in the subject. In another embodiment, both *HBB* alleles are repaired in the subject. In either situation, the subjects can be cured of disease. As the disease only displays a phenotype when both alleles are mutated, repair of a single allele is adequate for a cure.

In one aspect, methods and compositions discussed herein, provide for the correction of the underlying genetic cause of SCD, e.g., the correction of a mutation at a target position in the *HBB* gene, e.g., correction of a mutation at amino acid position 6, e.g., an E6V substitution in the *HBB* gene.

In an embodiment, the method provides for the correction of a mutation at a target position in the *HBB* gene, e.g., correction of a mutation at amino acid position 6, e.g., an E6V substitution in the *HBB* gene. As described herein, in one embodiment, the method comprises the introduction of one or more breaks (e.g., single strand breaks or double strand breaks) sufficiently close to (e.g., either 5' or 3' to) the target position in the *HBB* gene, e.g., E6V.

In an embodiment, the targeting domain of the gRNA molecule is configured to provide a cleavage event, e.g., a double strand break or a single strand break, sufficiently close to (e.g., either 5' or 3' to) the target position in the *HBB* gene, e.g., E6V to allow correction, e.g., alteration in the *HBB* gene, e.g., associated with HDR. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position in the *HBB* gene, e.g., E6V. The break, e.g., a double strand or single strand break, can be positioned upstream or downstream of the target position in the *HBB* gene, e.g., E6V.

In an embodiment, a second gRNA molecule is configured to provide a cleavage event, e.g., a double strand break or a single strand break, sufficiently close to (e.g., either 5' or 3' to) the target position in the *HBB* gene, e.g., E6V to allow correction, e.g., alteration associated with HDR in the *HBB* gene. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position in the *HBB* gene, e.g., E6V. The break, e.g., a double strand or single strand break, can be positioned upstream or downstream of the target position in the *HBB* gene, e.g., E6V.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position in the *HBB* gene, e.g., E6V. In an embodiment, the first and second gRNA molecules are configured such, that when guiding a Cas9 nickase, a single strand break will be accompanied by an additional single strand break, positioned by a second gRNA, sufficiently close to one another to result in alteration of the target position in the *HBB* gene, e.g., E6V. In an embodiment, the first and second gRNA molecules are configured such that a single strand break positioned by said second gRNA is within 10, 20, 30, 40, or 50 nucleotides of the break positioned by said first gRNA molecule, e.g., when the Cas9 is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream of the target position in the *HBB* gene, e.g., E6V, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domain of a second gRNA molecule is configured such that a double strand break is positioned downstream the target position in the *HBB* gene, e.g., E6V, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream of the target position in the *HBB* gene, e.g., E6V, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domains of a second and third gRNA molecule are configured such that two single strand breaks are positioned downstream of the target position in the *HBB* gene, e.g., E6V, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the targeting domain of the first, second and third gRNA molecules are configured such that a cleavage event, e.g., a double strand or single strand break, is positioned, independently for each of the gRNA molecules.

In an embodiment, a first and second single strand breaks can be accompanied by two additional single strand breaks positioned by a third gRNA molecule and a fourth gRNA molecule. For example, the targeting domain of a first and second gRNA molecule are configured such that two single strand breaks are positioned upstream of the target position in the *HBB* gene, e.g., E6V, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position in the *HBB* gene, e.g., E6V; and the targeting domains of a third and fourth gRNA molecule are configured such that two single strand breaks are positioned downstream of the target position in the *HBB* gene, e.g., E6V, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position in the *HBB* gene, e.g., E6V.

In an embodiment, a mutation in the *HBB* gene, e.g., E6V is corrected using an exogenously provided template nucleic acid, e.g., by HDR.

In another embodiment, a mutation in the *HBB* gene, e.g., E6V is corrected without using an exogenously provided template nucleic acid, e.g., by HDR. In an embodiment, alteration of the target sequence occurs with an endogenous genomic donor sequence, e.g., by HDR. In an embodiment, the endogenous genomic donor sequence comprises one or more nucleotides derived from the *HBB* gene. In an embodiment, a mutation in the *HBB* gene, e.g., E6V is corrected by an endogenous genomic donor sequence (e.g., an *HBB* gene).

### Methods of Altering BCL11A

One approach to increase the expression of HbF involves identification of genes whose products play a role in the regulation of globin gene expression. One such gene is *BCL11A.* It plays a role in the regulation of γ globin expression. It was first identified because of its role in lymphocyte development. *BCL11A* encodes a zinc finger protein that is thought to be involved in the stage specific regulation of γ globin expression. *BCL11A* is expressed in adult erythroid precursor cells and down-regulation of its expression leads to an increase in γ globin expression. In addition, it appears that the splicing of the *BCL11A* mRNA is developmentally regulated. In embryonic cells, it appears that the shorter *BCL11A* mRNA variants, known as *BCL11A*-S and *BCL11A*-XS are primary expressed, while in adult cells, the longer *BCL11A*-L and *BCL11A*-XL mRNA variants are predominantly expressed. See, Sankaran et al (2008) Science 322 p. 1839. The *BCL11A* protein appears to interact with the β globin locus to alter its conformation and thus its expression at different developmental stages. Thus, if *BCL11A* expression is altered e.g., disrupted (e.g., reduced or eliminated), it results in the elevation of γ globin and HbF production.

Disclosed herein are methods for altering the SCD target position in the *BCL11A* gene.

Altering the SCD target position is achieved, e.g., by:
(1) knocking out the *BCL11A* gene:
   (a) insertion or deletion (e.g., NHEJ-mediated insertion or deletion) of one or more nucleotides in close proximity to or within the early coding region of the *BCL11A* gene, or
   (b) deletion (e.g., NHEJ-mediated deletion) of a genomic sequence including the erythroid enhancer of the *BCL11A* gene, or
(2) knocking down the *BCL11A* gene mediated by enzymatically inactive Cas9 (eiCas9) or an eiCas9-fusion protein by targeting the promoter region of the gene.

All approaches give rise to alteration of the *BCL11A* gene.

In one embodiment, methods described herein introduce one or more breaks near the early coding region in at least one allele of the *BCL11A* gene. In another embodiment, methods described herein introduce two or more breaks to flank the erythroid enhancer of SCD target knockout position. The two or more breaks remove (e.g., delete) genomic sequence including the erythorid enhancer. In another embodiment, methods described herein comprises knocking down the *BCL11A* gene mediated by enzymatically inactive Cas9 (eiCas9) or an eiCas9-fusion protein by targeting the promoter region of SCD target knockdown position. All methods described herein result in alteration of the *BCL11A* gene.

### NHEJ-mediated introduction of an indel in close proximity to or within the early coding region of the SCD knockout position

In an embodiment, the method comprises introducing an NHEJ-mediated insertion or deletion of one more nucleotides in close proximity to the SCD target knockout position (e.g., the early coding region) of the *BCL11A* gene. As described herein, in one embodiment, the method comprises the introduction of one or more breaks (e.g., single strand breaks or double strand breaks) sufficiently close to (e.g., either 5' or 3' to) the early coding region of the SCD target knockout position, such that the break-induced indel could be reasonably expected to span the SCD target knockout position (e.g., the early coding region). While not wishing to be bound by theory, it is believed that NHEJ-mediated repair of the break(s) allows for the NHEJ-mediated introduction of an indel in close proximity to within the early coding region of the SCD target knockout position.

In an embodiment, the targeting domain of the gRNA molecule is configured to provide a cleavage event, e.g., a double strand break or a single strand break, sufficiently close to the early coding region in the *BCL11A* gene to allow alteration, e.g., alteration associated with NHEJ in the *BCL11A* gene. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of a SCD target knockout position. The break, e.g., a double strand or single strand break, can be positioned upstream or downstream of a SCD target knockout position in the *BCL11A* gene.

In an embodiment, a second gRNA molecule comprising a second targeting domain is configured to provide a cleavage event, e.g., a double strand break or a single strand break, sufficiently close to the early coding region in the *BCL11A* gene, to allow alteration, e.g., alteration associated with NHEJ in the *BCL11A* gene, either alone or in combination with the break positioned by said first gRNA molecule. In an embodiment, the targeting domains of the first and second gRNA molecules are configured such that a cleavage event, e.g., a double strand or single strand break, is positioned, independently for each of the gRNA molecules, within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the breaks, e.g., double strand or single strand breaks, are positioned on both sides of a nucleotide of a SCD target knockout position in the *BCL11A* gene. In an embodiment, the breaks, e.g., double strand or single strand breaks, are positioned on one side, e.g., upstream or downstream, of a nucleotide of a SCD target knockout position in the *BCL11A* gene.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the early coding region in the *BCL11A* gene. In an embodiment, the first and second gRNA molecules are configured such, that when guiding a Cas9 nickase, a single strand break will be accompanied by an additional single strand break, positioned by a second gRNA, sufficiently close to one another to result in alteration of the early coding region in the *BCL11A* gene in the *BCL11A* gene. In an embodiment, the first and second gRNA molecules are configured such that a single strand break positioned by said second gRNA is within 10, 20, 30, 40, or 50 nucleotides of the break positioned by said first gRNA molecule, e.g., when the Cas9 is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream of the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domain of a second gRNA molecule is configured such that a double strand break is positioned downstream of the early coding region in the *BCL11A* gene in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream of the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domains of a second and third gRNA molecule are configured such that two single strand breaks are positioned downstream of the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the targeting domain of the first, second and third gRNA molecules are configured such that a cleavage event, e.g., a double strand or single strand break, is positioned, independently for each of the gRNA molecules.

In an embodiment, a first and second single strand breaks can be accompanied by two additional single strand breaks positioned by a third gRNA molecule and a fourth gRNA molecule. For example, the targeting domain of a first and second gRNA molecule are configured such that two single strand breaks are positioned upstream of the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the early coding region in the *BCL11A* gene; and the targeting domains of a third and fourth gRNA molecule are configured such that two single strand breaks are positioned downstream of a SCD target knockout position in the *BCL11A* gene the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the early coding region in the *BCL11A* gene.

### NHEJ-mediated deletion of the erythroid enhancer at the SCD target position

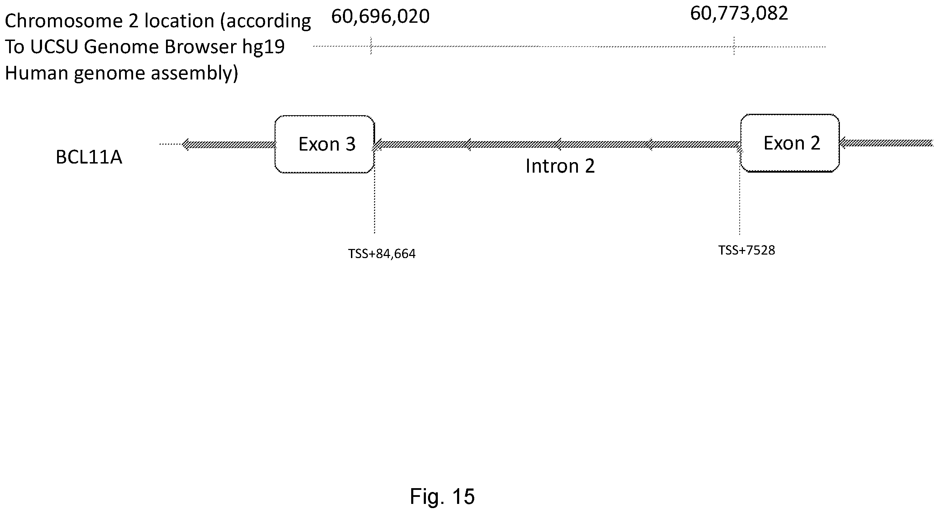

In an embodiment, the method comprises introducing a NHEJ-mediated deletion of a genomic sequence including the erythroid enhancer. As described herein, in one embodiment, the method comprises the introduction of two double strand breaks-one 5' and the other 3' to (i.e., flanking) the SCD target position (e.g., the erythroid enhancer). Two gRNAs, e.g., unimolecular (or chimeric) or modular gRNA molecules, are configured to position the two double strand breaks on opposite sides of the SCD target knockdown position (e.g., the erythroid enhancer) in the *BCL11A* gene. In an embodiment, the first double strand break is positioned upstream of the erythroid enhancer within intron 2 (e.g., between TSS+0.75kb to TSS+52.0kb), and the second double strand break is positioned downstream of the erythroid enhancer within intron 2 (e.g., between TSS+64.4kb to TSS+84.7kb) (see **Fig. 15****).** In an embodiment, the two double strand breaks are positioned to remove a portion of the erythroid enhancer resulting in disruption of one or more DHSs. In an embodiment, the breaks (i.e., the two double strand breaks) are positioned to avoid unwanted target chromosome elements, such as repeat elements, e.g., an *Alu* repeat, or the endogenous splice sites.

The first double strand break may be positioned as follows:
(1) upstream of the 5' end of the erythroid enhancer in intron 2 (e.g., between TSS+0.75kb to TSS+52.0kb), or
(2) within the erythroid enhancer provided that a portion of the erythroid enhancer is removed resulting in disruption of one or more DHSs (e.g., between TSS+52.0kb to TSS+64.4kb),
and the second double strand break to be paired with the first double strand break may be positioned as follows:
(1) downstream the 3' end of the erythroid enhancer in intron 2 (e.g., between TSS+64.4kb to TSS+84.7kb), or
(2) within the erythroid enhancer provided that a portion of the erythroid enhancer is removed resulting in disruption of one or more DHSs (e.g., between TSS+52.0kb to TSS+64.4kb).

For example, the first double strand break may be positioned in the *BCL11A* gene:
(1) between TSS+0.75kb to TSS+10kb,
(2) between TSS+10kb to TSS+20kb,
(3) between TSS+20kb to TSS+30kb,
(4) between TSS+30kb to TSS+40kb,
(5) between TSS+40kb to TSS+45kb,
(6) between TSS+45kb to TSS+47.5kb,
(7) between TSS+47.5kb to TSS+50kb,
(8) between TSS+50kb to TSS+51kb,
(9) between TSS+51kb to TSS+51.1kb,
(10) between TSS+51.1kb to TSS+51.2kb,
(11) between TSS+51.2kb to TSS+51.3kb,
(12) between TSS+51.3kb to TSS+51.4kb,
(13) between TSS+51.4kb to TSS+51.5kb,
(14) between TSS+51.5kb to TSS+51.6kb,
(15) between TSS+51.6kb to TSS+51.7kb,
(16) between TSS+51.7kb to TSS+51.8kb,
(17) between TSS+51.8kb to TSS+51.9kb,
(18) between TSS+51.9kb to TSS+52kb,
(19) between TSS+52kb to TSS+53kb,
(20) between TSS+53kb to TSS+54kb,
(21) between TSS+54kb to TSS+55kb,
(22) between TSS+55kb to TSS+56kb,
(23) between TSS+56kb to TSS+57kb,
(24) between TSS+57kb to TSS+58kb,
(25) between TSS+58kb to TSS+59kb,
(26) between TSS+59kb to TSS+60kb,
(27) between TSS+60kb to TSS+61kb,
(28) between TSS+61kb to TSS+62kb,
(29) between TSS+62kb to TSS+63kb,
(30) between TSS+63kb to TSS+64kb, or
(31) between TSS+64kb to TSS+64.4kb,
and the second double strand break to be paired with the first double strand break may be positioned in the *BCL11A* gene:
(1) between TSS+52kb to TSS+53kb,
(2) between TSS+53kb to TSS+54kb,
(3) between TSS+54kb to TSS+55kb,
(4) between TSS+55kb to TSS+56kb,
(5) between TSS+56kb to TSS+57kb,
(6) between TSS+57kb to TSS+58kb,
(7) between TSS+58kb to TSS+59kb,
(8) between TSS+59kb to TSS+60kb,
(9) between TSS+60kb to TSS+61kb,
(10) between TSS+61kb to TSS+62kb,
(11) between TSS+62kb to TSS+63kb,
(12) between TSS+63kb to TSS+64kb,
(13) between TSS+64kb to TSS+64.4kb,
(14) between TSS+64.4kb to TSS+65kb,
(15) between TSS+65kb to TSS+65.1kb,
(16) between TSS+65.1kb to TSS+65.2kb,
(17) between TSS+65.2kb to TSS+65.3kb,
(18) between TSS+65.3kb to TSS+65.4kb,
(19) between TSS+65.4kb to TSS+65.5kb,
(20) between TSS+65.5kb to TSS+65.7kb,
(21) between TSS+65.7kb to TSS+65.8kb,
(22) between TSS+65.8kb to TSS+65.9kb,
(23) between TSS+65.9kb to TSS+66kb,
(24) between TSS+66kb to TSS+67kb,
(25) between TSS+67kb to TSS+68kb,
(26) between TSS+68kb to TSS+69kb,
(27) between TSS+69kb to TSS+70kb,
(28) between TSS+70kb to TSS+75kb,
(29) between TSS+75kb to TSS+80kb, or
(30) between TSS+80kb to TSS+84.4kb.

While not wishing to be bound by theory, it is believed that the two double strand breaks allow for NHEJ-mediated deletion of erythroid enhancer in the *BCL11A* gene.

In an embodiment, the method comprises introducing a NHEJ-mediated deletion of a genomic sequence including the erythroid enhancer. As described herein, in one embodiment, the method comprises the introduction of two sets of breaks (e.g., one double strand break and a pair of single strand breaks)-one 5' and the other 3' to (i.e., flanking) the SCD target position (e.g., the erythroid enhancer). Two gRNAs, e.g., unimolecular (or chimeric) or modular gRNA molecules, are configured to position the two sets of breaks (either the double strand break or the pair of single strand breaks) on opposite sides of the SCD target knockdown position (e.g., the erythroid enhancer) in the *BCL11A* gene. In an embodiment, the first set of breaks (either the double strand break or the pair of single strand breaks) is positioned upstream of the erythroid enhancer within intron 2 (e.g., between TSS+0.75kb to TSS+52.0kb), and the second set of breaks (either the double strand break or the pair of single strand breaks) is positioned downstream of the erythroid enhancer within intron 2 (e.g., between TSS+64.4kb to TSS+84.7kb) (see **Fig. 15**). In an embodiment, the two sets of breaks (either the double strand break or the pair of single strand breaks) are positioned to remove a portion of the erythroid enhancer resulting in disruption of one or more DHSs. In an embodiment, the breaks (i.e., the two sets of breaks (either the double strand break or the pair of single strand breaks)) are positioned to avoid unwanted target chromosome elements, such as repeat elements, e.g., an *Alu* repeat, or the endogenous splice sites.

The first set of breaks (either the double strand break or the pair of single strand breaks) may be positioned as follows:
(1) upstream of the 5' end of the erythroid enhancer in intron 2 (e.g., between TSS+0.75kb to TSS+52.0kb), or
(2) within the erythroid enhancer provided that a portion of the erythroid enhancer is removed resulting in disruption of one or more DHSs (e.g., between TSS+52.0kb to TSS+64.4kb),
and the second set of breaks (either the double strand break or the pair of single strand breaks) to be paired with the first set of breaks (either the double strand break or the pair of single strand breaks) may be positioned as follows:
(1) downstream the 3' end of the erythroid enhancer in intron 2 (e.g., between TSS+64.4kb to TSS+84.7kb), or
(2) within the erythroid enhancer provided that a portion of the erythroid enhancer is removed resulting in disruption of one or more DHSs (e.g., between TSS+52.0kb to TSS+64.4kb).

For example, the first set of breaks (either the double strand break or the pair of single strand breaks) may be positioned in the *BCL11A* gene:
(1) between TSS+0.75kb to TSS+10kb,
(2) between TSS+10kb to TSS+20kb,
(3) between TSS+20kb to TSS+30kb,
(4) between TSS+30kb to TSS+40kb,
(5) between TSS+40kb to TSS+45kb,
(6) between TSS+45kb to TSS+47.5kb,
(7) between TSS+47.5kb to TSS+50kb,
(8) between TSS+50kb to TSS+51kb,
(9) between TSS+51kb to TSS+51.1kb,
(10) between TSS+51.1kb to TSS+51.2kb,
(11) between TSS+51.2kb to TSS+51.3kb,
(12) between TSS+51.3kb to TSS+51.4kb,
(13) between TSS+51.4kb to TSS+51.5kb,
(14) between TSS+51.5kb to TSS+51.6kb,
(15) between TSS+51.6kb to TSS+51.7kb,
(16) between TSS+51.7kb to TSS+51.8kb,
(17) between TSS+51.8kb to TSS+51.9kb,
(18) between TSS+51.9kb to TSS+52kb,
(19) between TSS+52kb to TSS+53kb,
(20) between TSS+53kb to TSS+54kb,
(21) between TSS+54kb to TSS+55kb,
(22) between TSS+55kb to TSS+56kb,
(23) between TSS+56kb to TSS+57kb,
(24) between TSS+57kb to TSS+58kb,
(25) between TSS+58kb to TSS+59kb,
(26) between TSS+59kb to TSS+60kb,
(27) between TSS+60kb to TSS+61kb,
(28) between TSS+61kb to TSS+62kb,
(29) between TSS+62kb to TSS+63kb,
(30) between TSS+63kb to TSS+64kb, or
(31) between TSS+64kb to TSS+64.4kb,
and the second set of breaks (either the double strand break or the pair of single strand breaks) to be paired with the first set of breaks (either the double strand break or the pair of single strand breaks)may be positioned in the *BCL11A* gene:
(1) between TSS+52kb to TSS+53kb,
(2) between TSS+53kb to TSS+54kb,
(3) between TSS+54kb to TSS+55kb,
(4) between TSS+55kb to TSS+56kb,
(5) between TSS+56kb to TSS+57kb,
(6) between TSS+57kb to TSS+58kb,
(7) between TSS+58kb to TSS+59kb,
(8) between TSS+59kb to TSS+60kb,
(9) between TSS+60kb to TSS+61kb,
(10) between TSS+61kb to TSS+62kb,
(11) between TSS+62kb to TSS+63kb,
(12) between TSS+63kb to TSS+64kb,
(13) between TSS+64kb to TSS+64.4kb,
(14) between TSS+64.4kb to TSS+65kb,
(15) between TSS+65kb to TSS+65.1kb,
(16) between TSS+65.1kb to TSS+65.2kb,
(17) between TSS+65.2kb to TSS+65.3kb,
(18) between TSS+65.3kb to TSS+65.4kb,
(19) between TSS+65.4kb to TSS+65.5kb,
(20) between TSS+65.5kb to TSS+65.7kb,
(21) between TSS+65.7kb to TSS+65.8kb,
(22) between TSS+65.8kb to TSS+65.9kb,
(23) between TSS+65.9kb to TSS+66kb,
(24) between TSS+66kb to TSS+67kb,
(25) between TSS+67kb to TSS+68kb,
(26) between TSS+68kb to TSS+69kb,
(27) between TSS+69kb to TSS+70kb,
(28) between TSS+70kb to TSS+75kb,
(29) between TSS+75kb to TSS+80kb, or
(30) between TSS+80kb to TSS+84.4kb.

While not wishing to be bound by theory, it is believed that the two sets of breaks (either the double strand break or the pair of single strand breaks) allow for NHEJ-mediated deletion of erythroid enhancer in the *BCL11A* gene.

In an embodiment, the method comprises introducing a NHEJ-mediated deletion of a genomic sequence including the erythroid enhancer. As described herein, in one embodiment, the method comprises the introduction of two sets of breaks (e.g., two pairs of single strand breaks)-one 5' and the other 3' to (i.e., flanking) the SCD target position (e.g., the erythroid enhancer). Two gRNAs, e.g., unimolecular (or chimeric) or modular gRNA molecules, are configured to position the two sets of breaks on opposite sides of the SCD target knockdown position (e.g., the erythroid enhancer) in the *BCL11A* gene. In an embodiment, the first set of breaks (i.e., the first pair of single strand breaks) is positioned upstream of the erythroid enhancer within intron 2 (e.g., between TSS+0.75kb to TSS+52.0kb), and the second set of breaks (i.e., the second pair of single strand breaks) is positioned downstream of the erythroid enhancer within intron 2 (e.g., between TSS+64.4kb to TSS+84.7kb) (see **Fig. 15**). In an embodiment, the two sets of breaks (e.g., two pairs of single strand breaks)) are positioned to remove a portion of the erythroid enhancer resulting in disruption of one or more DHSs. In an embodiment, the breaks (i.e., the two pairs of single strand breaks) are positioned to avoid unwanted target chromosome elements, such as repeat elements, e.g., an *Alu* repeat, or the endogenous splice sites.

The first pair of single strand breaks may be positioned as follows:
(1) upstream of the 5' end of the erythroid enhancer in intron 2 (e.g., between TSS+0.75kb to TSS+52.0kb), or
(2) within the erythroid enhancer provided that a portion of the erythroid enhancer is removed resulting in disruption of one or more DHSs (e.g., between TSS+52.0kb to TSS+64.4kb),
and the second pair of single strand breaks to be paired with the first pair of single strand breaks may be positioned as follows:
(1) downstream the 3' end of the erythroid enhancer in intron 2 (e.g., between TSS+64.4kb to TSS+84.7kb), or
(2) within the erythroid enhancer provided that a portion of the erythroid enhancer is removed resulting in disruption of one or more DHSs (e.g., between TSS+52.0kb to TSS+64.4kb).

For example, the pair of single strand breaks may be positioned in the *BCL11A* gene:
(1) between TSS+0.75kb to TSS+10kb,
(2) between TSS+10kb to TSS+20kb,
(3) between TSS+20kb to TSS+30kb,
(4) between TSS+30kb to TSS+40kb,
(5) between TSS+40kb to TSS+45kb,
(6) between TSS+45kb to TSS+47.5kb,
(7) between TSS+47.5kb to TSS+50kb,
(8) between TSS+50kb to TSS+51kb,
(9) between TSS+51kb to TSS+51.1kb,
(10) between TSS+51.1kb to TSS+51.2kb,
(11) between TSS+51.2kb to TSS+51.3kb,
(12) between TSS+51.3kb to TSS+51.4kb,
(13) between TSS+51.4kb to TSS+51.5kb,
(14) between TSS+51.5kb to TSS+51.6kb,
(15) between TSS+51.6kb to TSS+51.7kb,
(16) between TSS+51.7kb to TSS+51.8kb,
(17) between TSS+51.8kb to TSS+51.9kb,
(18) between TSS+51.9kb to TSS+52kb,
(19) between TSS+52kb to TSS+53kb,
(20) between TSS+53kb to TSS+54kb,
(21) between TSS+54kb to TSS+55kb,
(22) between TSS+55kb to TSS+56kb,
(23) between TSS+56kb to TSS+57kb,
(24) between TSS+57kb to TSS+58kb,
(25) between TSS+58kb to TSS+59kb,
(26) between TSS+59kb to TSS+60kb,
(27) between TSS+60kb to TSS+61kb,
(28) between TSS+61kb to TSS+62kb,
(29) between TSS+62kb to TSS+63kb,
(30) between TSS+63kb to TSS+64kb, or
(31) between TSS+64kb to TSS+64.4kb,
and the second pair of single strand breaks to be paired with the first pair of single strand breaks may be positioned in the *BCL11A* gene:
(1) between TSS+52kb to TSS+53kb,
(2) between TSS+53kb to TSS+54kb,
(3) between TSS+54kb to TSS+55kb,
(4) between TSS+55kb to TSS+56kb,
(5) between TSS+56kb to TSS+57kb,
(6) between TSS+57kb to TSS+58kb,
(7) between TSS+58kb to TSS+59kb,
(8) between TSS+59kb to TSS+60kb,
(9) between TSS+60kb to TSS+61kb,
(10) between TSS+61kb to TSS+62kb,
(11) between TSS+62kb to TSS+63kb,
(12) between TSS+63kb to TSS+64kb,
(13) between TSS+64kb to TSS+64.4kb,
(14) between TSS+64.4kb to TSS+65kb,
(15) between TSS+65kb to TSS+65.1kb,
(16) between TSS+65.1kb to TSS+65.2kb,
(17) between TSS+65.2kb to TSS+65.3kb,
(18) between TSS+65.3kb to TSS+65.4kb,
(19) between TSS+65.4kb to TSS+65.5kb,
(20) between TSS+65.5kb to TSS+65.7kb,
(21) between TSS+65.7kb to TSS+65.8kb,
(22) between TSS+65.8kb to TSS+65.9kb,
(23) between TSS+65.9kb to TSS+66kb,
(24) between TSS+66kb to TSS+67kb,
(25) between TSS+67kb to TSS+68kb,
(26) between TSS+68kb to TSS+69kb,
(27) between TSS+69kb to TSS+70kb,
(28) between TSS+70kb to TSS+75kb,
(29) between TSS+75kb to TSS+80kb, or
(30) between TSS+80kb to TSS+84.4kb.

While not wishing to be bound by theory, it is believed that the two sets of breaks (e.g., the two pair of single strand breaks) allow for NHEJ-mediated deletion of erythroid enhancer in the *BCL11A* gene.

Knocking down the *BCL11A* gene mediated by enzymatically inactive Cas9 (eiCas9) or an eiCas9-fusion protein by targeting the promoter region of the gene.

A targeted knockdown approach reduces or eliminates expression of functional *BCL11A* gene product. As described herein, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *BCL11A* gene.

Methods and compositions discussed herein may be used to alter the expression of the *BCL11A* gene to treat or prevent SCD by targeting a promoter region of the *BCL11A* gene. In an embodiment, the promoter region, e.g., at least 2 kb, at least 1.5 kb, at least 1.0 kb, or at least 0.5 kb upstream or downstream of the TSS is targeted to knockdown expression of the *BCL11A* gene. In an embodiment, the methods and compositions discussed herein may be used to knock down the *BCL11A* gene to treat or prevent SCD by targeting 0.5 kb upstream or downstream of the TSS. A targeted knockdown approach reduces or eliminates expression of functional *BCL11A* gene product. As described herein, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *BCL11A* gene.

### Other embodiments involving circulating blood cells and HBB and BCL11A genes (SCD)

In an embodiment, methods and compositions discussed herein, provide for the treatment and prevention of Sickle Cell Disease (SCD), also known as Sickle Cell Anemia (SCA). SCD is an inherited hematologic disease.

In healthy individuals, two beta-globin molecules pair with two alpha-globin molecules to form normal hemoglobin (Hb). In SCD, mutations in the *beta-globin* (*HBB*) gene, e.g., a point mutation (GAG → GTG) that results in the substitution of valine for glutamic acid at amino acid position 6 of the beta-globin molecule, cause production of sickle hemoglobin (HbS). HbS is more likely to polymerize and leads to the characteristic sickle shaped red blood cells (RBCs). Sickle shaped RBCs give rise to multiple manifestations of disease, such as, anemia, sickle cell crises, vaso-occlusive crises, aplastic crises and acute chest syndrome. Alpha-globin can also pair with fetal hemoglobin (HbF), which significantly moderates the severe anemia and other symptoms of SCD. However, the expression of HbF is negatively regulated by the *BCL11A* gene product.

Ine one aspect, methods and compositions disclosed herein provide a number of approaches for treating SCD. As is discussed in more detail herein, methods described herein provide for treating SCD by correcting a target position in the *HBB* gene to provide corrected, or functional, e.g., wild type, beta-globin. Methods and compositions discussed herein can be used to treat or prevent SCD by altering the *BCL11A* gene (also known as *B-cell CLL*/*lymphoma 11A, BCL11A-L, BCL11A-S, BCL11A-XL, CTIP1, HBFQTL5* and *ZNF*). *BCL11A* encodes a zinc-finger protein that is involved in the regulation of globin gene expression. By altering the *BCL11A* gene (e.g., one or both alleles of the *BCL11A* gene), the levels of gamma globin can be increased. Gamma globin can replace beta globin in the hemoglobin complex and effectively carry oxygen to tissues, thereby ameliorating SCD disease phenotypes.

In one aspect, methods and compositions discussed herein, provide for the correction of the underlying genetic cause of SCD, e.g., the correction of a mutation at a target position in the *HBB* gene, e.g., correction of a mutation at amino acid position 6, e.g., an E6V substitution in the *HBB* gene.

Mutations in the *HBB* gene (also known as *beta-globin, CD113t-C and HBD*) have been shown to cause SCD. Mutations leading to SCD can be described based on their target positions in the *HBB* gene. In an embodiment, the target position is E6, e.g., E6V, in the *HBB* gene.

"SCD target point position", as used herein, refers to a target position in the *HBB* gene, typically a single nucleotide, which, if mutated, can result in a protein having a mutant amino acid and give rise to SCD. In an embodiment, the SCD target position is the target position at which a change can give rise to an E6 mutant protein, e.g., a protein having an E6V substitution.

While much of the disclosure herein is presented in the context of the mutation in the *HBB* gene that gives rise to an E6 mutant protein (e.g., E6V mutant protein), the methods and compositions herein are broadly applicable to any mutation, e.g., a point mutation or a deletion, in the *HBB* gene that gives rise to SCD.

While not wishing to be bound by theory, it is believed that, in an embodiment, a mutation at an SCD target point position in the *HBB* gene is corrected, e.g., by homology directed repair (HDR), as described herein.

In one aspect, methods and compositions discussed herein may be used to alter the *BCL11A* gene to treat or prevent SCD, by targeting the *BCL11A* gene, e.g., coding or non-coding regions of the *BCL11A* gene. Altering the *BCL11A* gene herein refers to reducing or eliminating (1) *BCL11A* gene expression, (2) *BCL11A* protein function, or (3) the level of *BCL11A* protein.

In an embodiment, the coding region (e.g., an early coding region) of the *BCL11A* gene is targeted for alteration. In an embodiment, a non-coding sequence (e.g., an enhancer region, a promoter region, an intron, 5'UTR, 3'UTR, or polyadenylation signal) is targeted for alteration.

In an embodiment, the method provides an alteration that comprises disrupting the *BCL11A* gene by the insertion or deletion of one or more nucleotides mediated by Cas9 (e.g., enzymatically active Cas9 (eaCas9), e.g., Cas9 nuclease or Cas9 nickase) as described below. This type of alteration is also referred to as "knocking out" the *BCL11A* gene.

In another embodiment, the method provides an alteration that does not comprise nucleotide insertion or deletion in the *BCL11A* gene and is mediated by enzymatically inactive Cas9 (eiCas9) or an eiCas9-fusion protein, as described below. This type of alteration is also referred to as "knocking down" the *BCL11A* gene.

In an embodiment, the methods and compositions discussed herein may be used to alter the *BCL11A* gene to treat or prevent SCD by knocking out one or both alleles of the *BCL11A* gene. In an embodiment, the coding region (e.g., an early coding region) of the *BCL11A* gene, is targeted to alter the gene. In an embodiment, a non-coding region of the *BCL11A* gene (e.g., an enhancer region, a promoter region, an intron, 5' UTR, 3'UTR, polyadenylation signal) is targeted to alter the gene. In an embodiment, an enhancer (e.g., a tissue-specific enhancer, e.g., a myeloid enhancer, e.g., an erythroid enhancer) is targeted to alter the gene. *BCL11A* erythroid enhancer comprises an approximate12.4 kb fragment of *BCL11A* intron2, located between approximate +52.0 to +64.4 kilobases (kb) from the Transcription Start Site (TSS+52kb to TSS+64.4kb, see Fig. 15). It's also referred to herein as chromosome 2 location 60,716,189-60,728,612 (according to UCSC Genome Browser hg 19 human genome assembly). Three deoxyribonuclese I hypersensitive sites (DHSs), TSS+62kb, TSS+58kb and TSS+55kb are located in this region. Deoxyribonuclease I sensitivity is a marker for gene regulatory elements. While not wishing to be bound by theory, it's believed that deleting the ehancer region (e.g., TSS+52kb to TSS+64.4kb) may reduce or eliminate *BCL11A* expression in erythroid precursors which leads to gamma globin derepression while sparing *BCL11A* expression in nonerythoroid lineages. In an embodiment, the method provides an alteration that comprises a deletion of the enhancer region (e.g., a tissue-specific enhancer, e.g., a myleloid enhancer, e.g., an erythroid enhancer) or a protion of the region resulting in disruption of one or more DNase 1-hypersensitivie sites (DHS). In an embodiment, the method provides an alteration that comprises an insertion or deletion of one or more nucleotides. As described herein, in an embodiment, a targeted knockout approach is mediated by non-homologous end joining (NHEJ) using a CRISPR/Cas system comprising an enzymatically active Cas9 (eaCas9). In an embodiment, a targeted knockout approach alters the *BCL11A* gene. In an embodiment, a targeted knockout approach reduces or eliminates expression of functional *BCL11A* gene product. In an embodiment, targeting affects one or both alleles of the *BCL11A* gene. In an embodiment, an enhancer disruption approach reduces or eliminates expression of functional *BCL11A* gene product in the erythroid lineage.

"SCD target knockout position", as used herein, refers to a position in the *BCL11A* gene, which if altered, e.g., disrupted by insertion or deletion of one or more nucleotides, e.g., by NHEJ-mediated alteration, results in reduction or elimination of expression of functional *BCL11A* gene product. In an embodiment, the position is in the *BCL11A* coding region, e.g., an early coding region. In an embodiment, the position is in the *BCL11A* non-coding region, e.g., an enhancer region.

In an embodiment, methods and compositions discussed herein, provide for altering (e.g., knocking out) the *BCL11A* gene. In an embodiment, knocking out the *BCL11A* gene herein refers to (1) insertion or deletion (e.g., NHEJ-mediated insertion or deletion) of one or more nucleotides in close proximity to or within the early coding region of the *BCL11A* gene, or (2) deletion (e.g., NHEJ-mediated deletion) of a genomic sequence including the erythroid enhancer of the *BCL11A* gene.

In an embodiment, the SCD target knockout position is altered by genome editing using the CRISPR/Cas9 system. The SCD target knockout position may be targeted by cleaving with either a single nuclease or dual nickases, e.g., to induce insertion or deletion (e.g., NHEJ-mediated insertion or deletion) of one or more nucleotides in close proximity to or within the early coding region of the SCD target knockout position or to delete (e.g., mediated by NHEJ) a genomic sequence including the erythroid enhancer of the *BCL11A* gene.

In an embodiment, the methods and compositions described herein introduce one or more breaks in close proximity to or within the early coding region in at least one allele of the *BCL11A* gene. In an embodiment, a single strand break is introduced in close proximity to or within the early coding region in at least one allele of the *BCL11A* gene. In an embodiment, the single strand break will be accompanied by an additional single strand break, positioned by a second gRNA molecule.

In an embodiment, a double strand break is introduced in close proximity to or within the early coding region in at least one allele of the *BCL11A* gene. In an embodiment, a double strand break will be accompanied by an additional single strand break positioned by a second gRNA molecule. In an embodiment, a double strand break will be accompanied by two additional single strand breaks positioned by a second gRNA molecule and a third gRNA molecule.

In an embodiment, a pair of single strand breaks is introduced in close proximity to or within the early coding region in at least one allele of the *BCL11A* gene. In an embodiment, the pair of single strand breaks will be accompanied by an additional double strand break, positioned by a third gRNA molecule. In an embodiment, the pair of single strand breaks will be accompanied by an additional pair of single strand breaks positioned by a third gRNA molecule and a fourth gRNA molecule.

In an embodiment, two double strand breaks are introduced to flank the erythroid enhancer at the in the *BCL11A* gene (one 5' and the other one 3' to the erythroid enhancer) to remove (e.g., delete) the genomic sequence including the erythroid enhancer. It is contemplated herein that in an embodiment the deletion of the genomic sequence including the erythroid enhancer is mediated by NHEJ. In an embodiment, the breaks (i.e., the two double strand breaks) are positioned to avoid unwanted deletion of certain elements, such as endogenous splice sites. The breaks, i.e., two double strand breaks, can be positioned upstream and downstream of the erythroid enhancer, as discussed herein.

In an embodiment, two sets of breaks (e.g., one double strand break and a pair of single strand breaks) are introduced to flank the erythroid enhancer in the *BCL11A* gene (one set 5' and the other set 3' to the erythroid enhancer) to remove (e.g., delete) the genomic sequence including the erythroid enhancer. It is contemplated herein that in an embodiment the deletion of the genomic sequence including the erythroid enhancer is mediated by NHEJ. In an embodiment, the breaks (i.e., the double strand break and the pair of single strand breaks) are positioned to avoid unwanted deletion of certain chromosome elements, such as endogenous splice sites. The breaks, e.g., the double strand break and the pair of single strand breaks, can be positioned upstream and downstream of the erythroid enhancer, as discussed herein.

In an embodiment, two sets of breaks (e.g., two pairs of single strand breaks) are introduced to flank the erythroid enhancer at the SCD target position in the *BCL11A* gene (one set 5' and the other set 3' to the erythroid enhancer) to remove (e.g., delete) the genomic sequence including the erythroid enhancer. It is contemplated herein that in an embodiment the deletion of the genomic sequence including the erythroid enhancer is mediated by NHEJ. In an embodiment, the breaks (i.e., the two pairs of single strand breaks) are positioned to avoid unwanted deletion of certain chromosome elements, such as endogenous splice sites. The breaks, e.g., the two pairs of single strand breaks, can be positioned upstream and downstream of the erythroid enhancer, as discussed herein.

In an embodiment, the methods and compositions discussed herein may be used to alter the *BCL11A* gene to treat or prevent SCD by knocking down one or both alleles of the *BCL11A* gene. In one embodiment, the coding region of the *BCL11A* gene, is targeted to alter the gene. In another embodiment, a non-coding region (e.g., an enhancer region, the promoter region, an intron, 5' UTR, 3'UTR, polyadenylation signal) of the *BCL11A* gene is targeted to alter the gene. In an embodiment, the promoter region of the *BCL11A* gene is targeted to knock down the expression of the *BCL11A* gene. A targeted knockdown approach alters, e.g., reduces or eliminates the expression of the *BCL11A* gene. As described herein, in an embodiment, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *BCL11A* gene.

"SCD target knockdown position", as used herein, refers to a position, e.g., in the *BCL11A* gene, which if targeted by an eiCas9 or an eiCas9 fusion described herein, results in reduction or elimination of expression of functional *BCL11A* gene product. In an embodiment, transcription is reduced or eliminated. In an embodiment, the position is in the *BCL11A* promoter sequence, In an embodiment, a position in the promoter sequence of the *BCL11A* gene is targeted by an enzymatically inactive Cas9 (eiCas9) or an eiCas9-fusion protein, as described herein.

In an embodiment, one or more gRNA molecule comprising a targeting domain configured to target an enzymatically inactive Cas9 (eiCas9) or an eiCas9 fusion protein (e.g., an eiCas9 fused to a transcription repressor domain), sufficiently close to a SCD target knockdown position to reduce, decrease or repress expression of the *BCL11A* gene.

"SCD target position", as used herein, refers to any of an SCD target point position, SCD target knockout position, or SCD target knockdown position, as described herein.

In one aspect, disclosed herein is a gRNA molecule, e.g., an isolated or non-naturally occurring gRNA molecule, comprising a targeting domain which is complementary with a target domain from the *HBB* gene or *BCL11A* gene.

When two or more gRNAs are used to position two or more cleavage events, e.g., double strand or single strand breaks, in a target nucleic acid, it is contemplated that the two or more cleavage events may be made by the same or different Cas9 proteins. For example, when two gRNAs are used to position two double strand breaks, a single Cas9 nuclease may be used to create both double strand breaks. When two or more gRNAs are used to position two or more single stranded breaks (single strand breaks), a single Cas9 nickase may be used to create the two or more single strand breaks. When two or more gRNAs are used to position at least one double strand break and at least one single strand break, two Cas9 proteins may be used, e.g., one Cas9 nuclease and one Cas9 nickase. It is contemplated that when two or more Cas9 proteins are used that the two or more Cas9 proteins may be delivered sequentially to control specificity of a double strand versus a single strand break at the desired position in the target nucleic acid.

In an embodiment, the targeting domain of the first gRNA molecule and the targeting domain of the second gRNA molecule hybridize to the target domain through complementary base pairing to opposite strands of the target nucleic acid molecule. In an embodiment, the gRNA molecule and the second gRNA molecule are configured such that the PAMs are oriented outward.

In an embodiment, the targeting domain of a gRNA molecule is configured to avoid unwanted target chromosome elements, such as repeat elements, e.g., an *Alu* repeat, or the endogenous splice sites, in the target domain. The gRNA molecule may be a first, second, third and/or fourth gRNA molecule.

In an embodiment, the targeting domain of a gRNA molecule is configured to position a cleavage event sufficiently far from a preselected nucleotide, e.g., the nucleotide of a coding region, such that the nucleotide is not altered. In an embodiment, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

In an embodiment, a point mutation in the *HBB* gene, e.g., at E6, e.g., E6V, is targeted, e.g., for correction.

In an embodiment, the SCD target point position is E6, e.g., E6V, and two gRNAs are used to position two breaks, e.g., two single stranded breaks, in the target nucleic acid sequence.

In another embodiment, a position in the coding region, e.g., the early coding region, of the *BCL11A* gene is targeted, e.g., for knockout.

In an embodiment, the SCD target knockout position is the *BCL11A* coding region, e.g., early coding region, and more than one gRNA is used to position breaks, e.g., two single stranded breaks or two double stranded breaks, or a combination of single strand and double strand breaks, e.g., to create one or more indels, in the target nucleic acid sequence.

In another embodiment, a position in the non-coding region, e.g., the enhancer region, of the *BCL11A* gene is targeted, e.g., for knockout.

In an embodiment, the targeting domain of the gRNA molecule is configured to target an enzymatically inactive Cas9 (eiCas9) or an eiCas9 fusion protein (e.g., an eiCas9 fused to a transcription repressor domain), sufficiently close to an SCD knockdown target position to reduce, decrease or repress expression of the *BCL11A* gene. In an embodiment, the targeting domain is configured to target the promoter region of the *BCL11A* gene to block transcription initiation, binding of one or more transcription enhancers or activators, and/or RNA polymerase. One or more gRNA may be used to target an eiCas9 to the promoter region of the *BCL11A* gene.

In an embodiment, the SCD target knockdown position is the *BCL11A* promoter region more than one gRNA may be used to position an eiCas9 or an eiCas9-fusion protein (e.g., an eiCas9-transcription repressor domain fusion protein), in the target nucleic acid sequence.

In an embodiment, the targeting domain which is complementary with the *BCL11A* gene is 16 nucleotides or more in length. In an embodiment, the targeting domain is 16 nucleotides in length. In an embodiment, the targeting domain is 17 nucleotides in length. In another embodiment, the targeting domain is 18 nucleotides in length. In still another embodiment, the targeting domain is 19 nucleotides in length. In still another embodiment, the targeting domain is 20 nucleotides in length. In still another embodiment, the targeting domain is 21 nucleotides in length. In still another embodiment, the targeting domain is 22 nucleotides in length. In still another embodiment, the targeting domain is 23 nucleotides in length. In still another embodiment, the targeting domain is 24 nucleotides in length. In still another embodiment, the targeting domain is 25 nucleotides in length. In still another embodiment, the targeting domain is 26 nucleotides in length.

In an embodiment, the targeting domain comprises 16 nucleotides. In an embodiment, the targeting domain comprises 17 nucleotides. In an embodiment, the targeting domain comprises 18 nucleotides. In an embodiment, the targeting domain comprises 19 nucleotides. In an embodiment, the targeting domain comprises 20 nucleotides. In an embodiment, the targeting domain comprises 21 nucleotides. In an embodiment, the targeting domain comprises 22 nucleotides. In an embodiment, the targeting domain comprises 23 nucleotides. In an embodiment, the targeting domain comprises 24 nucleotides. In an embodiment, the targeting domain comprises 25 nucleotides. In an embodiment, the targeting domain comprises 26 nucleotides.

In an embodiment, the gRNA, e.g., a gRNA comprising a targeting domain, which is complementary with the *HBB* gene or *BCL11A* gene, is a modular gRNA. In another embodiment, the gRNA is a unimolecular or chimeric gRNA.

*HBB* gRNA as described herein may comprise from 5' to 3': a targeting domain (comprising a "core domain", and optionally a "secondary domain"); a first complementarity domain; a linking domain; a second complementarity domain; a proximal domain; and a tail domain. In an embodiment, the proximal domain and tail domain are taken together as a single domain.

In an embodiment, a gRNA comprises a linking domain of no more than 25 nucleotides in length; a proximal and tail domain, that taken together, are at least 20 nucleotides in length; and a targeting domain equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 40 nucleotides in length; and a targeting domain equal to or greater than 16, 17, 18, 19, 20, 21, 22, 23, 24, 25 or 26 nucleotides in length.

A cleavage event, e.g., a double strand or single strand break, is generated by a Cas9 molecule. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule). Alternatively, in an embodiment, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

In an embodiment, the eaCas9 molecule catalyzes a double strand break.

In an embodiment, the eaCas9 molecule comprises HNH-like domain cleavage activity but has no, or no significant, N-terminal RuvC-like domain cleavage activity. In this case, the eaCas9 molecule is an HNH-like domain nickase, e.g., the eaCas9 molecule comprises a mutation at D10, e.g., D10A. In another embodiment, the eaCas9 molecule comprises N-terminal RuvC-like domain cleavage activity but has no, or no significant, HNH-like domain cleavage activity. In an embodiment, the eaCas9 molecule is an N-terminal RuvC-like domain nickase, e.g., the eaCas9 molecule comprises a mutation at H840, e.g., H840A. In an embodiment, the eaCas9 molecule is an N-terminal RuvC-like domain nickase, e.g., the eaCas9 molecule comprises a mutation at N863, e.g., N863A.

In an embodiment, a single strand break is formed in the strand of the target nucleic acid to which the targeting domain of said gRNA is complementary. In another embodiment, a single strand break is formed in the strand of the target nucleic acid other than the strand to which the targeting domain of said gRNA is complementary.

In an embodiment, a mutation in the *HBB* gene is corrected, e.g., by HDR, using an exogenously provided template nucleic acid.

In an embodiment, the template nucleic acid is a single stranded nucleic acid. In another embodiment, the template nucleic acid is a double stranded nucleic acid. In an embodiment, the template nucleic acid comprises a nucleotide sequence, e.g., of one or more nucleotides, that will be added to or will template a change in the target nucleic acid. In another embodiment, the template nucleic acid comprises a nucleotide sequence that may be used to modify the target position. In another embodiment, the template nucleic acid comprises a nucleotide sequence, e.g., of one or more nucleotides, that corresponds to wild type sequence of the target nucleic acid, e.g., of the target position.

The template nucleic acid may comprise a replacement sequence, e.g., a replacement sequence from the **Table 650.** In an embodiment, the template nucleic acid comprises a 5' homology arm, e.g., a 5' homology arm from **Table 650.** In another embodiment, the template nucleic acid comprises a 3' homology arm, e.g., a 3' homology arm from **Table 650.**

In another embodiment, a mutation in the *HBB* gene is corrected, e.g., by HDR, without using an exogenously provided template nucleic acid. While not wishing to be bound by theory, it is believed that an endogenous region of homology can mediate HDR-based correction. In an embodiment, alteration of the target sequence occurs by HDR with an endogenous genomic donor sequence. In an embodiment, the endogenous genomic donor sequence is located on the same chromosome as the target sequence. In another embodiment, the endogenous genomic donor sequence is located on a different chromosome from the target sequence. In an embodiment, the endogenous genomic donor sequence comprises one or more nucleotides derived from the *HBB* gene. Mutations in the *HBB* gene that can be corrected (e.g., altered) by HDR with an endogenous genomic donor sequence include, e.g., a point mutation at E6, e.g., E6V.

In another aspect, disclosed herein is a composition comprising (a) a gRNA molecule comprising a targeting domain that is complementary with a target domain in the *HBB* gene or *BCL11A* gene, as described herein. The composition of (a) may further comprise (b) a Cas9 molecule, e.g., a Cas9 molecule as described herein. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule). Alternatively, in some embodiments, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

A composition of (a) and (b) may further comprise (c) a second, third and/or fourth gRNA molecule, e.g., a second, third and/or fourth gRNA molecule described herein. A composition of (a), (b) and (c) may further comprise (d) a template nucleic acid (in an embodiment where an exogenous template is used).

In another aspect, disclosed herein is a method of altering a cell, e.g., altering the structure, e.g., altering the sequence, of a target nucleic acid of a cell, comprising contacting said cell with: (a) a gRNA that targets the *HBB* gene or *BCL11A* gene, e.g., a gRNA as described herein; (b) a Cas9 molecule, e.g., a Cas9 molecule as described herein; and optionally, (c) a second, third and/or fourth gRNA that targets *HBB* gene or *BCL11A* gene, e.g., a gRNA; and optionally, (d) a template nucleic acid, as described herein.

In an embodiment, the method comprises contacting said cell with (a) and (b).

In an embodiment, the method comprises contacting said cell with (a), (b), and (c).

In an embodiment, the method comprises contacting said cell with (a), (b), (c) and (d).

In an embodiment, the gRNA targets the *HBB* gene and no exogenous template nucleic acid is contacted with the cell.

In an embodiment, the method comprises contacting a cell from a subject suffering from or likely to develop SCD. The cell may be from a subject having a mutation at an SCD target position in the *HBB* gene or a subject which would benefit from having a mutation at an SCD target position in the *BCL11A* gene.

In an embodiment, the cell being contacted in the disclosed method is an erythroid cell. The contacting may be performed *ex vivo* and the contacted cell may be returned to the subject's body after the contacting step.

In an embodiment, the method of altering a cell as described herein comprises acquiring knowledge of the sequence at an SCD target position in said cell, prior to the contacting step. Acquiring knowledge of the sequence at an SCD target position in the cell may be by sequencing the *HBB* gene or *BCL11A* gene, or a portion of the *HBB* gene or *BCL11A* gene.

In an embodiment, contacting comprises delivering to the cell a Cas9 molecule of (b), as a protein or an mRNA, said gRNA of (a), as an RNA, and optionally said second gRNA of (c), as an RNA.

In an embodiment, contacting comprises delivering to the cell a gRNA of (a) as an RNA, optionally said second gRNA of (c) as an RNA, and a nucleic acid that encodes the Cas9 molecule of (b).

In another aspect, disclosed herein is a method of treating or preventing a subject suffering from or likely to develop SCD, e.g., altering the structure, e.g., sequence, of a target nucleic acid of the subject, comprising contacting a cell from the subject with:
(a) a gRNA that targets the *HBB* gene or *BCL11A* gene, e.g., a gRNA disclosed herein;
(b) a Cas9 molecule, e.g., a Cas9 molecule disclosed herein; and
optionally, (c)(i) a second gRNA that targets the *HBB* gene or *BCL11A* gene, e.g., a second gRNA disclosed herein, and
further optionally, (c)(ii) a third gRNA, and still further optionally, (c)(iii) a fourth gRNA that target the *HBB* gene or *BCL11A* gene, e.g., a third and fourth gRNA disclosed herein.

The method of treating a subject may further comprise contacting a cell from the subject with (d) a template nucleic acid (in an embodiment where an exogenous template is used). In an embodiment, a template nucleic acid is used when the method of treating a subject uses HDR to alter the sequence of the target nucleic acid of the subject. In an embodiment, the gRNA targets the *HBB* gene and no exogenous template nucleic acid is contacted with a cell from the subject.

In an embodiment, contacting comprises contacting with (a) and (b).

In an embodiment, contacting comprises contacting with (a), (b), and (c)(i).

In an embodiment, contacting comprises contacting with (a), (b), (c)(i) and (c)(ii).

In an embodiment, contacting comprises contacting with (a), (b), (c)(i), (c)(ii) and (c)(iii).

In an embodiment, contacting comprises contacting with (a), (b), (c)(i) and (d).

In an embodiment, contacting comprises contacting with (a), (b), (c)(i), (c)(ii) and (d).

In an embodiment, contacting comprises contacting with (a), (b), (c)(i), (c)(ii), (c)(iii) and (d).

In an embodiment, the method comprises acquiring knowledge of the sequence (e.g., a mutation) of an SCD target position in said subject.

In an embodiment, the method comprises acquiring knowledge of the sequence (e.g., a mutation) of an SCD target position in said subject by sequencing the *HBB* gene or *BCL11A* gene or a portion of the *HBB* gene or *BCL11A* gene.

In an embodiment, the method comprises correcting a mutation at an SCD target position in the *HBB* gene.

In an embodiment, the method comprises correcting a mutation at an SCD target position in the *HBB* gene by HDR.

In an embodiment, the method comprises introducing a mutation at an SCD target position in the *BCL11A* gene.

In an embodiment, the method comprises introducing a mutation at an SCD target position in the *BCL11A* gene by NHEJ.

When the method comprises correcting the mutation at an SCD target position by HDR, a Cas9 of (b), at least one guide RNA, e.g., a guide RNA of (a) and a template nucleic acid (d) are included in the contacting step.

In an embodiment, a cell of the subject is contacted *ex vivo* with (a), (b), (d) and optionally (c). In an embodiment, said cell is returned to the subject's body.

In an embodiment, contacting comprises delivering to said subject said Cas9 molecule of (b), as a protein or mRNA, and a nucleic acid which encodes (a), a nucleic acid of (d) and optionally (c).

In an embodiment, contacting comprises delivering to the subject the Cas9 molecule of (b), as a protein or mRNA, the gRNA of (a), as an RNA, a nucleic acid of (d) and optionally the second gRNA of (c), as an RNA.

In an embodiment, contacting comprises delivering to the subject the gRNA of (a), as an RNA, optionally said second gRNA of (c), as an RNA, a nucleic acid that encodes the Cas9 molecule of (b), and a nucleic acid of (d).

When the method comprises (1) introducing a mutation at an SCD target position by NHEJ or (2) knocking down expression of the *BCL11A* gene by targeting the promoter region, a Cas9 of (b) and at least one guide RNA, e.g., a guide RNA of (a) are included in the contacting step.

In an embodiment, a cell of the subject is contacted *ex vivo* with (a), (b) and optionally (c). In an embodiment, said cell is returned to the subject's body.

In an embodiment, a populations of cells from a subject is contacted *ex vivo* with (a), (b) and optionally (c) to correct the E6V mutation in the *HBB* gene and a second population of cells from the subject is contacted *ex vivo* with (a), (b) and optionally (c) to introduce a mutation in the *BCL11A* gene to knockout the *BCL11A* gene. A mixture of the two cell populations may be returned to the subject's body to treat or prevent SCD.

In an embodiment, contacting comprises delivering to said subject said Cas9 molecule of (b), as a protein or mRNA, and a nucleic acid which encodes (a) and optionally (c).

In an embodiment, contacting comprises delivering to the subject the Cas9 molecule of (b), as a protein or mRNA, the gRNA of (a), as an RNA, and optionally the second gRNA of (c), as an RNA.

In an embodiment, contacting comprises delivering to the subject the gRNA of (a), as an RNA, optionally said second gRNA of (c), as an RNA, and a nucleic acid that encodes the Cas9 molecule of (b).

In another aspect, disclosed herein is a reaction mixture comprising a gRNA, a nucleic acid, or a composition described herein, and a cell, e.g., a cell from a subject having, or likely to develop SCD, or a subject having a mutation at an SCD target position in the *HBB* gene, or a cell from a subject which would benefit from having a mutation at an SCD target position in the *BCL11A* gene.

In another aspect, disclosed herein is a kit comprising, (a) gRNA molecule described herein, and one or more of the following:
(b) a Cas9 molecule, e.g., a Cas9 molecule described herein, or a nucleic acid or mRNA that encodes the Cas9;
(c)(i) a second gRNA molecule, e.g., a second gRNA molecule described herein;
(c)(ii) a third gRNA molecule, e.g., a second gRNA molecule described herein;
(c)(iii) a fourth gRNA molecule, e.g., a second gRNA molecule described herein;
(d) a template nucleic acid (in an embodiment where an exogenous template is used).

The compositions, reaction mixtures and kits, as disclosed herein, can also include a governing gRNA molecule, e.g., a governing gRNA molecule disclosed herein.

### VI.4 Circulating blood cells (e.g., targeting BCL11A gene for treating BT)

In another aspect, the target cell is a circulating blood cell, e.g., a reticulocyte, a myeloid progenitor cell, or a hematopoietic stem cell. In an embodiment, the target cell is a bone marrow cell (e.g., a myeloid progenitor cell, an erythroid progenitor cell, a hematopoietic stem cell, or a mesenchymal stem cell). In an embodiment, the target cell is a myeloid progenitor cell (e.g., a common myeloid progenitor (CMP) cell). In an embodiment, the target cell is an erythroid progenitor cell (e.g., a megakaryocyte erythroid progenitor (MEP) cell). In an embodiment, the target cell is a hematopoietic stem cell (e.g., a long term hematopoietic stem cell (LT-HSC), a short term hematopoietic stem cell (ST-HSC), a multipotent progenitor (MPP) cell, a lineage restricted progenitor (LRP) cell).

In an embodiment, the target cell is manipulated *ex vivo* by editing (e.g., inducing a mutation in) the *BCL11A* target gene and/or modulating the expression of the *BCL11A* target gene, and administered to the subject. Sources of target cells for *ex vivo* manipulation may include, by way of example, the subject's blood, the subject's cord blood, or the subject's bone marrow. Sources of target cells for *ex vivo* manipulation may also include, by way of example, heterologous donor blood, cord blood, or bone marrow.

In an embodiment, a myeloid progenitor cell is removed from the subject, manipulated *ex vivo* as described above, and the myeloid progenitor cell is returned to the subject. In an embodiment, an erythroid progenitor cell is removed from the subject, manipulated *ex vivo* as described above, and the erythroid progenitor cell is returned to the subject. In an embodiment, a hematopoietic stem cell is removed from the subject, manipulated *ex vivo* as described above, and the hematopoietic stem cell is returned to the subject. In an embodiment, a CD34+ hematopoietic stem cell is removed from the subject, manipulated *ex vivo* as described above, and the CD34+ hematopoietic stem cell is returned to the subject.

A suitable cell can also include a stem cell such as, by way of example, an embryonic stem cell, an induced pluripotent stem cell, a hematopoietic stem cell, a neuronal stem cell and a mesenchymal stem cell. In an embodiment, the cell is an induced pluripotent stem (iPS) cell or a cell derived from an iPS cell, e.g., an iPS cell generated from the subject, modified to induce a mutation and differentiated into a clinically relevant cell such as a myeloid progenitor cell, an erythroid progenitor cell or a hematopoietic stem cell. In an embodiment, AAV is used to transduce the target cells, e.g., the target cells described herein.

Cells produced by the methods described herein may be used immediately. Alternatively, the cells may be frozen (e.g., in liquid nitrogen) and stored for later use. The cells will usually be frozen in 10% dimehtylsulfoxide (DMSO), 50% serum, 40% buffered medium, or some other such solution as is commonly used in the art to preserve cells at such freezing temperature and thawed in such a manner as commonly known in the art for thawing frozen cultured cells.

### Methods to Treat or Prevent Beta-thalassemia (BT)

Disclosed herein are approaches to treat or prevent BT, including beta-thalassemia major (BTM) and BT intermedia, using the compositions and methods described herein.

In one approach, the *BCL11A* gene is targeted as a targeted knockout or knockdown, e.g., to increase expression of fetal hemoglobin.

While not wishing to be bound by theory, it is considered that increasing levels of fetal hemoglobin (HbF) in subjects with BT may ameliorate disease. Fetal hemoglobin can replace beta hemoglobin in the hemoglobin complex, form adequate tetramers with alpha hemoglobin, and effectively carry oxygen to tissues. Subjects with beta-thalassemia who express higher levels of fetal hemoglobin have been found to have a less severe phenotype. Hydroxyurea, often used in the treatment of beta-thalassemia, may exert its mechanism of action via increasing levels of HbF production.

In an embodiment, knockout or knockdown of the *BCL11A* gene increases fetal hemoglobin levels in beta-thalassemia subjects and improves phenotype and/or reduces or prevents disease progression. BCL11A is a zinc-finger repressor that is involved in the regulation of fetal hemoglobin and acts to repress the synthesis of fetal hemoglobin. Knockout of the *BCL11A* gene in erythroid cells induces increased fetal hemoglobin (HbF) synthesis and increased HbF can result in more effective oxygen carrying capacity in subjects with beta-thalassemia (HbF will form tetramers with hemoglobin alpha).

In an embodiment, the *BCL11A* knockout or knockdown is targeted specifically to cells of the erythroid lineage. *BCL11A* knockout in erythroid cells has been found in *in vitro* studies to have no effect on erythroid growth, maturation and function. In an embodiment, erythroid cells are preferentially targeted, e.g., at least 90%, 95%, 96%, 97%, 98%, 99%, or 100% of the targeted cells are erythroid cells. For example, if cells are treated *ex vivo* and returned to the subject, erythroid cells are preferentially modified.

In an embodiment, the methods described herein result in increased fetal hemoglobin synthesis in beta thalassemia subjects, thereby improving disease phenotype in subjects with BT. For example, subjects with beta thalassemia major will suffer from less severe anemia and will need fewer blood transfusions. They will therefore have fewer complications arising from transfusions and chelation therapy. In an embodiment, the method described herein increases fetal hemoglobin synthesis and improves the oxygen carrying capacity of erythroid cells. For example, subjects are expected to demonstrate decreased rates of extramedullary erythropoiesis and decreased erythroid hypertrophy within the bone marrow. In an embodiment, the method described herein results in reduction of bone fractures, bone abnormalities, splenomegaly, and thrombosis.

Knockdown or knockout of one or both *BCL11A* alleles may be performed prior to disease onset or after disease onset, but preferably early in the disease course.

In an embodiment, the method comprises initiating treatment of a subject prior to disease onset.

In an embodiment, the method comprises initiating treatment of a subject after disease onset.

In an embodiment, the method comprises initiating treatment of a subject well after disease onset, e.g., 1, 2, 3, 4, 5, 6, 7, 8, 9, 10, 12, 24, or 36 months after onset of BT, e.g., BTM. While not wishing to be bound by theory it is believed that this treatment may be effective if subjects present well into the course of illness.

In an embodiment, the method comprises initiating treatment of a subject in an advanced stage of disease.

Overall, initiation of treatment for subjects at all stages of disease is expected to prevent negative consequences of disease and be of benefit to subjects.

In an embodiment, the method comprises initiating treatment of a subject prior to disease expression. In an embodiment, the method comprises initiating treatment of a subject in an early stage of disease, e.g., when a subject has tested positive for beta-thalassemia mutations but has no signs or symptoms associated with beta-thalassemia major, minor or intermedia.

In an embodiment, the method comprises initiating treatment of a subject at the appearance of microcytic anemia, e.g., in an infant, child, adult or young adult.

In an embodiment, the method comprises initiating treatment of a subject who is transfusion-dependent.

In an embodiment, the method comprises initiating treatment of a subject who has tested positive for a mutation in a beta globin gene.

In an embodiment, the method comprises initiating treatment at the appearance of any one or more of the following findings consistent with beta-thalassemia major or beta-thalassemia minor: anemia, diarrhea, fever, failure to thrive, frontal bossing, broken long bones, hepatomegaly, splenomegaly, thrombosis, pulmonary embolus, stroke, leg ulcer, cardiomyopathy, cardiac arrhythmia, and evidence of extramedullary erythropoiesis.

In an embodiment, a cell is treated, e.g., *ex vivo.* In an embodiment, an *ex vivo* treated cell is returned to a subject.

In an embodiment, allogenic or autologous bone marrow or erythroid cells are treated *ex vivo.* In an embodiment, an ex vivo treated allogenic or autologous bone marrow or erythroid cells are administered to the subject. In an embodiment, an erythroid cell, e.g., an autologous erythroid cell, is treated *ex vivo* and returned to the subject. In an embodiment, an autologous stem cell, is treated *ex vivo* and returned to the subject. In an embodiment, the modified HSCs are administered to the patient following no myeloablative pre-conditioning. In an embodiment, the modified HSCs are administered to the patient following mild myeloablative pre-conditioning such that following engraftment, some of the hematopoietic cells are devied from the modified HSCs. In other aspects, the HSCs are administered after full myeloablation such that following engraftment, 100% of the hematopoietic cells are derived from the modified HSCs.

### Methods of Altering BCL11A

One approach to increase the expression of HbF involves identification of genes whose products play a role in the regulation of globin gene expression. One such gene is *BCL11A.* It plays a role in the regulation of γ globin expression. It was first identified because of its role in lymphocyte development. *BCL11A* encodes a zinc finger protein that is thought to be involved in the stage specific regulation of γ globin expression. BCL11A is expressed in adult erythroid precursor cells and down-regulation of its expression leads to an increase in γ globin expression. In addition, it appears that the splicing of the BCL11A mRNA is developmentally regulated. In embryonic cells, it appears that the shorter BCL11A mRNA variants, known as BCL11A-S and BCL11A-XS are primary expressed, while in adult cells, the longer BCL11A-L and BCL11A-XL mRNA variants are predominantly expressed. See, Sankaran et al (2008) Science 322 p. 1839. The BCL11A protein appears to interact with the β globin locus to alter its conformation and thus its expression at different developmental stages. Thus, if BCL11A expression is altered e.g., disrupted (e.g., reduced or eliminated), it results in the elevation of γ globin and HbF production.

Disclosed herein are methods for altering the BT target position in the *BCL11A* gene. Altering the BT target position is achieved, e.g., by:
(1) knocking out the *BCL11A* gene:
   (a) insertion or deletion (e.g., NHEJ-mediated insertion or deletion) of one or more nucleotides in close proximity to or within the early coding region of the *BCL11A* gene, or
   (b) deletion (e.g., NHEJ-mediated deletion) of genomic sequence including the erythroid enhancer of the *BCL11A* gene, or
(2) knocking down the BCL11A gene mediated by enzymatically inactive Cas9 (eiCas9) or an eiCas9-fusion protein by targeting the promoter region of the gene.

All approaches give rise to alteration of the BCL11A gene.

In one embodiment, methods described herein introduce one or more breaks near the early coding region in at least one allele of the *BCL11A* gene. In another embodiment, methods described herein introduce two or more breaks to flank the erythroid enhancer of BT target knockout position. The two or more breaks remove (e.g., delete) genomic sequence including the erythorid enhancer. In another embodiment, methods described herein comprises knocking down the *BCL11A* gene mediated by enzymatically inactive Cas9 (eiCas9) or an eiCas9-fusion protein by targeting the promoter region of BT target knockdown position. All methods described herein result in alteration of the BCL11A gene.

### NHEJ-mediated introduction of an indel in close proximity to or within the early coding region of the BT knockout position

In an embodiment, the method comprises introudicing a NHEJ-mediated insertion or deletion of one more nucleotides in close proximity to the BT target knockout position (e.g., the early coding region) of the *BCL11A* gene. As described herein, in one embodiment, the method comprises the introduction of one or more breaks (e.g., single strand breaks or double strand breaks) sufficiently close to (e.g., either 5' or 3' to) the early coding region of the BT target knockout position, such that the break-induced indel could be reasonably expected to span the BT target knockout position (e.g., the early coding region). While not wishing to be bound by theory, it is believed that NHEJ-mediated repair of the break(s) allows for the NHEJ-mediated introduction of an indel in close proximity to within the early coding region of the BT target knockout position.

In an embodiment, the targeting domain of the gRNA molecule is configured to provide a cleavage event, e.g., a double strand break or a single strand break, sufficiently close to the early coding region in the *BCL11A* gene to allow alteration, e.g., alteration associated with NHEJ in the *BCL11A* gene. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of a BT target knockout position. The break, e.g., a double strand or single strand break, can be positioned upstream or downstream of a BT target knockout position in the *BCL11A* gene.

In an embodiment, a second gRNA molecule comprising a second targeting domain is configured to provide a cleavage event, e.g., a double strand break or a single strand break, sufficiently close to the early coding region in the *BCL11A* gene, to allow alteration, e.g., alteration associated with NHEJ in the *BCL11A* gene, either alone or in combination with the break positioned by said first gRNA molecule. In an embodiment, the targeting domains of the first and second gRNA molecules are configured such that a cleavage event, e.g., a double strand or single strand break, is positioned, independently for each of the gRNA molecules, within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the breaks, e.g., double strand or single strand breaks, are positioned on both sides of a nucleotide of a BT target knockout position in the *BCL11A* gene. In an embodiment, the breaks, e.g., double strand or single strand breaks, are positioned on one side, e.g., upstream or downstream, of a nucleotide of a BT target knockout position in the *BCL11A* gene.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the early coding region in the *BCL11A* gene. In an embodiment, the first and second gRNA molecules are configured such, that when guiding a Cas9 nickase, a single strand break will be accompanied by an additional single strand break, positioned by a second gRNA, sufficiently close to one another to result in alteration of the early coding region in the *BCL11A* gene in the *BCL11A* gene. In an embodiment, the first and second gRNA molecules are configured such that a single strand break positioned by said second gRNA is within 10, 20, 30, 40, or 50 nucleotides of the break positioned by said first gRNA molecule, e.g., when the Cas9 is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream of the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domain of a second gRNA molecule is configured such that a double strand break is positioned downstream of the early coding region in the *BCL11A* gene in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream of the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domains of a second and third gRNA molecule are configured such that two single strand breaks are positioned downstream of the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the targeting domain of the first, second and third gRNA molecules are configured such that a cleavage event, e.g., a double strand or single strand break, is positioned, independently for each of the gRNA molecules.

In an embodiment, a first and second single strand breaks can be accompanied by two additional single strand breaks positioned by a third gRNA molecule and a fourth gRNA molecule. For example, the targeting domain of a first and second gRNA molecule are configured such that two single strand breaks are positioned upstream of the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the early coding region in the *BCL11A* gene; and the targeting domains of a third and fourth gRNA molecule are configured such that two single strand breaks are positioned downstream of a BT target knockout position in the *BCL11A* gene the early coding region in the *BCL11A* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the early coding region in the *BCL11A* gene.

### NHEJ-mediated deletion of the erythroid enhancer at the BT target position

In an embodiment, the method comprises introducing a NHEJ-mediated deletion of a genomic sequence including the erythroid enhancer. As described herein, in one embodiment, the method comprises the introduction of two double strand breaks-one 5' and the other 3' to (i.e., flanking) the BT target position (e.g., the erythroid enhancer). Two gRNAs, e.g., unimolecular (or chimeric) or modular gRNA molecules, are configured to position the two double strand breaks on opposite sides of the BT target knockdown position (e.g., the erythroid enhancer) in the *BCL11A* gene. In an embodiment, the first double strand break is positioned upstream of the the erythroid enhancer within intron 2 (e.g., between TSS+0.75kb to TSS+52.0kb), and the second double strand break is positioned downstream of the the erythroid enhancer within intron 2 (e.g., between TSS+64.4kb to TSS+84.7kb) (see **Fig. 15**). In an embodiment, the two double strand breaks are positioned to remove a portion of the erythroid enhancer resulting in disruption of one or more DHSs. In an embodiment, the breaks (i.e., the two double strand breaks) are positioned to avoid unwanted target chromosome elements, such as repeat elements, e.g., an *Alu* repeat, or the endogenous splice sites.

The first double strand break may be positioned as follows:
(1) upstream of the 5' end of the erythroid enhancer in intron 2 (e.g., between TSS+0.75kb to TSS+52.0kb), or
(2) within the erythroid enhancer provided that a portion of the erythroid enhancer is removed resulting in disruption of one or more DHSs (e.g., between TSS+52.0kb to TSS+64.4kb),
and the second double strand break to be paired with the first double strand break may be positioned as follows:
(3) downstream the 3' end of the erythroid enhancer in intron 2 (e.g., between TSS+64.4kb to TSS+84.7kb), or
(4) within the erythroid enhancer provided that a portion of the erythroid enhancer is removed resulting in disruption of one or more DHSs (e.g., between TSS+52.0kb to TSS+64.4kb).

For example, the first double strand break may be positioned in the BCL11A gene:
(1) between TSS+0.75kb to TSS+10kb,
(2) between TSS+10kb to TSS+20kb,
(3) between TSS+20kb to TSS+30kb,
(4) between TSS+30kb to TSS+40kb,
(5) between TSS+40kb to TSS+45kb,
(6) between TSS+45kb to TSS+47.5kb,
(7) between TSS+47.5kb to TSS+50kb,
(8) between TSS+50kb to TSS+51kb,
(9) between TSS+51kb to TSS+51.1kb,
(10) between TSS+51.1kb to TSS+51.2kb,
(11) between TSS+51.2kb to TSS+51.3kb,
(12) between TSS+51.3kb to TSS+51.4kb,
(13) between TSS+51.4kb to TSS+51.5kb,
(14) between TSS+51.5kb to TSS+51.6kb,
(15) between TSS+51.6kb to TSS+51.7kb,
(16) between TSS+51.7kb to TSS+51.8kb,
(17) between TSS+51.8kb to TSS+51.9kb,
(18) between TSS+51.9kb to TSS+52kb,
(19) between TSS+52kb to TSS+53kb,
(20) between TSS+53kb to TSS+54kb,
(21) between TSS+54kb to TSS+55kb,
(22) between TSS+55kb to TSS+56kb,
(23) between TSS+56kb to TSS+57kb,
(24) between TSS+57kb to TSS+58kb,
(25) between TSS+58kb to TSS+59kb,
(26) between TSS+59kb to TSS+60kb,
(27) between TSS+60kb to TSS+61kb,
(28) between TSS+61kb to TSS+62kb,
(29) between TSS+62kb to TSS+63kb,
(30) between TSS+63kb to TSS+64kb, or
(31) between TSS+64kb to TSS+64.4kb,
and the second double strand break to be paired with the first double strand break may be positioned in the BCL11A gene:
(1) between TSS+52kb to TSS+53kb,
(2) between TSS+53kb to TSS+54kb,
(3) between TSS+54kb to TSS+55kb,
(4) between TSS+55kb to TSS+56kb,
(5) between TSS+56kb to TSS+57kb,
(6) between TSS+57kb to TSS+58kb,
(7) between TSS+58kb to TSS+59kb,
(8) between TSS+59kb to TSS+60kb,
(9) between TSS+60kb to TSS+61kb,
(10) between TSS+61kb to TSS+62kb,
(11) between TSS+62kb to TSS+63kb,
(12) between TSS+63kb to TSS+64kb,
(13) between TSS+64kb to TSS+64.4kb,
(14) between TSS+64.4kb to TSS+65kb,
(15) between TSS+65kb to TSS+65.1kb,
(16) between TSS+65.1kb to TSS+65.2kb,
(17) between TSS+65.2kb to TSS+65.3kb,
(18) between TSS+65.3kb to TSS+65.4kb,
(19) between TSS+65.4kb to TSS+65.5kb,
(20) between TSS+65.5kb to TSS+65.7kb,
(21) between TSS+65.7kb to TSS+65.8kb,
(22) between TSS+65.8kb to TSS+65.9kb,
(23) between TSS+65.9kb to TSS+66kb,
(24) between TSS+66kb to TSS+67kb,
(25) between TSS+67kb to TSS+68kb,
(26) between TSS+68kb to TSS+69kb,
(27) between TSS+69kb to TSS+70kb,
(28) between TSS+70kb to TSS+75kb,
(29) between TSS+75kb to TSS+80kb, or
(30) between TSS+80kb to TSS+84.4kb.

While not wishing to be bound by theory, it is believed that the two double strand breaks allow for NHEJ-mediated deletion of erythroid enhancer in the BCL11A gene.

In an embodiment, the method comprises introducing a NHEJ-mediated deletion of a genomic sequence including the erythroid enhancer. As described herein, in one embodiment, the method comprises the introduction of two sets of breaks (e.g., one double strand break and a pair of single strand breaks)-one 5' and the other 3' to (i.e., flanking) the BT target position (e.g., the erythroid enhancer). Two gRNAs, e.g., unimolecular (or chimeric) or modular gRNA molecules, are configured to position the two sets of breaks (either the double strand break or the pair of single strand breaks) on opposite sides of the BT target knockdown position (e.g., the erythroid enhancer) in the *BCL11A* gene. In an embodiment, the first set of breaks (either the double strand break or the pair of single strand breaks) is positioned upstream of the the erythroid enhancer within intron 2 (e.g., between TSS+0.75kb to TSS+52.0kb), and the second set of breaks (either the double strand break or the pair of single strand breaks) is positioned downstream of the the erythroid enhancer within intron 2 (e.g., between TSS+64.4kb to TSS+84.7kb) (see **Fig. 15**). In an embodiment, the two sets of breaks (either the double strand break or the pair of single strand breaks) are positioned to remove a portion of the erythroid enhancer resulting in disruption of one or more DHSs. In an embodiment, the breaks (i.e., the two sets of breaks (either the double strand break or the pair of single strand breaks)) are positioned to avoid unwanted target chromosome elements, such as repeat elements, e.g., an *Alu* repeat, or the endogenous splice sites.

The first set of breaks (either the double strand break or the pair of single strand breaks) may be positioned as follows:
(1) upstream of the 5' end of the erythroid enhancer in intron 2 (e.g., between TSS+0.75kb to TSS+52.0kb), or
(2) within the erythroid enhancer provided that a portion of the erythroid enhancer is removed resulting in disruption of one or more DHSs (e.g., between TSS+52.0kb to TSS+64.4kb),
and the second set of breaks (either the double strand break or the pair of single strand breaks) to be paired with the first set of breaks (either the double strand break or the pair of single strand breaks) may be positioned as follows:
(3) downstream the 3' end of the erythroid enhancer in intron 2 (e.g., between TSS+64.4kb to TSS+84.7kb), or
(4) within the erythroid enhancer provided that a portion of the erythroid enhancer is removed resulting in disruption of one or more DHSs (e.g., between TSS+52.0kb to TSS+64.4kb).

For example, the first set of breaks (either the double strand break or the pair of single strand breaks) may be positioned in the BCL11A gene:
(1) between TSS+0.75kb to TSS+10kb,
(2) between TSS+10kb to TSS+20kb,
(3) between TSS+20kb to TSS+30kb,
(4) between TSS+30kb to TSS+40kb,
(5) between TSS+40kb to TSS+45kb,
(6) between TSS+45kb to TSS+47.5kb,
(7) between TSS+47.5kb to TSS+50kb,
(8) between TSS+50kb to TSS+51kb,
(9) between TSS+51kb to TSS+51.1kb,
(10) between TSS+51.1kb to TSS+51.2kb,
(11) between TSS+51.2kb to TSS+51.3kb,
(12) between TSS+51.3kb to TSS+51.4kb,
(13) between TSS+51.4kb to TSS+51.5kb,
(14) between TSS+51.5kb to TSS+51.6kb,
(15) between TSS+51.6kb to TSS+51.7kb,
(16) between TSS+51.7kb to TSS+51.8kb,
(17) between TSS+51.8kb to TSS+51.9kb,
(18) between TSS+51.9kb to TSS+52kb,
(19) between TSS+52kb to TSS+53kb,
(20) between TSS+53kb to TSS+54kb,
(21) between TSS+54kb to TSS+55kb,
(22) between TSS+55kb to TSS+56kb,
(23) between TSS+56kb to TSS+57kb,
(24) between TSS+57kb to TSS+58kb,
(25) between TSS+58kb to TSS+59kb,
(26) between TSS+59kb to TSS+60kb,
(27) between TSS+60kb to TSS+61kb,
(28) between TSS+61kb to TSS+62kb,
(29) between TSS+62kb to TSS+63kb,
(30) between TSS+63kb to TSS+64kb, or
(31) between TSS+64kb to TSS+64.4kb,
and the second set of breaks (either the double strand break or the pair of single strand breaks) to be paired with the first set of breaks (either the double strand break or the pair of single strand breaks)may be positioned in the BCL11A gene:
(1) between TSS+52kb to TSS+53kb,
(2) between TSS+53kb to TSS+54kb,
(3) between TSS+54kb to TSS+55kb,
(4) between TSS+55kb to TSS+56kb,
(5) between TSS+56kb to TSS+57kb,
(6) between TSS+57kb to TSS+58kb,
(7) between TSS+58kb to TSS+59kb,
(8) between TSS+59kb to TSS+60kb,
(9) between TSS+60kb to TSS+61kb,
(10) between TSS+61kb to TSS+62kb,
(11) between TSS+62kb to TSS+63kb,
(12) between TSS+63kb to TSS+64kb,
(13) between TSS+64kb to TSS+64.4kb,
(14) between TSS+64.4kb to TSS+65kb,
(15) between TSS+65kb to TSS+65.1kb,
(16) between TSS+65.1kb to TSS+65.2kb,
(17) between TSS+65.2kb to TSS+65.3kb,
(18) between TSS+65.3kb to TSS+65.4kb,
(19) between TSS+65.4kb to TSS+65.5kb,
(20) between TSS+65.5kb to TSS+65.7kb,
(21) between TSS+65.7kb to TSS+65.8kb,
(22) between TSS+65.8kb to TSS+65.9kb,
(23) between TSS+65.9kb to TSS+66kb,
(24) between TSS+66kb to TSS+67kb,
(25) between TSS+67kb to TSS+68kb,
(26) between TSS+68kb to TSS+69kb,
(27) between TSS+69kb to TSS+70kb,
(28) between TSS+70kb to TSS+75kb,
(29) between TSS+75kb to TSS+80kb, or
(30) between TSS+80kb to TSS+84.4kb.

While not wishing to be bound by theory, it is believed that the two sets of breaks (either the double strand break or the pair of single strand breaks) allow for NHEJ-mediated deletion of erythroid enhancer in the BCL11A gene.

In an embodiment, the method comprises introducing a NHEJ-mediated deletion of a genomic sequence including the erythroid enhancer. As described herein, in one embodiment, the method comprises the introduction of two sets of breaks (e.g., two pairs of single strand breaks)-one 5' and the other 3' to (i.e., flanking) the BT target position (e.g., the erythroid enhancer). Two gRNAs, e.g., unimolecular (or chimeric) or modular gRNA molecules, are configured to position the two sets of breaks on opposite sides of the BT target knockdown position (e.g., the erythroid enhancer) in the *BCL11A* gene. In an embodiment, the first set of breaks (i.e., the first pair of single strand breaks) is positioned upstream of the the erythroid enhancer within intron 2 (e.g., between TSS+0.75kb to TSS+52.0kb), and the second set of breaks (i.e., the second pair of single strand breaks) is positioned downstream of the the erythroid enhancer within intron 2 (e.g., between TSS+64.4kb to TSS+84.7kb) (see **Fig. 15**). In an embodiment, the two sets of breaks (e.g., two pairs of single strand breaks)) are positioned to remove a portion of the erythroid enhancer resulting in disruption of one or more DHSs. In an embodiment, the breaks (i.e., the two pairs of single strand breaks) are positioned to avoid unwanted target chromosome elements, such as repeat elements, e.g., an *Alu* repeat, or the endogenous splice sites.

The first pair of single strand breaks may be positioned as follows:
(1) upstream of the 5' end of the erythroid enhancer in intron 2 (e.g., between TSS+0.75kb to TSS+52.0kb), or
(2) within the erythroid enhancer provided that a portion of the erythroid enhancer is removed resulting in disruption of one or more DHSs (e.g., between TSS+52.0kb to TSS+64.4kb),
and the second pair of single strand breaks to be paired with the first pair of single strand breaks may be positioned as follows:
(3) downstream the 3' end of the erythroid enhancer in intron 2 (e.g., between TSS+64.4kb to TSS+84.7kb), or
(4) within the erythroid enhancer provided that a portion of the erythroid enhancer is removed resulting in disruption of one or more DHSs (e.g., between TSS+52.0kb to TSS+64.4kb).

For example, the pair of single strand breaks may be positioned in the BCL11A gene:
(1) between TSS+0.75kb to TSS+10kb,
(2) between TSS+10kb to TSS+20kb,
(3) between TSS+20kb to TSS+30kb,
(4) between TSS+30kb to TSS+40kb,
(5) between TSS+40kb to TSS+45kb,
(6) between TSS+45kb to TSS+47.5kb,
(7) between TSS+47.5kb to TSS+50kb,
(8) between TSS+50kb to TSS+51kb,
(9) between TSS+51kb to TSS+51.1kb,
(10) between TSS+51.1kb to TSS+51.2kb,
(11) between TSS+51.2kb to TSS+51.3kb,
(12) between TSS+51.3kb to TSS+51.4kb,
(13) between TSS+51.4kb to TSS+51.5kb,
(14) between TSS+51.5kb to TSS+51.6kb,
(15) between TSS+51.6kb to TSS+51.7kb,
(16) between TSS+51.7kb to TSS+51.8kb,
(17) between TSS+51.8kb to TSS+51.9kb,
(18) between TSS+51.9kb to TSS+52kb,
(19) between TSS+52kb to TSS+53kb,
(20) between TSS+53kb to TSS+54kb,
(21) between TSS+54kb to TSS+55kb,
(22) between TSS+55kb to TSS+56kb,
(23) between TSS+56kb to TSS+57kb,
(24) between TSS+57kb to TSS+58kb,
(25) between TSS+58kb to TSS+59kb,
(26) between TSS+59kb to TSS+60kb,
(27) between TSS+60kb to TSS+61kb,
(28) between TSS+61kb to TSS+62kb,
(29) between TSS+62kb to TSS+63kb,
(30) between TSS+63kb to TSS+64kb, or
(31) between TSS+64kb to TSS+64.4kb,
and the second pair of single strand breaks to be paired with the first pair of single strand breaks may be positioned in the BCL11A gene:
(1) between TSS+52kb to TSS+53kb,
(2) between TSS+53kb to TSS+54kb,
(3) between TSS+54kb to TSS+55kb,
(4) between TSS+55kb to TSS+56kb,
(5) between TSS+56kb to TSS+57kb,
(6) between TSS+57kb to TSS+58kb,
(7) between TSS+58kb to TSS+59kb,
(8) between TSS+59kb to TSS+60kb,
(9) between TSS+60kb to TSS+61kb,
(10) between TSS+61kb to TSS+62kb,
(11) between TSS+62kb to TSS+63kb,
(12) between TSS+63kb to TSS+64kb,
(13) between TSS+64kb to TSS+64.4kb,
(14) between TSS+64.4kb to TSS+65kb,
(15) between TSS+65kb to TSS+65.1kb,
(16) between TSS+65.1kb to TSS+65.2kb,
(17) between TSS+65.2kb to TSS+65.3kb,
(18) between TSS+65.3kb to TSS+65.4kb,
(19) between TSS+65.4kb to TSS+65.5kb,
(20) between TSS+65.5kb to TSS+65.7kb,
(21) between TSS+65.7kb to TSS+65.8kb,
(22) between TSS+65.8kb to TSS+65.9kb,
(23) between TSS+65.9kb to TSS+66kb,
(24) between TSS+66kb to TSS+67kb,
(25) between TSS+67kb to TSS+68kb,
(26) between TSS+68kb to TSS+69kb,
(27) between TSS+69kb to TSS+70kb,
(28) between TSS+70kb to TSS+75kb,
(29) between TSS+75kb to TSS+80kb, or
(30) between TSS+80kb to TSS+84.4kb.

While not wishing to be bound by theory, it is believed that the two sets of breaks (e.g., the two pair of single strand breaks) allow for NHEJ-mediated deletion of erythroid enhancer in the BCL11A gene.

### Knocking down the BCL11A gene mediated by enzymatically inactive Cas9 (eiCas9) or an eiCas9-fusion protein by targeting the promoter region of the gene

A targeted knockdown approach reduces or eliminates expression of functional *BCL11A* gene product. As described herein, in an embodiment, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *BCL11A* gene.

Methods and compositions discussed herein may be used to alter the expression of the *BCL11A* gene to treat or prevent BT by targeting a promoter region of the *BCL11A* gene. In an embodiment, the promoter region, e.g., at least 2 kb, at least 1.5 kb, at least 1.0 kb, or at least 0.5 kb upstream or downstream of the TSS is targeted to knockdown expression of the *BCL11A* gene. In an embodiment, the methods and compositions discussed herein may be used to knock down the *BCL11A* gene to treat or prevent BT by targeting 0.5 kb upstream or downstream of the TSS. A targeted knockdown approach reduces or eliminates expression of functional *BCL11A* gene product. As described herein, in an embodiment, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *BCL11A* gene.

### Other embodiments involving circulating blood cells and BCL11A gene (BT)

In an embodiment, methods and compositions discussed herein, can be used to treat or prevent BT, or its symptoms, e.g., by altering the *BCL11A* gene (also known as *B-cell CLL*/*lymphoma 11A, BCL11A-L, BCL11A-S, BCL11A-XL, CTIP1, HBFQTL5 and ZNF). BCL11A* encodes a zinc-finger protein that is involved in the regulation of globin gene expression. By altering the *BCL11A* gene (e.g., one or both alleles of the *BCL11A* gene), the levels of gamma globin can be increased. Gamma globin can replace beta globin in the hemoglobin complex and effectively carry oxygen to tissues, thereby ameliorating BT disease phenotypes.

In one aspect, methods and compositions discussed herein may be used to alter the *BCL11A* gene to treat or prevent BT, by targeting the *BCL11A* gene, e.g., coding or non-coding regions of the *BCL11A* gene. Altering the *BCL11A* gene herein refers to reducing or eliminating (1) *BCL11A* gene expression, (2) BCL11A protein function, or (3) the level of BCL11A protein.

In an embodiment, the coding region (e.g., an early coding region) of the *BCL11A* gene is targeted for alteration. In an embodiment, a non-coding sequence (e.g., an enhancer region, a promoter region, an intron, 5'UTR, 3'UTR, or polyadenylation signal) is targeted for alteration.

In an embodiment, the method provides an alteration that comprises disrupting the *BCL11A* gene by the insertion or deletion of one or more nucleotides mediated by Cas9 (e.g., enzymatically active Cas9 (eaCas9), e.g., Cas9 nuclease or Cas9 nickase) as described below. This type of alteration is also referred to as "knocking out" the *BCL11A* gene.

In another embodiment, the method provides an alteration that does not comprise nucleotide insertion or deletion in the *BCL11A* gene and is mediated by enzymatically inactive Cas9 (eiCas9) or an eiCas9-fusion protein, as described below. This type of alteration is also referred to as "knocking down" the *BCL11A* gene.

In an embodiment, the methods and compositions discussed herein may be used to alter the *BCL11A* gene to treat or prevent BT by knocking out one or both alleles of the *BCL11A* gene. In an embodiment, the coding region (e.g., an early coding region) of the *BCL11A* gene, is targeted to alter the gene. In an embodiment, a non-coding region of the *BCL11A* gene (e.g., an enhancer region, a promoter region, an intron, 5' UTR, 3'UTR, polyadenylation signal) is targeted to alter the gene. In an embodiment, an enhancer (e.g., a tissue-specific enhancer, e.g., a myeloid enhancer, e.g., an erythroid enhancer) is targeted to alter the gene. *BCL11A* erythroid enhancer comprises an approximate 12.4 kb fragment of *BCL11A* intron 2, located between approximate +52.0 to +64.4 kilobases (kb) from the Transcription Start Site (TSS+52kb to TSS+64.4kb, see **Fig. 15**). It's also referred to herein as chromosome 2 location 60,716,189-60,728,612 (according to UCSC Genome Browser hg 19 human genome assembly). Three deoxyribonuclese I hypersensitive sites (DHSs), TSS+62kb, TSS+58kb and TSS+55kb are located in this region. Deoxyribonuclease I sensitivity is a marker for gene regulatory elements. While not wishing to be bound by theory, it's believed that deleting the enhancer region (e.g., TSS+52kb to TSS+64.4kb) may reduce or eliminate *BCL11A* expression in erythroid precursors which leads to gamma globin depression while sparing *BCL11A* expression in nonerythroid lineages. In an embodiment, the method provides an alteration that comprises a deletion of the enhancer region (e.g., a tissue-specific enhancer, e.g., a myleloid enhancer, e.g., an erythroid enhancer) or a protion of the region resulting in disruption of one or more DNase 1-hypersensitivie sites (DHS). In an embodiment, the method provides an alteration that comprises an insertion or deletion of one or more nucleotides. As described herein, in an embodiment, a targeted knockout approach is mediated by non-homologous end joining (NHEJ) using a CRISPR/Cas system comprising an enzymatically active Cas9 (eaCas9). In an embodiment, a targeted knockout approach alters the *BCL11A* gene. In an embodiment, a targeted knockout approach reduces or eliminates expression of functional *BCL11A* gene product. In an embodiment, targeting affects one or both alleles of the *BCL11A* gene. In an embodiment, an enhancer disruption approach reduces or eliminates expression of functional *BCL11A* gene product in the erythroid lineage.

"BT target knockout position", as used herein, refers to a position in the *BCL11A* gene, which if altered, e.g., disrupted by insertion or deletion of one or more nucleotides, e.g., by NHEJ-mediated alteration, results in alteration of the *BCL11A* gene. In an embodiment, the position is in the *BCL11A* coding region, e.g., an early coding region. In an embodiment, the position is in a *BCL11A* non-coding region, e.g., an enhancer region.

In an embodiment, methods and compositions discussed herein, provide for altering (e.g., knocking out) the *BCL11A* gene. In an embodiment, knocking out the *BCL11A* gene herein refers to (1) insertion or deletion (e.g., NHEJ-mediated insertion or deletion) of one or more nucleotides in close proximity to or within the early coding region of the *BCL11A* gene, or (2) deletion (e.g., NHEJ-mediated deletion) of genomic sequence including the erythroid enhancer of the *BCL11A* gene. Both approaches give rise to alteration of the *BCL11A* gene as described above. In an embodiment, the BT target knockout position is altered by genome editing using the CRISPR/Cas9 system. The BT target knockout position may be targeted by cleaving with either a single nuclease or dual nickases, e.g., to induce insertion or deletion (e.g., NHEJ-mediated insertion or deletion) of one or more nucleotides in close proximity to or within the early coding region of the BT target knockout position or to delete (e.g., mediated by NHEJ) a genomic sequence including the erythroid enhancer of the *BCL11A* gene.

In an embodiment, the methods and compositions described herein introduce one or more breaks in close proximity to or within the early coding region in at least one allele of the *BCL11A* gene. In an embodiment, a single strand break is introduced in close proximity to or within the early coding region in at least one allele of the *BCL11A* gene. In an embodiment, the single strand break will be accompanied by an additional single strand break, positioned by a second gRNA molecule.

In an embodiment, a double strand break is introduced in close proximity to or within the early coding region in at least one allele of the *BCL11A* gene. In an embodiment, a double strand break will be accompanied by an additional single strand break positioned by a second gRNA molecule. In an embodiment, a double strand break will be accompanied by two additional single strand breaks positioned by a second gRNA molecule and a third gRNA molecule.

In an embodiment, a pair of single strand breaks is introduced in close proximity to or within the early coding region in at least one allele of the *BCL11A* gene. In an embodiment, the pair of single strand breaks will be accompanied by an additional double strand break, positioned by a third gRNA molecule. In an embodiment, the pair of single strand breaks will be accompanied by an additional pair of single strand breaks positioned by a third gRNA molecule and a fourth gRNA molecule.

In an embodiment, two double strand breaks are introduced to flank the erythroid enhancer at the in the *BCL11A* gene (one 5' and the other one 3' to the erythroid enhancer) to remove (e.g., delete) the genomic sequence including the erythroid enhancer. It is contemplated herein that in an embodiment the deletion of the genomic sequence including the erythroid enhancer is mediated by NHEJ. In an embodiment, the breaks (i.e., the two double strand breaks) are positioned to avoid unwanted deletion of certain elements, such as endogenous splice sites. The breaks, i.e., two double strand breaks, can be positioned upstream and downstream of the erythroid enhancer, as discussed herein.

In an embodiment, two sets of breaks (e.g., one double strand break and a pair of single strand breaks) are introduced to flank the erythroid enhancer in the *BCL11A* gene (one set 5' and the other set 3' to the erythroid enhancer) to remove (e.g., delete) the genomic sequence including the erythroid enhancer. It is contemplated herein that in an embodiment the deletion of the genomic sequence including the erythroid enhancer is mediated by NHEJ. In an embodiment, the breaks (i.e., the double strand break and the pair of single strand breaks) are positioned to avoid unwanted deletion of certain chromosome elements, such as endogenous splice sites. The breaks, e.g., the double strand break and the pair of single strand breaks, can be positioned upstream and downstream of the erythroid enhancer, as discussed herein.

In an embodiment, two sets of breaks (e.g., two pairs of single strand breaks) are introduced to flank the erythroid enhancer at the BT target position in the *BCL11A* gene (one set 5' and the other set 3' to the erythroid enhancer) to remove (e.g., delete) the genomic sequence including the erythroid enhancer. It is contemplated herein that in an embodiment the deletion of the genomic sequence including the erythroid enhancer is mediated by NHEJ. In an embodiment, the breaks (i.e., the two pairs of single strand breaks) are positioned to avoid unwanted deletion of certain chromosome elements, such as endogenous splice sites. The breaks, e.g., the two pairs of single strand breaks, can be positioned upstream and downstream of the erythroid enhancer, as discussed herein.

In an embodiment, the methods and compositions discussed herein may be used to alter the *BCL11A* gene to treat or prevent BT by knocking down one or both alleles of the *BCL11A* gene. In one embodiment, the coding region of the *BCL11A* gene, is targeted to alter the gene. In another embodiment, a non-coding region (e.g., an enhancer region, the promoter region, an intron, 5' UTR, 3'UTR, polyadenylation signal) of the *BCL11A* gene is targeted to alter the gene. In an embodiment, the promoter region of the *BCL11A* gene is targeted to knock down the expression of the *BCL11A* gene. A targeted knockdown approach alters, e.g., reduces or eliminates the expression of the *BCL11A* gene. As described herein, in an embodiment, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *BCL11A* gene.

"BT target knockdown position", as used herein, refers to a position, e.g., in the *BCL11A* gene, which if targeted by an eiCas9 or an eiCas9 fusion described herein, results in reduction or elimination of expression of functional *BCL11A* gene product. In an embodiment, transcription is reduced or eliminated. In an embodiment, the position is in the *BCL11A* promoter sequence, In an embodiment, a position in the promoter sequence of the *BCL11A* gene is targeted by an enzymatically inactive Cas9 (eiCas9) or an eiCas9-fusion protein, as described herein.

In an embodiment, one or more gRNA molecule comprising a targeting domain configured to target an enzymatically inactive Cas9 (eiCas9) or an eiCas9 fusion protein (e.g., an eiCas9 fused to a transcription repressor domain), sufficiently close to a BT target knockdown position to reduce, decrease or repress expression of the *BCL11A* gene.

"BT target position", as used herein, refers to any of a BT target knockout position, or BT target knockdown position.

In one aspect, disclosed herein is a gRNA molecule, e.g., an isolated or non-naturally occurring gRNA molecule, comprising a targeting domain which is complementary with a target domain from the *BCL11A* gene.

When two or more gRNAs are used to position two or more cleavage events, e.g., double strand or single strand breaks, in a target nucleic acid, it is contemplated that the two or more cleavage events may be made by the same or different Cas9 proteins. For example, when two gRNAs are used to position two double strand breaks, a single Cas9 nuclease may be used to create both double strand breaks. When two or more gRNAs are used to position two or more single stranded breaks (single strand breaks), a single Cas9 nickase may be used to create the two or more single strand breaks. When two or more gRNAs are used to position at least one double strand break and at least one single strand break, two Cas9 proteins may be used, e.g., one Cas9 nuclease and one Cas9 nickase. It is contemplated that when two or more Cas9 proteins are used that the two or more Cas9 proteins may be delivered sequentially to control specificity of a double strand versus a single strand break at the desired position in the target nucleic acid.

In an embodiment, the targeting domain of the first gRNA molecule and the targeting domain of the second gRNA molecule hybridize to the target domain through complementary base pairing to opposite strands of the target nucleic acid molecule. In an embodiment, the gRNA molecule and the second gRNA molecule are configured such that the PAMs are oriented outward.

In an embodiment, the targeting domain of a gRNA molecule is configured to avoid unwanted target chromosome elements, such as repeat elements, e.g., an Alu repeat, or the endogenous splice sites, in the target domain. The gRNA molecule may be a first, second, third and/or fourth gRNA molecule.

In an embodiment, the targeting domain of a gRNA molecule is configured to position a cleavage event sufficiently far from a preselected nucleotide, e.g., the nucleotide of a coding region, such that the nucleotide is not altered. In an embodiment, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

In another embodiment, a position in the coding region, e.g., the early coding region, of the *BCL11A* gene is targeted, e.g., for knockout.

In an embodiment, the BT target knockout position is the *BCL11A* coding region, e.g., early coding region, and more than one gRNA is used to position breaks, e.g., two single strand breaks or two double strand breaks, or a combination of single strand and double strand breaks, e.g., to create one or more indels, in the target nucleic acid sequence.

In another embodiment, a position in the non-coding region, e.g., the enhancer region, of the *BCL11A* gene is targeted, e.g., for knockout.

In an embodiment, the targeting domain of the gRNA molecule is configured to target an enzymatically inactive Cas9 (eiCas9) or an eiCas9 fusion protein (e.g., an eiCas9 fused to a transcription repressor domain), sufficiently close to the *BCL11A* transcription start site (TSS) to reduce (e.g., block) transcription initiation, binding of one or more transcription enhancers or activators, and/or RNA polymerase. In an embodiment, the targeting domain is configured to target between 1000bp upstream and 1000bp downstream of the TSS of the *BCL11A* gene. One or more gRNA may be used to target an eiCas9 to the promoter region of the *BCL11A* gene.

In an embodiment, the BT target knockdown position is the *BCL11A* promoter region and more than one gRNA is used to position an eiCas9 or an eiCas9-fusion protein (e.g., an eiCas9-transcription repressor domain fusion protein), in the target nucleic acid sequence.

In an embodiment, two gRNAs are used to position two breaks, e.g., two single strand breaks, in the target nucleic acid sequence. In an embodiment, the targeting domain which is complementary with the *BCL11A* gene is 16 nucleotides or more in length. In an embodiment, the targeting domain is 16 nucleotides in length. In an embodiment, the targeting domain is 17 nucleotides in length. In another embodiment, the targeting domain is 18 nucleotides in length. In still another embodiment, the targeting domain is 19 nucleotides in length. In still another embodiment, the targeting domain is 20 nucleotides in length. In still another embodiment, the targeting domain is 21 nucleotides in length. In still another embodiment, the targeting domain is 22 nucleotides in length. In still another embodiment, the targeting domain is 23 nucleotides in length. In still another embodiment, the targeting domain is 24 nucleotides in length. In still another embodiment, the targeting domain is 25 nucleotides in length. In still another embodiment, the targeting domain is 26 nucleotides in length.

In an embodiment, the targeting domain comprises 16 nucleotides. In an embodiment, the targeting domain comprises 17 nucleotides. In an embodiment, the targeting domain comprises 18 nucleotides. In an embodiment, the targeting domain comprises 19 nucleotides. In an embodiment, the targeting domain comprises 20 nucleotides. In an embodiment, the targeting domain comprises 21 nucleotides. In an embodiment, the targeting domain comprises 22 nucleotides. In an embodiment, the targeting domain comprises 23 nucleotides. In an embodiment, the targeting domain comprises 24 nucleotides. In an embodiment, the targeting domain comprises 25 nucleotides. In an embodiment, the targeting domain comprises 26 nucleotides.

In an embodiment, the gRNA, e.g., a gRNA comprising a targeting domain, which is complementary with the *BCL11A* gene, is a modular gRNA. In another embodiment, the gRNA is a unimolecular or chimeric gRNA.

A gRNA as described herein may comprise from 5' to 3': a targeting domain (comprising a "core domain", and optionally a "secondary domain"); a first complementarity domain; a linking domain; a second complementarity domain; a proximal domain; and a tail domain. In an embodiment, the proximal domain and tail domain are taken together as a single domain.

In an embodiment, a gRNA comprises a linking domain of no more than 25 nucleotides in length; a proximal and tail domain, that taken together, are at least 20 nucleotides in length; and a targeting domain equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 40 nucleotides in length; and a targeting domain equal to or greater than 16, 17, 18, 19, 20, 21, 22, 23, 24, 25 or 26 nucleotides in length.

A cleavage event, e.g., a double strand or single strand break, is generated by a Cas9 molecule. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule). Alternatively, In an embodiment, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

In an embodiment, the eaCas9 molecule catalyzes a double strand break.

In an embodiment, the eaCas9 molecule comprises HNH-like domain cleavage activity but has no, or no significant, N-terminal RuvC-like domain cleavage activity. In this case, the eaCas9 molecule is an HNH-like domain nickase, e.g., the eaCas9 molecule comprises a mutation at D10, e.g., D10A. In another embodiment, the eaCas9 molecule comprises N-terminal RuvC-like domain cleavage activity but has no, or no significant, HNH-like domain cleavage activity. In this instance, the eaCas9 molecule is an N-terminal RuvC-like domain nickase, e.g., the eaCas9 molecule comprises a mutation at H840, e.g., H840A. In an embodiment, the eaCas9 molecule is an N-terminal RuvC-like domain nickase, e.g., the eaCas9 molecule comprises a mutation at N863, e.g., N863A.

In an embodiment, a single strand break is formed in the strand of the target nucleic acid to which the targeting domain of said gRNA is complementary. In another embodiment, a single strand break is formed in the strand of the target nucleic acid other than the strand to which the targeting domain of said gRNA is complementary.

In another aspect, disclosed herein is a composition comprising (a) a gRNA molecule comprising a targeting domain that is complementary with a target domain in the *BCL11A* gene, as described herein. The composition of (a) may further comprise (b) a Cas9 molecule, e.g., a Cas9 molecule as described herein. A composition of (a) and (b) may further comprise (c) a second, third and/or fourth gRNA molecule, e.g., a second, third and/or fourth gRNA molecule described herein.

In another aspect, disclosed herein is a method of altering a cell, e.g., altering the structure, e.g., altering the sequence, of a target nucleic acid of a cell, comprising contacting said cell with: (a) a gRNA that targets the *BCL11A* gene, e.g., a gRNA as described herein; (b) a Cas9 molecule, e.g., a Cas9 molecule as described herein; and optionally, (c) a second, third and/or fourth gRNA that targets *BCL11A* gene, e.g., a gRNA, as described herein.

In an embodiment, the method comprises contacting said cell with (a) and (b).

In an embodiment, the method comprises contacting said cell with (a), (b), and (c).

In an embodiment, the method comprises contacting a cell from a subject suffering from or likely to develop BT. The cell may be from a subject that would benefit from having a mutation at a BT target position.

In an embodiment, the cell being contacted in the disclosed method is an erythroid cell. The contacting may be performed *ex vivo* and the contacted cell may be returned to the subject's body after the contacting step.

In an embodiment, the method of altering a cell as described herein comprises acquiring knowledge of the sequence of a BT target position in said cell, prior to the contacting step. Acquiring knowledge of the sequence of a BT target position in the cell may be by sequencing the *BCL11A* gene, or a portion of the *BCL11A* gene.

In an embodiment, contacting comprises delivering to the cell a Cas9 molecule of (b), as a protein or an mRNA, said gRNA of (a), as an RNA, and optionally said second gRNA of (c), as an RNA.

In an embodiment, contacting comprises delivering to the cell a gRNA of (a) as an RNA, optionally said second gRNA of (c) as an RNA, and a nucleic acid that encodes the Cas9 molecule of (b).

In another aspect, disclosed herein is a method of treating a subject suffering from or likely to develop BT, e.g., altering the structure, e.g., sequence, of a target nucleic acid of the subject, comprising contacting a cell from the subject with:
(a) a gRNA that targets the *BCL11A* gene, e.g., a gRNA disclosed herein;
(b) a Cas9 molecule, e.g., a Cas9 molecule disclosed herein; and
optionally, (c)(i) a second gRNA that targets the *BCL11A* gene, e.g., a second gRNA disclosed herein, and
further optionally, (c)(ii) a third gRNA, and still further optionally, (c)(iii) a fourth gRNA that target the BCL11A gene, e.g., a third and fourth gRNA disclosed herein.

In an embodiment, contacting comprises contacting with (a) and (b).

In an embodiment, contacting comprises contacting with (a), (b), and (c)(i).

In an embodiment, contacting comprises contacting with (a), (b), (c)(i) and (c)(ii).

In an embodiment, contacting comprises contacting with (a), (b), (c)(i), (c)(ii) and (c)(iii).

In an embodiment, the method comprises acquiring knowledge of the sequence at a BT target position in said subject.

In an embodiment, the method comprises acquiring knowledge of the sequence at a BT target position in said subject by sequencing the *BCL11A* gene or a portion of the *BCL11A* gene.

In an embodiment, the method comprises inducing a mutation at a BT target position.

In an embodiment, the method comprises inducing a mutation at a BT target position by NHEJ.

In an embodiment, a cell of the subject is contacted *ex vivo* with (a), (b), and optionally (c). In an embodiment, said cell is returned to the subject's body.

In an embodiment, contacting comprises delivering to said subject said Cas9 molecule of (b), as a protein or mRNA, and a nucleic acid which encodes (a), and optionally (c).

In an embodiment, contacting comprises delivering to the subject the Cas9 molecule of (b), as a protein or mRNA, the gRNA of (a), as an RNA, and optionally the second gRNA of (c), as an RNA.

In an embodiment, contacting comprises delivering to the subject the gRNA of (a), as an RNA, optionally said second gRNA of (c), as an RNA, a nucleic acid that encodes the Cas9 molecule of (b).

When the method comprises (1) inducing a mutation at a BT target position by NHEJ or (2) knocking down expression of the *BCL11A* gene, e.g., by targeting the promoter region, a Cas9 of (b) and at least one guide RNA, e.g., a guide RNA of (a) are included in the contacting step.

In an embodiment, a cell of the subject is contacted *ex vivo* with (a), (b) and optionally (c). In an embodiment, said cell is returned to the subject's body.

In an embodiment, contacting comprises delivering to said subject said Cas9 molecule of (b), as a protein or mRNA, and a nucleic acid which encodes (a) and optionally (c).

In an embodiment, contacting comprises delivering to the subject the Cas9 molecule of (b), as a protein or mRNA, the gRNA of (a), as an RNA, and optionally the second gRNA of (c), as an RNA.

In an embodiment, contacting comprises delivering to the subject the gRNA of (a), as an RNA, optionally said second gRNA of (c), as an RNA, and a nucleic acid that encodes the Cas9 molecule of (b).

In another aspect, disclosed herein is a reaction mixture comprising a, gRNA, a nucleic acid, or a composition described herein, and a cell, e.g., a cell from a subject having, or likely to develop BT, or a subject which would benefit from a mutation at a BT target position.

In another aspect, disclosed herein is a kit comprising, (a) gRNA molecule described herein, and one or more of the following:
(b) a Cas9 molecule, e.g., a Cas9 molecule described herein, or a nucleic acid or mRNA that encodes the Cas9;
(c)(i) a second gRNA molecule, e.g., a second gRNA molecule described herein;
(c)(ii) a third gRNA molecule, e.g., a second gRNA molecule described herein;
(c)(iii) a fourth gRNA molecule, e.g., a second gRNA molecule described herein.

The compositions, reaction mixtures and kits, as disclosed herein, can also include a governing gRNA molecule, e.g., a governing gRNA molecule disclosed herein.

### VI.5 T cells (e.g., targeting CXCR4 gene)

In one aspect, Cas9 molecules, gRNA molecules (e.g., Cas9 molecule/gRNA molecule complexes), and optionally donor template nucleic acids, Cas9 and gRNA molecules described herein can be delivered to a target cell. In an embodiment, the target cell is a circulating blood cell, e.g., a T cell (e.g., a CD4+ T cell, a CD8+ T cell, a helper T cell, a regulatory T cell, a cytotoxic T cell, a memory T cell, a T cell precursor or a natural killer T cell), a B cell (e.g., a progenitor B cell, a Pre B cell, a Pro B cell, a memory B cell, a plasma B cell), a monocyte, a megakaryocyte, a neutrophil, an eosinophil, a basophil, a mast cell, a reticulocyte, a lymphoid progenitor cell, a myeloid progenitor cell, a gut-associated lymphoid tissue (GALT) cell, a dendritic cell, a macrophage, a microglial cell,or a hematopoietic stem cell. In an embodiment, the target cell is a bone marrow cell, (e.g., a lymphoid progenitor cell, a myeloid progenitor cell, an erythroid progenitor cell, a hematopoietic stem cell, or a mesenchymal stem cell). In an embodiment, the target cell is a CD4+ T cell. In an embodiment, the target cell is a lymphoid progenitor cell (e.g. a common lymphoid progenitor (CLP) cell). In an embodiment, the target cell is a myeloid progenitor cell (e.g. a common myeloid progenitor (CMP) cell). In an embodiment, the target cell is a hematopoietic stem cell (e.g. a long term hematopoietic stem cell (LT-HSC), a short term hematopoietic stem cell (ST-HSC), a multipotent progenitor (MPP) cell, a lineage restricted progenitor (LRP) cell).

In an embodiment, the target cell is manipulated *ex vivo* by editing (e.g., introducing a mutation in) the CCR5 gene and/or modulating the expression of the CCR5 gene, and administered to the subject. In an embodiment, the target cell is manipulated *ex vivo* by editing (e.g., introducing a mutation in) the *CXCR4 gene* and/or modulating the expression of the *CXCR4 gene,* and administered to the subject. In an embodiment, the target cell is manipulated *ex vivo* by editing (e.g., introducing a mutation in) both the *CCR5* and the *CXCR4 gene* and/or modulating the expression of the both the *CCR5* and the *CXCR4 gene,* and administered to the subject. Sources of target cells for *ex vivo* manipulation may include, by way of example, the subject's blood, the subject's cord blood, or the subject's bone marrow. Sources of target cells for *ex vivo* manipulation may also include, by way of example, heterologous donor blood, cord blood, or bone marrow.

In an embodiment, a CD4+T cell is removed from the subject, manipulated *ex vivo* as described above, and the CD4+T cell is returned to the subject. In an embodiment, a lymphoid progenitor cell is removed from the subject, manipulated *ex vivo* as described above, and the lymphoid progenitor cell is returned to the subject. In an embodiment, a myeloid progenitor cell is removed from the subject, manipulated *ex vivo* as described above, and the myeloid progenitor cell is returned to the subject. In an embodiment, a hematopoietic stem cell is removed from the subject, manipulated *ex vivo* as described above, and the hematopoietic stem cell is returned to the subj ect.

A suitable cell can also include a stem cell such as, by way of example, an embryonic stem cell, an induced pluripotent stem cell, a hematopoietic stem cell, a neuronal stem cell and a mesenchymal stem cell. In an embodiment, the cell is an induced pluripotent stem cells (iPS) cell or a cell derived from an iPS cell, e.g., an iPS cell generated from the subject, modified as described above and differentiated into a clinically relevant cell such as e.g" a CD4+ T cell, a lymphoid progenitor cell, myeloid progenitor cell, a macrophage, dendritic cell, gut associated lymphoid tissue or a hematopoietic stem cell. In an embodiment, AAV is used to transduce the target cells, e.g., the target cells described herein.

In an embodiment, a cell is manipulated by altering or editing (e.g., introducing a mutation in) the *CXCR4 gene,* e.g., as described herein. In another embodiment, the expression of the *CXCR4 gene* is altered or modulated, e.g., *ex vivo.*

In an embodiment, a cell is manipulated by altering or editing (e.g., introducing a mutation in) both the *CCR5* and the *CXCR4 genes,* e.g., as described herein. In another embodiment, the expression of both the *CCR5* and the *CXCR4 genes* is altered or modulated, e.g., *ex vivo.*

### VI.6 Hematopoietic Stem/Progenitor Cells (HSPCs) (e.g., targeting CXCR4 gene)

In an embodiment, Cas9 molecules, gRNA molecules (e.g., Cas9 molecule/gRNA molecule complexes), and optionally donor template nucleic acids,the Cas9 and gRNA molecules described herein can be delivered to a target cell. In an embodiment, the target cell is a circulating blood cell, e.g., a reticulocyte, a myeloid progenitor cell, a lymphoid progenitor cell, a hematopoietic stem/progenitor cell, or an endothelial cell. In an embodiment, the target cell is a bone marrow cell (e.g., a myeloid progenitor cell, e.g., a lymphoid progenitor cell, e.g., an erythroid progenitor cell, e.g., a hematopoietic stem/progenitor cell, e.g., an endothelial cell, e.g., a mesenchymal stem cell). In an embodiment, the target cell is a myeloid progenitor cell (e.g. a common myeloid progenitor (CMP) or a granulocyte macrophage progenitor (GMP) cell). In an embodiment, the target cell is a lymphoid progenitor cell, e.g., a common lymphoid progenitor (CLP). In an embodiment, the target cell is an erythroid progenitor cell (e.g. a megakaryocyte erythroid progenitor (MEP) cell). In an embodiment, the target cell is a hematopoietic stem/progenitor cell (e.g. a long term hematopoietic stem/progenitor cell (LT-HSPC), a short term hematopoietic stem/progenitor cell (ST-HSPC), a multipotent progenitor (MPP) cell, a lineage restricted progenitor (LRP) cell). In an embodiment, the target cell is a CD34⁺ cell, a CD34⁺CD90⁺ cell, a CD34⁺CD38⁻ cell, a CD34⁺CD90⁺CD49f⁺CD38⁻CD45RA⁻ cell, a CD105⁺ cell, a CD31⁺, or a CD133⁺ cell. In an embodiment, the target cell is a an umbilical cord blood CD34⁺ HSPC, an umbilical cord venous endothelial cell, an umbilical cord arterial endothelial cells, an amniotic fluid CD34⁺ cell, an amniotic fluid endothelial cell, a placental endothelial cell or a placental hematopoietic CD34⁺ cell. In an embodiment, the target cell is a mobilized peripheral blood hematopoietic CD34⁺ cell (after the patient is treated with a mobilization agent, e.g., G-CSF or Plerixafor). In an embodiment, the target cell is a peripheral blood endothelial cell.

In an embodiment, the target cell is manipulated *ex vivo* and administered to a subject. Sources of target cells for *ex vivo* manipulation may include, by way of example, the subject's blood, cord blood, or the subject's bone marrow. Sources of target cells for *ex vivo* manipulation may also include, by way of example, heterologous donor blood, cord blood, or bone marrow.

In an embodiment, a myeloid progenitor cell is removed from the subject, manipulated *ex vivo* as described above, and the myeloid progenitor cell is returned to the subject. In an embodiment, an erythroid progenitor cell is removed from the subject, manipulated *ex vivo* as described above, and the erythroid progenitor cell is returned to the subject. In an embodiment, a lymphoid progenitor cell is removed from the subject, manipulated *ex vivo* as described above, and the lymphoid progenitor cell is returned to the subject. In an embodiment, a multipotent progenitor cell is removed from the subject, manipulated *ex vivo* as described above, and the hematopoietic stem cell is returned to the subject. In an embodiment, a hematopoietic stem/progenitor cell is removed from the subject, manipulated *ex vivo* as described above, and the hematopoietic stem/progenitor cell is returned to the subject. In an embodiment, a CD34⁺ hematopoietic stem cell is removed from the subject, manipulated *ex vivo* as described above, and the CD34⁺ hematopoietic stem/progenitor cell is returned to the subject.

A suitable cell can also include a stem cell such as, by way of example, an embryonic stem cell, an induced pluripotent stem cell, a hematopoietic stem cell, an endothelial cell, a hemogenic endothelial cell, and a mesenchymal stem cell. In an embodiment, the cell is an induced pluripotent stem (iPS) cell or a cell derived from an iPS cell, e.g., an iPS cell generated from the subject, modified to induce a mutation and differentiated into a clinically relevant cell such as a myeloid progenitor cell, a lymphoid progenitor cell, an erythroid progenitor cell, a multipotent progenitor cell, or a hematopoietic stem/progenitor cell. A suitable cell can also include an endothelial cell or amniotic cell that is differentiated into a hematopoietic stem cell.

In an embodiment, a viral vector is used to transduce the target cell. In an embodiment, AAV (e.g., AAV6 and AAVDJ) is used to transduce the target cell. In an embodiment, a lentivirus vector or an integration deficient lentivirus vector is used to transduce the target cell. In an embodiment, a ribonucleic acid (e.g., a gRNA molecule and an mRNA encoding a Cas9 molecule) is used to transfect the target cell. In an embodiment, a protein (e.g., a Cas9 molecule) and a ribonucleic acid (e.g., a gRNA molecule) are used to transfect the target cell. In an embodiment, a ribonucleoprotein complex (e.g., a Cas9 molecule/gRNA molecule complex) is used to transfect the target cell. In an embodiment, a deoxyribonucleic acid (e.g., a DNA encoding a gRNA molecule, a Cas9 molecule, or both) is used to transfect the target cells.

Cells produced by the methods described herein may be used immediately. Alternatively, the cells may be frozen (e.g., in liquid nitrogen) and stored for later use. The cells will usually be frozen in 10% dimehtylsulfoxide (DMSO), 50% serum, 40% buffered medium, or some other such solution as is commonly used in the art to preserve cells at such freezing temperature and thawed in such a manner as commonly known in the art for thawing frozen cultured cells.

### Human Immunodeficiency Virus

Human Immunodeficiency Virus (HIV) is a virus that causes severe immunodeficiency. In the United States, more than 1 million people are infected with the virus. Worldwide, approximately 30-40 million people are infected.

HIV is a single-stranded RNA virus that preferentially infects CD4 cells. The virus binds to receptors on the surface of CD4+ cells to enter and infect these cells. This binding and infection step is vital to the pathogenesis of HIV. The virus attaches to the CD4 receptor on the cell surface via its own surface glycoproteins, gp120 and gp41. These proteins are made from the cleavage product of gp160. Gp120 binds to a CD4 receptor and must also bind to another coreceptor in order for the virus to enter the host cell. In macrophage-(M-tropic) viruses, the coreceptor is CCR5 occassionaly referred to as the CCR5 receptor. M-tropic virus is found most commonly in the early stages of HIV infection.

There are two types of HIV-HIV-1 and HIV-2. HIV-1 is the predominant global form and is a more virulent strain of the virus. HIV-2 has lower rates of infection and, at present, predominantly affects populations in West Africa. HIV is transmitted primarily through sexual exposure, although the sharing of needles in intravenous drug use is another mode of transmission.

As HIV infection progresses, the virus infects CD4 cells and a subject's CD4 counts fall. With declining CD4 counts, a subject is subject to increasing risk of opportunistic infections (OI). Severely declining CD4 counts are associated with a very high likelihood of OIs, specific cancers (such as Kaposi's sarcoma, Burkitt's lymphoma) and wasting syndrome. Normal CD4 counts are between 600-1200 cells/microliter.

Untreated HIV infection is a chronic, progressive disease that leads to acquired immunodeficiency syndrome (AIDS) and death in the vast majority of subjects. Diagnosis of AIDS is made based on infection with a variety of opportunistic pathogens, presence of certain cancers and/or CD4 counts below 200 cells/µL.

HIV was untreatable and invariably led to death until the late 1980's. Since then, antiretroviral therapy (ART) has dramatically slowed the course of HIV infection. Highly active antiretroviral therapy (HAART) is the use of three or more agents in combination to slow HIV. Antiretroviral therapy (ART) is indicated in a subject whose CD4 counts has dropped below 500 cells/µL. Viral load is the most common measurement of the efficacy of HIV treatment and disease progression. Viral load measures the amount of HIV RNA present in the blood.

Treatment with HAART has significantly altered the life expectancy of those infected with HIV. A subject in the developed world who maintains their HAART regimen can expect to live into their 60's and possibly 70's. However, HAART regimens are associated with significant, long term side effects. First, the dosing regimens are complex and associated with strict food requirements. Compliance rates with dosing can be lower than 50% in some populations in the United States. In addition, there are significant toxicities associated with HAART treatment, including diabetes, nausea, malaise, sleep disturbances. A subject who does not adhere to dosing requirements of HAART therapy may have return of viral load in their blood and are at risk for progression to disease and its associated complications.

In thymic-(T-tropic) viruses, the coreceptor is *CXCR4. CXCR4* receptors are expressed by CD4 T cells, CD8 T cells, B cells, neutrophils and eosinophils. In the later stages of infection, 50-60% of subjects have T-tropic viruses that infect T cells through *CXCR4* receptors. Subjects may be infected with M-tropic viruses, T-tropic viruses, and/or M- and T-tropic viruses.

Most initial HIV infections and early stage HIV is due to entry and propogation of M-tropic virus. *CCRS*-Δ32 mutation (also refered to as *CCR5* delta 32 mutation) results in a non-functional CCR5 receptor that does not allow M-tropic HIV-1 virus entry. Individuals carrying two copies of the *CCRS*-Δ32 allele are resistant to HIV infection and *CCRS*-Δ32 heterozyous carriers have slow progression of the disease.

CCR5 antagonists (e.g. maraviroc) exist and are used in the treatment of HIV. However, current CCR5 antagonists decrease HIV progression but cannot cure the disease. In addition, there are considerable risks of side effects of these CCR5 antagonists, including severe liver toxicity.

As HIV progresses to later stage, the virus often becomes predominantly T-tropic. In later stage HIV infections, a slight majority of subjects have T-tropic viruses, which infect T cells via *CXCR4* coreceptors. *CXCR4* receptor tropism is associated with lower CD4 counts, and, often, later stage, more severe disease. There is no known protective mutation in the *CXCR4* gene that is equivalent to the *CCRS*-Δ32 mutation.

In spite of considerable advances in the treatment of HIV, there remain considerable needs for agents that could prevent, treat, and eliminate HIV infection or AIDS. Therapies that are free from significant toxicities and involve a single or multi-dose regimen (versus current daily dose regimen for the lifetime of a patient) would be superior to current HIV treatment. A reduction or complete elimination of *CCR5, CXCR4,* or both *CCR5* and *CXCR4* gene expression in myeloid and lymphoid cells would prevent HIV infection and progression, and even cure the disease.

### Other embodiments involving CXCR4 genes

In an embodiment, methods and compositions discussed herein, allow for the prevention and treatment of HIV infection and AIDS, by gene editing, e.g., using CRISPR-Cas9 mediated methods to alter a *CXCR4* gene. Altering the *CXCR4* gene herein refers to reducing or eliminating *(1) CXCR4* gene expression, (2) *CXCR4* protein function, or (3) the level of *CXCR4* protein. In an embodiment, altering the *CXCR4* gene can be achieved by (1) knocking out the *CXCR4* gene or (2) knocking down the *CXCR4* gene. The *CXCR4* gene is also known as *CD184, D2S201E, FB22, HM89, HSY3RR, LAP-3, LAP3, LCR1, LESTR, NPY3R, NPYR, NPYRL, NPYY3R, WHIM,* or *WHIMS.*

In an embodiment, methods and compositions discussed herein, allow for the prevention and treatment of HIV infection and AIDS, by gene editing, e.g., using CRISPR-Cas9 mediated methods to alter each of two genes: the gene for C-C chemokine receptor type 5 (*CCR5*) and the gene for chemokine (C-X-C motif) receptor 4 (*CXCR4*). The alteration, of two or more genes (e.g., *CCR5* and *CRCX4*) in the same cell or cells is referred to herein as "multiplexing". Multiplexing constitutes the modification of at least two genes (e.g., *CCR5* and *CRCX4*) in the same cell or cells.

Methods and compositions discussed herein, provide for prevention or reduction of HIV infection and/or prevention or reduction of the ability for HIV to enter host cells, e.g., in subjects who are already infected. Exemplary host cells for HIV include, but are not limited to, CD4 cells, CD8 cells, T cells, B cells, gut associated lymphatic tissue (GALT), macrophages, dendritic cells, myeloid progenitor cells, lymphoid progenitor cells, neutrophils, eosinophils, and microglia. Viral entry into the host cells requires interaction of the viral glycoproteins gp41 and gp120 with both the CD4 receptor and a co-receptor, e.g., CCR5, e.g., *CXCR4.* If a co-receptor, e.g., CCR5, e.g., *CXCR4,* is not present on the surface of the host cells, the virus cannot bind and enter the host cells. The progress of the disease is thus impeded. In an embodiment, by altering the *CCR5* gene, e.g., introducing one or more mutations in the gene for C-C chemokine receptor type 5 (CCR5), e.g., by introducing a protective mutation (such as a *CCR5* delta 32 mutation), knocking out the *CCR5* gene or knocking down the CCR5 gene, entry of the HIV virus into the host cells is reduced or prevented. In an embodiment, by altering the *CXCR4* gene, e.g., knocking out the *CXCR4* gene or knocking down the *CXCR4* gene, entry of the HIV virus into the host cells is reduced or prevented. In an embodiment, by multiplexing the alteration of both *CCR5* and *CXCR4,* entry of the HIV virus into the host cells is reduced or prevented. Examplary multiplexing alterations of *CCR5* and *CXCR4* genes include, but are not limited to: introducing one or more mutations in the gene for C-C chemokine receptor type 5 (*CCR5*), e.g., by introducing a protective mutation (such as a *CCR5* delta 32 mutation), and knocking out the *CXCR4* gene, introducing one or more mutations in the gene for C-C chemokine receptor type 5 (*CCR5*), e.g., by introducing a protective mutation (such as a *CCR5* delta 32 mutation), and knocking down the *CXCR4* gene, knocking out both *CCR5* and *CXCR4* genes, knocking down both *CCR5* and *CXCR4* genes, knocking out the *CCR5* gene and knocking down the *CXCR4* gene, or knocking down the *CCR5* gene and knocking out the *CXCR4* gene.

In an embodiment, altering both *CCR5* and *CXCR4* genes in myeloid and lymphoid cells reduces or prevents HIV infection and/or treats HIV disease. While not wishing to be bound by theory, both T-tropic and M-tropic viral entry into myeloid and lymphoid cells are prevented or reduced by altering both CCR5 and *CXCR4* genes. In an embodiment, a subject who has HIV and is treated with alteration of CCR5 and *CXCR4* genes would be expected to clear HIV and effectively be cured. In an embodiment, a subject who does not yet have HIV and is treated with altering both CCR5 and *CXCR4* genes would be expected to be immune to HIV.

In an embodiment, methods and compositions discussed herein, provide for treating or delaying the onset or progression of HIV infection or AIDS by gene editing, e.g., using CRISPR-Cas9 mediated methods to alter a *CXCR4* gene. Altering the *CXCR4* gene herein refers to reducing or eliminating (1) *CXCR4* gene expression, (2) *CXCR4* protein function, or (3) the level of *CXCR4* protein.

In an embodiment, methods and compositions discussed herein, provide for treating or delaying the onset or progression of HIV infection or AIDS by gene editing, e.g., using CRISPR-Cas9 mediated methods to alter two genes in a single cell or cells, e.g., a *CCR5* gene and a *CXCR4* gene. Altering the *CCR5* gene and the *CXCR4* gene herein refers to reducing or eliminating (1) *CCR5* and *CXCR4* gene expression, (2) CCR5 and *CXCR4* protein function, or (3) levels of CCR5 and *CXCR4* protein.

"CCR5 target position", as used herein, refers to any position that results in inactivation of the *CCR5* gene. In an embodiment, a CCR5 target position refers to any of a CCR5 target knockout position or a CCR5 target knockdown position, as described herein.

*"CXCR4* target position", as used herein, refers to any position that results in inactivation of the *CXCR4* gene. In an embodiment, a *CXCR4* target position refers to any of a *CXCR4* target knockout position or a *CXCR4* target knockdown position, as described herein.

### Methods to Treat or Prevent HIV Infection or AIDS

Methods and compositions described herein provide for a therapy, e.g., a one-time therapy, or a multi-dose therapy, that prevents or treats HIV infection and/or AIDS. In an embodiment, a disclosed therapy prevents, inhibits, or reduces the entry of HIV into CD4 cells of a subject who is already infected. In an embodiment, methods and compositions described herein prevent, inhibit, and/or reduce the entry of HIV into CD4 cells, CD8 cells, T cells, B cells, neutrophils, eosinophils, GALT, dendritic cells, microglia cells, myeloid progenitor cells, and/or lymphoid progenitor cells of a subject who is already infected. While not wishing to be bound by theory, in an embodiment, it is believed that knocking out CCR5 on CD4 cells, T cells, GALT, macrophages, dendritic cells, and microglia cells, renders the HIV virus unable to enter host immune cells. While not wishing to be bound by theory, in an embodiment, it is believed that knocking out *CXCR4* on CD4 cells, CD8 cells, T cells, B cells, neutrophils and eosinophils renders the HIV virus unable to enter host immune cells. While not wishing to be bound by theory, in an embodiment, it is believed that knocking out both CCR5 and *CXCR4* on CD4 cells, CD8 cells, T cells, B cells, neutrophils, eosinophils, GALT, dendritic cells, microglia cells, myeloid progenitor cells, and/or lymphoid progenitor cells renders the HIV virus unable to enter host immune cells.

Viral entry into CD4 cells, CD8 cells, T cells, B cells, neutrophils, eosinophils, GALT, dendritic cells, microglia cells, myeloid progenitor cells, and/or lymphoid progenitor cells requires interaction of the viral glycoproteins gp41 and gp120 with both the CD4 receptor and a coreceptor, e.g., CCR5, e.g., *CXCR4.* Once a functional coreceptor such as CCR5 and/or *CXCR4* has been eliminated from the surface of the CD4 cells, CD8 cells, T cells, B cells, neutrophils, eosinophils, GALT, dendritic cells, microglia cells, myeloid progenitor cells, and/or lymphoid progenitor cells, the virus is prevented from binding and entering the host cells. In an embodiment, the disease does not progress or has delayed progression compared to a subject who has not received the therapy.

While not wishing to be bound by theory, subjects with naturally occurring CCR5 receptor mutations who have delayed HIV progression may confer protection by the mechanism of action described herein. Subjects with a specific deletion in the *CCR5* gene (e.g., the delta 32 deletion) have been shown to have much higher likelihood of being long-term non-progressors (meaning they did not require HAART and their HIV infection did not progress). See, e.g., Stewart GJ et al., 1997 The Australian Long-Term Non-Progressor Study Group. Aids. 11:1833-1838. In addition, a subject who was CCR5+ (had a wild type CCR5 receptor) and infected with HIV underwent a bone marrow transplant for acute myeloid lymphoma. See, e.g., Hutter G et al., 2009N ENGL J MED.360:692-698. The bone marrow transplant (BMT) was from a subject homozygous for a CCR5 delta 32 deletion. Following BMT, the subject did not have progression of HIV and did not require treatment with ART. These subjects offer evidence for the fact that introduction of a protective mutation of the *CCR5* gene, or knockout or knockdown of the *CCR5* gene prevents, delays or diminishes the ability of HIV to infect the subject. Mutation or deletion of the *CCR5* gene, or reduced *CCR5* gene expression, should therefore reduce the progression, virulence and pathology of HIV.

While not wishing to be bound by theory, knockout or knockdown of the *CXCR4* gene will eliminate or reduce *CXCR4* gene expression. Decreased expression of coreceptor *CXCR4* on the surface of CD4 cells, CD8 cells, T cells, B cells, neutrophils and eosinophils will prevent, delay or diminish the ability of T-trophic HIV to infect the subject. Mutation or deletion of the *CXCR4* gene, or reduced *CXCR4* gene expression, should therefore reduce the progression, virulence and pathology of HIV.

While not wishing to be bound by theory, knockout or knockdown of both the *CCR5* and *CXCR4* gene will eliminate or reduce *CCR5* and *CXCR4* gene expression. Decreased expression of co-receptors CCR5 and *CXCR4* on the surface of CD4 cells, CD8 cells, T cells, B cells, neutrophils, eosinophils, GALT, dendritic cells, microglia cells, myeloid progenitor cells, and/or lymphoid progenitor cells will prevent, delay or diminish the ability of both M-trophic and T-trophic HIV to infect the subject. Mutation or deletion of both the *CCR5* and the *CXCR4* genes, or reduced *CCR5* and *CXCR4* gene expression, should therefore reduce the progression, virulence and pathology of HIV.

In an embodiment, a method described herein is used to treat a subject having HIV.

In an embodiment, a method described herein is used to treat a subject having AIDS.

In an embodiment, a method described herein is used to prevent, or delay the onset or progression of, HIV infection and AIDS in a subject at high risk for HIV infection.

In an embodiment, a method described herein results in a selective advantage to survival of treated CD4 cells. In an embodiment, a method described herein results in a selective advantage to survival of treated CD8 cells, T cells, B cells, neutrophils, eosinophils, GALT, dendritic cells, microglia cells, myeloid progenitor cells, and/or lymphoid progenitor cells. In an embodiment, some proportion of CD4 cells, CD8 cells, T cells, B cells, neutrophils, eosinophils, myeloid progenitor cells, lymphoid progenitor cells, and/or hematopoietic stem cells will be modified and have a *CXCR4* deletion mutation. In an embodiment, some proportion of CD4 cells, CD8 cells, T cells, B cells, neutrophils, eosinophils, myeloid progenitor cells, lymphoid progenitor cells, and/or hematopoietic stem cells will be modified and have a *CXCR4* mutation that decreases *CXCR4* gene expression.

In an embodiment, some proportion of CD4 cells, CD8 cells, T cells, B cells, neutrophils, eosinophils, GALT, dendritic cells, microglia cells, myeloid progenitor cells, lymphoid progenitor cells, and/or hematopoietic stem cells will be modified and have both a *CCR5* protective mutation and a *CXCR4* deletion mutation. In an embodiment, some proportion of CD4 cells, CD8 cells, T cells, B cells, neutrophils, eosinophils, GALT, dendritic cells, microglia cells, myeloid progenitor cells, lymphoid progenitor cells, and/or hematopoietic stem cells will be modified and have both a *CCR5* protective mutation and a mutation that decreases *CXCR4* gene expression.

In an embodiment, some proportion of CD4 cells, CD8 cells, T cells, B cells, neutrophils, eosinophils, GALT, dendritic cells, microglia cells, myeloid progenitor cells, lymphoid progenitor cells, and/or hematopoietic stem cells will be modified and have both a *CCR5* deletion mutation and a *CXCR4* deletion mutation. In an embodiment, some proportion of CD4 cells, CD8 cells, T cells, B cells, neutrophils, eosinophils, GALT, dendritic cells, microglia cells, myeloid progenitor cells, lymphoid progenitor cells, and/or hematopoietic stem cells will be modified and have both a *CCR5* deletion mutation and a mutation that decreases *CXCR4* gene expression.

In an embodiment, some proportion of CD4 cells, CD8 cells, T cells, B cells, neutrophils, eosinophils, GALT, dendritic cells, microglia cells, myeloid progenitor cells, lymphoid progenitor cells, and/or hematopoietic stem cells will be modified and have both a mutation that decreases *CCR5* gene expression and a *CXCR4* deletion mutation. In an embodiment, some proportion of CD4 cells, CD8 cells, T cells, B cells, neutrophils, eosinophils, GALT, dendritic cells, microglia cells, myeloid progenitor cells, lymphoid progenitor cells, and/or hematopoietic stem cells will be modified and have both a mutation that decreases *CCR5* gene expression and a mutation that decreases *CXCR4* gene expression. In an embodiment, these cells are not subject to infection with HIV. Cells that are not modified may be infected with HIV and are expected to undergo cell death. In an embodiment, after the treatment described herein, treated cells survive, while untreated cells die. In an embodiment, this selective advantage drives eventual colonization in all body compartments with 100% CCR5-negative CD4 cells, T cells, GALT, macrophages, dendritic cells, microglia cells, myeloid progenitor cells, lymphoid progenitor cells, and hematopoietic stem cells derived from treated cells, conferring complete protection in treated subjects against infection with M tropic HIV. In an embodiment, this selective advantage drives eventual colonization in all body compartments with 100% *CXCR4*-negative CD4 cells, CD8 cells, T cells, B cells, neutrophils, eosinophils, myeloid progenitor cells, lymphoid progenitor cells, and hematopoietic stem cells derived from treated cells, conferring complete protection in treated subjects against infection with T tropic HIV. In an embodiment, this selective advantage drives eventual colonization in all body compartments with 100% CCR5-negative and 100% *CXCR4*-negative CD4 cells, CD8 cells, T cells, B cells, neutrophils, eosinophils, GALT, dendritic cells, microglia cells, myeloid progenitor cells, lymphoid progenitor cells, and hematopoietic stem cells derived from treated cells, conferring complete protection in treated subjects against infection with both M tropic and T tropic HIV.

In an embodiment, the method comprises initiating treatment of a subject prior to disease onset.

In an embodiment, the method comprises initiating treatment of a subject after disease onset.

In an embodiment, the method comprises initiating treatment of a subject after disease onset, e.g., 1, 2, 3, 4, 5, 6, 7, 8, 9, 10, 12, 16, 24, 36, 48 or more months after onset of HIV infection or AIDS. While not wishing to be bound by theory, it is believed that this may be effective as disease progression is slow in some cases and a subject may present well into the course of illness.

In an embodiment, the method comprises initiating treatment of a subject in an advanced stage of disease, e.g., to slow viral replication and viral load.

Overall, initiation of treatment for a subject at all stages of disease is expected to prevent or reduce disease progression and benefit a subject.

In an embodiment, the method comprises initiating treatment of a subject prior to disease onset and prior to infection with HIV.

In an embodiment, the method comprises initiating treatment of a subject in an early stage of disease, e.g., when a subject has tested positive for HIV infection but has no signs or symptoms associated with HIV.

In an embodiment, the method comprises initiating treatment of a patient at the appearance of a reduced CD4 count or a positive HIV test.

In an embodiment, the method comprises treating a subject considered at risk for developing HIV infection.

In an embodiment, the method comprises treating a subject who is the spouse, partner, sexual partner, newborn, infant, or child of a subject with HIV.

In an embodiment, the method comprises treating a subject for the prevention or reduction of HIV infection.

In an embodiment, the method comprises treating a subject at the appearance of any of the following findings consistent with HIV: low CD4 count; opportunistic infections associated with HIV, including but not limited to: candidiasis, mycobacterium tuberculosis, cryptococcosis, cryptosporidiosis, cytomegalovirus; and/or malignancy associated with HIV, including but not limited to: lymphoma, Burkitt's lymphoma, or Kaposi's sarcoma.

In an embodiment, the method comprises treating a subject who is undergoing a heterologous hematopoietic stem cell transplant, including an umbilical cord blood transplant, e.g., in a subject with or without HIV.

In an embodiment, a cell is treated *ex vivo* and returned to a patient.

In an embodiment, an autologous CD4 cell can be treated *ex vivo* and returned to the subject. In an embodiment, an autologous CD8 cell, T cell, B cell, neutrophil, eosinophil, GALT, dendritic cell, microglia cell, myeloid progenitor cell, and/or lymphoid progenitor cell cell can be treated *ex vivo* and returned to the subject.

In an embodiment, a heterologous CD4 cell can be treated *ex vivo* and transplanted into the subject. In an embodiment, a heterologous CD8 cell, T cell, B cell, neutrophil, eosinophil, GALT, dendritic cell, microglia cell, myeloid progenitor cell, and/or lymphoid progenitor cell cell can be treated *ex vivo* and returned to the subject.

In an embodiment, an autologous stem cell, e.g., an autologous hematopoietic stem cell, e.g., an autologous umbilical cord blood transplant cell, can be treated *ex vivo* and returned to the subj ect.

In an embodiment, a heterologous stem cell, e.g., a heterologous hematopoietic stem cell, e.g., an autologous umbilical cord blood transplant cell, can be treated *ex vivo* and transplanted into the subject.

In an embodiment, the treatment comprises delivery of a gRNA molecule by a nanoparticle.

In an embodiment, treatment to eliminate or decrease *CXCR4* gene expression is initiated after a subject is determined to have a mutation (e.g., an inactivating mutation, e.g., an inactivating mutation in either or both alleles) in *CCR5* by genetic screening, e.g., genotyping, wherein the genetic testing was performed prior to or after disease onset.

### Methods of Altering CXCR4

In one aspect, the methods and compositions discussed herein, inhibit or block a critical aspect of the HIV life cycle, i.e., CXCR4-mediated entry into T cells, i.e., *CXCR4*-mediated entry into B cells, by alteration (e.g., inactivation) of the *CXCR4* gene. While not wishing to be bound by theory, exemplary mechanisms that can be associated with the alteration of the *CXCR4* gene include, but are not limited to, non-homologous end joining (NHEJ) (e.g., classical or alternative), microhomology-mediated end joining (MMEJ), homology-directed repair (e.g., endogenous donor template mediated), SDSA (synthesis dependent strand annealing), single strand annealing or single strand invasion. Alteration of the *CXCR4* gene, e.g., mediated by NHEJ, can result in a mutation, which typically comprises a deletion or insertion (indel). The introduced mutation can take place in any region of the *CXCR4* gene, e.g., a promoter region or other non-coding region, or a coding region, so long as the mutation results in reduced or loss of the ability to mediate HIV entry into the cell.

In another aspect, the methods and compositions discussed herein may be used to alter the *CXCR4* gene to treat or prevent HIV infection or AIDS by targeting the coding sequence of the *CXCR4* gene.

In an embodiment, the gene, e.g., the coding sequence of the *CXCR4* gene, is targeted to knock out the gene, e.g., to eliminate expression of the gene, e.g., to knock out both alleles of the *CXCR4* gene, e.g., by introduction of an alteration comprising a mutation (e.g., an insertion or deletion) in the *CXCR4* gene. This type of alteration is sometimes referred to as "knocking out" the *CXCR4* gene. While not wishing to be bound by theory, in an embodiment, a targeted knockout approach is mediated by NHEJ using a CRISPR/Cas system comprising a Cas9 molecule, e.g., an enzymatically active Cas9 (eaCas9) molecule, as described herein.

In another aspect, the methods and compositions discussed herein may be used to alter the *CXCR4* gene to treat or prevent HIV infection or AIDS by targeting a non-coding sequence of the *CXCR4* gene, e.g., a promoter, an enhancer, an intron, a 3'UTR, and/or a polyadenylation signal.

In one embodiment, the gene, e.g., the non-coding sequence of the *CXCR4* gene, is targeted to knock out the gene, e.g., to eliminate expression of the gene, e.g., to knock out both alleles of the *CXCR4* gene, e.g., by introduction of an alteration comprising a mutation (e.g., an insertion or deletion) in the *CXCR4* gene. In an embodiment, the method provides an alteration that comprises an insertion or deletion. This type of alteration is also sometimes referred to as "knocking out" the *CXCR4* gene. While not wishing to be bound by theory, in an embodiment, a targeted knockout approach is mediated by NHEJ using a CRISPR/Cas system comprising a Cas9 molecule, e.g., an enzymatically active Cas9 (eaCas9) molecule, as described herein.

In an embodiment, methods and compositions discussed herein, provide for altering (e.g., knocking out) the *CXCR4* gene. In an embodiment, knocking out the *CXCR4* gene herein refers to (1) insertion or deletion (e.g., NHEJ-mediated insertion or deletion) of one or more nucleotides of the *CXCR4* gene (e.g., in close proximity to or within an early coding region or in a non-coding region), or (2) deletion (e.g., NHEJ-mediated deletion) of a genomic sequence of the *CXCR4* gene (e.g., in a coding region or in a non-coding region). Both approaches can give rise to alteration (e.g., knockout) of the *CXCR4* gene as described herein. In an embodiment, a *CXCR4* target knockout position is altered by genome editing using the CRISPR/Cas9 system. The *CXCR4* target knockout position may be targeted by cleaving with either one or more nucleases, or one or more nickases, or a combination thereof.

*"CXCR4* target knockout position", as used herein, refers to a position in the *CXCR4* gene, which if altered, e.g., disrupted by insertion or deletion of one or more nucleotides, e.g., by NHEJ-mediated alteration, results in alteration of the *CXCR4* gene. In an embodiment, the position is in the *CXCR4* coding region, e.g., an early coding region. In another embodiment, the position is in a non-coding sequence of the *CXCR4* gene, e.g., a promoter, an enhancer, an intron, a 3'UTR, and/or a polyadenylation signal.

In another embodiment, the *CXCR4* gene is targeted to knock down the gene, e.g., to reduce or eliminate expression of the gene, e.g., to knock down one or both alleles of the *CXCR4* gene.

In one embodiment, the coding region of the *CXCR4* gene, is targeted to alter the expression of the gene. In another embodiment, a non-coding region (e.g., an enhancer region, a promoter region, an intron, a 5' UTR, a 3'UTR, or a polyadenylation signal) of the *CXCR4* gene is targeted to alter the expression of the gene. In an embodiment, the promoter region of the *CXCR4* gene is targeted to knock down the expression of the *CXCR4* gene. This type of alteration is also sometimes referred to as "knocking down" the *CXCR4* gene. While not wishing to be bound by theory, in an embodiment, a targeted knockdown approach is mediated by a CRISPR/Cas system comprising a Cas9 molecule, e.g., an enzymatically inactive Cas9 (eiCas9) molecule or an eiCas9 fusion protein (e.g., an eiCas9 fused to a transcription repressor domain or chromatin modifying protein), as described herein. In an embodiment, the *CXCR4* gene is targeted to alter (e.g., to block, reduce, or decrease) the transcription of the *CXCR4* gene. In another embodiment, the *CXCR4* gene is targeted to alter the chromatin structure (e.g., one or more histone and/or DNA modifications) of the *CXCR4* gene. In an embodiment, one or more gRNA molecules comprising a targeting domain are configured to target an enzymatically inactive Cas9 (eiCas9) molecule or an eiCas9 fusion protein (e.g., an eiCas9 fused to a transcription repressor domain), sufficiently close to a *CXCR4* target knockdown position to reduce, decrease or repress expression of the *CXCR4* gene.

*"CXCR4* target knockdown position", as used herein, refers to a position in the *CXCR4* gene, which if targeted, e.g., by an eiCas9 molecule or an eiCas9 fusion described herein, results in reduction or elimination of expression of functional *CXCR4* gene product. In an embodiment, the transcription of the *CXCR4* gene is reduced or eliminated. In another embodiment, the chromatin structure of the *CXCR4* gene is altered. In an embodiment, the position is in the *CXCR4* promoter sequence. In an embodiment, a position in the promoter sequence of the *CXCR4* gene is targeted by an enzymatically inactive Cas9 (eiCas9) molecule or an eiCas9 fusion protein, as described herein.

"*CXCR4* target position", as used herein, refers to any position that results in inactivation of the *CXCR4* gene. In an embodiment, a *CXCR4* target position refers to any of a *CXCR4* target knockout position or a *CXCR4* target knockdown position, as described herein.

In one aspect, disclosed herein is a gRNA molecule, e.g., an isolated or non-naturally occurring gRNA molecule, comprising a targeting domain which is complementary with a target domain from the *CXCR4* gene.

In an embodiment, the targeting domain of the gRNA molecule is configured to provide a cleavage event, e.g., a double strand break or a single strand break, sufficiently close to a *CXCR4* target position in the *CXCR4* gene to allow alteration, e.g., alteration associated with NHEJ, of a *CXCR4* target position in the *CXCR4* gene. In an embodiment, the alteration comprises an insertion or deletion. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 300, 400, 450, or 500 nucleotides of a *CXCR4* target position. The break, e.g., a double strand or single strand break, can be positioned upstream or downstream of a *CXCR4* target position in the *CXCR4* gene.

In an embodiment, a second gRNA molecule comprising a second targeting domain is configured to provide a cleavage event, e.g., a double strand break or a single strand break, sufficiently close to the *CXCR4* target position in the *CXCR4* gene, to allow alteration, e.g., alteration associated with NHEJ, of the *CXCR4* target position in the *CXCR4* gene, either alone or in combination with the break positioned by said first gRNA molecule. In an embodiment, the targeting domains of the first and second gRNA molecules are configured such that a cleavage event, e.g., a double strand or single strand break, is positioned, independently for each of the gRNA molecules, within 1, 2, 3, 4, 5, 10, 15, 20, 25, 30, 35, 40, 45, 50, 60, 70, 80, 90, 100, 150, 200, 300, 400, 450, or 500 nucleotides of the target position. In an embodiment, the breaks, e.g., double strand or single strand breaks, are positioned on both sides of a nucleotide of a *CXCR4* target position in the *CXCR4* gene. In an embodiment, the breaks, e.g., double strand or single strand breaks, are positioned on one side, e.g., upstream or downstream, of a nucleotide of a *CXCR4* target position in the *CXCR4* gene.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 300, 400, 450, or 500 nucleotides of a *CXCR4* target position. In an embodiment, the first and second gRNA molecules are configured such, that when guiding a Cas9 molecule, e.g., a Cas9 nickase, a single strand break will be accompanied by an additional single strand break, positioned by a second gRNA, sufficiently close to one another to result in alteration of a *CXCR4* target position in the *CXCR4* gene. In an embodiment, the first and second gRNA molecules are configured such that a single strand break positioned by said second gRNA is within 1, 2, 3, 4, 5, 10, 20, 30, 40, 50, 60, 70, 80, 90, 100, 200, 300, 400, 500, 600, 700, 800, 900, or 1000 nucleotides of the break positioned by said first gRNA molecule, e.g., when the Cas9 molecule is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream of a *CXCR4* target position in the *CXCR4* gene, e.g., within 1, 2, 3, 4, 5, 10, 15, 20, 25, 30, 35, 40, 45, 50, 60, 70, 80, 90, 100, 150, 200, 300, 400, 450, or 500 nucleotides of the target position; and the targeting domain of a second gRNA molecule is configured such that a double strand break is positioned downstream of a *CXCR4* target position in the *CXCR4* gene, e.g., within 1, 2, 3, 4, 5, 10, 15, 20, 25, 30, 35, 40, 45, 50, 60, 70, 80, 90, 100, 150, 200, 300, 400, 450, or 500 nucleotides of the target position. In an embodiment, the first and second gRNA molecules are configured such that a double strand break positioned by said second gRNA is within 10, 20, 30, 40, 50, 60, 70, 80, 90, 100, 200, 300, 400, 500, 600, 700, 800, 900, or 1000 nucleotides of the break positioned by said first gRNA molecule.

In an embodiment, the targeting domains of the first and second gRNA molecules are configured such that a cleavage event, e.g., a single strand break, is positioned, independently for each of the gRNA molecules.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream of a *CXCR4* target position in the *CXCR4* gene, e.g., within 1, 2, 3, 4, 5, 10, 15, 20, 25, 30, 35, 40, 45, 50, 60, 70, 80, 90, 100, 150, 200, 300, 400, 450, or 500 nucleotides of the target position; and the targeting domains of a second and third gRNA molecule are configured such that two single strand breaks are positioned downstream of a *CXCR4* target position in the *CXCR4* gene, e.g., within 1, 2, 3, 4, 5, 10, 15, 20, 25, 30, 35, 40, 45, 50, 60, 70, 80, 90, 100, 150, 200, 300, 400, 450, or 500 nucleotides of the target position. In an embodiment, the first, second and third gRNA molecules are configured such that a single strand break positioned by said second or third gRNA molecule is within 10, 20, 30, 40, 50, 60, 70, 80, 90, 100, 200, 300, 400, 500, 600, 700, 800, 900, or 1000 nucleotides of the break positioned by said first gRNA molecule. In an embodiment, the targeting domains of the first, second and third gRNA molecules are configured such that a cleavage event, e.g., a double strand or single strand break, is positioned, independently for each of the gRNA molecules.

In an embodiment, when *CXCR4* is targeted for knock out, a first and second single strand breaks can be accompanied by two additional single strand breaks positioned by a third gRNA molecule and a fourth gRNA molecule. For example, the targeting domain of a first and second gRNA molecule are configured such that two single strand breaks are positioned upstream of a *CXCR4* target position in the *CXCR4* gene, e.g., within 1, 2, 3, 4, 5, 10, 15, 20, 25, 30, 35, 40, 45, 50, 60, 70, 80, 90, 100, 150, 200, 300, 400, 450, or 500 nucleotides of the target position; and the targeting domains of a third and fourth gRNA molecule are configured such that two single strand breaks are positioned downstream of a *CXCR4* target position in the *CXCR4* gene, e.g., within 1, 2, 3, 4, 5, 10, 15, 20, 25, 30, 35, 40, 45, 50, 60, 70, 80, 90, 100, 150, 200, 300, 400, 450, or 500 nucleotides of the target position. In an embodiment, the first, second, third and fourth gRNA molecules are configured such that the single strand break positioned by said third or fourth gRNA molecule is within 10, 20, 30, 40, 50, 60, 70, 80, 90, 100, 200, 300, 400, 500, 600, 700, 800, 900, or 1000 nucleotides of the break positioned by said first or second gRNA molecule, e.g., when the Cas9 molecule is a nickase. In an embodiment, the targeting domains of the first, second, third and fourth gRNA molecules are configured such that a cleavage event, e.g., a single strand break, is positioned, independently for each of the gRNA molecules.

It is contemplated herein that, in an embodiment, when multiple gRNAs are used to generate (1) two single stranded breaks in close proximity, (2) two double stranded breaks, e.g., flanking a *CXCR4* target position (e.g., to remove a piece of DNA, e.g., a insertion or deletion mutation) or to create more than one indel in an early coding region, (3) one double stranded break and two paired nicks flanking a *CXCR4* target position (e.g., to remove a piece of DNA, e.g., a insertion or deletion mutation) or (4) four single stranded breaks, two on each side of a *CXCR4* target position, that they are targeting the same *CXCR4* target position. It is further contemplated herein that in an embodiment multiple gRNAs may be used to target more than one target position in the same gene.

In an embodiment, the targeting domain of the first gRNA molecule and the targeting domain of the second gRNA molecules are complementary to opposite strands of the target nucleic acid molecule. In an embodiment, the gRNA molecule and the second gRNA molecule are configured such that the PAMs are oriented outward.

In an embodiment, the targeting domain of a gRNA molecule is configured to avoid unwanted target chromosome elements, such as repeat elements, e.g., Alu repeats, in the target domain. The gRNA molecule may be a first, second, third and/or fourth gRNA molecule, as described herein.

In an embodiment, the targeting domain of a gRNA molecule is configured to position a cleavage event sufficiently far from a preselected nucleotide, e.g., the nucleotide of a coding region, such that the nucleotide is not altered. In an embodiment, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

In an embodiment, the targeting domain which is complementary with a target domain from the *CXCR4* target position in the *CXCR4* gene is 16 nucleotides or more in length. In an embodiment, the targeting domain is 16 nucleotides in length. In an embodiment, the targeting domain is 17 nucleotides in length. In other embodiments, the targeting domain is 18 nucleotides in length. In still other embodiments, the targeting domain is 19 nucleotides in length. In still other embodiments, the targeting domain is 20 nucleotides in length. In an embodiment, the targeting domain is 21 nucleotides in length. In an embodiment, the targeting domain is 22 nucleotides in length. In an embodiment, the targeting domain is 23 nucleotides in length. In an embodiment, the targeting domain is 24 nucleotides in length. In an embodiment, the targeting domain is 25 nucleotides in length. In an embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

A gRNA as described herein may comprise from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a gRNA comprises a linking domain of no more than 25 nucleotides in length; a proximal and tail domain, that taken together, are at least 20 nucleotides in length; and a targeting domain equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 25 nucleotides in length; and a targeting domain equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 40 nucleotides in length; and a targeting domain equal to or greater than 16, 17, 18, 19, 20, 21, 22, 23, 24, 25 or 26 nucleotides in length.

A cleavage event, e.g., a double strand or single strand break, is generated by a Cas9 molecule. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule).

In an embodiment, the eaCas9 molecule catalyzes a double strand break.

In some embodiments, the eaCas9 molecule comprises HNH-like domain cleavage activity but has no, or no significant, N-terminal RuvC-like domain cleavage activity. In this case, the eaCas9 molecule is an HNH-like domain nickase, e.g., the eaCas9 molecule comprises a mutation at D10, e.g., D10A. In other embodiments, the eaCas9 molecule comprises N-terminal RuvC-like domain cleavage activity but has no, or no significant, HNH-like domain cleavage activity. In an embodiment, the eaCas9 molecule is an N-terminal RuvC-like domain nickase, e.g., the eaCas9 molecule comprises a mutation at H840, e.g., H840A. In an embodiment, the eaCas9 molecule is an N-terminal RuvC-like domain nickase, e.g., the eaCas9 molecule comprises a mutation at N863, e.g., N863A.

In an embodiment, a single strand break is formed in the strand of the target nucleic acid to which the targeting domain of said gRNA is complementary. In another embodiment, a single strand break is formed in the strand of the target nucleic acid other than the strand to which the targeting domain of said gRNA is complementary.

In another aspect, disclosed herein is a nucleic acid, e.g., an isolated or non-naturally occurring nucleic acid, e.g., DNA, that comprises (a) a sequence that encodes a gRNA molecule comprising a targeting domain that is complementary with a *CXCR4* target position in the *CXCR4* gene as disclosed herein.

In an embodiment, the nucleic acid encodes a gRNA molecule, e.g., a first gRNA molecule, comprising a targeting domain configured to provide a cleavage event, e.g., a double strand break or a single strand break, sufficiently close to a *CXCR4* target position in the *CXCR4* gene to allow alteration, e.g., alteration associated with NHEJ, of a *CXCR4* target position in the *CXCR4* gene.

In an embodiment, the nucleic acid encodes a gRNA molecule, e.g., a first gRNA molecule, comprising a targeting domain configured to target an enzymatically inactive Cas9 (eiCas9) molecule or an eiCas9 fustion protein (e.g., an eiCas9 fused to a transcription repressor domain or chromatin modifying protein), sufficiently close to a *CXCR4* knockdown target position to reduce, decrease or repress expression of the *CXCR4* gene.

The Cas9 molecule may be a nickase molecule, an enzymatically active Cas9 (eaCas9) molecule, e.g., an eaCas9 molecule that forms a double strand break in a target nucleic acid and/or an eaCas9 molecule that forms a single strand break in a target nucleic acid. In an embodiment, a single strand break is formed in the strand of the target nucleic acid to which the targeting domain of said gRNA is complementary. In another embodiment, a single strand break is formed in the strand of the target nucleic acid other than the strand to which to which the targeting domain of said gRNA is complementary.

In an embodiment, the eaCas9 molecule catalyzes a double strand break.

In an embodiment, the eaCas9 molecule comprises HNH-like domain cleavage activity but has no, or no significant, N-terminal RuvC-like domain cleavage activity. In another embodiment, the said eaCas9 molecule is an HNH-like domain nickase, e.g., the eaCas9 molecule comprises a mutation at D10, e.g., D10A. In another embodiment, the eaCas9 molecule comprises N-terminal RuvC-like domain cleavage activity but has no, or no significant, HNH-like domain cleavage activity. In another embodiment, the eaCas9 molecule is an N-terminal RuvC-like domain nickase, e.g., the eaCas9 molecule comprises a mutation at H840, e.g., H840A. In another embodiment, the eaCas9 molecule is an N-terminal RuvC-like domain nickase, e.g., the eaCas9 molecule comprises a mutation at N863, e.g., N863A.

In an embodiment, the Cas9 molecule is an enzymatically active Cas9 (eaCas9) molecule. In an embodiment, the Cas9 molecule is an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

In an embodiment, a nucleic acid encodes a sequence that encodes a Cas9 molecule, e.g., a Cas9 molecule described herein. In an embodiment, a nucleic acid that encodes a Cas9 molecule is present on a nucleic acid molecule, e.g., a vector, e.g., a viral vector, e.g., an adeno-associated virus (AAV) vector. In an embodiment, the nucleic acid molecule is an AAV vector. Exemplary AAV vectors that may be used in any of the described compositions and methods include an AAV1 vector, a modified AAV1 vector, an AAV2 vector, a modified AAV2 vector, an AAV3 vector, an AAV4 vector, a modified AAV4 vector, an AAV5 vector, a modified AAV5 vector, a modified AAV3 vector, an AAV6 vector, a modified AAV6 vector, an AAV8 vector an AAV9 vector, an AAV.rh10 vector, a modified AAV.rh10 vector, an AAV.rh32/33 vector, a modified AAV.rh32/33 vector, an AAV.rh43 vector, a modified AAV.rh43 vector, an AAV.rh64R1 vector, and a modified AAV.rh64R1 vector.

In another aspect, disclosed herein is a composition comprising (e) a gRNA molecule comprising a targeting domain that is complementary with a target domain in the *CXCR4* gene, as described herein. The composition of (e) may further comprise (f) a Cas9 molecule, e.g., a Cas9 molecule as described herein. A composition of (e) and (f) may further comprise (g) a second, third and/or fourth gRNA molecule, e.g., a second, third and/or fourth gRNA molecule described herein. In an embodiment, the composition is a pharmaceutical composition. The compositions described herein, e.g., pharmaceutical compositions described herein, can be used in the treatment or prevention of HIV or AIDS in a subject, e.g., in accordance with a method disclosed herein.

In another aspect, disclosed herein is a method of altering a cell, e.g., altering the structure, e.g., altering the sequence, of a target nucleic acid of a cell, comprising contacting said cell with: (e) a gRNA that targets the *CXCR4* gene, e.g., a gRNA as described herein; (f) a Cas9 molecule, e.g., a Cas9 molecule as described herein; and optionally, (g) a second, third and/or fourth gRNA that targets *CXCR4* gene, e.g., a second, third and/or fourth gRNA as described herein.

In an embodiment, the method comprises contacting said cell with (e) and (f).

In an embodiment, the method comprises contacting said cell with (e), (f), and (g).

In an embodiment, the method comprises contacting a cell from a subject suffering from or likely to develop an HIV infection or AIDS. The cell may be from a subject who does not have a mutation at a *CXCR4* target position.

In an embodiment, the cell being contacted in the disclosed method is a target cell from a circulating blood cell, a progenitor cell, or a stem cell, e.g., a hematopoietic stem cell (HSC) or a hematopoietic stem/progenitor cell (HSPC). In an embodiment, the target cell is a T cell (e.g., a CD4+ T cell, a CD8+ T cell, a helper T cell, a regulatory T cell, a cytotoxic T cell, a memory T cell, a T cell precursor or a natural killer T cell), a B cell (e.g., a progenitor B cell, a Pre B cell, a Pro B cell, a memory B cell, a plasma B cell), a monocyte, a megakaryocyte, a neutrophil, an eosinophil, a basophil, a mast cell, a reticulocyte, a lymphoid progenitor cell, a myeloid progenitor cell, or a hematopoietic stem cell. In an embodiment, the target cell is a bone marrow cell, (e.g., a lymphoid progenitor cell, a myeloid progenitor cell, an erythroid progenitor cell, a hematopoietic stem cell, or a mesenchymal stem cell). In an embodiment, the cell is a CD4 cell, a T cell, a gut associated lymphatic tissue (GALT), a macrophage, a dendritic cell, a myeloid precursor cell, or a microglia. The contacting may be performed *ex vivo* and the contacted cell may be returned to the subject's body after the contacting step.

In an embodiment, the method of altering a cell as described herein comprises acquiring knowledge of the presence of a *CXCR4* target position in said cell, prior to the contacting step. Acquiring knowledge of the presence of a *CXCR4* target position in the cell may be by sequencing the *CXCR4* gene, or a portion of the *CXCR4* gene.

In an embodiment, the contacting step comprises contacting the cell with a nucleic acid, e.g., a vector, e.g., an AAV vector, e.g., an AAV1 vector, a modified AAV1 vector, an AAV2 vector, a modified AAV2 vector, an AAV3 vector, a modified AAV3 vector, an AAV4 vector, a modified AAV4 vector, an AAV5 vector, a modified AAV5 vector, an AAV6 vector, a modified AAV6 vector, an AAV7 vector, a modified AAV7 vector, an AAV8 vector, an AAV9 vector, an AAV.rh10 vector, a modified AAV.rh10 vector, an AAV.rh32/33 vector, a modified AAV.rh32/33 vector, an AAV.rh43vector, a modified AAV.rh43vector, an AAV.rh64R1vector, and a modified AAV.rh64R1vector.adescribed herein.

In an embodiment, the contacting step further comprises contacting the cell with an HSC self-renewal agonist, e.g., UM171 ((1r,4r)-N1-(2-benzyl-7-(2-methyl-2H-tetrazol-5-yl)-9H-pyrimido[4,5-b]indol-4-yl)cyclohexane-1,4-diamine) or a pyrimidoindole derivative described in Fares et al., Science, 2014, 345(6203): 1509-1512). In an embodiment, the cell is contacted with the HSC self-renewal agonist before (e.g., at least 1, 2, 4, 8, 12, 24, 36, or 48 hours before, e.g., about 2 hours before) the cell is contacted with a gRNA molecule and/or a Cas9 molecule. In another embodiment, the cell is contacted with the HSC self-renewal agonist after (e.g., at least 1, 2, 4, 8, 12, 24, 36, or 48 hours after, e.g., about 24 hours after) the cell is contacted with a gRNA molecule and/or a Cas9 molecule. In yet another embodiment, the cell is contacted with the HSC self-renewal agonist before (e.g., at least 1, 2, 4, 8, 12, 24, 36, or 48 hours before) and after (e.g., at least 1, 2, 4, 8, 12, 24, 36, or 48 hours after) the cell is contacted with a gRNA molecule and/or a Cas9 molecule. In an embodiment, the cell is contacted with the HSC self-renewal agonist about 2 hours before and about 24 hours after the cell is contacted with a gRNA molecule and/or a Cas9 molecule. In an embodiment, the cell is contacted with the HSC self-renewal agonist at the same time the cell is contacted with a gRNA molecule and/or a Cas9 molecule. In an embodiment, the HSC self-renewal agonist, e.g., UM171, is used at a concentration between 5 and 200 nM, e.g., between 10 and 100 nM or between 20 and 50 nM, e.g., about 40 nM.

In another aspect, disclosed herein is a cell or a population of cells produced (e.g., altered) by a method described herein.

In another aspect, disclosed herein is a method of treating a subject suffering from or likely to develop an HIV infection or AIDS, e.g., altering the structure, e.g., sequence, of a target nucleic acid of the subject, comprising contacting a cell from the subject with:
(e) a gRNA that targets the *CXCR4* gene, e.g., a gRNA disclosed herein;
(f) a Cas9 molecule, e.g., a Cas9 molecule disclosed herein; and
optionally, (g)(i) a second gRNA that targets the *CXCR4* gene, e.g., a second gRNA disclosed herein, and
further optionally, (g)(ii) a third gRNA, and still further optionally, (g)(iii) a fourth gRNA that target the *CXCR4* gene, e.g., a third and fourth gRNA disclosed herein.

In some embodiments, contacting comprises contacting with (e) and (f).

In some embodiments, contacting comprises contacting with (e), (f), and (g)(i).
In some embodiments, contacting comprises contacting with (e), (f), and (g) (i) and (g)(ii).
In some embodiments, contacting comprises contacting with (e), (f), and (g)(i), (g)(ii) and (g)(iii).

In an embodiment, the method comprises acquiring knowledge of the presence or absence of a mutation at a *CXCR4* target position in said subject.

In an embodiment, the method comprises acquiring knowledge of the presence or absence of a mutation at a *CXCR4* target position in said subject by sequencing the *CXCR4* gene or a portion of the *CXCR4* gene.

In an embodiment, the method comprises introducing a mutation at a *CXCR4* target position.

In an embodiment, the method comprises introducing a mutation at a *CXCR4* target position by NHEJ.

When the method comprises introducing a mutation at a *CXCR4* target position, e.g., by NHEJ in the coding region or a non-coding region, a Cas9 of (f) and at least one guide RNA (e.g., a guide RNA of (e)) are included in the contacting step.

In an embodiment, a cell of the subject is contacted *ex vivo* with (e), (f) and optionally (g)(i), further optionally (g)(ii), and still further optionally (g)(iii). In an embodiment, said cell is returned to the subject's body.

In another aspect, disclosed herein is a reaction mixture comprising a gRNA molecule, a nucleic acid, or a composition described herein, and a cell, e.g., a cell from a subject having, or likely to develop and HIV infection or AIDS, or a subject having a mutation at a *CXCR4* target position (e.g., a heterozygous carrier of a *CXCR4* mutation).

In another aspect, disclosed herein is a kit comprising, (e) a gRNA molecule described herein, or a nucleic acid that encodes the gRNA, and one or more of the following:
(f) a Cas9 molecule, e.g., a Cas9 molecule described herein, or a nucleic acid or mRNA that encodes the Cas9;
(g)(i) a second gRNA molecule, e.g., a second gRNA molecule described herein or a nucleic acid that encodes (g)(i);
(g)(ii) a third gRNA molecule, e.g., a third gRNA molecule described herein or a nucleic acid that encodes (g)(ii);
(g)(iii) a fourth gRNA molecule, e.g., a fourth gRNA molecule described herein or a nucleic acid that encodes (g)(iii).

In an embodiment, the kit comprises a nucleic acid, e.g., an AAV vector, that encodes one or more of (e), (f), (g)(i), (g)(ii), and (g)(iii).

In yet another aspect, disclosed herein is a gRNA molecule, e.g., a gRNA molecule described herein, for use in treating, or delaying the onset or progression of, HIV infection or AIDS in a subject, e.g., in accordance with a method of treating, or delaying the onset or progression of, HIV infection or AIDS as described herein.

In an embodiment, the gRNA molecule in used in combination with a Cas9 molecule, e.g., a Cas9 molecule described herein. Additionaly or alternatively, in an embodiment, the gRNA molecule is used in combination with a second, third and/or fouth gRNA molecule, e.g., a second, third and/or fouth gRNA molecule described herein.

In still another aspect, disclosed herein is use of a gRNA molecule, e.g., a gRNA molecule described herein, in the manufacture of a medicament for treating, or delaying the onset or progression of, HIV infection or AIDS in a subject, e.g., in accordance with a method of treating, or delaying the onset or progression of, HIV infection or AIDS as described herein.

In an embodiment, by multiplexing the alteration of both CCR5 and *CXCR4,* entry of the HIV virus into the host cells is reduced or prevented. Examplary multiplexing alterations of CCR5 and *CXCR4* gene are: introducing one or more mutations in the gene for C-C chemokine receptor type 5 (CCR5), e.g., by introducing a protective mutation (such as a CCR5 delta 32 mutation), and knocking out the *CXCR4* gene, introducing one or more mutations in the gene for C-C chemokine receptor type 5 (CCR5), e.g., by introducing a protective mutation (such as a CCR5 delta 32 mutation), and knocking down the *CXCR4* gene, knocking out both CCR5 and *CXCR4* genes, knocking down both CCR5 and *CXCR4* genes, knocking out the CCR5 gene and knocking down the *CXCR4* gene, or knocking down the CCR5 gene and knocking out the *CXCR4* gene.

In an embodiment, the medicament comprises a Cas9 molecule, e.g., a Cas9 molecule described herein. Additionally or alternatively, in an embodiment, the medicament comprises a second, third and/or fouth gRNA molecule, e.g., a second, third and/or fouth gRNA molecule described herein.

### Methods of Targeting CXCR4

As disclosed herein, the *CXCR4* gene can be targeted (e.g., altered) by gene editing, e.g., using CRISPR-Cas9 mediated methods as described herein.

Methods and compositions discussed herein, provide for targeting (e.g., altering) a *CXCR4* target position in the *CXCR4* gene. A *CXCR4* target position can be targeted (e.g., altered) by gene editing, e.g., using CRISPR-Cas9 mediated methods to target (e.g. alter) the *CXCR4* gene.

Disclosed herein are methods for targeting (e.g., altering) a *CXCR4* target position in the *CXCR4* gene. Targeting (e.g., altering) the *CXCR4* target position is achieved, e.g., by:
(3) knocking out the *CXCR4* gene:
   (3a) insertion or deletion (e.g., NHEJ-mediated insertion or deletion) of one or more nucleotides in close proximity to or within the early coding region of the *CXCR4* gene, or
   (3b) deletion (e.g., NHEJ-mediated deletion) of a genomic sequence including at least a portion of the *CXCR4* gene, or
(4) knocking down the *CXCR4* gene mediated by enzymatically inactive Cas9 (eiCas9) molecule or an eiCas9-fusion protein by targeting non-coding region, e.g., a promoter region, of the gene.

Methods below all give rise to targeting (e.g., alteration) of the *CXCR4* gene.

In one embodiment, methods described herein introduce one or more breaks near the early coding region in at least one allele of the *CXCR4* gene. In another embodiment, methods described herein introduce two or more breaks to flank at least a portion of the *CXCR4* gene. The two or more breaks remove (e.g., delete) a genomic sequence including at least a portion of the *CXCR4* gene. In another embodiment, methods described herein comprise knocking down the *CXCR4* gene mediated by enzymatically inactive Cas9 (eiCas9) molecule or an eiCas9-fusion protein by targeting the promoter region of *CXCR4* target knockdown position. Methods 3a, 3b and 4 described herein result in targeting (e.g., alteration) of the *CXCR4* gene.

The targeting (e.g., alteration) of the *CXCR4* gene can be mediated by any mechanism. Exemplary mechanisms that can be associated with the alteration of the *CXCR4* gene include, but are not limited to, non-homologous end joining (e.g., classical or alternative), microhomology-mediated end joining (MMEJ), homology-directed repair (e.g., endogenous donor template mediated), SDSA (synthesis dependent strand annealing), single strand annealing or single strand invasion.

### Knocking out CXCR4 by introducing an indel in the CXCR4 gene

In an embodiment, the method comprises introducing an insertion of one more nucleotides in close proximity to the *CXCR4* target knockout position (e.g., the early coding region) of the *CXCR4* gene. As described herein, in one embodiment, the method comprises the introduction of one or more breaks (e.g., single strand breaks or double strand breaks) sufficiently close to (e.g., either 5' or 3' to) the early coding region of the *CXCR4* target knockout position, such that the break-induced indel could be reasonably expected to span the *CXCR4* target knockout position (e.g., the early coding region). While not wishing to be bound by theory, it is believed that NHEJ-mediated repair of the break(s) allows for the NHEJ-mediated introduction of an indel in close proximity to within the early coding region of the *CXCR4* target knockout position.

In an embodiment, the method comprises introducing a deletion of a genomic sequence comprising at least a portion of the *CXCR4* gene. As described herein, in an embodiment, the method comprises the introduction of two double stand breaks - one 5' and the other 3' to (i.e., flanking) the *CXCR4* target position. In an embodiment, two gRNAs, e.g., unimolecular (or chimeric) or modular gRNA molecules, are configured to position the two double strand breaks on opposite sides of the *CXCR4* target knockout position in the *CXCR4* gene.

In an embodiment, a single strand break is introduced (e.g., positioned by one gRNA molecule) at or in close proximity to a *CXCR4* target position in the *CXCR4* gene. In an embodiment, a single gRNA molecule (e.g., with a Cas9 nickase) is used to create a single strand break at or in close proximity to the *CXCR4* target position, e.g., the gRNA is configured such that the single strand break is positioned either upstream (e.g., within 500 bp upstream, e.g., within 200 bp upstream) or downstream (e.g., within 500 bp downstream, e.g., within 200 bp downstream) of the *CXCR4* target position. In an embodiment, the break is positioned to avoid unwanted target chromosome elements, such as repeat elements, e.g., *an Alu* repeat.

In an embodiment, a double strand break is introduced (e.g., positioned by one gRNA molecule) at or in close proximity to a *CXCR4* target position in the *CXCR4* gene. In an embodiment, a single gRNA molecule (e.g., with a Cas9 nuclease other than a Cas9 nickase) is used to create a double strand break at or in close proximity to the *CXCR4* target position, e.g., the gRNA molecule is configured such that the double strand break is positioned either upstream (e.g., within 500 bp upstream, e.g., within 200 bp upstream) or downstream of (e.g., within 500 bp downstream, e.g., within 200 bp downstream) of a *CXCR4* target position. In an embodiment, the break is positioned to avoid unwanted target chromosome elements, such as repeat elements, e.g., an *Alu* repeat.

In an embodiment, two single strand breaks are introduced (e.g., positioned by two gRNA molecules) at or in close proximity to a *CXCR4* target position in the *CXCR4* gene. In an embodiment, two gRNA molecules (e.g., with one or two Cas9 nickcases) are used to create two single strand breaks at or in close proximity to the *CXCR4* target position, e.g., the gRNAs molecules are configured such that both of the single strand breaks are positioned e.g., within500 bp upstream, e.g., within 200 bp upstream) or downstream (e.g., within 500 bp downstream, e.g., within 200 bp downstream) of the *CXCR4* target position. In another embodiment, two gRNA molecules (e.g., with two Cas9 nickcases) are used to create two single strand breaks at or in close proximity to the *CXCR4* target position, e.g., the gRNAs molecules are configured such that one single strand break is positioned upstream (e.g., within 200 bp upstream) and a second single strand break is positioned downstream (e.g., within 200 bp downstream) of the *CXCR4* target position. In an embodiment, the breaks are positioned to avoid unwanted target chromosome elements, such as repeat elements, e.g., *an Alu* repeat.

In an embodiment, two double strand breaks are introduced (e.g., positioned by two gRNA molecules) at or in close proximity to a *CXCR4* target position in the *CXCR4* gene. In an embodiment, two gRNA molecules (e.g., with one or two Cas9 nucleases that are not Cas9 nickases) are used to create two double strand breaks to flank a *CXCR4* target position, e.g., the gRNA molecules are configured such that one double strand break is positioned upstream (e.g., within500 bp upstream, e.g., within 200 bp upstream) and a second double strand break is positioned downstream (e.g., within 500 bp downstream, e.g., within 200 bp downstream) of the *CXCR4* target position. In an embodiment, the breaks are positioned to avoid unwanted target chromosome elements, such as repeat elements, e.g., *an Alu* repeat.

In an embodiment, one double strand break and two single strand breaks are introduced (e.g., positioned by three gRNA molecules) at or in close proximity to a *CXCR4* target position in the *CXCR4* gene. In an embodiment, three gRNA molecules (e.g., with a Cas9 nuclease other than a Cas9 nickase and one or two Cas9 nickases) to create one double strand break and two single strand breaks to flank a *CXCR4* target position, e.g., the gRNA molecules are configured such that the double strand break is positioned upstream or downstream of (e.g., within 500 bp, e.g., within 200bp upstreamor downstream) of the *CXCR4* target position, and the two single strand breaks are positioned at the opposite site, e.g., downstream or upstrea m (e.g., within 500 bp, e.g., within 200 bp downstream or upstream), of the *CXCR4* target position. In an embodiment, the breaks are positioned to avoid unwanted target chromosome elements, such as repeat elements, e.g., *an Alu* repeat.

In an embodiment, four single strand breaks are introduced (e.g., positioned by four gRNA molecules) at or in close proximity to a *CXCR4* target position in the *CXCR4* gene. In an embodiment, four gRNA molecule (e.g., with one or more Cas9 nickases are used to create four single strand breaks to flank a *CXCR4* target position in the *CXCR4* gene, e.g., the gRNA molecules are configured such that a first and second single strand breaks are positioned upstream (e.g., within500 bp upstream, e.g., within 200 bp upstream) of the *CXCR4* target position, and a third and a fourth single stranded breaks are positioned downstream (e.g., within 500 bp downstream, e.g., within 200 bp downstream) of the *CXCR4* target position. In an embodiment, the breaks are positioned to avoid unwanted target chromosome elements, such as repeat elements, e.g., an *Alu* repeat.

In an embodiment, two or more (e.g., three or four) gRNA molecules are used with one Cas9 molecule. In another embodiment, when two ore more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

### Knocking out CXCR4 by deleting a genomic sequence including at least a portion of the CXCR4 gene

In an embodiment, the method comprises deleting (e.g., NHEJ-mediated deletion) a genomic sequence including at least a portion of the *CXCR4* gene. As described herein, in one embodiment, the method comprises the introduction two sets of breaks (e.g., a pair of double strand breaks, one double strand break or a pair of single strand breaks, or two pairs of single strand breaks) to flank a region of the *CXCR4* gene (e.g., a coding region, e.g., an early coding region, or a non-coding region, e.g., a non-coding sequence of the *CXCR4* gene, e.g., a promoter, an enhancer, an intron, a 3'UTR, and/or a polyadenylation signal). While not wishing to be bound by theory, it is believed that NHEJ-mediated repair of the break(s) allows for alteration of the *CXCR4* gene as described herein, which reduces or eliminates expression of the gene, e.g., to knock out one or both alleles of the *CXCR4* gene.

In an embodiment, two double strand breaks are introduced (e.g., positioned by two gRNA molecules) at or in close proximity to a *CXCR4* target position in the *CXCR4* gene. In an embodiment, two gRNA molecules (e.g., with one or two Cas9 nucleases that are not Cas9 nickases) are used to create two double strand breaks to flank a *CXCR4* target position, e.g., the gRNA molecules are configured such that one double strand break is positioned upstream (e.g., within 500 bp upstream, e.g., within 200 bp upstream) and a second double strand break is positioned downstream (e.g., within 500 bp downstream, e.g., within 200 bp downstream) of the *CXCR4* target position. In an embodiment, the breaks are positioned to avoid unwanted target chromosome elements, such as repeat elements, e.g., *an Alu* repeat.

In an embodiment, one double strand break and two single strand breaks are introduced (e.g., positioned by three gRNA molecules) at or in close proximity to a *CXCR4* target position in the *CXCR4* gene. In an embodiment, three gRNA molecules (e.g., with a Cas9 nuclease other than a Cas9 nickase and one or two Cas9 nickases) to create one double strand break and two single strand breaks to flank a *CXCR4* target position, e.g., the gRNA molecules are configured such that the double strand break is positioned upstream or downstream of (e.g., within 500 bp, e.g., within 200bp upstreamor downstream) of the *CXCR4* target position, and the two single strand breaks are positioned at the opposite site, e.g., downstream or upstrea m (e.g., within 500 bp, e.g., within 200 bp downstream or upstream), of the *CXCR4* target position. In an embodiment, the breaks are positioned to avoid unwanted target chromosome elements, such as repeat elements, e.g., *an Alu* repeat.

In an embodiment, four single strand breaks are introduced (e.g., positioned by four gRNA molecules) at or in close proximity to a *CXCR4* target position in the *CXCR4* gene. In an embodiment, four gRNA molecule (e.g., with one or more Cas9 nickases are used to create four single strand breaks to flank a *CXCR4* target position in the *CXCR4* gene, e.g., the gRNA molecules are configured such that a first and second single strand breaks are positioned upstream (e.g., within500 bp upstream, e.g., within 200 bp upstream) of the *CXCR4* target position, and a third and a fourth single stranded breaks are positioned downstream (e.g., within500 bp downstream, e.g., within 200 bp downstream) of the *CXCR4* target position. In an embodiment, the breaks are positioned to avoid unwanted target chromosome elements, such as repeat elements, e.g., *an Alu* repeat.

In an embodiment, two or more (e.g., three or four) gRNA molecules are used with one Cas9 molecule. In another embodiment, when two ore more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

### Knocking down CXCR4 mediated by an enzymatically inactive Cas9 (eiCas9) molecule

A targeted knockdown approach reduces or eliminates expression of functional *CXCR4* gene product. As described herein, in an embodiment, a targeted knockdown is mediated by targeting an enzymatically inactive Cas9 (eiCas9) molecule or an eiCas9 fused to a transcription repressor domain or chromatin modifying protein to alter transcription, e.g., to block, reduce, or decrease transcription, of the *CXCR4* gene.

Methods and compositions discussed herein may be used to alter the expression of the *CXCR4* gene to treat or prevent HIV infection or AIDS by targeting a promoter region of the *CXCR4* gene. In an embodiment, the promoter region is targeted to knock down expression of the *CXCR4* gene. A targeted knockdown approach reduces or eliminates expression of functional *CXCR4* gene product. As described herein, in an embodiment, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *CXCR4* gene.

In an embodiment, one or more eiCas9s may be used to block binding of one or more endogenous transcription factors. In another embodiment, an eiCas9 can be fused to a chromatin modifying protein. Altering chromatin status can result in decreased expression of the target gene. One or more eiCas9s fused to one or more chromatin modifying proteins may be used to alter chromatin status.

### Multiplexing alteration of two or more genes

The alteration, of two or more genes in the same cell or cells is referred to herein as "multiplexing". Multiplexing constitutes the modification of at least two genes in the same cell or cells.

When two or more genes (e.g., CCR5 and CXCR4) are targeted for alteration, the two or more genes (e.g., CCR5 and CXCR4) may be altered sequentially or simultaneously. In an embodiment, the alteration of the *CXCR4* gene is prior to the alteration of the *CCR5* gene. In an embodiment, the alteration of the *CXCR4* gene is concurrent with the alteration of the *CCR5* gene. In an embodiment, the alteration of the *CXCR4* gene is subsequent to the alteration of the *CCR5* gene. In an embodiment, the effect of the alterations is synergistic. In an embodiment, the two or more genes (e.g., CCR5 and CXCR4) are altered sequentially in order to reduce the probability of introducing genomic rearrangements (e.g., translocations) involving the two target positions.

### VII. Delivery of Components to Target Cells, Formulations

The components, e.g., Cas9 molecules, gRNA molecules (e.g., Cas9 molecule/gRNA molecule complexes), and optionally donor template nucleic acids, can be introduced into target cells in a variety of forms using a variety of delivery methods and formulations, see, e.g., **Tables 700** and **800.** In an embodiment, one Cas9 molecule and two or more (e.g., 2, 3, 4, or more) different gRNA molecules are delivered. When a Cas9 is encoded as DNA for delivery, the DNA may typically but not necessarily include a control region, e.g., comprising a promoter, to effect expression. In an embodiment, the promoter is a constitutive promoter. Useful promoters for Cas9 molecule sequences include, e.g., CMV, EF-1a, EFS, MSCV, PGK, or CAG promoters. In an embodiment,the promoter is a constitutive promoter. Useful promoters for gRNAs include H1, 7SK, tRNA, and U6 promoters. Promoters with similar or dissimilar strengths can be selected to tune the expression of components. Sequences encoding a Cas9 molecule can comprise a nuclear localization signal (NLS), e.g., an SV40 NLS. In an embodiment, the sequence or sequences encoding a Cas9 molecule comprises at least two nuclear localization signals. In an embodiment a promoter for a Cas9 molecule may be inducible or cell specific.

**Table 700** provides examples of how the components can be delivered to a target cell.

**Table 700**

| **Elements** | | | |
|---|---|---|---|
| **Cas9 Molecule(s)** | **gRNA molecule(s)** | **Donor Template** | **Comments** |
| DNA | RNA | DNA | In this embodiment, a Cas9 molecule, typically an eaCas9 molecule, is transcribed from DNA, and a gRNA is provided as *in vitro* transcribed or synthesized RNA. In an embodiment, multiple Cas9 molecules (e.g., 2 or 3 or 4), are transcribed from DNA, and multiple gRNAs (e.g., 2 or 3 or 4 or more) are provided as *in vitro* transcribed or synthesized RNA. In this embodiment, the donor template is provided as a separate DNA molecule. |
| mRNA | RNA | DNA | In this embodiment, a Cas9 molecule, typically an eaCas9 molecule, is translated from *in vitro* transcribed mRNA, and a gRNA is provided as *in vitro* transcribed or synthesized RNA. In an embodiment, multiple Cas9 molecules (e.g., 2 or 3 or 4), are translated from *in vitro* transcribed mRNA, and multiple gRNAs (e.g., 2 or 3 or 4 or more) are provided as *in vitro* transcribed or synthesized RNA. In this embodiment, the donor template is provided on the same DNA molecule that encodes the Cas9. |
| Protein | RNA | DNA | In this embodiment, an eaCas9 molecule is provided as a protein, and a gRNA is provided as transcribed or synthesized RNA. In an embodiment, multiple Cas9 molecules (e.g., 2 or 3 or 4), are provided as a protein, and multiple gRNAs (e.g., 2 or 3 or 4 or more) are provided as transcribed or synthesized RNA. In this embodiment, the donor template is provided as a DNA molecule. |

**Table 800** summarizes various delivery methods for the components of a Cas system, e.g., the Cas9 molecule component or components, the gRNA molecule component or components, and/or the donor template component, as described herein.

**Table 800**

| **Delivery Vector/Mode** | | **Delivery into Non-Divi ding Cells** | **Duration of Expression** | **Genome Integration** | **Type of Molecule Delivered** |
|---|---|---|---|---|---|
| **Physical (e.g., electroporation, particle gun, Calcium Phosphate transfection, cell compression or squeezing)** | | YES | Transient | NO | Nucleic Acids and Proteins |
| ***Viral*** | **Retrovirus** | NO | Stable | YES | RNA |
| | **Lentivirus** | YES | Stable | YES/NO with | RNA |
| | | | | modifications | |
| | **Adenovirus** | YES | Transient | NO | DNA |
| | **Adeno-Associat ed Virus (AAV)** | YES | Stable | NO | DNA |
| | **Vaccinia Virus** | YES | Very Transient | NO | DNA |
| | **Herpes Simplex Virus** | YES | Stable | NO | DNA |
| ***Non-Viral*** | **Cationic Liposomes** | YES | Transient | Depends on what is delivered | Nucleic Acids and Proteins |
| | **Polymeric Nanoparticles** | YES | Transient | Depends on what is delivered | Nucleic Acids and Proteins |
| ***Biological Non-Viral Delivery Vehicles*** | **Attenuated Bacteria** | YES | Transient | NO | Nucleic Acids |
| | **Engineered Bacteriophages** | YES | Transient | NO | Nucleic Acids |
| | **Mammalian Virus-like Particles** | YES | Transient | NO | Nucleic Acids |
| | **Biological liposomes: Erythrocyte Ghosts and** | YES | Transient | NO | Nucleic Acids |
| | **Exosomes** | | | | |

### DNA-based Delivery of a Cas9 molecule and/or donor template

Nucleic acids encoding Cas9 molecules (e.g., eaCas9 molecules) and/or a donor template nucleic acid, or any combination (e.g., two or all) thereof, can be delivered into cells by art-known methods or as described herein. For example, Cas9-encoding DNA, as well as donor template nucleic acids, can be delivered, e.g., by vectors (e.g., viral or non-viral vectors), non-vector based methods (e.g., using naked DNA or DNA complexes), or a combination thereof.

DNA encoding Cas9 molecules (e.g., eaCas9 molecules) can be conjugated to molecules (e.g., N-acetylgalactosamine) promoting uptake by the target cells (e.g., the target cells described herein).

Donor template molecules can be conjugated to molecules to promote uptake by the target cells (e.g., the target cells described herein). In some embodiments, the Cas9-encoding DNA is delivered by a vector (e.g., viral vector/virus or plasmid).

A vector can comprise a sequence that encodes a Cas9 molecule. A vector can also comprise a sequence encoding a signal peptide (e.g., for nuclear localization, nucleolar localization, mitochondrial localization), fused, e.g., to a Cas9 molecule sequence. For example, a vector can comprise a nuclear localization sequence (e.g., from SV40) fused to the sequence encoding the Cas9 molecule.

One or more regulatory/control elements, e.g., a promoter, an enhancer, an intron, a polyadenylation signal, a Kozak consensus sequence, internal ribosome entry sites (IRES), a 2A sequence, and splice acceptor or donor can be included in the vectors. In some embodiments, the promoter is recognized by RNA polymerase II (e.g., a CMV promoter). In other embodiments, the promoter is recognized by RNA polymerase III (e.g., a U6 promoter). In some embodiments, the promoter is a regulated promoter (e.g., inducible promoter). In other embodiments, the promoter is a constitutive promoter. In some embodiments, the promoter is a viral promoter. In other embodiments, the promoter is a non-viral promoter.

In some embodiments, the vector or delivery vehicle is a viral vector (e.g., for generation of recombinant viruses). In some embodiments, the virus is a DNA virus (e.g., dsDNA or ssDNA virus). In other embodiments, the virus is an RNA virus (e.g., an ssRNA virus). Exemplary viral vectors/viruses include, e.g., retroviruses, lentiviruses, adenovirus, adeno-associated virus (AAV), vaccinia viruses, poxviruses, and herpes simplex viruses.

In some embodiments, the virus infects dividing cells. In other embodiments, the virus infects non-dividing cells. In some embodiments, the virus infects both dividing and non-dividing cells. In some embodiments, the virus can integrate into the host genome. In some embodiments, the virus is replication-competent. In other embodiments, the virus is replication-defective, e.g., having one or more coding regions for the genes necessary for additional rounds of virion replication and/or packaging replaced with other genes or deleted. In some embodiments, the virus causes transient expression of the Cas9 molecule or molecules. In other embodiments, the virus causes long-lasting, e.g., at least 1 week, 2 weeks, 1 month, 2 months, 3 months, 6 months, 9 months, 1 year, 2 years, or permanent expression, of the Cas9 molecule or molecules. The packaging capacity of the viruses may vary, e.g., from at least about 4 kb to at least about 30 kb, e.g., at least about 5 kb, 10 kb, 15 kb, 20 kb, 25 kb, 30 kb, 35 kb, 40 kb, 45 kb, or 50 kb.

In an embodiment, the viral vector recognizes a specific cell type. For example, the viral vector can be pseudotyped with a different/alternative viral envelope glycoprotein; engineered with a cell type-specific receptor (e.g., genetic modification(s) of one or more viral envelope glycoproteins to incorporate a targeting ligand such as a peptide ligand, a single chain antibody, or a growth factor); and/or engineered to have a molecular bridge with dual specificities with one end recognizing a viral glycoprotein and the other end recognizing a moiety of the target cell surface (e.g., a ligand-receptor, monoclonal antibody, avidin-biotin and chemical conjugation).

Exemplary viral vectors/viruses include, e.g., retroviruses, lentiviruses, adenovirus, adeno-associated virus (AAV), vaccinia viruses, poxviruses, and herpes simplex viruses.

In some embodiments, the Cas9-encoding DNA is delivered by a recombinant retrovirus. In some embodiments, the donor template nucleic acid is delivered by a recombinant retrovirus. In some embodiments, the retrovirus (e.g., Moloney murine leukemia virus) comprises a reverse transcriptase, e.g., that allows integration into the host genome. In some embodiments, the retrovirus is replication-competent. In other embodiments, the retrovirus is replication-defective, e.g., having one of more coding regions for the genes necessary for additional rounds of virion replication and packaging replaced with other genes, or deleted.

In some embodiments, the Cas9-encoding DNA is delivered by a recombinant lentivirus. For example, the lentivirus is replication-defective, e.g., does not comprise one or more genes required for viral replication. For example, the lentivirus is replication-defective, e.g., does not comprise one or more genes required for viral replication.

In some embodiments, the Cas9-encoding DNA is delivered by a recombinant adenovirus. In some embodiments, the donor template nucleic acid is delivered by a recombinant adenovirus.

In some embodiments, the Cas9-encoding DNA is delivered by a recombinant AAV. In some embodiments, the donor template nucleic acid is delivered by a recombinant AAV. In some embodiments, the AAV does not incorporate its geneome into that of a host cell, e.g., a target cell as describe herein. In some embodiments, the AAV can incorporate at least part of its genome into that of a host cell, e.g., a target cell as described herein. In some embodiments, the AAV is a self-complementary adeno-associated virus (scAAV), e.g., a scAAV that packages both strands which anneal together to form double stranded DNA. AAV serotypes that may be used in the disclosed methods, include AAV1, AAV2, modified AAV2 (e.g., modifications at Y444F, Y500F, Y730F and/or S662V), AAV3, modified AAV3 (e.g., modifications at Y705F, Y731F and/or T492V), AAV4, AAV5, AAV6, modified AAV6 (e.g., modifications at S663V and/or T492V), AAV8, AAV 8.2, AAV9, AAV rh 10, and pseudotyped AAV, such as AAV2/8, AAV2/5 and AAV2/6 can also be used in the disclosed methods. In an embodiment, an AAV capsid that can be used in the methods described herein is a capsid sequence from serotype AAV1, AAV2, AAV3, AAV4, AAV5, AAV6, AAV7, AAV8, AAV9, AAV.rh8, AAV.rh10, AAV.rh32/33, AAV.rh43, AAV.rh64R1, or AAV7m8.

In an embodiment, the Cas9-encoding DNA is delivered in a re-engineered AAV capsid, e.g., with 50% or greater, e.g., 60% or greater, 70% or greater, 80% or greater, 90% or greater, or 95% or greater, sequence homology with a capsid sequence from serotypes AAV1, AAV2, AAV3, AAV4, AAV5, AAV6, AAV7, AAV8, AAV9, AAV.rh8, AAV.rh10, AAV.rh32/33, AAV.rh43, or AAV.rh64R1.

In an embodiment, the Cas9-encoding DNA is delivered by a chimeric AAV capsid. In some embodiments, the donor template nucleic acid is delivered by a chimeric AAV capsid. Exemplary chimeric AAV capsids include, but are not limited to, AAV9i1, AAV2i8, AAV-DJ, AAV2G9, AAV2i8G9, or AAV8G9.

In an embodiment, the AAV is a self-complementary adeno-associated virus (scAAV), e.g., a scAAV that packages both strands which anneal together to form double stranded DNA.

In some embodiments, the Cas9-encoding DNA is delivered by a hybrid virus, e.g., a hybrid of one or more of the viruses described herein. In an embodiment, the hybrid virus is hybrid of an AAV (e.g., of any AAV serotype), with a Bocavirus, B19 virus, porcine AAV, goose AAV, feline AAV, canine AAV, or MVM

A packaging cell is used to form a virus particle that is capable of infecting a target cell. Such a cell includes a 293 cell, which can package adenovirus, and a ψ2 cell or a PA317 cell, which can package retrovirus. A viral vector used in gene therapy is usually generated by a producer cell line that packages a nucleic acid vector into a viral particle. The vector typically contains the minimal viral sequences required for packaging and subsequent integration into a host or target cell (if applicable), with other viral sequences being replaced by an expression cassette encoding the protein to be expressed, e.g., components for a Cas9 molecule, e.g., two Cas9 components. For example, an AAV vector used in gene therapy typically only possesses inverted terminal repeat (ITR) sequences from the AAV genome which are required for packaging and gene expression in the host or target cell. The missing viral functions can be supplied in trans by the packaging cell line and/or plasmid containing E2A, E4, and VA genes from adenovirus, and plasmid encoding *Rep* and *Cap* genes from AAV, as described in "Triple Transfection Protocol." Henceforth, the viral DNA is packaged in a cell line, which contains a helper plasmid encoding the other AAV genes, namely rep and cap, but lacking ITR sequences. In embodiment, the viral DNA is packaged in a producer cell line, which contains E1A and/or E1B genes from adenovirus. The cell line is also infected with adenovirus as a helper. The helper virus (e.g., adenovirus or HSV) or helper plasmid promotes replication of the AAV vector and expression of AAV genes from the helper plasmid with ITRs. The helper plasmid is not packaged in significant amounts due to a lack of ITR sequences. Contamination with adenovirus can be reduced by, e.g., heat treatment to which adenovirus is more sensitive than AAV.

In an embodiment, the viral vector has the ability of cell type recognition. For example, the viral vector can be pseudotyped with a different/alternative viral envelope glycoprotein; engineered with a cell type-specific receptor (e.g., genetic modification of the viral envelope glycoproteins to incorporate targeting ligands such as a peptide ligand, a single chain antibody, a growth factor); and/or engineered to have a molecular bridge with dual specificities with one end recognizing a viral glycoprotein and the other end recognizing a moiety of the target cell surface (e.g., ligand-receptor, monoclonal antibody, avidin-biotin and chemical conjugation).

In an embodiment, the viral vector achieves cell type specific expression. For example, a promoter can be constructed to restrict expression of the transgene (Cas 9) in only a specific target cell. The specificity of the vector can also be mediated by microRNA-dependent control of transgene expression. In an embodiment, the viral vector has increased efficiency of fusion of the viral vector and a target cell membrane. For example, a fusion protein such as fusion-competent hemagglutinin (HA) can be incorporated to increase viral uptake into cells. In an embodiment, the viral vector has the ability of nuclear localization. For example, a virus that requires the breakdown of the cell wall (during cell division) and therefore will not infect a non-diving cell can be altered to incorporate a nuclear localization peptide in the matrix protein of the virus thereby enabling the transduction of non-proliferating cells.

In some embodiments, the Cas9-encoding DNA is delivered by a non-vector based method (e.g., using naked DNA or DNA complexes). For example, the DNA can be delivered, e.g., by organically modified silica or silicate (Ormosil), electroporation, transient cell compression or squeezing (e.g., as described in Lee, et al, 2012, Nano Lett 12: 6322-27), gene gun, sonoporation, magnetofection, lipid-mediated transfection, dendrimers, inorganic nanoparticles, calcium phosphates, or a combination thereof.

In an embodiment, delivery via electroporation comprises mixing the cells with the Cas9-encoding DNA in a cartridge, chamber or cuvette and applying one or more electrical impulses of defined duration and amplitude. In an embodiment, delivery via electroporation is performed using a system in which cells are mixed with the Cas9-encoding DNA in a vessel connected to a device (e.g., a pump) which feeds the mixture into a cartridge, chamber or cuvette wherein one or more electrical impulses of defined duration and amplitude are applied, after which the cells are delivered to a second vessel.

In some embodiments, the Cas9-encoding DNA is delivered by a combination of a vector and a non-vector based method. In some embodiments, the donor template nucleic acid is delivered by a combination of a vector and a non-vector based method. For example, a virosome comprises a liposome combined with an inactivated virus (e.g., HIV or influenza virus), which can result in more efficient gene transfer, e.g., in a respiratory epithelial cell than either a viral or a liposomal method alone.

In an embodiment, the delivery vehicle is a non-viral vector. In an embodiment, the non-viral vector is an inorganic nanoparticle. Exemplary inorganic nanoparticles include, e.g., magnetic nanoparticles (e.g., Fe₃MnO₂) and silica. The outer surface of the nanoparticle can be conjugated with a positively charged polymer (e.g., polyethylenimine, polylysine, polyserine) which allows for attachment (e.g., conjugation or entrapment) of payload. In an embodiment, the non-viral vector is an organic nanoparticle (e.g., entrapment of the payload inside the nanoparticle). Exemplary organic nanoparticles include, e.g., SNALP liposomes that contain cationic lipids together with neutral helper lipids which are coated with polyethylene glycol (PEG) and protamine and nucleic acid complex coated with lipid coating.

Exemplary lipids for gene transfer are shown below in **Table 900.**

**Table 900. Lipids Used for Gene Transfer**

| **Lipid** | **Abbreviation** | **Feature** |
|---|---|---|
| 1,2-Dioleoyl-sn-glycero-3-phosphatidylcholine | DOPC | Helper |
| 1,2-Dioleoyl-sn-glycero-3-phosphatidylethanolamine | DOPE | Helper |
| Cholesterol | | Helper |
| *N*-[1-(2,3-Dioleyloxy)prophyl]*N,N,N*-trimethylammonium chloride | DOTMA | Cationic |
| 1,2-Dioleoyloxy-3-trimethylammonium-propane | DOTAP | Cationic |
| Dioctadecylamidoglycylspermine | DOGS | Cationic |
| *N-*(3-Aminopropyl)-*N,N-*dimethyl-2,3-bis(dodecyloxy)-1-propana minium bromide | GAP-DLRIE | Cationic |
| Cetyltrimethylammonium bromide | CTAB | Cationic |
| 6-Lauroxyhexyl ornithinate | LHON | Cationic |
| 1-(2,3-Dioleoyloxypropyl)-2,4,6-trimethylpyridinium | 2Oc | Cationic |
| 2,3-Dioleyloxy-*N*-[2(sperminecarboxamido-ethyl]-*N,N*-dimethyl-1-propanaminium trifluoroacetate | DOSPA | Cationic |
| 1,2-Dioleyl-3-trimethylammonium-propane | DOPA | Cationic |
| *N*-(2-Hydroxyethyl)-*N,N*-dimethyl-2,3-bis(tetradecyloxy)-1-propa naminium bromide | MDRIE | Cationic |
| Dimyristooxypropyl dimethyl hydroxyethyl ammonium bromide | DMRI | Cationic |
| 3β-[*N*(*N',N'*-Dimethylaminoethane)-carbamoyl]cholesterol | DC-Chol | Cationic |
| Bis-guanidium-tren-cholesterol | BGTC | Cationic |
| 1,3-Diodeoxy-2-(6-carboxy-spermyl)-propylamide | DOSPER | Cationic |
| Dimethyloctadecylammonium bromide | DDAB | Cationic |
| Dioctadecylamidoglicylspermidin | DSL | Cationic |
| rac-[(2,3-Dioctadecyloxypropyl)(2-hydroxyethyl)]-dimethylamm onium chloride | CLIP-1 | Cationic |
| rac-[2(2,3-Dihexadecyloxypropyl-oxymethyloxy)ethyl]trimethyla mmonium bromide | CLIP-6 | Cationic |
| Ethyldimyristoylphosphatidylcholine | EDMPC | Cationic |
| 1,2-Distearyloxy-*N,N*-dimethyl-3-aminopropane | DSDMA | Cationic |
| 1,2-Dimyristoyl-trimethylammonium propane | DMTAP | Cationic |
| *O,O*'-Dimyristyl-N-lysyl aspartate | DMKE | Cationic |
| 1,2-Distearoyl-sn-glycero-3-ethylphosphocholine | DSEPC | Cationic |
| *N*-Palmitoyl D-erythro-sphingosyl carbamoyl-spermine | CCS | Cationic |
| *N*-*t*-Butyl-*N*0-tetradecyl-3-tetradecylaminopropionamidine | diC14-amidine | Cationic |
| Octadecenolyoxy[ethyl-2-heptadecenyl-3 hydroxyethyl] imidazolinium chloride | DOTIM | Cationic |
| *N*1-Cholesterloxycarbonyl-3,7-diazanonane-1,9-diamine | CDAN | Cationic |
| 2-(3-[Bis(3-amino-propyl)-amino]propylamino)-*N-*ditetradecylcar bamoylme-ethyl-acetamide | RPR209120 | Cationic |
| 1,2-dilinoleyloxy-3-dimethylaminopropane | DLinDMA | Cationic |
| 2,2-dilinoleyl-4-dimethylaminoethyl-[1,3]-dioxolane | DLin-KC2-DM A | Cationic |
| dilinoleyl-methyl-4-dimethylaminobutyrate | DLin-MC3-DM A | Cationic |

Exemplary polymers for gene transfer are shown below in **Table 1000.**

**Table 1000. Polymers Used for Gene Transfer**

| **Polymer** | **Abbreviation** |
|---|---|
| Poly(ethylene)glycol | PEG |
| Polyethylenimine | PEI |
| Dithiobis(succinimidylpropionate) | DSP |
| Dimethyl-3,3'-dithiobispropionimidate | DTBP |
| Poly(ethylene imine) biscarbamate | PEIC |
| Poly(L-lysine) | PLL |
| Histidine modified PLL | |
| Poly(*N*-vinylpyrrolidone) | PVP |
| Poly(propylenimine) | PPI |
| Poly(amidoamine) | PAMAM |
| Poly(amido ethylenimine) | SS-PAEI |
| Triethylenetetramine | TETA |
| Poly(β-aminoester) | |
| Poly(4-hydroxy-L-proline ester) | PHP |
| Poly(allylamine) | |
| Poly(α-[4-aminobutyl]-L-glycolic acid) | PAGA |
| Poly(D,L-lactic-co-glycolic acid) | PLGA |
| Poly(*N*-ethyl-4-vinylpyridinium bromide) | |
| Poly(phosphazene)s | PPZ |
| Poly(phosphoester)s | PPE |
| Poly(phosphoramidate)s | PPA |
| Poly(*N*-2-hydroxypropylmethacrylamide) | pHPMA |
| Poly (2-(dimethylamino)ethyl methacrylate) | pDMAEMA |
| Poly(2-aminoethyl propylene phosphate) | PPE-EA |
| Chitosan | |
| Galactosylated chitosan | |
| *N*-Dodacylated chitosan | |
| Histone | |
| Collagen | |
| Dextran-spermine | D-SPM |

In an embodiment, the vehicle has targeting modifications to increase target cell update of nanoparticles and liposomes, e.g., cell specific antigens, monoclonal antibodies, single chain antibodies, aptamers, polymers, sugars, and cell penetrating peptides. In an embodiment, the vehicle uses fusogenic and endosome-destabilizing peptides/polymers. In an embodiment, the vehicle undergoes acid-triggered conformational changes (e.g., to accelerate endosomal escape of the cargo). In an embodiment, a stimuli-cleavable polymer is used, e.g., for release in a cellular compartment. For example, disulfide-based cationic polymers that are cleaved in the reducing cellular environment can be used.

In an embodiment, the delivery vehicle is a biological non-viral delivery vehicle. In an embodiment, the vehicle is an attenuated bacterium (e.g., naturally or artificially engineered to be invasive but attenuated to prevent pathogenesis and expressing the transgene, e.g., *Listeria monocytogenes,* certain *Salmonella strains, Bifidobacterium longum,* and modified *Escherichia coli*). In an embodiment, the vehicle is a genetically modified bacteriophage (e.g., engineered phages having large packaging capacity, less immunogenic, containing mammalian plasmid maintenance sequences and having incorporated targeting ligands). In an embodiment, the vehicle is a mammalian virus-like particle. For example, modified viral particles can be generated (e.g., by purification of the "empty" particles followed by *ex vivo* assembly of the virus with the desired cargo). The vehicle can also be engineered to incorporate targeting ligands to alter cell type specificity. In an embodiment, the vehicle is a biological liposome. For example, the biological liposome is a phospholipid-based particle derived from human cells (e.g., erythrocyte ghosts, which are red blood cells broken down into spherical structures derived from the subject, or secretory exosomes -subject- (e.g., patient) derived membrane-bound nanovescicle (30 -100 nm) of endocytic origin (e.g., can be produced from various cell types and can therefore be taken up by cells without the need of targeting ligands).

In an embodiment, one or more nucleic acid molecules (e.g., DNA molecules) other than the components of a Cas system, e.g., the Cas9 molecule component or components described herein, are delivered. In an embodiment, the nucleic acid molecule is delivered at the same time as one or more of the components of the Cas system are delivered. In an embodiment, the nucleic acid molecule is delivered before or after (e.g., less than about 30 minutes, 1 hour, 2 hours, 3 hours, 6 hours, 9 hours, 12 hours, 1 day, 2 days, 3 days, 1 week, 2 weeks, or 4 weeks) one or more of the components of the Cas system are delivered. In an embodiment, the nucleic acid molecule is delivered by a different means than one or more of the components of the Cas system, e.g., the Cas9 molecule component, are delivered. The nucleic acid molecule can be delivered by any of the delivery methods described herein. For example, the nucleic acid molecule can be delivered by a viral vector, e.g., an integration-deficient lentivirus, and the Cas9 molecule component or components can be delivered by electroporation, e.g., such that the toxicity caused by nucleic acids (e.g., DNAs) can be reduced. In an embodiment, the nucleic acid molecule encodes a therapeutic protein, e.g., a protein described herein. In an embodiment, the nucleic acid molecule encodes an RNA molecule, e.g., an RNA molecule described herein.

### Delivery of RNA encoding a Cas9 molecule and/or gRNA molecule

RNA encoding Cas9 molecules (e.g., eaCas9 molecules or eiCas9 molecules) and/or gRNA molecules, can be delivered into cells, e.g., target cells described herein, by art-known methods or as described herein. For example, Cas9-encoding and/or gRNA-encoding RNA can be delivered, e.g., by microinjection, electroporation, transient cell compression or squeezing (e.g., as described in Lee, et al., 2012, Nano Lett 12: 6322-27), lipid-mediated transfection, peptide-mediated delivery, or a combination thereof. Cas9-encoding and/or gRNA-encoding RNA can be conjugated to molecules to promote uptake by the target cells (e.g., target cells described herein).

In an embodiment, delivery via electroporation comprises mixing the cells with the RNA encoding Cas9 molecules (e.g., eaCas9 molecules, eiCas9 molecules or eiCas9 fusion proteins) and/or gRNA molecules in a cartridge, chamber or cuvette and applying one or more electrical impulses of defined duration and amplitude. In an embodiment, delivery via electroporation is performed using a system in which cells are mixed with the RNA encoding Cas9 molecules (e.g., eaCas9 molecules, eiCas9 molecules or eiCas9 fusion protiens) and/or gRNA molecules in a vessel connected to a device (e.g., a pump) which feeds the mixture into a cartridge, chamber or cuvette wherein one or more electrical impulses of defined duration and amplitude are applied, after which the cells are delivered to a second vessel. Cas9-encoding RNA can be conjugated to molecules to promote uptake by the target cells (e.g., target cells described herein).

### Delivery of Cas9 molecule protein

Cas9 protein molecules (e.g., eaCas9 molecules, eiCas9 molecules or eiCas9 fusion proteins) can be delivered into cells by art-known methods or as described herein. For example, Cas9 protein molecules can be delivered, e.g., by microinjection, electroporation, transient cell compression or squeezing (e.g., as described in Lee, et al [2012] Nano Lett 12: 6322-27), lipid-mediated transfection, peptide-mediated delivery, or a combination thereof. Delivery can be accompanied by a gRNA. In some embodiments, Cas9 protein can be conjugated to molecules promoting uptake by the target cells (e.g., target cells described herein).

In an embodiment, delivery via electroporation comprises mixing the cells with the Cas9 molecules (e.g., eaCas9 molecules, eiCas9 molecules or eiCas9 fusion proteins) with or without gRNA molecules in a cartridge, chamber or cuvette and applying one or more electrical impulses of defined duration and amplitude. In an embodiment, delivery via electroporation is performed using a system in which cells are mixed with the Cas9 molecules (e.g., eaCas9 molecules, eiCas9 molecules or eiCas9 fusion proteins) with or without gRNA molecules in a vessel connected to a device (e.g., a pump) which feeds the mixture into a cartridge, chamber or cuvette wherein one or more electrical impulses of defined duration and amplitude are applied, after which the cells are delivered to a second vessel.

### Ex vivo delivery

In some embodiments, components described in **Table 700** are introduced into cells which are then introduced into the subject, e.g., cells are removed from a subject, manipulated *ex vivo* and then introduced into the subject. Methods of introducing the components can include, e.g., any of the delivery methods described in **Table 800.**

### VIII. Modified Nucleosides, Nucleotides, and Nucleic Acids

Modified nucleosides and modified nucleotides can be present in nucleic acids, e.g., particularly gRNA, but also nucleic acids encoding a Cas9 molecule. Modifications disclosed in this section can be made in addition to or instead of any of the specific gRNA molecule modifications described above. As described herein, "nucleoside" is defined as a compound containing a five-carbon sugar molecule (a pentose or ribose) or derivative thereof, and an organic base, purine or pyrimidine, or a derivative thereof. As described herein, "nucleotide" is defined as a nucleoside further comprising a phosphate group.

Modified nucleosides and nucleotides can include one or more of:
(i) alteration, e.g., replacement, of one or both of the non-linking phosphate oxygens and/or of one or more of the linking phosphate oxygens in the phosphodiester backbone linkage;
(ii) alteration, e.g., replacement, of a constituent of the ribose sugar, e.g., of the 2' hydroxyl on the ribose sugar;
(iii) wholesale replacement of the phosphate moiety with "dephospho" linkers;
(iv) modification or replacement of a naturally occurring nucleobase;
(v) replacement or modification of the ribose-phosphate backbone;
(vi) modification of the 3' end or 5' end of the oligonucleotide, e.g., removal, modification or replacement of a terminal phosphate group or conjugation of a moiety; and
(vii) modification of the sugar.

The modifications listed above can be combined to provide modified nucleosides and nucleotides that can have two, three, four, or more modifications. For example, a modified nucleoside or nucleotide can have a modified sugar and a modified nucleobase. In an embodiment, every base of a gRNA is modified, e.g., all bases have a modified phosphate group, e.g., all are phosphorothioate groups. In an embodiment, all, or substantially all, of the phosphate groups of a unimolecular or modular gRNA molecule are replaced with phosphorothioate groups.

In an embodiment, modified nucleotides, e.g., nucleotides having modifications as described herein, can be incorporated into a nucleic acid, e.g., a "modified nucleic acid." In some embodiments, the modified nucleic acids comprise one, two, three or more modified nucleotides. In some embodiments, at least 5% (e.g., at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) of the positions in a modified nucleic acid are a modified nucleotides.

Unmodified nucleic acids can be prone to degradation by, e.g., cellular nucleases. For example, nucleases can hydrolyze nucleic acid phosphodiester bonds. Accordingly, in one aspect the modified nucleic acids described herein can contain one or more modified nucleosides or nucleotides, e.g., to introduce stability toward nucleases.

### Definitions of Chemical Groups

As used herein, "alkyl" is meant to refer to a saturated hydrocarbon group which is straight-chained or branched. Example alkyl groups include methyl (Me), ethyl (Et), propyl (*e.g.,* n-propyl and isopropyl), butyl (*e.g.,* n-butyl, isobutyl, t-butyl), pentyl (*e.g.,* n-pentyl, isopentyl, neopentyl), and the like. An alkyl group can contain from 1 to about 20, from 2 to about 20, from 1 to about 12, from 1 to about 8, from 1 to about 6, from 1 to about 4, or from 1 to about 3 carbon atoms.

As used herein, "aryl" refers to monocyclic or polycyclic (e.g., having 2, 3 or 4 fused rings) aromatic hydrocarbons such as, for example, phenyl, naphthyl, anthracenyl, phenanthrenyl, indanyl, indenyl, and the like. In some embodiments, aryl groups have from 6 to about 20 carbon atoms.

As used herein, "alkenyl" refers to an aliphatic group containing at least one double bond.

As used herein, "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl.

As used herein, "arylalkyl" or "aralkyl" refers to an alkyl moiety in which an alkyl hydrogen atom is replaced by an aryl group. Aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups.

As used herein, "cycloalkyl" refers to a cyclic, bicyclic, tricyclic, or polycyclic non-aromatic hydrocarbon groups having 3 to 12 carbons. Examples of cycloalkyl moieties include, but are not limited to, cyclopropyl, cyclopentyl, and cyclohexyl.

As used herein, "heterocyclyl" refers to a monovalent radical of a heterocyclic ring system. Representative heterocyclyls include, without limitation, tetrahydrofuranyl, tetrahydrothienyl, pyrrolidinyl, pyrrolidonyl, piperidinyl, pyrrolinyl, piperazinyl, dioxanyl, dioxolanyl, diazepinyl, oxazepinyl, thiazepinyl, and morpholinyl.

As used herein, "heteroaryl" refers to a monovalent radical of a heteroaromatic ring system. Examples of heteroaryl moieties include, but are not limited to, imidazolyl, oxazolyl, thiazolyl, triazolyl, pyrrolyl, furanyl, indolyl, thiophenyl pyrazolyl, pyridinyl, pyrazinyl, pyridazinyl, pyrimidinyl, indolizinyl, purinyl, naphthyridinyl, quinolyl, and pteridinyl. In some embodiments, a heteroaryl is a 6-membered heteroaryl. In some embodiments a 6-membered heteroaryl is pyridinyl, pyrazinyl, pyridazinyl, or pyrimidinyl. In some embodiments, a 6-membered heteroaryl is optionally substituted with one, two or three C₁₋₄ alkyl and/or halogen groups.

### Phosphate Backbone Modifications

### The Phosphate Group

In some embodiments, the phosphate group of a modified nucleotide can be modified by replacing one or more of the oxygens with a different substituent. Further, the modified nucleotide, e.g., modified nucleotide present in a modified nucleic acid, can include the wholesale replacement of an unmodified phosphate moiety with a modified phosphate as described herein. In some embodiments, the modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. In some embodiments, one of the non-bridging phosphate oxygen atoms in the phosphate backbone moiety can be replaced by any of the following groups: sulfur (S), selenium (Se), BR₃ (wherein R can be, e.g., hydrogen, alkyl, or aryl), C (e.g., an alkyl group, an aryl group, and the like), H, NR₂ (wherein R can be, e.g., hydrogen, alkyl, or aryl), or OR (wherein R can be, e.g., alkyl or aryl). The phosphorous atom in an unmodified phosphate group is achiral. However, replacement of one of the non-bridging oxygens with one of the above atoms or groups of atoms can render the phosphorous atom chiral; that is to say that a phosphorous atom in a phosphate group modified in this way is a stereogenic center. The stereogenic phosphorous atom can possess either the "R" configuration (herein Rp) or the "S" configuration (herein Sp).

Phosphorodithioates have both non-bridging oxygens replaced by sulfur. The phosphorus center in the phosphorodithioates is achiral which precludes the formation of oligoribonucleotide diastereomers. In some embodiments, modifications to one or both non-bridging oxygens can also include the replacement of the non-bridging oxygens with a group independently selected from S, Se, B, C, H, N, and OR (R can be, e.g., alkyl or aryl).

The phosphate linker can also be modified by replacement of a bridging oxygen, (i.e., the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

### Replacement of the Phosphate Group

The phosphate group can be replaced by non-phosphorus containing connectors. In some embodiments, the charge phosphate group can be replaced by a neutral moiety.

Examples of moieties which can replace the phosphate group can include, without limitation, e.g., methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

### Replacement of the Ribophosphate Backbone

Scaffolds that can mimic nucleic acids can also be constructed wherein the phosphate linker and ribose sugar are replaced by nuclease resistant nucleoside or nucleotide surrogates. In some embodiments, the nucleobases can be tethered by a surrogate backbone. Examples can include, without limitation, the morpholino, cyclobutyl, pyrrolidine and peptide nucleic acid (PNA) nucleoside surrogates.

### Sugar Modifications

The modified nucleosides and modified nucleotides can include one or more modifications to the sugar group. For example, the 2' hydroxyl group (OH) can be modified or replaced with a number of different "oxy" or "deoxy" substituents. In some embodiments, modifications to the 2' hydroxyl group can enhance the stability of the nucleic acid since the hydroxyl can no longer be deprotonated to form a 2'-alkoxide ion. The 2'-alkoxide can catalyze degradation by intramolecular nucleophilic attack on the linker phosphorus atom.

Examples of "oxy"-2' hydroxyl group modifications can include alkoxy or aryloxy (OR, wherein "R" can be, e.g., alkyl, cycloalkyl, aryl, aralkyl, heteroaryl or a sugar); polyethyleneglycols (PEG), O(CH₂CH₂O)ₙCH₂CH₂OR wherein R can be, e.g., H or optionally substituted alkyl, and n can be an integer from 0 to 20 (e.g., from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). In some embodiments, the "oxy"-2' hydroxyl group modification can include "locked" nucleic acids (LNA) in which the 2' hydroxyl can be connected, e.g., by a C₁₋₆ alkylene or C₁₋₆ heteroalkylene bridge, to the 4' carbon of the same ribose sugar, where exemplary bridges can include methylene, propylene, ether, or amino bridges; O-amino (wherein amino can be, e.g., NH₂; alkylamino, dialkylamino, heterocyclylamino, arylamino, diarylamino, heteroarylamino, or diheteroarylamino, ethylenediamine, or polyamino) and aminoalkoxy, O(CH₂)ₙ-amino, (wherein amino can be, e.g., NH₂; alkylamino, dialkylamino, heterocyclylamino, arylamino, diarylamino, heteroarylamino, or diheteroarylamino, ethylenediamine, or polyamino). In some embodiments, the "oxy"-2' hydroxyl group modification can include the methoxyethyl group (MOE), (OCH₂CH₂OCH₃, e.g., a PEG derivative).

"Deoxy" modifications can include hydrogen (i.e. deoxyribose sugars, e.g., at the overhang portions of partially ds RNA); halo (e.g., bromo, chloro, fluoro, or iodo); amino (wherein amino can be, e.g., NH₂; alkylamino, dialkylamino, heterocyclylamino, arylamino, diarylamino, heteroarylamino, diheteroarylamino, or amino acid); NH(CH₂CH₂NH)ₙCH₂CH₂-amino (wherein amino can be, e.g., as described herein), -NHC(O)R (wherein R can be, e.g., alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino as described herein.

The sugar group can also contain one or more carbons that possess the opposite stereochemical configuration than that of the corresponding carbon in ribose. Thus, a modified nucleic acid can include nucleotides containing e.g., arabinose, as the sugar. The nucleotide "monomer" can have an alpha linkage at the 1' position on the sugar, e.g., alpha-nucleosides. The modified nucleic acids can also include "abasic" sugars, which lack a nucleobase at C-1'. These abasic sugars can also be further modified at one or more of the constituent sugar atoms. The modified nucleic acids can also include one or more sugars that are in the L form, e.g. L-nucleosides.

Generally, RNA includes the sugar group ribose, which is a 5-membered ring having an oxygen. Exemplary modified nucleosides and modified nucleotides can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). In some embodiments, the modified nucleotides can include multicyclic forms (e.g., tricyclo; and "unlocked" forms, such as glycol nucleic acid (GNA) (e.g., R-GNA or S-GNA, where ribose is replaced by glycol units attached to phosphodiester bonds), threose nucleic acid (TNA, where ribose is replaced with α-L-threofuranosyl-(3'→2')).

### Modifications on the Nucleobase

The modified nucleosides and modified nucleotides described herein, which can be incorporated into a modified nucleic acid, can include a modified nucleobase. Examples of nucleobases include, but are not limited to, adenine (A), guanine (G), cytosine (C), and uracil (U). These nucleobases can be modified or wholly replaced to provide modified nucleosides and modified nucleotides that can be incorporated into modified nucleic acids. The nucleobase of the nucleotide can be independently selected from a purine, a pyrimidine, a purine or pyrimidine analog. In some embodiments, the nucleobase can include, for example, naturally-occurring and synthetic derivatives of a base.

### Uracil

In some embodiments, the modified nucleobase is a modified uracil. Exemplary nucleobases and nucleosides having a modified uracil include without limitation pseudouridine (ψ), pyridin-4-one ribonucleoside, 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho⁵U), 5-aminoallyl-uridine, 5-halo-uridine (e.g., 5-iodo-uridine or 5-bromo-uridine), 3-methyl-uridine (m³U), 5-methoxy-uridine (mo⁵U), uridine 5-oxyacetic acid (cmo⁵U), uridine 5-oxyacetic acid methyl ester (mcmo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s2U), 5-aminomethyl-2-thio-uridine (nm⁵s2U), 5-methylaminomethyl-uridine (mnm⁵U), 5-methylaminomethyl-2-thio-uridine (mnm⁵s2U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm⁵s2U), 5-propynyl-uridine, 1-propynyl-pseudouridine, 5-taurinomethyl-uridine (τcm⁵U), 1-taurinomethyl-pseudouridine, 5-taurinomethyl-2-thio-uridine(τm⁵s2U), 1-taurinomethyl-4-thio-pseudouridine, 5-methyl-uridine (m⁵U, i.e., having the nucleobase deoxythymine), 1-methyl-pseudouridine (m¹ψ), 5-methyl-2-thio-uridine (m⁵s2U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ψ), 5-(isopentenylaminomethyl)uridine (inm⁵U), 5-(isopentenylaminomethyl)-2-thio-uridine (inm⁵s2U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s2Um), 5-methoxycarbonylmethyl-2'-O-methyl-uridine (mcm ⁵Um), 5-carbamoylmethyl-2'-O-methyl-uridine (ncm ⁵Um), 5-carboxymethylaminomethyl-2'-O-methyl-uridine (cmnm ⁵Um), 3,2'-O-dimethyl-uridine (m³Um), 5-(isopentenylaminomethyl)-2'-O-methyl-uridine (inm ⁵Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, pyrazolo[3,4-d]pyrimidines, xanthine, and hypoxanthine.

### Cytosine

In some embodiments, the modified nucleobase is a modified cytosine. Exemplary nucleobases and nucleosides having a modified cytosine include without limitation 5-aza-cytidine, 6-aza-cytidine, pseudoisocytidine, 3-methyl-cytidine (m³C), N4-acetyl-cytidine (act), 5-formyl-cytidine (f⁵C), N4-methyl-cytidine (m⁴C), 5-methyl-cytidine (m⁵C), 5-halo-cytidine (e.g., 5-iodo-cytidine), 5-hydroxymethyl-cytidine (hm⁵C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k²C), α-thio-cytidine, 2'-O-methyl-cytidine (Cm), 5,2'-O-dimethyl-cytidine (m⁵Cm), N4-acetyl-2'-O-methyl-cytidine (ac⁴Cm), N4,2'-O-dimethyl-cytidine (m⁴Cm), 5-formyl-2'-O-methyl-cytidine (f⁵Cm), N4,N4,2'-O-trimethyl-cytidine (m⁴₂Cm), 1-thio-cytidine, 2'-F-ara-cytidine, 2'-F-cytidine, and 2'-OH-ara-cytidine.

### Adenine

In some embodiments, the modified nucleobase is a modified adenine. Exemplary nucleobases and nucleosides having a modified adenine include without limitation 2-amino-purine, 2,6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenosine, 7-deaza-8-aza-adenosine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m¹A), 2-methyl-adenosine (m²A), N6-methyl-adenosine (m⁶A), 2-methylthio-N6-methyl-adenosine (ms2m⁶A), N6-isopentenyl-adenosine (i⁶A), 2-methylthio-N6-isopentenyl-adenosine (ms²i⁶A), N6-(cis-hydroxyisopentenyl)adenosine (io⁶A), 2-methylthio-N6-(cis-hydroxyisopentenyl)adenosine (ms2io⁶A), N6-glycinylcarbamoyl-adenosine (g⁶A), N6-threonylcarbamoyl-adenosine (t⁶A), N6-methyl-N6-threonylcarbamoyl-adenosine (m⁶t⁶A), 2-methylthio-N6-threonylcarbamoyl-adenosine (ms²g⁶A), N6,N6-dimethyl-adenosine (m⁶₂A), N6-hydroxynorvalylcarbamoyl-adenosine (hn⁶A), 2-methylthio-N6-hydroxynorvalylcarbamoyl-adenosine (ms2hn⁶A), N6-acetyl-adenosine (ac⁶A), 7-methyl-adenosine, 2-methylthio-adenosine, 2-methoxy-adenosine, α-thio-adenosine, 2'-O-methyl-adenosine (Am), N⁶,2'-O-dimethyl-adenosine (m⁶Am), N⁶-Methyl-2'-deoxyadenosine, N6,N6,2'-O-trimethyl-adenosine (m⁶₂Am), 1,2'-O-dimethyl-adenosine (m¹Am), 2'-O-ribosyladenosine (phosphate) (Ar(p)), 2-amino-N6-methyl-purine, 1-thio-adenosine, 8-azido-adenosine, 2'-F-ara-adenosine, 2'-F-adenosine, 2'-OH-ara-adenosine, and N6-(19-amino-pentaoxanonadecyl)-adenosine.

### Guanine

In some embodiments, the modified nucleobase is a modified guanine. Exemplary nucleobases and nucleosides having a modified guanine include without limitation inosine (I), 1-methyl-inosine (m¹I), wyosine (imG), methylwyosine (mimG), 4-demethyl-wyosine (imG-14), isowyosine (imG2), wybutosine (yW), peroxywybutosine (o₂yW), hydroxywybutosine (OHyW), undermodified hydroxywybutosine (OHyW*), 7-deaza-guanosine, queuosine (Q), epoxyqueuosine (oQ), galactosyl-queuosine (galQ), mannosyl-queuosine (manQ), 7-cyano-7-deaza-guanosine (preQ₀), 7-aminomethyl-7-deaza-guanosine (preQ₁), archaeosine (G⁺), 7-deaza-8-aza-guanosine, 6-thio-guanosine, 6-thio-7-deaza-guanosine, 6-thio-7-deaza-8-aza-guanosine, 7-methyl-guanosine (m⁷G), 6-thio-7-methyl-guanosine, 7-methyl-inosine, 6-methoxy-guanosine, 1-methyl-guanosine (m'G), N2-methyl-guanosine (m²G), N2,N2-dimethyl-guanosine (m²₂G), N2,7-dimethyl-guanosine (m²,7G), N2, N2,7-dimethyl-guanosine (m²,2,7G), 8-oxo-guanosine, 7-methyl-8-oxo-guanosine, 1-methyl-6-thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m²Gm), N2,N2-dimethyl-2'-O-methyl-guanosine (m²₂Gm), 1-methyl-2'-O-methyl-guanosine (m'Gm), N2,7-dimethyl-2'-O-methyl-guanosine (m²,7Gm), 2'-O-methyl-inosine (Im), 1,2'-O-dimethyl-inosine (m'Im), O⁶-phenyl-2'-deoxyinosine, 2'-O-ribosylguanosine (phosphate) (Gr(p)), 1-thio-guanosine, O⁶-methyl-guanosine, O⁶-Methyl-2'-deoxyguanosine, 2'-F-ara-guanosine, and 2'-F-guanosine.

### miRNA binding sites

microRNAs (or miRNAs) are naturally occurring cellular 19-25 nucleotide long noncoding RNAs. They bind to nucleic acid molecules having an appropriate miRNA binding site, e.g., in the 3' UTR of an mRNA, and down-regulate gene expression. While not wishing to be bound by theory it is believed that the down regulation is either by reducing nucleic acid molecule stability or by inhibiting translation. An RNA species disclosed herein, e.g., an mRNA encoding Cas9 can comprise an miRNA binding site, e.g., in its 3'UTR. The miRNA binding site can be selected to promote down regulation of expression is a selected cell type. By way of example, the incorporation of a binding site for miR-122, a microRNA abundant in liver, can inhibit the expression of a gene in the liver.

### XI. Governing gRNA molecules and the use thereof to limit the activity of a Cas9 system

Methods and compositions that use, or include, a nucleic acid, e.g., DNA, that encodes a Cas9 molecule or a gRNA molecule, can, in addition, use or include a "governing gRNA molecule." The governing gRNA can limit the activity of the other CRISPR/Cas components introduced into a cell. In an embodiment, a gRNA molecule comprises a targeting domain that is complementary to a target domain on a nucleic acid that comprises a sequence that encodes a component of the CRISPR/Cas system that is introduced into a cell. In an embodiment, a governing gRNA molecule comprises a targeting domain that is complementary with a target sequence on: (a) a nucleic acid that encodes a Cas9 molecule; (b) a nucleic acid that encodes a gRNA which comprises a targeting domain that targets the gene (a target gene gRNA); or on more than one nucleic acid that encodes a CRISPR/Cas component, e.g., both (a) and (b). The governing gRNA molecule can complex with the Cas9 molecule to inactivate a component of the system. In an embodiment, a Cas9 molecule/governing gRNA molecule complex inactivates a nucleic acid that comprises the sequence encoding the Cas9 molecule. In an embodiment, a Cas9 molecule/governing gRNA molecule complex inactivates the nucleic acid that comprises the sequence encoding a target gene gRNA molecule. In an embodiment, a Cas9 molecule/governing gRNA molecule complex places temporal, level of expression, or other limits, on activity of the Cas9 molecule/target gene gRNA molecule complex. In an embodiment, a Cas9 molecule/governing gRNA molecule complex reduces off-target or other unwanted activity. In an embodiment, a governing gRNA molecule targets the coding sequence, or a control region, e.g., a promoter, for the CRISPR/Cas system component to be negatively regulated. For example, a governing gRNA can target the coding sequence for a Cas9 molecule, or a control region, e.g., a promoter, that regulates the expression of the Cas9 molecule coding sequence, or a sequence disposed between the two. In an embodiment, a governing gRNA molecule targets the coding sequence, or a control region, e.g., a promoter, for a target gene gRNA. In an embodiment, a governing gRNA, e.g., a Cas9-targeting or target gene gRNA-targeting, governing gRNA molecule, or a nucleic acid that encodes it, is introduced separately, e.g., later, than is the Cas9 molecule or a nucleic acid that encodes it. For example, a first vector, e.g., a viral vector, e.g., an AAV vector, can introduce nucleic acid encoding a Cas9 molecule, and a second vector, e.g., a viral vector, e.g., an AAV vector, can introduce nucleic acid encoding a governing gRNA molecule, e.g., a Cas9-targeting or target gene gRNA targeting, gRNA molecule. In an embodiment, the second vector can be introduced after the first. In other embodiments, a governing gRNA molecule, e.g., a Cas9-targeting or target gene gRNA targeting, governing gRNA molecule, or a nucleic acid that encodes it, can be introduced together, e.g., at the same time or in the same vector, with the Cas9 molecule or a nucleic acid that encodes it, but, e.g., under transcriptional control elements, e.g., a promoter or an enhancer, that are activated at a later time, e.g., such that after a period of time the transcription of Cas9 is reduced. In an embodiment, the transcriptional control element is activated intrinsically. In an embodiment, the transcriptional element is activated via the introduction of an external trigger.

Typically a nucleic acid sequence encoding a governing gRNA molecule, e.g., a Cas9-targeting gRNA molecule, is under the control of a different control region, e.g., promoter, than is the component it negatively modulates, e.g., a nucleic acid encoding a Cas9 molecule. In an embodiment, "different control region" refers to simply not being under the contol of one control region, e.g., promoter, that is functionally coupled to both controlled sequences. In an embodiment, different refers to "different control region" in kind or type of control region. For example, the sequence encoding a governing gRNA molecule, e.g., a Cas9-targeting gRNA molecule, is under the control of a control region, e.g., a promoter, that has a lower level of expression, or is expressed later than the sequence which encodes is the component it negatively modulates, e.g., a nucleic acid encoding a Cas9 molecule.

By way of example, a sequence that encodes a governing gRNA molecule, e.g., a Cas9-targeting governing gRNA molecule, can be under the control of a control region (e.g., a promoter) described herein, e.g., human U6 small nuclear promoter, or human HI promoter. In an embodiment, a sequence that encodes the component it negatively regulates, e.g., a nucleic acid encoding a Cas9 molecule, can be under the control of a control region (e.g., a promoter) described herein, e.g., CMV, EF-1a, EFS, MSCV, PGK, CAG control promoters.

### EXAMPLES

The following Examples are merely illustrative and are not intended to limit the scope or content of the invention in any way.

### Example 1: Cloning and Initial Screening of gRNAs for Cas9 molecules

The suitability of candidate gRNAs can be evaluated as described in this example. Although described for a chimeric gRNA, the approach can also be used to evaluate modular gRNAs.

### Cloning gRNAs into vectors

For each gRNA, a pair of overlapping oligonucleotides is designed and obtained. Oligonucleotides are annealed and ligated into a digested vector backbone containing an upstream U6 promoter and the remaining sequence of a long chimeric gRNA. Plasmid is sequence-verified and prepped to generate sufficient amounts of transfection-quality DNA. Alternate promoters may be used for *in vitro* transcription (e.g., a T7 promoter including modified T7 promoter as described herein where one or more of the 3' terminal Gs have been removed).

### Cloning gRNAs in linear dsDNA molecule (STITCHR)

For each gRNA, a single oligonucleotide is designed and obtained. The U6 promoter and the gRNA scaffold (e.g., including everything except the targeting domain, e.g., including sequences derived from the crRNA and tracrRNA, e.g., including a first complementarity domain; a linking domain; a second complementarity domain; a proximal domain; and a tail domain) are separately PCR amplified and purified as dsDNA molecules. The gRNA-specific oligonucleotide is used in a PCR reaction to stitch together the U6 and the gRNA scaffold, linked by the targeting domain specified in the oligonucleotide. Resulting dsDNA molecule (STITCHR product) is purified for transfection. Alternate promoters may be used to drive *in vitro* transcription (e.g., T7 promoter). Any gRNA scaffold may be used to create gRNAs compatible with Cas9 molecules from any bacterial species.

### Initial gRNA Screen

Each gRNA to be tested is transfected, along with a plasmid expressing Cas9 and a small amount of a GFP-expressing plasmid into human cells. In preliminary experiments, these cells can be immortalized human cell lines such as 293T, K562 or U2OS. Alternatively, primary human cells may be used. In this case, cells may be relevant to the eventual therapeutic cell target (for example, an erythroid cell). The use of primary cells similar to the potential therapeutic target cell population may provide important information on gene targeting rates in the context of endogenous chromatin and gene expression.

Transfection may be performed using lipid transfection (such as Lipofectamine or Fugene) or by electroporation (such as Lonza Nucleofection). Following transfection, GFP expression can be determined either by fluorescence microscopy or by flow cytometry to confirm consistent and high levels of transfection. These preliminary transfections can comprise different gRNAs and different targeting approaches (17-mers, 20-mers, nuclease, dual-nickase, etc.) to determine which gRNAs/combinations of gRNAs give the greatest activity.

Efficiency of cleavage with each gRNA may be assessed by measuring NHEJ-induced indel formation at the target locus by a T7E1-type assay or by sequencing. Alternatively, other mismatch-sensitive enzymes, such as Cell/Surveyor nuclease, may also be used.

For the T7E1 assay, PCR amplicons are approximately 500-700bp with the intended cut site placed asymmetrically in the amplicon. Following amplification, purification and size-verification of PCR products, DNA is denatured and re-hybridized by heating to 95°C and then slowly cooling. Hybridized PCR products are then digested with T7 Endonuclease I (or other mismatch-sensitive enzyme) which recognizes and cleaves non-perfectly matched DNA. If indels are present in the original template DNA, when the amplicons are denatured and re-annealed, this results in the hybridization of DNA strands harboring different indels and therefore lead to double-stranded DNA that is not perfectly matched. Digestion products may be visualized by gel electrophoresis or by capillary electrophoresis. The fraction of DNA that is cleaved (density of cleavage products divided by the density of cleaved and uncleaved) may be used to estimate a percent NHEJ using the following equation: %NHEJ = (1-(1-fraction cleaved)^{½}). The T7E1 assay is sensitive down to about 2-5% NHEJ.

Sequencing may be used instead of, or in addition to, the T7E1 assay. For Sanger sequencing, purified PCR amplicons are cloned into a plasmid backbone, transformed, miniprepped and sequenced with a single primer. Sanger sequencing may be used for determining the exact nature of indels after determining the NHEJ rate by T7E1.

Sequencing may also be performed using next generation sequencing techniques. When using next generation sequencing, amplicons may be 300-500bp with the intended cut site placed asymmetrically. Following PCR, next generation sequencing adapters and barcodes (for example Illumina multiplex adapters and indexes) may be added to the ends of the amplicon, e.g., for use in high throughput sequencing (for example on an Illumina MiSeq). This method allows for detection of very low NHEJ rates.

### Example 2: Assessment of Gene Targeting by NHEJ

The gRNAs that induce the greatest levels of NHEJ in initial tests can be selected for further evaluation of gene targeting efficiency. In this case, cells are derived from disease subjects and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency to generate the desired mutations (either knockout of a target gene or removal of a target sequence motif) may be determined by sequencing. For Sanger sequencing, PCR amplicons may be 500-700bp long. For next generation sequencing, PCR amplicons may be 300-500bp long. If the goal is to knockout gene function, sequencing may be used to assess what percent of alleles have undergone NHEJ-induced indels that result in a frameshift or large deletion or insertion that would be expected to destroy gene function. If the goal is to remove a specific sequence motif, sequencing may be used to assess what percent of alleles have undergone NHEJ-induced deletions that span this sequence.

### Example 3: Assessing effect of gRNA modifications on T cell viability

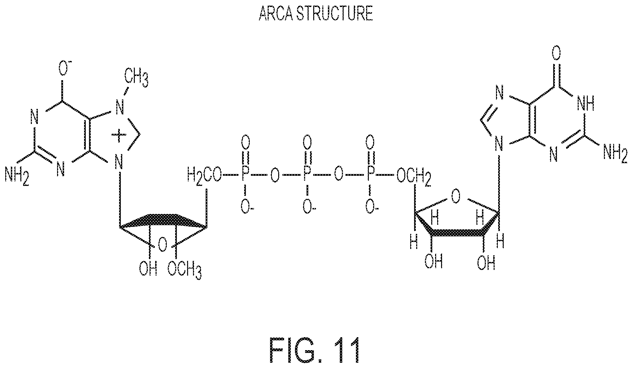

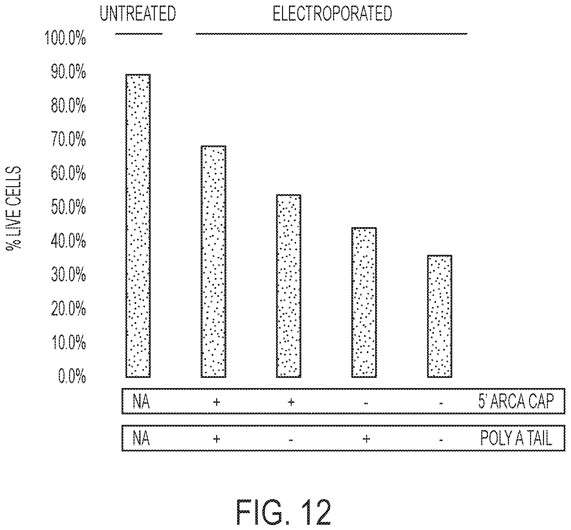

In order to assess how gRNA modifications affect T cell viability, *S. pyogenes* Cas9 mRNA was delivered to Jurkat T cells in combination with an *AAVS1* gRNA with or without modification. Specifically, 4 different combinations of modification were analyzed, (1) gRNA with a 5' Anti-Reverse Cap Analog (ARCA) cap (see **Fig. 11**) and polyA tail, (2) gRNA with a 5' ARCA cap only, (3) gRNA with a polyA tail only, (4) gRNA without any modification. In order to generate all of four of the aforemention versions of modified gRNAs, a DNA template comprising a T7 promoter, *AAVS1* gRNA target sequence (GUCCCCUCCACCCCACAGUG) (SEQ ID NO:387), and *S. pyogenes* tracr sequence. For all gRNAs, T7 polymerase was used to generate the gRNA in the presence of 7.5 mM UTP, 7.5 mM GTP, 7.5 mM CTP and 7.5 mM ATP. In order to modify the gRNAs with a 5' ARCA cap, 6.0 mM of ARCA analog was added to the NTP pool. Consequently, only 1.5 mM of GTP was added while the rest of the NTP pool remained at the same concentration: 7.5 mM UTP, 7.5 mM CTP and 7.5 mM ATP. In order to add a polyA tail to the gRNAs a recombinant polyA polymerase that was purified from *E. coli* was used to add a series of As to the end of the transcribed gRNA after the *in vitro* polymerase reaction was terminated. Termination was achieved by eliminating the DNA template with DNase I. The polyA tail reaction was performed for approximately 40 minutes. Regardless of the gRNA modification, all gRNA preparations were purified by phenol:choroform extraction followed by isopropanol precipitation. Once the gRNAs were generated, Jurkat T cells were electroporated with *S*. *pyogenes* Cas9 mRNA (modified with a 5' ARCA cap and polyA tail) and one of the 4 different modified *AAVS1*-specific gRNAs. Following electroporation cell viability was determined by performing an Annexin-V and propidium iodide double stain. The fraction of live cells, which do not stain for Annexin-V and PI was determined by flow cytometry. The results are quantified in **Fig. 12****.** Based on the fraction of live cells, it is concluded that gRNAs that have been modified with both a 5' ARCA cap and polyA tail are the least toxic to Jurkat T cells when introduced by electroporation.

### Example 4: Enzymatic synthesis and delivery of gRNAs to primary T cells

### Delivery of Cas9 mRNA and gRNA as RNA molecules to T cells

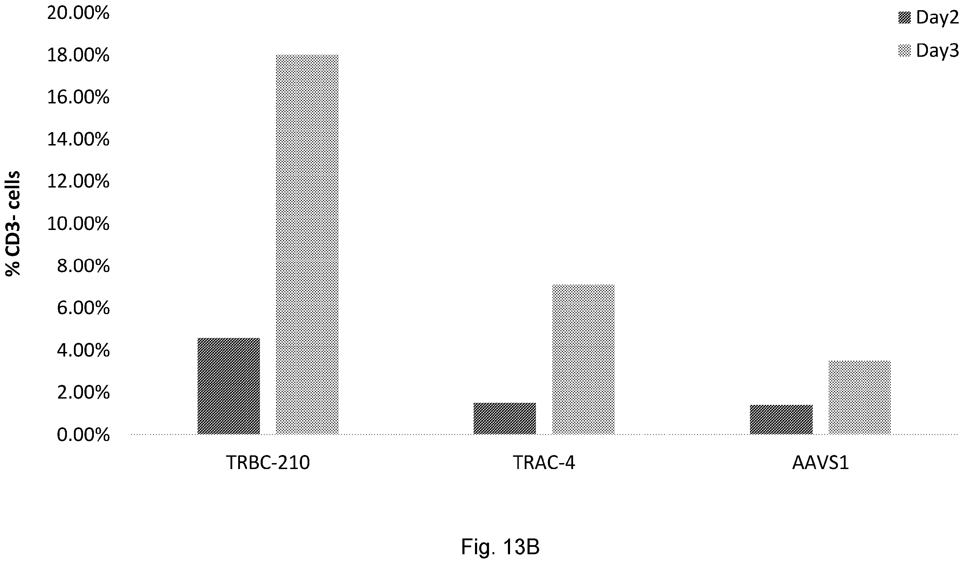

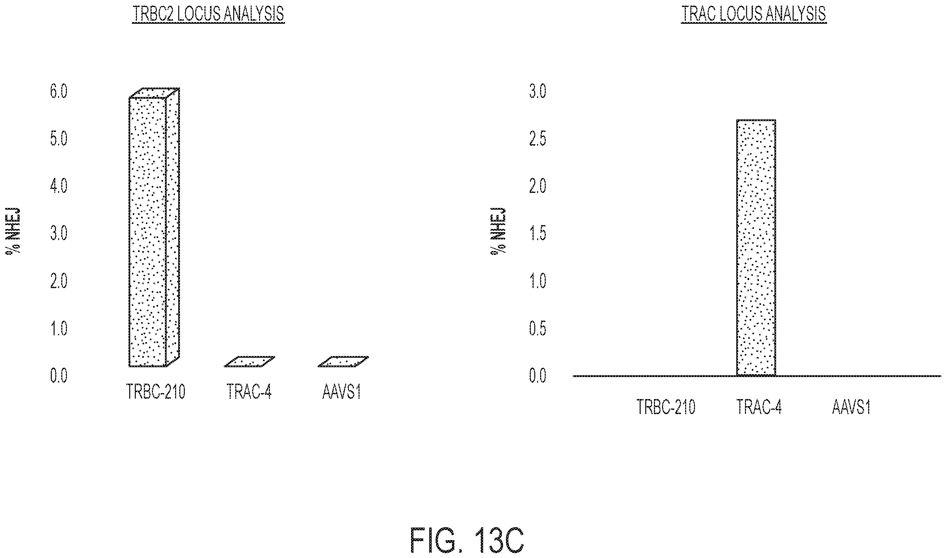

To demonstrate Cas9-mediated cutting in primary CD4+ T cells, *S. pyogenes* Cas9 and a gRNA designed against the TCR beta chain (TRBC-210 (SEQ ID NO:388)) or the TCR alpha chain (TRAC-4 (SEQ ID NO:389)) were delivered *ex vivo* as RNA molecules to T cells via electroporation (see **Table 1** for targeting domain sequences). In this embodiment, both the Cas9 and gRNA were *in vitro* transcribed using a T7 polymerase. A 5' ARCA cap was added to both RNA species simultaneous to transcription while a polyA tail was added after transcription to the 3' end of the RNA species by an *E*. *coli* polyA polymerase. To generate CD4+ T cells modified at the *TRBC1* and *TRBC2* loci, 10ug of Cas9 mRNA and 10ug of TRBC-210 gRNA (SEQ ID NO:388) were introduced to the cells by electroporation. In the same experiment, we also targeted the *TRAC* gene by introducing 10ug of Cas9 mRNA with 10ug of TRAC-4 gRNA (SEQ ID NO:389). A gRNA targeting the *AAVS1* genomic site was used as an experimental control. Prior to electroporation, the T cells were cultured in RPMI 1640 supplemented with 10% FBS and recombinant IL-2. The cells were activated using CD3/CD28 beads and expanded for at least 3 days. Subsequent to introduction of the mRNA to the activated T cells, CD3 expression on the cells was monitored at 24, 48 and 72 hours post electroporation by flow cytometry using a fluorescein (APC) conjugated antibody specific for CD3. At 72 hours, a population of CD3 negative cells was observed **(****Fig. 13A** and **Fig. 13B****).** To confirm that the generation of CD3 negative cells was a result of genome editing at the *TRBC* loci, genomic DNA was harvested and a T7E1 assay was performed. Indeed, the data confirm the presence of DNA modifications at the *TRBC2* locus and *TRAC* locus **(****Fig. 13C****).**

**Table 1**

| gRNA Name Targeting Domain | Targeting Domain | Cas9 species |
|---|---|---|
| TRBC-210 | GCGCUGACGAUCUGGGUGAC (SEQ ID NO:388) | *S. pyogenes* |
| TRAC-4 | GCUGGUACACGGCAGGGUCA (SEQ ID NO:389) | *S. pyogenes* |

### Delivery of Cas9/gRNA RNP to T cells

To demonstrate Cas9-mediated cutting in Jurkat T cells, *S. aureus* Cas9 and a gRNA designed against the TCR alpha chain (TRAC-233 (SEQ ID NO:390)) were delivered *ex vivo* as a ribonucleic acid protein complex (RNP) by electroporation (see **Table 2** for targeting domain sequences). In this embodiment, the Cas9 was expressed in *E. coli* and purified. Specifically, the HJ29 plasmid encoding Cas9 was transformed into Rosetta™ 2 (DE3) chemically competent cells (EMD Millipore #71400-4) and plated onto LB plates with appropriate antibiotics for selection and incubated at 37°C overnight. A 10mL starter culture of Brain Heart Infusion Broth (Teknova #B9993) with appropriate antibiotics was inoculated with 4 colonies and grown at 37°C with shaking at 220rpms. After growing overnight, the starter culture was added to 1L of Terrific Broth Complete (Teknova #T7060) with appropriate antibiotics plus supplements and grown at 37°C with shaking at 220rpms. The temperature was gradually reduced to 18°C and expression of the gene was induced by addition of IPTG to 0.5mM when the OD600 was greater than 2.0. The induction was allowed to continue overnight followed by harvesting the cells by centrifugation and resuspension in TG300 (50mM Tris pH8.0, 300mM NaCl, 20% glycerol, 1mM TCEP, protease inhibitor tablets (Thermo Scientific #88266)) and stored at -80°C.

The cells were lysed by thawing the frozen suspension, followed by two passes through a LM10 Microfluidizer® set to 18000psi. The extract was clarified via centrifugation and the soluble extract was captured via batch incubation with Ni-NTA Agarose resin (Qiagen #30230) at 4°C. The slurry was poured into a gravity flow column, washed with TG300+30mM Imidazole and then eluted the protein of interest with TG300+300mM Imidazole. The Ni eluent was diluted with an equal volume of HG100 (50mM Hepes pH7.5, 100mM NaCl, 10% glycerol, 0.5mM TCEP) and loaded onto a HiTrap SP HP column (GE Healthcare Life Sciences #17-1152-01) and eluted with a 30 column volume gradient from HG100 to HG1000 (50mM Hepes pH7.5, 1000mM NaCl, 10% glycerol, 0.5mM TCEP). Appropriate fractions were pooled after assaying with an SDS-PAGE gel and concentrated for loading onto a SRT10 SEC300 column (Sepax #225300-21230) equilibrated in HG150 (10mM Hepes pH7.5, 150mM NaCl, 20% glycerol, 1mM TCEP). Fractions were assayed by SDS-PAGE and appropriately pooled, concentrated to at least 5mg/ml.

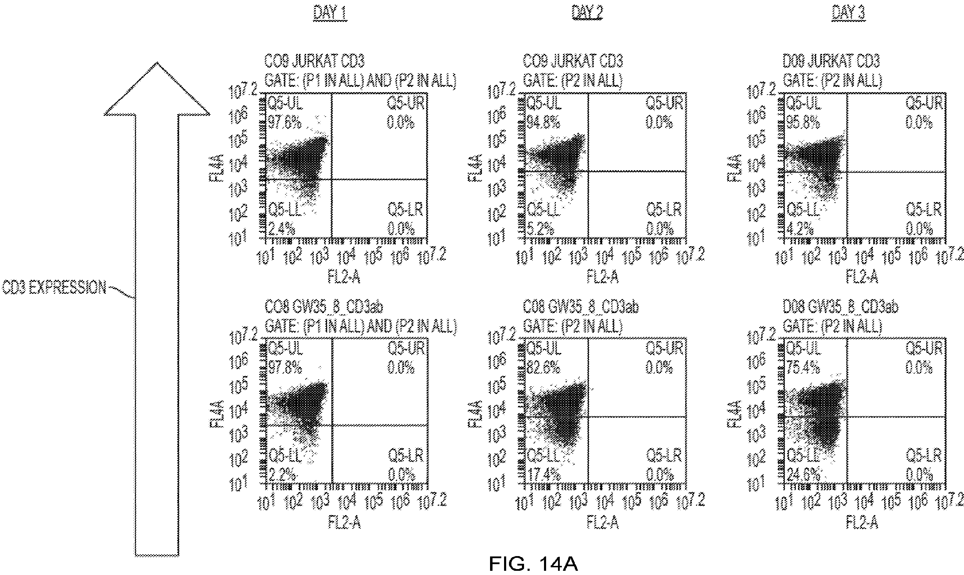

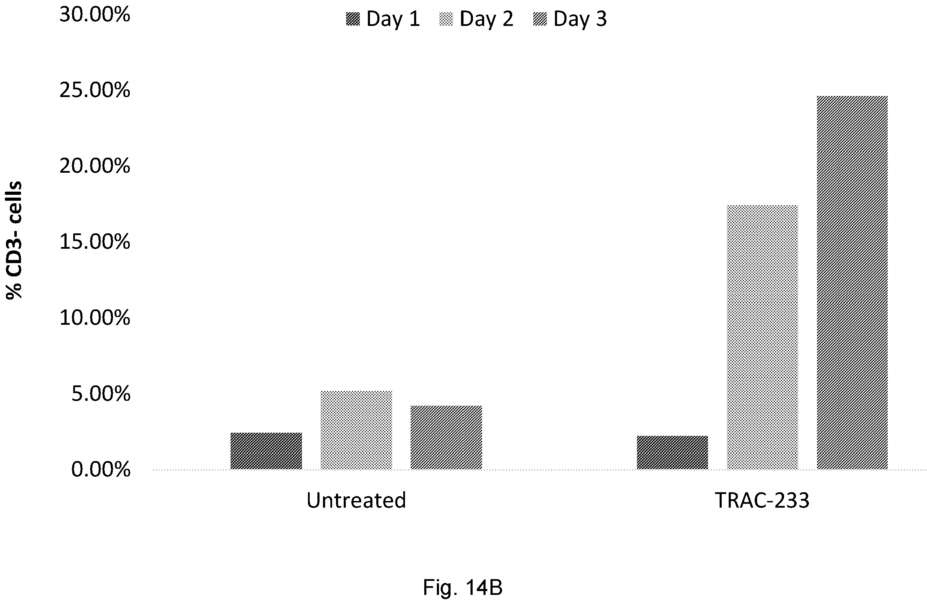

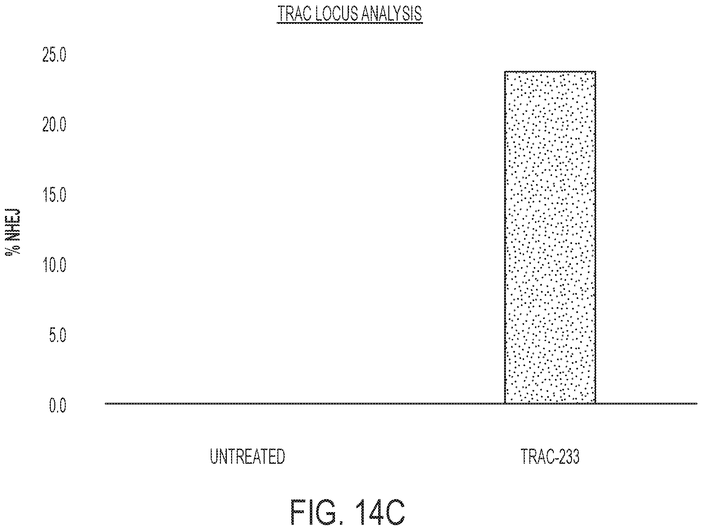

The gRNA was generated by *in vitro* transcription using a T7 polymerase. A 5' ARCA cap was added to the RNA simultaneous to transcription while a polyA tail was added after transcription to the 3' end of the RNA species by an *E. coli* polyA polymerase. Prior to introduction into the cells, the purified Cas9 and gRNA were mixed and allowed to form complexes for 10 minutes. The RNP solution was subsequently introduced into Jurkat T cells by electroporation. Prior to and after electroporation, the cells were cultured in RPMI1640 media supplemented with 10% FBS. CD3 expression on the cells was monitored at 24, 48 and 72 hours post electroporation by flow cytometry using a fluorescein conjugated antibody specific for CD3. At 48 and 72 hours, a population of CD3 negative cells was observed **(****Fig. 14A** and **Fig. 14B****).** To confirm that the generation of CD3 negative cells was a result of genome editing at the *TRAC* locus, genomic DNA was harvested and a T7E1 assay was performed. Indeed, the data confirm the presence of DNA modifications at the *TRAC* locus **(****Fig. 14C****).**

**Table 2**

| gRNA Name | Targeting Domain | Cas9 species |
|---|---|---|
| TRAC-233 | 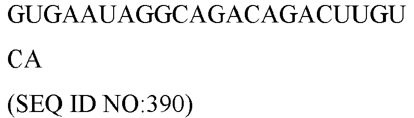 | *S. aureus* |

### Example 5: Assessment of Gene Targeting by HDR

The gRNAs that induce the greatest levels of NHEJ in initial tests can be selected for further evaluation of gene targeting efficiency. In this case, cells are derived from disease subjects and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency can be determined by several methods.

Determination of gene targeting frequency involves measuring the percentage of alleles that have undergone homologous directed repair (HDR) with the exogenously provided donor template or endogenous genomic donor sequence and which therefore have incorporated the desired correction. If the desired HDR event creates or destroys a restriction enzyme site, the frequency of gene targeting may be determined by a RFLP assay. If no restriction site is created or destroyed, sequencing may be used to determine gene targeting frequency. If a RFLP assay is used, sequencing may still be used to verify the desired HDR event and ensure that no other mutations are present. If an exogenously provided donor template is employed, at least one of the primers is placed in the endogenous gene sequence outside of the region included in the homology arms, which prevents amplification of donor template still present in the cells. Therefore, the length of the homology arms present in the donor template may affect the length of the PCR amplicon. PCR amplicons can either span the entire donor region (both primers placed outside the homology arms) or they can span only part of the donor region and a single junction between donor and endogenous DNA (one internal and one external primer). If the amplicons span less than the entire donor region, two different PCRs should be used to amplify and sequence both the 5' and the 3' junction.

If the PCR amplicon is short (less than 600bp) it is possible to use next generation sequencing. Following PCR, next generation sequencing adapters and barcodes (for example Illumina multiplex adapters and indexes) may be added to the ends of the amplicon, e.g., for use in high throughput sequencing (for example on an Illumina MiSeq). This method allows for detection of very low gene targeting rates.

If the PCR amplicon is too long for next generation sequencing, Sanger sequencing can be performed. For Sanger sequencing, purified PCR amplicons will be cloned into a plasmid backbone (for example, TOPO cloned using the LifeTech Zero Blunt® TOPO® cloning kit), transformed, miniprepped and sequenced.

The same or similar assays described above can be used to measure the percentage of alleles that have undergone HDR with endogenous genomic donor sequence and which therefore have incorporated the desired correction.

### Example 6: Gene Targeting of the HBB Locus by CRISPR/Cas9 to Investigate Repair Pathway Choice in Response to Different Types of DNA Lesions

The CRISPR/Cas9 system was used to target the human *HBB* gene in the region of the sickle cell anemia-causing mutation.

To examine how the nature of the targeted break affects the frequency of different DNA repair outcomes, blunt double-strand breaks, single-strand nicks, and dual-nicks in which the nicks are placed on opposite strands and leave either 3' or 5' overhangs of varying lengths, were introduced by utilizing the wild type Cas9 nuclease, as well as two different Cas9 nickases. Several different DNA repair outcomes including indel mutations resulting from non-homologous end-joining, homology-dependent repair (HDR) using the donor as a template, and HDR using the closely related *HBD* gene as an endogenous template, were characterized using either single-strand oligonucleotide (ssODN) or plasmid DNA donors. The frequency of these various repair outcomes under different conditions offer insight into the mechanisms of DNA repair and how it is impacted by the nature of the DNA break. The data also indicates a therapeutic approach in which correction of the sickle-cell mutation is efficiently mediated through HDR with either a donor template or with the *HBD* gene.

In this study different gRNA molecules for the *HBB* region that surrounds the nucleotides encoding the amino acid most commonly mutated in sickle cell disease had been tested in 293T cells with wild type Cas9 molecule. The gRNAs that induced similar high rates of NHEJ and had PAMs facing in opposite orientations were selected to test as pairs with Cas9 D10A and Cas9 N863A nickases.

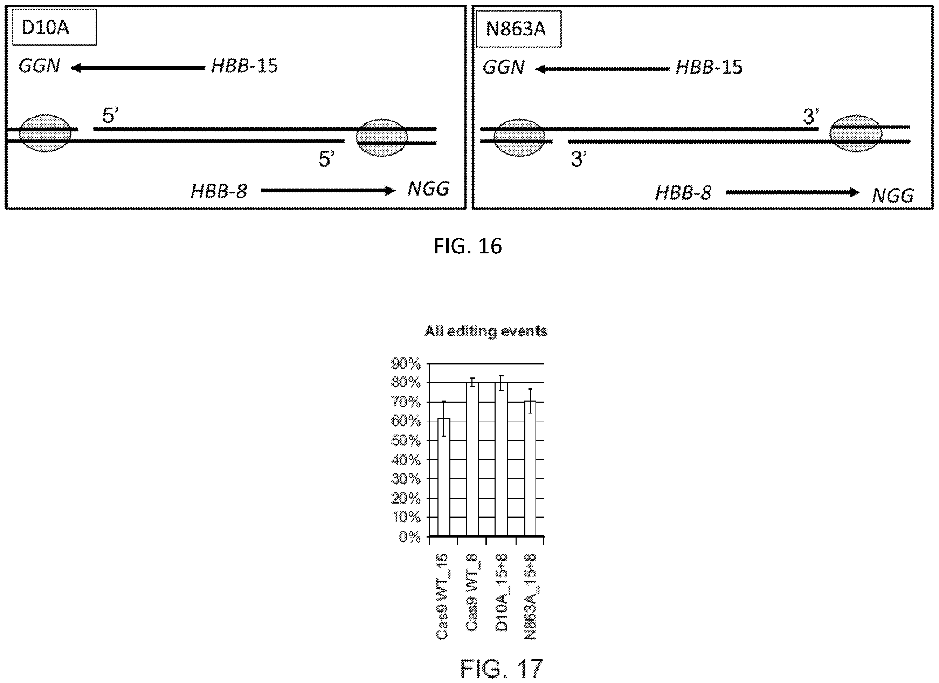

As shown in **Fig. 16****,** the gRNA pair 8/15 ("*HBB-*8"/*"HBB-*15" pair) was selected as one of the best pairs of gRNA molecules. *"HBB*-8*"* has the targeting domain sequence of GUAACGGCAGACUUCUCCUC (SEQ ID NO:398) and *"HBB*-15" has the targeting domain sequence of AAGGUGAACGUGGAUGAAGU (SEQ ID NO:397). This pair of gRNAs in combination with the mutant Cas9 D10A would generate a 5' overhang of 47 bp, and in combination with the mutant N863A would generate a 3' overhang of 47 bp.

In this Example, U20S cells were electroporated with 200 ng of each gRNA and 750 ng of plasmid that encodes wild type Cas9 or mutant Cas9. Cells were collected 6 days after electroporation and genomic DNA was extracted. PCR amplification of the *HBB* locus was performed and subcloned into a Topo Blunt Vector. For each condition in each experiment 96 colonies were sequenced with Sanger sequencing. In the experiments assessing HDR efficacy, cells were electroporated with 2.5 ug of single stranded oligo or double stranded oligo in addition to the gRNA and the Cas9-encoding plasmid.

As shown in **Fig. 17****,** the total percentages of all editing events detected by Sanger sequencing of the *HBB* locus were similar using wild type Cas9 or Cas9 nickases (D10A, N863A).

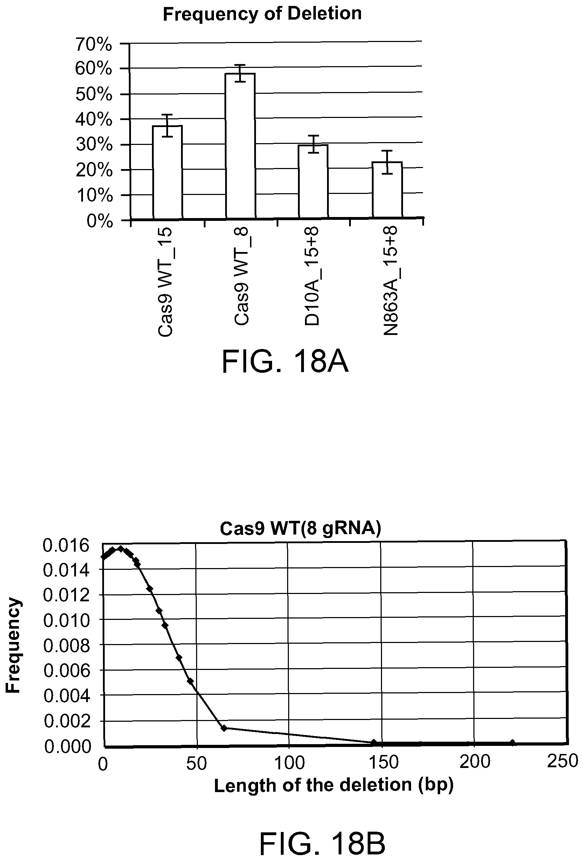

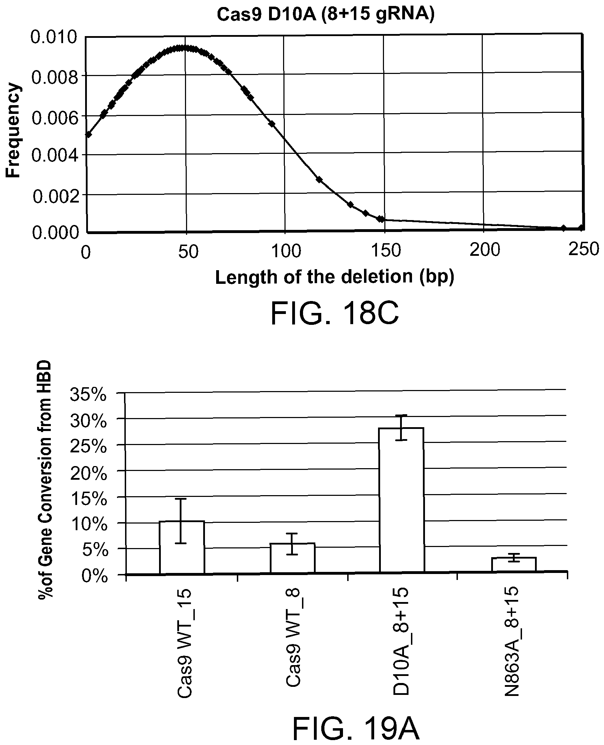

**Figs. 18A-18B** show that a majority of the total gene editing events (about 3/4 of the total) were small deletions (<10 bp). This is consistent with the notion that wildtype Cas9 generates a blunt end which are preferentially repaired by canonical NHEJ. In contrast, deletions represented only about a quarter of the total events using either nickase (D10A or N863A). Moreover, larger deletions of ∼50 bp that can be mapped to the region between the two nickase sites were observed **(****Fig. 18A** or **18C****).** The remaining gene-editing events were substantially different between the two nickases.

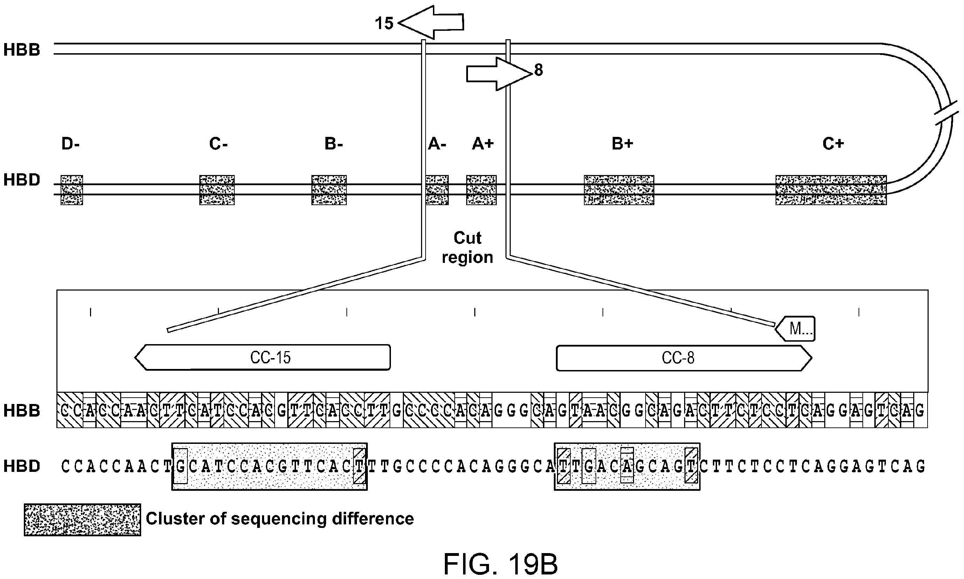

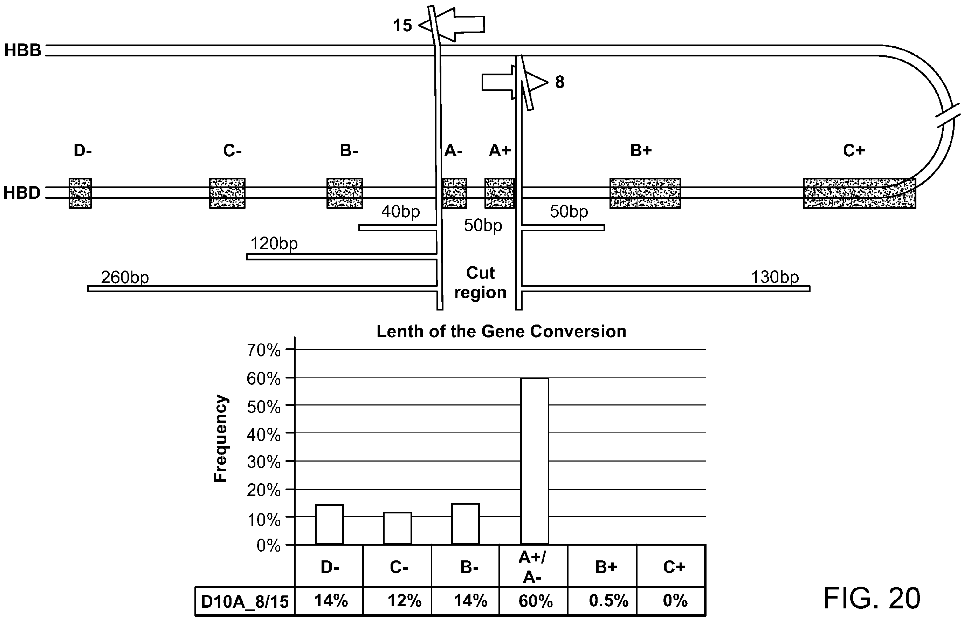

As shown in **Fig. 19A****,** in the case of Cas9 D10A nickase which leaves a 5' protruding end, the lesion is mostly repaired through a mechanism defined as gene conversion. In gene conversion, the *HBD* locus will serve as a template to repair the *HBB* gene. *HBD* is a highly similar gene (92% identity with *HBB*) that does not carry the sickle-cell mutation **(****Fig. 19B****).** **Fig. 20** shows that the majority of the *HBD* sequence that got incorporated in the *HBB* locus was in the region between the nickase cuts. In contrast, a low frequency of gene conversion was observed when the N863 nicase was used **(****Fig. 19A****).** In the case of Cas9 N863A nickase, a majority of the gene editing events were insertions in which the inserted part was a duplication of the overhangs **(****Figs. 20A-20B****).**

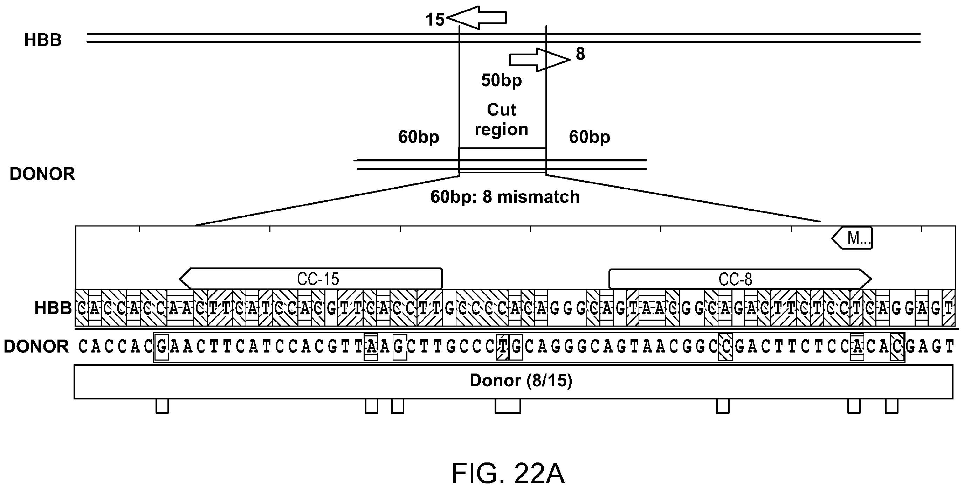

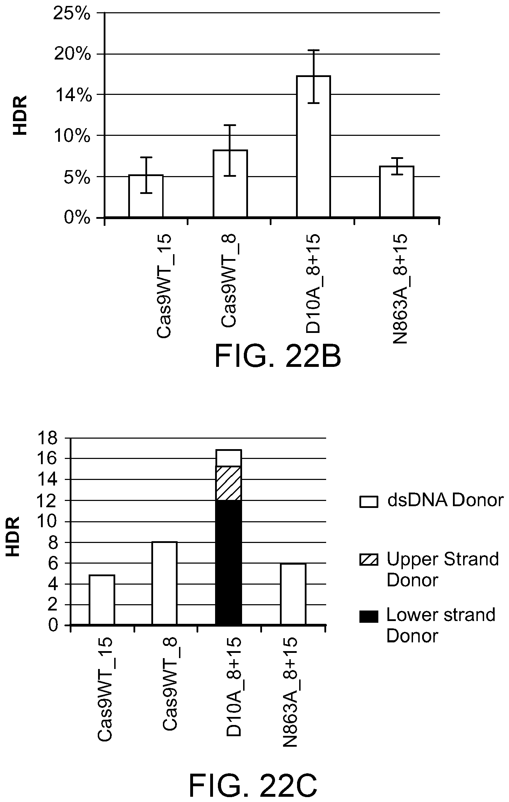

To test the effect that different lesions had on the engagement of HDR, a donor template was provided as a single strand oligo or as ds DNA donor. In both cases the length of the donor is approximately 170 bp with 60 bp of homology outside the nicks and with 8 mismatches **(****Fig. 22A****).** As shown in **Fig. 22B****,** the Cas9 D10A nickase that resulted in a 5' overhang gave a significantly higher rate of HDR, especially when using the upper stand as a single-strand oligo donor. **Fig. 22C** shows different forms of donors (dsDNA, upper stand, and lower strand) and there contribution to HDR.

In gene conversion, the *HBD* locus will serve as a template to repair the *HBB* gene. *HBD* is a highly similar gene (92% identity with *HBB*) that does not carry the sickle-cell mutation **(****Fig. 19B****).**

In summary, Cas9 nickases (D10A and N863A) showed comparable levels of efficacy compared to wildtype Cas9. Different DNA ends engage different repair pathways. The use of a wildtype Cas9 generates a blunt end, which are preferentially repaired by canonical NHEJ. Use of a Cas9 nickase with two gRNAs generates either 3' or 5' overhangs, which are not suitable substrates to be repaired by canonical NHEJ but can be repaired by alternative pathways.

The 5' protruding end was mostly repaired through a mechanism called gene conversion in which the *HBB* gene is repaired by using the *HBD* locus as a template. Use of nickase is advantageous to promote HDR. In the experiments in which a donor was provided, a significantly higher rate of HDR was observed using a nickase compared to the wildtype Cas9. The nature of the donor template also influences the outcome as HDR was preferentially observed when an single stranded Oligo (ss Oligo) was used.

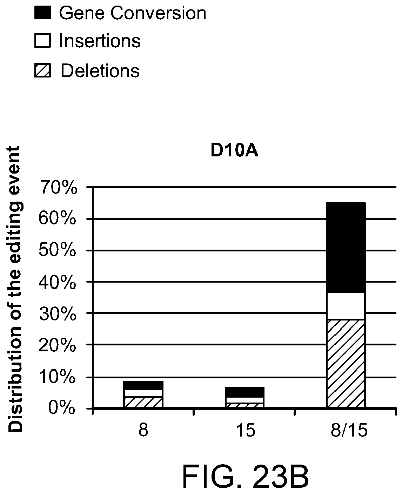

As shown in **Fig. 23B****,** gene conversion was observed with Cas9 D10A with a single gRNA molecule, compared to two gRNA moleculess. With one single nickase, a reduction of the overall frequency of gene editing compared to the use of two pairs of gRNA molecules was observed, but there was a similar distribution across the different types of editing.

### Example 7: Assessment of Gene Targeting in Hematopoietic Stem Cells

Transplantation of autologous CD34⁺ hematopoietic stem cells (HSCs, also known as hematopoietic stem/progenitor cells or HSPCs) genetically modified to correct the Sickle Cell Disease (SCD) mutation in the human hemoglobin gene (*HBB*) would prevent deformability (sickling) after deoxygenation in the erythrocyte progeny of corrected HSCs which could ameliorate symptoms associated with SCD. Genome editing with the CRISPR/Cas9 platform precisely alters endogenous gene targets by creating an indel at the targeted cut site that can lead to knock down of gene expression at the edited locus. In this Example, genome editing in the human K562 bone marrow erythroleukemia cell line, which serve as a proxy for HSCs and which can be predictive of genome editing in HSCs, were electroporated with Cas9 mRNA and gRNA pair 8/15 (*"HBB*-8"/*"HBB*-15*"* pair)to induce gene editing at the human *HBB* locus.

K562 cells were grown in RPMI media (Life Technologies) containing 10% fetal bovine serum (FBS). For the RNA electroporation, the Maxcyte GT device (www.maxcyte.com/) was used. *S. pyogenes* Cas9 mRNA and gRNA pair 8/15 ("*HBB-*8"/*"HBB*-15*"* pair) were prepared by *in vitro* transcription using linearized plasmid DNA as templates and the Ambion mMessage mMachine® T7 Ultra Transcription kit (Life Technologies) according to the manufacturer's instructions. In this embodiment, both the Cas9 and gRNA were *in vitro* transcribed using a T7 polymerase. For example, a 5' ARCA cap was added to both RNA species simultaneous to transcription while a polyA tail was added after transcription to the 3' end of the RNA species by an E. coli poly A polymerase. Capped and tailed gRNA pair 8/15 ("*HBB-*8"/"*HBB-*15" pair) were complexed at room temperature with *S. pyogenes* H-NLS-Cas9 protein at a molar ratio of ∼25:1 (gRNA : Cas9 protein) in a total of 30 µg RNP. Briefly, three million K562 cells were suspended in 100 µL Maxcyte EP buffer and transferred to the RNP solution (13µL). In addition, K562 cells were electroporated with *S. pyogenes* Cas9 mRNA and each of the gRNA pair 8/15 ("*HBB-*8"/ *"HBB*-15*"* pair). For the mRNA/gRNA electroporation with the Maxcyte device, 10 µg of *HBB*-8 (SEQ ID NO:398) (or 10 µg of *HBB*-15 (SEQ ID NO:397)) were mixed with 10 µg of Cas9 mRNA. Four million K562 cells were suspended in 100 µL Maxcyte EP buffer and then transferred to the mRNA/gRNA solution (13 µL). K562 cells mixed with either RNP or RNA were electroporated with the Maxcyte GT device. At 48 hours after electroporation, K562 cells were enumerated by trypan blue exclusion and were determined to have >88% viability in the electroporated cell populations. Genomic DNA was extracted from K562 cells 48 hours after electroporation and *HBB* locus-specific PCR reactions were performed.

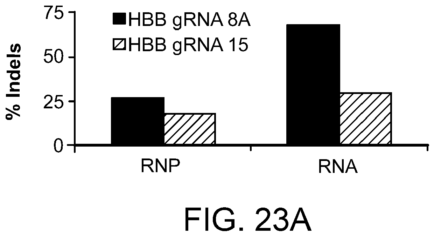

In order to detect indels at the *HBB l*ocus, T7E1 assays were performed on *HBB* locus-specific PCR products that were amplified from genomic DNA samples from electroporated K562 cells and the percentage of indels detected at the *HBB* locus was calculated **(****Fig. 23A****).**

Co-delivery of 10 µg RNP which contains wild-type *S. pyogenes* Cas9 protein with *HBB-8* or *HBB-15* resulted in 26.8% and 16.1% indels, respectively, at the *HBB* locus in gDNA from K562 cells (molar ratio protein: gRNA 24:1). Co-delivery of Cas9 mRNA with *HBB-*8 (SEQ ID NO:398) or *HBB*-15 (SEQ ID NO:397) led to 66.9% and 29.5% indels at the *HBB* locus in gDNA from K562 cells (10 µg of each RNA/4 million cells). This example shows that delivery of Cas9 mRNA/gRNA and Cas9 RNPs leads to editing of the *HBB* locus in a relevant bone marrow derived hematopoietic cell line (K562 cells). Clinically, transplantation of autologous HSCs in which the *HBB* locus has been edited to correct the genetic mutation that causes red blood cell sickling could be used to ameliorate symptoms of SCD.

### Example 8: Modification of gRNA by addition of 5' cap and 3' poly-A tail increases genome editing at target genetic loci and improves CD34+ cell viability and survival

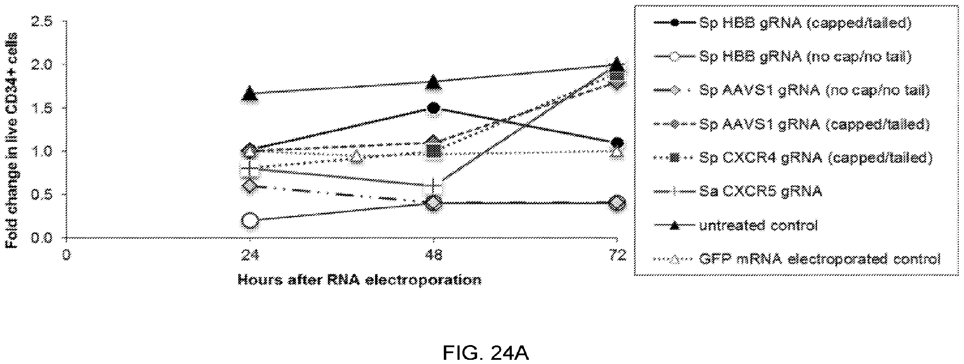

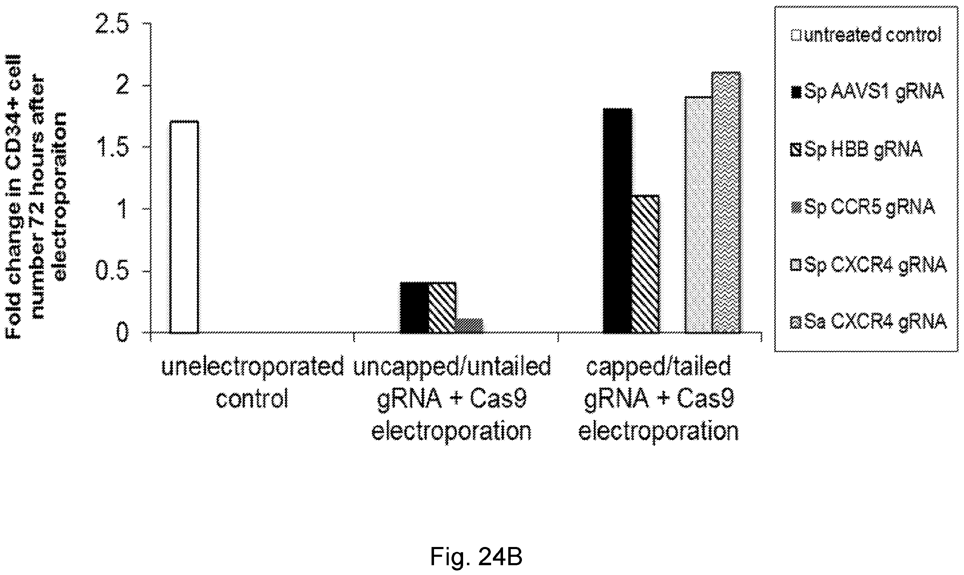

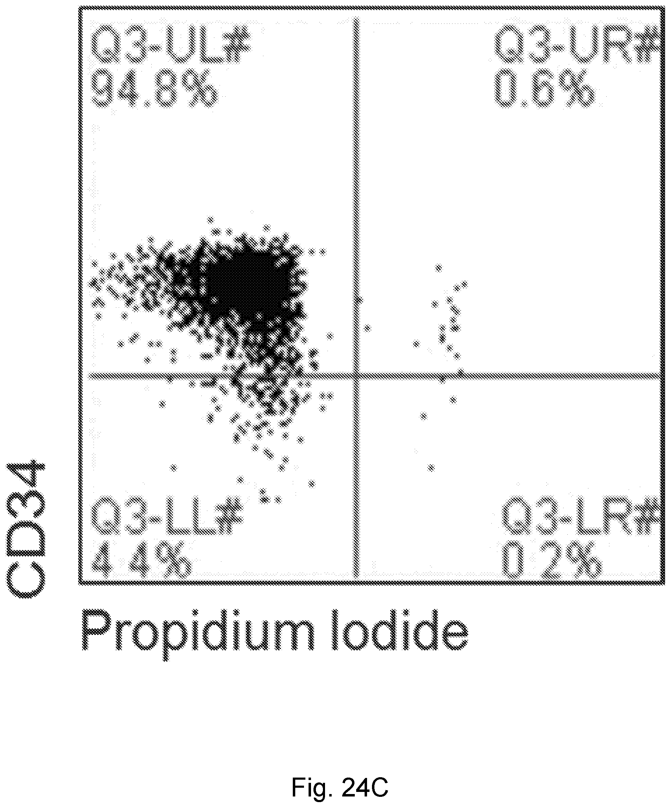

During virus-host co-evolution, viral RNA capping that mimics capping of mRNA evolved to allow viral RNA to escape detection from the cell's innate immune system (Delcroy et al., 2012, NATURE REVIEWS MICROBIOLOGY, 10:51-65). Toll-like receptors in hematopoietic stem/progenitor cells sense the presence of foreign single and double stranded RNA that can lead to innate immune response, cell senescence, and programmed cell death (Kajaste-Rudnitski and Naldini, 2015, HUMAN GENE THERAPY, 26:201-209). Results from initial experiments showed that human hematopoietic stem/progenitor cells electroporated with unmodified target specific gRNA and Cas9 mRNA led to reduced cell survival, proliferation potential, multipotency (e.g., loss of erythroid differentiation potential and skewed myeloid differentiation potential) compared to cells electroporated with GFP mRNA alone. Toward optimization of genome editing in hematopoietic/stem progenitor cells, human CD34⁺ cells from mobilized peripheral blood and bone marrow were electroporated with *S. pyogenes* Cas9 mRNA co-delivered with *HBB* or *AAVS1* targeted gRNA *in vitro* transcribed with or without the addition of a 5' cap and 3' poly-A tail. Human CD34⁺ cells that were electroporated with Cas9 paired with a single uncapped and untailed *HBB* or *AAVS1* gRNA exhibited decreased proliferation potential over 3 days in culture compared to cells that were electroporated with the same gRNA sequence that was *in vitro* transcribed to have a 5' cap and a 3' polyA tail **(****Fig. 24A****).** Other capped and tailed gRNAs (targeted to *HBB, AAVS1, CXCR4,* and *CCR5* loci) delivered with Cas9 mRNA did not negatively impact HSPC viability, proliferation, or multipotency, as determined by comparison of the fold expansion of total live CD34⁺ cells over three days after delivery. Importantly, there was no difference in the proliferative potential of CD34⁺ cells contacted with capped and tailed gRNA and Cas9 mRNA compared to cells contacted with GFP mRNA or cells that were untreated. Analysis of cell viability (by co-staining with either 7-aminoactinomycin D or propidium iodide with AnnexinV antibody followed by flow cytometry analysis) at seventy-two hours after contacting Cas9 mRNA and gRNAs indicated that cells that contacted capped and tailed gRNAs expanded in culture and maintained viability HSPCs that contacted uncapped and tailed gRNAs exhibited a decrease in viable cell number **(****Fig. 24B****).** Viable cells (propidium iodide negative) that contacted capped and tailed gRNAs also maintained expression of the CD34 cell surface marker **(****Fig. 24C****).**

In addition to the improved survival, target cells that contacted capped and tailed *AAVS1* specific gRNA also exhibited a higher percentage of on-target genome editing (% indels) compared to cells that contacted Cas9 mRNA and uncapped/untailed gRNAs **(****Fig. 25A****).** In addition, a higher level of targeted editing was detected in the progeny of CD34⁺ cells that contacted Cas9 mRNA with capped/tailed gRNA compared to the progeny of CD34⁺ cells that contacted Cas9 mRNA with uncapped/untailed gRNA **(****Fig. 25A****, CFCs).** Delivery of uncapped/untailed gRNA also reduced the ex vivo hematopoietic potential of CD34⁺ cells, as determined in colony forming cell (CFC) assays. Cells that contacted uncapped an untailed gRNAs with Cas9 mRNA exhibited a loss in total colony forming potential (e.g., potency) and a reduction in the diversity of colony subtype (e.g. loss of erythroid and progenitor potential and skewing toward myeloid macrophage phenotype in progeny) **(****Fig. 25B****).** In contrast, cells that contacted capped and tailed gRNAs maintained CFC potential both with respect to the total number of colonies differentiated from the CD34+ cells and with respect to colony diversity (detected of mixed hematopoietic colonies [GEMMs] and erythroid colonies [E]).

Next capped and tailed *HBB* specific gRNAs were co-delivered with either Cas9 mRNA or complexed with Cas9 ribonucleoprotein (RNP) and then electroporated into K562 cells, a erythroleukemia cell line that been shown to mimic certain characteristics of HSPCs. Co-delivery of capped and tailed gRNA with Cas9 mRNA or RNP led to high level of genome editing at the *HBB* locus, as determined by T7E1 assay analysis of *HBB* locus PCR products **(****Fig. 25C****).** Next, 3 different capped and tailed gRNAs (targeting the *HBB, AAVS1,* and *CXCR4* loci) were co-delivered with *S. pyogenes* Cas9 mRNA into CD34⁺ cells isolated from umbilical cord blood (CB). Here, different amounts of gRNA (2 or 10 µg gRNA plus 10 µg of *S. pyogenes* Cas9 mRNA) were electroporated into the cells and the percentages of genome editing evaluated at target loci by T7E1 assay analysis of locus PCR products. In contrast, no cleavage was detected at the *HBB* locus in the genomic DNA from CB CD34⁺ cells that were electroporated with uncapped and untailed *HBB* gRNA with Cas9 mRNA. The results indicated that CB CD34⁺ cells electroporated with Cas9 mRNA and capped and tailed gRNAs maintained proliferative potential and colony forming potential. Five to 20% indels were detected at target loci and the amount of capped and tailed gRNA co-delivered with the Cas9 mRNA did not impact the percentage of targeted editing **(****Fig. 25D****).**

A representative gel image of the indicated locus specific PCR products after T7E1 assay was performed shows cleavage at the targeted loci in CB CD34⁺ cells 72 hours after delivery of capped and tailed locus-specific gRNAs (*AAVS1, HBB,* and *CXCR4* gRNAs) co-delivered with *S*. *pyogenes* Cas9 mRNA by electroporation **(****Fig. 25F****).** Importantly, there was no difference in the viability of the cells electroporated with capped and tailed *AAVS1*-specific gRNA, *HBB*-specific gRNA, or *CXCR4*-specific gRNA co-delivered with *S. pyogenes* Cas9 mRNA compared to cells that did not contact Cas9 mRNA or gRNA (i.e., untreated control). Live cells are indicated by negative staining for 7-AAD and AnnexinV as determined by flow cytometry analysis (bottom left quadrants of flow cytometry plots, **Fig. 25G**). CB CD34⁺ cells electroporated with capped and tailed *AAVS1* specific gRNA, *HBB*-specific gRNA, or *CXCR4*-specific gRNA co-delivered with *S*. *pyogenes* Cas9 mRNA maintained *ex vivo* hematopoietic colony forming potential as determined by CFC assays. The representation *ex vivo* hematopoietic potential in CFC assays for cells that contacted *HBB*-specific gRNA and Cas9 is shown in the **Fig. 25E****.**

### Example 9: A 5' cap and 3' polyA tail on a gRNA improves the viability and gene editing in Jurkat T cells

To determine whether the addition of a cap and tail on a gRNA is important for cell viability and subsequent editing, K562s and Jurkat T cells were transfected with *S. pyogenes* Cas9 mRNA and an *AAVS1* specific gRNA that was either modified or non-modified. In either condition, Cas9 mRNA was *in vitro* transcribed using a T7 polymerase with an ARCA cap added to the 5' end during transcription. After transcription, a polyA tail was added to the 3' end of the RNA species by an E. coli polyA polymerase. The modified gRNA was generated in the same fashion resulting in a gRNA that has a 5' ARCA cap and polyA tail. The non-modified gRNA was generated by *in vitro* transcription using T7 polymerase. The non-modified gRNA did not have a 5' ARCA cap or 3' polyA tail. The K562s and Jurkat T cells were both grown in RPMI1640+10%FBS. The cells were electroporated with either 10 ug of Cas9 mRNA and 10 ug of a modified *AAVS1* gRNA or 10 ug of Cas9 mRNA and 10 ug of a non-modified *AAVS1* gRNA. At day 3 post electroporation, the cells were harvested and analyzed by Annexin V/PI staining to look at cell viability. Briefly, the cells were stained with an antibody that recognizes AnnexinV. Prior to analysis on a flow cytometer cells were stained with propidium iodide. Cells that remained unstained were considered "viable" cells and are shown in **Fig. 26A****.** Gene editing rates were determined by harvesting genomic DNA at day 3, and a T7E1 assay was performed at the *AAVS1* insertion site locus and are shown in **Fig. 26B****.**

### Example 10: Delivery of Cas9/gRNA RNP to T cells

To demonstrate Cas9-mediated cutting in primary human CD4⁺ or CD8⁺ T cells, *S. aureus* Cas9 and a gRNA designed against the TCR alpha chain (TRAC-233 (SEQ ID NO:390)) or *S*. *pyogenes* Cas9 and a gRNA designed against the TCR beta chain (TRBC-210 (SEQ ID NO:388)) or *S. pyogenes* Cas9 and a gRNA designed against *PDCD1* (PDCD1-108 (SEQ ID NO:399)) were delivered as a ribonucleic acid protein complex (RNP) by electroporation (see **Table 3** for targeting domain sequences). In this embodiment, the Cas9 was expressed in E. coli and purified. Specifically, the HJ29 plasmid encoding Cas9 was transformed into Rosetta™ 2 (DE3) chemically competent cells (EMD Millipore #71400-4) and plated onto LB plates with appropriate antibiotics for selection and incubated at 37°C overnight. A 10mL starter culture of Brain Heart Infusion Broth (Teknova #B9993) with appropriate antibiotics was inoculated with 4 colonies and grown at 37°C with shaking at 220rpms. After growing overnight, the starter culture was added to 1L of Terrific Broth Complete (Teknova #T7060) with appropriate antibiotics plus supplements and grown at 37°C with shaking at 220rpms. The temperature was gradually reduced to 18°C and expression of the gene was induced by addition of IPTG to 0.5mM when the OD600 was greater than 2.0. The induction was allowed to continue overnight followed by harvesting the cells by centrifugation and resuspension in TG300 (50mM Tris pH8.0, 300mM NaCl, 20% glycerol, 1mM TCEP, protease inhibitor tablets (Thermo Scientific #88266)) and stored at -80°C.

**Table 3**

| gRNA Name | Targeting Domain | Cas9 species |
|---|---|---|
| TRAC-233 | GUGAAUAGGCAGACAGACUUGUCA (SEQ ID NO:390) | *S. aureus* |
| TRBC-210 | GCGCUGACGAUCUGGGUGAC (SEQ ID NO:388) | *S. pyogenes* |
| PDCD1-108 | GUCUGGGCGGUGCUACAACU (SEQ ID NO:399) | *S. pyogenes* |

The cells were lysed by thawing the frozen suspension, followed by two passes through a LM10 Microfluidizer® set to 18000psi. The extract was clarified via centrifugation and the soluble extract was captured via batch incubation with Ni-NTA Agarose resin (Qiagen #30230) at 4°C. The slurry was poured into a gravity flow column, washed with TG300+30mM Imidazole and then eluted the protein of interest with TG300+300mM Imidazole. The Ni eluent was diluted with an equal volume of HG100 (50mM Hepes pH7.5, 100mM NaCl, 10% glycerol, 0.5mM TCEP) and loaded onto a HiTrap SP HP column (GE Healthcare Life Sciences #17-1152-01) and eluted with a 30 column volume gradient from HG100 to HG1000 (50mM Hepes pH7.5, 1000mM NaCl, 10% glycerol, 0.5mM TCEP). Appropriate fractions were pooled after assaying with an SDS-PAGE gel and concentrated for loading onto a SRT10 SEC300 column (Sepax #225300-21230) equilibrated in HG150 (10mM Hepes pH7.5, 150mM NaCl, 20% glycerol, 1mM TCEP). Fractions were assayed by SDS-PAGE and appropriately pooled, concentrated to at least 5mg/ml.

The gRNA was generated by *in vitro* transcription using a T7 polymerase. A 5' ARCA cap was added to the RNA simultaneous to transcription while a polyA tail was added after transcription to the 3' end of the RNA species by an E. coli polyA polymerase. Prior to introduction into the cells, the purified Cas9 and gRNA were mixed and allowed to form complexes for 10 minutes. The RNP solution was subsequently introduced into primary T cells by electroporation. In this embodiment, CD4⁺ or CD8⁺ T cells were isolated from the peripheral blood of donors. Briefly, the peripheral blood mononuclear cells were separated by FICOLL gradient centrifugation and subsequently treated with either a CD4 or CD8 specific antibody to positively select for the respective T cells. The cells, cultured in T cell media, were activated with CD3/CD28-conjugated beads and then electroporated with the aforementioned target specific RNPs. Cell viability was monitored each day for 4 days post electroporation and CD3 cell surface expression was monitored by flow cytometry using a fluorescein conjugated antibody specific for CD3 during the same period. By 2 days, a population of CD3 negative cells was observed **(****Figs. 27A-27C****,** **Figs. 28A-28C****,** **Figs. 29A-29C****)** which continued to increase during the period of analysis. To confirm that the generation of CD3 negative cells was a result of genome editing at the *TRAC* or *TRBC* locus, genomic DNA was harvested and the *TRAC* and *TRBC* loci were amplified by specific PCR. The products were cloned into a sequencing vector and sequenced by Sanger sequencing. Indeed, the data confirm the presence of DNA modifications at the *TRAC* and *TRBC* locus (see **Fig. 30****;** SEQ ID NOS:419-444 and **Fig. 31****;** SEQ ID NOS:445-461).

PD-1 expression was monitored by FACS using a fluorescein conjugated antibody specific for PD-1. Since PD-1 is expressed on activated T cells, cells were reactivated 4 days post electroporation by incubating the cells for 48 hours with CD3/CD28-conjugated beads. The level of PD-1 expression was analyzed on the cells by FACS 24 hours post removal of the beads (7 days post electroporation). Cells that were treated with the PDCD1 specific RNP showed a marked reduction in PD-1 expression **(****Figs. 32A-32C****).** To confirm that the generation of PD-1 negative cells was a result of genome editing at the *PDCD1* locus, genomic DNA was harvested and the *PDCD1* locus was amplified by PCR. The product was cloned into a sequencing vector and sequenced by Sanger sequencing. Indeed, the data confirm the presence of DNA modifications at *PDCD1* locus (see **Fig. 33****;** SEQ ID NOS:462-486).

### Example 11: Contact between S. pyogenes Cas9 ribonucleoprotein complexed to gRNAs targeting the HBB genetic locus supports gene editing in adult human hematopoietic stem cells

Transplantation of autologous CD34⁺ hematopoietic stem cells (HSCs) collected from patients affected with hemoglobinopathies (e.g., sickle cell disease [SCD], β-thalessemia), that have been genetically modified with a lentivirus vector that expresses non-sickling β-hemoglobin gene (*HBB*) has been shown to restore expression of functional adult hemoglobin (HbA) thus preventing the formation of sickle hemoglobin (HbSS), in erythroid cells derived from transduced CD34⁺ cells and ameliorating clinical symptoms in affected patients (Press Release from Bluebird Bio, June 13, 2015, "bluebird bio Reports New Beta-thalassemia major and Severe Sickle Cell Disease Data from HGB-205 study at EHA"). However, delivery of a transgene encoding a non-sickling β-hemoglobin does not correct the causative mutation or prevent expression of the mutant (e.g., sickling) form of *HBB.* Furthermore, lentivirus vector transduction of CD34⁺ cells can lead to the occurrence of multiple transgene integration sites per cell and the long-term effects of multiple transgene integration sites is currently undetermined.

In contrast, genome editing with the CRISPR/Cas9 platform precisely alters endogenous gene targets by creating an insertion or deletion (indel) at the cut site that can lead to gene disruption at the edited locus. Co-delivery of two gRNAs each targeting regions proximal to the single nucleotide polymorphism (SNP) that encodes HbSS (e.g., GAG→ GTG which results in a change in the amino acid residue from glutamic acid to valine) co-delivered with a Cas9 D10A nickase supports a low level of homology directed repair (HDR) in human cell lines (e.g., gene conversion using a region of homology in the *HBD* locus as DNA repair template).

In this Example, genome editing in adult human mobilized peripheral blood CD34⁺ HSCs after co-delivery of Cas9 D10A nickase with two gRNAs targeting the *HBB* locus was evaluated. The edited CD34⁺ cells were then differentiated into myeloid and erythroid cells to determine the hematopoietic activity of the HSCs. Gene editing at the *HBB* locus was evaluated by T7E1 analysis and DNA sequencing. Expression of *HBB* protein was also analyzed in erythroid progeny.

Human CD34⁺ HSCs cells from mobilized peripheral blood (AllCells®) were thawed into StemSpan Serum-Free Expansion Medium (SFEM™, StemCell Technologies) containing 300 ng/mL each of human stem cell factor (SCF) and flt-3 ligand (FL), 100 ng/mL thrombopoietin (TPO), and 60 ng/mL of IL-6, and 10 µM PGE2 (Cayman Biochemicals; all other supplements were from PeproTech® unless otherwise indicated). Cells were grown for 3 days in a humidified incubator and 5% CO₂ 20% O₂. On day 3 (morning), media was replaced with fresh Stemspan-SFEM™ supplemented with human SCF, TPO, FL and PGE2. In the afternoon of day 3, 2.5 million CD34⁺ cells per sample were suspended in electroporation buffer.

The gRNA was generated by *in vitro* transcription using a T7 polymerase. A 5' ARCA cap was added to the RNA simultaneous to transcription while a polyA tail was added after transcription to the 3' end of the RNA species by an *E. coli* polyA polymerase.

After the gRNAs were *in vitro* transcribed and tailed, the quality and quantity of gRNAs were evaluated with the Bioanalyzer (Nanochip®) to determine RNA concentration and by Differential Scanning Fluorimetry (DSF) assay, a thermal shift assay that quantifies the change in the thermal denaturation temperature of Cas9 protein with and without complexing to gRNA. In DSF assays, the Cas9 protein was mixed with gRNA and allowed to form complexes for 10 minutes. Cas9 protein and gRNA were mixed at a molar ration of 1:1, and the DSF assay performed as a measure of Cas9 stability and as an indirect measure of gRNA quality, since a 1:1 ratio of gRNA : Cas9 should support a thermal shift if the gRNA is of good quality **(****Fig. 34A****).**

For half of the samples, *in vitro* transcribed capped and tailed gRNAs *HBB*-8 (SEQ ID NO:398) and *HBB*-15 (SEQ ID NO:397) were added at a 2:1 molar ratio to 12.5µg D10A Cas9 nickase ribonucleoprotein (RNP) (5 µg RNP per million cells) to 2.5 million cells. *"HBB*-8*"* has the targeting domain sequence of GUAACGGCAGACUUCUCCUC (SEQ ID NO:398) and *"HBB*-15*"* has the targeting domain sequence of AAGGUGAACGUGGAUGAAGU (SEQ ID NO:397). D10A nickase and gRNAs as RNP complexes were transferred to 2.5 million adult CD34⁺ cells in electroporation buffer. The RNP/cell mixture was transferred to the cells then electroporated ("Program 2" and "Program 3") (Alt Program).

For the second portion of CD34⁺ cell samples, equal amounts (5 µg or 10 µg [2XgRNA] each) of *in vitro* transcribed capped and tailed gRNAs *HBB*-8 (SEQ ID NO:398) and *HBB*-15 (SEQ ID NO:397) were added to 10 µg of *in vitro* transcribed Cas9 D10A nickase mRNA. The mRNA : gRNA: cell mixture was electroporated with Program 2 (P2).

For all samples, the cells were collected and placed at 37°C for 20 minutes (recovery period). Then the cells were either transferred to pre-warmed cytokine supplemented Stemspan-SFEM™ media and placed at 30°C for 2 hours (cold shock samples) or placed directly into a 37°C incubator. For the cold shocked samples, the cells were transferred to the 37°C incubator after the 2-hour incubation period at 30°C. At 24, 48, and 72 hours after electroporation, the CD34⁺ cells were counted by trypan blue exclusion (cell survival) and divided into 3 portions for the following analyses: a) flow cytometry analysis for assessment of viability by co-staining with 7-Aminoactinomycin-D (7-AAD) and allophycocyanin (APC)-conjugated Annexin-V antibody (eBioscience); b) flow cytometry analysis for maintenance of HSC phenotype (after co-staining with phycoerythrin (PE)-conjugated anti-human CD34 antibody (BD Biosciences) and APC-conjugated CD133 (Miltenyi Biotech; c) hematopoietic colony forming cell (CFC) analysis by plating 800 cells in semi-solid methylcellulose based Methocult medium (StemCell Technologies H4435) that supports differentiation of erythroid and myeloid blood cell colonies from HSCs and serves as a surrogate assay to evaluate HSC multipotency and differentiation potential ex vivo; d) genomic DNA analysis for detection of editing at the *HBB* locus. Genomic DNA was extracted from the HSCs at 48 and 72 hours after electroporation and *HBB* locus-specific PCR reactions were performed. The purified PCR products were analyzed for insertions/deletions (indels) in T7E1 assays and by DNA sequencing of individual clones (PCR product was transformed and sub-cloned into TOPO-vector, individual colonies picked, and plasmid DNA containing individual PCR products were sequenced).

Western blot analysis of cell lysates extracted from the Cas9/gRNA electroporated CD34⁺ cells indicated the presence of Cas9 protein at 72 hours after electroporation of CD34⁺ cells that received Cas9 RNP and gRNA pair. Very low levels of Cas9 protein were detected in the lysates of cells that were electroporated with Cas9 mRNA **(****Fig. 34B****).**

Electroporated CD34⁺ cells maintained a stem cell phenotype (e.g., co-expression of CD34 and CD133) and viability (e.g., 75% AnnexinV⁻7AAD⁻) as determined by flow cytometry analysis **(****Fig. 35A****).** The absolute number of viable CD34⁺ cells was maintained across most samples over a 72-hour culture period after electroporation **(****Fig. 35B****).** In addition, gene edited CD34⁺ cells maintained *ex vivo* hematopoietic activity and multipotency as indicated by their ability to give rise to erythroid (e.g., CFU-E or CFU-GEMM) and myeloid (e.g., CFU-G, -M or-GM) cells **(****Fig. 35C****).**

Gene editing at the *HBB* locus was then evaluated at 72 hours after electroporation of D10A nickase mRNA or RNP co-delivered with two gRNAs (*HBB*-8 (SEQ ID NO:398) and *HBB*-15 (SEQ ID NO:397)). Briefly, genomic (g)DNA was isolated from electroporated CD34⁺ cells at 72 hours after electroporation, and PCR amplification of a ∼607bp fragment of the *HBB* locus (which captured both of the individual genomic locations that were targeted by the two gRNAs *HBB*-8 (SEQ ID NO:398) and *HBB*-15 (SEQ ID NO:397)) was performed. After cleanup of the *HBB* PCR product with AMPURE beads, insertions/deletions (indels) at the targeted genomic location were evaluated by T7E1 assay and by DNA sequencing. For the CD34⁺ cells electroporated with D10A nickase mRNA and *HBB* gRNA pair, no indels were detected **(Table 4).** In contrast to the negative results obtained after delivery of D10A nickase mRNA, ∼30-60% indels were detected by T7E1 and sequencing analysis of CD34⁺ cells that were electroporated with D10A nickase RNP with the *HBB* gRNA pair **(Table 4,** **Figs. 36A-36C****).** The cells that were cultured for 2 hours at 30°C (after a 20-minute recovery period at 37°C) exhibited 57% editing as determined by DNA sequencing. In addition, gene conversion (e.g., *HBD* genomic sequence used as a template copy for DNA repair of the disrupted *HBB* locus) was detected in the gDNA from CD34⁺ cells that were 'cold shocked' (30°C incubation) at a frequency of 3% relative to the total gene editing events **(****Fig. 36C****).**

Table 4 shows a summary of gene editing results in human adult CD34⁺ HSCs 72 hours after co-delivery of D10A Cas9 nickase and *HBB* specific gRNA pair.

**Table 4**

| D10A source | µg D10A | µg *HBB*-8 gRNA | µg *HBB*-15 gRNA | Temperature of 2-hour recovery | Electroporation Program | %indels (T7E1) | %indels (sequencing) |
|---|---|---|---|---|---|---|---|
| mRNA | 10 | 5 | 5 | 37°C | 2 | 0 | ND |
| mRNA | 10 | 5 | 5 | 30°C | 2 | 0 | ND |
| mRNA | 10 | 10 | 10 | 37°C | 2 | 0 | ND |
| RNP | 12.5 | 3.3 | 3.3 | 37°C | 2 | 45 | 30 |
| RNP | 12.5 | 3.3 | 3.3 | 30°C | 2 | 39 | 57 |
| RNP | 12.5 | 3.3 | 3.3 | 37°C | 3 | 39 | 39 |

To determine whether targeted disruption of the *HBB* locus induced changes in expression of β-hemoglobin protein, CFU-E colonies were picked, dissociated, fixed, permeabilized and stained with PE-conjugated mouse anti-human β-hemoglobin antibody (Santa Cruz Biotechnology®). The erythroid progeny of *HBB* gene edited cells exhibited a 7-to-10 fold reduction in β-hemoglobin expression compared to the CFU-Es differentiated from untreated control CD34⁺ cells **(****Fig. 37****).** These data show that the progeny of gene edited cells retain erythroid differentiation potential and that gene editing events detected in the parental CD34⁺ cells result in reduced protein expression in erythroid progeny.

### Example 12: Contact between S. pyogenes D10A Cas9 nickase ribonucleoprotein complexed to gRNAs targeting the HBB genetic locus supports gene editing in fresh umbilical cord blood derived human CD34⁺ hematopoietic stem cells

In this Example, genome editing in freshly collected human umbilical cord blood (CB) CD34⁺ HSCs after co-delivery of D10A Cas9 nickase with 2 gRNAs targeting the *HBB* locus was evaluated. The edited human CB CD34⁺ HSCs were then differentiated into myeloid and erythroid cells to determine the hematopoietic activity of the HSCs. Targeted disruption of the *HBB* locus was evaluated by T7E1 analysis and DNA sequencing.

Human CD34⁺ HSCs cells were isolated from freshly obtained human umbilical cord blood by ficoll gradient density centrifugation followed by MACS® (antibody conjugated immunomagnetic bead sorting) with mouse anti-human CD34⁺ immunomagnetic beads using the human CD34 cell enrichment kit and LS magnetic columns from Miltenyi Biotech. The cells were plated into StemSpan Serum-Free Expansion Medium (SFEM™, StemCell Technologies) containing 100 ng/mL each of human stem cell factor (SCF) and flt-3 ligand (FL), 20 ng/mL each of thrombopoietin (TPO) and IL-6, and 10 µM PGE2 (Cayman Biochemicals; all other supplements were from Peprotech unless otherwise indicated). Cells were grown for 3 days in a humidified incubator and 5% CO₂ 20% O₂. On day 3 (morning), media was replaced with fresh Stemspan-SFEM™ supplemented with human SCF, TPO, FL and PGE2. In the afternoon of day 3, 2.5 million CD34⁺ cells per sample were suspended in electroporation buffer.

The gRNAs were generated by *in vitro* transcription using a T7 polymerase. A 5' ARCA cap was added to the RNA simultaneous to transcription while a polyA tail was added after transcription to the 3' end of the RNA species by an *E. coli* polyA polymerase. After the gRNAs were *in vitro* transcribed and tailed, the quality and quantity of gRNAs were evaluated with the Bioanalyzer (Nanochip) to determine RNA concentration and by DSF assay performed as a measure of D10A Cas9 nickase RNP stability and as an indirect measure of gRNA quality.

*In vitro* transcribed capped and tailed gRNAs *HBB-8* (SEQ ID NO:398) and *HBB*-15 (SEQ ID NO:397) were added at a 2:1 molar ratio (total gRNA : Cas9 protein) to 12.5µg D10A nickase ribonucleoprotein (RNP) (5µg RNP per million cells) to each of two samples each containing 2.5 million CB CD34⁺cells. A third human CB CD34⁺HSC aliquot was mixed with 25µg D10A nickase RNP and *HBB* gRNAs (total gRNA: D10A ratio at 2:1). For each experimental sample, the D10A nickase RNP/cell mixture was transferred to the cells then electroporated.

For all samples, the cells were collected and placed at 37°C for 20 minutes (recovery period). For the human CB CD34⁺ HSCs that were contacted with 12.5µg D10A nickase RNP, one sample was transferred to pre-warmed cytokine supplemented Stemspan-SFEM™ media and placed at 30°C for 2 hours (cold shock samples) or placed directly into the same media at 37°C. For the cold shocked samples, the cells were transferred to the 37°C incubator after the 2-hour incubation period at 30°C. At 24, 48, and 72 hours after electroporation, the human CB CD34⁺ HSCs were counted by trypan blue exclusion (cell survival) and divided into 3 portions for the following analyses: a) flow cytometry analysis for assessment of viability by co-staining with 7-Aminoactinomycin-D (7-AAD) and allophycocyanin (APC)-conjugated Annexin-V antibody (eBioscience); b) flow cytometry analysis for maintenance of HSC phenotype (after co-staining with phycoerythrin (PE)-conjugated anti-human CD34 antibody (BD Biosciences) and APC-conjugated CD133 (Miltenyi Biotech; c) hematopoietic colony forming cell (CFC) analysis by plating 800 CB CD34⁺ HSCs in semi-solid methylcellulose based Methocult medium (StemCell Technologies H4435) that supports differentiation of erythroid and myeloid blood cell colonies from HSCs and serves as a surrogate assay to evaluate HSC multipotency and differentiation potential ex vivo; d) genomic DNA analysis for detection of editing at the *HBB* locus. Genomic DNA was extracted from the HSCs at 48 and 72 hours after electroporation and *HBB* locus-specific PCR reactions were performed. The purified PCR products were analyzed for insertions/deletions (indels) in T7E1 assays and by DNA sequencing of individual clones (PCR product was transformed and subcloned into TOPO-vector, individual colonies picked, and plasmid DNA containing individual PCR products were sequenced).

Electroporated CB CD34⁺ cells maintained a stem cell phenotype (e.g., co-expression of CD34 and CD133, ∼90% CD34⁺CD133⁺) and viability (e.g., 91% AnnexinV⁻7AAD⁻) as determined by flow cytometry analysis **(****Fig. 38A****).** The absolute number of viable CD34⁺ cells was maintained across most samples over a 72-hour culture period after electroporation. In addition, gene edited CD34⁺ cells maintained *ex vivo* hematopoietic activity and multipotency as indicated by their ability to give rise to erythroid (e.g., CFU-E or CFU-GEMM) and myeloid (e.g., CFU-G, -M, or -GM) cells **(****Fig. 38B****).**

Gene editing at the *HBB* locus was then evaluated at 72 hours after electroporation of D10A nickase RNP co-delivered with 2 gRNAs *(HBB*-8 (SEQ ID NO:398) and *HBB*-15 (SEQ ID NO:397)). Briefly, gDNA was isolated from electroporated CD34⁺ cells at 72 hours after electroporation, and PCR amplification of a ∼607bp fragment of the *HBB* locus (which captures both of the individual genomic locations that were targeted by gRNAs *HBB-8* (SEQ ID NO:398) and *HBB*-15 (SEQ ID NO:397)) was performed. After cleanup of the *HBB* PCR product with AMPURE beads, insertions/deletions (indels) at the targeted genomic location were evaluated by T7E1 assay and by DNA sequencing. For the CD34⁺ cells electroporated with 5 µg per million cells of D10A nickase RNP and *HBB*-specific gRNA pair, ∼20% indels were detected by T7E1 analysis **(Table 5).** In contrast to adult CD34⁺ cells, a 2-hour incubation at 30°C did not alter the level of gene editing as determined by either T7E1 analysis or DNA sequence analysis. In addition, doubling the D10A nickase RNP/gRNA concentration to 10 µg RNP per million cells nearly doubled the gene editing at the *HBB* locus to 57%, as determined by DNA sequencing analysis **(Table 5,** **Figs. 39A-39C****).** Stratification of DNA repair events through DNA sequencing analysis revealed that ∼50-70% of the sequence reads contained small insertions, ∼20-40% contained large deletions, and ∼8% showed evidence of *HBB*/*HBD* gene conversion events in the targeted *HBB* genomic location **(****Fig. 39C****).**

Table 5 shows a summary of gene editing results in CB CD34⁺ cells 72 hours after co-delivery of D10A nickase RNP and *HBB*-specific gRNA pair.

**Table 5**

| Total µg D10A nickase RNP | µg D10A nickase RNP/1E6 cells | µg *HBB-*8 gRNA | µg *HBB*-15 gRNA | Temperature of 2-hour recovery | %indels (T7E1) | %indels (sequencing) |
|---|---|---|---|---|---|---|
| 12.5 | 5 | 3.3 | 3.3 | 37°C | 20 | 23 |
| 12.5 | 5 | 3.3 | 3.3 | 30°C | 27 | 20 |
| 25 | 10 | 6.6 | 6.6 | 37°C | 36 | 51 |

In contrast to human adult CD34⁺ cells, human CB CD34⁺ cells are fetal in origin and therefore the progeny of human CB CD34⁺ cells express fetal hemoglobin (HbF) which contains γ-hemoglobin instead of β-hemoglobin. Given the lack of β-hemoglobin by CB eyrthroblasts, disruption of the *HBB* locus in this model system will not impact expression of hemoglobin protein. Human CB CD34⁺ cells are used as a model system for evaluation of gene editing in HSCs, since these umbilical cord blood derived CD34⁺ cells are more readily available for research use and reconstitute immune-deficient mouse xenografts more efficiently compared to human adult CD34⁺ cells. To determine whether gene edited cells retained their erythroid differentiation potential, the edited human CD34⁺ cells were induced to differentiate into erythroblasts. Briefly, human CD34⁺ cells were co-cultured with human plasma, holotransferrin, insulin, hydrocortisone, and cytokines (erythropoietin, SCF, IL3)) for 20 days in which the latter 4 growth factors were added at different stages of differentiation to direct erythroid specification program. The cells were then evaluated by flow cytometry for the acquisition of erythroid phenotypic characteristics including: co-expression of the transferrin receptor (CD71) and Glycophorin A (CD235); expression of HbF, and enucleation (as indicated by the absence dsDNA detected by the dsDNA dye DRAQ5) and loss of CD45 expression. By day 18 of differentiation, the erythroblast progeny of edited CD34⁺ cells possessed this red blood cell phenotype **(****Figs. 40A-40C****).** These data, along with the CFC data shown in **Figs. 38A-38B** show that the gene editing does not negatively impact the differentiation potential of CD34⁺ cells.

In summary, the data in this Example indicate: 1) electroporation of fresh CB CD34⁺ HSCs with D10A nickase and paired capped/tailed gRNAs does not impact cell viability, proliferation potential, or multipotency; and 2) contact between human CB CD34⁺ HSCs and 10 µg D10A nickase RNP (per million cells) supports >50% gene editing with HDR events (8% gene conversion events of total).

### Example 13: Contact between S. pyogenes wild-type Cas9 RNP or D10A nickase RNP complexed to gRNAs targeting the HBB genetic locus supports up to 60% gene editing in human cord blood hematopoietic stem cells

In this Example, human umbilical cord blood (CB) CD34⁺ HSCs were contacted with *S*. *pyogenes* wild-type Cas9 RNP, N863A nickase RNP, or D10A nickase RNP complexed with 2 gRNAs targeting the *HBB* locus. The percentage of gene editing and type of editing event (e.g., HDR, [e.g., gene conversion] or NHEJ) were evaluated to determine the optimal Cas9 activity (e.g., type of cut, e.g., double strand break from wild-type Cas9 or off-set nicks on opposite DNA strands by nickases) for gene editing in HSCs that would favor HDR (e.g., gene conversion) over NHEJ (e.g., ends of the DNA left exposed after the cut, e.g., blunt ends left by wild-type Cas9 cut, 5' overhang left by D10A nickase cut or 3' overhang left by N863A nickase cut). Other optimizations included: 1) removal of endotoxin from Cas9 protein preparation to reduce toxicity of Cas9 protein; 2) use of 10 µg RNP per million CD34⁺ cells to increase gene editing (shown to double gene editing in fresh CB CD34⁺ cells compared to 5 µg RNP, as indicated in **Example 12**); 3) testing of human CD34⁺ cells that were isolated from cord blood (CB), cryopreserved, and confirm that gene editing was not impacted by cryopreservation (compared to freshly isolated HSCs, described in **Example 12**); and 4) evaluate Cas9 RNP levels in CD34⁺ cells over time to understand Cas9 RNP stability in HSCs.

Human CD34⁺ HSCs cells were isolated from freshly obtained human umbilical cord blood by ficoll gradient density centrifugation followed by MACS sorting with mouse anti-human CD34⁺immunomagnetic beads. CD34⁺ cells were cryopreserved, thawed at a later date, and plated into StemSpan Serum-Free Expansion Medium (SFEM™, StemCell Technologies) containing 100 ng/mL each of human stem cell factor (SCF) and flt-3 ligand (FL), 20 ng/mL each of thrombopoietin (TPO) and IL-6, and 10 µM PGE2 (Cayman Biochemicals; all other supplements were from PeproTech® unless otherwise indicated). Cells were grown for 3 days in a humidified incubator and 5% CO₂ 20% O₂. On day 3 (morning), media was replaced with fresh Stemspan-SFEM™ supplemented with human SCF, TPO, FL and PGE2. In the afternoon of day 3, 2.2 million CD34⁺ cells per sample were suspended in electroporation buffer.

The gRNAs were generated by *in vitro* transcription using a T7 polymerase. A 5' ARCA cap was added to the RNA simultaneous to transcription while a polyA tail was added after transcription to the 3' end of the RNA species by an *E. coli* polyA polymerase. After the gRNAs were *in vitro* transcribed and tailed, the quality and quantity of gRNAs were evaluated with the Bioanalyzer (Nanochip) to determine RNA concentration and by DSF assay performed as a measure of D10A Cas9 nickase RNP stability and as an indirect measure of gRNA quality.

*In vitro* transcribed capped and tailed guide gRNAs *HBB*-8 (SEQ ID NO:398) and *HBB*-15 (SEQ ID NO:397) were added at a 2:1 molar ratio (total gRNA : Cas9 protein) to 10µg RNP per million cells. The RNPs tested include the following: wild-type (WT) Cas9, endotoxin-free WT Cas9, N863A nickase, D10A nickase. For each experimental sample, the D10A nickase RNP/cell mixture was transferred to the cells then electroporated.

For all samples, the cells were collected and placed at 37°C for 20 minutes (recovery period). For the human CB CD34⁺ cells that were contacted with 10µg D10A nickase RNP (per million cells), one sample was transferred to pre-warmed cytokine supplemented Stemspan-SFEM™ media and placed at 30°C for 2 hours (cold shock recovery) or placed directly into the same media at 37°C. For the cold shocked samples, the cells were transferred to the 37°C incubator after the 2-hour incubation period at 30°C. At 24, 48, and 72 hours after electroporation, the human CB CD34⁺ cells were counted by trypan blue exclusion (cell survival) and divided into 3 portions for the following analyses: a) flow cytometry analysis for assessment of viability by co-staining with 7-Aminoactinomycin-D (7-AAD) and allophycocyanin (APC)-conjugated Annexin-V antibody (eBioscience); b) flow cytometry analysis for maintenance of HSC phenotype (after co-staining with phycoerythrin (PE)-conjugated anti-human CD34 antibody (BD Biosciences) and APC-conjugated CD133 (Miltenyi Biotech; c) hematopoietic colony forming cell (CFC) analysis by plating 800 cells in semi-solid methylcellulose based Methocult medium (StemCell Technologies H4435) that supports differentiation of erythroid and myeloid blood cell colonies from HSCs and serves as a surrogate assay to evaluate HSC multipotency and differentiation potential ex vivo; d) genomic DNA analysis for detection of editing at the *HBB* locus; and e) Western blot analysis of protein to evaluate the stability of Cas9 RNP in human CD34⁺ HSCs. gDNA was extracted from the HSCs at 48 and 72 hours after electroporation and *HBB* locus-specific PCR reactions were performed. The purified PCR products were analyzed for insertions/deletions (indels) in T7E1 assays and by DNA sequencing of individual clones (PCR product was transformed and subcloned into TOPO-vector, individual colonies picked, and plasmid DNA containing individual PCR products were sequenced).

Electroporated human CB CD34⁺ cells maintained a stem cell phenotype (e.g., co-expression of CD34 and CD133, >90% CD34⁺CD133⁺) as determined by flow cytometry analysis. The absolute number of viable human CD34⁺ cells was maintained across most samples over a 72-hour culture period after electroporation **(****Fig. 41A****).** Gene edited human CD34⁺ cells maintained *ex vivo* hematopoietic activity and multipotency as indicated by their ability to give rise to erythroid (e.g., CFU-E, or CFU-GEMM) and myeloid (e.g., CFU-G, -M, or -GM) cells **(****Fig. 41B****).**

Gene editing at the *HBB* locus was then evaluated at 72 hours after electroporation of WT Cas9, N863A, or D10A nickases co-delivered with 2 gRNAs (*HBB*-8 (SEQ ID NO:398) and *HBB*-15 (SEQ ID NO:397)). Briefly, gDNA was isolated from electroporated human CD34⁺ cells at 72 hours after electroporation, and PCR amplification of a ∼607bp fragment of the *HBB* locus (which captured both of the individual genomic locations that were targeted by gRNAs *HBB-8* (SEQ ID NO:398) and *HBB*-15 (SEQ ID NO:397) was performed. After cleanup of the *HBB* PCR product with AMPURE beads, insertions/deletions (indels) at the targeted genomic location were evaluated by T7E1 assay and by DNA sequencing. For the CD34⁺ cells electroporated with WT Cas9 and endotoxin-free WT Cas9 the percentages of indels detected by T7E1 analysis at 72 hours was 59% and 51%, respectively **(****Fig. 42A****),** which correlated with the indels detected by DNA sequencing **(Table 6).** Human CD34⁺ cells electroporated with N863A Cas9 nickase and *HBB*-specific gRNA pair had only 1% indels detected by T7E1 analysis. CD34⁺ HSCs electroporated with D10A nickase with and without cold shock supported gene editing at percentages of 39% and 48% indels detected by T7E1 analysis, respectively. In order to confirm that this low level of editing observed in human CD34⁺ HSCs contacted with N863A nickase, was not due to the lack of N863A nickase RNP contacting the cells, western blot analysis was performed **(****Fig. 42B****).**

Cas9 protein was present in all electroporated samples (e.g., cells that received WT Cas9, D10A nickase, and N863A nickase). For these samples, Cas9 protein was detected at 24 and 48 hours after electroporation, suggesting that the lack of N863A nickase activity in the human CD34⁺ HSCs was not due to the lack of protein.

Gene editing in human CD34⁺ HSCs that contacted WT Cas9, endotoxin-free Cas9, and D10A nickase was 54-60%, based on DNA sequencing analysis **(Table 6,** **Fig. 43A****).** Stratification of DNA repair events through DNA sequencing analysis revealed that >90% of the gene editing events were deletions in human CD34⁺ HSCs that contacted WT Cas9 and endotoxin-free (EF) WT Cas9 (insertions or combination of insertion and deletion comprised the remaining 3-6% of editing events) **(****Fig. 43A****).** In contrast, gDNA from human CD34⁺ HSCs that contacted D10A nickases had 3% HDR (e.g., gene conversion), up to 75% insertions, and up to 22% deletions **(****Fig. 43B****).**

Without wishing to be bound to any theory, the data in this Example suggest: 1) endotoxin removal does not negatively impact Cas9 functionality or human CD34⁺ HSC cell viability, proliferation potential, or multipotency; 2) use of 10 µg D10A nickase RNP supports 60% gene editing in human CD34⁺ HSCs with HDR events (e.g., gene conversion); and 3) after contacting human CD34⁺ HSCs, WT Cas9 and nickase RNPs are detected for up to 48 hours, but is not detectable thereafter.

Table 6 shows a summary of gene editing results in human CB CD34⁺ cells 72 hours after co-delivery of wild-type Cas9, N863A nickase, D10A nickase RNP and *HBB*-specific gRNA pair.

**Table 6**

| Cas9 | Total µg RNP/1E6 cells | Temperature of 2-hour recovery | %indels (T7E1) | %indels (sequencing) |
|---|---|---|---|---|
| WT | 10 | 37°C | 59 | 56 |
| Endo-Free WT | 10 | 37°C | 51 | 60 |
| N863A | 10 | 37°C | 1 | ND |
| D10A | 10 | 37°C | 39 | 60 |
| D10A | 10 | 30°C | 48 | 54 |

### Example 14: Gene editing at the HBB locus in human CD34⁺ hematopoietic stem/progenitor cells after delivery of Cas9 protein complexed to in vitro transcribed modified gRNAs engineered with a polyA tail encoded in a DNA template

### Encoding poly-A tail in the DNA template that encodes the gRNA.

Adult human hematopoietic stem/progenitor cells (HSCs) electroporated with Cas9 and gRNAs that were unmodified (e.g., absence of ARCA cap and polyA tail) had reduced survival, viability, and hematopoietic potential and low percentages of gene editing compared to adult human HSCs that were electroporated with *in vitro* transcribed capped and tailed gRNAs **(****Figs. 24A-C** **and** **Figs. 25A-G****).** For all HSPC experiments, the DNA template that encoded gRNAs contained a modified T7 promoter sequence as described in **Example 19,** e.g., for gRNA target sequences that start with G (e.g., *HBB-*8), the GG at the 3' end of the T7 promoter sequence was removed and for gRNA target sequences that did not start with G (e.g., *HBB*-15)*,* the last G at the 3' end of the T7 promoter sequence was removed. After *in vitro* transcription of the ARCA capped gRNA (mMessage Machine™ T7 Ultra Transcription Kit, Ambion), the gRNA was incubated with *E. coli* PolyA Polymerase (E-PAP), and the capped/tailed gRNA was then cleaned up using the MegaClear™ Kit (Ambion). The polyA tail added by E-PAP tailing reaction varied between experiments. In order to standardize the length of the polyA tail at the 3' end of the gRNA, the 3' antisense primers encoding the gRNA tracr were altered to contain specific length polyT sequences, which results in a DNA template for the specific length polyA tail. The length of poly A tails generated by the antisense primers were: 10, 20, 50, and 100. The DNA templates encoding *HBB* specific gRNAs (*HBB*-8 (SEQ ID NO:398) and *HBB*-15 (SEQ ID NO:397)) were generated by PCR and *in vitro* transcribed with the mMessage Machine™ T7 Ultra Transcription Kit according to the manufacturer's protocol with one modification: the E-PAP tailing reaction was omitted since the poly A tail was encoded in the DNA template.

The PCR products generated from the reactions for in vitro transcription DNA templates for the *HBB* gRNAs with 10, 20, and 50 length poly A tails yielded clean PCR products (**Fig. 44A**). In contrast, the DNA template for the *HBB* gRNAs with 100 length poly A tails yielded several products of different sizes. Therefore, *in vitro* transcription was only performed with the *HBB* gRNA DNA templates with the 10A, 20A, and 50A length polyA tails encoded in the templates. The purified PCR products were *in vitro* transcribed with the mMessage Machine™ T7 Ultra Transcription Kit excluding the E-PAP tailing reaction, since the tails were encoded in the DNA template for each gRNA. Bioanalyzer results of the *HBB* gRNA products indicated gRNAs were generated of the appropriate size products consistent with the DNA templates **(****Fig. 44B****)** and the poly A tail lengths when encoded in the DNA templates yielded gRNA products of defined size compared to gRNAs generated with a poly A tail generated enzymatically by incubation with E-PAP.

In this example, human umbilical cord blood CD34⁺ HSCs were then electroporated with D10A Cas9 RNP complexed with *HBB*-8 (SEQ ID NO:398) and *HBB*-15 (SEQ ID NO:397) gRNAs with polyA tails of defined lengths (e.g., 10A, 20A, 50A). Viability analysis by flow cytometry (AnnexinV⁻7AAD⁻) indicated no difference in the percentage of live CD34⁺ cells 72 hours after electroporation with D10A Cas9 RNP complexed with gRNAs with engineered poly A tails of defined lengths compared to cells that were electroporated with D10A Cas9 RNP and gRNAs with polyA tails added enzymatically with E-PAP **(****Fig. 45A-45B****)**. Gene editing for cells contacted with D10A RNP and gRNA pair (*HBB*-8 (SEQ ID NO:398) and *HBB*-15 (SEQ ID NO:397)) containing 10A or 20A length polyA tail was ∼51% based on T7E1 endonuclease assay **(****Fig. 45C****).** DNA sequence analysis by Sanger DNA sequencing of the *HBB* locus confirmed 61% and 59% gene editing was achieved in human CD34⁺ cells electroporated with Cas9 RNP containing gRNAs that were modified to have a 10A or 20A length tail, respectively **(****Fig. 45C****).** These findings indicate that D10A Cas9 protein complexed to gRNAs modified to include a 5' cap and 3' polyA tail of specific length supported gene editing in primary human hematopoietic stem/progenitor cells.

### Example 15: A gRNA with a specified length of polyA tail can be used to generate viable, edited primary T cells

In order to determine whether a gRNA with a specified 3' poly-A tail length can be used to generate edited primary T cells, primary CD4+ T cells were electroporated with RNP complexes targeting *PDCD1* or *TRBC.* ARCA-capped gRNAs, as described in **Example 10,** with a polyA tail of 10 or 20 were generated by *in vitro* transcription from polyA tail-encoded templates. The gRNAs were complexed with purified *S*. *pyogenes* protein to generate a RNP that targets *PDCD1* (*PDCD1-108* (SEQ ID NO:399)) or *TRBC* (*TRBC-210* (SEQ ID NO:388)). Briefly, the templated polyA gRNAs were complexed with *S. pyogenes* Cas9 at a 2:1 molar ratio (gRNA:protein) for 15 minutes at room temperature prior to electroporation. The RNP complexes were delivered to primary T cells using electroporation. The primary T cells were activated prior to treatment using anti-CD3/CD28 conjugated activation beads. Post-electroporation, cells were allowed to recover for 72hrs before taking a subset of the *PDCD1*-targeted cells for reactivation and subsequent PD1 FACS analysis. The *TRBC* targeted cells were cultured without stimulation post electroporation. Viability of the cells was monitored for the first 72 hours after electroporation **(****Fig. 46A** and **Fig. 47A****).** FACS analysis of PD1 and CD3 expression was performed 6 days after electroporation. Both the 10A and 20A templated gRNA containing RNPs were capable of generating significantly modified primary CD4+ T cells at both the *PDCD1* and *TRBC* locus **(****Fig. 46B** and **Fig. 47B****).** These findings demonstrate that primary T cells can be effectively edited with a gRNA that is modified to contain a polyA tail of a specified length.

### Example 16: Addition of a 20A polyA tail to gRNA improves gene editing by wild-type CRISPR/Cas9 protein in human CD34⁺ cells without reducing cell viability of hematopoietic activity.

Previous experiments described earlier in this document indicated that modification of the 5' and 3' ends of gRNAs (e.g., addition of ARCA cap at the 5' end and enzymatic addition of a polyA tail at the 3' end by treatment with E-PAP) increases HSC viability and supports efficient gene editing in HSCs. The purpose of the current example was to determine whether an optimal, minimal, and specific 3' end modification could be defined (e.g., poly-adenylation was the only 3' end modification). The criteria for selecting a 3' end modification is that the 3' end modification does not reduce HSC viability and functionality (e.g., hematopoietic activity, e.g., colony forming potential) when complexed to Cas9 protein and electroporated into human HSCs and also is able to support efficient gene editing (e.g., high percentage of indel formation at targeted locus). In this example, several different modifications were engineered into the PCR DNA template for *in vitro* transcription of the gRNAs. For all gRNAs differentially modified at the 3' end, each gRNA was in vitro transcribed and was modified at the 5' end with an ARCA cap which was added during the in vitro transcription process. The *HBB* genetic locus was targeted for gene modification in which the sgRNA *HBB*-8 was modified to have the following 3' end modifications: polyA tail of varying lengths (e.g., 2A, 5A, 10A, 15A, 20A, and 25A), poly(T) tail (e.g., 10T, 20T), and poly(G) tail (e.g., 10G, 20G). These modifications were assessed in the DNA IVT template by gel electrophoresis and in the gRNA product with the Bioanalyzer. After validation, the gRNAs were subjected to QC analysis in DSF shift assays, in which wild-type Cas9 protein was complexed with different molar ratios of gRNA to determine the optimal molar ratio of Cas9 protein: gRNA (e.g., 1:1, 1:1.5, 1:2, etc) at which the Cas9 protein was fully occupied or complexed with gRNA. Human CB CD34⁺ cells were then electroporated (e.g., Amaxa Nucleofector) with Cas9 RNP in which the gRNAs complexed to Cas9 protein were differentially modified on the 3' end. Three days after electroporation, the CB CD34⁺ cells were analyzed for fold expansion (e.g., viability), HSC functionality (e.g., plated into CFC assays to test differentiation potential), and gDNA extracted from samples for assessment of gene editing at the target locus by T7E1 assay and DNA sequencing analysis of the human *HBB* locus specific PCR products. In this experiment, gRNAs with polyA tail modifications supported the highest frequency of gene editing, as determined both by T7E1 assay analysis (**Fig. 48A**) and DNA sequencing analysis (**Fig. 48B**). DNA sequencing analysis indicated that the optimal polyA tail length was 20As. CFC analysis of colony forming potential of the RNP treated cells indicated that gene edited CD34⁺ cells treated with RNP containing a gRNA with the 5' and 3' end maintained colony forming potential in comparison to untreated (negative) control cells from the same HSC donor.

### Example 17: Combination of 5' end and 3' end modifications to gRNAs increase gene editing in HSCs that maintain their viability and hematopoietic activity ex vivo using a CRISPR/Cas9 D10A dual nickase approach

The purpose of this example was to determine whether both 5' and 3' end modifications are required to achieve both high gene editing and maintain HSC viability and functionality. In this example, the D10A Cas9 variant protein was complexed to modified *HBB-8* (SEQ ID NO:398) and *HBB-15* (SEQ ID NO:397) gRNAs (e.g., each modified gRNA was complexed with D10A protein). The two D10A RNP complexes in which each gRNA had the same 5' and 3' end modification were mixed and electroporated (Amaxa Nucleofector) into CB CD34+ cells (**Fig**. **49A**). Three days after electroporation, CD34⁺ cells were analyzed for gene editing (e.g., T7E1 assay analysis of PCR products of the *HBB* locus) and by hematopoietic functionality ex vivo (e.g., CFC assay). In cells electroporated with Cas9 RNP containing gRNAs with no 5' or 3' end modifications, no gene editing was detected by T7E1 assay analysis (**Fig**. **49A**). In contrast, addition of either a 5' ARCA cap, a 3' polyA tail (e.g., 10A or 20A), or both a 5' cap and 3' polyA tail supported ∼10% gene editing at the *HBB* locus. When a combination of 5' and 3' end modifications were used (e.g., two gRNAs modified to contain both a 5' ARCA cap and a 3' polyA tail), gene editing increased (e.g., about a 2.5-fold increase with the combination of a 5' ARCA cap and 3' 20A polyA tail compared to addition of a 5' ARCA cap, a 3' 10A polyA tail alone, or a 3' 20A polyA tail alone). For all samples treated with RNPs containing gRNAs with different modifications, CFC potential was maintained (**Fig. 49A****,** right panel).

In order to validate that the combination of 5' and 3' end modifications had a positive impact on both HSC maintenance and gene editing, CD34⁺ cells from another CB donor were electroporated with in vitro transcribed gRNAs (*HBB*-8 and *HBB-*15) complexed to D10A Cas9 nickase protein. Here, the gRNAs had a 5' cap and 3' 20A tail or unmodified 5' end and a 20A polyA tail. Both samples had substantial levels of gene editing as determined by DNA sequence analysis (**Fig. 49B**). The subtypes of editing events detected include insertions, deletions, and gene conversion (HDR using *HBD* genomic sequence as DNA repair template).

Furthermore, any differences between the two samples in total gene editing levels (35% and 47%) could not be attributed to the gRNA modification, in that 10-20% is the normal sample to sample variability observed between electroporation replicates using the same RNP preparations and HSC donor. Importantly, the cells electroporated with D10A RNP containing gRNAs with both 5' cap and 3' polyA tail had higher fold expansion and colony forming potential, compared to cells treated with RNP containing 3' modified gRNAs, suggesting that the combination of 5' and 3' end modifications, maintained HSC expansion and colony forming potential (**Fig. 49B****,** middle and right panels).

In order to confirm the synergistic effect of modifying both the 5' and 3' ends of the gRNA an additional CB CD34⁺ cells were obtained from an additional donor, and then the cells were electroporated with in vitro transcribed gRNAs (*HBB*-8 and *HBB*-15) complexed to D10A protein. In this experiment, cells treated with RNP that contained unmodified gRNAs had the lowest level of gene editing, followed by cells treated with RNP that contained capped gRNAs (**Fig. 49C**). T7E1 analysis suggested no difference in the editing achieved by RNP containing gRNAs with a polyA tail (20A) alone and RNP containing capped/polyA tailed gRNAs. Gene editing was lower in cells treated with RNP containing unmodified gRNAs or gRNAs with a 5' cap alone. However, analysis of hematopoietic activity (CFC potential) indicated that cells treated with RNP containing unmodified gRNAs or gRNAs modified at the 3' end alone also gave rise to fewer hematopoietic colonies (**Fig. 49C**, right panel). These data suggest that modification of gRNAs with a 5' cap and 3' polyA tail of defined length (20A) supports gene editing in both adult and cord blood CD34+ cells that maintain their expansion, viability, and hematopoietic potential ex vivo.

### Example 18: CRISPR/Cas9 RNP supports highly efficient gene editing and HDR at the HBB locus in human adult and cord blood CD34⁺ hematopoietic stem/progenitor cells from 15 different stem cell donors

To determine the reproducibility of Cas9 RNP mediated gene editing in hematopoietic stem/progenitor cells, cryopreserved CD34⁺ cells obtained from 15 different patient donors (12 cord blood CD34⁺ cell donors and 3 adult mobilized peripheral blood CD34⁺ cell donors) were thawed into StemSpan SFEM medium with human cytokines (SCF, TPO, FL, and IL6), 10 µMPOPE₂ with or without 1 µM SRI for 48-72 hours and then electroporated with *S. pyogenes* Cas9 RNP (D10A nickase or WT) pre-complexed to gRNA targeting *HBB* (D10A nickase pre-complexed with *HBB-8* or *HBB-15* gRNAs and the 2 pre-complexed RNPs mixed and added to the CD34⁺ cells) or *AAVS1* (WT Cas9 with sgRNA AAVS1-1). For all experiments described in this example, gRNAs were *in vitro* transcribed from a PCR template that encodes a modified T7 promoter, gRNA, and a polyA tail (20A) 3' to the gRNA. An ARCA cap was added to the 5' end of the gRNA in the *in vitro* transcriptions process, thus, all gRNAs tested in these experiments were modified gRNAs *(i.e.,* modified at 5' end with ARCA cap and 3' end with polyA tail). For the 15 donor CD34⁺ cell populations tested in separate experiments, 5 experiments were conducted to test gene editing with WT Cas9 RNP delivery (sgRNA, either AAVS1-1 or *HBB*-8) and 10 experiments were conducted to test editing with D10A nickase and 2 gRNAs *(HBB-8* (SEQ ID NO:398). and *HBB*-15 (SEQ ID NO:397)).

Composite analysis across the 15 separate experiments and donors showed 57% editing (mean ± stdev: 56.9 ± 8%) in cord blood CD34⁺ cells (56% with WT Cas9 RNP + sgRNA, 58% D10A RNP + 2 gRNAs) and 51% editing (mean 51.3±10%) in adult mobilized peripheral blood CD34⁺ cells (**Figure 50A**). Gene editing was determined by DNA sequencing analysis of genomic DNA that was extracted from CD34⁺ cells electroporated with Cas9 RNP. In depth analysis of the subtypes of editing events that occurred after CD34⁺ cells contacted Cas9 D10A RNP and 2 gRNAs (*HBB-*8 (SEQ ID NO:398) and *HBB*-15 (SEQ ID NO:397) showed that a mean of 31±11% of the events were insertions (range 11-41%), 14±6% were small deletions (range 5-17%), and 3±2% were repaired through gene conversion or HDR (range 2-7%) (**Figure 50B**). Given that human CD34⁺ cells are highly sensitive to perturbation by electroporation or by contact with foreign proteins and nucleic acid, the viability of HSCs after contacting Cas9 RNP was evaluated by flow cytometry analysis for detection of viable (7-AAD⁻ AnnexinV⁻) CD34⁺ cells. The percentage of viable CD34⁺ cells was measured by flow cytometry analysis and then the percentage of live CD34⁺ cells was multiplied by the total cell number which was determined by trypan blue exclusion after electroporation and then divided by the input cell number in order to calculate the fold expansion of untreated and RNP electroporated (treated) CD34⁺ cells from the same donor. The mean and standard deviation of RNP treated and control (*i.e*., untreated) cells for multiple donors (n=10) is shown **(****Figure 50C**). For each stem cell donor, the fold expansion of untreated control and RNP treated CD34⁺ cells from each donor was compared in a paired 2-tailed t-test to determine if RNP contact altered cell viability. Statistical analysis of these data showed no statistically significant difference between RNP treated and controlled CD34⁺ cells (RNP treated vs. control mean fold expansion: 1.5 vs. 2.0; P-value summary not significant, P-value=0.1217). To determine whether RNP treatment and gene editing affected HSC multipotency and differentiation potential, the mean colony forming cell potential (CFCs) and individual colony subtypes were scored and then analyzed in paired t-test (n=10). There were no significant differences detected in the total CFCs or the subsets of CFCs between RNP treated and control CD34⁺ cells **(****Figure 50D**). The level of disruption in individual CD34⁺ cells was then determined by DNA sequencing analysis of CFCs (each CFC is differentiated from a single CD34⁺ cells, thereby allowing for single cell analysis of HSCs by assaying the clonal progeny of the plated cells). DNA sequencing analysis of the *HBB* locus PCR products showed higher levels of gene editing detected the erythroid and myeloid progeny of the CD34+ (∼80-90% edited CFCs) from differentiated from mPB HSCs and CB HSCs (**Figure 50E**). Monoallelic and biallelic editing of the locus was detected. A 2-hour cold shock after electroporation and before plating cells into colony assays altered the distribution of monoallelic and biallelic editing detected in the myeloid and erythroid CFC progeny of the edited CD34+ cells (**Figure 50E and 50F**).

### Example 19: Modification of T7 promoter sequence for optimal activity of in vitro transcribed gRNAs

### Modification of T7 promoter sequence:

The DNA template encoding a T7 promoter (TAATACGACTCACTATAGG (SEQ ID NO:401)) or a modified T7 promoter (different variants described below), a 20-nt target sequence (CCR5 U43 (SEQ ID NO:488)), and the chimeric gRNA scaffold was assembled by PCR. The PCR's 5' sense oligonucleotide consisted of the T7 promoter, gRNA targeting sequence (which is modified for each specific gRNA target site), and sequence from the 5' end of the *S. pyogenes* tracr sequence (GTTTTAGAGCTAGAAATA (SEQ ID NO:402)). The 3' anti-sense oligonucleotide (AAAAGCACCGACTCGGTGCCACTTTTTCAAGTTGATA (SEQ ID NO:403)) was the reverse complement to the 3' end of the *S. pyogenes* tracr sequence. The DNA template for the PCR reactions was a plasmid containing the *S. pyogenes* gRNA tracr sequence. The amplified PCR product which was used as a DNA template for *in vitro* transcription of a target specific gRNA encoded the following: modified T7 promoter-gRNA target-tracr (description of modifications to T7 promoter is described below).

Given that the T7 RNA polymerase requires a G to initiate transcription, the T7 promoter typically has two Gs at its 3' end to ensure transcription of the entire RNA sequence downstream of the promoter. The consequence, however, is that the transcript that is produced may contain at least one if not both of the Gs from the promoter sequence, which may alter the gRNA specificity or the interaction between the gRNA and the Cas9 protein. To address this concern in cases where the gRNA target sequence starts with a G (e.g., *HBB_*8 gRNA target region DNA template: GTAACGGCAGACTTCTCCTC (SEQ ID NO:404)), the two GGs were removed from the T7 promoter sequence in the gRNA PCR template by designing a new 5' sense primer (CACCGCTAGCTAATACGACTCACTATAGTAACGGCAGACTTCTCCTCGTTTTAGAGC TAGAAATA (SEQ ID NO:405) where the modified T7 promoter sequence is underlined). For gRNA target sequences that don't start with a G (e.g., *HBB*_15 gRNA target region DNA template: AAGGTGAACGTGGATGAAGT (SEQ ID NO:406)), the T7 promoter sequence encoded in the gRNA PCR template was modified such that only one of the Gs at the 3' end of the T7 promoter was removed: (modified T7 promoter sequence: TAATACGACTCACTATA**G** (SEQ ID NO:487)).

A T7 promoter sequence and modiftied T7 promoter sequence is not limited to the sequences described herein. For example, T7 promoter sequences (and modifications thereof) can be at least any of the sequences refered to in "Promoters/Catalog/T7" of the Registry of Standard Biological Parts (located at the following http:// address: parts.igem.org/Promoters/Catalog/T7). It is to be understood that the present disclosure encompasses methods where a gRNA of the invention is prepared by *in vitro* transcription from a DNA template that includes a modified T7 promoter as described herein where one or more of the 3' terminal Gs have been removed (e.g., where the sequence TAATACGACTCACTATA**G** (SEQ ID NO:487) is located immediately upstream of a targeting domain that lacks a G at it's 5' end or the sequence TAATACGACTCACTATA (SEQ ID NO:407) is located immediately upstream of a targeting domain that has a G at it's 5' end). Other variations on these modified T7 promoters will be recognized by those skilled in the art based on other T7 promoter sequences including at least any of the sequences refered to in "Promoters/Catalog/T7" of the Registry of Standard Biological Parts (located at the following http:// address: parts.igem.org/Promoters/Catalog/T7 ).

To determine whether additional Gs at the 5' end of the gRNA sequence alters gene editing of Cas9 RNP, DNA templates encoding the gRNAs were generated by PCR with a 5' sense primer containing the T7 promoter (TAATACGACTCACTATA**GG** (SEQ ID NO:401)) or modified T7 promoter (TAATACGACTCACTATA (SEQ ID NO:407)) and gRNA sequences. The gRNAs were generated by *in vitro* transcription of the DNA templates with the Message Machine™ T7 Ultra Transcription Kit (Ambion) **(Table 7).** The single gRNAs *in vitro transcribed* from the T7 or modified T7 promoters (*S. pyogenes* gRNAs: CCR5_U43, AAVS1_1, *S aureus* gRNA: CXCR4-836 which all include a G at the 5' end of the targeting sequence) were complexed with wild-type Cas9 RNP and then delivered by transfection (lipofectamine) into 293FT cells. For the synthesized gRNAs, addition of two GGs to the 5' end of these gRNAs reduced gene editing in 293FT cells from 48-52% to 0-10%, as indicated by T7E1 endonuclease assay analysis **(Table 7).** These data indicate that two GGs added to 5' end of gRNA sequence reduced gene editing by Cas9 RNP.

Table 7 shows the percentage of gene editing in 293FT cells after co-delivery of wild-type Cas9 RNP with in vitro transcribed single gRNAs with two GGs added to 5' end of sequence in the DNA template.

**Table 7**

| gRNA | **Sequence of 5' sense primers used for PCR production of gRNA DNA template** (T7promoter "**GG**"target *traer forward primer*) | % Indels (T7E:1 assay) |
|---|---|---|
| *S. pyogenes* CCR5_U43 | CACCGCTAGCTAATACGACTCACTATAGCTGCCGCCC AGTGGGACTTGTTTTAGAGCTAGAAATA (SEQ ID NO:408) | 52% |
| *S. pyogenes* CCR5_U43 +GG | CACCGCTAGCTAATACGACTCACTATAGGGCTGCCG CCCAGTGGGACTTGTTTTAGAGCTAGAAATA (SEQ ID NO:409) | 10% |
| *S. aureus* CXCR4-836 | CACCGCTAGCTAATACGACTCACTATAGCTCCAAGG AAAGCATAGAGGAGTTTTAGTACTCTGGAAA (SEQ ID NO:410) | 52% |
| *S. aureus* CXCR4-836 + GG | CACCGCTAGCTAATACGACTCACTATAGGGCTCCAA GGAAAGCATAGAGGAGTTTTAGTACTCTGGAAA (SEQ ID NO:411) | 0% |
| *S. pyogenes* AAVS1_1 | CACCGCTAGCTAATACGACTCACTATAGTCCCCTCCA CCCCACAGTGGTTTTAGAGCTAGAAATA (SEQ ID NO:412) | 48% |
| *S. pyogenes* AAVS1_1 +GG | CACCGCTAGCTAATACGACTCACTATAGGGTCCCCTC CACCCCACAGTGGTTTTAGAGCTAGAAATA (SEQ ID NO:413) | 0% |

### Example 20: CRISPR/Cas9 RNP supports gene editing and gene correction of Sickle Cell Disease patient T lymphocytes

To determine whether Cas9 RNP mediated gene editing would correct the sickle cell disease (SCD) mutation, peripheral blood mononuclear cells (MNCs) from a SCD patient was obtained (Conversant Bio). To expand the T lymphocyte fraction from bulk MNCs, MNCs were thawed into T cell culture media (X-VIVO 15 supplemented with human AB serum, N-acetylcysteine, Glutamax (L-glutamine), and human cytokines (IL2, IL7, IL15). The T lymphocyte fraction was activated with CD3/CD28 immunomagnetic beads. By day 3 of MNC culture in T cell media, the cell population was 72% CD3⁺. After 7 days a population of > 98% CD3⁺ T lymphocytes was obtained, most of which were also CD4⁺ (**Fig. 51A**). Genomic (g)DNA was extracted from patient CD3⁺ T lymphocytes and sequenced to confirm the presence of the SCD mutation.

After confirmation of the sickle mutation in the patient T lymphocytes, sgRNA was designed to target the SCD mutation (*HBB*-8-sickle gRNA targeting sequence: GUAACGGCAGACUUCUCCAC (SEQ ID NO:414), underline indicates SCD mutation). The gRNA was *in vitro* transcribed from DNA PCR product template. The *HBB*-8-sickle was modified to contain a 5' ARCA cap and a 3' polyA tail. Another gRNA *HBB*-15 (SEQ ID NO:397) was also *in vitro* transcribed and was modified to contain a 5' ARCA cap and a 3' polyA tail. The gRNAs were complexed to *S. pyogenes* Cas9 protein for RNP production.

To evaluate Cas RNP editing in SCD patient cells, 2 million cells were electroporated with D10A Cas9 RNP in which the protein was complex to 2 different gRNA pairs to target the sequence specific to the *HBB* gDNA of SCD patients (*HBB*-8-sickle (SEQ ID NO:414) and *HBB-*15 (SEQ ID NO:397)). Dual D10A nickase RNP (10µg per million cells) was co-electroporated with a single strand oligonucleotide donor (SSODN, 250 pmoles per million cells). After electroporation, cells were plated into T cell media with or without CD3/CD28 immunomagnetic beads to determine whether restimulation of T cells after electroporation would improve survival and viability of gene edited cells. Re-plating the edited cells into culture with CD3/CD28 beads for cell reactivation improved the total viability of the electroporated cells in comparison to cells plated in T cell media without CD3/CD28 beads (% viability determined by flow cytometry analysis after staining with apoptosis stains 7-AAD and Annexin V; **Fig. 51B**). Gene editing was evaluated by DNA sequencing. The gRNA combination *HBB*-8-sickle (SEQ ID NO:414) and *HBB-15* (SEQ ID NO:397) (each complexed to D10A Cas9 protein) supported 48% total editing, as detected by T7E1 endonuclease assay analysis of the *HBB* PCR product (**Fig**. **51C**). Ten percent higher editing was detected in gDNA from the T lymphocytes that were reactivated with CD3/CD28 beads after electroporation, compared to cells cultured in T cell medial alone. These data show that Cas9 RNP targeting the SCD mutation supports gene editing in SCD patient cells.

## Claims

1. A composition comprising a gRNA molecule comprising a targeting domain which is complementary with a target domain from a gene expressed in a eukaryotic cell, wherein the gRNA molecule:
(a) comprises a 3' poly A tail; and
(b) is complexed with a Cas9 protein.

2. The composition according to claim 1, wherein the gRNA molecule is modified at its 5' end such that it lacks a 5' triphosphate group.

3. The composition according to any one of the preceding claims, wherein the gRNA molecule is complexed with the Cas9 protein in a ribonucleoprotein (RNP) complex.

4. The composition according to claim 1, wherein the 3' polyA tail comprises between 15 and 25 adenine nucleotides.

5. An *in vitro* or *ex vivo* method of editing or modulating expression of a gene in a eukaryotic cell from a subject, the method comprising contacting the cell with a composition comprising a gRNA molecule comprising a targeting domain which is complementary with a target domain from a gene expressed in a eukaryotic cell, wherein the gRNA molecule:
(a) comprises a 3' poly A tail; and
(b) complexed with a Cas9 protein.

6. The *in vitro* or *ex vivo* method according to claim 5, wherein the gRNA molecule is modified at its 5' end such that it lacks a 5' triphosphate group.

7. The *in vitro* or *ex vivo* method according to claim 5, wherein the gRNA molecule is complexed with the Cas9 protein in a ribonucleoprotein (RNP) complex.

8. The *in vitro* or *ex vivo* method according to claim 5, wherein the 3' polyA tail comprises between 15 and 25 adenine nucleotides.

## Patentansprüche

1. Zusammensetzung, die ein gRNA-Molekül umfasst, das eine Targeting-Domäne umfasst, die komplementär zu einer Target-Domäne eines in einer eukaryotischen Zelle exprimierten Gens ist, wobei das gRNA-Molekül:
(a) einen 3'-Poly-A-Schwanz aufweist; und
(b) mit einem Cas9-Protein komplexiert ist.

2. Zusammensetzung gemäß Anspruch 1, wobei das gRNA-Molekül an seinem 5'-Ende so verändert wurde, dass eine 5'-Triphosphatgruppe fehlt.

3. Zusammensetzung gemäß einem der vorhergehenden Ansprüche, wobei das gRNA-Molekül mit dem Cas9-Protein zu einem Ribonukleoprotein (RNP)-Komplex komplexiert ist.

4. Zusammensetzung gemäß Anspruch 1, wobei der 3'-PolyA-Schwanz zwischen 15 und 25 Adenin-Nukleotide umfasst.

5. *In vitro-* oder ex vivo-Verfahren zum Editieren oder Modulieren der Expression eines Gens in einer eukaryotischen Zelle eines Subjekts, wobei das Verfahren Schritte umfasst, bei denen man die Zelle mit einer Zusammensetzung in Kontakt bringt, die ein gRNA-Molekül umfasst, das eine Targeting-Domäne umfasst, die komplementär zu einer Target-Domäne eines in einer eukaryotischen Zelle exprimierten Gens ist, wobei das gRNA-Molekül:
(a) einen 3'-Poly-A-Schwanz aufweist; und
(b) mit einem Cas9-Protein komplexiert ist.

6. *In vitro-* oder *ex vivo*-Verfahren gemäß Anspruch 5, wobei das gRNA-Molekül an seinem 5'-Ende so verändert wurde, dass ihm eine 5'-Triphosphatgruppe fehlt.

7. *In vitro-* oder *ex vivo*-Verfahren gemäß gemäß Anspruch 5, wobei das gRNA-Molekül mit dem Cas9-Protein zu einem Ribonukleoprotein (RNP)-Komplex komplexiert ist.

8. *In vitro-* oder *ex vivo*-Verfahren gemäß Anspruch 5, wobei der 3'-PolyA-Schwanz zwischen 15 und 25 Adenin-Nukleotide umfasst.

## Revendications

1. Composition comprenant une molécule d'ARNg comportant un domaine de ciblage qui est complémentaire d'un domaine cible d'un gène exprimé dans une cellule eucaryote, laquelle molécule d'ARNg :
a) comporte une queue 3' polyA,
b) et est complexée avec une protéine Cas9.

2. Composition conforme à la revendication 1, dans laquelle la molécule d'ARNg est modifiée en son extrémité 5' de telle sorte qu'il lui manque un groupe 5'-triphosphate.

3. Composition conforme à l'une des revendications précédentes, dans laquelle la molécule d'ARNg est complexée avec la protéine Cas9 au sein d'un complexe ribonucléoprotéique (RNP).

4. Composition conforme à la revendication 1, dans laquelle la queue 3' poly A comporte entre 15 et 25 nucléotides à adénine.

5. Procédé *in vitro* ou *ex vivo* d'édition ou de modulation de l'expression d'un gène dans une cellule eucaryote provenant d'un sujet, lequel procédé comporte le fait de mettre la cellule en contact avec une composition comprenant une molécule d'ARNg comportant un domaine de ciblage qui est complémentaire d'un domaine cible d'un gène exprimé dans une cellule eucaryote, et dans lequel procédé la molécule d'ARNg :
a) comporte une queue 3' polyA,
b) et est complexée avec une protéine Cas9.

6. Procédé *in vitro* ou *ex vivo* conforme à la revendication 5, dans lequel la molécule d'ARNg est modifiée en son extrémité 5' de telle sorte qu'il lui manque un groupe 5'-triphosphate.

7. Procédé *in vitro* ou *ex vivo* conforme à la revendication 5, dans lequel la molécule d'ARNg est complexée avec une protéine Cas9 au sein d'un complexe ribonucléoprotéique (RNP).

8. Procédé *in vitro* ou *ex vivo* conforme à la revendication 5, dans lequel la queue 3' polyA comporte entre 15 et 25 nucléotides à adénine.
